(19) Europäisches Patentamt
European Patent Office
Office européen des brevets

(11) EP 2 677 035 A1

(12) # EUROPEAN PATENT APPLICATION

(43) Date of publication:
**25.12.2013 Bulletin 2013/52**

(51) Int Cl.:
***C12N 15/82*** *(2006.01)*

(21) Application number: **12173160.8**

(22) Date of filing: **22.06.2012**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicants:
- **BASF Plant Science Company GmbH**
  **67056 Ludwigshafen (DE)**
- **Universiteit Gent**
  **9000 Gent (BE)**
- **VIB vzw**
  **9052 Gent, Zwijnaarde (BE)**

(72) Inventors:
- **Russinova, Jenny**
  **9800 Astene (BE)**
- **Reuzeau, Christophe**
  **24350 La Chapelle Gonaguet (FR)**

(74) Representative: **Oswald, Oliver**
**BASF SE**
**GVX/B - C 6**
**Carl-Bosch-Str. 38**
**67056 Ludwigshafen (DE)**

(54) **Plants having enhanced yield-related traits and a method for making the same**

(57) The present invention relates generally to the field of molecular biology and concerns a method for enhancing various economically important yield-related traits in plants. More specifically, the present invention concerns a method for enhancing one or more yield-related traits in plants by modulating expression in a plant of a nucleic acid encoding a POI (Protein Of Interest) polypeptide. The present invention also concerns plants having modulated expression of a nucleic acid encoding a POI polypeptide, which plants have one or more enhanced yield-related traits compared with control plants. The invention also provides constructs useful in performing the methods of the invention.

EP 2 677 035 A1

SEQUENCE LISTING

```
<110> BASF Plant Science Company GmbH

<120> Plants having enhanced yield-related traits and a method
      for making the same

<130> PF 73670 EP+US

<160> 85

<170> Biomax PatentTool according to PatentIN 3.1 format

<210> 1
<211> 2448
<212> DNA
<213> Arabidopsis thaliana

<220>
<221> CDS
<222> (1)..(2448)

<400> 1
atg aga gct gga ggc tgc acg gtg gag caa gcc ctc acg gcg gat gct        48
Met Arg Ala Gly Gly Cys Thr Val Glu Gln Ala Leu Thr Ala Asp Ala
1               5                   10                  15
gca aac gtg gtg aaa caa gcc atg ggg ttg gct aga cgg aga gga cat        96
Ala Asn Val Val Lys Gln Ala Met Gly Leu Ala Arg Arg Arg Gly His
            20                  25                  30
gct cag gtc aca cct ctc cat gta gct agc acc atg ctc tct gct ccc        144
Ala Gln Val Thr Pro Leu His Val Ala Ser Thr Met Leu Ser Ala Pro
        35                  40                  45
acg ggt tta ctt aga aca gct tgt ctc caa tct cac act cac cct ctt        192
Thr Gly Leu Leu Arg Thr Ala Cys Leu Gln Ser His Thr His Pro Leu
    50                  55                  60
caa tgc aga gcc ctc gag ctc tgc ttc aac gta gct ttg aac cgc ctc        240
Gln Cys Arg Ala Leu Glu Leu Cys Phe Asn Val Ala Leu Asn Arg Leu
65                  70                  75                  80
ccg acc tct aca ggg agt cct atg tta ggg gtt cca act tcc cct ttc        288
Pro Thr Ser Thr Gly Ser Pro Met Leu Gly Val Pro Thr Ser Pro Phe
                85                  90                  95
cct tct atc tcc aac gct ctc ggt gca gct ttc aaa cgc gca caa gct        336
Pro Ser Ile Ser Asn Ala Leu Gly Ala Ala Phe Lys Arg Ala Gln Ala
            100                 105                 110
cac caa cga cgt ggt tct ata gag agc cag caa caa ccg atc tta gca        384
His Gln Arg Arg Gly Ser Ile Glu Ser Gln Gln Gln Pro Ile Leu Ala
        115                 120                 125
gtc aag atc gaa gtg gag cag ctc ata ata tct atc ctc gat gat cct        432
Val Lys Ile Glu Val Glu Gln Leu Ile Ile Ser Ile Leu Asp Asp Pro
    130                 135                 140
agc gtg agt aga gtg atg aga gaa gct ggt ttc tcg agt cct caa gtg        480
Ser Val Ser Arg Val Met Arg Glu Ala Gly Phe Ser Ser Pro Gln Val
145                 150                 155                 160
aag act aaa gtc gag caa gct gtt tct tta gag att tgc tct aaa aca        528
Lys Thr Lys Val Glu Gln Ala Val Ser Leu Glu Ile Cys Ser Lys Thr
                165                 170                 175
acc tct tcg agt aaa ccc aaa gag ggg aag tta tta act cct gtc agg        576
Thr Ser Ser Ser Lys Pro Lys Glu Gly Lys Leu Leu Thr Pro Val Arg
            180                 185                 190
aac gag gat gtt atg aac gtt atc aac aat ctt gtc gac aaa aag aga        624
Asn Glu Asp Val Met Asn Val Ile Asn Asn Leu Val Asp Lys Lys Arg
        195                 200                 205
aga aat ttc gtg atc gta gga gag tgt tta gcc act att gac gga gtt        672
Arg Asn Phe Val Ile Val Gly Glu Cys Leu Ala Thr Ile Asp Gly Val
```

```
    210                 215                 220
gtg aag acg gtg atg gaa aaa gtc gac aaa aaa gac gtg ccc gag gtt        720
Val Lys Thr Val Met Glu Lys Val Asp Lys Lys Asp Val Pro Glu Val
225                 230                 235                 240
tta aaa gac gta aag ttc ata act tta tcg ttc tca tcg ttc ggg caa        768
Leu Lys Asp Val Lys Phe Ile Thr Leu Ser Phe Ser Ser Phe Gly Gln
                245                 250                 255
cca agt aga gcc gat gtg gaa cgt aag cta gag gag cta gag aca ctc        816
Pro Ser Arg Ala Asp Val Glu Arg Lys Leu Glu Glu Leu Glu Thr Leu
            260                 265                 270
gtg aag agc tgt gta ggg aaa gga gtg att cta aat ctg gga gat cta        864
Val Lys Ser Cys Val Gly Lys Gly Val Ile Leu Asn Leu Gly Asp Leu
        275                 280                 285
aac tgg ttc gta gag tca aga acc aga ggt tct tca ctg tat aac aac        912
Asn Trp Phe Val Glu Ser Arg Thr Arg Gly Ser Ser Leu Tyr Asn Asn
    290                 295                 300
aac gat agc tac tgt gtt gtg gag cat atg ata atg gag att ggg aag        960
Asn Asp Ser Tyr Cys Val Val Glu His Met Ile Met Glu Ile Gly Lys
305                 310                 315                 320
ttg gct tgt ggg tta gtc atg gga gat cat gga agg ttt tgg tta atg       1008
Leu Ala Cys Gly Leu Val Met Gly Asp His Gly Arg Phe Trp Leu Met
                325                 330                 335
ggt ctt gcg act tca cag act tac gtg aga tgc aag tct ggt caa ccc       1056
Gly Leu Ala Thr Ser Gln Thr Tyr Val Arg Cys Lys Ser Gly Gln Pro
            340                 345                 350
tcg ctt gaa tct ctc tgg tgt ctc act act ctt act att ccg gcg act       1104
Ser Leu Glu Ser Leu Trp Cys Leu Thr Thr Leu Thr Ile Pro Ala Thr
        355                 360                 365
agt aat agt ctc cgg ttg agt ctc gtc tcc gag agt gag ctt gaa gtc       1152
Ser Asn Ser Leu Arg Leu Ser Leu Val Ser Glu Ser Glu Leu Glu Val
    370                 375                 380
aag aaa tca gag aac gtg tct ctc cag ctg cag caa tca tcg gat cag       1200
Lys Lys Ser Glu Asn Val Ser Leu Gln Leu Gln Gln Ser Ser Asp Gln
385                 390                 395                 400
ctc agt ttc tgt gag gaa tgt tcc gtc aag ttt gaa tcg gaa gct cga       1248
Leu Ser Phe Cys Glu Glu Cys Ser Val Lys Phe Glu Ser Glu Ala Arg
                405                 410                 415
ttc tta aag agt agc aat agc aat gta acc act gta gca tta ccg gca       1296
Phe Leu Lys Ser Ser Asn Ser Asn Val Thr Thr Val Ala Leu Pro Ala
            420                 425                 430
tgg ctt cag cag tat aag aag gag aat caa aat agt cac act gat tca       1344
Trp Leu Gln Gln Tyr Lys Lys Glu Asn Gln Asn Ser His Thr Asp Ser
        435                 440                 445
gat tct att aag gaa ctt gtg gtg aag tgg aac tca atc tgt gat tca       1392
Asp Ser Ile Lys Glu Leu Val Val Lys Trp Asn Ser Ile Cys Asp Ser
    450                 455                 460
atc cac aaa aga cca tct cta aaa aca ctc aca ctc tct tct cct act       1440
Ile His Lys Arg Pro Ser Leu Lys Thr Leu Thr Leu Ser Ser Pro Thr
465                 470                 475                 480
tct tcc ttt tct ggt tcc act caa cct tca atc agt act ctt cat cat       1488
Ser Ser Phe Ser Gly Ser Thr Gln Pro Ser Ile Ser Thr Leu His His
                485                 490                 495
ctt caa acc aac ggt gat tgg ccc gtg atc gag aca aac acg cat cgt       1536
Leu Gln Thr Asn Gly Asp Trp Pro Val Ile Glu Thr Asn Thr His Arg
            500                 505                 510
cat cat tct gtt gtc cat gag aca tct cac tta aga ctg ttc att cca       1584
His His Ser Val Val His Glu Thr Ser His Leu Arg Leu Phe Ile Pro
        515                 520                 525
gaa cat gac agc gag caa aag act gag cta gtc tgt tcg aat cct aat       1632
Glu His Asp Ser Glu Gln Lys Thr Glu Leu Val Cys Ser Asn Pro Asn
    530                 535                 540
tct aca atg aac tct gaa gcc tct tca agc gat gca atg gag ttg gaa       1680
Ser Thr Met Asn Ser Glu Ala Ser Ser Ser Asp Ala Met Glu Leu Glu
545                 550                 555                 560
cat gcc tct tca agg ttt aaa gag atg aat gca gaa aac cta gca act       1728
```

```
His Ala Ser Ser Arg Phe Lys Glu Met Asn Ala Glu Asn Leu Ala Thr
                565                 570                 575
tta tgt gct gcc ttg gaa agc aag gta ccg tgg caa aag gat tta gtc      1776
Leu Cys Ala Ala Leu Glu Ser Lys Val Pro Trp Gln Lys Asp Leu Val
            580                 585                 590
ccg gag tta gcc aaa aca gtc ttg aaa tgc agg tca gga tcg agt acg      1824
Pro Glu Leu Ala Lys Thr Val Leu Lys Cys Arg Ser Gly Ser Ser Thr
        595                 600                 605
agg aag att aac gga aac gag gat aaa aaa gaa gat aca tgg atg ttc      1872
Arg Lys Ile Asn Gly Asn Glu Asp Lys Lys Glu Asp Thr Trp Met Phe
    610                 615                 620
ttc caa ggt ctt gat gta gat gct aaa gag aag atc gct aga gaa ttg      1920
Phe Gln Gly Leu Asp Val Asp Ala Lys Glu Lys Ile Ala Arg Glu Leu
625                 630                 635                 640
gcc aaa ctc gtg ttt gga tcg caa gac agc ttt gtc tct atc tgt ttg      1968
Ala Lys Leu Val Phe Gly Ser Gln Asp Ser Phe Val Ser Ile Cys Leu
                645                 650                 655
agt agt ttc tcg tcg aca aga tcg gat tct gca gaa gat ttg agg aac      2016
Ser Ser Phe Ser Ser Thr Arg Ser Asp Ser Ala Glu Asp Leu Arg Asn
            660                 665                 670
aag agg ttg aga gat gaa caa agc ctg agt tac ata gag aga ttc tcg      2064
Lys Arg Leu Arg Asp Glu Gln Ser Leu Ser Tyr Ile Glu Arg Phe Ser
        675                 680                 685
gaa gct gtt tcg ttg gat cca aat aga gtg atc tta gtt gaa gat att      2112
Glu Ala Val Ser Leu Asp Pro Asn Arg Val Ile Leu Val Glu Asp Ile
    690                 695                 700
gag caa gct gat tat ttg tct caa gtg ggt ttc aag aga gct gtt gag      2160
Glu Gln Ala Asp Tyr Leu Ser Gln Val Gly Phe Lys Arg Ala Val Glu
705                 710                 715                 720
aga gga aga gtt tgt aat tcg agt ggt gaa gaa gct tcg ctt aaa gac      2208
Arg Gly Arg Val Cys Asn Ser Ser Gly Glu Glu Ala Ser Leu Lys Asp
                725                 730                 735
gcg att gtg atc ttg agc tgt gaa aga ttt cgt tca aga tca aga gct      2256
Ala Ile Val Ile Leu Ser Cys Glu Arg Phe Arg Ser Arg Ser Arg Ala
            740                 745                 750
tgt tct cct cct agt aac cag aaa tcg gac ggt tca gat caa cct gag      2304
Cys Ser Pro Pro Ser Asn Gln Lys Ser Asp Gly Ser Asp Gln Pro Glu
        755                 760                 765
gac aag aat gtt gct act tgt gtt gca ctt gat cta aac cta tct att      2352
Asp Lys Asn Val Ala Thr Cys Val Ala Leu Asp Leu Asn Leu Ser Ile
    770                 775                 780
gat agt gcg tac gtt tgt gaa gaa gaa tca tgt gat gag att ggc tta      2400
Asp Ser Ala Tyr Val Cys Glu Glu Glu Ser Cys Asp Glu Ile Gly Leu
785                 790                 795                 800
ctc gaa gcg gta gat gca cgg ttt cat ttc aag tgt tca tca aca taa      2448
Leu Glu Ala Val Asp Ala Arg Phe His Phe Lys Cys Ser Ser Thr
                805                 810                 815
```

<210> 2
<211> 815
<212> PRT
<213> Arabidopsis thaliana

<400> 2

```
Met Arg Ala Gly Gly Cys Thr Val Glu Gln Ala Leu Thr Ala Asp Ala
1               5                   10                  15
Ala Asn Val Val Lys Gln Ala Met Gly Leu Ala Arg Arg Arg Gly His
            20                  25                  30
Ala Gln Val Thr Pro Leu His Val Ala Ser Thr Met Leu Ser Ala Pro
        35                  40                  45
Thr Gly Leu Leu Arg Thr Ala Cys Leu Gln Ser His Thr His Pro Leu
    50                  55                  60
Gln Cys Arg Ala Leu Glu Leu Cys Phe Asn Val Ala Leu Asn Arg Leu
65                  70                  75                  80
Pro Thr Ser Thr Gly Ser Pro Met Leu Gly Val Pro Thr Ser Pro Phe
```

```
                85                  90                  95
Pro Ser Ile Ser Asn Ala Leu Gly Ala Ala Phe Lys Arg Ala Gln Ala
            100                 105                 110
His Gln Arg Arg Gly Ser Ile Glu Ser Gln Gln Gln Pro Ile Leu Ala
        115                 120                 125
Val Lys Ile Glu Val Glu Gln Leu Ile Ile Ser Ile Leu Asp Asp Pro
    130                 135                 140
Ser Val Ser Arg Val Met Arg Glu Ala Gly Phe Ser Ser Pro Gln Val
145                 150                 155                 160
Lys Thr Lys Val Glu Gln Ala Val Ser Leu Glu Ile Cys Ser Lys Thr
                165                 170                 175
Thr Ser Ser Ser Lys Pro Lys Glu Gly Lys Leu Leu Thr Pro Val Arg
            180                 185                 190
Asn Glu Asp Val Met Asn Val Ile Asn Asn Leu Val Asp Lys Lys Arg
        195                 200                 205
Arg Asn Phe Val Ile Val Gly Glu Cys Leu Ala Thr Ile Asp Gly Val
    210                 215                 220
Val Lys Thr Val Met Glu Lys Val Asp Lys Lys Asp Val Pro Glu Val
225                 230                 235                 240
Leu Lys Asp Val Lys Phe Ile Thr Leu Ser Phe Ser Ser Phe Gly Gln
                245                 250                 255
Pro Ser Arg Ala Asp Val Glu Arg Lys Leu Glu Glu Leu Glu Thr Leu
            260                 265                 270
Val Lys Ser Cys Val Gly Lys Gly Val Ile Leu Asn Leu Gly Asp Leu
        275                 280                 285
Asn Trp Phe Val Glu Ser Arg Thr Arg Gly Ser Ser Leu Tyr Asn Asn
    290                 295                 300
Asn Asp Ser Tyr Cys Val Val Glu His Met Ile Met Glu Ile Gly Lys
305                 310                 315                 320
Leu Ala Cys Gly Leu Val Met Gly Asp His Gly Arg Phe Trp Leu Met
                325                 330                 335
Gly Leu Ala Thr Ser Gln Thr Tyr Val Arg Cys Lys Ser Gly Gln Pro
            340                 345                 350
Ser Leu Glu Ser Leu Trp Cys Leu Thr Thr Leu Thr Ile Pro Ala Thr
        355                 360                 365
Ser Asn Ser Leu Arg Leu Ser Leu Val Ser Glu Ser Glu Leu Glu Val
    370                 375                 380
Lys Lys Ser Glu Asn Val Ser Leu Gln Leu Gln Gln Ser Ser Asp Gln
385                 390                 395                 400
Leu Ser Phe Cys Glu Glu Cys Ser Val Lys Phe Glu Ser Glu Ala Arg
                405                 410                 415
Phe Leu Lys Ser Ser Asn Ser Asn Val Thr Thr Val Ala Leu Pro Ala
            420                 425                 430
Trp Leu Gln Gln Tyr Lys Lys Glu Asn Gln Asn Ser His Thr Asp Ser
        435                 440                 445
Asp Ser Ile Lys Glu Leu Val Val Lys Trp Asn Ser Ile Cys Asp Ser
    450                 455                 460
Ile His Lys Arg Pro Ser Leu Lys Thr Leu Thr Leu Ser Ser Pro Thr
465                 470                 475                 480
Ser Ser Phe Ser Gly Ser Thr Gln Pro Ser Ile Ser Thr Leu His His
                485                 490                 495
Leu Gln Thr Asn Gly Asp Trp Pro Val Ile Glu Thr Asn Thr His Arg
            500                 505                 510
His His Ser Val Val His Glu Thr Ser His Leu Arg Leu Phe Ile Pro
        515                 520                 525
Glu His Asp Ser Glu Gln Lys Thr Glu Leu Val Cys Ser Asn Pro Asn
    530                 535                 540
Ser Thr Met Asn Ser Glu Ala Ser Ser Ser Asp Ala Met Glu Leu Glu
545                 550                 555                 560
His Ala Ser Ser Arg Phe Lys Glu Met Asn Ala Glu Asn Leu Ala Thr
                565                 570                 575
Leu Cys Ala Ala Leu Glu Ser Lys Val Pro Trp Gln Lys Asp Leu Val
            580                 585                 590
Pro Glu Leu Ala Lys Thr Val Leu Lys Cys Arg Ser Gly Ser Ser Thr
        595                 600                 605
```

```
Arg Lys Ile Asn Gly Asn Glu Asp Lys Lys Glu Asp Thr Trp Met Phe
    610                 615                 620
Phe Gln Gly Leu Asp Val Asp Ala Lys Glu Lys Ile Ala Arg Glu Leu
625                 630                 635                 640
Ala Lys Leu Val Phe Gly Ser Gln Asp Ser Phe Val Ser Ile Cys Leu
                645                 650                 655
Ser Ser Phe Ser Ser Thr Arg Ser Asp Ser Ala Glu Asp Leu Arg Asn
            660                 665                 670
Lys Arg Leu Arg Asp Glu Gln Ser Leu Ser Tyr Ile Glu Arg Phe Ser
        675                 680                 685
Glu Ala Val Ser Leu Asp Pro Asn Arg Val Ile Leu Val Glu Asp Ile
    690                 695                 700
Glu Gln Ala Asp Tyr Leu Ser Gln Val Gly Phe Lys Arg Ala Val Glu
705                 710                 715                 720
Arg Gly Arg Val Cys Asn Ser Ser Gly Glu Glu Ala Ser Leu Lys Asp
                725                 730                 735
Ala Ile Val Ile Leu Ser Cys Glu Arg Phe Arg Ser Arg Ser Arg Ala
            740                 745                 750
Cys Ser Pro Pro Ser Asn Gln Lys Ser Asp Gly Ser Asp Gln Pro Glu
        755                 760                 765
Asp Lys Asn Val Ala Thr Cys Val Ala Leu Asp Leu Asn Leu Ser Ile
    770                 775                 780
Asp Ser Ala Tyr Val Cys Glu Glu Glu Ser Cys Asp Glu Ile Gly Leu
785                 790                 795                 800
Leu Glu Ala Val Asp Ala Arg Phe His Phe Lys Cys Ser Ser Thr
                805                 810                 815

<210> 3
<211> 2361
<212> DNA
<213> Zea mays

<220>
<221> CDS
<222> (1)..(2361)

<400> 3
atg cgt agc ggc ggc tgc gcg gtg cag cag aag cta gcg ggg gac gcg       48
Met Arg Ser Gly Gly Cys Ala Val Gln Gln Lys Leu Ala Gly Asp Ala
1               5                   10                  15
gcc gcc gtg atg cgg cag gcg gtg agc ctg gcg cgt cgc cgg ggg cac       96
Ala Ala Val Met Arg Gln Ala Val Ser Leu Ala Arg Arg Arg Gly His
            20                  25                  30
gcg cag gtc acg ccg ctg cac gtc gcc agc gcc gtg ctc ttg gac gcc      144
Ala Gln Val Thr Pro Leu His Val Ala Ser Ala Val Leu Leu Asp Ala
        35                  40                  45
ggg ggc ctc ctc cgc gcc gcc tgc ctc cgg tcg cgg gcg act tcc cac      192
Gly Gly Leu Leu Arg Ala Ala Cys Leu Arg Ser Arg Ala Thr Ser His
    50                  55                  60
ccg ctc cag tgc aag gcg ctg gag ctc tgc ttc aac gtc gcc ctc aac      240
Pro Leu Gln Cys Lys Ala Leu Glu Leu Cys Phe Asn Val Ala Leu Asn
65                  70                  75                  80
cgc ctc gcg acg gcg ggg ggc ccg gcc ccg ccg ccg gcc atg ttc gag      288
Arg Leu Ala Thr Ala Gly Gly Pro Ala Pro Pro Pro Ala Met Phe Glu
                85                  90                  95
ttc cac atc cac tcc ggc cac cgc gag ccg gcg ctg tcc aac gcg ctc      336
Phe His Ile His Ser Gly His Arg Glu Pro Ala Leu Ser Asn Ala Leu
            100                 105                 110
gcc gcc gcg ttc aag cgc gcg cag ggg aac cag cgc cgg ggc ggc ggc      384
Ala Ala Ala Phe Lys Arg Ala Gln Gly Asn Gln Arg Arg Gly Gly Gly
        115                 120                 125
tcg gcc gcg gac ggc cag cac cag cag aac gtc gcc gcc aag gtg gag      432
Ser Ala Ala Asp Gly Gln His Gln Gln Asn Val Ala Ala Lys Val Glu
    130                 135                 140
ctc gac caa ctc gtc atc tcc atc ctc gac gac ccc agc gtc agc cgc      480
```

```
Leu Asp Gln Leu Val Ile Ser Ile Leu Asp Asp Pro Ser Val Ser Arg
145                 150                 155                 160
gtc atg cgc gag gcc ggc ttc tcg agc gcc gag gtc aag gcc aac gtc      528
Val Met Arg Glu Ala Gly Phe Ser Ser Ala Glu Val Lys Ala Asn Val
                165                 170                 175
gag aag gcc atc tcg tcg tcg gag cag tcc tcc aac acg gca agc agc      576
Glu Lys Ala Ile Ser Ser Ser Glu Gln Ser Ser Asn Thr Ala Ser Ser
            180                 185                 190
agc agc gct tcc ccg agt acc att acc aag gag ccc aga gcc aag gcc      624
Ser Ser Ala Ser Pro Ser Thr Ile Thr Lys Glu Pro Arg Ala Lys Ala
        195                 200                 205
gac gcc gtc caa gtc gta ggt gac gcc gcg cgc gtg ctc gac tgc atg      672
Asp Ala Val Gln Val Val Gly Asp Ala Ala Arg Val Leu Asp Cys Met
    210                 215                 220
gcg agc ggg acg aac cgg tgc gtg gcg gtc gtc ggc gag agc gcc gcc      720
Ala Ser Gly Thr Asn Arg Cys Val Ala Val Val Gly Glu Ser Ala Ala
225                 230                 235                 240
gcc gcg gag ggt gtg gtc aag gcg gtg atg gac aag gtg agc aag ggg      768
Ala Ala Glu Gly Val Val Lys Ala Val Met Asp Lys Val Ser Lys Gly
                245                 250                 255
gag ctg cgg cgt cag cac cag cgc ctc aag aac gcc cag ttc gtg ccc      816
Glu Leu Arg Arg Gln His Gln Arg Leu Lys Asn Ala Gln Phe Val Pro
            260                 265                 270
ttc tcg gcc gcg tcc ttc cag cgc acg ccg agg gag gag gtc gag gcc      864
Phe Ser Ala Ala Ser Phe Gln Arg Thr Pro Arg Glu Glu Val Glu Ala
        275                 280                 285
agg gcc ggc gac ctg tgc gcg ctc gtg cgc gag tgc tgc gcc gcc ggc      912
Arg Ala Gly Asp Leu Cys Ala Leu Val Arg Glu Cys Cys Ala Ala Gly
    290                 295                 300
aaa ggc gtc gtg ctc gtc ctc gag gac ctc ggc tac gcg gcg gag gcc      960
Lys Gly Val Val Leu Val Leu Glu Asp Leu Gly Tyr Ala Ala Glu Ala
305                 310                 315                 320
tgg acc gcc gcg ctg tgg acg aga agt gac cgc agc gcg cgt ggc ctc      1008
Trp Thr Ala Ala Leu Trp Thr Arg Ser Asp Arg Ser Ala Arg Gly Leu
                325                 330                 335
cgc cac tac tgc ccc gtc gag cac gcg gtc atg gag ttg agc agc ctc      1056
Arg His Tyr Cys Pro Val Glu His Ala Val Met Glu Leu Ser Ser Leu
            340                 345                 350
gtg cgc ggc ggc gga ggc cgc gac aag gac atg ttc tgg gtg ctg ggg      1104
Val Arg Gly Gly Gly Gly Arg Asp Lys Asp Met Phe Trp Val Leu Gly
        355                 360                 365
ttc gcg gcc tat gcg ccc tac acg agc tgt agg tcg ggg cag cct tct      1152
Phe Ala Ala Tyr Ala Pro Tyr Thr Ser Cys Arg Ser Gly Gln Pro Ser
    370                 375                 380
ctg gag acc gtc ctg gga cta cgc ccc gtc gtc gtg ccg gac ggc agc      1200
Leu Glu Thr Val Leu Gly Leu Arg Pro Val Val Val Pro Asp Gly Ser
385                 390                 395                 400
ctc ggc ggc gaa agt gag gaa aca cac tgc ggc gcc ggc acg gtg gtg      1248
Leu Gly Gly Glu Ser Glu Glu Thr His Cys Gly Ala Gly Thr Val Val
                405                 410                 415
gcg ccg gcg agc gtc ccc tcg tgg att cgc cgt tgc caa ggt cca gta      1296
Ala Pro Ala Ser Val Pro Ser Trp Ile Arg Arg Cys Gln Gly Pro Val
            420                 425                 430
gtc gtc act gga tcg gcg ctc acg ctg agc ttc tca tct ccg gct tct      1344
Val Val Thr Gly Ser Ala Leu Thr Leu Ser Phe Ser Ser Pro Ala Ser
        435                 440                 445
tcc tcc cac tgt ggc ttt acc cat cat cat gac gac acc aac aag agc      1392
Ser Ser His Cys Gly Phe Thr His His His Asp Asp Thr Asn Lys Ser
    450                 455                 460
tgc gtg cca tgg cat gat ctc atg gac cag cag cag caa aag ctg ttg      1440
Cys Val Pro Trp His Asp Leu Met Asp Gln Gln Gln Gln Lys Leu Leu
465                 470                 475                 480
ctg aac cgc cgg cac gat ggt cca ctg gcc gag cca tac gat cag ctg      1488
Leu Asn Arg Arg His Asp Gly Pro Leu Ala Glu Pro Tyr Asp Gln Leu
                485                 490                 495
```

```
tcg ccc gca aac cct aat tca gga tcc tct aat aac tcg gtg tct aga      1536
Ser Pro Ala Asn Pro Asn Ser Gly Ser Ser Asn Asn Ser Val Ser Arg
            500                 505                 510
tct aac tca tcg gac ggc gcg acg acg gct tgc cgc cgc cgg cca aag      1584
Ser Asn Ser Ser Asp Gly Ala Thr Thr Ala Cys Arg Arg Arg Pro Lys
        515                 520                 525
ttc acc gag ctc agc gcg gag aat ctc aag atc ctg tgc cgc gcg ctg      1632
Phe Thr Glu Leu Ser Ala Glu Asn Leu Lys Ile Leu Cys Arg Ala Leu
    530                 535                 540
gag acg cgc gta ccg cgc cac aga gac ata gct ccg ggt atc gcc agc      1680
Glu Thr Arg Val Pro Arg His Arg Asp Ile Ala Pro Gly Ile Ala Ser
545                 550                 555                 560
gcc gtg ctc cag cgc cgc tcc ggc gtg acg agg acg gcg cgg ccg acc      1728
Ala Val Leu Gln Arg Arg Ser Gly Val Thr Arg Thr Ala Arg Pro Thr
                565                 570                 575
ccg gcg acg tgg ctc ctc ttc cga ggg agg gac aac gac ggc aag atg      1776
Pro Ala Thr Trp Leu Leu Phe Arg Gly Arg Asp Asn Asp Gly Lys Met
            580                 585                 590
tcc atg gct cgg gag ctt gcc agg ctt gtc ttt ggc tcg cac gac gac      1824
Ser Met Ala Arg Glu Leu Ala Arg Leu Val Phe Gly Ser His Asp Asp
        595                 600                 605
ttc acc agc atc gcc gca gca gcg tca aag ctc gca ccg gat cac tcc      1872
Phe Thr Ser Ile Ala Ala Ala Ala Ser Lys Leu Ala Pro Asp His Ser
    610                 615                 620
ggt tcc agc agc ccc ggc aag cac agc ctc aag agg cag agg tcg ccg      1920
Gly Ser Ser Ser Pro Gly Lys His Ser Leu Lys Arg Gln Arg Ser Pro
625                 630                 635                 640
ccg gac aac gag cac gga ggc ttc atg cag acg ttc tac gag gcg atc      1968
Pro Asp Asn Glu His Gly Gly Phe Met Gln Thr Phe Tyr Glu Ala Ile
                645                 650                 655
cgc gag aac cct cac cgt gtc gtc ctg atc gat ggc gtc gag cac cac      2016
Arg Glu Asn Pro His Arg Val Val Leu Ile Asp Gly Val Glu His His
            660                 665                 670
tcg aaa cta caa gcc ggc atc atg ggt tcc atg gag aac gga act gtg      2064
Ser Lys Leu Gln Ala Gly Ile Met Gly Ser Met Glu Asn Gly Thr Val
        675                 680                 685
agg ggt tgc gat ggt ggt gtc gtc agc ttg gag gac tcc atc gta gtg      2112
Arg Gly Cys Asp Gly Gly Val Val Ser Leu Glu Asp Ser Ile Val Val
    690                 695                 700
tgc tgt gaa gcg ttc gag tcg aga tcg ttg cct cgg gtt tct tca tcc      2160
Cys Cys Glu Ala Phe Glu Ser Arg Ser Leu Pro Arg Val Ser Ser Ser
705                 710                 715                 720
ccg cgg cct gtg aag caa atg att acg agt gac gtc gac agc aag gtg      2208
Pro Arg Pro Val Lys Gln Met Ile Thr Ser Asp Val Asp Ser Lys Val
                725                 730                 735
gaa ggt gat gac gca gat aaa gga gtc gtg cca cat ttc agc ttg gat      2256
Glu Gly Asp Asp Ala Asp Lys Gly Val Val Pro His Phe Ser Leu Asp
            740                 745                 750
ttg aat acc tgc gcc att gac gac ggc gaa ggg gag gaa gcg agt agt      2304
Leu Asn Thr Cys Ala Ile Asp Asp Gly Glu Gly Glu Glu Ala Ser Ser
        755                 760                 765
tct tgg tat gat gcc atg gag atc caa aat gat gta gac ggc gtt ttc      2352
Ser Trp Tyr Asp Ala Met Glu Ile Gln Asn Asp Val Asp Gly Val Phe
    770                 775                 780
ttc ttc tag                                                          2361
Phe Phe
785
```

<210> 4
<211> 786
<212> PRT
<213> Zea mays

<400> 4
Met Arg Ser Gly Gly Cys Ala Val Gln Gln Lys Leu Ala Gly Asp Ala

```
1               5                   10                  15
Ala Ala Val Met Arg Gln Ala Val Ser Leu Ala Arg Arg Arg Gly His
            20                  25                  30
Ala Gln Val Thr Pro Leu His Val Ala Ser Ala Val Leu Leu Asp Ala
        35                  40                  45
Gly Gly Leu Leu Arg Ala Ala Cys Leu Arg Ser Arg Ala Thr Ser His
    50                  55                  60
Pro Leu Gln Cys Lys Ala Leu Glu Leu Cys Phe Asn Val Ala Leu Asn
65                  70                  75                  80
Arg Leu Ala Thr Ala Gly Gly Pro Ala Pro Pro Pro Ala Met Phe Glu
                85                  90                  95
Phe His Ile His Ser Gly His Arg Glu Pro Ala Leu Ser Asn Ala Leu
            100                 105                 110
Ala Ala Ala Phe Lys Arg Ala Gln Gly Asn Gln Arg Arg Gly Gly Gly
        115                 120                 125
Ser Ala Ala Asp Gly Gln His Gln Gln Asn Val Ala Ala Lys Val Glu
    130                 135                 140
Leu Asp Gln Leu Val Ile Ser Ile Leu Asp Asp Pro Ser Val Ser Arg
145                 150                 155                 160
Val Met Arg Glu Ala Gly Phe Ser Ser Ala Glu Val Lys Ala Asn Val
                165                 170                 175
Glu Lys Ala Ile Ser Ser Ser Glu Gln Ser Ser Asn Thr Ala Ser Ser
            180                 185                 190
Ser Ser Ala Ser Pro Ser Thr Ile Thr Lys Glu Pro Arg Ala Lys Ala
        195                 200                 205
Asp Ala Val Gln Val Val Gly Asp Ala Ala Arg Val Leu Asp Cys Met
    210                 215                 220
Ala Ser Gly Thr Asn Arg Cys Val Ala Val Val Gly Glu Ser Ala Ala
225                 230                 235                 240
Ala Ala Glu Gly Val Val Lys Ala Val Met Asp Lys Val Ser Lys Gly
                245                 250                 255
Glu Leu Arg Arg Gln His Gln Arg Leu Lys Asn Ala Gln Phe Val Pro
            260                 265                 270
Phe Ser Ala Ala Ser Phe Gln Arg Thr Pro Arg Glu Glu Val Glu Ala
        275                 280                 285
Arg Ala Gly Asp Leu Cys Ala Leu Val Arg Glu Cys Cys Ala Ala Gly
    290                 295                 300
Lys Gly Val Val Leu Val Leu Glu Asp Leu Gly Tyr Ala Ala Glu Ala
305                 310                 315                 320
Trp Thr Ala Ala Leu Trp Thr Arg Ser Asp Arg Ser Ala Arg Gly Leu
                325                 330                 335
Arg His Tyr Cys Pro Val Glu His Ala Val Met Glu Leu Ser Ser Leu
            340                 345                 350
Val Arg Gly Gly Gly Gly Arg Asp Lys Asp Met Phe Trp Val Leu Gly
        355                 360                 365
Phe Ala Ala Tyr Ala Pro Tyr Thr Ser Cys Arg Ser Gly Gln Pro Ser
    370                 375                 380
Leu Glu Thr Val Leu Gly Leu Arg Pro Val Val Val Pro Asp Gly Ser
385                 390                 395                 400
Leu Gly Gly Glu Ser Glu Glu Thr His Cys Gly Ala Gly Thr Val Val
                405                 410                 415
Ala Pro Ala Ser Val Pro Ser Trp Ile Arg Arg Cys Gln Gly Pro Val
            420                 425                 430
Val Val Thr Gly Ser Ala Leu Thr Leu Ser Phe Ser Ser Pro Ala Ser
        435                 440                 445
Ser Ser His Cys Gly Phe Thr His His His Asp Asp Thr Asn Lys Ser
    450                 455                 460
Cys Val Pro Trp His Asp Leu Met Asp Gln Gln Gln Gln Lys Leu Leu
465                 470                 475                 480
Leu Asn Arg Arg His Asp Gly Pro Leu Ala Glu Pro Tyr Asp Gln Leu
                485                 490                 495
Ser Pro Ala Asn Pro Asn Ser Gly Ser Ser Asn Asn Ser Val Ser Arg
            500                 505                 510
Ser Asn Ser Ser Asp Gly Ala Thr Thr Ala Cys Arg Arg Arg Pro Lys
        515                 520                 525
```

```
Phe Thr Glu Leu Ser Ala Glu Asn Leu Lys Ile Leu Cys Arg Ala Leu
    530                 535                 540
Glu Thr Arg Val Pro Arg His Arg Asp Ile Ala Pro Gly Ile Ala Ser
545                 550                 555                 560
Ala Val Leu Gln Arg Arg Ser Gly Val Thr Arg Thr Ala Arg Pro Thr
                565                 570                 575
Pro Ala Thr Trp Leu Leu Phe Arg Gly Arg Asp Asn Asp Gly Lys Met
            580                 585                 590
Ser Met Ala Arg Glu Leu Ala Arg Leu Val Phe Gly Ser His Asp Asp
        595                 600                 605
Phe Thr Ser Ile Ala Ala Ala Ala Ser Lys Leu Ala Pro Asp His Ser
    610                 615                 620
Gly Ser Ser Ser Pro Gly Lys His Ser Leu Lys Arg Gln Arg Ser Pro
625                 630                 635                 640
Pro Asp Asn Glu His Gly Gly Phe Met Gln Thr Phe Tyr Glu Ala Ile
                645                 650                 655
Arg Glu Asn Pro His Arg Val Val Leu Ile Asp Gly Val Glu His His
            660                 665                 670
Ser Lys Leu Gln Ala Gly Ile Met Gly Ser Met Glu Asn Gly Thr Val
        675                 680                 685
Arg Gly Cys Asp Gly Gly Val Val Ser Leu Glu Asp Ser Ile Val Val
    690                 695                 700
Cys Cys Glu Ala Phe Glu Ser Arg Ser Leu Pro Arg Val Ser Ser Ser
705                 710                 715                 720
Pro Arg Pro Val Lys Gln Met Ile Thr Ser Asp Val Asp Ser Lys Val
                725                 730                 735
Glu Gly Asp Asp Ala Asp Lys Gly Val Val Pro His Phe Ser Leu Asp
            740                 745                 750
Leu Asn Thr Cys Ala Ile Asp Asp Gly Glu Gly Glu Glu Ala Ser Ser
        755                 760                 765
Ser Trp Tyr Asp Ala Met Glu Ile Gln Asn Asp Val Asp Gly Val Phe
    770                 775                 780
Phe Phe
785

<210> 5
<211> 2520
<212> DNA
<213> Glycine max

<220>
<221> CDS
<222> (1)..(2520)

<400> 5
atg aga gga ggt att tgt agc ata cag ctt cag gct ctt aca cct gaa       48
Met Arg Gly Gly Ile Cys Ser Ile Gln Leu Gln Ala Leu Thr Pro Glu
  1               5                  10                  15
gct gca act gtt gtt aag caa gct gtt aat ctt gca aca aga aga ggc       96
Ala Ala Thr Val Val Lys Gln Ala Val Asn Leu Ala Thr Arg Arg Gly
            20                  25                  30
cat gca caa gtg aca cct ctt cat gtt gca agt gcc atg ctt gca act      144
His Ala Gln Val Thr Pro Leu His Val Ala Ser Ala Met Leu Ala Thr
        35                  40                  45
tcc aca ggc ctt ctc cgc aag gct tgt ctt cag tgt cac tct cac ccc      192
Ser Thr Gly Leu Leu Arg Lys Ala Cys Leu Gln Cys His Ser His Pro
    50                  55                  60
ctc cag tgc aag gct ctt gag ctt tgt ttc aac gtt gca ctc aac cgt      240
Leu Gln Cys Lys Ala Leu Glu Leu Cys Phe Asn Val Ala Leu Asn Arg
 65                  70                  75                  80
cta cca gct tcc act tca agc cct ctt ctg gca cct caa tat tcc acc      288
Leu Pro Ala Ser Thr Ser Ser Pro Leu Leu Ala Pro Gln Tyr Ser Thr
                85                  90                  95
cct tca ctc tcc aat gcc ttg gtt gca gcc ttc aaa cgt gct cag gct      336
Pro Ser Leu Ser Asn Ala Leu Val Ala Ala Phe Lys Arg Ala Gln Ala
```

```
          100                     105                     110
cac caa cgc cgt ggt tct att gag aac cag cag cag cat att ctg gcc        384
His Gln Arg Arg Gly Ser Ile Glu Asn Gln Gln Gln His Ile Leu Ala
      115                     120                     125
ttg aag att gag gtg gag cag ctc gtg att tca att ctt gat gat cct        432
Leu Lys Ile Glu Val Glu Gln Leu Val Ile Ser Ile Leu Asp Asp Pro
  130                     135                     140
agt gtt agc agg gtc atg aga gaa gct ggt ttc tct agc acc ctt gtg        480
Ser Val Ser Arg Val Met Arg Glu Ala Gly Phe Ser Ser Thr Leu Val
145                     150                     155                     160
aaa aca agg gtc gaa caa gct gtt tca atg gaa gtt tgt tca caa aag        528
Lys Thr Arg Val Glu Gln Ala Val Ser Met Glu Val Cys Ser Gln Lys
                165                     170                     175
gct caa gca aaa gaa aat atc act aag cct cat cat caa gtt gtt ctt        576
Ala Gln Ala Lys Glu Asn Ile Thr Lys Pro His His Gln Val Val Leu
          180                     185                     190
ggt ggt aga aat aat gtg tct cca tct gga cct ttt ggt caa gtt ggt        624
Gly Gly Arg Asn Asn Val Ser Pro Ser Gly Pro Phe Gly Gln Val Gly
      195                     200                     205
ggc tcc ttc atg aag cct aat ttg gac cat gtt aac aat gat gat gtg        672
Gly Ser Phe Met Lys Pro Asn Leu Asp His Val Asn Asn Asp Asp Val
  210                     215                     220
act agt gtg ttg agt gag ttg gca aag aga aga aac aca gtt att gta        720
Thr Ser Val Leu Ser Glu Leu Ala Lys Arg Arg Asn Thr Val Ile Val
225                     230                     235                     240
ggg gag agt gtg acc aat gct gag ggt gta gtt agg gga gtg ata gag        768
Gly Glu Ser Val Thr Asn Ala Glu Gly Val Val Arg Gly Val Ile Glu
                245                     250                     255
agg ttt gag gtt ggg aat gtt cct gga gac ttg aga aac att agc aag        816
Arg Phe Glu Val Gly Asn Val Pro Gly Asp Leu Arg Asn Ile Ser Lys
          260                     265                     270
gag gag gtt gaa cag aag ctt atg gag gtt aga aac ctt gtg aag agc        864
Glu Glu Val Glu Gln Lys Leu Met Glu Val Arg Asn Leu Val Lys Ser
      275                     280                     285
tat gtg gga gga ggg gtt gtt ctt tac ttg ggt gat cta aaa tgg ctg        912
Tyr Val Gly Gly Gly Val Val Leu Tyr Leu Gly Asp Leu Lys Trp Leu
  290                     295                     300
ttt gag ttc tgg gct aat ttt cgt gag caa aaa aca aac tac tgt tct        960
Phe Glu Phe Trp Ala Asn Phe Arg Glu Gln Lys Thr Asn Tyr Cys Ser
305                     310                     315                     320
gta gag cac atg gtc atg gag ctt aaa aaa ttg gta tgt ggt agt ggg       1008
Val Glu His Met Val Met Glu Leu Lys Lys Leu Val Cys Gly Ser Gly
                325                     330                     335
gag tct agt aga ttg tgg ctt atg ggg att tca act ttt aag aca tac       1056
Glu Ser Ser Arg Leu Trp Leu Met Gly Ile Ser Thr Phe Lys Thr Tyr
          340                     345                     350
atg aag tgt aaa ata tgt cac cca tcc cta gaa acc att tgg gag ctt       1104
Met Lys Cys Lys Ile Cys His Pro Ser Leu Glu Thr Ile Trp Glu Leu
      355                     360                     365
cat ccc ttc aca att cct gtt ggc atc ctc agc cta agt tta aat ctt       1152
His Pro Phe Thr Ile Pro Val Gly Ile Leu Ser Leu Ser Leu Asn Leu
  370                     375                     380
gac agt gat ttt caa gct caa gag aga aac aag gta ttt ttc aag gat       1200
Asp Ser Asp Phe Gln Ala Gln Glu Arg Asn Lys Val Phe Phe Lys Asp
385                     390                     395                     400
gtg gct ttt gaa gat aga gca gga gtc aga aac cat cta act tgc tgc       1248
Val Ala Phe Glu Asp Arg Ala Gly Val Arg Asn His Leu Thr Cys Cys
                405                     410                     415
agg gat tgc aca ata aat ttt gaa aaa gaa gct cag agc ata act agc       1296
Arg Asp Cys Thr Ile Asn Phe Glu Lys Glu Ala Gln Ser Ile Thr Ser
          420                     425                     430
act ata agt aag aaa gct tgc act act agt agc ttg cca acg tgg ctc       1344
Thr Ile Ser Lys Lys Ala Cys Thr Thr Ser Ser Leu Pro Thr Trp Leu
      435                     440                     445
caa aat tgt aag gaa gag aga agt gac ata atg gaa gat cag gaa aat       1392
```

```
Gln Asn Cys Lys Glu Glu Arg Ser Asp Ile Met Glu Asp Gln Glu Asn
    450                 455                 460
gca agg ctt aag gat ctg tgc aag aaa tgg aac tca tta tgc aac tca      1440
Ala Arg Leu Lys Asp Leu Cys Lys Lys Trp Asn Ser Leu Cys Asn Ser
465                 470                 475                 480
ata cac aga cac cct tcc att aat gag aaa caa gtt ttc ttt gtt tca      1488
Ile His Arg His Pro Ser Ile Asn Glu Lys Gln Val Phe Phe Val Ser
                485                 490                 495
tca tct cct tca tct ccc act tct gtt tcc tca cat gaa aga aag tcc      1536
Ser Ser Pro Ser Ser Pro Thr Ser Val Ser Ser His Glu Arg Lys Ser
            500                 505                 510
aac ttt cac cac agc cac cta aat tgg cca atc att tct gaa tca gaa      1584
Asn Phe His His Ser His Leu Asn Trp Pro Ile Ile Ser Glu Ser Glu
        515                 520                 525
aag tca cca aaa gaa tgt gag tta tac act gaa act ggt gat gat ggc      1632
Lys Ser Pro Lys Glu Cys Glu Leu Tyr Thr Glu Thr Gly Asp Asp Gly
    530                 535                 540
tat gat agc aac ttt ata atg ttc atg cca gat agt gat gtc cct aaa      1680
Tyr Asp Ser Asn Phe Ile Met Phe Met Pro Asp Ser Asp Val Pro Lys
545                 550                 555                 560
cca gat ctt ctg tca aat ccc aac tct agc ccc aac tca gct tct tca      1728
Pro Asp Leu Leu Ser Asn Pro Asn Ser Ser Pro Asn Ser Ala Ser Ser
                565                 570                 575
agc gaa gca gtg gat ggt ttg gaa agc act caa atg ttc aaa gaa cct      1776
Ser Glu Ala Val Asp Gly Leu Glu Ser Thr Gln Met Phe Lys Glu Pro
            580                 585                 590
aat gct gag aat cat aag att ctg tgt gat gcc ttg gag aaa aag gtt      1824
Asn Ala Glu Asn His Lys Ile Leu Cys Asp Ala Leu Glu Lys Lys Val
        595                 600                 605
cca cag cat aaa gaa gtt att cca gag att gca agt acc gtc ctt cat      1872
Pro Gln His Lys Glu Val Ile Pro Glu Ile Ala Ser Thr Val Leu His
    610                 615                 620
tgt aga tca gga atg aga aag aga gat cag aac cac tcg atg aag aga      1920
Cys Arg Ser Gly Met Arg Lys Arg Asp Gln Asn His Ser Met Lys Arg
625                 630                 635                 640
gaa gac aat caa gaa aca tgg atg ttc ttt ctt ggt gtg aat tct caa      1968
Glu Asp Asn Gln Glu Thr Trp Met Phe Phe Leu Gly Val Asn Ser Gln
                645                 650                 655
gcc aaa gag agc atc tca aga gag cta gct aaa gtt gtt ttt ggc tct      2016
Ala Lys Glu Ser Ile Ser Arg Glu Leu Ala Lys Val Val Phe Gly Ser
            660                 665                 670
tat agc aac ttt gtc aca att ggc atg agt agt ttc tct tcc cca gaa      2064
Tyr Ser Asn Phe Val Thr Ile Gly Met Ser Ser Phe Ser Ser Pro Glu
        675                 680                 685
gat gat gat gat tcc act gat gaa aaa tcc aaa agg aag agg cca aga      2112
Asp Asp Asp Asp Ser Thr Asp Glu Lys Ser Lys Arg Lys Arg Pro Arg
    690                 695                 700
gaa gag ttg aaa agc agt tat gct caa aga ttc ggg gaa gca gtg aat      2160
Glu Glu Leu Lys Ser Ser Tyr Ala Gln Arg Phe Gly Glu Ala Val Asn
705                 710                 715                 720
gag aac cct cac agg gtg ttc ttc tta gaa gac ttg gat caa gtt gat      2208
Glu Asn Pro His Arg Val Phe Phe Leu Glu Asp Leu Asp Gln Val Asp
                725                 730                 735
tac ttt tct caa aag ggt gta gag caa gca att caa agt gga agc ata      2256
Tyr Phe Ser Gln Lys Gly Val Glu Gln Ala Ile Gln Ser Gly Ser Ile
            740                 745                 750
aca ctt cct ggt ggt gaa tct gtt cct ctc atg gat gcc att gtc att      2304
Thr Leu Pro Gly Gly Glu Ser Val Pro Leu Met Asp Ala Ile Val Ile
        755                 760                 765
ttc agt tgc gaa agc ttc ttc tct tca cca aag ctg aga aaa tca cca      2352
Phe Ser Cys Glu Ser Phe Phe Ser Ser Pro Lys Leu Arg Lys Ser Pro
    770                 775                 780
tgt gct gaa aac aaa ggg aaa gag act gtg gaa gat gaa agt tct agc      2400
Cys Ala Glu Asn Lys Gly Lys Glu Thr Val Glu Asp Glu Ser Ser Ser
785                 790                 795                 800
```

```
ctc tct ttg gat ctg aac ata gcc att gaa gat gaa agt gga ggt gtg       2448
Leu Ser Leu Asp Leu Asn Ile Ala Ile Glu Asp Glu Ser Gly Gly Val
                805                 810                 815
gca ttc ggt gga gac aat ggt atc cta gag tta gtt gat aaa caa att       2496
Ala Phe Gly Gly Asp Asn Gly Ile Leu Glu Leu Val Asp Lys Gln Ile
            820                 825                 830
aat ttc aac ata caa gaa tcg tga                                       2520
Asn Phe Asn Ile Gln Glu Ser
        835

<210> 6
<211> 839
<212> PRT
<213> Glycine max

<400> 6
Met Arg Gly Gly Ile Cys Ser Ile Gln Leu Gln Ala Leu Thr Pro Glu
1               5                   10                  15
Ala Ala Thr Val Val Lys Gln Ala Val Asn Leu Ala Thr Arg Arg Gly
            20                  25                  30
His Ala Gln Val Thr Pro Leu His Val Ala Ser Ala Met Leu Ala Thr
        35                  40                  45
Ser Thr Gly Leu Leu Arg Lys Ala Cys Leu Gln Cys His Ser His Pro
    50                  55                  60
Leu Gln Cys Lys Ala Leu Glu Leu Cys Phe Asn Val Ala Leu Asn Arg
65                  70                  75                  80
Leu Pro Ala Ser Thr Ser Ser Pro Leu Leu Ala Pro Gln Tyr Ser Thr
                85                  90                  95
Pro Ser Leu Ser Asn Ala Leu Val Ala Ala Phe Lys Arg Ala Gln Ala
            100                 105                 110
His Gln Arg Arg Gly Ser Ile Glu Asn Gln Gln Gln His Ile Leu Ala
        115                 120                 125
Leu Lys Ile Glu Val Glu Gln Leu Val Ile Ser Ile Leu Asp Asp Pro
    130                 135                 140
Ser Val Ser Arg Val Met Arg Glu Ala Gly Phe Ser Ser Thr Leu Val
145                 150                 155                 160
Lys Thr Arg Val Glu Gln Ala Val Ser Met Glu Val Cys Ser Gln Lys
                165                 170                 175
Ala Gln Ala Lys Glu Asn Ile Thr Lys Pro His His Gln Val Val Leu
            180                 185                 190
Gly Gly Arg Asn Asn Val Ser Pro Ser Gly Pro Phe Gly Gln Val Gly
        195                 200                 205
Gly Ser Phe Met Lys Pro Asn Leu Asp His Val Asn Asn Asp Asp Val
    210                 215                 220
Thr Ser Val Leu Ser Glu Leu Ala Lys Arg Arg Asn Thr Val Ile Val
225                 230                 235                 240
Gly Glu Ser Val Thr Asn Ala Glu Gly Val Val Arg Gly Val Ile Glu
                245                 250                 255
Arg Phe Glu Val Gly Asn Val Pro Gly Asp Leu Arg Asn Ile Ser Lys
            260                 265                 270
Glu Glu Val Glu Gln Lys Leu Met Glu Val Arg Asn Leu Val Lys Ser
        275                 280                 285
Tyr Val Gly Gly Gly Val Val Leu Tyr Leu Gly Asp Leu Lys Trp Leu
    290                 295                 300
Phe Glu Phe Trp Ala Asn Phe Arg Glu Gln Lys Thr Asn Tyr Cys Ser
305                 310                 315                 320
Val Glu His Met Val Met Glu Leu Lys Lys Leu Val Cys Gly Ser Gly
                325                 330                 335
Glu Ser Ser Arg Leu Trp Leu Met Gly Ile Ser Thr Phe Lys Thr Tyr
            340                 345                 350
Met Lys Cys Lys Ile Cys His Pro Ser Leu Glu Thr Ile Trp Glu Leu
        355                 360                 365
His Pro Phe Thr Ile Pro Val Gly Ile Leu Ser Leu Ser Leu Asn Leu
    370                 375                 380
Asp Ser Asp Phe Gln Ala Gln Glu Arg Asn Lys Val Phe Phe Lys Asp
```

```
385                 390                 395                 400
Val Ala Phe Glu Asp Arg Ala Gly Val Arg Asn His Leu Thr Cys Cys
                405                 410                 415
Arg Asp Cys Thr Ile Asn Phe Glu Lys Glu Ala Gln Ser Ile Thr Ser
            420                 425                 430
Thr Ile Ser Lys Lys Ala Cys Thr Thr Ser Ser Leu Pro Thr Trp Leu
        435                 440                 445
Gln Asn Cys Lys Glu Glu Arg Ser Asp Ile Met Glu Asp Gln Glu Asn
    450                 455                 460
Ala Arg Leu Lys Asp Leu Cys Lys Lys Trp Asn Ser Leu Cys Asn Ser
465                 470                 475                 480
Ile His Arg His Pro Ser Ile Asn Glu Lys Gln Val Phe Phe Val Ser
                485                 490                 495
Ser Ser Pro Ser Ser Pro Thr Ser Val Ser Ser His Glu Arg Lys Ser
            500                 505                 510
Asn Phe His His Ser His Leu Asn Trp Pro Ile Ile Ser Glu Ser Glu
        515                 520                 525
Lys Ser Pro Lys Glu Cys Glu Leu Tyr Thr Glu Thr Gly Asp Asp Gly
    530                 535                 540
Tyr Asp Ser Asn Phe Ile Met Phe Met Pro Asp Ser Asp Val Pro Lys
545                 550                 555                 560
Pro Asp Leu Leu Ser Asn Pro Asn Ser Ser Pro Asn Ser Ala Ser Ser
                565                 570                 575
Ser Glu Ala Val Asp Gly Leu Glu Ser Thr Gln Met Phe Lys Glu Pro
            580                 585                 590
Asn Ala Glu Asn His Lys Ile Leu Cys Asp Ala Leu Glu Lys Lys Val
        595                 600                 605
Pro Gln His Lys Glu Val Ile Pro Glu Ile Ala Ser Thr Val Leu His
    610                 615                 620
Cys Arg Ser Gly Met Arg Lys Arg Asp Gln Asn His Ser Met Lys Arg
625                 630                 635                 640
Glu Asp Asn Gln Glu Thr Trp Met Phe Phe Leu Gly Val Asn Ser Gln
                645                 650                 655
Ala Lys Glu Ser Ile Ser Arg Glu Leu Ala Lys Val Val Phe Gly Ser
            660                 665                 670
Tyr Ser Asn Phe Val Thr Ile Gly Met Ser Ser Phe Ser Ser Pro Glu
        675                 680                 685
Asp Asp Asp Asp Ser Thr Asp Glu Lys Ser Lys Arg Lys Arg Pro Arg
    690                 695                 700
Glu Glu Leu Lys Ser Ser Tyr Ala Gln Arg Phe Gly Glu Ala Val Asn
705                 710                 715                 720
Glu Asn Pro His Arg Val Phe Phe Leu Glu Asp Leu Asp Gln Val Asp
                725                 730                 735
Tyr Phe Ser Gln Lys Gly Val Glu Gln Ala Ile Gln Ser Gly Ser Ile
            740                 745                 750
Thr Leu Pro Gly Gly Glu Ser Val Pro Leu Met Asp Ala Ile Val Ile
        755                 760                 765
Phe Ser Cys Glu Ser Phe Phe Ser Ser Pro Lys Leu Arg Lys Ser Pro
    770                 775                 780
Cys Ala Glu Asn Lys Gly Lys Glu Thr Val Glu Asp Glu Ser Ser Ser
785                 790                 795                 800
Leu Ser Leu Asp Leu Asn Ile Ala Ile Glu Asp Glu Ser Gly Gly Val
                805                 810                 815
Ala Phe Gly Gly Asp Asn Gly Ile Leu Glu Leu Val Asp Lys Gln Ile
            820                 825                 830
Asn Phe Asn Ile Gln Glu Ser
        835
```

<210> 7
<211> 2598
<212> DNA
<213> Glycine max

<220>
<221> CDS

<222> (1)..(2598)

<400> 7

```
atg aga gga ggt att tgt agc ata cag ctt cag gct ctt aca cct gaa        48
Met Arg Gly Gly Ile Cys Ser Ile Gln Leu Gln Ala Leu Thr Pro Glu
1               5                   10                  15
gct aca act gtt gtc aag caa gct gtg aat ctt gca aca aga aga ggc        96
Ala Thr Thr Val Val Lys Gln Ala Val Asn Leu Ala Thr Arg Arg Gly
            20                  25                  30
cat gcg caa gtg aca cct ctt cat gtt gca agt gcc atg ctt gca act        144
His Ala Gln Val Thr Pro Leu His Val Ala Ser Ala Met Leu Ala Thr
        35                  40                  45
tcc aca ggc ctt ctt cgt aag gct tgt ctt cag tgt cac tct cac cct        192
Ser Thr Gly Leu Leu Arg Lys Ala Cys Leu Gln Cys His Ser His Pro
    50                  55                  60
ctt cag tgc aag gct ctt gag ctt tgt ttc aac gtt gca ctc aac cgt        240
Leu Gln Cys Lys Ala Leu Glu Leu Cys Phe Asn Val Ala Leu Asn Arg
65                  70                  75                  80
tta cca gct tcc act tca agc cct ctt ctg gct cct caa tat tcc acc        288
Leu Pro Ala Ser Thr Ser Ser Pro Leu Leu Ala Pro Gln Tyr Ser Thr
                85                  90                  95
cct tca ctc tcc aat gcc ttg gtt gca gcc ttc aaa cgt gct cag gct        336
Pro Ser Leu Ser Asn Ala Leu Val Ala Ala Phe Lys Arg Ala Gln Ala
            100                 105                 110
cac caa cgc cgt ggt tct att gag aac cag cag cag cat att ctt gcc        384
His Gln Arg Arg Gly Ser Ile Glu Asn Gln Gln Gln His Ile Leu Ala
        115                 120                 125
ttg aag atc gag gtg gag cag ctc gtg att tca atc ctt gat gac cct        432
Leu Lys Ile Glu Val Glu Gln Leu Val Ile Ser Ile Leu Asp Asp Pro
    130                 135                 140
agt gtt agt agg gtc atg aga gaa gct ggt ttc tct agc acc ctt gtg        480
Ser Val Ser Arg Val Met Arg Glu Ala Gly Phe Ser Ser Thr Leu Val
145                 150                 155                 160
aaa aca agg gtt gaa caa gct gtt tca atg gaa gtt tgt tca cag aag        528
Lys Thr Arg Val Glu Gln Ala Val Ser Met Glu Val Cys Ser Gln Lys
                165                 170                 175
gct tct tct gat agg agc cat gcc aaa gaa aat atc act aag ccc cat        576
Ala Ser Ser Asp Arg Ser His Ala Lys Glu Asn Ile Thr Lys Pro His
            180                 185                 190
cat gtt gtt ctt ggt ggt agt aat aat gtg tct cca tca tct gga cct        624
His Val Val Leu Gly Gly Ser Asn Asn Val Ser Pro Ser Ser Gly Pro
        195                 200                 205
ttt ggt caa gtt gct ggt ggc tcc ttc atg aag cct aat tta gac cat        672
Phe Gly Gln Val Ala Gly Gly Ser Phe Met Lys Pro Asn Leu Asp His
    210                 215                 220
gtt aac aat gat gat gta act agt gtg ctg agt gag ttg gtg agg aga        720
Val Asn Asn Asp Asp Val Thr Ser Val Leu Ser Glu Leu Val Arg Arg
225                 230                 235                 240
aaa aac aca gtt att gta ggg gag ggt gtg gcc aat gct gag gga gta        768
Lys Asn Thr Val Ile Val Gly Glu Gly Val Ala Asn Ala Glu Gly Val
                245                 250                 255
gct agg gaa gtg atg gag agg ttt gag gta ggg aat gtt cct ggg gac        816
Ala Arg Glu Val Met Glu Arg Phe Glu Val Gly Asn Val Pro Gly Asp
            260                 265                 270
tta agg tat gtg caa ttt gtg agc ctt cct ctc atg tgc ttc aga aac        864
Leu Arg Tyr Val Gln Phe Val Ser Leu Pro Leu Met Cys Phe Arg Asn
        275                 280                 285
att agc aag gag gaa gtt gaa cag aag ctt atg gag att aga aac ctt        912
Ile Ser Lys Glu Glu Val Glu Gln Lys Leu Met Glu Ile Arg Asn Leu
    290                 295                 300
gtg aag agc tat gtg gga aga ggg gtt gtt ctt tac ttg ggt gat cta        960
Val Lys Ser Tyr Val Gly Arg Gly Val Val Leu Tyr Leu Gly Asp Leu
305                 310                 315                 320
aaa tgg ttg ttt gag ttc tgg gct aat ttt tgt gag caa aaa aga aac        1008
Lys Trp Leu Phe Glu Phe Trp Ala Asn Phe Cys Glu Gln Lys Arg Asn
```

```
                325                     330                     335
tac tac tgt tct ata gag caa atg gtc atg gag ctt aaa aaa ttg gta        1056
Tyr Tyr Cys Ser Ile Glu Gln Met Val Met Glu Leu Lys Lys Leu Val
            340                     345                     350
tgt ggt agt ggg gag tct agt aga ctg tgg ctt atg ggg att gca act        1104
Cys Gly Ser Gly Glu Ser Ser Arg Leu Trp Leu Met Gly Ile Ala Thr
        355                     360                     365
ttt aag gca tac atg aag tgt aaa ata tgt cac cca tcc cta gaa gct        1152
Phe Lys Ala Tyr Met Lys Cys Lys Ile Cys His Pro Ser Leu Glu Ala
    370                     375                     380
att tgg gag ctt cac ccc ttc aca att cct gtt ggc agc ctc agc cta        1200
Ile Trp Glu Leu His Pro Phe Thr Ile Pro Val Gly Ser Leu Ser Leu
385                     390                     395                     400
agt tta aat ttt cac agt gat ttt caa gct caa gag aga agc aag gtg        1248
Ser Leu Asn Phe His Ser Asp Phe Gln Ala Gln Glu Arg Ser Lys Val
                405                     410                     415
ttt ttc aag gat gtg gct ttt gaa gat aga aca gga gtc aga aac cat        1296
Phe Phe Lys Asp Val Ala Phe Glu Asp Arg Thr Gly Val Arg Asn His
            420                     425                     430
cta act tgc tgc agg gat tgc ttg ata aat ttt gaa aaa gaa gct cag        1344
Leu Thr Cys Cys Arg Asp Cys Leu Ile Asn Phe Glu Lys Glu Ala Gln
        435                     440                     445
agc ata act aac tgt ata agt aag aaa gtt tgc act gct agt agc ttg        1392
Ser Ile Thr Asn Cys Ile Ser Lys Lys Val Cys Thr Ala Ser Ser Leu
    450                     455                     460
cca acg tgg ctt caa aat tgt aag gaa gag aga agt gac ata atg gaa        1440
Pro Thr Trp Leu Gln Asn Cys Lys Glu Glu Arg Ser Asp Ile Met Glu
465                     470                     475                     480
gat cag gaa agt tca agg ctt gag tat ctg tgc aag aaa tgg aat tcc        1488
Asp Gln Glu Ser Ser Arg Leu Glu Tyr Leu Cys Lys Lys Trp Asn Ser
                485                     490                     495
tta tgc aat tca ata cac aga cga cac cct tcc att att gag aaa cca        1536
Leu Cys Asn Ser Ile His Arg Arg His Pro Ser Ile Ile Glu Lys Pro
            500                     505                     510
gct gtt ttc ttt gtt tca tca tcc cct tca tct ccc act tct gtt tcc        1584
Ala Val Phe Phe Val Ser Ser Ser Pro Ser Ser Pro Thr Ser Val Ser
        515                     520                     525
tca aat gaa aga aag tcc aac ttt cat cac agc cac cta aat tgg cca        1632
Ser Asn Glu Arg Lys Ser Asn Phe His His Ser His Leu Asn Trp Pro
    530                     535                     540
atc att tct gaa tca gaa aag tca cca aaa gaa tgt gag tta tac act        1680
Ile Ile Ser Glu Ser Glu Lys Ser Pro Lys Glu Cys Glu Leu Tyr Thr
545                     550                     555                     560
gaa act ggt gat gat gat gat gat ggc tat gat agc aac ttt ata atg        1728
Glu Thr Gly Asp Asp Asp Asp Asp Gly Tyr Asp Ser Asn Phe Ile Met
                565                     570                     575
ttc atg cca gat aga gat gtc cct aaa cca gat ctt ctg tca aat cca        1776
Phe Met Pro Asp Arg Asp Val Pro Lys Pro Asp Leu Leu Ser Asn Pro
            580                     585                     590
aac tct agc ccc aac tcg gct tct tca agt gaa gca gtg gat ggt ttg        1824
Asn Ser Ser Pro Asn Ser Ala Ser Ser Ser Glu Ala Val Asp Gly Leu
        595                     600                     605
gaa agc act caa atg ttc aag gaa cct aat gct gag aat cat aag att        1872
Glu Ser Thr Gln Met Phe Lys Glu Pro Asn Ala Glu Asn His Lys Ile
    610                     615                     620
ctg tgt gat gct ttg gag aaa aag att cca cag cat aaa gat gtt att        1920
Leu Cys Asp Ala Leu Glu Lys Lys Ile Pro Gln His Lys Asp Val Ile
625                     630                     635                     640
gtt cca gag att gca agt act gtc ctt cat tgt aga tca gga atg aga        1968
Val Pro Glu Ile Ala Ser Thr Val Leu His Cys Arg Ser Gly Met Arg
                645                     650                     655
aaa aga gga tta aac cac ttg atg aac aga gaa gaa aat caa gaa act        2016
Lys Arg Gly Leu Asn His Leu Met Asn Arg Glu Glu Asn Gln Glu Thr
            660                     665                     670
tgg atg ttc ttt ctt ggt gtg aat tcc caa gcc aaa gag agc atc tca        2064
```

```
Trp Met Phe Phe Leu Gly Val Asn Ser Gln Ala Lys Glu Ser Ile Ser
    675                 680                 685
agg gag cta gct aaa gtt gtt ttt ggc tct tat agc aac ttt gtc tca      2112
Arg Glu Leu Ala Lys Val Val Phe Gly Ser Tyr Ser Asn Phe Val Ser
    690                 695                 700
att ggc atg agc aat ttc tct tcc cca gaa gat gat cat gat tcc act      2160
Ile Gly Met Ser Asn Phe Ser Ser Pro Glu Asp Asp His Asp Ser Thr
705                 710                 715                 720
gat gaa aaa tcc aaa agg aag agg cca aga gaa gag ttg aaa agc agt      2208
Asp Glu Lys Ser Lys Arg Lys Arg Pro Arg Glu Glu Leu Lys Ser Ser
                725                 730                 735
tat gtt caa aga ttc ggg gaa gca gtg aat gag aac cct cat agg gtg      2256
Tyr Val Gln Arg Phe Gly Glu Ala Val Asn Glu Asn Pro His Arg Val
            740                 745                 750
ttc ttc ttg gaa gac ttg gat caa gtt gat tac ttc tct caa aag ggt      2304
Phe Phe Leu Glu Asp Leu Asp Gln Val Asp Tyr Phe Ser Gln Lys Gly
        755                 760                 765
gta aag caa gca att caa agt gga agc ata aca ctc cct agt ggt gaa      2352
Val Lys Gln Ala Ile Gln Ser Gly Ser Ile Thr Leu Pro Ser Gly Glu
    770                 775                 780
tct gtt cct ctc aag gat gcc att gtc att ttc agt tgc gaa agc ttc      2400
Ser Val Pro Leu Lys Asp Ala Ile Val Ile Phe Ser Cys Glu Ser Phe
785                 790                 795                 800
tct tca cca aag ttg aga aaa tca cca tgt gct gaa aac aaa ggg aaa      2448
Ser Ser Pro Lys Leu Arg Lys Ser Pro Cys Ala Glu Asn Lys Gly Lys
                805                 810                 815
gag atc act gtg gat gat gaa agt tct agc ctc tct ttg gat ctg aac      2496
Glu Ile Thr Val Asp Asp Glu Ser Ser Ser Leu Ser Leu Asp Leu Asn
            820                 825                 830
cta gcc att gaa gat gaa agt gga ggt gtg gca tta ggt gga gac aat      2544
Leu Ala Ile Glu Asp Glu Ser Gly Gly Val Ala Leu Gly Gly Asp Asn
        835                 840                 845
ggc atc cta gag tta gtt gac aaa caa att aat ttc aac ata caa gaa      2592
Gly Ile Leu Glu Leu Val Asp Lys Gln Ile Asn Phe Asn Ile Gln Glu
    850                 855                 860
ttg tga                                                              2598
Leu
865
```

<210> 8
<211> 865
<212> PRT
<213> Glycine max

<400> 8

```
Met Arg Gly Gly Ile Cys Ser Ile Gln Leu Gln Ala Leu Thr Pro Glu
1               5                   10                  15
Ala Thr Thr Val Val Lys Gln Ala Val Asn Leu Ala Thr Arg Arg Gly
            20                  25                  30
His Ala Gln Val Thr Pro Leu His Val Ala Ser Ala Met Leu Ala Thr
        35                  40                  45
Ser Thr Gly Leu Leu Arg Lys Ala Cys Leu Gln Cys His Ser His Pro
    50                  55                  60
Leu Gln Cys Lys Ala Leu Glu Leu Cys Phe Asn Val Ala Leu Asn Arg
65                  70                  75                  80
Leu Pro Ala Ser Thr Ser Ser Pro Leu Leu Ala Pro Gln Tyr Ser Thr
                85                  90                  95
Pro Ser Leu Ser Asn Ala Leu Val Ala Ala Phe Lys Arg Ala Gln Ala
            100                 105                 110
His Gln Arg Arg Gly Ser Ile Glu Asn Gln Gln Gln His Ile Leu Ala
        115                 120                 125
Leu Lys Ile Glu Val Glu Gln Leu Val Ile Ser Ile Leu Asp Asp Pro
    130                 135                 140
Ser Val Ser Arg Val Met Arg Glu Ala Gly Phe Ser Ser Thr Leu Val
145                 150                 155                 160
```

```
Lys Thr Arg Val Glu Gln Ala Val Ser Met Glu Val Cys Ser Gln Lys
                165                 170                 175
Ala Ser Ser Asp Arg Ser His Ala Lys Glu Asn Ile Thr Lys Pro His
            180                 185                 190
His Val Val Leu Gly Gly Ser Asn Asn Val Ser Pro Ser Ser Gly Pro
        195                 200                 205
Phe Gly Gln Val Ala Gly Gly Ser Phe Met Lys Pro Asn Leu Asp His
    210                 215                 220
Val Asn Asn Asp Asp Val Thr Ser Val Leu Ser Glu Leu Val Arg Arg
225                 230                 235                 240
Lys Asn Thr Val Ile Val Gly Glu Gly Val Ala Asn Ala Glu Gly Val
                245                 250                 255
Ala Arg Glu Val Met Glu Arg Phe Glu Val Gly Asn Val Pro Gly Asp
            260                 265                 270
Leu Arg Tyr Val Gln Phe Val Ser Leu Pro Leu Met Cys Phe Arg Asn
        275                 280                 285
Ile Ser Lys Glu Glu Val Glu Gln Lys Leu Met Glu Ile Arg Asn Leu
    290                 295                 300
Val Lys Ser Tyr Val Gly Arg Gly Val Val Leu Tyr Leu Gly Asp Leu
305                 310                 315                 320
Lys Trp Leu Phe Glu Phe Trp Ala Asn Phe Cys Glu Gln Lys Arg Asn
                325                 330                 335
Tyr Tyr Cys Ser Ile Glu Gln Met Val Met Glu Leu Lys Lys Leu Val
            340                 345                 350
Cys Gly Ser Gly Glu Ser Ser Arg Leu Trp Leu Met Gly Ile Ala Thr
        355                 360                 365
Phe Lys Ala Tyr Met Lys Cys Lys Ile Cys His Pro Ser Leu Glu Ala
    370                 375                 380
Ile Trp Glu Leu His Pro Phe Thr Ile Pro Val Gly Ser Leu Ser Leu
385                 390                 395                 400
Ser Leu Asn Phe His Ser Asp Phe Gln Ala Gln Glu Arg Ser Lys Val
                405                 410                 415
Phe Phe Lys Asp Val Ala Phe Glu Asp Arg Thr Gly Val Arg Asn His
            420                 425                 430
Leu Thr Cys Cys Arg Asp Cys Leu Ile Asn Phe Glu Lys Glu Ala Gln
        435                 440                 445
Ser Ile Thr Asn Cys Ile Ser Lys Lys Val Cys Thr Ala Ser Ser Leu
    450                 455                 460
Pro Thr Trp Leu Gln Asn Cys Lys Glu Glu Arg Ser Asp Ile Met Glu
465                 470                 475                 480
Asp Gln Glu Ser Ser Arg Leu Glu Tyr Leu Cys Lys Lys Trp Asn Ser
                485                 490                 495
Leu Cys Asn Ser Ile His Arg Arg His Pro Ser Ile Ile Glu Lys Pro
            500                 505                 510
Ala Val Phe Phe Val Ser Ser Ser Pro Ser Ser Pro Thr Ser Val Ser
        515                 520                 525
Ser Asn Glu Arg Lys Ser Asn Phe His His Ser His Leu Asn Trp Pro
    530                 535                 540
Ile Ile Ser Glu Ser Glu Lys Ser Pro Lys Glu Cys Glu Leu Tyr Thr
545                 550                 555                 560
Glu Thr Gly Asp Asp Asp Asp Asp Gly Tyr Asp Ser Asn Phe Ile Met
                565                 570                 575
Phe Met Pro Asp Arg Asp Val Pro Lys Pro Asp Leu Leu Ser Asn Pro
            580                 585                 590
Asn Ser Ser Pro Asn Ser Ala Ser Ser Ser Glu Ala Val Asp Gly Leu
        595                 600                 605
Glu Ser Thr Gln Met Phe Lys Glu Pro Asn Ala Glu Asn His Lys Ile
    610                 615                 620
Leu Cys Asp Ala Leu Glu Lys Lys Ile Pro Gln His Lys Asp Val Ile
625                 630                 635                 640
Val Pro Glu Ile Ala Ser Thr Val Leu His Cys Arg Ser Gly Met Arg
                645                 650                 655
Lys Arg Gly Leu Asn His Leu Met Asn Arg Glu Glu Asn Gln Glu Thr
            660                 665                 670
Trp Met Phe Phe Leu Gly Val Asn Ser Gln Ala Lys Glu Ser Ile Ser
```

```
        675                 680                 685
Arg Glu Leu Ala Lys Val Val Phe Gly Ser Tyr Ser Asn Phe Val Ser
    690                 695                 700
Ile Gly Met Ser Asn Phe Ser Ser Pro Glu Asp Asp His Asp Ser Thr
705                 710                 715                 720
Asp Glu Lys Ser Lys Arg Lys Arg Pro Arg Glu Glu Leu Lys Ser Ser
                725                 730                 735
Tyr Val Gln Arg Phe Gly Glu Ala Val Asn Glu Asn Pro His Arg Val
            740                 745                 750
Phe Phe Leu Glu Asp Leu Asp Gln Val Asp Tyr Phe Ser Gln Lys Gly
        755                 760                 765
Val Lys Gln Ala Ile Gln Ser Gly Ser Ile Thr Leu Pro Ser Gly Glu
    770                 775                 780
Ser Val Pro Leu Lys Asp Ala Ile Val Ile Phe Ser Cys Glu Ser Phe
785                 790                 795                 800
Ser Ser Pro Lys Leu Arg Lys Ser Pro Cys Ala Glu Asn Lys Gly Lys
                805                 810                 815
Glu Ile Thr Val Asp Asp Glu Ser Ser Leu Ser Leu Asp Leu Asn
            820                 825                 830
Leu Ala Ile Glu Asp Glu Ser Gly Gly Val Ala Leu Gly Gly Asp Asn
        835                 840                 845
Gly Ile Leu Glu Leu Val Asp Lys Gln Ile Asn Phe Asn Ile Gln Glu
    850                 855                 860
Leu
865

<210> 9
<211> 2787
<212> DNA
<213> Zea mays

<220>
<221> CDS
<222> (1)..(2787)

<400> 9
atg gca agt tgg caa cca cct gca gct cta cat cta cgc aca cat ctc      48
Met Ala Ser Trp Gln Pro Pro Ala Ala Leu His Leu Arg Thr His Leu
1               5                   10                  15
cgt gat ctc ttc agc ctc gcg act gcc acc aaa tcg gtc ttg gca ggt      96
Arg Asp Leu Phe Ser Leu Ala Thr Ala Thr Lys Ser Val Leu Ala Gly
            20                  25                  30
tgc tgc gcc aaa tcg gcc ggt ctt ggc agg ttg ctg cgc caa atc ggc      144
Cys Cys Ala Lys Ser Ala Gly Leu Gly Arg Leu Leu Arg Gln Ile Gly
        35                  40                  45
cgg tct tgg cag gtt gcg cta gct acc cat agg ttg gct gga gag atg      192
Arg Ser Trp Gln Val Ala Leu Ala Thr His Arg Leu Ala Gly Glu Met
    50                  55                  60
agg gcc ggg ggg tgc acg gtg cag cag tcg ctg aca gcg gag gcc gcg      240
Arg Ala Gly Gly Cys Thr Val Gln Gln Ser Leu Thr Ala Glu Ala Ala
65                  70                  75                  80
gcg gtg gtg aag cag gcg gtg agc ctt gcg cgg cgg cgc ggg aac gcg      288
Ala Val Val Lys Gln Ala Val Ser Leu Ala Arg Arg Arg Gly Asn Ala
                85                  90                  95
cag gtc acg ccg ctg cac gtg gcg agc gcg atg ctg gcg gcc gca ccc      336
Gln Val Thr Pro Leu His Val Ala Ser Ala Met Leu Ala Ala Ala Pro
            100                 105                 110
gcg ggg cag ctg ctg cgc gcg gcg tgc ctc cgc tca cac tcc cac ccg      384
Ala Gly Gln Leu Leu Arg Ala Ala Cys Leu Arg Ser His Ser His Pro
        115                 120                 125
ctg cag tgc aag gcg ctg gag ctg tgc ttc aac gtg gcg ctc aac cgc      432
Leu Gln Cys Lys Ala Leu Glu Leu Cys Phe Asn Val Ala Leu Asn Arg
    130                 135                 140
ctc ccc gcg tcg gca tcg tcg ccg ctg ctc ggc ggc aac cac cac tgc      480
Leu Pro Ala Ser Ala Ser Ser Pro Leu Leu Gly Gly Asn His His Cys
```

```
145                 150                 155                 160
tac cac ccg ccg tcc ctg tcc aac gcg ctc gtc gcg gcg ttc aag cgc       528
Tyr His Pro Pro Ser Leu Ser Asn Ala Leu Val Ala Ala Phe Lys Arg
                165                 170                 175
gcg cag gcg cac cag cgg cgc ggc ggc tcc gtc gag agc cag cat cag       576
Ala Gln Ala His Gln Arg Arg Gly Gly Ser Val Glu Ser Gln His Gln
            180                 185                 190
cag ccg gtg gtc gcc gcc gtc aag atc gag ctg gac cag ctc gtc gtc       624
Gln Pro Val Val Ala Ala Val Lys Ile Glu Leu Asp Gln Leu Val Val
        195                 200                 205
tcc atc ctc gac gac ccc agc gtc agc cgc gtg atg cgc gac gcc ggc       672
Ser Ile Leu Asp Asp Pro Ser Val Ser Arg Val Met Arg Asp Ala Gly
    210                 215                 220
ttc tcc agc acc cag gtc aag gcc aac gtg gag cag gcc gtg tgc agc       720
Phe Ser Ser Thr Gln Val Lys Ala Asn Val Glu Gln Ala Val Cys Ser
225                 230                 235                 240
agc agc acc atg gct acg gcc gca gcc gca ccc agc aaa aac cct aac       768
Ser Ser Thr Met Ala Thr Ala Ala Ala Ala Pro Ser Lys Asn Pro Asn
                245                 250                 255
cct agc agc tcg gcc acc acc aca agc cca ccg cct aaa gaa gct aaa       816
Pro Ser Ser Ser Ala Thr Thr Thr Ser Pro Pro Pro Lys Glu Ala Lys
            260                 265                 270
gcc aag ccg ccg ctc gcg ctc cac cac caa gcg cgc gac gag gac gtc       864
Ala Lys Pro Pro Leu Ala Leu His His Gln Ala Arg Asp Glu Asp Val
        275                 280                 285
gcc gcc gtc ctg gac tgc ctg gcc tcg cgg agc aag agg agg gtc gtc       912
Ala Ala Val Leu Asp Cys Leu Ala Ser Arg Ser Lys Arg Arg Val Val
    290                 295                 300
gtc atc gcg gag agc gcg gcc gcg gcc gag gcc atg gcg cac gcg gcc       960
Val Ile Ala Glu Ser Ala Ala Ala Ala Glu Ala Met Ala His Ala Ala
305                 310                 315                 320
gtg gac aag atc aag aga ggc gag gtg aag cac gag cac gac gcg ctg      1008
Val Asp Lys Ile Lys Arg Gly Glu Val Lys His Glu His Asp Ala Leu
                325                 330                 335
cga ggc gcg cag gtc gtc agc ctc cgc gtg tcg tcg ttc cgt gag atg      1056
Arg Gly Ala Gln Val Val Ser Leu Arg Val Ser Ser Phe Arg Glu Met
            340                 345                 350
ccg agg gag gag gcc gag cgg cgg ctc ggc gag ctg cgg tgc ctt gtc      1104
Pro Arg Glu Glu Ala Glu Arg Arg Leu Gly Glu Leu Arg Cys Leu Val
        355                 360                 365
aag cag ggc agg agg cag cgg gtc gtg ctc gtc gtg gag gac ctc aag      1152
Lys Gln Gly Arg Arg Gln Arg Val Val Leu Val Val Glu Asp Leu Lys
    370                 375                 380
tgg gcg gcg gag ttc tgg gcc ggc cac gac ggc cag agc ggg agg aga      1200
Trp Ala Ala Glu Phe Trp Ala Gly His Asp Gly Gln Ser Gly Arg Arg
385                 390                 395                 400
ggc ggg tac tac tac tgc gcc gtc gag cac gtc gtc aac gag ctg cgc      1248
Gly Gly Tyr Tyr Tyr Cys Ala Val Glu His Val Val Asn Glu Leu Arg
                405                 410                 415
gcc ctg gcg agc ggc ggc agc ctg tgc tgg ctc ctc ggg ttc ggg acg      1296
Ala Leu Ala Ser Gly Gly Ser Leu Cys Trp Leu Leu Gly Phe Gly Thr
            420                 425                 430
tac cag gcc tac acg aag tgt cgg gtg ggg cag ccg tcg ctg gag agc      1344
Tyr Gln Ala Tyr Thr Lys Cys Arg Val Gly Gln Pro Ser Leu Glu Ser
        435                 440                 445
ctg tgg ggg ctc cag acg ctc acc gtg ccc gcc ggc agc ctc gcg ctc      1392
Leu Trp Gly Leu Gln Thr Leu Thr Val Pro Ala Gly Ser Leu Ala Leu
    450                 455                 460
agc ctc acc tgc gcc ttc gac gac agt gcc cta ggc aca gtc aat cag      1440
Ser Leu Thr Cys Ala Phe Asp Asp Ser Ala Leu Gly Thr Val Asn Gln
465                 470                 475                 480
tcc ctg aaa gcg ggc tct gac gcg gat ggg aac ggg ccg gcg tct tgc      1488
Ser Leu Lys Ala Gly Ser Asp Ala Asp Gly Asn Gly Pro Ala Ser Cys
                485                 490                 495
tgg ccg ctt ttg ggc ggc agc cag ctg agc ctg agc tcc aga tgc tgc      1536
```

```
Trp Pro Leu Leu Gly Gly Ser Gln Leu Ser Leu Ser Ser Arg Cys Cys
            500                 505                 510
ggc gac ggc gat tgc tct gcc gcg agg atc gac acg aaa gcg gcg ttg      1584
Gly Asp Gly Asp Cys Ser Ala Ala Arg Ile Asp Thr Lys Ala Ala Leu
        515                 520                 525
cct cgg ccc tct gtc tca tcg tcc aac ctc cct tcc tgg ctt cag cat      1632
Pro Arg Pro Ser Val Ser Ser Ser Asn Leu Pro Ser Trp Leu Gln His
    530                 535                 540
tgc cgt gat cat cag gag cct act acg agc cat ctt acg gat ctt agc      1680
Cys Arg Asp His Gln Glu Pro Thr Thr Ser His Leu Thr Asp Leu Ser
545                 550                 555                 560
aag agc aag aca tgg agc tcc atc tgc agc agg ccg tcg cag cgg atg      1728
Lys Ser Lys Thr Trp Ser Ser Ile Cys Ser Arg Pro Ser Gln Arg Met
                565                 570                 575
acg ctg cat ttt tcc gca ccg gtg tct ccg gcc tcc tcc atc tcc tcc      1776
Thr Leu His Phe Ser Ala Pro Val Ser Pro Ala Ser Ser Ile Ser Ser
            580                 585                 590
tac gag cac ggc ggc gac cag tcg tcg cag acg ccg cct cag cac tcg      1824
Tyr Glu His Gly Gly Asp Gln Ser Ser Gln Thr Pro Pro Gln His Ser
        595                 600                 605
tgg ctc ctt gct ggt ctc gac gcc gcg cac cac cgg tgg gaa ccc aag      1872
Trp Leu Leu Ala Gly Leu Asp Ala Ala His His Arg Trp Glu Pro Lys
    610                 615                 620
cgc gag acc agc ggg aat aag gcc gcc agc agg tcc cac gac tcc ggc      1920
Arg Glu Thr Ser Gly Asn Lys Ala Ala Ser Arg Ser His Asp Ser Gly
625                 630                 635                 640
gcc tcg aac ggc ggc tcc gtg gag gtg gag tgc cgc gcc agg ttc aag      1968
Ala Ser Asn Gly Gly Ser Val Glu Val Glu Cys Arg Ala Arg Phe Lys
                645                 650                 655
gag ctc agc gcc gag aac ctc aag ctg ctc tgc ggc gcg ctg gag aag      2016
Glu Leu Ser Ala Glu Asn Leu Lys Leu Leu Cys Gly Ala Leu Glu Lys
            660                 665                 670
gaa gtg ccg tgg cag aag gag atc atc ccg gag gtc gcc agc gcc gtc      2064
Glu Val Pro Trp Gln Lys Glu Ile Ile Pro Glu Val Ala Ser Ala Val
        675                 680                 685
ctg cag tgc cgg tca ggg atc gcc aag agg cgc gac aag tcg agg tcg      2112
Leu Gln Cys Arg Ser Gly Ile Ala Lys Arg Arg Asp Lys Ser Arg Ser
    690                 695                 700
gcg gac gca aag gag gag acg tgg atg ctc ttc ctc gga ggc gac gcc      2160
Ala Asp Ala Lys Glu Glu Thr Trp Met Leu Phe Leu Gly Gly Asp Ala
705                 710                 715                 720
gac ggc aag gag agg gtg gcg agg gag ctc gcc agc ctc gtc ttc ggg      2208
Asp Gly Lys Glu Arg Val Ala Arg Glu Leu Ala Ser Leu Val Phe Gly
                725                 730                 735
tca cgc gac agc ttc tta gcc atc agg cct ggt gcc tcg tcg tcg tcg      2256
Ser Arg Asp Ser Phe Leu Ala Ile Arg Pro Gly Ala Ser Ser Ser Ser
            740                 745                 750
ccg ccg cgg tca ggt tcc tcc gag ggt cac cgg agt aac aag cgg cca      2304
Pro Pro Arg Ser Gly Ser Ser Glu Gly His Arg Ser Asn Lys Arg Pro
        755                 760                 765
cgg atg tcg ccg ccg ccg cct ggg gag gag tcg gcg ccg gcc tgc ctg      2352
Arg Met Ser Pro Pro Pro Pro Gly Glu Glu Ser Ala Pro Ala Cys Leu
    770                 775                 780
gaa cgg ttg cac gac gct gta tcc gag aac ccg cac cgg gtc ata ttc      2400
Glu Arg Leu His Asp Ala Val Ser Glu Asn Pro His Arg Val Ile Phe
785                 790                 795                 800
atg gag ggc gtc gag cag gct ggc cgg gac tgc cag ctc ggc atc aag      2448
Met Glu Gly Val Glu Gln Ala Gly Arg Asp Cys Gln Leu Gly Ile Lys
                805                 810                 815
gag gcg atc gag agc ggg gtc gtg cgg aac cga gct ggc gtg gag gtc      2496
Glu Ala Ile Glu Ser Gly Val Val Arg Asn Arg Ala Gly Val Glu Val
            820                 825                 830
ggc gtg ggc gat gcc atc gtc gtc ctg agc tgc gag ggc ttt ggc agc      2544
Gly Val Gly Asp Ala Ile Val Val Leu Ser Cys Glu Gly Phe Gly Ser
        835                 840                 845
```

```
acc ggc agg tcc aga gct tgc tcg cca ccg agc aag aag gtg aag gta       2592
Thr Gly Arg Ser Arg Ala Cys Ser Pro Pro Ser Lys Lys Val Lys Val
    850                 855                 860
gag atg gag gag gct aag gag gag cgc aca ggt gac att gaa cac aac       2640
Glu Met Glu Glu Ala Lys Glu Glu Arg Thr Gly Asp Ile Glu His Asn
865                 870                 875                 880
gga gat ggt ggt tcc tcg tcg tct ccg tcg tgc atc gat ttg aac gtg       2688
Gly Asp Gly Gly Ser Ser Ser Ser Pro Ser Cys Ile Asp Leu Asn Val
                885                 890                 895
gac atg gag agt gac cag gcg gac gag gga agt ctc gat gat gat gtc       2736
Asp Met Glu Ser Asp Gln Ala Asp Glu Gly Ser Leu Asp Asp Asp Val
            900                 905                 910
tgt ctg ctc aca gcc gtc gac agg acc ctg ttc ttc aga aga cag ggg       2784
Cys Leu Leu Thr Ala Val Asp Arg Thr Leu Phe Phe Arg Arg Gln Gly
        915                 920                 925
tga                                                                   2787
```

<210> 10
<211> 928
<212> PRT
<213> Zea mays

<400> 10

```
Met Ala Ser Trp Gln Pro Pro Ala Ala Leu His Leu Arg Thr His Leu
1               5                   10                  15
Arg Asp Leu Phe Ser Leu Ala Thr Ala Thr Lys Ser Val Leu Ala Gly
            20                  25                  30
Cys Cys Ala Lys Ser Ala Gly Leu Gly Arg Leu Leu Arg Gln Ile Gly
        35                  40                  45
Arg Ser Trp Gln Val Ala Leu Ala Thr His Arg Leu Ala Gly Glu Met
    50                  55                  60
Arg Ala Gly Gly Cys Thr Val Gln Gln Ser Leu Thr Ala Glu Ala Ala
65                  70                  75                  80
Ala Val Val Lys Gln Ala Val Ser Leu Ala Arg Arg Arg Gly Asn Ala
                85                  90                  95
Gln Val Thr Pro Leu His Val Ala Ser Ala Met Leu Ala Ala Ala Pro
            100                 105                 110
Ala Gly Gln Leu Leu Arg Ala Ala Cys Leu Arg Ser His Ser His Pro
        115                 120                 125
Leu Gln Cys Lys Ala Leu Glu Leu Cys Phe Asn Val Ala Leu Asn Arg
    130                 135                 140
Leu Pro Ala Ser Ala Ser Ser Pro Leu Leu Gly Gly Asn His His Cys
145                 150                 155                 160
Tyr His Pro Pro Ser Leu Ser Asn Ala Leu Val Ala Ala Phe Lys Arg
                165                 170                 175
Ala Gln Ala His Gln Arg Arg Gly Gly Ser Val Glu Ser Gln His Gln
            180                 185                 190
Gln Pro Val Val Ala Ala Val Lys Ile Glu Leu Asp Gln Leu Val Val
        195                 200                 205
Ser Ile Leu Asp Asp Pro Ser Val Ser Arg Val Met Arg Asp Ala Gly
    210                 215                 220
Phe Ser Ser Thr Gln Val Lys Ala Asn Val Glu Gln Ala Val Cys Ser
225                 230                 235                 240
Ser Ser Thr Met Ala Thr Ala Ala Ala Ala Pro Ser Lys Asn Pro Asn
                245                 250                 255
Pro Ser Ser Ser Ala Thr Thr Thr Ser Pro Pro Pro Lys Glu Ala Lys
            260                 265                 270
Ala Lys Pro Pro Leu Ala Leu His His Gln Ala Arg Asp Glu Asp Val
        275                 280                 285
Ala Ala Val Leu Asp Cys Leu Ala Ser Arg Ser Lys Arg Arg Val Val
    290                 295                 300
Val Ile Ala Glu Ser Ala Ala Ala Ala Glu Ala Met Ala His Ala Ala
305                 310                 315                 320
```

```
Val Asp Lys Ile Lys Arg Gly Glu Val Lys His Glu His Asp Ala Leu
                325                 330                 335
Arg Gly Ala Gln Val Val Ser Leu Arg Val Ser Ser Phe Arg Glu Met
            340                 345                 350
Pro Arg Glu Glu Ala Glu Arg Arg Leu Gly Glu Leu Arg Cys Leu Val
        355                 360                 365
Lys Gln Gly Arg Arg Gln Arg Val Val Leu Val Val Glu Asp Leu Lys
    370                 375                 380
Trp Ala Ala Glu Phe Trp Ala Gly His Asp Gly Gln Ser Gly Arg Arg
385                 390                 395                 400
Gly Gly Tyr Tyr Tyr Cys Ala Val Glu His Val Val Asn Glu Leu Arg
                405                 410                 415
Ala Leu Ala Ser Gly Gly Ser Leu Cys Trp Leu Leu Gly Phe Gly Thr
            420                 425                 430
Tyr Gln Ala Tyr Thr Lys Cys Arg Val Gly Gln Pro Ser Leu Glu Ser
        435                 440                 445
Leu Trp Gly Leu Gln Thr Leu Thr Val Pro Ala Gly Ser Leu Ala Leu
    450                 455                 460
Ser Leu Thr Cys Ala Phe Asp Asp Ser Ala Leu Gly Thr Val Asn Gln
465                 470                 475                 480
Ser Leu Lys Ala Gly Ser Asp Ala Asp Gly Asn Gly Pro Ala Ser Cys
                485                 490                 495
Trp Pro Leu Leu Gly Gly Ser Gln Leu Ser Leu Ser Ser Arg Cys Cys
            500                 505                 510
Gly Asp Gly Asp Cys Ser Ala Ala Arg Ile Asp Thr Lys Ala Ala Leu
        515                 520                 525
Pro Arg Pro Ser Val Ser Ser Ser Asn Leu Pro Ser Trp Leu Gln His
    530                 535                 540
Cys Arg Asp His Gln Glu Pro Thr Thr Ser His Leu Thr Asp Leu Ser
545                 550                 555                 560
Lys Ser Lys Thr Trp Ser Ser Ile Cys Ser Arg Pro Ser Gln Arg Met
                565                 570                 575
Thr Leu His Phe Ser Ala Pro Val Ser Pro Ala Ser Ser Ile Ser Ser
            580                 585                 590
Tyr Glu His Gly Gly Asp Gln Ser Ser Gln Thr Pro Pro Gln His Ser
        595                 600                 605
Trp Leu Leu Ala Gly Leu Asp Ala Ala His His Arg Trp Glu Pro Lys
    610                 615                 620
Arg Glu Thr Ser Gly Asn Lys Ala Ala Ser Arg Ser His Asp Ser Gly
625                 630                 635                 640
Ala Ser Asn Gly Gly Ser Val Glu Val Glu Cys Arg Ala Arg Phe Lys
                645                 650                 655
Glu Leu Ser Ala Glu Asn Leu Lys Leu Leu Cys Gly Ala Leu Glu Lys
            660                 665                 670
Glu Val Pro Trp Gln Lys Glu Ile Ile Pro Glu Val Ala Ser Ala Val
        675                 680                 685
Leu Gln Cys Arg Ser Gly Ile Ala Lys Arg Arg Asp Lys Ser Arg Ser
    690                 695                 700
Ala Asp Ala Lys Glu Glu Thr Trp Met Leu Phe Leu Gly Gly Asp Ala
705                 710                 715                 720
Asp Gly Lys Glu Arg Val Ala Arg Glu Leu Ala Ser Leu Val Phe Gly
                725                 730                 735
Ser Arg Asp Ser Phe Leu Ala Ile Arg Pro Gly Ala Ser Ser Ser Ser
            740                 745                 750
Pro Pro Arg Ser Gly Ser Ser Glu Gly His Arg Ser Asn Lys Arg Pro
        755                 760                 765
Arg Met Ser Pro Pro Pro Pro Gly Glu Glu Ser Ala Pro Ala Cys Leu
    770                 775                 780
Glu Arg Leu His Asp Ala Val Ser Glu Asn Pro His Arg Val Ile Phe
785                 790                 795                 800
Met Glu Gly Val Glu Gln Ala Gly Arg Asp Cys Gln Leu Gly Ile Lys
                805                 810                 815
Glu Ala Ile Glu Ser Gly Val Val Arg Asn Arg Ala Gly Val Glu Val
            820                 825                 830
Gly Val Gly Asp Ala Ile Val Val Leu Ser Cys Glu Gly Phe Gly Ser
```

```
        835                 840                 845
Thr Gly Arg Ser Arg Ala Cys Ser Pro Pro Ser Lys Lys Val Lys Val
    850                 855                 860
Glu Met Glu Glu Ala Lys Glu Glu Arg Thr Gly Asp Ile Glu His Asn
865                 870                 875                 880
Gly Asp Gly Gly Ser Ser Ser Ser Pro Ser Cys Ile Asp Leu Asn Val
                885                 890                 895
Asp Met Glu Ser Asp Gln Ala Asp Glu Gly Ser Leu Asp Asp Asp Val
            900                 905                 910
Cys Leu Leu Thr Ala Val Asp Arg Thr Leu Phe Phe Arg Arg Gln Gly
        915                 920                 925

<210> 11
<211> 2589
<212> DNA
<213> Zea mays

<220>
<221> CDS
<222> (1)..(2589)

<400> 11
atg agg gcc ggg ggg tgc acg gtg cag cag tcg ctg acg gcg gag gcc       48
Met Arg Ala Gly Gly Cys Thr Val Gln Gln Ser Leu Thr Ala Glu Ala
 1               5                  10                  15
gcg gcg gtg gtg aag cag gcg gtg agc ctt gcg cgg cgg cgc ggg aac       96
Ala Ala Val Val Lys Gln Ala Val Ser Leu Ala Arg Arg Arg Gly Asn
            20                  25                  30
gcg cag gtg acg ccg cta cac gtg gcg agc gcg atg cta gcg gcg ccc      144
Ala Gln Val Thr Pro Leu His Val Ala Ser Ala Met Leu Ala Ala Pro
        35                  40                  45
acg ggg ttg ctg cgc gcg gcg tgc ctc cgc tcg cac tcc cac ccg ctg      192
Thr Gly Leu Leu Arg Ala Ala Cys Leu Arg Ser His Ser His Pro Leu
    50                  55                  60
cag tgc aag gcg ctg gag ctg tgc ttc aac gtg gcg ctg aac cgc ctc      240
Gln Cys Lys Ala Leu Glu Leu Cys Phe Asn Val Ala Leu Asn Arg Leu
65                  70                  75                  80
ccc gcg tcg gcc gcg gtc gcc tcg tcg ccg ctg ctc ggc ggg cac ggc      288
Pro Ala Ser Ala Ala Val Ala Ser Ser Pro Leu Leu Gly Gly His Gly
                85                  90                  95
cac cac cac cac cac cac tac tac ccg ccg tcc ctg tcc aac gcg ctc      336
His His His His His His Tyr Tyr Pro Pro Ser Leu Ser Asn Ala Leu
            100                 105                 110
gtg gcg gcg ttc aag cgc gcg cag gcg cac cag cgg cgc ggg tcc gtg      384
Val Ala Ala Phe Lys Arg Ala Gln Ala His Gln Arg Arg Gly Ser Val
        115                 120                 125
gag agc cag cag cag ccg gtg ctc gcc gtc aag atc gag ctg gag cag      432
Glu Ser Gln Gln Gln Pro Val Leu Ala Val Lys Ile Glu Leu Glu Gln
    130                 135                 140
ctc gtg gtg tcc atc ctc gac gac ccc agc gtc agc cgc gtg atg cgc      480
Leu Val Val Ser Ile Leu Asp Asp Pro Ser Val Ser Arg Val Met Arg
145                 150                 155                 160
gag gct ggc ttc tcc agc acc cag gtg aag gcc aac gtg gag cag gcc      528
Glu Ala Gly Phe Ser Ser Thr Gln Val Lys Ala Asn Val Glu Gln Ala
                165                 170                 175
gtg tgc agc agc acc acc aca aca agc acc gcg gcc gcg gcc acc gct      576
Val Cys Ser Ser Thr Thr Thr Thr Ser Thr Ala Ala Ala Ala Thr Ala
            180                 185                 190
ccc agc aaa aac cct aat cct agc agc tct gct gcc acc gca agc cca      624
Pro Ser Lys Asn Pro Asn Pro Ser Ser Ser Ala Ala Thr Ala Ser Pro
        195                 200                 205
cct cag gaa gcc gcc aaa ggc aac aag ctg ccg ctc cac caa gcg cgc      672
Pro Gln Glu Ala Ala Lys Gly Asn Lys Leu Pro Leu His Gln Ala Arg
    210                 215                 220
gac gag gac gtc gcc gcc gtc ctg gac tgc ctg gcc tcg cgg agc aag      720
```

```
Asp Glu Asp Val Ala Ala Val Leu Asp Cys Leu Ala Ser Arg Ser Lys
225                 230                 235                 240
agg agg gtg gtc gtc atc gcg gag agc gcg gcg gcg gcc gag gcc acg      768
Arg Arg Val Val Val Ile Ala Glu Ser Ala Ala Ala Ala Glu Ala Thr
                245                 250                 255
gcg cat gcg gcc atg gac aag atc aag agc gcc gag gcg aag cac gac      816
Ala His Ala Ala Met Asp Lys Ile Lys Ser Ala Glu Ala Lys His Asp
            260                 265                 270
gcg ctg cga ggc gcg cag gtc gtc agc gtc cgc gtg tcg tcg ttc cgc      864
Ala Leu Arg Gly Ala Gln Val Val Ser Val Arg Val Ser Ser Phe Arg
        275                 280                 285
gag atg gcg agg gag gag acc gag cgg cgg ctt ggc gag ctg cgg tgc      912
Glu Met Ala Arg Glu Glu Thr Glu Arg Arg Leu Gly Glu Leu Arg Cys
    290                 295                 300
ctc gtc agg ggc agg agg ggg cag gtc gtc gtg ctc gtc gtg gag gac      960
Leu Val Arg Gly Arg Arg Gly Gln Val Val Val Leu Val Val Glu Asp
305                 310                 315                 320
ctc aag tgg gcg gcc gag ttc tgg gcg ggt cac gtc gtc cag agc ggg     1008
Leu Lys Trp Ala Ala Glu Phe Trp Ala Gly His Val Val Gln Ser Gly
                325                 330                 335
agg aga ggg tac tac tac tgc tcc gtc gag cac gtc gtc acc gag ctg     1056
Arg Arg Gly Tyr Tyr Tyr Cys Ser Val Glu His Val Val Thr Glu Leu
            340                 345                 350
cgc gcc ctg gcg agc ggc ggc ggc ggc agc ctg tgc tgg ctc ctc ggg     1104
Arg Ala Leu Ala Ser Gly Gly Gly Gly Ser Leu Cys Trp Leu Leu Gly
        355                 360                 365
ttc ggg acg tac cag gcc tac acg agg tgt cgg gtg ggg cag ccg tcg     1152
Phe Gly Thr Tyr Gln Ala Tyr Thr Arg Cys Arg Val Gly Gln Pro Ser
    370                 375                 380
ctg gag agc ctg tgg gag ctc cag acg ctc acc gtg ccc gcc ggc agc     1200
Leu Glu Ser Leu Trp Glu Leu Gln Thr Leu Thr Val Pro Ala Gly Ser
385                 390                 395                 400
ctc gcg ctg agc ctc aac tgc gcc ttc gac gac agt gct cta ggt cta     1248
Leu Ala Leu Ser Leu Asn Cys Ala Phe Asp Asp Ser Ala Leu Gly Leu
                405                 410                 415
ggc acg gtc aat cag tcc atg aaa gcg ggc tca tct gac acg gat ggg     1296
Gly Thr Val Asn Gln Ser Met Lys Ala Gly Ser Ser Asp Thr Asp Gly
            420                 425                 430
aac gga cca gcg tct tgc tgg ccg ctt ttg gcc ggc agc aag ctg atc     1344
Asn Gly Pro Ala Ser Cys Trp Pro Leu Leu Ala Gly Ser Lys Leu Ile
        435                 440                 445
tcc aga tgc tgc ggc ggc gac tgc tcc gcc gcg agg atc gag acg acg     1392
Ser Arg Cys Cys Gly Gly Asp Cys Ser Ala Ala Arg Ile Glu Thr Thr
    450                 455                 460
aag gcg gcg ctg cct cgg acg ccg ttc gtc tcg tcg tcc agc ctc cct     1440
Lys Ala Ala Leu Pro Arg Thr Pro Phe Val Ser Ser Ser Ser Leu Pro
465                 470                 475                 480
tcc tgg ctc cag cat tgc cgt gat cat cag gag ccc gct acc cat ctt     1488
Ser Trp Leu Gln His Cys Arg Asp His Gln Glu Pro Ala Thr His Leu
                485                 490                 495
acg gat gat ctc ggc aag aca tgg agc tcc atc tgc agc agc agc agg     1536
Thr Asp Asp Leu Gly Lys Thr Trp Ser Ser Ile Cys Ser Ser Ser Arg
            500                 505                 510
ccg tca cag cga acg acg acg ctg cat ttc tcg gcg ccg gtg tct ccg     1584
Pro Ser Gln Arg Thr Thr Thr Leu His Phe Ser Ala Pro Val Ser Pro
        515                 520                 525
gcc tcc tcc atc tct tcc tac gag cac ggc ggc ggc cag tcg cag cag     1632
Ala Ser Ser Ile Ser Ser Tyr Glu His Gly Gly Gly Gln Ser Gln Gln
    530                 535                 540
cct cgc cac tcg tgg ctc ctc gcc ggc ctc gac gcc gcg cac cac ccg     1680
Pro Arg His Ser Trp Leu Leu Ala Gly Leu Asp Ala Ala His His Pro
545                 550                 555                 560
tgg aga ccc aag cgc gag gcc agc atc agg tcc cac gac tcc ggc gcc     1728
Trp Arg Pro Lys Arg Glu Ala Ser Ile Arg Ser His Asp Ser Gly Ala
                565                 570                 575
```

```
tcc aac ggc tcc gtg gag gtg gag tgc cgc gcc agg ttc aag gag ctc         1776
Ser Asn Gly Ser Val Glu Val Glu Cys Arg Ala Arg Phe Lys Glu Leu
            580                 585                 590
aac gcc gag aac ctc aag ctg ctc tgc ggc gcg ctg gag aag gag gtg         1824
Asn Ala Glu Asn Leu Lys Leu Leu Cys Gly Ala Leu Glu Lys Glu Val
        595                 600                 605
ccg tgg cag aag gag atc gtc ccc gag gtc gcg agc gcc gtc ctg cag         1872
Pro Trp Gln Lys Glu Ile Val Pro Glu Val Ala Ser Ala Val Leu Gln
    610                 615                 620
tgc cgg tcg ggg atc gcc aag agg cgc gac aag tcc agg tcg gcg gac         1920
Cys Arg Ser Gly Ile Ala Lys Arg Arg Asp Lys Ser Arg Ser Ala Asp
625                 630                 635                 640
gcc aag gag gag acg tgg atg ctc ttc ctc gga ggc gac gcc gac ggc         1968
Ala Lys Glu Glu Thr Trp Met Leu Phe Leu Gly Gly Asp Ala Asp Gly
                645                 650                 655
aag gag agg gtt gcg agg gag ctc gcc agg ctc gtg ttc ggg tta cgc         2016
Lys Glu Arg Val Ala Arg Glu Leu Ala Arg Leu Val Phe Gly Leu Arg
            660                 665                 670
agc agc ttt ttg tcc atc agg cct ggc ggt gtc gtc tcc gcc tcg tcg         2064
Ser Ser Phe Leu Ser Ile Arg Pro Gly Gly Val Val Ser Ala Ser Ser
        675                 680                 685
cca ccg ccg gcc tcg tca ggc tcc tcc gag ggg cac agg agt agt aag         2112
Pro Pro Pro Ala Ser Ser Gly Ser Ser Glu Gly His Arg Ser Ser Lys
    690                 695                 700
cgc cca cgg atg ccg gag gag gag ccg gcg gcc tac tac cta gag cgg         2160
Arg Pro Arg Met Pro Glu Glu Glu Pro Ala Ala Tyr Tyr Leu Glu Arg
705                 710                 715                 720
ttg cac gag gct gtc tcc gaa aac cca cac cgg gtc ata ttc atg gag         2208
Leu His Glu Ala Val Ser Glu Asn Pro His Arg Val Ile Phe Met Glu
                725                 730                 735
gac gtc gag cgg gct gac cgg gac tgc cag ctc cgc atc aag gag gcg         2256
Asp Val Glu Arg Ala Asp Arg Asp Cys Gln Leu Arg Ile Lys Glu Ala
            740                 745                 750
atc gag agc ggg gtg gtg cgg aac cat gct ggt caa gag gtc ggc gtg         2304
Ile Glu Ser Gly Val Val Arg Asn His Ala Gly Gln Glu Val Gly Val
        755                 760                 765
ggc gac gcc atc gtc atc ctc agc tgc gag agc ttt ggc gac agc agg         2352
Gly Asp Ala Ile Val Ile Leu Ser Cys Glu Ser Phe Gly Asp Ser Arg
    770                 775                 780
tct agt aga gct tgc tcg ccg ccg agc aag aag gtg aag gta gag atg         2400
Ser Ser Arg Ala Cys Ser Pro Pro Ser Lys Lys Val Lys Val Glu Met
785                 790                 795                 800
gag gag gcc aag gag gag cgc gca ggt gac cat gaa cac aac caa gat         2448
Glu Glu Ala Lys Glu Glu Arg Ala Gly Asp His Glu His Asn Gln Asp
                805                 810                 815
ggt gtt tcc aag ccg tct cca tct tgc ata gat ttg aac gtg gac atg         2496
Gly Val Ser Lys Pro Ser Pro Ser Cys Ile Asp Leu Asn Val Asp Met
            820                 825                 830
gag agt gat cag gcg gac gag cca agt ctc ggt gat cag tgt ctg ctc         2544
Glu Ser Asp Gln Ala Asp Glu Pro Ser Leu Gly Asp Gln Cys Leu Leu
        835                 840                 845
acg gcc gtc gac agg gcc cta ttc ttc aga aga cag gac aac tag             2589
Thr Ala Val Asp Arg Ala Leu Phe Phe Arg Arg Gln Asp Asn
    850                 855                 860
```

<210> 12
<211> 862
<212> PRT
<213> Zea mays

<400> 12

```
Met Arg Ala Gly Gly Cys Thr Val Gln Gln Ser Leu Thr Ala Glu Ala
1               5                   10                  15
Ala Ala Val Val Lys Gln Ala Val Ser Leu Ala Arg Arg Arg Gly Asn
            20                  25                  30
```

```
Ala Gln Val Thr Pro Leu His Val Ala Ser Ala Met Leu Ala Ala Pro
        35                  40                  45
Thr Gly Leu Leu Arg Ala Ala Cys Leu Arg Ser His Ser His Pro Leu
    50                  55                  60
Gln Cys Lys Ala Leu Glu Leu Cys Phe Asn Val Ala Leu Asn Arg Leu
65                  70                  75                  80
Pro Ala Ser Ala Ala Val Ala Ser Ser Pro Leu Leu Gly Gly His Gly
                85                  90                  95
His His His His His His Tyr Tyr Pro Pro Ser Leu Ser Asn Ala Leu
            100                 105                 110
Val Ala Ala Phe Lys Arg Ala Gln Ala His Gln Arg Arg Gly Ser Val
        115                 120                 125
Glu Ser Gln Gln Gln Pro Val Leu Ala Val Lys Ile Glu Leu Glu Gln
    130                 135                 140
Leu Val Val Ser Ile Leu Asp Asp Pro Ser Val Ser Arg Val Met Arg
145                 150                 155                 160
Glu Ala Gly Phe Ser Ser Thr Gln Val Lys Ala Asn Val Glu Gln Ala
                165                 170                 175
Val Cys Ser Ser Thr Thr Thr Thr Ser Thr Ala Ala Ala Ala Thr Ala
            180                 185                 190
Pro Ser Lys Asn Pro Asn Pro Ser Ser Ser Ala Ala Thr Ala Ser Pro
        195                 200                 205
Pro Gln Glu Ala Ala Lys Gly Asn Lys Leu Pro Leu His Gln Ala Arg
    210                 215                 220
Asp Glu Asp Val Ala Ala Val Leu Asp Cys Leu Ala Ser Arg Ser Lys
225                 230                 235                 240
Arg Arg Val Val Val Ile Ala Glu Ser Ala Ala Ala Ala Glu Ala Thr
                245                 250                 255
Ala His Ala Ala Met Asp Lys Ile Lys Ser Ala Glu Ala Lys His Asp
            260                 265                 270
Ala Leu Arg Gly Ala Gln Val Val Ser Val Arg Val Ser Ser Phe Arg
        275                 280                 285
Glu Met Ala Arg Glu Glu Thr Glu Arg Arg Leu Gly Glu Leu Arg Cys
    290                 295                 300
Leu Val Arg Gly Arg Arg Gly Gln Val Val Val Leu Val Val Glu Asp
305                 310                 315                 320
Leu Lys Trp Ala Ala Glu Phe Trp Ala Gly His Val Val Gln Ser Gly
                325                 330                 335
Arg Arg Gly Tyr Tyr Tyr Cys Ser Val Glu His Val Val Thr Glu Leu
            340                 345                 350
Arg Ala Leu Ala Ser Gly Gly Gly Gly Ser Leu Cys Trp Leu Leu Gly
        355                 360                 365
Phe Gly Thr Tyr Gln Ala Tyr Thr Arg Cys Arg Val Gly Gln Pro Ser
    370                 375                 380
Leu Glu Ser Leu Trp Glu Leu Gln Thr Leu Thr Val Pro Ala Gly Ser
385                 390                 395                 400
Leu Ala Leu Ser Leu Asn Cys Ala Phe Asp Asp Ser Ala Leu Gly Leu
                405                 410                 415
Gly Thr Val Asn Gln Ser Met Lys Ala Gly Ser Ser Asp Thr Asp Gly
            420                 425                 430
Asn Gly Pro Ala Ser Cys Trp Pro Leu Leu Ala Gly Ser Lys Leu Ile
        435                 440                 445
Ser Arg Cys Cys Gly Gly Asp Cys Ser Ala Ala Arg Ile Glu Thr Thr
    450                 455                 460
Lys Ala Ala Leu Pro Arg Thr Pro Phe Val Ser Ser Ser Ser Leu Pro
465                 470                 475                 480
Ser Trp Leu Gln His Cys Arg Asp His Gln Glu Pro Ala Thr His Leu
                485                 490                 495
Thr Asp Asp Leu Gly Lys Thr Trp Ser Ser Ile Cys Ser Ser Ser Arg
            500                 505                 510
Pro Ser Gln Arg Thr Thr Thr Leu His Phe Ser Ala Pro Val Ser Pro
        515                 520                 525
Ala Ser Ser Ile Ser Ser Tyr Glu His Gly Gly Gly Gln Ser Gln Gln
    530                 535                 540
Pro Arg His Ser Trp Leu Leu Ala Gly Leu Asp Ala Ala His His Pro
```

545 550 555 560
Trp Arg Pro Lys Arg Glu Ala Ser Ile Arg Ser His Asp Ser Gly Ala
565 570 575
Ser Asn Gly Ser Val Glu Val Glu Cys Arg Ala Arg Phe Lys Glu Leu
580 585 590
Asn Ala Glu Asn Leu Lys Leu Leu Cys Gly Ala Leu Glu Lys Glu Val
595 600 605
Pro Trp Gln Lys Glu Ile Val Pro Glu Val Ala Ser Ala Val Leu Gln
610 615 620
Cys Arg Ser Gly Ile Ala Lys Arg Arg Asp Lys Ser Arg Ser Ala Asp
625 630 635 640
Ala Lys Glu Glu Thr Trp Met Leu Phe Leu Gly Gly Asp Ala Asp Gly
645 650 655
Lys Glu Arg Val Ala Arg Glu Leu Ala Arg Leu Val Phe Gly Leu Arg
660 665 670
Ser Ser Phe Leu Ser Ile Arg Pro Gly Gly Val Val Ser Ala Ser Ser
675 680 685
Pro Pro Pro Ala Ser Ser Gly Ser Ser Glu Gly His Arg Ser Ser Lys
690 695 700
Arg Pro Arg Met Pro Glu Glu Glu Pro Ala Ala Tyr Tyr Leu Glu Arg
705 710 715 720
Leu His Glu Ala Val Ser Glu Asn Pro His Arg Val Ile Phe Met Glu
725 730 735
Asp Val Glu Arg Ala Asp Arg Asp Cys Gln Leu Arg Ile Lys Glu Ala
740 745 750
Ile Glu Ser Gly Val Val Arg Asn His Ala Gly Gln Glu Val Gly Val
755 760 765
Gly Asp Ala Ile Val Ile Leu Ser Cys Glu Ser Phe Gly Asp Ser Arg
770 775 780
Ser Ser Arg Ala Cys Ser Pro Pro Ser Lys Lys Val Lys Val Glu Met
785 790 795 800
Glu Glu Ala Lys Glu Glu Arg Ala Gly Asp His Glu His Asn Gln Asp
805 810 815
Gly Val Ser Lys Pro Ser Pro Ser Cys Ile Asp Leu Asn Val Asp Met
820 825 830
Glu Ser Asp Gln Ala Asp Glu Pro Ser Leu Gly Asp Gln Cys Leu Leu
835 840 845
Thr Ala Val Asp Arg Ala Leu Phe Phe Arg Arg Gln Asp Asn
850 855 860

<210> 13
<211> 2625
<212> DNA
<213> Glycine max

<220>
<221> CDS
<222> (1)..(2625)

<400> 13
atg aga act gga agc tgc gcc gtg caa cag ggt cta acc ccg gag gcc 48
Met Arg Thr Gly Ser Cys Ala Val Gln Gln Gly Leu Thr Pro Glu Ala
1 5 10 15
gcg agc ata gtg aag caa gcc gtg acg ctg gcg aag cgt cgc ggc cat 96
Ala Ser Ile Val Lys Gln Ala Val Thr Leu Ala Lys Arg Arg Gly His
20 25 30
gca cag gtg acg ccc ctc cac gtg gcg aac acc atg ctc tcc atc acc 144
Ala Gln Val Thr Pro Leu His Val Ala Asn Thr Met Leu Ser Ile Thr
35 40 45
aac ggt ctt ctg aga acc gcg tgt ctc caa tcc cac tcc cac cct ctc 192
Asn Gly Leu Leu Arg Thr Ala Cys Leu Gln Ser His Ser His Pro Leu
50 55 60
cag tgc aag gcc ttg gag ctt tgc ttc aac gtt gcc ctg aat cgc tta 240
Gln Cys Lys Ala Leu Glu Leu Cys Phe Asn Val Ala Leu Asn Arg Leu
65 70 75 80

```
ccg gct tcg act tct tct agc ccc atg ttg caa ggc tct cac cac cac      288
Pro Ala Ser Thr Ser Ser Ser Pro Met Leu Gln Gly Ser His His His
                85                  90                  95
cac tct cac gcg tgc ccc tcc atc tca aac gct ttg gtt gca gcg ttc      336
His Ser His Ala Cys Pro Ser Ile Ser Asn Ala Leu Val Ala Ala Phe
            100                 105                 110
aaa cgc gct caa gcc cac cag cga cgt gga tcc gtc gag aat cag caa      384
Lys Arg Ala Gln Ala His Gln Arg Arg Gly Ser Val Glu Asn Gln Gln
        115                 120                 125
caa cct ctc ttg gca gtt aaa ata aaa ctc gag caa ctc att att tcg      432
Gln Pro Leu Leu Ala Val Lys Ile Lys Leu Glu Gln Leu Ile Ile Ser
    130                 135                 140
atc ttg gat gac cct agt gtt agc cga gtc atg aga gaa gct ggt ttc      480
Ile Leu Asp Asp Pro Ser Val Ser Arg Val Met Arg Glu Ala Gly Phe
145                 150                 155                 160
tct agc acc caa gtt aaa agc aac gtt gaa caa gct gtt tct ttg gaa      528
Ser Ser Thr Gln Val Lys Ser Asn Val Glu Gln Ala Val Ser Leu Glu
                165                 170                 175
ata tgc tct cag gat aat ggt agt ggt aaa aat aac aac aat agt aat      576
Ile Cys Ser Gln Asp Asn Gly Ser Gly Lys Asn Asn Asn Asn Ser Asn
            180                 185                 190
aag gca aag gaa aac aat agc agt gga gag aaa ggg tcg gtg ttg gat      624
Lys Ala Lys Glu Asn Asn Ser Ser Gly Glu Lys Gly Ser Val Leu Asp
        195                 200                 205
cct att agg gtt gag gat gtt gct agt gtt att gaa aat ttg ggg agt      672
Pro Ile Arg Val Glu Asp Val Ala Ser Val Ile Glu Asn Leu Gly Ser
    210                 215                 220
gag aga aag aga agt gtt gtg att gtg gga gag tgt gtt act agt ctt      720
Glu Arg Lys Arg Ser Val Val Ile Val Gly Glu Cys Val Thr Ser Leu
225                 230                 235                 240
gaa ggt gta gtt agg gga gtg atg gaa aaa gtt gat aaa ggg gat gtt      768
Glu Gly Val Val Arg Gly Val Met Glu Lys Val Asp Lys Gly Asp Val
                245                 250                 255
ggt gat gag tgt act ctt agg ggt gtt aag ttt atc tct ctt tct ctt      816
Gly Asp Glu Cys Thr Leu Arg Gly Val Lys Phe Ile Ser Leu Ser Leu
            260                 265                 270
tct tct ttt ggg aat gtt tcg aga gtg gag gtg gag caa aag gtg ggg      864
Ser Ser Phe Gly Asn Val Ser Arg Val Glu Val Glu Gln Lys Val Gly
        275                 280                 285
gag ctt agg agt ctt gtg aag gca agt gaa cat agc aaa ggg tat gtt      912
Glu Leu Arg Ser Leu Val Lys Ala Ser Glu His Ser Lys Gly Tyr Val
    290                 295                 300
ttg tat ttg ggg gat ctc aaa tgg gtg ttt gat ttt agg gct cgt gga      960
Leu Tyr Leu Gly Asp Leu Lys Trp Val Phe Asp Phe Arg Ala Arg Gly
305                 310                 315                 320
tca caa ggg gga ggg tgt tat tgt cct gtg gat cac atg gta gta gag     1008
Ser Gln Gly Gly Gly Cys Tyr Cys Pro Val Asp His Met Val Val Glu
                325                 330                 335
att ggg aag ctt gtg aat ggg gtt gaa gag aat ggt gca agg ttt tgg     1056
Ile Gly Lys Leu Val Asn Gly Val Glu Glu Asn Gly Ala Arg Phe Trp
            340                 345                 350
gtg atg ggt gtt gcc act ttt caa gct tac atg aga tgc aaa aat ggc     1104
Val Met Gly Val Ala Thr Phe Gln Ala Tyr Met Arg Cys Lys Asn Gly
        355                 360                 365
cag cca tcg ttg gag act ctt tgg ggt ctt cat cct atc act att cct     1152
Gln Pro Ser Leu Glu Thr Leu Trp Gly Leu His Pro Ile Thr Ile Pro
    370                 375                 380
gcg ggg agc ttg cgc ttg agt ctc atc act gac agt ggt gta caa aac     1200
Ala Gly Ser Leu Arg Leu Ser Leu Ile Thr Asp Ser Gly Val Gln Asn
385                 390                 395                 400
cag ccc aca aac gag aaa gct gac aac aga act acc tgg ctc tta ctt     1248
Gln Pro Thr Asn Glu Lys Ala Asp Asn Arg Thr Thr Trp Leu Leu Leu
                405                 410                 415
gaa gga gtg ggg gat gat cac aaa caa caa cct tgc ttc gcg gaa cct     1296
Glu Gly Val Gly Asp Asp His Lys Gln Gln Pro Cys Phe Ala Glu Pro
```

```
            420                 425                 430
tcc acc aag aat gag acc acc gaa gtt cga agc ttg caa agc agt agt      1344
Ser Thr Lys Asn Glu Thr Thr Glu Val Arg Ser Leu Gln Ser Ser Ser
        435                 440                 445
act tgt aat agt gac tca agt tca acc ctt cct gca tgg ctc caa cag      1392
Thr Cys Asn Ser Asp Ser Ser Ser Thr Leu Pro Ala Trp Leu Gln Gln
    450                 455                 460
tac aaa aat gag aat aaa gga atc acc cat aat gat cag aac tgt gtc      1440
Tyr Lys Asn Glu Asn Lys Gly Ile Thr His Asn Asp Gln Asn Cys Val
465                 470                 475                 480
cca gtg gga gag ctt tgc aaa aag tgg aac tct atg tgt agt tca atc      1488
Pro Val Gly Glu Leu Cys Lys Lys Trp Asn Ser Met Cys Ser Ser Ile
                485                 490                 495
caa aag caa cct tat cct tcc gac aaa acc ctc tca tta tcc tcg gtg      1536
Gln Lys Gln Pro Tyr Pro Ser Asp Lys Thr Leu Ser Leu Ser Ser Val
            500                 505                 510
tca cct tct tca tca aac tca aac ttc tca tac gaa cag cag cat cct      1584
Ser Pro Ser Ser Ser Asn Ser Asn Phe Ser Tyr Glu Gln Gln His Pro
        515                 520                 525
aat ttg ctc caa acc cat cac gag tgg caa gtg ggt gaa cca ccc aaa      1632
Asn Leu Leu Gln Thr His His Glu Trp Gln Val Gly Glu Pro Pro Lys
    530                 535                 540
gat tca ttg aac aac tac cat ttc tgg atc tcc aac aat ggc acc aac      1680
Asp Ser Leu Asn Asn Tyr His Phe Trp Ile Ser Asn Asn Gly Thr Asn
545                 550                 555                 560
aac aac acc aat gaa ccc act ttg aga gtg tac att cca gag aat aac      1728
Asn Asn Thr Asn Glu Pro Thr Leu Arg Val Tyr Ile Pro Glu Asn Asn
                565                 570                 575
aat aag caa cca ttc tca tca cca aat cca agt tca aac cct aat tcc      1776
Asn Lys Gln Pro Phe Ser Ser Pro Asn Pro Ser Ser Asn Pro Asn Ser
            580                 585                 590
act tct tcc agt gac atc atg gaa gtg gag cac gtg aga gag ttc aaa      1824
Thr Ser Ser Ser Asp Ile Met Glu Val Glu His Val Arg Glu Phe Lys
        595                 600                 605
gag tta aac acg gag aat ctg aaa aca ctg tgc aat gct ttg gag aaa      1872
Glu Leu Asn Thr Glu Asn Leu Lys Thr Leu Cys Asn Ala Leu Glu Lys
    610                 615                 620
aaa gtg ccg tgg cag aaa gac ata ata ccc gaa att gcg agc acc ctt      1920
Lys Val Pro Trp Gln Lys Asp Ile Ile Pro Glu Ile Ala Ser Thr Leu
625                 630                 635                 640
ttg caa tgc cgt tct ggc atg gtg aga aga aaa ggg aaa gtg atg aga      1968
Leu Gln Cys Arg Ser Gly Met Val Arg Arg Lys Gly Lys Val Met Arg
                645                 650                 655
aac agt gaa gaa gtg aaa gaa gag acg tgg ttg ttc ttc caa ggt gtt      2016
Asn Ser Glu Glu Val Lys Glu Glu Thr Trp Leu Phe Phe Gln Gly Val
            660                 665                 670
gat gtt gaa gcc aag gag aaa ata gca aga gaa cta gct aga ctt gtt      2064
Asp Val Glu Ala Lys Glu Lys Ile Ala Arg Glu Leu Ala Arg Leu Val
        675                 680                 685
ttt ggg tcc caa aac gac gtc gtt tcg att gca ctt agc act ttt gct      2112
Phe Gly Ser Gln Asn Asp Val Val Ser Ile Ala Leu Ser Thr Phe Ala
    690                 695                 700
tca aca aga gca gat tct act gaa gat tat agc agg aac aaa aga tca      2160
Ser Thr Arg Ala Asp Ser Thr Glu Asp Tyr Ser Arg Asn Lys Arg Ser
705                 710                 715                 720
aga gaa gaa aca agt tgc agt tac att gag agg ttc gca gaa gca atg      2208
Arg Glu Glu Thr Ser Cys Ser Tyr Ile Glu Arg Phe Ala Glu Ala Met
                725                 730                 735
gct tgt aac cct cac aga gtg ttt ctt gtt gaa gat ata gag caa gca      2256
Ala Cys Asn Pro His Arg Val Phe Leu Val Glu Asp Ile Glu Gln Ala
            740                 745                 750
gat tat tgt tct caa ctt ggt ttc aaa agg gcc att gag agg gga agg      2304
Asp Tyr Cys Ser Gln Leu Gly Phe Lys Arg Ala Ile Glu Arg Gly Arg
        755                 760                 765
gtt gcg gat tca aaa ggc gaa gag gtt gca ctt tgc gat gct atc atc      2352
```

```
Val Ala Asp Ser Lys Gly Glu Glu Val Ala Leu Cys Asp Ala Ile Ile
    770                 775                 780
att cta agc tgt gaa agt ttc agt tct agg tca agg gct tgt tct ccg      2400
Ile Leu Ser Cys Glu Ser Phe Ser Ser Arg Ser Arg Ala Cys Ser Pro
785                 790                 795                 800
tcg gtc aaa caa aaa ccg ctg act gag gaa gaa aag aat ggt ggt gac      2448
Ser Val Lys Gln Lys Pro Leu Thr Glu Glu Glu Lys Asn Gly Gly Asp
                805                 810                 815
atg gtt gca act ttg gag gtg aca agt cct tgt gtt tct ttg gat ttg      2496
Met Val Ala Thr Leu Glu Val Thr Ser Pro Cys Val Ser Leu Asp Leu
            820                 825                 830
aat att tcc att gat gat gaa aac gag gtt gag gat aag tca gtg gat      2544
Asn Ile Ser Ile Asp Asp Glu Asn Glu Val Glu Asp Lys Ser Val Asp
        835                 840                 845
gaa att ggg ctt ctt gaa tca gta gac aaa aag cta ggg atg aaa gaa      2592
Glu Ile Gly Leu Leu Glu Ser Val Asp Lys Lys Leu Gly Met Lys Glu
    850                 855                 860
att gcg tgc cct tca act tta tta tgt aac tag                          2625
Ile Ala Cys Pro Ser Thr Leu Leu Cys Asn
865                 870

<210> 14
<211> 874
<212> PRT
<213> Glycine max

<400> 14
Met Arg Thr Gly Ser Cys Ala Val Gln Gln Gly Leu Thr Pro Glu Ala
1               5                   10                  15
Ala Ser Ile Val Lys Gln Ala Val Thr Leu Ala Lys Arg Arg Gly His
            20                  25                  30
Ala Gln Val Thr Pro Leu His Val Ala Asn Thr Met Leu Ser Ile Thr
        35                  40                  45
Asn Gly Leu Leu Arg Thr Ala Cys Leu Gln Ser His Ser His Pro Leu
    50                  55                  60
Gln Cys Lys Ala Leu Glu Leu Cys Phe Asn Val Ala Leu Asn Arg Leu
65                  70                  75                  80
Pro Ala Ser Thr Ser Ser Ser Pro Met Leu Gln Gly Ser His His His
                85                  90                  95
His Ser His Ala Cys Pro Ser Ile Ser Asn Ala Leu Val Ala Ala Phe
            100                 105                 110
Lys Arg Ala Gln Ala His Gln Arg Arg Gly Ser Val Glu Asn Gln Gln
        115                 120                 125
Gln Pro Leu Leu Ala Val Lys Ile Lys Leu Glu Gln Leu Ile Ile Ser
    130                 135                 140
Ile Leu Asp Asp Pro Ser Val Ser Arg Val Met Arg Glu Ala Gly Phe
145                 150                 155                 160
Ser Ser Thr Gln Val Lys Ser Asn Val Glu Gln Ala Val Ser Leu Glu
                165                 170                 175
Ile Cys Ser Gln Asp Asn Gly Ser Gly Lys Asn Asn Asn Asn Ser Asn
            180                 185                 190
Lys Ala Lys Glu Asn Asn Ser Ser Gly Glu Lys Gly Ser Val Leu Asp
        195                 200                 205
Pro Ile Arg Val Glu Asp Val Ala Ser Val Ile Glu Asn Leu Gly Ser
    210                 215                 220
Glu Arg Lys Arg Ser Val Val Ile Val Gly Glu Cys Val Thr Ser Leu
225                 230                 235                 240
Glu Gly Val Val Arg Gly Val Met Glu Lys Val Asp Lys Gly Asp Val
                245                 250                 255
Gly Asp Glu Cys Thr Leu Arg Gly Val Lys Phe Ile Ser Leu Ser Leu
            260                 265                 270
Ser Ser Phe Gly Asn Val Ser Arg Val Glu Val Glu Gln Lys Val Gly
        275                 280                 285
Glu Leu Arg Ser Leu Val Lys Ala Ser Glu His Ser Lys Gly Tyr Val
    290                 295                 300
```

```
Leu Tyr Leu Gly Asp Leu Lys Trp Val Phe Asp Phe Arg Ala Arg Gly
305                 310                 315                 320
Ser Gln Gly Gly Gly Cys Tyr Cys Pro Val Asp His Met Val Val Glu
                325                 330                 335
Ile Gly Lys Leu Val Asn Gly Val Glu Glu Asn Gly Ala Arg Phe Trp
            340                 345                 350
Val Met Gly Val Ala Thr Phe Gln Ala Tyr Met Arg Cys Lys Asn Gly
        355                 360                 365
Gln Pro Ser Leu Glu Thr Leu Trp Gly Leu His Pro Ile Thr Ile Pro
    370                 375                 380
Ala Gly Ser Leu Arg Leu Ser Leu Ile Thr Asp Ser Gly Val Gln Asn
385                 390                 395                 400
Gln Pro Thr Asn Glu Lys Ala Asp Asn Arg Thr Thr Trp Leu Leu Leu
                405                 410                 415
Glu Gly Val Gly Asp Asp His Lys Gln Gln Pro Cys Phe Ala Glu Pro
            420                 425                 430
Ser Thr Lys Asn Glu Thr Thr Glu Val Arg Ser Leu Gln Ser Ser Ser
        435                 440                 445
Thr Cys Asn Ser Asp Ser Ser Ser Thr Leu Pro Ala Trp Leu Gln Gln
    450                 455                 460
Tyr Lys Asn Glu Asn Lys Gly Ile Thr His Asn Asp Gln Asn Cys Val
465                 470                 475                 480
Pro Val Gly Glu Leu Cys Lys Lys Trp Asn Ser Met Cys Ser Ser Ile
                485                 490                 495
Gln Lys Gln Pro Tyr Pro Ser Asp Lys Thr Leu Ser Leu Ser Ser Val
            500                 505                 510
Ser Pro Ser Ser Ser Asn Ser Asn Phe Ser Tyr Glu Gln Gln His Pro
        515                 520                 525
Asn Leu Leu Gln Thr His His Glu Trp Gln Val Gly Glu Pro Pro Lys
    530                 535                 540
Asp Ser Leu Asn Asn Tyr His Phe Trp Ile Ser Asn Asn Gly Thr Asn
545                 550                 555                 560
Asn Asn Thr Asn Glu Pro Thr Leu Arg Val Tyr Ile Pro Glu Asn Asn
                565                 570                 575
Asn Lys Gln Pro Phe Ser Ser Pro Asn Pro Ser Ser Asn Pro Asn Ser
            580                 585                 590
Thr Ser Ser Ser Asp Ile Met Glu Val Glu His Val Arg Glu Phe Lys
        595                 600                 605
Glu Leu Asn Thr Glu Asn Leu Lys Thr Leu Cys Asn Ala Leu Glu Lys
    610                 615                 620
Lys Val Pro Trp Gln Lys Asp Ile Ile Pro Glu Ile Ala Ser Thr Leu
625                 630                 635                 640
Leu Gln Cys Arg Ser Gly Met Val Arg Arg Lys Gly Lys Val Met Arg
                645                 650                 655
Asn Ser Glu Glu Val Lys Glu Glu Thr Trp Leu Phe Phe Gln Gly Val
            660                 665                 670
Asp Val Glu Ala Lys Glu Lys Ile Ala Arg Glu Leu Ala Arg Leu Val
        675                 680                 685
Phe Gly Ser Gln Asn Asp Val Val Ser Ile Ala Leu Ser Thr Phe Ala
    690                 695                 700
Ser Thr Arg Ala Asp Ser Thr Glu Asp Tyr Ser Arg Asn Lys Arg Ser
705                 710                 715                 720
Arg Glu Glu Thr Ser Cys Ser Tyr Ile Glu Arg Phe Ala Glu Ala Met
                725                 730                 735
Ala Cys Asn Pro His Arg Val Phe Leu Val Glu Asp Ile Glu Gln Ala
            740                 745                 750
Asp Tyr Cys Ser Gln Leu Gly Phe Lys Arg Ala Ile Glu Arg Gly Arg
        755                 760                 765
Val Ala Asp Ser Lys Gly Glu Glu Val Ala Leu Cys Asp Ala Ile Ile
    770                 775                 780
Ile Leu Ser Cys Glu Ser Phe Ser Ser Arg Ser Arg Ala Cys Ser Pro
785                 790                 795                 800
Ser Val Lys Gln Lys Pro Leu Thr Glu Glu Glu Lys Asn Gly Gly Asp
                805                 810                 815
Met Val Ala Thr Leu Glu Val Thr Ser Pro Cys Val Ser Leu Asp Leu
```

```
            820                 825                 830
Asn Ile Ser Ile Asp Asp Glu Asn Glu Val Glu Asp Lys Ser Val Asp
        835                 840                 845
Glu Ile Gly Leu Leu Glu Ser Val Asp Lys Lys Leu Gly Met Lys Glu
    850                 855                 860
Ile Ala Cys Pro Ser Thr Leu Leu Cys Asn
865                 870

<210> 15
<211> 2610
<212> DNA
<213> Glycine max

<220>
<221> CDS
<222> (1)..(2610)

<400> 15
atg aga aca gga agc tgc gcc gtg caa cag ggt cta acc cca gag gcc        48
Met Arg Thr Gly Ser Cys Ala Val Gln Gln Gly Leu Thr Pro Glu Ala
1               5                   10                  15
gcg agc ata gtg aag caa gcg gtg acg ctt gcg aag cgt cgc ggc cac        96
Ala Ser Ile Val Lys Gln Ala Val Thr Leu Ala Lys Arg Arg Gly His
            20                  25                  30
gcg cag gtg acg ccc ctc cac gtg gcg aac acc atg ctt tcc atc acc       144
Ala Gln Val Thr Pro Leu His Val Ala Asn Thr Met Leu Ser Ile Thr
        35                  40                  45
aac ggg ctt ctg aga aca gct tgt ctc caa tcc cac tct cac cct ctc       192
Asn Gly Leu Leu Arg Thr Ala Cys Leu Gln Ser His Ser His Pro Leu
    50                  55                  60
cag tgc aag gcc ttg gag ctt tgc ttc aac gtt gcc cta aat cgc tta       240
Gln Cys Lys Ala Leu Glu Leu Cys Phe Asn Val Ala Leu Asn Arg Leu
65                  70                  75                  80
ccg gct tcg act tct tcg agt ccc atg ttg caa ggc tct cac cac cac       288
Pro Ala Ser Thr Ser Ser Ser Pro Met Leu Gln Gly Ser His His His
                85                  90                  95
cac tct cac gcg tgc ccc tct atc tca aac gcc ctt gtt gca gct ttc       336
His Ser His Ala Cys Pro Ser Ile Ser Asn Ala Leu Val Ala Ala Phe
            100                 105                 110
aaa cgc gct caa gca cac caa cga cgt gga tct gtt gag aat cag caa       384
Lys Arg Ala Gln Ala His Gln Arg Arg Gly Ser Val Glu Asn Gln Gln
        115                 120                 125
cag cct ctc ttg gca gtg aaa att gaa ctc gag caa ctc att att tcg       432
Gln Pro Leu Leu Ala Val Lys Ile Glu Leu Glu Gln Leu Ile Ile Ser
    130                 135                 140
atc ttg gat gac cct agt gtt agc cga gtc atg aga gaa gct gat ttc       480
Ile Leu Asp Asp Pro Ser Val Ser Arg Val Met Arg Glu Ala Asp Phe
145                 150                 155                 160
aat agc aca caa gtt aaa agc aac gtt gaa caa gcg gtt tct ttg gaa       528
Asn Ser Thr Gln Val Lys Ser Asn Val Glu Gln Ala Val Ser Leu Glu
                165                 170                 175
ata tgc tct cag aat aat ggt agt ggt aat aac aat aac aac aat aat       576
Ile Cys Ser Gln Asn Asn Gly Ser Gly Asn Asn Asn Asn Asn Asn Asn
            180                 185                 190
aat aag gca gag gaa aat aat agt agt agt gga gag aaa ggg ttg gtg       624
Asn Lys Ala Glu Glu Asn Asn Ser Ser Ser Gly Glu Lys Gly Leu Val
        195                 200                 205
ttg gat cct att agg gtt gag gat gtt gct agt gtt att gaa aat ttg       672
Leu Asp Pro Ile Arg Val Glu Asp Val Ala Ser Val Ile Glu Asn Leu
    210                 215                 220
ggg tgt gag aga aag aga agt gtt gtg att gtg gga gag tgt gtt act       720
Gly Cys Glu Arg Lys Arg Ser Val Val Ile Val Gly Glu Cys Val Thr
225                 230                 235                 240
agc ctt gaa ggt gta gtt agg gga gtg atg gaa aag att gat aaa ggg       768
Ser Leu Glu Gly Val Val Arg Gly Val Met Glu Lys Ile Asp Lys Gly
```

```
                245                 250                 255
gat gtt ggt gat gag tgt act ctc agg ggt gtc aag ttt atc tct ctt        816
Asp Val Gly Asp Glu Cys Thr Leu Arg Gly Val Lys Phe Ile Ser Leu
            260                 265                 270
tct ctt tct tct ttt ggg aat gtt tca aga gtg gag gtg gag caa aag        864
Ser Leu Ser Ser Phe Gly Asn Val Ser Arg Val Glu Val Glu Gln Lys
        275                 280                 285
gtg gag gag ctt agg ggt ctt gtg aag gca agt gaa cat agc aaa ggg        912
Val Glu Glu Leu Arg Gly Leu Val Lys Ala Ser Glu His Ser Lys Gly
    290                 295                 300
tat gtt ttg tat ttg ggg gat ctc aag tgg gtg tta gat ttt agg gct        960
Tyr Val Leu Tyr Leu Gly Asp Leu Lys Trp Val Leu Asp Phe Arg Ala
305                 310                 315                 320
agc gga tca caa ggc aga ggg tgt tat tgt cct gtg gat cac atg gtt       1008
Ser Gly Ser Gln Gly Arg Gly Cys Tyr Cys Pro Val Asp His Met Val
                325                 330                 335
ggg gag att ggg aag ctt gtg aat ggg act gaa gag aat ggt gga agg       1056
Gly Glu Ile Gly Lys Leu Val Asn Gly Thr Glu Glu Asn Gly Gly Arg
            340                 345                 350
ttt tgg gtt atg ggt gtt gcc act ttt caa gct tac atg aga tgc aaa       1104
Phe Trp Val Met Gly Val Ala Thr Phe Gln Ala Tyr Met Arg Cys Lys
        355                 360                 365
aat ggc cag cca tcg ttg gag act ctt tgg tgt ctt cat cct atc act       1152
Asn Gly Gln Pro Ser Leu Glu Thr Leu Trp Cys Leu His Pro Ile Thr
    370                 375                 380
att cct gct gga agc ttg cgc ttg agt ctc atc act gac agt ggt cta       1200
Ile Pro Ala Gly Ser Leu Arg Leu Ser Leu Ile Thr Asp Ser Gly Leu
385                 390                 395                 400
caa gac cag ccc aca aac aag aaa gct gac aac aga act agc tgg ctc       1248
Gln Asp Gln Pro Thr Asn Lys Lys Ala Asp Asn Arg Thr Ser Trp Leu
                405                 410                 415
tta ctt gaa gga gtg ggg gat gat cag aaa caa caa gct tgc ttc gcg       1296
Leu Leu Glu Gly Val Gly Asp Asp Gln Lys Gln Gln Ala Cys Phe Ala
            420                 425                 430
gaa cct tcc aca aag aat gag acc atc acc gaa gtt cga agc ttg caa       1344
Glu Pro Ser Thr Lys Asn Glu Thr Ile Thr Glu Val Arg Ser Leu Gln
        435                 440                 445
agc agt agt act tgt aat agt gac tcc tca agt tca acc ctt cct gca       1392
Ser Ser Ser Thr Cys Asn Ser Asp Ser Ser Ser Ser Thr Leu Pro Ala
    450                 455                 460
tgg ctc caa cag tac aaa aat gag aat aaa gga atc aac tat aat gat       1440
Trp Leu Gln Gln Tyr Lys Asn Glu Asn Lys Gly Ile Asn Tyr Asn Asp
465                 470                 475                 480
cag aac tct gtc cca gtg gga gag ctt tgc aaa aag tgg aaa ttt atg       1488
Gln Asn Ser Val Pro Val Gly Glu Leu Cys Lys Lys Trp Lys Phe Met
                485                 490                 495
tgt agt tca atc caa aag caa cct tat cct tcc gac aaa acc atc aca       1536
Cys Ser Ser Ile Gln Lys Gln Pro Tyr Pro Ser Asp Lys Thr Ile Thr
            500                 505                 510
tta tcc tcg gtg tca cct tct tca tca acc tca aac ttc tca tac gga       1584
Leu Ser Ser Val Ser Pro Ser Ser Ser Thr Ser Asn Phe Ser Tyr Gly
        515                 520                 525
caa caa cat cct aat ttg cac caa acc cat aac gag tgg caa gtg gct       1632
Gln Gln His Pro Asn Leu His Gln Thr His Asn Glu Trp Gln Val Ala
    530                 535                 540
gaa cca ccc aaa gat tca tta aac aac cac cat ttc tgg atc tcc aac       1680
Glu Pro Pro Lys Asp Ser Leu Asn Asn His His Phe Trp Ile Ser Asn
545                 550                 555                 560
aat ggt tcc aac aac acc aat gaa ccc act ttg aga gtg tac att cca       1728
Asn Gly Ser Asn Asn Thr Asn Glu Pro Thr Leu Arg Val Tyr Ile Pro
                565                 570                 575
gag aac aat aag caa cca ttc tca tca cca aat cca agt tct aac cct       1776
Glu Asn Asn Lys Gln Pro Phe Ser Ser Pro Asn Pro Ser Ser Asn Pro
            580                 585                 590
aat tcc act tct tcc agt gac atc atg gaa gtg gaa cac gtg agc aag       1824
```

```
Asn Ser Thr Ser Ser Ser Asp Ile Met Glu Val Glu His Val Ser Lys
        595                 600                 605
ttc aaa gag tta aac tcg gag aat ctt aaa acc tta tgc aat gct ttg      1872
Phe Lys Glu Leu Asn Ser Glu Asn Leu Lys Thr Leu Cys Asn Ala Leu
    610                 615                 620
gag aaa aaa ttg ccg tgg cag aaa gac ata ata ccc gaa att gcg agc      1920
Glu Lys Lys Leu Pro Trp Gln Lys Asp Ile Ile Pro Glu Ile Ala Ser
625                 630                 635                 640
acc ctt ttg caa tgc cgt tct ggc atg gtg aga aga aaa ggg aaa gtg      1968
Thr Leu Leu Gln Cys Arg Ser Gly Met Val Arg Arg Lys Gly Lys Val
                645                 650                 655
atg ata aac agt gaa gaa gtg aaa gaa gaa acg tgg ttg ttc ttc caa      2016
Met Ile Asn Ser Glu Glu Val Lys Glu Glu Thr Trp Leu Phe Phe Gln
            660                 665                 670
ggt gtt gat gtt gaa gcc aag gag aaa ata gca aga gaa ttg gct cgg      2064
Gly Val Asp Val Glu Ala Lys Glu Lys Ile Ala Arg Glu Leu Ala Arg
        675                 680                 685
ctt gtt ttc ggg tcc caa aac cac gtc gtt tcg att gca ctt agc act      2112
Leu Val Phe Gly Ser Gln Asn His Val Val Ser Ile Ala Leu Ser Thr
    690                 695                 700
ttt gct tca aca aga gca gat tct act gag gat tat agc agg aac aaa      2160
Phe Ala Ser Thr Arg Ala Asp Ser Thr Glu Asp Tyr Ser Arg Asn Lys
705                 710                 715                 720
aga tca aga gaa gaa aca agt tgc agt tac att gaa agg ttc gta gaa      2208
Arg Ser Arg Glu Glu Thr Ser Cys Ser Tyr Ile Glu Arg Phe Val Glu
                725                 730                 735
gca atg gct tct aac cct cat agg gtg ttt ctt gtt gaa gat ata gag      2256
Ala Met Ala Ser Asn Pro His Arg Val Phe Leu Val Glu Asp Ile Glu
            740                 745                 750
caa gca gat tat tgt tct caa ctt ggt ttc aaa agg gcc att gag agg      2304
Gln Ala Asp Tyr Cys Ser Gln Leu Gly Phe Lys Arg Ala Ile Glu Arg
        755                 760                 765
gga aga gtg gtg gat tca aaa ggt gaa gag gtt gca ctt cgt gat gct      2352
Gly Arg Val Val Asp Ser Lys Gly Glu Glu Val Ala Leu Arg Asp Ala
    770                 775                 780
atc atc att cta agc tgt gaa agt atc agt tct agg tca agg gct tgt      2400
Ile Ile Ile Leu Ser Cys Glu Ser Ile Ser Ser Arg Ser Arg Ala Cys
785                 790                 795                 800
tct ccc tcg gtc aaa caa aaa tcg tta acc gag gta gaa atg aat ggc      2448
Ser Pro Ser Val Lys Gln Lys Ser Leu Thr Glu Val Glu Met Asn Gly
                805                 810                 815
gac att aat aat gcc act ttg gag gag aca agc cct ttt gtt tct ttg      2496
Asp Ile Asn Asn Ala Thr Leu Glu Glu Thr Ser Pro Phe Val Ser Leu
            820                 825                 830
gat ttg aat att tcc att gat gat gaa aac aat gtt gag gat agg tca      2544
Asp Leu Asn Ile Ser Ile Asp Asp Glu Asn Asn Val Glu Asp Arg Ser
        835                 840                 845
gag gat gaa att ggg ctt ctt gaa tcg gta gac gga aaa gtt atc ttc      2592
Glu Asp Glu Ile Gly Leu Leu Glu Ser Val Asp Gly Lys Val Ile Phe
    850                 855                 860
aac ttt gag gaa ttg tga                                              2610
Asn Phe Glu Glu Leu
865

<210> 16
<211> 869
<212> PRT
<213> Glycine max

<400> 16
Met Arg Thr Gly Ser Cys Ala Val Gln Gln Gly Leu Thr Pro Glu Ala
1               5                   10                  15
Ala Ser Ile Val Lys Gln Ala Val Thr Leu Ala Lys Arg Arg Gly His
            20                  25                  30
Ala Gln Val Thr Pro Leu His Val Ala Asn Thr Met Leu Ser Ile Thr
```

```
                35                  40                  45
Asn Gly Leu Leu Arg Thr Ala Cys Leu Gln Ser His Ser His Pro Leu
        50                  55                  60
Gln Cys Lys Ala Leu Glu Leu Cys Phe Asn Val Ala Leu Asn Arg Leu
65                  70                  75                  80
Pro Ala Ser Thr Ser Ser Ser Pro Met Leu Gln Gly Ser His His His
                85                  90                  95
His Ser His Ala Cys Pro Ser Ile Ser Asn Ala Leu Val Ala Ala Phe
            100                 105                 110
Lys Arg Ala Gln Ala His Gln Arg Arg Gly Ser Val Glu Asn Gln Gln
        115                 120                 125
Gln Pro Leu Leu Ala Val Lys Ile Glu Leu Glu Gln Leu Ile Ile Ser
    130                 135                 140
Ile Leu Asp Asp Pro Ser Val Ser Arg Val Met Arg Glu Ala Asp Phe
145                 150                 155                 160
Asn Ser Thr Gln Val Lys Ser Asn Val Glu Gln Ala Val Ser Leu Glu
                165                 170                 175
Ile Cys Ser Gln Asn Asn Gly Ser Gly Asn Asn Asn Asn Asn Asn Asn
            180                 185                 190
Asn Lys Ala Glu Glu Asn Asn Ser Ser Ser Gly Glu Lys Gly Leu Val
        195                 200                 205
Leu Asp Pro Ile Arg Val Glu Asp Val Ala Ser Val Ile Glu Asn Leu
    210                 215                 220
Gly Cys Glu Arg Lys Arg Ser Val Val Ile Val Gly Glu Cys Val Thr
225                 230                 235                 240
Ser Leu Glu Gly Val Val Arg Gly Val Met Glu Lys Ile Asp Lys Gly
                245                 250                 255
Asp Val Gly Asp Glu Cys Thr Leu Arg Gly Val Lys Phe Ile Ser Leu
            260                 265                 270
Ser Leu Ser Ser Phe Gly Asn Val Ser Arg Val Glu Val Glu Gln Lys
        275                 280                 285
Val Glu Glu Leu Arg Gly Leu Val Lys Ala Ser Glu His Ser Lys Gly
    290                 295                 300
Tyr Val Leu Tyr Leu Gly Asp Leu Lys Trp Val Leu Asp Phe Arg Ala
305                 310                 315                 320
Ser Gly Ser Gln Gly Arg Gly Cys Tyr Cys Pro Val Asp His Met Val
                325                 330                 335
Gly Glu Ile Gly Lys Leu Val Asn Gly Thr Glu Glu Asn Gly Gly Arg
            340                 345                 350
Phe Trp Val Met Gly Val Ala Thr Phe Gln Ala Tyr Met Arg Cys Lys
        355                 360                 365
Asn Gly Gln Pro Ser Leu Glu Thr Leu Trp Cys Leu His Pro Ile Thr
    370                 375                 380
Ile Pro Ala Gly Ser Leu Arg Leu Ser Leu Ile Thr Asp Ser Gly Leu
385                 390                 395                 400
Gln Asp Gln Pro Thr Asn Lys Lys Ala Asp Asn Arg Thr Ser Trp Leu
                405                 410                 415
Leu Leu Glu Gly Val Gly Asp Asp Gln Lys Gln Gln Ala Cys Phe Ala
            420                 425                 430
Glu Pro Ser Thr Lys Asn Glu Thr Ile Thr Glu Val Arg Ser Leu Gln
        435                 440                 445
Ser Ser Ser Thr Cys Asn Ser Asp Ser Ser Ser Ser Thr Leu Pro Ala
    450                 455                 460
Trp Leu Gln Gln Tyr Lys Asn Glu Asn Lys Gly Ile Asn Tyr Asn Asp
465                 470                 475                 480
Gln Asn Ser Val Pro Val Gly Glu Leu Cys Lys Lys Trp Lys Phe Met
                485                 490                 495
Cys Ser Ser Ile Gln Lys Gln Pro Tyr Pro Ser Asp Lys Thr Ile Thr
            500                 505                 510
Leu Ser Ser Val Ser Pro Ser Ser Ser Thr Ser Asn Phe Ser Tyr Gly
        515                 520                 525
Gln Gln His Pro Asn Leu His Gln Thr His Asn Glu Trp Gln Val Ala
    530                 535                 540
Glu Pro Pro Lys Asp Ser Leu Asn Asn His His Phe Trp Ile Ser Asn
545                 550                 555                 560
```

Asn Gly Ser Asn Asn Thr Asn Glu Pro Thr Leu Arg Val Tyr Ile Pro
565 570 575
Glu Asn Asn Lys Gln Pro Phe Ser Ser Pro Asn Pro Ser Ser Asn Pro
580 585 590
Asn Ser Thr Ser Ser Ser Asp Ile Met Glu Val Glu His Val Ser Lys
595 600 605
Phe Lys Glu Leu Asn Ser Glu Asn Leu Lys Thr Leu Cys Asn Ala Leu
610 615 620
Glu Lys Lys Leu Pro Trp Gln Lys Asp Ile Ile Pro Glu Ile Ala Ser
625 630 635 640
Thr Leu Leu Gln Cys Arg Ser Gly Met Val Arg Arg Lys Gly Lys Val
645 650 655
Met Ile Asn Ser Glu Glu Val Lys Glu Glu Thr Trp Leu Phe Phe Gln
660 665 670
Gly Val Asp Val Glu Ala Lys Glu Lys Ile Ala Arg Glu Leu Ala Arg
675 680 685
Leu Val Phe Gly Ser Gln Asn His Val Val Ser Ile Ala Leu Ser Thr
690 695 700
Phe Ala Ser Thr Arg Ala Asp Ser Thr Glu Asp Tyr Ser Arg Asn Lys
705 710 715 720
Arg Ser Arg Glu Glu Thr Ser Cys Ser Tyr Ile Glu Arg Phe Val Glu
725 730 735
Ala Met Ala Ser Asn Pro His Arg Val Phe Leu Val Glu Asp Ile Glu
740 745 750
Gln Ala Asp Tyr Cys Ser Gln Leu Gly Phe Lys Arg Ala Ile Glu Arg
755 760 765
Gly Arg Val Val Asp Ser Lys Gly Glu Glu Val Ala Leu Arg Asp Ala
770 775 780
Ile Ile Ile Leu Ser Cys Glu Ser Ile Ser Ser Arg Ser Arg Ala Cys
785 790 795 800
Ser Pro Ser Val Lys Gln Lys Ser Leu Thr Glu Val Glu Met Asn Gly
805 810 815
Asp Ile Asn Asn Ala Thr Leu Glu Glu Thr Ser Pro Phe Val Ser Leu
820 825 830
Asp Leu Asn Ile Ser Ile Asp Asp Glu Asn Asn Val Glu Asp Arg Ser
835 840 845
Glu Asp Glu Ile Gly Leu Leu Glu Ser Val Asp Gly Lys Val Ile Phe
850 855 860
Asn Phe Glu Glu Leu
865

<210> 17
<211> 2532
<212> DNA
<213> Glycine max

<220>
<221> CDS
<222> (1)..(2532)

<400> 17
atg aga gca gga gtt tgc agc ata cag cta cag gct ctg aca tca gag 48
Met Arg Ala Gly Val Cys Ser Ile Gln Leu Gln Ala Leu Thr Ser Glu
1 5 10 15
gct gca acc cta gtt aaa caa gct gtt act ctt gcc aca aga aga ggg 96
Ala Ala Thr Leu Val Lys Gln Ala Val Thr Leu Ala Thr Arg Arg Gly
20 25 30
cat gct caa gtg acc cct ctt cac att gcc act gtc atg ctt gct act 144
His Ala Gln Val Thr Pro Leu His Ile Ala Thr Val Met Leu Ala Thr
35 40 45
tcc aca ggc ctt ctc cgc aaa gct tgc ctt caa tgc cac tct cac cct 192
Ser Thr Gly Leu Leu Arg Lys Ala Cys Leu Gln Cys His Ser His Pro
50 55 60
ctc caa tac aag gca ttg gaa ctt tgc ttc aac gtt tcc ctc aac cgt 240
Leu Gln Tyr Lys Ala Leu Glu Leu Cys Phe Asn Val Ser Leu Asn Arg

```
65                  70                  75                  80
ttg cca gca tcc aca cca aac cct ctt ctg ata agt cct cca tat aat        288
Leu Pro Ala Ser Thr Pro Asn Pro Leu Leu Ile Ser Pro Pro Tyr Asn
                85                  90                  95
tcc acc act act act ccc tca ctt tcc aat gcc ttg gtt gca gcc ttc        336
Ser Thr Thr Thr Thr Pro Ser Leu Ser Asn Ala Leu Val Ala Ala Phe
            100                 105                 110
aaa cgt gct cag gct cac caa cgc cgt gga tcc att gat cag aac cag        384
Lys Arg Ala Gln Ala His Gln Arg Arg Gly Ser Ile Asp Gln Asn Gln
        115                 120                 125
cag caa ccc att ttg act tta aag att aag gtg gag cag ctc ata gtc        432
Gln Gln Pro Ile Leu Thr Leu Lys Ile Lys Val Glu Gln Leu Ile Val
    130                 135                 140
tct atc ctt gat gac cct agt att agt agg gtc atg cga gaa gct ggt        480
Ser Ile Leu Asp Asp Pro Ser Ile Ser Arg Val Met Arg Glu Ala Gly
145                 150                 155                 160
ttc tct agc tcc ctt gtt aaa aca agg caa caa gct tat tcc aag gaa        528
Phe Ser Ser Ser Leu Val Lys Thr Arg Gln Gln Ala Tyr Ser Lys Glu
                165                 170                 175
aac acc acc gag ctt caa gtt ctt ggt ggt ggc tca ttc aag tct atg        576
Asn Thr Thr Glu Leu Gln Val Leu Gly Gly Gly Ser Phe Lys Ser Met
            180                 185                 190
gag gac ctt gtt cat gat gat gct ggt gat cat gta gta gat gat gta        624
Glu Asp Leu Val His Asp Asp Ala Gly Asp His Val Val Asp Asp Val
        195                 200                 205
act agt gtt ttg agc gaa ctt gtg agc aag aga aga aac aca gtc att        672
Thr Ser Val Leu Ser Glu Leu Val Ser Lys Arg Arg Asn Thr Val Ile
    210                 215                 220
gtt ggg gag agt ctt gct agt cct gag gga ata gtt agg gga ctg ata        720
Val Gly Glu Ser Leu Ala Ser Pro Glu Gly Ile Val Arg Gly Leu Ile
225                 230                 235                 240
gag aat tta gag aga ggg agt gtt caa gga gag ttg agg ttt gta caa        768
Glu Asn Leu Glu Arg Gly Ser Val Gln Gly Glu Leu Arg Phe Val Gln
                245                 250                 255
ttt gtg agt ctc cct ctt gtc tcc ttc agg aac att ggc aag aag gag        816
Phe Val Ser Leu Pro Leu Val Ser Phe Arg Asn Ile Gly Lys Lys Glu
            260                 265                 270
gtt gaa agg aag ctt gtg gag ctt aga aac ctt gta aaa agt cat gtg        864
Val Glu Arg Lys Leu Val Glu Leu Arg Asn Leu Val Lys Ser His Val
        275                 280                 285
gga aga ggg ttt att cta tac ttg ggt gat ctg aag tgg ttg ttt gag        912
Gly Arg Gly Phe Ile Leu Tyr Leu Gly Asp Leu Lys Trp Leu Phe Glu
    290                 295                 300
ttc tgg tca agt tac tgt gag caa aga aca aac tac tac tgt tct gtg        960
Phe Trp Ser Ser Tyr Cys Glu Gln Arg Thr Asn Tyr Tyr Cys Ser Val
305                 310                 315                 320
gtg cat ata gtg atg gag ctt aaa aaa ttg att agt gga aat ggg gag       1008
Val His Ile Val Met Glu Leu Lys Lys Leu Ile Ser Gly Asn Gly Glu
                325                 330                 335
aat ggt aga ttg tgg ctt atg ggg att gca act ttt gga aca tac atg       1056
Asn Gly Arg Leu Trp Leu Met Gly Ile Ala Thr Phe Gly Thr Tyr Met
            340                 345                 350
aag ggc caa gca tgt cac ccc tcc ctt gaa act att tgg gat ctt cac       1104
Lys Gly Gln Ala Cys His Pro Ser Leu Glu Thr Ile Trp Asp Leu His
        355                 360                 365
ctc ttt aca gtt cca gtg tta cta tca tcc ttg aga ctt ggt tta act       1152
Leu Phe Thr Val Pro Val Leu Leu Ser Ser Leu Arg Leu Gly Leu Thr
    370                 375                 380
ttt gat agt gat ttt caa gtt cag gag aga agc aag gta aca ttc aag       1200
Phe Asp Ser Asp Phe Gln Val Gln Glu Arg Ser Lys Val Thr Phe Lys
385                 390                 395                 400
gac gaa tct ttt gaa gaa cga gcc aaa gtg cgc aag tat cta act tgc       1248
Asp Glu Ser Phe Glu Glu Arg Ala Lys Val Arg Lys Tyr Leu Thr Cys
                405                 410                 415
tgc aga gat ttc tca tta aat ttt gaa aaa gaa gct aaa agc acc act       1296
```

```
Cys Arg Asp Phe Ser Leu Asn Phe Glu Lys Glu Ala Lys Ser Thr Thr
            420                 425                 430
aat agt att act ata agc aag aga gat tgc acc act aac ttg cca aca      1344
Asn Ser Ile Thr Ile Ser Lys Arg Asp Cys Thr Thr Asn Leu Pro Thr
        435                 440                 445
tgg ctc caa aat tgc aag gaa gag aga agt cgc atc atg gag aat cag      1392
Trp Leu Gln Asn Cys Lys Glu Glu Arg Ser Arg Ile Met Glu Asn Gln
    450                 455                 460
gaa aat gct aag ctt agg gat att tgt aag aaa tgg aac tca ttt tgc      1440
Glu Asn Ala Lys Leu Arg Asp Ile Cys Lys Lys Trp Asn Ser Phe Cys
465                 470                 475                 480
agt tca gcg cat gga ttt cct tcc aat cct gag aaa caa ttt ttt ttc      1488
Ser Ser Ala His Gly Phe Pro Ser Asn Pro Glu Lys Gln Phe Phe Phe
                485                 490                 495
att tca tcc tct cct tca tcc cct act tca gtc tct tca cat gaa aga      1536
Ile Ser Ser Ser Pro Ser Ser Pro Thr Ser Val Ser Ser His Glu Arg
            500                 505                 510
aag ctt agt ttg aat ctg aaa cac cta aat tgg cca ctc att tct gaa      1584
Lys Leu Ser Leu Asn Leu Lys His Leu Asn Trp Pro Leu Ile Ser Glu
        515                 520                 525
cca aaa caa gta cct aaa gaa tgt gag tta tac act gag act act gtt      1632
Pro Lys Gln Val Pro Lys Glu Cys Glu Leu Tyr Thr Glu Thr Thr Val
    530                 535                 540
agt gat gat tcc tat gaa gga aac ttg ata atg ttc atg cca gag aag      1680
Ser Asp Asp Ser Tyr Glu Gly Asn Leu Ile Met Phe Met Pro Glu Lys
545                 550                 555                 560
aac att cct aag cca gac ctt ttg tca aat ccc aat tct agc ccc aat      1728
Asn Ile Pro Lys Pro Asp Leu Leu Ser Asn Pro Asn Ser Ser Pro Asn
                565                 570                 575
tct gct tct tca agt gag gca gtg gaa ggt ttg gac agc act caa ata      1776
Ser Ala Ser Ser Ser Glu Ala Val Glu Gly Leu Asp Ser Thr Gln Ile
            580                 585                 590
ttc aag gag cat aat gat gaa aat cta aag att ctc tgt gat gca ttg      1824
Phe Lys Glu His Asn Asp Glu Asn Leu Lys Ile Leu Cys Asp Ala Leu
        595                 600                 605
ttg aaa aag gtt tca caa cag aaa gaa ata gtt aag gag att gca agc      1872
Leu Lys Lys Val Ser Gln Gln Lys Glu Ile Val Lys Glu Ile Ala Ser
    610                 615                 620
act gtg ctt ctt tgc agg tca gga atg aga gaa gga gtg aat cac tta      1920
Thr Val Leu Leu Cys Arg Ser Gly Met Arg Glu Gly Val Asn His Leu
625                 630                 635                 640
gtg aag aga gat gat agg caa gaa att tgg ttt ttc ttt tta ggt ttg      1968
Val Lys Arg Asp Asp Arg Gln Glu Ile Trp Phe Phe Phe Leu Gly Leu
                645                 650                 655
gat tct caa gca aaa gaa atg gtc tca aaa gag cta gct aaa gtt gtt      2016
Asp Ser Gln Ala Lys Glu Met Val Ser Lys Glu Leu Ala Lys Val Val
            660                 665                 670
ttt ggc tct tac agt aac ttt gtt tcc att ggt ata agt agt ttc tct      2064
Phe Gly Ser Tyr Ser Asn Phe Val Ser Ile Gly Ile Ser Ser Phe Ser
        675                 680                 685
tcc act cat gag gag tcc aaa aat aaa aga cca aga gat gag ttt ggt      2112
Ser Thr His Glu Glu Ser Lys Asn Lys Arg Pro Arg Asp Glu Phe Gly
    690                 695                 700
ggc agt tat ctt cag agg ttt ggg gaa gca ctg aat gag aac cct cat      2160
Gly Ser Tyr Leu Gln Arg Phe Gly Glu Ala Leu Asn Glu Asn Pro His
705                 710                 715                 720
agg gtg ttc ttc ttg gaa gat ttg gag caa gtt gat cat ttt tct aaa      2208
Arg Val Phe Phe Leu Glu Asp Leu Glu Gln Val Asp His Phe Ser Lys
                725                 730                 735
aag ggt gtt aag aaa gga att gaa agt gga act ata acc ctt cct ggt      2256
Lys Gly Val Lys Lys Gly Ile Glu Ser Gly Thr Ile Thr Leu Pro Gly
            740                 745                 750
ggt gaa tct gtg cct ctc aag gat gca att gtc att ttc agt agt gaa      2304
Gly Glu Ser Val Pro Leu Lys Asp Ala Ile Val Ile Phe Ser Ser Glu
        755                 760                 765
```

```
agc ttc agt tca gtg cca aga gct tgt tct cct gca aga acc act tct        2352
Ser Phe Ser Ser Val Pro Arg Ala Cys Ser Pro Ala Arg Thr Thr Ser
    770                 775                 780
cca ttt tcc gat gaa gac atg gaa aag aat aat att aac aaa tca gag        2400
Pro Phe Ser Asp Glu Asp Met Glu Lys Asn Asn Ile Asn Lys Ser Glu
785                 790                 795                 800
gag aaa act cca tgc ctc tct ttg gat ttg aac atg gcc att gaa gtt        2448
Glu Lys Thr Pro Cys Leu Ser Leu Asp Leu Asn Met Ala Ile Glu Val
                805                 810                 815
gat gtg caa aaa aat gtg cat tta gat gga gac act gct gag ata cta        2496
Asp Val Gln Lys Asn Val His Leu Asp Gly Asp Thr Ala Glu Ile Leu
            820                 825                 830
gag tta gtt gat aag caa att aat ttt aaa ata tga                        2532
Glu Leu Val Asp Lys Gln Ile Asn Phe Lys Ile
        835                 840

<210> 18
<211> 843
<212> PRT
<213> Glycine max

<400> 18
Met Arg Ala Gly Val Cys Ser Ile Gln Leu Gln Ala Leu Thr Ser Glu
1               5                   10                  15
Ala Ala Thr Leu Val Lys Gln Ala Val Thr Leu Ala Thr Arg Arg Gly
            20                  25                  30
His Ala Gln Val Thr Pro Leu His Ile Ala Thr Val Met Leu Ala Thr
        35                  40                  45
Ser Thr Gly Leu Leu Arg Lys Ala Cys Leu Gln Cys His Ser His Pro
    50                  55                  60
Leu Gln Tyr Lys Ala Leu Glu Leu Cys Phe Asn Val Ser Leu Asn Arg
65                  70                  75                  80
Leu Pro Ala Ser Thr Pro Asn Pro Leu Leu Ile Ser Pro Pro Tyr Asn
                85                  90                  95
Ser Thr Thr Thr Thr Pro Ser Leu Ser Asn Ala Leu Val Ala Ala Phe
            100                 105                 110
Lys Arg Ala Gln Ala His Gln Arg Arg Gly Ser Ile Asp Gln Asn Gln
        115                 120                 125
Gln Gln Pro Ile Leu Thr Leu Lys Ile Lys Val Glu Gln Leu Ile Val
    130                 135                 140
Ser Ile Leu Asp Asp Pro Ser Ile Ser Arg Val Met Arg Glu Ala Gly
145                 150                 155                 160
Phe Ser Ser Ser Leu Val Lys Thr Arg Gln Gln Ala Tyr Ser Lys Glu
                165                 170                 175
Asn Thr Thr Glu Leu Gln Val Leu Gly Gly Gly Ser Phe Lys Ser Met
            180                 185                 190
Glu Asp Leu Val His Asp Asp Ala Gly Asp His Val Val Asp Asp Val
        195                 200                 205
Thr Ser Val Leu Ser Glu Leu Val Ser Lys Arg Arg Asn Thr Val Ile
    210                 215                 220
Val Gly Glu Ser Leu Ala Ser Pro Glu Gly Ile Val Arg Gly Leu Ile
225                 230                 235                 240
Glu Asn Leu Glu Arg Gly Ser Val Gln Gly Glu Leu Arg Phe Val Gln
                245                 250                 255
Phe Val Ser Leu Pro Leu Val Ser Phe Arg Asn Ile Gly Lys Lys Glu
            260                 265                 270
Val Glu Arg Lys Leu Val Glu Leu Arg Asn Leu Val Lys Ser His Val
        275                 280                 285
Gly Arg Gly Phe Ile Leu Tyr Leu Gly Asp Leu Lys Trp Leu Phe Glu
    290                 295                 300
Phe Trp Ser Ser Tyr Cys Glu Gln Arg Thr Asn Tyr Tyr Cys Ser Val
305                 310                 315                 320
Val His Ile Val Met Glu Leu Lys Lys Leu Ile Ser Gly Asn Gly Glu
                325                 330                 335
Asn Gly Arg Leu Trp Leu Met Gly Ile Ala Thr Phe Gly Thr Tyr Met
```

```
            340                 345                 350
Lys Gly Gln Ala Cys His Pro Ser Leu Glu Thr Ile Trp Asp Leu His
        355                 360                 365
Leu Phe Thr Val Pro Val Leu Leu Ser Ser Leu Arg Leu Gly Leu Thr
    370                 375                 380
Phe Asp Ser Asp Phe Gln Val Gln Glu Arg Ser Lys Val Thr Phe Lys
385                 390                 395                 400
Asp Glu Ser Phe Glu Glu Arg Ala Lys Val Arg Lys Tyr Leu Thr Cys
                405                 410                 415
Cys Arg Asp Phe Ser Leu Asn Phe Glu Lys Glu Ala Lys Ser Thr Thr
            420                 425                 430
Asn Ser Ile Thr Ile Ser Lys Arg Asp Cys Thr Thr Asn Leu Pro Thr
        435                 440                 445
Trp Leu Gln Asn Cys Lys Glu Glu Arg Ser Arg Ile Met Glu Asn Gln
    450                 455                 460
Glu Asn Ala Lys Leu Arg Asp Ile Cys Lys Lys Trp Asn Ser Phe Cys
465                 470                 475                 480
Ser Ser Ala His Gly Phe Pro Ser Asn Pro Glu Lys Gln Phe Phe Phe
                485                 490                 495
Ile Ser Ser Ser Pro Ser Ser Pro Thr Ser Val Ser Ser His Glu Arg
            500                 505                 510
Lys Leu Ser Leu Asn Leu Lys His Leu Asn Trp Pro Leu Ile Ser Glu
        515                 520                 525
Pro Lys Gln Val Pro Lys Glu Cys Glu Leu Tyr Thr Glu Thr Thr Val
    530                 535                 540
Ser Asp Asp Ser Tyr Glu Gly Asn Leu Ile Met Phe Met Pro Glu Lys
545                 550                 555                 560
Asn Ile Pro Lys Pro Asp Leu Leu Ser Asn Pro Asn Ser Ser Pro Asn
                565                 570                 575
Ser Ala Ser Ser Ser Glu Ala Val Glu Gly Leu Asp Ser Thr Gln Ile
            580                 585                 590
Phe Lys Glu His Asn Asp Glu Asn Leu Lys Ile Leu Cys Asp Ala Leu
        595                 600                 605
Leu Lys Lys Val Ser Gln Gln Lys Glu Ile Val Lys Glu Ile Ala Ser
    610                 615                 620
Thr Val Leu Leu Cys Arg Ser Gly Met Arg Glu Gly Val Asn His Leu
625                 630                 635                 640
Val Lys Arg Asp Asp Arg Gln Glu Ile Trp Phe Phe Phe Leu Gly Leu
                645                 650                 655
Asp Ser Gln Ala Lys Glu Met Val Ser Lys Glu Leu Ala Lys Val Val
            660                 665                 670
Phe Gly Ser Tyr Ser Asn Phe Val Ser Ile Gly Ile Ser Ser Phe Ser
        675                 680                 685
Ser Thr His Glu Glu Ser Lys Asn Lys Arg Pro Arg Asp Glu Phe Gly
    690                 695                 700
Gly Ser Tyr Leu Gln Arg Phe Gly Glu Ala Leu Asn Glu Asn Pro His
705                 710                 715                 720
Arg Val Phe Phe Leu Glu Asp Leu Glu Gln Val Asp His Phe Ser Lys
                725                 730                 735
Lys Gly Val Lys Lys Gly Ile Glu Ser Gly Thr Ile Thr Leu Pro Gly
            740                 745                 750
Gly Glu Ser Val Pro Leu Lys Asp Ala Ile Val Ile Phe Ser Ser Glu
        755                 760                 765
Ser Phe Ser Ser Val Pro Arg Ala Cys Ser Pro Ala Arg Thr Thr Ser
    770                 775                 780
Pro Phe Ser Asp Glu Asp Met Glu Lys Asn Asn Ile Asn Lys Ser Glu
785                 790                 795                 800
Glu Lys Thr Pro Cys Leu Ser Leu Asp Leu Asn Met Ala Ile Glu Val
                805                 810                 815
Asp Val Gln Lys Asn Val His Leu Asp Gly Asp Thr Ala Glu Ile Leu
            820                 825                 830
Glu Leu Val Asp Lys Gln Ile Asn Phe Lys Ile
        835                 840
```

<210> 19

```
<211> 2523
<212> DNA
<213> Glycine max

<220>
<221> CDS
<222> (1)..(2523)

<400> 19
atg aga gga ggt att tgt agc ata cag ctt cag gct ctt aca cct gaa        48
Met Arg Gly Gly Ile Cys Ser Ile Gln Leu Gln Ala Leu Thr Pro Glu
1               5                   10                  15
gct aca act gtt gtc aag caa gct gtg aat ctt gca aca aga aga ggc        96
Ala Thr Thr Val Val Lys Gln Ala Val Asn Leu Ala Thr Arg Arg Gly
            20                  25                  30
cat gcg caa gtg aca cct ctt cat gtt gca agt gcc atg ctt gca act        144
His Ala Gln Val Thr Pro Leu His Val Ala Ser Ala Met Leu Ala Thr
        35                  40                  45
tcc aca ggc ctt ctt cgt aag gct tgt ctt cag tgt cac tct cac cct        192
Ser Thr Gly Leu Leu Arg Lys Ala Cys Leu Gln Cys His Ser His Pro
    50                  55                  60
ctt cag tgc aag gct ctt gag ctt tgt ttc aac gtt gca ctc aac cgt        240
Leu Gln Cys Lys Ala Leu Glu Leu Cys Phe Asn Val Ala Leu Asn Arg
65                  70                  75                  80
tta cca gct tcc act tca agc cct ctt ctg gct cct caa tat tcc acc        288
Leu Pro Ala Ser Thr Ser Ser Pro Leu Leu Ala Pro Gln Tyr Ser Thr
                85                  90                  95
cct tca ctc tcc aat gcc ttg gtt gca gcc ttc aaa cgt gct cag gct        336
Pro Ser Leu Ser Asn Ala Leu Val Ala Ala Phe Lys Arg Ala Gln Ala
            100                 105                 110
cac caa cgc cgt ggt tct att gag aac cag cag cag cat att ctt gcc        384
His Gln Arg Arg Gly Ser Ile Glu Asn Gln Gln Gln His Ile Leu Ala
        115                 120                 125
ttg aag atc gag gtg gag cag ctc gtg att tca atc ctt gat gac cct        432
Leu Lys Ile Glu Val Glu Gln Leu Val Ile Ser Ile Leu Asp Asp Pro
    130                 135                 140
agt gtt agt agg gtc atg aga gaa gct ggt ttc tct agc acc ctt gtg        480
Ser Val Ser Arg Val Met Arg Glu Ala Gly Phe Ser Ser Thr Leu Val
145                 150                 155                 160
aaa aca agg gtt gaa caa gct gtt tca atg gaa gtt tgt tca cag aag        528
Lys Thr Arg Val Glu Gln Ala Val Ser Met Glu Val Cys Ser Gln Lys
                165                 170                 175
gct tct tct gat agg agc cat gcc aaa gaa aat atc act aag ccc cat        576
Ala Ser Ser Asp Arg Ser His Ala Lys Glu Asn Ile Thr Lys Pro His
            180                 185                 190
cat gtt gtt ctt ggt gac cat gtt aac aat gat gat gta act agt gtg        624
His Val Val Leu Gly Asp His Val Asn Asn Asp Asp Val Thr Ser Val
        195                 200                 205
ctg agt gag ttg gtg agg aga aaa aac aca gtt att gta ggg gag ggt        672
Leu Ser Glu Leu Val Arg Arg Lys Asn Thr Val Ile Val Gly Glu Gly
    210                 215                 220
gtg gcc aat gct gag gga gta gct agg gaa gtg atg gag agg ttt gag        720
Val Ala Asn Ala Glu Gly Val Ala Arg Glu Val Met Glu Arg Phe Glu
225                 230                 235                 240
gta ggg aat gtt cct ggg gac tta agg tat gtg caa ttt gtg agc ctt        768
Val Gly Asn Val Pro Gly Asp Leu Arg Tyr Val Gln Phe Val Ser Leu
                245                 250                 255
cct ctc atg tgc ttc aga aac att agc aag gag gaa gtt gaa cag aag        816
Pro Leu Met Cys Phe Arg Asn Ile Ser Lys Glu Glu Val Glu Gln Lys
            260                 265                 270
ctt atg gag att aga aac ctt gtg aag agc tat gtg gga aga ggg gtt        864
Leu Met Glu Ile Arg Asn Leu Val Lys Ser Tyr Val Gly Arg Gly Val
        275                 280                 285
gtt ctt tac ttg ggt gat cta aaa tgg ttg ttt gag ttc tgg gct aat        912
Val Leu Tyr Leu Gly Asp Leu Lys Trp Leu Phe Glu Phe Trp Ala Asn
```

```
        290                 295                 300
    ttt tgt gag caa aaa aga aac tac tac tgt tct ata gag caa atg gtc       960
    Phe Cys Glu Gln Lys Arg Asn Tyr Tyr Cys Ser Ile Glu Gln Met Val
    305                 310                 315                 320
    atg gag ctt aaa aaa ttg gta tgt ggt agt ggg gag tct agt aga ctg      1008
    Met Glu Leu Lys Lys Leu Val Cys Gly Ser Gly Glu Ser Ser Arg Leu
                    325                 330                 335
    tgg ctt atg ggg att gca act ttt aag gca tac atg aag tgt aaa ata      1056
    Trp Leu Met Gly Ile Ala Thr Phe Lys Ala Tyr Met Lys Cys Lys Ile
                340                 345                 350
    tgt cac cca tcc cta gaa gct att tgg gag ctt cac ccc ttc aca att      1104
    Cys His Pro Ser Leu Glu Ala Ile Trp Glu Leu His Pro Phe Thr Ile
            355                 360                 365
    cct gtt ggc agc ctc agc cta agt tta aat ttt cac agt gat ttt caa      1152
    Pro Val Gly Ser Leu Ser Leu Ser Leu Asn Phe His Ser Asp Phe Gln
        370                 375                 380
    gct caa gag aga agc aag gtg ttt ttc aag gat gtg gct ttt gaa gat      1200
    Ala Gln Glu Arg Ser Lys Val Phe Phe Lys Asp Val Ala Phe Glu Asp
    385                 390                 395                 400
    aga aca gga gtc aga aac cat cta act tgc tgc agg gat tgc ttg ata      1248
    Arg Thr Gly Val Arg Asn His Leu Thr Cys Cys Arg Asp Cys Leu Ile
                    405                 410                 415
    aat ttt gaa aaa gaa gct cag agc ata act aac tgt ata agt aag aaa      1296
    Asn Phe Glu Lys Glu Ala Gln Ser Ile Thr Asn Cys Ile Ser Lys Lys
                420                 425                 430
    gtt tgc act gct agt agc ttg cca acg tgg ctt caa aat tgt aag gaa      1344
    Val Cys Thr Ala Ser Ser Leu Pro Thr Trp Leu Gln Asn Cys Lys Glu
            435                 440                 445
    gag aga agt gac ata atg gaa gat cag gaa agt tca agg ctt gag tat      1392
    Glu Arg Ser Asp Ile Met Glu Asp Gln Glu Ser Ser Arg Leu Glu Tyr
        450                 455                 460
    ctg tgc aag aaa tgg aat tcc tta tgc aat tca ata cac aga cga cac      1440
    Leu Cys Lys Lys Trp Asn Ser Leu Cys Asn Ser Ile His Arg Arg His
    465                 470                 475                 480
    cct tcc att att gag aaa cca gct gtt ttc ttt gtt tca tca tcc cct      1488
    Pro Ser Ile Ile Glu Lys Pro Ala Val Phe Phe Val Ser Ser Ser Pro
                    485                 490                 495
    tca tct ccc act tct gtt tcc tca aat gaa aga aag tcc aac ttt cat      1536
    Ser Ser Pro Thr Ser Val Ser Ser Asn Glu Arg Lys Ser Asn Phe His
                500                 505                 510
    cac agc cac cta aat tgg cca atc att tct gaa tca gaa aag tca cca      1584
    His Ser His Leu Asn Trp Pro Ile Ile Ser Glu Ser Glu Lys Ser Pro
            515                 520                 525
    aaa gaa tgt gag tta tac act gaa act ggt gat gat gat gat gat ggc      1632
    Lys Glu Cys Glu Leu Tyr Thr Glu Thr Gly Asp Asp Asp Asp Asp Gly
        530                 535                 540
    tat gat agc aac ttt ata atg ttc atg cca gat aga gat gtc cct aaa      1680
    Tyr Asp Ser Asn Phe Ile Met Phe Met Pro Asp Arg Asp Val Pro Lys
    545                 550                 555                 560
    cca gat ctt ctg tca aat cca aac tct agc ccc aac tcg gct tct tca      1728
    Pro Asp Leu Leu Ser Asn Pro Asn Ser Ser Pro Asn Ser Ala Ser Ser
                    565                 570                 575
    agt gaa gca gtg gat ggt ttg gaa agc act caa atg ttc aag gaa cct      1776
    Ser Glu Ala Val Asp Gly Leu Glu Ser Thr Gln Met Phe Lys Glu Pro
                580                 585                 590
    aat gct gag aat cat aag att ctg tgt gat gct ttg gag aaa aag att      1824
    Asn Ala Glu Asn His Lys Ile Leu Cys Asp Ala Leu Glu Lys Lys Ile
            595                 600                 605
    cca cag cat aaa gat gtt att gtt cca gag att gca agt act gtc ctt      1872
    Pro Gln His Lys Asp Val Ile Val Pro Glu Ile Ala Ser Thr Val Leu
        610                 615                 620
    cat tgt aga tca gga atg aga aaa aga gga tta aac cac ttg atg aac      1920
    His Cys Arg Ser Gly Met Arg Lys Arg Gly Leu Asn His Leu Met Asn
    625                 630                 635                 640
    aga gaa gaa aat caa gaa act tgg atg ttc ttt ctt ggt gtg aat tcc      1968
```

```
Arg Glu Glu Asn Gln Glu Thr Trp Met Phe Phe Leu Gly Val Asn Ser
                645                 650                 655
caa gcc aaa gag agc atc tca agg gag cta gct aaa gtt gtt ttt ggc      2016
Gln Ala Lys Glu Ser Ile Ser Arg Glu Leu Ala Lys Val Val Phe Gly
            660                 665                 670
tct tat agc aac ttt gtc tca att ggc atg agc aat ttc tct tcc cca      2064
Ser Tyr Ser Asn Phe Val Ser Ile Gly Met Ser Asn Phe Ser Ser Pro
        675                 680                 685
gaa gat gat cat gat tcc act gat gaa aaa tcc aaa agg aag agg cca      2112
Glu Asp Asp His Asp Ser Thr Asp Glu Lys Ser Lys Arg Lys Arg Pro
    690                 695                 700
aga gaa gag ttg aaa agc agt tat gtt caa aga ttc ggg gaa gca gtg      2160
Arg Glu Glu Leu Lys Ser Ser Tyr Val Gln Arg Phe Gly Glu Ala Val
705                 710                 715                 720
aat gag aac cct cat agg gtg ttc ttc ttg gaa gac ttg gat caa gtt      2208
Asn Glu Asn Pro His Arg Val Phe Phe Leu Glu Asp Leu Asp Gln Val
                725                 730                 735
gat tac ttc tct caa aag ggt gta aag caa gca att caa agt gga agc      2256
Asp Tyr Phe Ser Gln Lys Gly Val Lys Gln Ala Ile Gln Ser Gly Ser
            740                 745                 750
ata aca ctc cct agt ggt gaa tct gtt cct ctc aag gat gcc att gtc      2304
Ile Thr Leu Pro Ser Gly Glu Ser Val Pro Leu Lys Asp Ala Ile Val
        755                 760                 765
att ttc agt tgc gaa agc ttc tct tca cca aag ttg aga aaa tca cca      2352
Ile Phe Ser Cys Glu Ser Phe Ser Ser Pro Lys Leu Arg Lys Ser Pro
    770                 775                 780
tgt gct gaa aac aaa ggg aaa gag atc act gtg gat gat gaa agt tct      2400
Cys Ala Glu Asn Lys Gly Lys Glu Ile Thr Val Asp Asp Glu Ser Ser
785                 790                 795                 800
agc ctc tct ttg gat ctg aac cta gcc att gaa gat gaa agt gga ggt      2448
Ser Leu Ser Leu Asp Leu Asn Leu Ala Ile Glu Asp Glu Ser Gly Gly
                805                 810                 815
gtg gca tta ggt gga gac aat ggc atc cta gag tta gtt gac aaa caa      2496
Val Ala Leu Gly Gly Asp Asn Gly Ile Leu Glu Leu Val Asp Lys Gln
            820                 825                 830
att aat ttc aac ata caa gaa ttg tga                                  2523
Ile Asn Phe Asn Ile Gln Glu Leu
        835                 840

<210> 20
<211> 840
<212> PRT
<213> Glycine max

<400> 20
Met Arg Gly Gly Ile Cys Ser Ile Gln Leu Gln Ala Leu Thr Pro Glu
1               5                   10                  15
Ala Thr Thr Val Val Lys Gln Ala Val Asn Leu Ala Thr Arg Arg Gly
            20                  25                  30
His Ala Gln Val Thr Pro Leu His Val Ala Ser Ala Met Leu Ala Thr
        35                  40                  45
Ser Thr Gly Leu Leu Arg Lys Ala Cys Leu Gln Cys His Ser His Pro
    50                  55                  60
Leu Gln Cys Lys Ala Leu Glu Leu Cys Phe Asn Val Ala Leu Asn Arg
65                  70                  75                  80
Leu Pro Ala Ser Thr Ser Ser Pro Leu Leu Ala Pro Gln Tyr Ser Thr
                85                  90                  95
Pro Ser Leu Ser Asn Ala Leu Val Ala Ala Phe Lys Arg Ala Gln Ala
            100                 105                 110
His Gln Arg Arg Gly Ser Ile Glu Asn Gln Gln Gln His Ile Leu Ala
        115                 120                 125
Leu Lys Ile Glu Val Glu Gln Leu Val Ile Ser Ile Leu Asp Asp Pro
    130                 135                 140
Ser Val Ser Arg Val Met Arg Glu Ala Gly Phe Ser Ser Thr Leu Val
145                 150                 155                 160
```

```
Lys Thr Arg Val Glu Gln Ala Val Ser Met Glu Val Cys Ser Gln Lys
                165                 170                 175
Ala Ser Ser Asp Arg Ser His Ala Lys Glu Asn Ile Thr Lys Pro His
            180                 185                 190
His Val Val Leu Gly Asp His Val Asn Asn Asp Asp Val Thr Ser Val
        195                 200                 205
Leu Ser Glu Leu Val Arg Arg Lys Asn Thr Val Ile Val Gly Glu Gly
    210                 215                 220
Val Ala Asn Ala Glu Gly Val Ala Arg Glu Val Met Glu Arg Phe Glu
225                 230                 235                 240
Val Gly Asn Val Pro Gly Asp Leu Arg Tyr Val Gln Phe Val Ser Leu
                245                 250                 255
Pro Leu Met Cys Phe Arg Asn Ile Ser Lys Glu Glu Val Glu Gln Lys
            260                 265                 270
Leu Met Glu Ile Arg Asn Leu Val Lys Ser Tyr Val Gly Arg Gly Val
        275                 280                 285
Val Leu Tyr Leu Gly Asp Leu Lys Trp Leu Phe Glu Phe Trp Ala Asn
    290                 295                 300
Phe Cys Glu Gln Lys Arg Asn Tyr Tyr Cys Ser Ile Glu Gln Met Val
305                 310                 315                 320
Met Glu Leu Lys Lys Leu Val Cys Gly Ser Gly Glu Ser Ser Arg Leu
                325                 330                 335
Trp Leu Met Gly Ile Ala Thr Phe Lys Ala Tyr Met Lys Cys Lys Ile
            340                 345                 350
Cys His Pro Ser Leu Glu Ala Ile Trp Glu Leu His Pro Phe Thr Ile
        355                 360                 365
Pro Val Gly Ser Leu Ser Leu Ser Leu Asn Phe His Ser Asp Phe Gln
    370                 375                 380
Ala Gln Glu Arg Ser Lys Val Phe Phe Lys Asp Val Ala Phe Glu Asp
385                 390                 395                 400
Arg Thr Gly Val Arg Asn His Leu Thr Cys Cys Arg Asp Cys Leu Ile
                405                 410                 415
Asn Phe Glu Lys Glu Ala Gln Ser Ile Thr Asn Cys Ile Ser Lys Lys
            420                 425                 430
Val Cys Thr Ala Ser Ser Leu Pro Thr Trp Leu Gln Asn Cys Lys Glu
        435                 440                 445
Glu Arg Ser Asp Ile Met Glu Asp Gln Glu Ser Ser Arg Leu Glu Tyr
    450                 455                 460
Leu Cys Lys Lys Trp Asn Ser Leu Cys Asn Ser Ile His Arg Arg His
465                 470                 475                 480
Pro Ser Ile Ile Glu Lys Pro Ala Val Phe Phe Val Ser Ser Ser Pro
                485                 490                 495
Ser Ser Pro Thr Ser Val Ser Ser Asn Glu Arg Lys Ser Asn Phe His
            500                 505                 510
His Ser His Leu Asn Trp Pro Ile Ile Ser Glu Ser Glu Lys Ser Pro
        515                 520                 525
Lys Glu Cys Glu Leu Tyr Thr Glu Thr Gly Asp Asp Asp Asp Asp Gly
    530                 535                 540
Tyr Asp Ser Asn Phe Ile Met Phe Met Pro Asp Arg Asp Val Pro Lys
545                 550                 555                 560
Pro Asp Leu Leu Ser Asn Pro Asn Ser Ser Pro Asn Ser Ala Ser Ser
                565                 570                 575
Ser Glu Ala Val Asp Gly Leu Glu Ser Thr Gln Met Phe Lys Glu Pro
            580                 585                 590
Asn Ala Glu Asn His Lys Ile Leu Cys Asp Ala Leu Glu Lys Lys Ile
        595                 600                 605
Pro Gln His Lys Asp Val Ile Val Pro Glu Ile Ala Ser Thr Val Leu
    610                 615                 620
His Cys Arg Ser Gly Met Arg Lys Arg Gly Leu Asn His Leu Met Asn
625                 630                 635                 640
Arg Glu Glu Asn Gln Glu Thr Trp Met Phe Phe Leu Gly Val Asn Ser
                645                 650                 655
Gln Ala Lys Glu Ser Ile Ser Arg Glu Leu Ala Lys Val Val Phe Gly
            660                 665                 670
Ser Tyr Ser Asn Phe Val Ser Ile Gly Met Ser Asn Phe Ser Ser Pro
```

```
        675                 680                 685
Glu Asp Asp His Asp Ser Thr Asp Glu Lys Ser Lys Arg Lys Arg Pro
    690                 695                 700
Arg Glu Glu Leu Lys Ser Ser Tyr Val Gln Arg Phe Gly Glu Ala Val
705                 710                 715                 720
Asn Glu Asn Pro His Arg Val Phe Phe Leu Glu Asp Leu Asp Gln Val
                725                 730                 735
Asp Tyr Phe Ser Gln Lys Gly Val Lys Gln Ala Ile Gln Ser Gly Ser
            740                 745                 750
Ile Thr Leu Pro Ser Gly Glu Ser Val Pro Leu Lys Asp Ala Ile Val
        755                 760                 765
Ile Phe Ser Cys Glu Ser Phe Ser Ser Pro Lys Leu Arg Lys Ser Pro
    770                 775                 780
Cys Ala Glu Asn Lys Gly Lys Glu Ile Thr Val Asp Asp Glu Ser Ser
785                 790                 795                 800
Ser Leu Ser Leu Asp Leu Asn Leu Ala Ile Glu Asp Glu Ser Gly Gly
                805                 810                 815
Val Ala Leu Gly Gly Asp Asn Gly Ile Leu Glu Leu Val Asp Lys Gln
            820                 825                 830
Ile Asn Phe Asn Ile Gln Glu Leu
        835                 840

<210> 21
<211> 2487
<212> DNA
<213> Glycine max

<220>
<221> CDS
<222> (1)..(2487)

<400> 21
atg aga gga ggt att tgt agc ata cag ctt cag gct ctt aca cct gaa        48
Met Arg Gly Gly Ile Cys Ser Ile Gln Leu Gln Ala Leu Thr Pro Glu
1               5                   10                  15
gct gca act gtt gtt aag caa gct gtt aat ctt gca aca aga aga ggc        96
Ala Ala Thr Val Val Lys Gln Ala Val Asn Leu Ala Thr Arg Arg Gly
            20                  25                  30
cat gca caa gtg aca cct ctt cat gtt gca agt gcc atg ctt gca act       144
His Ala Gln Val Thr Pro Leu His Val Ala Ser Ala Met Leu Ala Thr
        35                  40                  45
tcc aca ggc ctt ctc cgc aag gct tgt ctt cag tgt cac tct cac ccc       192
Ser Thr Gly Leu Leu Arg Lys Ala Cys Leu Gln Cys His Ser His Pro
    50                  55                  60
ctc cag tgc aag gct ctt gag ctt tgt ttc aac gtt gca ctc aac cgt       240
Leu Gln Cys Lys Ala Leu Glu Leu Cys Phe Asn Val Ala Leu Asn Arg
65                  70                  75                  80
cta cca gct tcc act tca agc cct ctt ctg gca cct caa tat tcc acc       288
Leu Pro Ala Ser Thr Ser Ser Pro Leu Leu Ala Pro Gln Tyr Ser Thr
                85                  90                  95
cct tca ctc tcc aat gcc ttg gtt gca gcc ttc aaa cgt gct cag gct       336
Pro Ser Leu Ser Asn Ala Leu Val Ala Ala Phe Lys Arg Ala Gln Ala
            100                 105                 110
cac caa cgc cgt ggt tct att gag aac cag cag cag cat att ctg gcc       384
His Gln Arg Arg Gly Ser Ile Glu Asn Gln Gln Gln His Ile Leu Ala
        115                 120                 125
ttg aag att gag gtg gag cag ctc gtg att tca att ctt gat gat cct       432
Leu Lys Ile Glu Val Glu Gln Leu Val Ile Ser Ile Leu Asp Asp Pro
    130                 135                 140
agt gtt agc agg gtc atg aga gaa gct ggt ttc tct agc acc ctt gtg       480
Ser Val Ser Arg Val Met Arg Glu Ala Gly Phe Ser Ser Thr Leu Val
145                 150                 155                 160
aaa aca agg gtc gaa caa gct gtt tca atg gaa gtt tgt tca caa aag       528
Lys Thr Arg Val Glu Gln Ala Val Ser Met Glu Val Cys Ser Gln Lys
                165                 170                 175
```

```
gct caa gca aaa gaa aat atc act aag cct cat cat caa cct aat ttg      576
Ala Gln Ala Lys Glu Asn Ile Thr Lys Pro His His Gln Pro Asn Leu
            180                 185                 190
gac cat gtt aac aat gat gat gtg act agt gtg ttg agt gag ttg gca      624
Asp His Val Asn Asn Asp Asp Val Thr Ser Val Leu Ser Glu Leu Ala
        195                 200                 205
aag aga aga aac aca gtt att gta ggg gag agt gtg acc aat gct gag      672
Lys Arg Arg Asn Thr Val Ile Val Gly Glu Ser Val Thr Asn Ala Glu
    210                 215                 220
ggt gta gtt agg gga gtg ata gag agg ttt gag gtt ggg aat gtt cct      720
Gly Val Val Arg Gly Val Ile Glu Arg Phe Glu Val Gly Asn Val Pro
225                 230                 235                 240
gga gac ttg agg tat gtg caa ttt gtg agc ctt cct ctc atg tgc ttc      768
Gly Asp Leu Arg Tyr Val Gln Phe Val Ser Leu Pro Leu Met Cys Phe
                245                 250                 255
aga aac att agc aag gag gag gtt gaa cag aag ctt atg gag gtt aga      816
Arg Asn Ile Ser Lys Glu Glu Val Glu Gln Lys Leu Met Glu Val Arg
            260                 265                 270
aac ctt gtg aag agc tat gtg gga gga ggg gtt gtt ctt tac ttg ggt      864
Asn Leu Val Lys Ser Tyr Val Gly Gly Gly Val Val Leu Tyr Leu Gly
        275                 280                 285
gat cta aaa tgg ctg ttt gag ttc tgg gct aat ttt cgt gag caa aaa      912
Asp Leu Lys Trp Leu Phe Glu Phe Trp Ala Asn Phe Arg Glu Gln Lys
    290                 295                 300
aca aac tac tgt tct gta gag cac atg gtc atg gag ctt aaa aaa ttg      960
Thr Asn Tyr Cys Ser Val Glu His Met Val Met Glu Leu Lys Lys Leu
305                 310                 315                 320
gta tgt ggt agt ggg gag tct agt aga ttg tgg ctt atg ggg att tca      1008
Val Cys Gly Ser Gly Glu Ser Ser Arg Leu Trp Leu Met Gly Ile Ser
                325                 330                 335
act ttt aag aca tac atg aag tgt aaa ata tgt cac cca tcc cta gaa      1056
Thr Phe Lys Thr Tyr Met Lys Cys Lys Ile Cys His Pro Ser Leu Glu
            340                 345                 350
acc att tgg gag ctt cat ccc ttc aca att cct gtt ggc atc ctc agc      1104
Thr Ile Trp Glu Leu His Pro Phe Thr Ile Pro Val Gly Ile Leu Ser
        355                 360                 365
cta agt tta aat ctt gac agt gat ttt caa gct caa gag aga aac aag      1152
Leu Ser Leu Asn Leu Asp Ser Asp Phe Gln Ala Gln Glu Arg Asn Lys
    370                 375                 380
gta ttt ttc aag gat gtg gct ttt gaa gat aga gca gga gtc aga aac      1200
Val Phe Phe Lys Asp Val Ala Phe Glu Asp Arg Ala Gly Val Arg Asn
385                 390                 395                 400
cat cta act tgc tgc agg gat tgc aca ata aat ttt gaa aaa gaa gct      1248
His Leu Thr Cys Cys Arg Asp Cys Thr Ile Asn Phe Glu Lys Glu Ala
                405                 410                 415
cag agc ata act agc act ata agt aag aaa gct tgc act act agt agc      1296
Gln Ser Ile Thr Ser Thr Ile Ser Lys Lys Ala Cys Thr Thr Ser Ser
            420                 425                 430
ttg cca acg tgg ctc caa aat tgt aag gaa gag aga agt gac ata atg      1344
Leu Pro Thr Trp Leu Gln Asn Cys Lys Glu Glu Arg Ser Asp Ile Met
        435                 440                 445
gaa gat cag gaa aat gca agg ctt aag gat ctg tgc aag aaa tgg aac      1392
Glu Asp Gln Glu Asn Ala Arg Leu Lys Asp Leu Cys Lys Lys Trp Asn
    450                 455                 460
tca tta tgc aac tca ata cac aga cac cct tcc att aat gag aaa caa      1440
Ser Leu Cys Asn Ser Ile His Arg His Pro Ser Ile Asn Glu Lys Gln
465                 470                 475                 480
gtt ttc ttt gtt tca tca tct cct tca tct ccc act tct gtt tcc tca      1488
Val Phe Phe Val Ser Ser Ser Pro Ser Ser Pro Thr Ser Val Ser Ser
                485                 490                 495
cat gaa aga aag tcc aac ttt cac cac agc cac cta aat tgg cca atc      1536
His Glu Arg Lys Ser Asn Phe His His Ser His Leu Asn Trp Pro Ile
            500                 505                 510
att tct gaa tca gaa aag tca cca aaa gaa tgt gag tta tac act gaa      1584
Ile Ser Glu Ser Glu Lys Ser Pro Lys Glu Cys Glu Leu Tyr Thr Glu
```

```
        515                 520                 525
act ggt gat gat ggc tat gat agc aac ttt ata atg ttc atg cca gat      1632
Thr Gly Asp Asp Gly Tyr Asp Ser Asn Phe Ile Met Phe Met Pro Asp
    530                 535                 540
agt gat gtc cct aaa cca gat ctt ctg tca aat ccc aac tct agc ccc      1680
Ser Asp Val Pro Lys Pro Asp Leu Leu Ser Asn Pro Asn Ser Ser Pro
545                 550                 555                 560
aac tca gct tct tca agc gaa gca gtg gat ggt ttg gaa agc act caa      1728
Asn Ser Ala Ser Ser Ser Glu Ala Val Asp Gly Leu Glu Ser Thr Gln
                565                 570                 575
atg ttc aaa gaa cct aat gct gag aat cat aag att ctg tgt gat gcc      1776
Met Phe Lys Glu Pro Asn Ala Glu Asn His Lys Ile Leu Cys Asp Ala
            580                 585                 590
ttg gag aaa aag gtt cca cag cat aaa gaa gtt att cca gag att gca      1824
Leu Glu Lys Lys Val Pro Gln His Lys Glu Val Ile Pro Glu Ile Ala
        595                 600                 605
agt acc gtc ctt cat tgt aga tca gga atg aga aag aga gat cag aac      1872
Ser Thr Val Leu His Cys Arg Ser Gly Met Arg Lys Arg Asp Gln Asn
    610                 615                 620
cac tcg atg aag aga gaa gac aat caa gaa aca tgg atg ttc ttt ctt      1920
His Ser Met Lys Arg Glu Asp Asn Gln Glu Thr Trp Met Phe Phe Leu
625                 630                 635                 640
ggt gtg aat tct caa gcc aaa gag agc atc tca aga gag cta gct aaa      1968
Gly Val Asn Ser Gln Ala Lys Glu Ser Ile Ser Arg Glu Leu Ala Lys
                645                 650                 655
gtt gtt ttt ggc tct tat agc aac ttt gtc aca att ggc atg agt agt      2016
Val Val Phe Gly Ser Tyr Ser Asn Phe Val Thr Ile Gly Met Ser Ser
            660                 665                 670
ttc tct tcc cca gaa gat gat gat gat tcc act gat gaa aaa tcc aaa      2064
Phe Ser Ser Pro Glu Asp Asp Asp Asp Ser Thr Asp Glu Lys Ser Lys
        675                 680                 685
agg aag agg cca aga gaa gag ttg aaa agc agt tat gct caa aga ttc      2112
Arg Lys Arg Pro Arg Glu Glu Leu Lys Ser Ser Tyr Ala Gln Arg Phe
    690                 695                 700
ggg gaa gca gtg aat gag aac cct cac agg gtg ttc ttc tta gaa gac      2160
Gly Glu Ala Val Asn Glu Asn Pro His Arg Val Phe Phe Leu Glu Asp
705                 710                 715                 720
ttg gat caa gtt gat tac ttt tct caa aag ggt gta gag caa gca att      2208
Leu Asp Gln Val Asp Tyr Phe Ser Gln Lys Gly Val Glu Gln Ala Ile
                725                 730                 735
caa agt gga agc ata aca ctt cct ggt ggt gaa tct gtt cct ctc atg      2256
Gln Ser Gly Ser Ile Thr Leu Pro Gly Gly Glu Ser Val Pro Leu Met
            740                 745                 750
gat gcc att gtc att ttc agt tgc gaa agc ttc ttc tct tca cca aag      2304
Asp Ala Ile Val Ile Phe Ser Cys Glu Ser Phe Phe Ser Ser Pro Lys
        755                 760                 765
ctg aga aaa tca cca tgt gct gaa aac aaa ggg aaa gag act gtg gaa      2352
Leu Arg Lys Ser Pro Cys Ala Glu Asn Lys Gly Lys Glu Thr Val Glu
    770                 775                 780
gat gaa agt tct agc ctc tct ttg gat ctg aac ata gcc att gaa gat      2400
Asp Glu Ser Ser Ser Leu Ser Leu Asp Leu Asn Ile Ala Ile Glu Asp
785                 790                 795                 800
gaa agt gga ggt gtg gca ttc ggt gga gac aat ggt atc cta gag tta      2448
Glu Ser Gly Gly Val Ala Phe Gly Gly Asp Asn Gly Ile Leu Glu Leu
                805                 810                 815
gtt gat aaa caa att aat ttc aac ata caa gaa tcg tga                  2487
Val Asp Lys Gln Ile Asn Phe Asn Ile Gln Glu Ser
            820                 825
```

<210> 22
<211> 828
<212> PRT
<213> Glycine max

<400> 22

Met Arg Gly Gly Ile Cys Ser Ile Gln Leu Gln Ala Leu Thr Pro Glu
1               5                   10                  15
Ala Ala Thr Val Val Lys Gln Ala Val Asn Leu Ala Thr Arg Arg Gly
            20                  25                  30
His Ala Gln Val Thr Pro Leu His Val Ala Ser Ala Met Leu Ala Thr
        35                  40                  45
Ser Thr Gly Leu Leu Arg Lys Ala Cys Leu Gln Cys His Ser His Pro
    50                  55                  60
Leu Gln Cys Lys Ala Leu Glu Leu Cys Phe Asn Val Ala Leu Asn Arg
65                  70                  75                  80
Leu Pro Ala Ser Thr Ser Ser Pro Leu Leu Ala Pro Gln Tyr Ser Thr
                85                  90                  95
Pro Ser Leu Ser Asn Ala Leu Val Ala Ala Phe Lys Arg Ala Gln Ala
            100                 105                 110
His Gln Arg Arg Gly Ser Ile Glu Asn Gln Gln Gln His Ile Leu Ala
        115                 120                 125
Leu Lys Ile Glu Val Glu Gln Leu Val Ile Ser Ile Leu Asp Asp Pro
    130                 135                 140
Ser Val Ser Arg Val Met Arg Glu Ala Gly Phe Ser Ser Thr Leu Val
145                 150                 155                 160
Lys Thr Arg Val Glu Gln Ala Val Ser Met Glu Val Cys Ser Gln Lys
                165                 170                 175
Ala Gln Ala Lys Glu Asn Ile Thr Lys Pro His His Gln Pro Asn Leu
            180                 185                 190
Asp His Val Asn Asn Asp Asp Val Thr Ser Val Leu Ser Glu Leu Ala
        195                 200                 205
Lys Arg Arg Asn Thr Val Ile Val Gly Glu Ser Val Thr Asn Ala Glu
    210                 215                 220
Gly Val Val Arg Gly Val Ile Glu Arg Phe Glu Val Gly Asn Val Pro
225                 230                 235                 240
Gly Asp Leu Arg Tyr Val Gln Phe Val Ser Leu Pro Leu Met Cys Phe
                245                 250                 255
Arg Asn Ile Ser Lys Glu Glu Val Glu Gln Lys Leu Met Glu Val Arg
            260                 265                 270
Asn Leu Val Lys Ser Tyr Val Gly Gly Gly Val Val Leu Tyr Leu Gly
        275                 280                 285
Asp Leu Lys Trp Leu Phe Glu Phe Trp Ala Asn Phe Arg Glu Gln Lys
    290                 295                 300
Thr Asn Tyr Cys Ser Val Glu His Met Val Met Glu Leu Lys Lys Leu
305                 310                 315                 320
Val Cys Gly Ser Gly Glu Ser Ser Arg Leu Trp Leu Met Gly Ile Ser
                325                 330                 335
Thr Phe Lys Thr Tyr Met Lys Cys Lys Ile Cys His Pro Ser Leu Glu
            340                 345                 350
Thr Ile Trp Glu Leu His Pro Phe Thr Ile Pro Val Gly Ile Leu Ser
        355                 360                 365
Leu Ser Leu Asn Leu Asp Ser Asp Phe Gln Ala Gln Glu Arg Asn Lys
    370                 375                 380
Val Phe Phe Lys Asp Val Ala Phe Glu Asp Arg Ala Gly Val Arg Asn
385                 390                 395                 400
His Leu Thr Cys Cys Arg Asp Cys Thr Ile Asn Phe Glu Lys Glu Ala
                405                 410                 415
Gln Ser Ile Thr Ser Thr Ile Ser Lys Lys Ala Cys Thr Thr Ser Ser
            420                 425                 430
Leu Pro Thr Trp Leu Gln Asn Cys Lys Glu Glu Arg Ser Asp Ile Met
        435                 440                 445
Glu Asp Gln Glu Asn Ala Arg Leu Lys Asp Leu Cys Lys Lys Trp Asn
    450                 455                 460
Ser Leu Cys Asn Ser Ile His Arg His Pro Ser Ile Asn Glu Lys Gln
465                 470                 475                 480
Val Phe Phe Val Ser Ser Ser Pro Ser Ser Pro Thr Ser Val Ser Ser
                485                 490                 495
His Glu Arg Lys Ser Asn Phe His His Ser His Leu Asn Trp Pro Ile
            500                 505                 510
Ile Ser Glu Ser Glu Lys Ser Pro Lys Glu Cys Glu Leu Tyr Thr Glu

```
        515                 520                 525
Thr Gly Asp Asp Gly Tyr Asp Ser Asn Phe Ile Met Phe Met Pro Asp
    530                 535                 540
Ser Asp Val Pro Lys Pro Asp Leu Leu Ser Asn Pro Asn Ser Ser Pro
545                 550                 555                 560
Asn Ser Ala Ser Ser Ser Glu Ala Val Asp Gly Leu Glu Ser Thr Gln
                565                 570                 575
Met Phe Lys Glu Pro Asn Ala Glu Asn His Lys Ile Leu Cys Asp Ala
            580                 585                 590
Leu Glu Lys Lys Val Pro Gln His Lys Glu Val Ile Pro Glu Ile Ala
        595                 600                 605
Ser Thr Val Leu His Cys Arg Ser Gly Met Arg Lys Arg Asp Gln Asn
    610                 615                 620
His Ser Met Lys Arg Glu Asp Asn Gln Glu Thr Trp Met Phe Phe Leu
625                 630                 635                 640
Gly Val Asn Ser Gln Ala Lys Glu Ser Ile Ser Arg Glu Leu Ala Lys
                645                 650                 655
Val Val Phe Gly Ser Tyr Ser Asn Phe Val Thr Ile Gly Met Ser Ser
            660                 665                 670
Phe Ser Ser Pro Glu Asp Asp Asp Asp Ser Thr Asp Glu Lys Ser Lys
        675                 680                 685
Arg Lys Arg Pro Arg Glu Glu Leu Lys Ser Ser Tyr Ala Gln Arg Phe
    690                 695                 700
Gly Glu Ala Val Asn Glu Asn Pro His Arg Val Phe Phe Leu Glu Asp
705                 710                 715                 720
Leu Asp Gln Val Asp Tyr Phe Ser Gln Lys Gly Val Glu Gln Ala Ile
                725                 730                 735
Gln Ser Gly Ser Ile Thr Leu Pro Gly Gly Glu Ser Val Pro Leu Met
            740                 745                 750
Asp Ala Ile Val Ile Phe Ser Cys Glu Ser Phe Phe Ser Ser Pro Lys
        755                 760                 765
Leu Arg Lys Ser Pro Cys Ala Glu Asn Lys Gly Lys Glu Thr Val Glu
    770                 775                 780
Asp Glu Ser Ser Ser Leu Ser Leu Asp Leu Asn Ile Ala Ile Glu Asp
785                 790                 795                 800
Glu Ser Gly Gly Val Ala Phe Gly Gly Asp Asn Gly Ile Leu Glu Leu
                805                 810                 815
Val Asp Lys Gln Ile Asn Phe Asn Ile Gln Glu Ser
            820                 825
```

<210> 23
<211> 2550
<212> DNA
<213> Medicago truncatula

<220>
<221> CDS
<222> (1)..(2550)

<400> 23

```
atg aga gga gga att tgt agt ata cag ctt caa gca ctt aca caa gaa       48
Met Arg Gly Gly Ile Cys Ser Ile Gln Leu Gln Ala Leu Thr Gln Glu
 1               5                  10                  15
gct gca act gtt gtt aaa caa gca gtg aat cta gca aca aga aga ggt       96
Ala Ala Thr Val Val Lys Gln Ala Val Asn Leu Ala Thr Arg Arg Gly
            20                  25                  30
cat gca caa gtg aca cct ctt cat gtt gct agt gct atg ctt gca act      144
His Ala Gln Val Thr Pro Leu His Val Ala Ser Ala Met Leu Ala Thr
        35                  40                  45
tca aca gga att tta aga aaa gct tgt ctt caa tgt cac tct cac cct      192
Ser Thr Gly Ile Leu Arg Lys Ala Cys Leu Gln Cys His Ser His Pro
    50                  55                  60
ctt caa tgc aaa gct ctt gag ctt tgt ttc aat gtt gca ctc aac cgt      240
Leu Gln Cys Lys Ala Leu Glu Leu Cys Phe Asn Val Ala Leu Asn Arg
65                  70                  75                  80
```

```
tta cct gca tca aca caa agt cca cta tta ggt cct caa tat tca acc        288
Leu Pro Ala Ser Thr Gln Ser Pro Leu Leu Gly Pro Gln Tyr Ser Thr
                85                  90                  95
act cct tca ctt tct aat gct ttg gtt gca gct ttc aaa cgt gct cag        336
Thr Pro Ser Leu Ser Asn Ala Leu Val Ala Ala Phe Lys Arg Ala Gln
            100                 105                 110
gct cac caa cga cgt gga acg atc gag aat cag caa cag cag cat att        384
Ala His Gln Arg Arg Gly Thr Ile Glu Asn Gln Gln Gln Gln His Ile
        115                 120                 125
ctt gct ttg aaa att gaa gtt gaa cag ctc ata atc tct ata ctt gat        432
Leu Ala Leu Lys Ile Glu Val Glu Gln Leu Ile Ile Ser Ile Leu Asp
    130                 135                 140
gat cca agt gtt agt aga gtt atg aga gaa gct ggt ttc tct agc act        480
Asp Pro Ser Val Ser Arg Val Met Arg Glu Ala Gly Phe Ser Ser Thr
145                 150                 155                 160
ctt gtt aaa tca aga gtt gaa gtt gaa caa gct ctt cca att gaa gtt        528
Leu Val Lys Ser Arg Val Glu Val Glu Gln Ala Leu Pro Ile Glu Val
                165                 170                 175
tct tca aca aaa gtt tct tct gaa tat cat aaa aac cag tcc aaa gaa        576
Ser Ser Thr Lys Val Ser Ser Glu Tyr His Lys Asn Gln Ser Lys Glu
            180                 185                 190
ctt agc ctt aag cct caa gtt ctt agc ctt ggt ggt tca tac acc aaa        624
Leu Ser Leu Lys Pro Gln Val Leu Ser Leu Gly Gly Ser Tyr Thr Lys
        195                 200                 205
cct ata gat tgt gtt aac aat gat gat gta aca agt gtc ttg agt gaa        672
Pro Ile Asp Cys Val Asn Asn Asp Asp Val Thr Ser Val Leu Ser Glu
    210                 215                 220
tta gta aag agg aga aga aac act gtt att gta gga gag agt gtt tct        720
Leu Val Lys Arg Arg Arg Asn Thr Val Ile Val Gly Glu Ser Val Ser
225                 230                 235                 240
aat gct gag gga gta gct aag gga gtg atg gaa agg ttt gag ata ggt        768
Asn Ala Glu Gly Val Ala Lys Gly Val Met Glu Arg Phe Glu Ile Gly
                245                 250                 255
gat gtt cct atg gag tta agg tat gta caa ttt gtg agt ctt cct cta        816
Asp Val Pro Met Glu Leu Arg Tyr Val Gln Phe Val Ser Leu Pro Leu
            260                 265                 270
ata tgt ttt agg aat att agt aaa gag gaa gtt gaa aag aaa ttt gtg        864
Ile Cys Phe Arg Asn Ile Ser Lys Glu Glu Val Glu Lys Lys Phe Val
        275                 280                 285
gaa gtt aga agc ctt gtg aaa agc tat atg gga aga ggg gtt att ctt        912
Glu Val Arg Ser Leu Val Lys Ser Tyr Met Gly Arg Gly Val Ile Leu
    290                 295                 300
tat tta ggt gat tta aaa tgg ttg ttt gag ttt tgg tca agt tac tgt        960
Tyr Leu Gly Asp Leu Lys Trp Leu Phe Glu Phe Trp Ser Ser Tyr Cys
305                 310                 315                 320
gaa caa aaa agg aac tac tat tgt tct gtt gaa cat atg gtg atg gag        1008
Glu Gln Lys Arg Asn Tyr Tyr Cys Ser Val Glu His Met Val Met Glu
                325                 330                 335
att aaa aaa ttg gtt agt gga agt ggt gag agt agt aga tta tgg ctt        1056
Ile Lys Lys Leu Val Ser Gly Ser Gly Glu Ser Ser Arg Leu Trp Leu
            340                 345                 350
atg ggg att gca aat ttt aaa aca tac atg aag tgt aaa ata tct cac        1104
Met Gly Ile Ala Asn Phe Lys Thr Tyr Met Lys Cys Lys Ile Ser His
        355                 360                 365
cct tcc cta gaa act att tgg gag ctt cat cct ttt aca att cct gtt        1152
Pro Ser Leu Glu Thr Ile Trp Glu Leu His Pro Phe Thr Ile Pro Val
    370                 375                 380
ggc agc cta agc cta agt ttg aac ttt gac agt gat ttt caa gct aag        1200
Gly Ser Leu Ser Leu Ser Leu Asn Phe Asp Ser Asp Phe Gln Ala Lys
385                 390                 395                 400
gag aga agt atg gta tta ttc aat gac ttg act ttt gaa gat aaa gta        1248
Glu Arg Ser Met Val Leu Phe Asn Asp Leu Thr Phe Glu Asp Lys Val
                405                 410                 415
ggt gtt gga aag caa cta act tgc tgc aga gat tgt tca ata aaa ttt        1296
Gly Val Gly Lys Gln Leu Thr Cys Cys Arg Asp Cys Ser Ile Lys Phe
```

```
            420                 425                 430
gaa aat gaa gct ctg agt ttg act aac aat ata agc aag aaa gca tgc        1344
Glu Asn Glu Ala Leu Ser Leu Thr Asn Asn Ile Ser Lys Lys Ala Cys
        435                 440                 445
agt tca agc ttg cca aca tgg ctt caa aat tgc aag gaa gaa aga agt        1392
Ser Ser Ser Leu Pro Thr Trp Leu Gln Asn Cys Lys Glu Glu Arg Ser
    450                 455                 460
tac aca gtg gaa gat cag gaa aat gca aga ctt aag gat ctc tgc aag        1440
Tyr Thr Val Glu Asp Gln Glu Asn Ala Arg Leu Lys Asp Leu Cys Lys
465                 470                 475                 480
aaa tgg aac tca ata tgc aat tca ata cac aga caa cct tcc att ctt        1488
Lys Trp Asn Ser Ile Cys Asn Ser Ile His Arg Gln Pro Ser Ile Leu
                485                 490                 495
gac aaa caa gat ttg ttt gtt tta tca tct tcc cct tca tca cca act        1536
Asp Lys Gln Asp Leu Phe Val Leu Ser Ser Ser Pro Ser Ser Pro Thr
            500                 505                 510
tca ttt tcc tca ctt gaa aaa aag tct aac ttc caa cat agc caa cta        1584
Ser Phe Ser Ser Leu Glu Lys Lys Ser Asn Phe Gln His Ser Gln Leu
        515                 520                 525
aat tgg cct ata att tct gaa caa gaa aaa gta cca aaa gaa tgt gag        1632
Asn Trp Pro Ile Ile Ser Glu Gln Glu Lys Val Pro Lys Glu Cys Glu
    530                 535                 540
tta tta tac act gaa agt gct ggt ggt gat gat gat ggt tgc tat gat        1680
Leu Leu Tyr Thr Glu Ser Ala Gly Gly Asp Asp Asp Gly Cys Tyr Asp
545                 550                 555                 560
ggt aac ttg atc atg ttc atg cca cag aga aat gtt cca aaa cca gat        1728
Gly Asn Leu Ile Met Phe Met Pro Gln Arg Asn Val Pro Lys Pro Asp
                565                 570                 575
ctt ttg tca aat cct aac tca agt cca aac tca gct tct tca agt gaa        1776
Leu Leu Ser Asn Pro Asn Ser Ser Pro Asn Ser Ala Ser Ser Ser Glu
            580                 585                 590
gca gtg gat ggt tta gaa agt act gaa ttg ttc aat gaa cat aat gaa        1824
Ala Val Asp Gly Leu Glu Ser Thr Glu Leu Phe Asn Glu His Asn Glu
        595                 600                 605
gag aat ctc aag att ctc tgt gat gcc ttg gag aac aag ttt cca caa        1872
Glu Asn Leu Lys Ile Leu Cys Asp Ala Leu Glu Asn Lys Phe Pro Gln
    610                 615                 620
cat aaa gaa atc att caa gag att gca agt act gtc ctt ttt tgt aga        1920
His Lys Glu Ile Ile Gln Glu Ile Ala Ser Thr Val Leu Phe Cys Arg
625                 630                 635                 640
tca gga atg aga aaa aga ggg aac aac ttc ttc aaa aga gaa aat cat        1968
Ser Gly Met Arg Lys Arg Gly Asn Asn Phe Phe Lys Arg Glu Asn His
                645                 650                 655
aag caa gaa aca tgg atg ttt ttt ctt ggt gat gat tct caa gcc aga        2016
Lys Gln Glu Thr Trp Met Phe Phe Leu Gly Asp Asp Ser Gln Ala Arg
            660                 665                 670
gag aat atc tca aag gag cta gct aaa gtt gtt ttt gga tct tgt aac        2064
Glu Asn Ile Ser Lys Glu Leu Ala Lys Val Val Phe Gly Ser Cys Asn
        675                 680                 685
aac ttt atg aca att ggc atg agc act ttc tct tcc cta gga aat gat        2112
Asn Phe Met Thr Ile Gly Met Ser Thr Phe Ser Ser Leu Gly Asn Asp
    690                 695                 700
gat tcc tca agt gat gaa aaa tcc aaa agg aaa aga cca aga gct gag        2160
Asp Ser Ser Ser Asp Glu Lys Ser Lys Arg Lys Arg Pro Arg Ala Glu
705                 710                 715                 720
ttg gga agc act tat ttg cag aga ttt tgt gag gca gtg aat gag aat        2208
Leu Gly Ser Thr Tyr Leu Gln Arg Phe Cys Glu Ala Val Asn Glu Asn
                725                 730                 735
cct cat aga gtg ttc ttc atg gaa gat ttg gag gag gaa gtt gat cac        2256
Pro His Arg Val Phe Phe Met Glu Asp Leu Glu Glu Glu Val Asp His
            740                 745                 750
ttt act caa aag ggt atc aag aaa gca att gaa tgt gga agt ata aca        2304
Phe Thr Gln Lys Gly Ile Lys Lys Ala Ile Glu Cys Gly Ser Ile Thr
        755                 760                 765
att cct ggt ggt gaa tct gtt cct ctc aag gat gcc att gtc att ttc        2352
```

```
Ile Pro Gly Gly Glu Ser Val Pro Leu Lys Asp Ala Ile Val Ile Phe
    770                 775                 780
agt agt gaa agc ttc agt tca gtg tca aag tca tca caa tca tca tgt      2400
Ser Ser Glu Ser Phe Ser Ser Val Ser Lys Ser Ser Gln Ser Ser Cys
785                 790                 795                 800
gct gaa aac aaa ggg aaa gag acc atg ata gag gat cat caa agt aat      2448
Ala Glu Asn Lys Gly Lys Glu Thr Met Ile Glu Asp His Gln Ser Asn
                805                 810                 815
cta aac ctc tct ttg gat cta aat atc gcc att gaa gat cat gat aat      2496
Leu Asn Leu Ser Leu Asp Leu Asn Ile Ala Ile Glu Asp His Asp Asn
            820                 825                 830
gct gac att ggt att ctt gag tta gtt gac aag aaa ttt agt ttt aat      2544
Ala Asp Ile Gly Ile Leu Glu Leu Val Asp Lys Lys Phe Ser Phe Asn
        835                 840                 845
ttg taa                                                              2550
Leu


<210> 24
<211> 849
<212> PRT
<213> Medicago truncatula

<400> 24
Met Arg Gly Gly Ile Cys Ser Ile Gln Leu Gln Ala Leu Thr Gln Glu
1               5                   10                  15
Ala Ala Thr Val Val Lys Gln Ala Val Asn Leu Ala Thr Arg Arg Gly
            20                  25                  30
His Ala Gln Val Thr Pro Leu His Val Ala Ser Ala Met Leu Ala Thr
        35                  40                  45
Ser Thr Gly Ile Leu Arg Lys Ala Cys Leu Gln Cys His Ser His Pro
    50                  55                  60
Leu Gln Cys Lys Ala Leu Glu Leu Cys Phe Asn Val Ala Leu Asn Arg
65                  70                  75                  80
Leu Pro Ala Ser Thr Gln Ser Pro Leu Leu Gly Pro Gln Tyr Ser Thr
                85                  90                  95
Thr Pro Ser Leu Ser Asn Ala Leu Val Ala Ala Phe Lys Arg Ala Gln
            100                 105                 110
Ala His Gln Arg Arg Gly Thr Ile Glu Asn Gln Gln Gln Gln His Ile
        115                 120                 125
Leu Ala Leu Lys Ile Glu Val Glu Gln Leu Ile Ile Ser Ile Leu Asp
    130                 135                 140
Asp Pro Ser Val Ser Arg Val Met Arg Glu Ala Gly Phe Ser Ser Thr
145                 150                 155                 160
Leu Val Lys Ser Arg Val Glu Val Glu Gln Ala Leu Pro Ile Glu Val
                165                 170                 175
Ser Ser Thr Lys Val Ser Ser Glu Tyr His Lys Asn Gln Ser Lys Glu
            180                 185                 190
Leu Ser Leu Lys Pro Gln Val Leu Ser Leu Gly Gly Ser Tyr Thr Lys
        195                 200                 205
Pro Ile Asp Cys Val Asn Asn Asp Asp Val Thr Ser Val Leu Ser Glu
    210                 215                 220
Leu Val Lys Arg Arg Arg Asn Thr Val Ile Val Gly Glu Ser Val Ser
225                 230                 235                 240
Asn Ala Glu Gly Val Ala Lys Gly Val Met Glu Arg Phe Glu Ile Gly
                245                 250                 255
Asp Val Pro Met Glu Leu Arg Tyr Val Gln Phe Val Ser Leu Pro Leu
            260                 265                 270
Ile Cys Phe Arg Asn Ile Ser Lys Glu Glu Val Glu Lys Lys Phe Val
        275                 280                 285
Glu Val Arg Ser Leu Val Lys Ser Tyr Met Gly Arg Gly Val Ile Leu
    290                 295                 300
Tyr Leu Gly Asp Leu Lys Trp Leu Phe Glu Phe Trp Ser Ser Tyr Cys
305                 310                 315                 320
Glu Gln Lys Arg Asn Tyr Tyr Cys Ser Val Glu His Met Val Met Glu
```

```
                325                 330                 335
Ile Lys Lys Leu Val Ser Gly Ser Gly Glu Ser Ser Arg Leu Trp Leu
            340                 345                 350
Met Gly Ile Ala Asn Phe Lys Thr Tyr Met Lys Cys Lys Ile Ser His
        355                 360                 365
Pro Ser Leu Glu Thr Ile Trp Glu Leu His Pro Phe Thr Ile Pro Val
    370                 375                 380
Gly Ser Leu Ser Leu Ser Leu Asn Phe Asp Ser Asp Phe Gln Ala Lys
385                 390                 395                 400
Glu Arg Ser Met Val Leu Phe Asn Asp Leu Thr Phe Glu Asp Lys Val
                405                 410                 415
Gly Val Gly Lys Gln Leu Thr Cys Cys Arg Asp Cys Ser Ile Lys Phe
            420                 425                 430
Glu Asn Glu Ala Leu Ser Leu Thr Asn Asn Ile Ser Lys Lys Ala Cys
        435                 440                 445
Ser Ser Ser Leu Pro Thr Trp Leu Gln Asn Cys Lys Glu Glu Arg Ser
    450                 455                 460
Tyr Thr Val Glu Asp Gln Glu Asn Ala Arg Leu Lys Asp Leu Cys Lys
465                 470                 475                 480
Lys Trp Asn Ser Ile Cys Asn Ser Ile His Arg Gln Pro Ser Ile Leu
                485                 490                 495
Asp Lys Gln Asp Leu Phe Val Leu Ser Ser Ser Pro Ser Ser Pro Thr
            500                 505                 510
Ser Phe Ser Ser Leu Glu Lys Lys Ser Asn Phe Gln His Ser Gln Leu
        515                 520                 525
Asn Trp Pro Ile Ile Ser Glu Gln Glu Lys Val Pro Lys Glu Cys Glu
    530                 535                 540
Leu Leu Tyr Thr Glu Ser Ala Gly Gly Asp Asp Asp Gly Cys Tyr Asp
545                 550                 555                 560
Gly Asn Leu Ile Met Phe Met Pro Gln Arg Asn Val Pro Lys Pro Asp
                565                 570                 575
Leu Leu Ser Asn Pro Asn Ser Ser Pro Asn Ser Ala Ser Ser Ser Glu
            580                 585                 590
Ala Val Asp Gly Leu Glu Ser Thr Glu Leu Phe Asn Glu His Asn Glu
        595                 600                 605
Glu Asn Leu Lys Ile Leu Cys Asp Ala Leu Glu Asn Lys Phe Pro Gln
    610                 615                 620
His Lys Glu Ile Ile Gln Glu Ile Ala Ser Thr Val Leu Phe Cys Arg
625                 630                 635                 640
Ser Gly Met Arg Lys Arg Gly Asn Asn Phe Phe Lys Arg Glu Asn His
                645                 650                 655
Lys Gln Glu Thr Trp Met Phe Phe Leu Gly Asp Asp Ser Gln Ala Arg
            660                 665                 670
Glu Asn Ile Ser Lys Glu Leu Ala Lys Val Val Phe Gly Ser Cys Asn
        675                 680                 685
Asn Phe Met Thr Ile Gly Met Ser Thr Phe Ser Ser Leu Gly Asn Asp
    690                 695                 700
Asp Ser Ser Ser Asp Glu Lys Ser Lys Arg Lys Arg Pro Arg Ala Glu
705                 710                 715                 720
Leu Gly Ser Thr Tyr Leu Gln Arg Phe Cys Glu Ala Val Asn Glu Asn
                725                 730                 735
Pro His Arg Val Phe Phe Met Glu Asp Leu Glu Glu Glu Val Asp His
            740                 745                 750
Phe Thr Gln Lys Gly Ile Lys Lys Ala Ile Glu Cys Gly Ser Ile Thr
        755                 760                 765
Ile Pro Gly Gly Glu Ser Val Pro Leu Lys Asp Ala Ile Val Ile Phe
    770                 775                 780
Ser Ser Glu Ser Phe Ser Ser Val Ser Lys Ser Ser Gln Ser Ser Cys
785                 790                 795                 800
Ala Glu Asn Lys Gly Lys Glu Thr Met Ile Glu Asp His Gln Ser Asn
                805                 810                 815
Leu Asn Leu Ser Leu Asp Leu Asn Ile Ala Ile Glu Asp His Asp Asn
            820                 825                 830
Ala Asp Ile Gly Ile Leu Glu Leu Val Asp Lys Lys Phe Ser Phe Asn
        835                 840                 845
```

Leu

<210> 25
<211> 2634
<212> DNA
<213> Glycine max

<220>
<221> CDS
<222> (1)..(2634)

<400> 25
```
atg agg gcc ggg ggg tgc acg gtg cag cag gcg ctg acg gcg gag gcg       48
Met Arg Ala Gly Gly Cys Thr Val Gln Gln Ala Leu Thr Ala Glu Ala
 1               5                  10                  15
gcc gcc gtg gtg aag cag gcg gtg acc ctt gcg cgg cgg agg ggc aac       96
Ala Ala Val Val Lys Gln Ala Val Thr Leu Ala Arg Arg Arg Gly Asn
            20                  25                  30
gcg cag gtg acg ccg ctg cac gtg gcg agc gcg atg ctg gcg ccg ccg      144
Ala Gln Val Thr Pro Leu His Val Ala Ser Ala Met Leu Ala Pro Pro
        35                  40                  45
ggg ggg ctc ctc cgc gcg gcg tgc ctc cgg tcg cac tcc cac ccg ctg      192
Gly Gly Leu Leu Arg Ala Ala Cys Leu Arg Ser His Ser His Pro Leu
    50                  55                  60
cag tgc aag gcc ctg gag ctc tgc ttc aac gtg gcg ctc aac cgc ctc      240
Gln Cys Lys Ala Leu Glu Leu Cys Phe Asn Val Ala Leu Asn Arg Leu
65                  70                  75                  80
ccg gcc tcc gcc gcc gtc gcc tcg tcg ccg ctg ctc ggc ggc cac ggg      288
Pro Ala Ser Ala Ala Val Ala Ser Ser Pro Leu Leu Gly Gly His Gly
                85                  90                  95
cac ggc cac cac ggc cac tac tac ccg ccg tcg ctc tcc aac gcg ctc      336
His Gly His His Gly His Tyr Tyr Pro Pro Ser Leu Ser Asn Ala Leu
            100                 105                 110
gtc gcg gcg ttc aag cgg gcg cag gcg cac cag cgg cgc ggg tcc gtg      384
Val Ala Ala Phe Lys Arg Ala Gln Ala His Gln Arg Arg Gly Ser Val
        115                 120                 125
gag acc cag cag cag ccg gtg ctc gcc gtg aag atc gag ctg gag cag      432
Glu Thr Gln Gln Gln Pro Val Leu Ala Val Lys Ile Glu Leu Glu Gln
    130                 135                 140
ctc gtc gtc tcc atc ctc gac gac ccc agc gtc agc cgc gtc atg cgg      480
Leu Val Val Ser Ile Leu Asp Asp Pro Ser Val Ser Arg Val Met Arg
145                 150                 155                 160
gag gcc ggc ttc tcc agc acc cag gtc aag gcc aac gtg gag cag gcg      528
Glu Ala Gly Phe Ser Ser Thr Gln Val Lys Ala Asn Val Glu Gln Ala
                165                 170                 175
tgc agc acc acc acg gcc acc agc gcg cca ccg aac caa aac cct aac      576
Cys Ser Thr Thr Thr Ala Thr Ser Ala Pro Pro Asn Gln Asn Pro Asn
            180                 185                 190
cct agc tgc acc ggc gcc gca gcc acc gcc acc gcc acc acc agc ccg      624
Pro Ser Cys Thr Gly Ala Ala Ala Thr Ala Thr Ala Thr Thr Ser Pro
        195                 200                 205
gct cat cct ccg gag atc aaa gcc aaa ctg cca ctg ctc gac atg ctg      672
Ala His Pro Pro Glu Ile Lys Ala Lys Leu Pro Leu Leu Asp Met Leu
    210                 215                 220
gcg cgc gac gag gac atc gcc gcg gtg ctc gac tgc ctt gcc ccg gca      720
Ala Arg Asp Glu Asp Ile Ala Ala Val Leu Asp Cys Leu Ala Pro Ala
225                 230                 235                 240
gcg gct ggc ggc cgg ggc ggc ggc ggc agc agg agg agg gtc gtc gtc      768
Ala Ala Gly Gly Arg Gly Gly Gly Gly Ser Arg Arg Arg Val Val Val
                245                 250                 255
gtc gcc gag agc acg gcc gcc gcg gag gcc acg gcg cgc gcc gcc gtg      816
Val Ala Glu Ser Thr Ala Ala Ala Glu Ala Thr Ala Arg Ala Ala Val
            260                 265                 270
gac agg gtc agg aga ggg gag gcg aag cag cac gac gcg ctg cgt ggc      864
```

```
Asp Arg Val Arg Arg Gly Glu Ala Lys Gln His Asp Ala Leu Arg Gly
        275                 280                 285

gcg cag gtg gtc agc ctc cgc gtg tcg tcg ttc cgc gac atg ccg agg      912
Ala Gln Val Val Ser Leu Arg Val Ser Ser Phe Arg Asp Met Pro Arg
    290                 295                 300

gag gag gcg gag cgg cgg ctc gcc gag ctc cgg tgc ctc gtg aag agc      960
Glu Glu Ala Glu Arg Arg Leu Ala Glu Leu Arg Cys Leu Val Lys Ser
305                 310                 315                 320

cgc ggc gcg cgg gtg ctc ctc gtc gtc gag gac ctc aag tgg gcg gcc     1008
Arg Gly Ala Arg Val Leu Leu Val Val Glu Asp Leu Lys Trp Ala Ala
                325                 330                 335

gac ttc tgg gcc gcc gcg cac gcc ggc gcc agg agg gtc ggc agc ggc     1056
Asp Phe Trp Ala Ala Ala His Ala Gly Ala Arg Arg Val Gly Ser Gly
            340                 345                 350

ggc ggc ggc ggc tac tac tgc tct gtc gag cac gtc gtc acc gag gtg     1104
Gly Gly Gly Gly Tyr Tyr Cys Ser Val Glu His Val Val Thr Glu Val
        355                 360                 365

cgc gcc ctg gcg tcg tgc gac ggc ggc atc tgg ctc gtc ggg ttc ggg     1152
Arg Ala Leu Ala Ser Cys Asp Gly Gly Ile Trp Leu Val Gly Phe Gly
    370                 375                 380

acg tac cag acg tac atg aag tgc agg gcg ggg cat ccg tcg ctg gag     1200
Thr Tyr Gln Thr Tyr Met Lys Cys Arg Ala Gly His Pro Ser Leu Glu
385                 390                 395                 400

agc atg tgg ggg ctc cag acg ctc gcc gtc ccc gcc ggc agc ctc gcg     1248
Ser Met Trp Gly Leu Gln Thr Leu Ala Val Pro Ala Gly Ser Leu Ala
                405                 410                 415

ctg agc ctc acc tgc gcc ttc gac gac agc gcg ctg ggc gca gtc aat     1296
Leu Ser Leu Thr Cys Ala Phe Asp Asp Ser Ala Leu Gly Ala Val Asn
            420                 425                 430

cag tcc atg aaa gcg agc cct cac acc acc gat ggg aac cgc ccg gcg     1344
Gln Ser Met Lys Ala Ser Pro His Thr Thr Asp Gly Asn Arg Pro Ala
        435                 440                 445

ccg tct tgc ggg ccg ctt ttg ggc ggc agc cac ctc ctc tcc aga tgc     1392
Pro Ser Cys Gly Pro Leu Leu Gly Gly Ser His Leu Leu Ser Arg Cys
    450                 455                 460

tgc ggc ggc gac tgc tcc gcc gcg acg acg acg cac gag cac gac acc     1440
Cys Gly Gly Asp Cys Ser Ala Ala Thr Thr Thr His Glu His Asp Thr
465                 470                 475                 480

aag gcg tcg ttg ccc cgc tcc ttc gtg tcg tcg tcg agc ctc cct tct     1488
Lys Ala Ser Leu Pro Arg Ser Phe Val Ser Ser Ser Ser Leu Pro Ser
                485                 490                 495

tgg ctc cag cat tgc cgc gac cag cag ctg cag gag tcg aca cat ttc     1536
Trp Leu Gln His Cys Arg Asp Gln Gln Leu Gln Glu Ser Thr His Phe
            500                 505                 510

gcg gat ctt ggc aag aca tgg gga tcc atc tgc ggc aag ccg tct cag     1584
Ala Asp Leu Gly Lys Thr Trp Gly Ser Ile Cys Gly Lys Pro Ser Gln
        515                 520                 525

cgg atg acg ctg cat ttc tcg gcg ccg gtg tcg ccg gcg tcc tcc atc     1632
Arg Met Thr Leu His Phe Ser Ala Pro Val Ser Pro Ala Ser Ser Ile
    530                 535                 540

tcc tcc tac gag cac ggc cac ggc cac cag cag cag cag cac cag cct     1680
Ser Ser Tyr Glu His Gly His Gly His Gln Gln Gln Gln His Gln Pro
545                 550                 555                 560

cac cac tcg tgg ctt ctc gcc gac ctc gac gcc aag cac cca tgg aag     1728
His His Ser Trp Leu Leu Ala Asp Leu Asp Ala Lys His Pro Trp Lys
                565                 570                 575

ccc aag cgc gag gac gac gac gac gag aag gcc aag tcg cac gac gac     1776
Pro Lys Arg Glu Asp Asp Asp Asp Glu Lys Ala Lys Ser His Asp Asp
            580                 585                 590

tgc tcc ggc gcc tcc aat ggc tcg gtg gag gtg gag tgc cgt tcc agg     1824
Cys Ser Gly Ala Ser Asn Gly Ser Val Glu Val Glu Cys Arg Ser Arg
        595                 600                 605

ttc aag gag ctc aac gcc gag aac ctg aag ctc ctg tgc gcc gcg ctg     1872
Phe Lys Glu Leu Asn Ala Glu Asn Leu Lys Leu Leu Cys Ala Ala Leu
    610                 615                 620
```

```
gag aag gag gtg ccg tgg cag aag gag atc gtc ccg gag gtg gcg agc       1920
Glu Lys Glu Val Pro Trp Gln Lys Glu Ile Val Pro Glu Val Ala Ser
625                 630                 635                 640
gcc gtc ctc cag tgc agg tcc ggg atc gcc aag cgg cgg gac agg tcg       1968
Ala Val Leu Gln Cys Arg Ser Gly Ile Ala Lys Arg Arg Asp Arg Ser
                645                 650                 655
agg tcg acg gag gcg aag gag gag acg tgg ctc ttc ttc ctc gga ggc       2016
Arg Ser Thr Glu Ala Lys Glu Glu Thr Trp Leu Phe Phe Leu Gly Gly
            660                 665                 670
gac ggg cac ggc aag gag agg gtg gcg agg gag ctc gcc ggc ctc gtc       2064
Asp Gly His Gly Lys Glu Arg Val Ala Arg Glu Leu Ala Gly Leu Val
        675                 680                 685
ttc ggg tca cgc aag agc ttc ctc tcc gtc aag ctc ggc gcc tcc tcc       2112
Phe Gly Ser Arg Lys Ser Phe Leu Ser Val Lys Leu Gly Ala Ser Ser
    690                 695                 700
tcc tcg ccg tcc gcg tcg ggc tcc acc gag gat cac cac cgg agc aag       2160
Ser Ser Pro Ser Ala Ser Gly Ser Thr Glu Asp His His Arg Ser Lys
705                 710                 715                 720
cgg ccg cgg acg acg acg acg tcg tcg gcg agc gag gcc tac ctc gag       2208
Arg Pro Arg Thr Thr Thr Thr Ser Ser Ala Ser Glu Ala Tyr Leu Glu
                725                 730                 735
cgg ctc tac gac gcc gtc tcg gag aac ccg cac cgc gtc atc ctc atc       2256
Arg Leu Tyr Asp Ala Val Ser Glu Asn Pro His Arg Val Ile Leu Ile
            740                 745                 750
gag gac gtc gag cag ggc gac cat cgc tgg cag gtg ggc gtc aag gag       2304
Glu Asp Val Glu Gln Gly Asp His Arg Trp Gln Val Gly Val Lys Glu
        755                 760                 765
gcc atc gac aga ggg gtt ctc cgg agc cag gcc ggc gac gag gtc ggc       2352
Ala Ile Asp Arg Gly Val Leu Arg Ser Gln Ala Gly Asp Glu Val Gly
    770                 775                 780
gtg ggc gac gcc atc atc atc ctg agc tgc gag agc ttc gag gcg agg       2400
Val Gly Asp Ala Ile Ile Ile Leu Ser Cys Glu Ser Phe Glu Ala Arg
785                 790                 795                 800
tct aga gct ggc tcg ccg ctg atg aac aag aag atg aag gtg gag aaa       2448
Ser Arg Ala Gly Ser Pro Leu Met Asn Lys Lys Met Lys Val Glu Lys
                805                 810                 815
gag gaa gcc aac aca agt gat cat gat cac aaa tta gaa atc gaa tca       2496
Glu Glu Ala Asn Thr Ser Asp His Asp His Lys Leu Glu Ile Glu Ser
            820                 825                 830
ggc gcc ccc tct tct tgc ttc gat ttg aac ctt gac atg gag agc gat       2544
Gly Ala Pro Ser Ser Cys Phe Asp Leu Asn Leu Asp Met Glu Ser Asp
        835                 840                 845
caa gca gca gat gag ctc agc tcc ggt gat gtc tgt ctg ctc acg gcg       2592
Gln Ala Ala Asp Glu Leu Ser Ser Gly Asp Val Cys Leu Leu Thr Ala
    850                 855                 860
gtc gac cgg gtg ctg ctc ttc aga agg cag gat gaa atg taa               2634
Val Asp Arg Val Leu Leu Phe Arg Arg Gln Asp Glu Met
865                 870                 875

<210> 26
<211> 877
<212> PRT
<213> Glycine max

<400> 26
Met Arg Ala Gly Gly Cys Thr Val Gln Gln Ala Leu Thr Ala Glu Ala
1               5                   10                  15
Ala Ala Val Val Lys Gln Ala Val Thr Leu Ala Arg Arg Arg Gly Asn
            20                  25                  30
Ala Gln Val Thr Pro Leu His Val Ala Ser Ala Met Leu Ala Pro Pro
        35                  40                  45
Gly Gly Leu Leu Arg Ala Ala Cys Leu Arg Ser His Ser His Pro Leu
    50                  55                  60
Gln Cys Lys Ala Leu Glu Leu Cys Phe Asn Val Ala Leu Asn Arg Leu
65                  70                  75                  80
```

```
Pro Ala Ser Ala Ala Val Ala Ser Ser Pro Leu Leu Gly Gly His Gly
                85                  90                  95
His Gly His His Gly His Tyr Tyr Pro Pro Ser Leu Ser Asn Ala Leu
            100                 105                 110
Val Ala Ala Phe Lys Arg Ala Gln Ala His Gln Arg Arg Gly Ser Val
        115                 120                 125
Glu Thr Gln Gln Gln Pro Val Leu Ala Val Lys Ile Glu Leu Glu Gln
    130                 135                 140
Leu Val Val Ser Ile Leu Asp Asp Pro Ser Val Ser Arg Val Met Arg
145                 150                 155                 160
Glu Ala Gly Phe Ser Ser Thr Gln Val Lys Ala Asn Val Glu Gln Ala
                165                 170                 175
Cys Ser Thr Thr Thr Ala Thr Ser Ala Pro Pro Asn Gln Asn Pro Asn
            180                 185                 190
Pro Ser Cys Thr Gly Ala Ala Ala Thr Ala Thr Ala Thr Thr Ser Pro
        195                 200                 205
Ala His Pro Pro Glu Ile Lys Ala Lys Leu Pro Leu Leu Asp Met Leu
    210                 215                 220
Ala Arg Asp Glu Asp Ile Ala Ala Val Leu Asp Cys Leu Ala Pro Ala
225                 230                 235                 240
Ala Ala Gly Gly Arg Gly Gly Gly Gly Ser Arg Arg Arg Val Val Val
                245                 250                 255
Val Ala Glu Ser Thr Ala Ala Ala Glu Ala Thr Ala Arg Ala Ala Val
            260                 265                 270
Asp Arg Val Arg Arg Gly Glu Ala Lys Gln His Asp Ala Leu Arg Gly
        275                 280                 285
Ala Gln Val Val Ser Leu Arg Val Ser Ser Phe Arg Asp Met Pro Arg
    290                 295                 300
Glu Glu Ala Glu Arg Arg Leu Ala Glu Leu Arg Cys Leu Val Lys Ser
305                 310                 315                 320
Arg Gly Ala Arg Val Leu Leu Val Val Glu Asp Leu Lys Trp Ala Ala
                325                 330                 335
Asp Phe Trp Ala Ala Ala His Ala Gly Ala Arg Arg Val Gly Ser Gly
            340                 345                 350
Gly Gly Gly Gly Tyr Tyr Cys Ser Val Glu His Val Val Thr Glu Val
        355                 360                 365
Arg Ala Leu Ala Ser Cys Asp Gly Gly Ile Trp Leu Val Gly Phe Gly
    370                 375                 380
Thr Tyr Gln Thr Tyr Met Lys Cys Arg Ala Gly His Pro Ser Leu Glu
385                 390                 395                 400
Ser Met Trp Gly Leu Gln Thr Leu Ala Val Pro Ala Gly Ser Leu Ala
                405                 410                 415
Leu Ser Leu Thr Cys Ala Phe Asp Asp Ser Ala Leu Gly Ala Val Asn
            420                 425                 430
Gln Ser Met Lys Ala Ser Pro His Thr Thr Asp Gly Asn Arg Pro Ala
        435                 440                 445
Pro Ser Cys Gly Pro Leu Leu Gly Gly Ser His Leu Leu Ser Arg Cys
    450                 455                 460
Cys Gly Gly Asp Cys Ser Ala Ala Thr Thr Thr His Glu His Asp Thr
465                 470                 475                 480
Lys Ala Ser Leu Pro Arg Ser Phe Val Ser Ser Ser Ser Leu Pro Ser
                485                 490                 495
Trp Leu Gln His Cys Arg Asp Gln Gln Leu Gln Glu Ser Thr His Phe
            500                 505                 510
Ala Asp Leu Gly Lys Thr Trp Gly Ser Ile Cys Gly Lys Pro Ser Gln
        515                 520                 525
Arg Met Thr Leu His Phe Ser Ala Pro Val Ser Pro Ala Ser Ser Ile
    530                 535                 540
Ser Ser Tyr Glu His Gly His Gly His Gln Gln Gln Gln His Gln Pro
545                 550                 555                 560
His His Ser Trp Leu Leu Ala Asp Leu Asp Ala Lys His Pro Trp Lys
                565                 570                 575
Pro Lys Arg Glu Asp Asp Asp Asp Glu Lys Ala Lys Ser His Asp Asp
            580                 585                 590
Cys Ser Gly Ala Ser Asn Gly Ser Val Glu Val Glu Cys Arg Ser Arg
```

```
        595                 600                 605
Phe Lys Glu Leu Asn Ala Glu Asn Leu Lys Leu Leu Cys Ala Ala Leu
    610                 615                 620
Glu Lys Glu Val Pro Trp Gln Lys Glu Ile Val Pro Glu Val Ala Ser
625                 630                 635                 640
Ala Val Leu Gln Cys Arg Ser Gly Ile Ala Lys Arg Arg Asp Arg Ser
                645                 650                 655
Arg Ser Thr Glu Ala Lys Glu Glu Thr Trp Leu Phe Phe Leu Gly Gly
            660                 665                 670
Asp Gly His Gly Lys Glu Arg Val Ala Arg Glu Leu Ala Gly Leu Val
        675                 680                 685
Phe Gly Ser Arg Lys Ser Phe Leu Ser Val Lys Leu Gly Ala Ser Ser
    690                 695                 700
Ser Ser Pro Ser Ala Ser Gly Ser Thr Glu Asp His His Arg Ser Lys
705                 710                 715                 720
Arg Pro Arg Thr Thr Thr Thr Ser Ser Ala Ser Glu Ala Tyr Leu Glu
                725                 730                 735
Arg Leu Tyr Asp Ala Val Ser Glu Asn Pro His Arg Val Ile Leu Ile
            740                 745                 750
Glu Asp Val Glu Gln Gly Asp His Arg Trp Gln Val Gly Val Lys Glu
        755                 760                 765
Ala Ile Asp Arg Gly Val Leu Arg Ser Gln Ala Gly Asp Glu Val Gly
    770                 775                 780
Val Gly Asp Ala Ile Ile Ile Leu Ser Cys Glu Ser Phe Glu Ala Arg
785                 790                 795                 800
Ser Arg Ala Gly Ser Pro Leu Met Asn Lys Lys Met Lys Val Glu Lys
                805                 810                 815
Glu Glu Ala Asn Thr Ser Asp His Asp His Lys Leu Glu Ile Glu Ser
            820                 825                 830
Gly Ala Pro Ser Ser Cys Phe Asp Leu Asn Leu Asp Met Glu Ser Asp
        835                 840                 845
Gln Ala Ala Asp Glu Leu Ser Ser Gly Asp Val Cys Leu Leu Thr Ala
    850                 855                 860
Val Asp Arg Val Leu Leu Phe Arg Arg Gln Asp Glu Met
865                 870                 875

<210> 27
<211> 2538
<212> DNA
<213> Oryza sativa

<220>
<221> CDS
<222> (1)..(2538)

<400> 27
atg cgt gcc ggc ggc tgc gcg gtg cag cag gcg ctg gcg gcg gag gcg       48
Met Arg Ala Gly Gly Cys Ala Val Gln Gln Ala Leu Ala Ala Glu Ala
  1               5                  10                  15
gcg ggg gtg gtg agg cag gcg gtg acg ctg gcg cgg cgg cgc ggc cac       96
Ala Gly Val Val Arg Gln Ala Val Thr Leu Ala Arg Arg Arg Gly His
            20                  25                  30
gcg cag gtc acg ccg ctc cac gtc gcc agc gcc atg ctc tcc gcc gcc      144
Ala Gln Val Thr Pro Leu His Val Ala Ser Ala Met Leu Ser Ala Ala
        35                  40                  45
ggc ctc ctc cgc gcc gcc tgc ctc cag tcc cac tcc cac ccg ctc cag      192
Gly Leu Leu Arg Ala Ala Cys Leu Gln Ser His Ser His Pro Leu Gln
    50                  55                  60
tgc aag gcc ctc gag ctc tgc ttc aac gtc gcc ctc aac cgc ctc ccc      240
Cys Lys Ala Leu Glu Leu Cys Phe Asn Val Ala Leu Asn Arg Leu Pro
65                  70                  75                  80
acc gcc ggc ccc gcc gcc gcc gcg gcc atc ttc cac cac cac cca cac      288
Thr Ala Gly Pro Ala Ala Ala Ala Ala Ile Phe His His His Pro His
                85                  90                  95
cac ccc ggc ggc ggc ggc ggt cac cac ccc gcg ctg tcg aac gcg ctc      336
```

His Pro Gly Gly Gly Gly Gly His His Pro Ala Leu Ser Asn Ala Leu
100 105 110

gtc gcc gcg ttc aag cgc gcc cag gcg cac cag cgg cgt ggg tcc gtc 384
Val Ala Ala Phe Lys Arg Ala Gln Ala His Gln Arg Arg Gly Ser Val
115 120 125

gag gga cag ccg ccg ccg cag ccg ccg ccg tcg ccg gtg gtc gcg tcc 432
Glu Gly Gln Pro Pro Pro Gln Pro Pro Pro Ser Pro Val Val Ala Ser
130 135 140

aag gtc gag ctc gag cag ctc atc atc tcc atc ctc gat gac ccc agc 480
Lys Val Glu Leu Glu Gln Leu Ile Ile Ser Ile Leu Asp Asp Pro Ser
145 150 155 160

gtc agc cgc gtc atg cgc gag gcc ggc ttc tcc agc tcg cag gtc aag 528
Val Ser Arg Val Met Arg Glu Ala Gly Phe Ser Ser Ser Gln Val Lys
165 170 175

gcc aac gtc gag aag gcc gtc gtc gcg tcg ttg gac cac gcg aac gcg 576
Ala Asn Val Glu Lys Ala Val Val Ala Ser Leu Asp His Ala Asn Ala
180 185 190

gca ggc ggc ggc ggc ggc cac gcg ggc tct ccc aac tcc ggc cat gga 624
Ala Gly Gly Gly Gly Gly His Ala Gly Ser Pro Asn Ser Gly His Gly
195 200 205

ggg agg cgc aag gag agc tca tca tcc aga gca agg gtc gac gac gac 672
Gly Arg Arg Lys Glu Ser Ser Ser Ser Arg Ala Arg Val Asp Asp Asp
210 215 220

gcc atg cgc gtg ctg gac tgc atg gcg agc ggg acg aaa cgg tgc gtg 720
Ala Met Arg Val Leu Asp Cys Met Ala Ser Gly Thr Lys Arg Cys Val
225 230 235 240

gtg gtc gtc ggc ggc gag ggc gcg gcc gcg gcg gag gcg gtg gtg aag 768
Val Val Val Gly Gly Glu Gly Ala Ala Ala Ala Glu Ala Val Val Lys
245 250 255

gcg gtg atg gac agg gtg agc aag gcg gag ctg cac cac cgg cac gag 816
Ala Val Met Asp Arg Val Ser Lys Ala Glu Leu His His Arg His Glu
260 265 270

cgc ctc aag aac ctc cag ttc gtg ccc ctc tcc atc gcg tcg ttc cac 864
Arg Leu Lys Asn Leu Gln Phe Val Pro Leu Ser Ile Ala Ser Phe His
275 280 285

ggc gcg ccg agg gag gag gtg gag gcc aag gcc agc gac ctc cgc gcg 912
Gly Ala Pro Arg Glu Glu Val Glu Ala Lys Ala Ser Asp Leu Arg Ala
290 295 300

ctc gtc cgc tcc ggc tgc gcc gcc ggc aag ggc gtc gtg ctc gtc ctc 960
Leu Val Arg Ser Gly Cys Ala Ala Gly Lys Gly Val Val Leu Val Leu
305 310 315 320

gag gac ctc gcc tac gcc gcc gac gcg tgg gcc gcc gcg tcg aac acc 1008
Glu Asp Leu Ala Tyr Ala Ala Asp Ala Trp Ala Ala Ala Ser Asn Thr
325 330 335

aga cgc cgc gcc gcc gcc gcc gcc ggc ggg cag agc tac tgc ccc atg 1056
Arg Arg Arg Ala Ala Ala Ala Ala Gly Gly Gln Ser Tyr Cys Pro Met
340 345 350

gaa cac gcc gtg atg gag gtg agc agc ttg gtg tcc ggc ggc ggc ggc 1104
Glu His Ala Val Met Glu Val Ser Ser Leu Val Ser Gly Gly Gly Gly
355 360 365

ggc ggc gag agg ttc tgg gtg cta ggg ttc ggg agc tac cag gtg tac 1152
Gly Gly Glu Arg Phe Trp Val Leu Gly Phe Gly Ser Tyr Gln Val Tyr
370 375 380

atg aag tgc agg gcc gcc ggg cag ccg ccg ctc gag gcc gtc tgg gag 1200
Met Lys Cys Arg Ala Ala Gly Gln Pro Pro Leu Glu Ala Val Trp Glu
385 390 395 400

ctc cac ccc gtc gtc gtc ccc gac ggc ggc ctc gcc ttg agc ctc acc 1248
Leu His Pro Val Val Val Pro Asp Gly Gly Leu Ala Leu Ser Leu Thr
405 410 415

tgc agt gaa gct tca caa gct act cat caa gcg gcg gcg cca aca gct 1296
Cys Ser Glu Ala Ser Gln Ala Thr His Gln Ala Ala Ala Pro Thr Ala
420 425 430

ggg tgg ccc ttc gtc aat ggc gcc ggc gag gcg gcg gcg acg acg gcg 1344
Gly Trp Pro Phe Val Asn Gly Ala Gly Glu Ala Ala Ala Thr Thr Ala
435 440 445

```
agc ccg acc att ccg ccg tgg ctt cgc cgg tat caa gat cca gat cac       1392
Ser Pro Thr Ile Pro Pro Trp Leu Arg Arg Tyr Gln Asp Pro Asp His
    450                 455                 460
gcc acg ccg gcg agc tgc ggc acc ggt ttg cag ata caa gat ctg tgg       1440
Ala Thr Pro Ala Ser Cys Gly Thr Gly Leu Gln Ile Gln Asp Leu Trp
465                 470                 475                 480
aat ccc atg aga aat gga tca gca cca cac cac aca tcc gag ctt aca       1488
Asn Pro Met Arg Asn Gly Ser Ala Pro His His Thr Ser Glu Leu Thr
                485                 490                 495
ttg agt ttc tcc tct cca tca cct tct tcc atc tca gga tat acc agc       1536
Leu Ser Phe Ser Ser Pro Ser Pro Ser Ser Ile Ser Gly Tyr Thr Ser
            500                 505                 510
tgc tac aac aac aac aac atg atg agc tcc aag cca tgg caa ctc gaa       1584
Cys Tyr Asn Asn Asn Asn Met Met Ser Ser Lys Pro Trp Gln Leu Glu
        515                 520                 525
gcg agg cag cca tgg cca att cat gga cat gaa ggt cag aga atg gcc       1632
Ala Arg Gln Pro Trp Pro Ile His Gly His Glu Gly Gln Arg Met Ala
    530                 535                 540
atg gca tca tat cat gat cat cat ccg ttg gat aca aac cct agc cca       1680
Met Ala Ser Tyr His Asp His His Pro Leu Asp Thr Asn Pro Ser Pro
545                 550                 555                 560
gag tcc aac tcg gtg tcc aat tca ttg gat ggc ggt gag acg agg cgg       1728
Glu Ser Asn Ser Val Ser Asn Ser Leu Asp Gly Gly Glu Thr Arg Arg
                565                 570                 575
ccc aag ttc atc gag ctc aat gca gag aac ctc aag atc ttg tgc aat       1776
Pro Lys Phe Ile Glu Leu Asn Ala Glu Asn Leu Lys Ile Leu Cys Asn
            580                 585                 590
gcg ctc gag agc cgc gtc ccg caa cat agc aac att gtt cca gac att       1824
Ala Leu Glu Ser Arg Val Pro Gln His Ser Asn Ile Val Pro Asp Ile
        595                 600                 605
gcg agc acc gtg ctc caa tgt cga tcc ggc atg aag aag atg aag ttg       1872
Ala Ser Thr Val Leu Gln Cys Arg Ser Gly Met Lys Lys Met Lys Leu
    610                 615                 620
agg cat aag gag atc atc aag gca agc tca act act tgg tta cta ttc       1920
Arg His Lys Glu Ile Ile Lys Ala Ser Ser Thr Thr Trp Leu Leu Phe
625                 630                 635                 640
caa gga aga gat gtt gat ggt aag aag gcc atg gca caa gag ctc gca       1968
Gln Gly Arg Asp Val Asp Gly Lys Lys Ala Met Ala Gln Glu Leu Ala
                645                 650                 655
aag ctt gtc ttt ggt tca tct acc gag ttc tct tcc atc tcc ttc gat       2016
Lys Leu Val Phe Gly Ser Ser Thr Glu Phe Ser Ser Ile Ser Phe Asp
            660                 665                 670
gag ctc aca tcg ccc tac tcc gat tct agc tcc ggt gag ctc acc ctc       2064
Glu Leu Thr Ser Pro Tyr Ser Asp Ser Ser Ser Gly Glu Leu Thr Leu
        675                 680                 685
aag agg caa aga tca gca gac agc aat gag cat agc ttt gcg cag aga       2112
Lys Arg Gln Arg Ser Ala Asp Ser Asn Glu His Ser Phe Ala Gln Arg
    690                 695                 700
cta tgt gaa att gtg agc aaa aac cca cac cag gtc ata gtg atc aat       2160
Leu Cys Glu Ile Val Ser Lys Asn Pro His Gln Val Ile Val Ile Asn
705                 710                 715                 720
gac att gag caa ctt gat caa gat tca gaa att agt atc aag aaa gcg       2208
Asp Ile Glu Gln Leu Asp Gln Asp Ser Glu Ile Ser Ile Lys Lys Ala
                725                 730                 735
att gcg aac gga aga atg aga gga tgc acc ggt gaa gaa gtt gat ttc       2256
Ile Ala Asn Gly Arg Met Arg Gly Cys Thr Gly Glu Glu Val Asp Phe
            740                 745                 750
gag gat gct atc ata gtg ttg agc tat gaa gaa gaa ttc gat tcg agg       2304
Glu Asp Ala Ile Ile Val Leu Ser Tyr Glu Glu Glu Phe Asp Ser Arg
        755                 760                 765
tct agg gct tcc tcc tcc cct cgg gtc aag cag agg ctc atg aac aat       2352
Ser Arg Ala Ser Ser Ser Pro Arg Val Lys Gln Arg Leu Met Asn Asn
    770                 775                 780
aat gat gat gaa gaa agt agc agc acg gag aag gga gat aat tca cca       2400
Asn Asp Asp Glu Glu Ser Ser Ser Thr Glu Lys Gly Asp Asn Ser Pro
```

```
785                 790                 795                 800
caa cgt ttt agc ttg gat ttg aat gca tgt ctc gag gat gaa gaa gag      2448
Gln Arg Phe Ser Leu Asp Leu Asn Ala Cys Leu Glu Asp Glu Glu Glu
                805                 810                 815
gat gag ggg ttt ttg cta att gat aat ggt gtg ggg atg cat gat att      2496
Asp Glu Gly Phe Leu Leu Ile Asp Asn Gly Val Gly Met His Asp Ile
            820                 825                 830
gtg gat ggt gtt ttc ttc ttc ggg ttg atg gca gat ttc tga              2538
Val Asp Gly Val Phe Phe Phe Gly Leu Met Ala Asp Phe
        835                 840                 845
```

<210> 28
<211> 845
<212> PRT
<213> Oryza sativa

<400> 28

```
Met Arg Ala Gly Gly Cys Ala Val Gln Gln Ala Leu Ala Ala Glu Ala
1               5                   10                  15
Ala Gly Val Val Arg Gln Ala Val Thr Leu Ala Arg Arg Arg Gly His
            20                  25                  30
Ala Gln Val Thr Pro Leu His Val Ala Ser Ala Met Leu Ser Ala Ala
        35                  40                  45
Gly Leu Leu Arg Ala Ala Cys Leu Gln Ser His Ser His Pro Leu Gln
    50                  55                  60
Cys Lys Ala Leu Glu Leu Cys Phe Asn Val Ala Leu Asn Arg Leu Pro
65                  70                  75                  80
Thr Ala Gly Pro Ala Ala Ala Ala Ala Ile Phe His His His Pro His
                85                  90                  95
His Pro Gly Gly Gly Gly His His Pro Ala Leu Ser Asn Ala Leu
            100                 105                 110
Val Ala Ala Phe Lys Arg Ala Gln Ala His Gln Arg Arg Gly Ser Val
        115                 120                 125
Glu Gly Gln Pro Pro Pro Gln Pro Pro Pro Ser Pro Val Val Ala Ser
    130                 135                 140
Lys Val Glu Leu Glu Gln Leu Ile Ile Ser Ile Leu Asp Asp Pro Ser
145                 150                 155                 160
Val Ser Arg Val Met Arg Glu Ala Gly Phe Ser Ser Ser Gln Val Lys
                165                 170                 175
Ala Asn Val Glu Lys Ala Val Val Ala Ser Leu Asp His Ala Asn Ala
            180                 185                 190
Ala Gly Gly Gly Gly Gly His Ala Gly Ser Pro Asn Ser Gly His Gly
        195                 200                 205
Gly Arg Arg Lys Glu Ser Ser Ser Ser Arg Ala Arg Val Asp Asp Asp
    210                 215                 220
Ala Met Arg Val Leu Asp Cys Met Ala Ser Gly Thr Lys Arg Cys Val
225                 230                 235                 240
Val Val Val Gly Gly Glu Gly Ala Ala Ala Ala Glu Ala Val Val Lys
                245                 250                 255
Ala Val Met Asp Arg Val Ser Lys Ala Glu Leu His His Arg His Glu
            260                 265                 270
Arg Leu Lys Asn Leu Gln Phe Val Pro Leu Ser Ile Ala Ser Phe His
        275                 280                 285
Gly Ala Pro Arg Glu Glu Val Glu Ala Lys Ala Ser Asp Leu Arg Ala
    290                 295                 300
Leu Val Arg Ser Gly Cys Ala Ala Gly Lys Gly Val Val Leu Val Leu
305                 310                 315                 320
Glu Asp Leu Ala Tyr Ala Ala Asp Ala Trp Ala Ala Ala Ser Asn Thr
                325                 330                 335
Arg Arg Arg Ala Ala Ala Ala Ala Gly Gly Gln Ser Tyr Cys Pro Met
            340                 345                 350
Glu His Ala Val Met Glu Val Ser Ser Leu Val Ser Gly Gly Gly Gly
        355                 360                 365
Gly Gly Glu Arg Phe Trp Val Leu Gly Phe Gly Ser Tyr Gln Val Tyr
    370                 375                 380
```

```
Met Lys Cys Arg Ala Ala Gly Gln Pro Pro Leu Glu Ala Val Trp Glu
385                 390                 395                 400
Leu His Pro Val Val Val Pro Asp Gly Gly Leu Ala Leu Ser Leu Thr
                405                 410                 415
Cys Ser Glu Ala Ser Gln Ala Thr His Gln Ala Ala Ala Pro Thr Ala
            420                 425                 430
Gly Trp Pro Phe Val Asn Gly Ala Gly Glu Ala Ala Ala Thr Thr Ala
        435                 440                 445
Ser Pro Thr Ile Pro Pro Trp Leu Arg Arg Tyr Gln Asp Pro Asp His
    450                 455                 460
Ala Thr Pro Ala Ser Cys Gly Thr Gly Leu Gln Ile Gln Asp Leu Trp
465                 470                 475                 480
Asn Pro Met Arg Asn Gly Ser Ala Pro His His Thr Ser Glu Leu Thr
                485                 490                 495
Leu Ser Phe Ser Ser Pro Ser Pro Ser Ser Ile Ser Gly Tyr Thr Ser
            500                 505                 510
Cys Tyr Asn Asn Asn Asn Met Met Ser Ser Lys Pro Trp Gln Leu Glu
        515                 520                 525
Ala Arg Gln Pro Trp Pro Ile His Gly His Glu Gly Gln Arg Met Ala
    530                 535                 540
Met Ala Ser Tyr His Asp His His Pro Leu Asp Thr Asn Pro Ser Pro
545                 550                 555                 560
Glu Ser Asn Ser Val Ser Asn Ser Leu Asp Gly Gly Glu Thr Arg Arg
                565                 570                 575
Pro Lys Phe Ile Glu Leu Asn Ala Glu Asn Leu Lys Ile Leu Cys Asn
            580                 585                 590
Ala Leu Glu Ser Arg Val Pro Gln His Ser Asn Ile Val Pro Asp Ile
        595                 600                 605
Ala Ser Thr Val Leu Gln Cys Arg Ser Gly Met Lys Lys Met Lys Leu
    610                 615                 620
Arg His Lys Glu Ile Ile Lys Ala Ser Ser Thr Thr Trp Leu Leu Phe
625                 630                 635                 640
Gln Gly Arg Asp Val Asp Gly Lys Lys Ala Met Ala Gln Glu Leu Ala
                645                 650                 655
Lys Leu Val Phe Gly Ser Ser Thr Glu Phe Ser Ser Ile Ser Phe Asp
            660                 665                 670
Glu Leu Thr Ser Pro Tyr Ser Asp Ser Ser Ser Gly Glu Leu Thr Leu
        675                 680                 685
Lys Arg Gln Arg Ser Ala Asp Ser Asn Glu His Ser Phe Ala Gln Arg
    690                 695                 700
Leu Cys Glu Ile Val Ser Lys Asn Pro His Gln Val Ile Val Ile Asn
705                 710                 715                 720
Asp Ile Glu Gln Leu Asp Gln Asp Ser Glu Ile Ser Ile Lys Lys Ala
                725                 730                 735
Ile Ala Asn Gly Arg Met Arg Gly Cys Thr Gly Glu Glu Val Asp Phe
            740                 745                 750
Glu Asp Ala Ile Ile Val Leu Ser Tyr Glu Glu Glu Phe Asp Ser Arg
        755                 760                 765
Ser Arg Ala Ser Ser Ser Pro Arg Val Lys Gln Arg Leu Met Asn Asn
    770                 775                 780
Asn Asp Asp Glu Glu Ser Ser Ser Thr Glu Lys Gly Asp Asn Ser Pro
785                 790                 795                 800
Gln Arg Phe Ser Leu Asp Leu Asn Ala Cys Leu Glu Asp Glu Glu Glu
                805                 810                 815
Asp Glu Gly Phe Leu Leu Ile Asp Asn Gly Val Gly Met His Asp Ile
            820                 825                 830
Val Asp Gly Val Phe Phe Phe Gly Leu Met Ala Asp Phe
        835                 840                 845
```

<210> 29
<211> 2529
<212> DNA
<213> Populus trichocarpa

<220>

<221> CDS
<222> (1)..(2529)

<400> 29

```
atg aga gca gga ggt tgt aca gtg caa caa gct ctg acg gca gac gca       48
Met Arg Ala Gly Gly Cys Thr Val Gln Gln Ala Leu Thr Ala Asp Ala
1               5                   10                  15
gca agt gtt att aaa caa gca gta acc ctt gct aga agg cga ggc cat       96
Ala Ser Val Ile Lys Gln Ala Val Thr Leu Ala Arg Arg Arg Gly His
            20                  25                  30
gcc caa gtc act cct ctc cat gtt gct aac acc atg ctt tct gcc tcc      144
Ala Gln Val Thr Pro Leu His Val Ala Asn Thr Met Leu Ser Ala Ser
        35                  40                  45
act ggc cta ttt aga aca gct tgc ctt cag tca cac tct cat cct ctt      192
Thr Gly Leu Phe Arg Thr Ala Cys Leu Gln Ser His Ser His Pro Leu
    50                  55                  60
cag tgc aaa gcc cta gag ctt tgc ttc aac gtc gcg ctt aat cgg ctc      240
Gln Cys Lys Ala Leu Glu Leu Cys Phe Asn Val Ala Leu Asn Arg Leu
65                  70                  75                  80
cca gct tca act tca agc ccc ata tta ggt act cac tct cag cag ttc      288
Pro Ala Ser Thr Ser Ser Pro Ile Leu Gly Thr His Ser Gln Gln Phe
                85                  90                  95
cct tca atc tct aat gcc ttg gta gca gct ttt aag cgc gct cag gct      336
Pro Ser Ile Ser Asn Ala Leu Val Ala Ala Phe Lys Arg Ala Gln Ala
            100                 105                 110
cac caa cgg cgt ggc tct att gaa aac cag cag cag cca ctc ctt gca      384
His Gln Arg Arg Gly Ser Ile Glu Asn Gln Gln Gln Pro Leu Leu Ala
        115                 120                 125
gtg aaa ata gag tta gag cag ctc ata ata tcc att tta gac gat cct      432
Val Lys Ile Glu Leu Glu Gln Leu Ile Ile Ser Ile Leu Asp Asp Pro
    130                 135                 140
agt gtt agt aga gtc atg aga gaa gct ggt ttc tct agt act caa gtg      480
Ser Val Ser Arg Val Met Arg Glu Ala Gly Phe Ser Ser Thr Gln Val
145                 150                 155                 160
aaa agc aat gtt gag caa gct gtt tct tta gaa ata tgt tca caa agt      528
Lys Ser Asn Val Glu Gln Ala Val Ser Leu Glu Ile Cys Ser Gln Ser
                165                 170                 175
gct cct tct gtg agt agc aag tcc aag gaa agc aat ggt ttg gtc ctt      576
Ala Pro Ser Val Ser Ser Lys Ser Lys Glu Ser Asn Gly Leu Val Leu
            180                 185                 190
tca cag tct cca act tca agt caa gtt gga gca aaa gca aca gtt tta      624
Ser Gln Ser Pro Thr Ser Ser Gln Val Gly Ala Lys Ala Thr Val Leu
        195                 200                 205
gat cca att aaa aac gaa gat gtg atg tgt gtc ata gag aat tta gtg      672
Asp Pro Ile Lys Asn Glu Asp Val Met Cys Val Ile Glu Asn Leu Val
    210                 215                 220
aac aaa agg agg aga agt ttt gtt att gtt gga gag tct ctg gcc agt      720
Asn Lys Arg Arg Arg Ser Phe Val Ile Val Gly Glu Ser Leu Ala Ser
225                 230                 235                 240
ata gaa gtt gtg gtg aaa gga gtc att gat aag gtt caa aag gga gat      768
Ile Glu Val Val Val Lys Gly Val Ile Asp Lys Val Gln Lys Gly Asp
                245                 250                 255
gtt cct gag gct ttg agg gaa gtg aag ttt tta aca atc cca gtt tct      816
Val Pro Glu Ala Leu Arg Glu Val Lys Phe Leu Thr Ile Pro Val Ser
            260                 265                 270
tct ttt ggc cat ttc tct agg gta gag gta gaa cat aaa ctc gaa gag      864
Ser Phe Gly His Phe Ser Arg Val Glu Val Glu His Lys Leu Glu Glu
        275                 280                 285
ctt aaa atc cat gtg aga agc tac atg ggc aaa gga gtt gtt ttg aat      912
Leu Lys Ile His Val Arg Ser Tyr Met Gly Lys Gly Val Val Leu Asn
    290                 295                 300
ttg gga gat ctc aaa tgg gct att gag aat agg gct agt agt tca tca      960
Leu Gly Asp Leu Lys Trp Ala Ile Glu Asn Arg Ala Ser Ser Ser Ser
305                 310                 315                 320
agt gag caa gga agg tgc ttt ttc tgt cca atg gaa tac atg atc ata     1008
```

```
Ser Glu Gln Gly Arg Cys Phe Phe Cys Pro Met Glu Tyr Met Ile Ile
                325                 330                 335
gag ctt ggc aaa tta gct tgt gga att gga gag aat att aat gga aga      1056
Glu Leu Gly Lys Leu Ala Cys Gly Ile Gly Glu Asn Ile Asn Gly Arg
            340                 345                 350
ttt tgg ctt atg ggg att gcc act ttc caa act tac atg aag tgt aaa      1104
Phe Trp Leu Met Gly Ile Ala Thr Phe Gln Thr Tyr Met Lys Cys Lys
        355                 360                 365
tca ggc cat cca tca gga ggt act gtt ttg ggc ctc cat cca ctt aca      1152
Ser Gly His Pro Ser Gly Gly Thr Val Leu Gly Leu His Pro Leu Thr
    370                 375                 380
att cct gca ggc agc ttg cgc ttg agt ctc atc agt gac agt gat cta      1200
Ile Pro Ala Gly Ser Leu Arg Leu Ser Leu Ile Ser Asp Ser Asp Leu
385                 390                 395                 400
cga tgt cag tcc aca aga aac aaa gct gga aat ggg tct agc agc tgg      1248
Arg Cys Gln Ser Thr Arg Asn Lys Ala Gly Asn Gly Ser Ser Ser Trp
                405                 410                 415
att cta cat gaa ggt ggg gaa gat aaa cag ctc act tgc tgt gct gat      1296
Ile Leu His Glu Gly Gly Glu Asp Lys Gln Leu Thr Cys Cys Ala Asp
            420                 425                 430
tgt tca gcc aag ttt gag tca gaa gct cga agc tta cca act agc acc      1344
Cys Ser Ala Lys Phe Glu Ser Glu Ala Arg Ser Leu Pro Thr Ser Thr
        435                 440                 445
tgt gac agt gat tcc aca act tct ggc ctt cct gca tgg ctt caa caa      1392
Cys Asp Ser Asp Ser Thr Thr Ser Gly Leu Pro Ala Trp Leu Gln Gln
    450                 455                 460
tgc aaa aat gag aaa aac tta caa aat agt gat aat cag aac tct atg      1440
Cys Lys Asn Glu Lys Asn Leu Gln Asn Ser Asp Asn Gln Asn Ser Met
465                 470                 475                 480
tca atc aaa gat ctt tgc aga aaa tgg aac tca ttt tgc agt tca atc      1488
Ser Ile Lys Asp Leu Cys Arg Lys Trp Asn Ser Phe Cys Ser Ser Ile
                485                 490                 495
cac cga caa cac tac ttt tct gag aaa acc ctc aca ttt tct tct gta      1536
His Arg Gln His Tyr Phe Ser Glu Lys Thr Leu Thr Phe Ser Ser Val
            500                 505                 510
tca cct tct tct tct act tca tat gac cag caa tac cct att ttt caa      1584
Ser Pro Ser Ser Ser Thr Ser Tyr Asp Gln Gln Tyr Pro Ile Phe Gln
        515                 520                 525
caa acc cac aat gag tgg ccc att gtt gaa ccc aag cat ttg aga atg      1632
Gln Thr His Asn Glu Trp Pro Ile Val Glu Pro Lys His Leu Arg Met
    530                 535                 540
tac att cca gaa cat aaa gat cat acc aaa caa tta cca ttt tca tca      1680
Tyr Ile Pro Glu His Lys Asp His Thr Lys Gln Leu Pro Phe Ser Ser
545                 550                 555                 560
aat cct aat tct act cca aac tca act tca tca agt gat gtc atg gaa      1728
Asn Pro Asn Ser Thr Pro Asn Ser Thr Ser Ser Ser Asp Val Met Glu
                565                 570                 575
gtg gtg tat ctc cac aag ttc aag gag cta aat gct gag aac ttg aaa      1776
Val Val Tyr Leu His Lys Phe Lys Glu Leu Asn Ala Glu Asn Leu Lys
            580                 585                 590
atc cta tcc att gca ttg gag aaa aaa gtg cca tgg cag aga gat ata      1824
Ile Leu Ser Ile Ala Leu Glu Lys Lys Val Pro Trp Gln Arg Asp Ile
        595                 600                 605
atc cct gaa ata gca agc act atc ttg caa tgc cgg tct ggc atg ata      1872
Ile Pro Glu Ile Ala Ser Thr Ile Leu Gln Cys Arg Ser Gly Met Ile
    610                 615                 620
aga aga aag ggg aag atg aaa aac agt gaa tcc aaa gaa gaa act tgg      1920
Arg Arg Lys Gly Lys Met Lys Asn Ser Glu Ser Lys Glu Glu Thr Trp
625                 630                 635                 640
ttg ttt ttt caa gga gtt gat gtg gaa gct aaa gag aag ata gct aaa      1968
Leu Phe Phe Gln Gly Val Asp Val Glu Ala Lys Glu Lys Ile Ala Lys
                645                 650                 655
gaa ttg gcc agg cta gtt ttt ggc tcc aat gac agc ttc att tca gtt      2016
Glu Leu Ala Arg Leu Val Phe Gly Ser Asn Asp Ser Phe Ile Ser Val
            660                 665                 670
```

```
tct tta agc agt ttt tct tct aca aga gca gac tcg acc gaa gat tgt        2064
Ser Leu Ser Ser Phe Ser Ser Thr Arg Ala Asp Ser Thr Glu Asp Cys
        675                 680                 685
aga aat aaa aga tca aga gat gaa caa agt tgc agc tat atc gag aga        2112
Arg Asn Lys Arg Ser Arg Asp Glu Gln Ser Cys Ser Tyr Ile Glu Arg
    690                 695                 700
ttt tct gag gca gca tca aac aat cca cgt aga gtt ttc tta gtg gaa        2160
Phe Ser Glu Ala Ala Ser Asn Asn Pro Arg Arg Val Phe Leu Val Glu
705                 710                 715                 720
gac gtt gag caa gca gat tat tgc tct caa att ggt ttc aaa aga gct        2208
Asp Val Glu Gln Ala Asp Tyr Cys Ser Gln Ile Gly Phe Lys Arg Ala
                725                 730                 735
att gaa agt gga aga ata acc aat tcc aat ggt caa gaa gtt ggc ctt        2256
Ile Glu Ser Gly Arg Ile Thr Asn Ser Asn Gly Gln Glu Val Gly Leu
            740                 745                 750
agt gat gct atc atc att ttg agc tgt gaa agc ttc agc tca aga tct        2304
Ser Asp Ala Ile Ile Ile Leu Ser Cys Glu Ser Phe Ser Ser Arg Ser
        755                 760                 765
aga gcc tgc tct cct cca atc aag cag aga aca gat ggg tct tat gaa        2352
Arg Ala Cys Ser Pro Pro Ile Lys Gln Arg Thr Asp Gly Ser Tyr Glu
    770                 775                 780
gaa gaa gac aat gct ggt gcc ggt gct gca tta atg gag gac aca acc        2400
Glu Glu Asp Asn Ala Gly Ala Gly Ala Ala Leu Met Glu Asp Thr Thr
785                 790                 795                 800
cct tgc ata tcc ctg gat ttg aat att tcc gtt gac gat gat aac ata        2448
Pro Cys Ile Ser Leu Asp Leu Asn Ile Ser Val Asp Asp Asp Asn Ile
                805                 810                 815
ttg gaa gat caa tcg att gat gac att ggc ctc ctt gaa tcc gtc gat        2496
Leu Glu Asp Gln Ser Ile Asp Asp Ile Gly Leu Leu Glu Ser Val Asp
            820                 825                 830
aga cgg att ata ttc aaa att caa gag ttt tga                            2529
Arg Arg Ile Ile Phe Lys Ile Gln Glu Phe
        835                 840

<210> 30
<211> 842
<212> PRT
<213> Populus trichocarpa

<400> 30
Met Arg Ala Gly Gly Cys Thr Val Gln Gln Ala Leu Thr Ala Asp Ala
1               5                   10                  15
Ala Ser Val Ile Lys Gln Ala Val Thr Leu Ala Arg Arg Arg Gly His
            20                  25                  30
Ala Gln Val Thr Pro Leu His Val Ala Asn Thr Met Leu Ser Ala Ser
        35                  40                  45
Thr Gly Leu Phe Arg Thr Ala Cys Leu Gln Ser His Ser His Pro Leu
    50                  55                  60
Gln Cys Lys Ala Leu Glu Leu Cys Phe Asn Val Ala Leu Asn Arg Leu
65                  70                  75                  80
Pro Ala Ser Thr Ser Ser Pro Ile Leu Gly Thr His Ser Gln Gln Phe
                85                  90                  95
Pro Ser Ile Ser Asn Ala Leu Val Ala Ala Phe Lys Arg Ala Gln Ala
            100                 105                 110
His Gln Arg Arg Gly Ser Ile Glu Asn Gln Gln Gln Pro Leu Leu Ala
        115                 120                 125
Val Lys Ile Glu Leu Glu Gln Leu Ile Ile Ser Ile Leu Asp Asp Pro
    130                 135                 140
Ser Val Ser Arg Val Met Arg Glu Ala Gly Phe Ser Ser Thr Gln Val
145                 150                 155                 160
Lys Ser Asn Val Glu Gln Ala Val Ser Leu Glu Ile Cys Ser Gln Ser
                165                 170                 175
Ala Pro Ser Val Ser Ser Lys Ser Lys Glu Ser Asn Gly Leu Val Leu
            180                 185                 190
Ser Gln Ser Pro Thr Ser Ser Gln Val Gly Ala Lys Ala Thr Val Leu
```

```
        195                 200                 205
Asp Pro Ile Lys Asn Glu Asp Val Met Cys Val Ile Glu Asn Leu Val
    210                 215                 220
Asn Lys Arg Arg Arg Ser Phe Val Ile Val Gly Glu Ser Leu Ala Ser
225                 230                 235                 240
Ile Glu Val Val Val Lys Gly Val Ile Asp Lys Val Gln Lys Gly Asp
                245                 250                 255
Val Pro Glu Ala Leu Arg Glu Val Lys Phe Leu Thr Ile Pro Val Ser
            260                 265                 270
Ser Phe Gly His Phe Ser Arg Val Glu Val Glu His Lys Leu Glu Glu
        275                 280                 285
Leu Lys Ile His Val Arg Ser Tyr Met Gly Lys Gly Val Val Leu Asn
    290                 295                 300
Leu Gly Asp Leu Lys Trp Ala Ile Glu Asn Arg Ala Ser Ser Ser Ser
305                 310                 315                 320
Ser Glu Gln Gly Arg Cys Phe Phe Cys Pro Met Glu Tyr Met Ile Ile
                325                 330                 335
Glu Leu Gly Lys Leu Ala Cys Gly Ile Gly Glu Asn Ile Asn Gly Arg
            340                 345                 350
Phe Trp Leu Met Gly Ile Ala Thr Phe Gln Thr Tyr Met Lys Cys Lys
        355                 360                 365
Ser Gly His Pro Ser Gly Gly Thr Val Leu Gly Leu His Pro Leu Thr
    370                 375                 380
Ile Pro Ala Gly Ser Leu Arg Leu Ser Leu Ile Ser Asp Ser Asp Leu
385                 390                 395                 400
Arg Cys Gln Ser Thr Arg Asn Lys Ala Gly Asn Gly Ser Ser Ser Trp
                405                 410                 415
Ile Leu His Glu Gly Gly Glu Asp Lys Gln Leu Thr Cys Cys Ala Asp
            420                 425                 430
Cys Ser Ala Lys Phe Glu Ser Glu Ala Arg Ser Leu Pro Thr Ser Thr
        435                 440                 445
Cys Asp Ser Asp Ser Thr Thr Ser Gly Leu Pro Ala Trp Leu Gln Gln
    450                 455                 460
Cys Lys Asn Glu Lys Asn Leu Gln Asn Ser Asp Asn Gln Asn Ser Met
465                 470                 475                 480
Ser Ile Lys Asp Leu Cys Arg Lys Trp Asn Ser Phe Cys Ser Ser Ile
                485                 490                 495
His Arg Gln His Tyr Phe Ser Glu Lys Thr Leu Thr Phe Ser Ser Val
            500                 505                 510
Ser Pro Ser Ser Ser Thr Ser Tyr Asp Gln Gln Tyr Pro Ile Phe Gln
        515                 520                 525
Gln Thr His Asn Glu Trp Pro Ile Val Glu Pro Lys His Leu Arg Met
    530                 535                 540
Tyr Ile Pro Glu His Lys Asp His Thr Lys Gln Leu Pro Phe Ser Ser
545                 550                 555                 560
Asn Pro Asn Ser Thr Pro Asn Ser Thr Ser Ser Ser Asp Val Met Glu
                565                 570                 575
Val Val Tyr Leu His Lys Phe Lys Glu Leu Asn Ala Glu Asn Leu Lys
            580                 585                 590
Ile Leu Ser Ile Ala Leu Glu Lys Lys Val Pro Trp Gln Arg Asp Ile
        595                 600                 605
Ile Pro Glu Ile Ala Ser Thr Ile Leu Gln Cys Arg Ser Gly Met Ile
    610                 615                 620
Arg Arg Lys Gly Lys Met Lys Asn Ser Glu Ser Lys Glu Glu Thr Trp
625                 630                 635                 640
Leu Phe Phe Gln Gly Val Asp Val Glu Ala Lys Glu Lys Ile Ala Lys
                645                 650                 655
Glu Leu Ala Arg Leu Val Phe Gly Ser Asn Asp Ser Phe Ile Ser Val
            660                 665                 670
Ser Leu Ser Ser Phe Ser Ser Thr Arg Ala Asp Ser Thr Glu Asp Cys
        675                 680                 685
Arg Asn Lys Arg Ser Arg Asp Glu Gln Ser Cys Ser Tyr Ile Glu Arg
    690                 695                 700
Phe Ser Glu Ala Ala Ser Asn Asn Pro Arg Arg Val Phe Leu Val Glu
705                 710                 715                 720
```

```
Asp Val Glu Gln Ala Asp Tyr Cys Ser Gln Ile Gly Phe Lys Arg Ala
                725                 730                 735
Ile Glu Ser Gly Arg Ile Thr Asn Ser Asn Gly Gln Glu Val Gly Leu
            740                 745                 750
Ser Asp Ala Ile Ile Ile Leu Ser Cys Glu Ser Phe Ser Ser Arg Ser
        755                 760                 765
Arg Ala Cys Ser Pro Pro Ile Lys Gln Arg Thr Asp Gly Ser Tyr Glu
    770                 775                 780
Glu Glu Asp Asn Ala Gly Ala Gly Ala Ala Leu Met Glu Asp Thr Thr
785                 790                 795                 800
Pro Cys Ile Ser Leu Asp Leu Asn Ile Ser Val Asp Asp Asp Asn Ile
                805                 810                 815
Leu Glu Asp Gln Ser Ile Asp Asp Ile Gly Leu Leu Glu Ser Val Asp
            820                 825                 830
Arg Arg Ile Ile Phe Lys Ile Gln Glu Phe
        835                 840
```

<210> 31
<211> 2565
<212> DNA
<213> Populus trichocarpa

<220>
<221> CDS
<222> (1)..(2565)

<400> 31

```
atg agg gct gga att tgc agt gta cag cag gct ctt act ccc gag gct       48
Met Arg Ala Gly Ile Cys Ser Val Gln Gln Ala Leu Thr Pro Glu Ala
1               5                   10                  15
gtt agt tta gtg aaa caa gca gtt ggt ctt gct agg cgg cga ggc cat       96
Val Ser Leu Val Lys Gln Ala Val Gly Leu Ala Arg Arg Arg Gly His
            20                  25                  30
gct caa gtc act cct ctt cat gta gca agc aca atg ctt gct tcc tct      144
Ala Gln Val Thr Pro Leu His Val Ala Ser Thr Met Leu Ala Ser Ser
        35                  40                  45
act ggt ctt ctc aga agg gct tgc ctt caa tcc cac tct cat cct ctt      192
Thr Gly Leu Leu Arg Arg Ala Cys Leu Gln Ser His Ser His Pro Leu
    50                  55                  60
caa tgc aaa gct ctg gag ctc tgc ttc aat gtg gcg ctt aat cgc ctc      240
Gln Cys Lys Ala Leu Glu Leu Cys Phe Asn Val Ala Leu Asn Arg Leu
65                  70                  75                  80
cct gcg tcc act tct agt gcc cta tta ggc cct cac tcc tca tac cct      288
Pro Ala Ser Thr Ser Ser Ala Leu Leu Gly Pro His Ser Ser Tyr Pro
                85                  90                  95
tcc ctc tcc aat gcc ttg gtt gcg gcc ttt aag cgt gct cag gct cac      336
Ser Leu Ser Asn Ala Leu Val Ala Ala Phe Lys Arg Ala Gln Ala His
            100                 105                 110
caa cgg cga ggg tcc atc gaa aac cag caa cag ccc att tta gct ttg      384
Gln Arg Arg Gly Ser Ile Glu Asn Gln Gln Gln Pro Ile Leu Ala Leu
        115                 120                 125
aaa att gag ata gaa cag ctc att atc tct atc cta gat gat cct agt      432
Lys Ile Glu Ile Glu Gln Leu Ile Ile Ser Ile Leu Asp Asp Pro Ser
    130                 135                 140
gtt agt aga gtc atg aag gag gct ggt ttc tct agt act caa gtg aaa      480
Val Ser Arg Val Met Lys Glu Ala Gly Phe Ser Ser Thr Gln Val Lys
145                 150                 155                 160
aac aag gta gaa caa act gtt tct ttg gag att tgt cct caa agt tct      528
Asn Lys Val Glu Gln Thr Val Ser Leu Glu Ile Cys Pro Gln Ser Ser
                165                 170                 175
ctt act gtc agc tgt cag ccc aaa gaa atc atc aaa cct caa gtt ctt      576
Leu Thr Val Ser Cys Gln Pro Lys Glu Ile Ile Lys Pro Gln Val Leu
            180                 185                 190
agc gct agt gtg tct caa tct cta cct ttc agt cag ttt gga atc ata      624
Ser Ala Ser Val Ser Gln Ser Leu Pro Phe Ser Gln Phe Gly Ile Ile
```

```
        195                 200                 205
cac agc aaa cca tta gat caa gta agg aat gat gat gta atg agt gtc       672
His Ser Lys Pro Leu Asp Gln Val Arg Asn Asp Asp Val Met Ser Val
    210                 215                 220
tta aac aca ttg gtg ggc aaa aaa aga aac act atc atc aca gga gag       720
Leu Asn Thr Leu Val Gly Lys Lys Arg Asn Thr Ile Ile Thr Gly Glu
225                 230                 235                 240
tgc ctg gct act gct gag agc gta gtt agg ggg gtg atg gac aag ttt       768
Cys Leu Ala Thr Ala Glu Ser Val Val Arg Gly Val Met Asp Lys Phe
                245                 250                 255
gag aga ggg gag gtt tct ggg gat ttg agg tct gtg agg ttc aaa aac       816
Glu Arg Gly Glu Val Ser Gly Asp Leu Arg Ser Val Arg Phe Lys Asn
            260                 265                 270
ctt cct ctt ttc tct ttc aga agt ctt tcc aaa gag gac ctt gaa cag       864
Leu Pro Leu Phe Ser Phe Arg Ser Leu Ser Lys Glu Asp Leu Glu Gln
        275                 280                 285
aag ctt atg gag ctt agg tgt att gtg aag agc tat ata agc act ggg       912
Lys Leu Met Glu Leu Arg Cys Ile Val Lys Ser Tyr Ile Ser Thr Gly
    290                 295                 300
gtg gtt tta tat ctg ggt gat ctc aaa tgg ata gct gat ttt tgg tca       960
Val Val Leu Tyr Leu Gly Asp Leu Lys Trp Ile Ala Asp Phe Trp Ser
305                 310                 315                 320
agc tat ggt gag caa agg aga agc tac tac tgc act gca gat cac ata      1008
Ser Tyr Gly Glu Gln Arg Arg Ser Tyr Tyr Cys Thr Ala Asp His Ile
                325                 330                 335
atc ttg gag ctc aaa aga tta gtt cat gga ttc agt gaa acg gga agg      1056
Ile Leu Glu Leu Lys Arg Leu Val His Gly Phe Ser Glu Thr Gly Arg
            340                 345                 350
ttg tgg ctt atg ggg atc gct act ttc caa act tac atg aag tgt aaa      1104
Leu Trp Leu Met Gly Ile Ala Thr Phe Gln Thr Tyr Met Lys Cys Lys
        355                 360                 365
gca ggc cac cct tct cta gag act atg tgg gaa ctt aat cct gta aca      1152
Ala Gly His Pro Ser Leu Glu Thr Met Trp Glu Leu Asn Pro Val Thr
    370                 375                 380
att cca gtt ggg agc ctg aac cta agt ctc aaa ctc gat agt gat tca      1200
Ile Pro Val Gly Ser Leu Asn Leu Ser Leu Lys Leu Asp Ser Asp Ser
385                 390                 395                 400
caa tct cat caa tcc aga agc aag gca tct ttg aat ggg tcc agt tgg      1248
Gln Ser His Gln Ser Arg Ser Lys Ala Ser Leu Asn Gly Ser Ser Trp
                405                 410                 415
cca ctg ctt gaa tct aga gtt gat aac cac tta act tgc tgg aca gat      1296
Pro Leu Leu Glu Ser Arg Val Asp Asn His Leu Thr Cys Trp Thr Asp
            420                 425                 430
tac tct gtc aac ttc aat aaa gaa gct caa agc cta gta ggt agg acc      1344
Tyr Ser Val Asn Phe Asn Lys Glu Ala Gln Ser Leu Val Gly Arg Thr
        435                 440                 445
cat aac aag gaa tcc act tct agc gtt act att tcc aac aat tca agc      1392
His Asn Lys Glu Ser Thr Ser Ser Val Thr Ile Ser Asn Asn Ser Ser
    450                 455                 460
ttg ccg tta tgg ctc cag cag tgc aaa gaa act gaa aga aat aca acc      1440
Leu Pro Leu Trp Leu Gln Gln Cys Lys Glu Thr Glu Arg Asn Thr Thr
465                 470                 475                 480
aat gac cag gaa tat cta tgc aat aaa ggg att tct ctt ttt ggt tca      1488
Asn Asp Gln Glu Tyr Leu Cys Asn Lys Gly Ile Ser Leu Phe Gly Ser
                485                 490                 495
gtc cac aaa cag tcc tat tat cct gag aaa acc atc aaa ttt gct tcg      1536
Val His Lys Gln Ser Tyr Tyr Pro Glu Lys Thr Ile Lys Phe Ala Ser
            500                 505                 510
tct cct cct tca ccg aat tca ttc tcc tcg caa gag cgc aac acc gac      1584
Ser Pro Pro Ser Pro Asn Ser Phe Ser Ser Gln Glu Arg Asn Thr Asp
        515                 520                 525
cca caa cag aca cac cta agt tgg cca gtg att ttt gaa cat aaa cag      1632
Pro Gln Gln Thr His Leu Ser Trp Pro Val Ile Phe Glu His Lys Gln
    530                 535                 540
ttt gaa aag gag aac cag att tgg att tca gaa tgc agc aat gaa ggt      1680
```

```
Phe Glu Lys Glu Asn Gln Ile Trp Ile Ser Glu Cys Ser Asn Glu Gly
545                 550                 555                 560
tat gaa agc agt ttg aga aat gtt ccc aaa ccc gac ctt ttg tca aat      1728
Tyr Glu Ser Ser Leu Arg Asn Val Pro Lys Pro Asp Leu Leu Ser Asn
                565                 570                 575
ccc aac tct agt cca aat tca gct tct tca agt gag gca atg gat gat      1776
Pro Asn Ser Ser Pro Asn Ser Ala Ser Ser Ser Glu Ala Met Asp Asp
            580                 585                 590
att gag ggt gtc caa agc ttc aag gag ttc aat gac tac agc ttg aag      1824
Ile Glu Gly Val Gln Ser Phe Lys Glu Phe Asn Asp Tyr Ser Leu Lys
        595                 600                 605
aat cta cgt agt ggc ttg gag aag aag gtt cca tgg cag aag gat atc      1872
Asn Leu Arg Ser Gly Leu Glu Lys Lys Val Pro Trp Gln Lys Asp Ile
    610                 615                 620
att cct gaa att gca acc act atc ctt gag tgc agg tct gga atg agg      1920
Ile Pro Glu Ile Ala Thr Thr Ile Leu Glu Cys Arg Ser Gly Met Arg
625                 630                 635                 640
aaa agg aaa gga aag ttg aat cac ata gaa gac aaa gca gaa act tgg      1968
Lys Arg Lys Gly Lys Leu Asn His Ile Glu Asp Lys Ala Glu Thr Trp
                645                 650                 655
ctg ttt ttc tta ggt gtt gat ttt gaa ggt aaa gaa aag att gca agg      2016
Leu Phe Phe Leu Gly Val Asp Phe Glu Gly Lys Glu Lys Ile Ala Arg
            660                 665                 670
gag cta gct aaa cta gtt ttt ggc tct caa agc aac ttt gta tcg att      2064
Glu Leu Ala Lys Leu Val Phe Gly Ser Gln Ser Asn Phe Val Ser Ile
        675                 680                 685
ggt tta agc aat ttc tcc tca tca aga gct gat tct atc gaa gaa tcc      2112
Gly Leu Ser Asn Phe Ser Ser Ser Arg Ala Asp Ser Ile Glu Glu Ser
    690                 695                 700
aaa aac aag agg gca aga gat gag ttg ggt tgc agt tat ctt gag aga      2160
Lys Asn Lys Arg Ala Arg Asp Glu Leu Gly Cys Ser Tyr Leu Glu Arg
705                 710                 715                 720
ctt ggc cta gct ttg aat gaa aat cct cat cgc gtg ttc ttc atg gaa      2208
Leu Gly Leu Ala Leu Asn Glu Asn Pro His Arg Val Phe Phe Met Glu
                725                 730                 735
gat gtg gat caa gtt gac aac tgt tct caa aag ggt atc aag caa gca      2256
Asp Val Asp Gln Val Asp Asn Cys Ser Gln Lys Gly Ile Lys Gln Ala
            740                 745                 750
att gaa aat gga aat gta aca ctt cct gat ggt gaa aaa gtc cct ctt      2304
Ile Glu Asn Gly Asn Val Thr Leu Pro Asp Gly Glu Lys Val Pro Leu
        755                 760                 765
aag gat gcc att att att ttc agc tgt gaa agc ttc tgt tcg gtg tcc      2352
Lys Asp Ala Ile Ile Ile Phe Ser Cys Glu Ser Phe Cys Ser Val Ser
    770                 775                 780
aga aca tgt tct cct cca aga agg caa aaa acc ggt gac aat cat gaa      2400
Arg Thr Cys Ser Pro Pro Arg Arg Gln Lys Thr Gly Asp Asn His Glu
785                 790                 795                 800
gac aag gaa gat gag gat gtc atg gag gag aaa agt cta gtt ctt tca      2448
Asp Lys Glu Asp Glu Asp Val Met Glu Glu Lys Ser Leu Val Leu Ser
                805                 810                 815
ctt gac ctg aat att tcc ttt ggc gat aat gga gat gat caa tgt tca      2496
Leu Asp Leu Asn Ile Ser Phe Gly Asp Asn Gly Asp Asp Gln Cys Ser
            820                 825                 830
ctt gca gaa tat ggg att tta gaa tct gtg gat agg cag gtt gtt ttc      2544
Leu Ala Glu Tyr Gly Ile Leu Glu Ser Val Asp Arg Gln Val Val Phe
        835                 840                 845
aaa att caa gaa tta agt tag                                          2565
Lys Ile Gln Glu Leu Ser
    850
```

```
<210> 32
<211> 854
<212> PRT
<213> Populus trichocarpa
```

<400> 32

```
Met Arg Ala Gly Ile Cys Ser Val Gln Gln Ala Leu Thr Pro Glu Ala
1               5                   10                  15
Val Ser Leu Val Lys Gln Ala Val Gly Leu Ala Arg Arg Arg Gly His
            20                  25                  30
Ala Gln Val Thr Pro Leu His Val Ala Ser Thr Met Leu Ala Ser Ser
        35                  40                  45
Thr Gly Leu Leu Arg Arg Ala Cys Leu Gln Ser His Ser His Pro Leu
    50                  55                  60
Gln Cys Lys Ala Leu Glu Leu Cys Phe Asn Val Ala Leu Asn Arg Leu
65                  70                  75                  80
Pro Ala Ser Thr Ser Ser Ala Leu Leu Gly Pro His Ser Ser Tyr Pro
                85                  90                  95
Ser Leu Ser Asn Ala Leu Val Ala Ala Phe Lys Arg Ala Gln Ala His
            100                 105                 110
Gln Arg Arg Gly Ser Ile Glu Asn Gln Gln Gln Pro Ile Leu Ala Leu
        115                 120                 125
Lys Ile Glu Ile Glu Gln Leu Ile Ile Ser Ile Leu Asp Asp Pro Ser
    130                 135                 140
Val Ser Arg Val Met Lys Glu Ala Gly Phe Ser Ser Thr Gln Val Lys
145                 150                 155                 160
Asn Lys Val Glu Gln Thr Val Ser Leu Glu Ile Cys Pro Gln Ser Ser
                165                 170                 175
Leu Thr Val Ser Cys Gln Pro Lys Glu Ile Ile Lys Pro Gln Val Leu
            180                 185                 190
Ser Ala Ser Val Ser Gln Ser Leu Pro Phe Ser Gln Phe Gly Ile Ile
        195                 200                 205
His Ser Lys Pro Leu Asp Gln Val Arg Asn Asp Asp Val Met Ser Val
    210                 215                 220
Leu Asn Thr Leu Val Gly Lys Lys Arg Asn Thr Ile Ile Thr Gly Glu
225                 230                 235                 240
Cys Leu Ala Thr Ala Glu Ser Val Val Arg Gly Val Met Asp Lys Phe
                245                 250                 255
Glu Arg Gly Glu Val Ser Gly Asp Leu Arg Ser Val Arg Phe Lys Asn
            260                 265                 270
Leu Pro Leu Phe Ser Phe Arg Ser Leu Ser Lys Glu Asp Leu Glu Gln
        275                 280                 285
Lys Leu Met Glu Leu Arg Cys Ile Val Lys Ser Tyr Ile Ser Thr Gly
    290                 295                 300
Val Val Leu Tyr Leu Gly Asp Leu Lys Trp Ile Ala Asp Phe Trp Ser
305                 310                 315                 320
Ser Tyr Gly Glu Gln Arg Arg Ser Tyr Tyr Cys Thr Ala Asp His Ile
                325                 330                 335
Ile Leu Glu Leu Lys Arg Leu Val His Gly Phe Ser Glu Thr Gly Arg
            340                 345                 350
Leu Trp Leu Met Gly Ile Ala Thr Phe Gln Thr Tyr Met Lys Cys Lys
        355                 360                 365
Ala Gly His Pro Ser Leu Glu Thr Met Trp Glu Leu Asn Pro Val Thr
    370                 375                 380
Ile Pro Val Gly Ser Leu Asn Leu Ser Leu Lys Leu Asp Ser Asp Ser
385                 390                 395                 400
Gln Ser His Gln Ser Arg Ser Lys Ala Ser Leu Asn Gly Ser Ser Trp
                405                 410                 415
Pro Leu Leu Glu Ser Arg Val Asp Asn His Leu Thr Cys Trp Thr Asp
            420                 425                 430
Tyr Ser Val Asn Phe Asn Lys Glu Ala Gln Ser Leu Val Gly Arg Thr
        435                 440                 445
His Asn Lys Glu Ser Thr Ser Ser Val Thr Ile Ser Asn Asn Ser Ser
    450                 455                 460
Leu Pro Leu Trp Leu Gln Gln Cys Lys Glu Thr Glu Arg Asn Thr Thr
465                 470                 475                 480
Asn Asp Gln Glu Tyr Leu Cys Asn Lys Gly Ile Ser Leu Phe Gly Ser
                485                 490                 495
Val His Lys Gln Ser Tyr Tyr Pro Glu Lys Thr Ile Lys Phe Ala Ser
            500                 505                 510
```

```
Ser Pro Pro Ser Pro Asn Ser Phe Ser Ser Gln Glu Arg Asn Thr Asp
        515                 520                 525
Pro Gln Gln Thr His Leu Ser Trp Pro Val Ile Phe Glu His Lys Gln
    530                 535                 540
Phe Glu Lys Glu Asn Gln Ile Trp Ile Ser Glu Cys Ser Asn Glu Gly
545                 550                 555                 560
Tyr Glu Ser Ser Leu Arg Asn Val Pro Lys Pro Asp Leu Leu Ser Asn
                565                 570                 575
Pro Asn Ser Ser Pro Asn Ser Ala Ser Ser Ser Glu Ala Met Asp Asp
            580                 585                 590
Ile Glu Gly Val Gln Ser Phe Lys Glu Phe Asn Asp Tyr Ser Leu Lys
        595                 600                 605
Asn Leu Arg Ser Gly Leu Glu Lys Lys Val Pro Trp Gln Lys Asp Ile
    610                 615                 620
Ile Pro Glu Ile Ala Thr Thr Ile Leu Glu Cys Arg Ser Gly Met Arg
625                 630                 635                 640
Lys Arg Lys Gly Lys Leu Asn His Ile Glu Asp Lys Ala Glu Thr Trp
                645                 650                 655
Leu Phe Phe Leu Gly Val Asp Phe Glu Gly Lys Glu Lys Ile Ala Arg
            660                 665                 670
Glu Leu Ala Lys Leu Val Phe Gly Ser Gln Ser Asn Phe Val Ser Ile
        675                 680                 685
Gly Leu Ser Asn Phe Ser Ser Ser Arg Ala Asp Ser Ile Glu Glu Ser
    690                 695                 700
Lys Asn Lys Arg Ala Arg Asp Glu Leu Gly Cys Ser Tyr Leu Glu Arg
705                 710                 715                 720
Leu Gly Leu Ala Leu Asn Glu Asn Pro His Arg Val Phe Phe Met Glu
                725                 730                 735
Asp Val Asp Gln Val Asp Asn Cys Ser Gln Lys Gly Ile Lys Gln Ala
            740                 745                 750
Ile Glu Asn Gly Asn Val Thr Leu Pro Asp Gly Glu Lys Val Pro Leu
        755                 760                 765
Lys Asp Ala Ile Ile Ile Phe Ser Cys Glu Ser Phe Cys Ser Val Ser
    770                 775                 780
Arg Thr Cys Ser Pro Pro Arg Arg Gln Lys Thr Gly Asp Asn His Glu
785                 790                 795                 800
Asp Lys Glu Asp Glu Asp Val Met Glu Glu Lys Ser Leu Val Leu Ser
                805                 810                 815
Leu Asp Leu Asn Ile Ser Phe Gly Asp Asn Gly Asp Asp Gln Cys Ser
            820                 825                 830
Leu Ala Glu Tyr Gly Ile Leu Glu Ser Val Asp Arg Gln Val Val Phe
        835                 840                 845
Lys Ile Gln Glu Leu Ser
    850

<210> 33
<211> 2583
<212> DNA
<213> Populus trichocarpa

<220>
<221> CDS
<222> (1)..(2583)

<400> 33
atg aga gca gga ggt tgc aca gtg caa caa gct cta aca gca gag gca       48
Met Arg Ala Gly Gly Cys Thr Val Gln Gln Ala Leu Thr Ala Glu Ala
  1               5                  10                  15
gca agt gtt att aaa caa gca gta act ctt gct aga agg aga ggc cat       96
Ala Ser Val Ile Lys Gln Ala Val Thr Leu Ala Arg Arg Arg Gly His
            20                  25                  30
gcc caa gtc act cct ctc cat gtt gct aat acc atg ctt tct gcc tcc      144
Ala Gln Val Thr Pro Leu His Val Ala Asn Thr Met Leu Ser Ala Ser
        35                  40                  45
act ggc cta ctg aga aca gct tgc ctt cag tca cac tct cac cct ctt      192
```

```
Thr Gly Leu Leu Arg Thr Ala Cys Leu Gln Ser His Ser His Pro Leu
     50                  55                  60
cag tgc aaa gcc cta gag ctt tgc ttc aat gtc gcg ctt aat cgt ctc      240
Gln Cys Lys Ala Leu Glu Leu Cys Phe Asn Val Ala Leu Asn Arg Leu
 65                  70                  75                  80
cca aca tca act tca agc ccc atg ata ggt act ccc tct caa cag ttc      288
Pro Thr Ser Thr Ser Ser Pro Met Ile Gly Thr Pro Ser Gln Gln Phe
                 85                  90                  95
cct tca atc tct aat gcc ttg gtc gca gcc ttt aag cgc gct cag gct      336
Pro Ser Ile Ser Asn Ala Leu Val Ala Ala Phe Lys Arg Ala Gln Ala
            100                 105                 110
cac cag cgg cgt ggc tct att gag aac cag cag caa cct ctc ctt gca      384
His Gln Arg Arg Gly Ser Ile Glu Asn Gln Gln Gln Pro Leu Leu Ala
        115                 120                 125
gtg aag ata gag tta gag cag ctg atg ata tcc att tta gat gat cct      432
Val Lys Ile Glu Leu Glu Gln Leu Met Ile Ser Ile Leu Asp Asp Pro
    130                 135                 140
agt gtt agt aga gtc atg aga gaa gct ggt ttc tcc agt act caa gtg      480
Ser Val Ser Arg Val Met Arg Glu Ala Gly Phe Ser Ser Thr Gln Val
145                 150                 155                 160
aaa agc aat gtt gag gaa gct gtt tcc cta gaa ata tgt tca caa agt      528
Lys Ser Asn Val Glu Glu Ala Val Ser Leu Glu Ile Cys Ser Gln Ser
                165                 170                 175
gtt cct tct gtg agt atc aag tcc aac gaa agc aat ggt tta gtc cat      576
Val Pro Ser Val Ser Ile Lys Ser Asn Glu Ser Asn Gly Leu Val His
            180                 185                 190
cca gag tct cca cct tgg agt caa gtt gga gca aaa gca gca gtt tta      624
Pro Glu Ser Pro Pro Trp Ser Gln Val Gly Ala Lys Ala Ala Val Leu
        195                 200                 205
gat cca att aaa aat gaa gat gtg atg tgt gtt ata gag aat tta atg      672
Asp Pro Ile Lys Asn Glu Asp Val Met Cys Val Ile Glu Asn Leu Met
    210                 215                 220
aat aaa agg agg aga agt ttt gtt att gta ggg gaa tct ctg gca agc      720
Asn Lys Arg Arg Arg Ser Phe Val Ile Val Gly Glu Ser Leu Ala Ser
225                 230                 235                 240
att gaa gtt gta gtt aaa gga gtc aag gac aaa gtt caa aag gga gat      768
Ile Glu Val Val Val Lys Gly Val Lys Asp Lys Val Gln Lys Gly Asp
                245                 250                 255
gtt cct gag ggc ttg agg gaa gtg aag ttc tta cca att cca gtt tcc      816
Val Pro Glu Gly Leu Arg Glu Val Lys Phe Leu Pro Ile Pro Val Ser
            260                 265                 270
tct ttt ggc agt ttc tct agg gta gag gta gaa cat aaa ctt gag gag      864
Ser Phe Gly Ser Phe Ser Arg Val Glu Val Glu His Lys Leu Glu Glu
        275                 280                 285
ctt aaa ggc cat gtg aga agc tac atg ggc aaa gga gtg gtt ttg aat      912
Leu Lys Gly His Val Arg Ser Tyr Met Gly Lys Gly Val Val Leu Asn
    290                 295                 300
ttg gga gat ctt aaa tgg gct att gag aat agg gat act agt tca tca      960
Leu Gly Asp Leu Lys Trp Ala Ile Glu Asn Arg Asp Thr Ser Ser Ser
305                 310                 315                 320
agt cat gag caa ggg agc tgc tat ttc tgt cca ttg gta tac ctg atc     1008
Ser His Glu Gln Gly Ser Cys Tyr Phe Cys Pro Leu Val Tyr Leu Ile
                325                 330                 335
gta gaa ctt ggc aaa ttt gct tgt gca att gga gat aac aac gga aga     1056
Val Glu Leu Gly Lys Phe Ala Cys Ala Ile Gly Asp Asn Asn Gly Arg
            340                 345                 350
ttt tgg ctt atg ggg att gcc act ttc caa act tac atg aag tat aaa     1104
Phe Trp Leu Met Gly Ile Ala Thr Phe Gln Thr Tyr Met Lys Tyr Lys
        355                 360                 365
tca gac cat cca ccg gga gat act gtt ttg ggt ctc cat cca ctt aca     1152
Ser Asp His Pro Pro Gly Asp Thr Val Leu Gly Leu His Pro Leu Thr
    370                 375                 380
att cca gcc ggc agc tta cgc ttg agt ctc atc agt gac agt gat cta     1200
Ile Pro Ala Gly Ser Leu Arg Leu Ser Leu Ile Ser Asp Ser Asp Leu
385                 390                 395                 400
```

```
cta cgt cag tcg aca agc aac aaa gct gaa aat ggg tgt aga agc tgg        1248
Leu Arg Gln Ser Thr Ser Asn Lys Ala Glu Asn Gly Cys Arg Ser Trp
                405                 410                 415
att ata ctt gaa ggt ggg gag gac aaa caa ctc act tct tgt tct aat        1296
Ile Ile Leu Glu Gly Gly Glu Asp Lys Gln Leu Thr Ser Cys Ser Asn
            420                 425                 430
tat tca gca aag ttt gag act gaa gct cga cgc tta cca aat agt acc        1344
Tyr Ser Ala Lys Phe Glu Thr Glu Ala Arg Arg Leu Pro Asn Ser Thr
        435                 440                 445
tgt aat agt gac tca act tct aca ctt cct gca tgg ctt caa aaa tac        1392
Cys Asn Ser Asp Ser Thr Ser Thr Leu Pro Ala Trp Leu Gln Lys Tyr
    450                 455                 460
aaa aat gag aaa aaa gta caa aat agt gat aat cag gat tct atg cca        1440
Lys Asn Glu Lys Lys Val Gln Asn Ser Asp Asn Gln Asp Ser Met Pro
465                 470                 475                 480
atc aaa gat ctt tgc aga aaa tgg aac tca ttt tgc ggt tca atc cac        1488
Ile Lys Asp Leu Cys Arg Lys Trp Asn Ser Phe Cys Gly Ser Ile His
                485                 490                 495
caa caa aac tac tct tct gag gaa acc ctc acg ttt tct tct gta tca        1536
Gln Gln Asn Tyr Ser Ser Glu Glu Thr Leu Thr Phe Ser Ser Val Ser
            500                 505                 510
cct tct tct tcc act tca tat gac cat caa tac cct aat ttg tac cga        1584
Pro Ser Ser Ser Thr Ser Tyr Asp His Gln Tyr Pro Asn Leu Tyr Arg
        515                 520                 525
aac caa aat gaa tgg ccc att gtt gaa ccc cag cag tca tct agg gac        1632
Asn Gln Asn Glu Trp Pro Ile Val Glu Pro Gln Gln Ser Ser Arg Asp
    530                 535                 540
aac cat ttc tgg atc ggt aca gag gct atc aac aag tgc agc att gaa        1680
Asn His Phe Trp Ile Gly Thr Glu Ala Ile Asn Lys Cys Ser Ile Glu
545                 550                 555                 560
cca agt ttg agg aag tac att cca gag cat aaa gat cat acc aaa caa        1728
Pro Ser Leu Arg Lys Tyr Ile Pro Glu His Lys Asp His Thr Lys Gln
                565                 570                 575
cta cca ttt tca tca aat act aat tct act ccg aac tca gct tcc tca        1776
Leu Pro Phe Ser Ser Asn Thr Asn Ser Thr Pro Asn Ser Ala Ser Ser
            580                 585                 590
agt gat gtc atg gaa atg gag cat ctc cac aag ttc aag gag ctg aat        1824
Ser Asp Val Met Glu Met Glu His Leu His Lys Phe Lys Glu Leu Asn
        595                 600                 605
gct gag aac ttg aaa acc cta tgc aat gca ttg gag aaa aaa gtg ccg        1872
Ala Glu Asn Leu Lys Thr Leu Cys Asn Ala Leu Glu Lys Lys Val Pro
    610                 615                 620
tgg cag aaa gat ata atc cct gaa ata gca agc act atc ttg caa tgc        1920
Trp Gln Lys Asp Ile Ile Pro Glu Ile Ala Ser Thr Ile Leu Gln Cys
625                 630                 635                 640
cgg tct ggc atg gca aga aga aag ggg aag gta aaa aac agt gtg gcc        1968
Arg Ser Gly Met Ala Arg Arg Lys Gly Lys Val Lys Asn Ser Val Ala
                645                 650                 655
aag gaa gaa act tgg ttg ttc ttt caa gga gta gat atg gaa gat aaa        2016
Lys Glu Glu Thr Trp Leu Phe Phe Gln Gly Val Asp Met Glu Asp Lys
            660                 665                 670
gag aag ata gct aaa gaa ttg gct agg cta gtt ttt ggc tcc cat gaa        2064
Glu Lys Ile Ala Lys Glu Leu Ala Arg Leu Val Phe Gly Ser His Glu
        675                 680                 685
agc ttc att tcg att tct tta agc agt ttt tcc tct aca aga gca gac        2112
Ser Phe Ile Ser Ile Ser Leu Ser Ser Phe Ser Ser Thr Arg Ala Asp
    690                 695                 700
tcc acc gaa gat tgt aga aac aaa aga aca aga gat gaa caa agt tgc        2160
Ser Thr Glu Asp Cys Arg Asn Lys Arg Thr Arg Asp Glu Gln Ser Cys
705                 710                 715                 720
agc tac atc gag aga ttt tct gac gcg gta tca agc aat cca cat aga        2208
Ser Tyr Ile Glu Arg Phe Ser Asp Ala Val Ser Ser Asn Pro His Arg
                725                 730                 735
gtt ttc tta gtg gaa gat gtc gag caa gca gat ttt ttc tct caa att        2256
Val Phe Leu Val Glu Asp Val Glu Gln Ala Asp Phe Phe Ser Gln Ile
```

```
            740                 745                 750
cgt ttc aaa agg gct att gaa aaa gga aga ata acc aat tac aat ggt      2304
Arg Phe Lys Arg Ala Ile Glu Lys Gly Arg Ile Thr Asn Tyr Asn Gly
        755                 760                 765
caa gaa gtt ggc ctt agt gat gct atc ata att ttg agc tgt gaa agc      2352
Gln Glu Val Gly Leu Ser Asp Ala Ile Ile Ile Leu Ser Cys Glu Ser
    770                 775                 780
ttt agc tca aga tcc aga gcc tgc tct cct cca atc aag cag aga aca      2400
Phe Ser Ser Arg Ser Arg Ala Cys Ser Pro Pro Ile Lys Gln Arg Thr
785                 790                 795                 800
gat ggg tca cat gaa gaa gag aac agc gct ggt gct aca tta atg gag      2448
Asp Gly Ser His Glu Glu Glu Asn Ser Ala Gly Ala Thr Leu Met Glu
                805                 810                 815
ggc aca agc cct tgt gtt tct ctg gat ttg aat att tcc att gac gat      2496
Gly Thr Ser Pro Cys Val Ser Leu Asp Leu Asn Ile Ser Ile Asp Asp
            820                 825                 830
gat agc gtg gaa gat cag tca atc gat gac att ggc ctt ctt gaa tcc      2544
Asp Ser Val Glu Asp Gln Ser Ile Asp Asp Ile Gly Leu Leu Glu Ser
        835                 840                 845
gtt gat aga cgg att atc ttc aaa att caa gac ttt tga                  2583
Val Asp Arg Arg Ile Ile Phe Lys Ile Gln Asp Phe
    850                 855                 860
```

<210> 34
<211> 860
<212> PRT
<213> Populus trichocarpa

<400> 34

```
Met Arg Ala Gly Gly Cys Thr Val Gln Gln Ala Leu Thr Ala Glu Ala
1               5                   10                  15
Ala Ser Val Ile Lys Gln Ala Val Thr Leu Ala Arg Arg Arg Gly His
            20                  25                  30
Ala Gln Val Thr Pro Leu His Val Ala Asn Thr Met Leu Ser Ala Ser
        35                  40                  45
Thr Gly Leu Leu Arg Thr Ala Cys Leu Gln Ser His Ser His Pro Leu
    50                  55                  60
Gln Cys Lys Ala Leu Glu Leu Cys Phe Asn Val Ala Leu Asn Arg Leu
65                  70                  75                  80
Pro Thr Ser Thr Ser Ser Pro Met Ile Gly Thr Pro Ser Gln Gln Phe
                85                  90                  95
Pro Ser Ile Ser Asn Ala Leu Val Ala Ala Phe Lys Arg Ala Gln Ala
            100                 105                 110
His Gln Arg Arg Gly Ser Ile Glu Asn Gln Gln Gln Pro Leu Leu Ala
        115                 120                 125
Val Lys Ile Glu Leu Glu Gln Leu Met Ile Ser Ile Leu Asp Asp Pro
    130                 135                 140
Ser Val Ser Arg Val Met Arg Glu Ala Gly Phe Ser Ser Thr Gln Val
145                 150                 155                 160
Lys Ser Asn Val Glu Glu Ala Val Ser Leu Glu Ile Cys Ser Gln Ser
                165                 170                 175
Val Pro Ser Val Ser Ile Lys Ser Asn Glu Ser Asn Gly Leu Val His
            180                 185                 190
Pro Glu Ser Pro Pro Trp Ser Gln Val Gly Ala Lys Ala Ala Val Leu
        195                 200                 205
Asp Pro Ile Lys Asn Glu Asp Val Met Cys Val Ile Glu Asn Leu Met
    210                 215                 220
Asn Lys Arg Arg Arg Ser Phe Val Ile Val Gly Glu Ser Leu Ala Ser
225                 230                 235                 240
Ile Glu Val Val Val Lys Gly Val Lys Asp Lys Val Gln Lys Gly Asp
                245                 250                 255
Val Pro Glu Gly Leu Arg Glu Val Lys Phe Leu Pro Ile Pro Val Ser
            260                 265                 270
Ser Phe Gly Ser Phe Ser Arg Val Glu Val Glu His Lys Leu Glu Glu
        275                 280                 285
```

```
Leu Lys Gly His Val Arg Ser Tyr Met Gly Lys Gly Val Val Leu Asn
    290                 295                 300
Leu Gly Asp Leu Lys Trp Ala Ile Glu Asn Arg Asp Thr Ser Ser Ser
305                 310                 315                 320
Ser His Glu Gln Gly Ser Cys Tyr Phe Cys Pro Leu Val Tyr Leu Ile
                325                 330                 335
Val Glu Leu Gly Lys Phe Ala Cys Ala Ile Gly Asp Asn Asn Gly Arg
            340                 345                 350
Phe Trp Leu Met Gly Ile Ala Thr Phe Gln Thr Tyr Met Lys Tyr Lys
        355                 360                 365
Ser Asp His Pro Pro Gly Asp Thr Val Leu Gly Leu His Pro Leu Thr
    370                 375                 380
Ile Pro Ala Gly Ser Leu Arg Leu Ser Leu Ile Ser Asp Ser Asp Leu
385                 390                 395                 400
Leu Arg Gln Ser Thr Ser Asn Lys Ala Glu Asn Gly Cys Arg Ser Trp
                405                 410                 415
Ile Ile Leu Glu Gly Gly Glu Asp Lys Gln Leu Thr Ser Cys Ser Asn
            420                 425                 430
Tyr Ser Ala Lys Phe Glu Thr Glu Ala Arg Arg Leu Pro Asn Ser Thr
        435                 440                 445
Cys Asn Ser Asp Ser Thr Ser Thr Leu Pro Ala Trp Leu Gln Lys Tyr
    450                 455                 460
Lys Asn Glu Lys Lys Val Gln Asn Ser Asp Asn Gln Asp Ser Met Pro
465                 470                 475                 480
Ile Lys Asp Leu Cys Arg Lys Trp Asn Ser Phe Cys Gly Ser Ile His
                485                 490                 495
Gln Gln Asn Tyr Ser Ser Glu Glu Thr Leu Thr Phe Ser Ser Val Ser
            500                 505                 510
Pro Ser Ser Ser Thr Ser Tyr Asp His Gln Tyr Pro Asn Leu Tyr Arg
        515                 520                 525
Asn Gln Asn Glu Trp Pro Ile Val Glu Pro Gln Gln Ser Ser Arg Asp
    530                 535                 540
Asn His Phe Trp Ile Gly Thr Glu Ala Ile Asn Lys Cys Ser Ile Glu
545                 550                 555                 560
Pro Ser Leu Arg Lys Tyr Ile Pro Glu His Lys Asp His Thr Lys Gln
                565                 570                 575
Leu Pro Phe Ser Ser Asn Thr Asn Ser Thr Pro Asn Ser Ala Ser Ser
            580                 585                 590
Ser Asp Val Met Glu Met Glu His Leu His Lys Phe Lys Glu Leu Asn
        595                 600                 605
Ala Glu Asn Leu Lys Thr Leu Cys Asn Ala Leu Glu Lys Lys Val Pro
    610                 615                 620
Trp Gln Lys Asp Ile Ile Pro Glu Ile Ala Ser Thr Ile Leu Gln Cys
625                 630                 635                 640
Arg Ser Gly Met Ala Arg Arg Lys Gly Lys Val Lys Asn Ser Val Ala
                645                 650                 655
Lys Glu Glu Thr Trp Leu Phe Phe Gln Gly Val Asp Met Glu Asp Lys
            660                 665                 670
Glu Lys Ile Ala Lys Glu Leu Ala Arg Leu Val Phe Gly Ser His Glu
        675                 680                 685
Ser Phe Ile Ser Ile Ser Leu Ser Ser Phe Ser Ser Thr Arg Ala Asp
    690                 695                 700
Ser Thr Glu Asp Cys Arg Asn Lys Arg Thr Arg Asp Glu Gln Ser Cys
705                 710                 715                 720
Ser Tyr Ile Glu Arg Phe Ser Asp Ala Val Ser Ser Asn Pro His Arg
                725                 730                 735
Val Phe Leu Val Glu Asp Val Glu Gln Ala Asp Phe Phe Ser Gln Ile
            740                 745                 750
Arg Phe Lys Arg Ala Ile Glu Lys Gly Arg Ile Thr Asn Tyr Asn Gly
        755                 760                 765
Gln Glu Val Gly Leu Ser Asp Ala Ile Ile Ile Leu Ser Cys Glu Ser
    770                 775                 780
Phe Ser Ser Arg Ser Arg Ala Cys Ser Pro Pro Ile Lys Gln Arg Thr
785                 790                 795                 800
Asp Gly Ser His Glu Glu Glu Asn Ser Ala Gly Ala Thr Leu Met Glu
```

```
                805                 810                 815
Gly Thr Ser Pro Cys Val Ser Leu Asp Leu Asn Ile Ser Ile Asp Asp
            820                 825                 830
Asp Ser Val Glu Asp Gln Ser Ile Asp Asp Ile Gly Leu Leu Glu Ser
        835                 840                 845
Val Asp Arg Arg Ile Ile Phe Lys Ile Gln Asp Phe
    850                 855                 860

<210> 35
<211> 2643
<212> DNA
<213> Sorghum bicolor

<220>
<221> CDS
<222> (1)..(2643)

<400> 35
atg aga gcc ggg ggc tgc acg gtg cag cag gcg ctg gcg ccg gag gcg       48
Met Arg Ala Gly Gly Cys Thr Val Gln Gln Ala Leu Ala Pro Glu Ala
1               5                   10                  15
gcg gcg gtg gtg aag cag gcg gtg agc ctg gcg cgg cgc cgc ggg aac       96
Ala Ala Val Val Lys Gln Ala Val Ser Leu Ala Arg Arg Arg Gly Asn
            20                  25                  30
gca cag gtg acg ccg ctg cac gtg gcc agc gcc atg ctg cac cag cag      144
Ala Gln Val Thr Pro Leu His Val Ala Ser Ala Met Leu His Gln Gln
        35                  40                  45
gta gtg gcg gcg gcc acg ggc acg ggc acg ggg ccg tcg tcg tcg acg      192
Val Val Ala Ala Ala Thr Gly Thr Gly Thr Gly Pro Ser Ser Ser Thr
    50                  55                  60
gcg gcg ggg ctg ctg cgc gcc gcg tgc ctg cag tcg cac tcg cac ccg      240
Ala Ala Gly Leu Leu Arg Ala Ala Cys Leu Gln Ser His Ser His Pro
65                  70                  75                  80
cta cag tgc aag gcg ctg gag ctc tgc ttc aac gtc gcg ctc aac cgc      288
Leu Gln Cys Lys Ala Leu Glu Leu Cys Phe Asn Val Ala Leu Asn Arg
                85                  90                  95
ctg ccg gcc tcg gcc tcg ccg ctc ctc ggc ggg cac ggc cac gtc tac      336
Leu Pro Ala Ser Ala Ser Pro Leu Leu Gly Gly His Gly His Val Tyr
            100                 105                 110
tac ccg ccg tcg ctc tcc aac gcg ctc gtc gcc gcc ttc aag cgc gcg      384
Tyr Pro Pro Ser Leu Ser Asn Ala Leu Val Ala Ala Phe Lys Arg Ala
        115                 120                 125
cag gcg cac cag cgc cgg ggg tcc gtc gac acg cag cag cag ccc gtg      432
Gln Ala His Gln Arg Arg Gly Ser Val Asp Thr Gln Gln Gln Pro Val
    130                 135                 140
ctc gcc gtc aag atc gag ctg gag cag ctc gtc atc tcc atc ctc gat      480
Leu Ala Val Lys Ile Glu Leu Glu Gln Leu Val Ile Ser Ile Leu Asp
145                 150                 155                 160
gac ccc agc gtc agc cgc gtc atg cgc gag gcc ggc ttc tcc agc acc      528
Asp Pro Ser Val Ser Arg Val Met Arg Glu Ala Gly Phe Ser Ser Thr
                165                 170                 175
cag gtg aag gcc aac gtc gag cag gcc gtc tct tcc atc gaa gca aac      576
Gln Val Lys Ala Asn Val Glu Gln Ala Val Ser Ser Ile Glu Ala Asn
            180                 185                 190
aac tcg gcc tct acc aac acc gcc gcc gcc gcg cac caa aac cct aac      624
Asn Ser Ala Ser Thr Asn Thr Ala Ala Ala Ala His Gln Asn Pro Asn
        195                 200                 205
cct agg gca gct cca cac gaa gag act atg cct agt aag ctg cag cta      672
Pro Arg Ala Ala Pro His Glu Glu Thr Met Pro Ser Lys Leu Gln Leu
    210                 215                 220
ccc ctc gac ctc gac caa gtg cgc gac gag gac gtc gcc gtc gtc ctg      720
Pro Leu Asp Leu Asp Gln Val Arg Asp Glu Asp Val Ala Val Val Leu
225                 230                 235                 240
gac tgc ctg gcg tcc cgg agc aag aaa cgc gtc atg gtc gtc gcc gag      768
Asp Cys Leu Ala Ser Arg Ser Lys Lys Arg Val Met Val Val Ala Glu
```

```
                245                 250                 255
tgc gcg gcc acg gcc gag gcg ccg acg cgg gcg gcg gtc gag aag atc      816
Cys Ala Ala Thr Ala Glu Ala Pro Thr Arg Ala Ala Val Glu Lys Ile
            260                 265                 270
aag cgc ggc gag gcg ctg cgc ggc gcg caa gtg gtc agc ctc aga gtg      864
Lys Arg Gly Glu Ala Leu Arg Gly Ala Gln Val Val Ser Leu Arg Val
        275                 280                 285
tcc agg ttc cgc gac ctg ccg agg gac gag gcg gag agg ctg ctc gtg      912
Ser Arg Phe Arg Asp Leu Pro Arg Asp Glu Ala Glu Arg Leu Leu Val
    290                 295                 300
gag ctc cgg tgc gcg gtg aag gtg ggc ggc agg gcg ggc ggc gtg gtg      960
Glu Leu Arg Cys Ala Val Lys Val Gly Gly Arg Ala Gly Gly Val Val
305                 310                 315                 320
ctg gtg gtg gag gac ctc ggg tgg gcg gcc gag ttc tgg gcc gcg cgc     1008
Leu Val Val Glu Asp Leu Gly Trp Ala Ala Glu Phe Trp Ala Ala Arg
                325                 330                 335
gcc gag tcc ggg agg gcc agg tgg ccg tcg agc tgc tgc tac tac tgc     1056
Ala Glu Ser Gly Arg Ala Arg Trp Pro Ser Ser Cys Cys Tyr Tyr Cys
            340                 345                 350
gcc gtc gag cac gcc gtc gcg gag gtg cgc gcc ctg gcg tgc cgc ggc     1104
Ala Val Glu His Ala Val Ala Glu Val Arg Ala Leu Ala Cys Arg Gly
        355                 360                 365
ggc gac ggc gtg tgg ctc atc ggc tac ggg acg tac cag agc tac atg     1152
Gly Asp Gly Val Trp Leu Ile Gly Tyr Gly Thr Tyr Gln Ser Tyr Met
    370                 375                 380
agg tgc agg gcc ggg cag ccc tcg ctg gag act ctc tgg ggg ctc cag     1200
Arg Cys Arg Ala Gly Gln Pro Ser Leu Glu Thr Leu Trp Gly Leu Gln
385                 390                 395                 400
acg ctc gcc gtc ccc gct ggt agc ctc gcc ttg agc ctc aac ttc gtc     1248
Thr Leu Ala Val Pro Ala Gly Ser Leu Ala Leu Ser Leu Asn Phe Val
                405                 410                 415
ggc gac agt gca act gca atg aca atc aat cac ctg gcc aag tgc gac     1296
Gly Asp Ser Ala Thr Ala Met Thr Ile Asn His Leu Ala Lys Cys Asp
            420                 425                 430
gac gac aga agt ggg aac aac ggg tcg gcg cca cgt tgc ctg tca ctt     1344
Asp Asp Arg Ser Gly Asn Asn Gly Ser Ala Pro Arg Cys Leu Ser Leu
        435                 440                 445
ttg gac gcc ggt ggt tcc ggc cac ctg acg gcc gtc tcc agc ttc ttc     1392
Leu Asp Ala Gly Gly Ser Gly His Leu Thr Ala Val Ser Ser Phe Phe
    450                 455                 460
gcc gac gac tgc tcc gca acc gca acc aag tgc gag ccg gcg ctg aag     1440
Ala Asp Asp Cys Ser Ala Thr Ala Thr Lys Cys Glu Pro Ala Leu Lys
465                 470                 475                 480
tcg agt atc cct cct tgg ctt cag cat tgc cgg gat cag gat cct tcc     1488
Ser Ser Ile Pro Pro Trp Leu Gln His Cys Arg Asp Gln Asp Pro Ser
                485                 490                 495
cgt tgt aag aaa agc tcg aca tgc ggt ggc tcg ctg tct cat cat cac     1536
Arg Cys Lys Lys Ser Ser Thr Cys Gly Gly Ser Leu Ser His His His
            500                 505                 510
cgg acg gcc cta aat ttc tcc acg gcg gtg gtg tcg ccg tcc tcc tcg     1584
Arg Thr Ala Leu Asn Phe Ser Thr Ala Val Val Ser Pro Ser Ser Ser
        515                 520                 525
gtc tcg tcg tat gag cag cac tac cac ctg cac cag ccg tcg tac cag     1632
Val Ser Ser Tyr Glu Gln His Tyr His Leu His Gln Pro Ser Tyr Gln
    530                 535                 540
cca tgg gtc gtc gtc gct gac gcc cac gag gac aac cac ccg tct aac     1680
Pro Trp Val Val Val Ala Asp Ala His Glu Asp Asn His Pro Ser Asn
545                 550                 555                 560
aag gcc agg tgc gct gcg gcg gcc gtt cag cta cac gtc gtc gac gat     1728
Lys Ala Arg Cys Ala Ala Ala Ala Val Gln Leu His Val Val Asp Asp
                565                 570                 575
gag gac gtg aag ctt ctg agc gcc gcg ata aag gtc aag tcc gac gac     1776
Glu Asp Val Lys Leu Leu Ser Ala Ala Ile Lys Val Lys Ser Asp Asp
            580                 585                 590
tcg agc gcg tcc aat aat agc tcg gtg gag tgc cgc tcc cgg ttc aag     1824
```

```
Ser Ser Ala Ser Asn Asn Ser Ser Val Glu Cys Arg Ser Arg Phe Lys
        595                 600                 605
gag ctc agc gcc gag aac ctt aag gtc ctc tgc tcc gcg ctg gag aaa      1872
Glu Leu Ser Ala Glu Asn Leu Lys Val Leu Cys Ser Ala Leu Glu Lys
    610                 615                 620
gag gtg cca tgg cag gcg gag atc gtg cct gag atc gcg agc acg gtc      1920
Glu Val Pro Trp Gln Ala Glu Ile Val Pro Glu Ile Ala Ser Thr Val
625                 630                 635                 640
ctg cag tgc cgt tcc ggc atg gcg agg agg cgc gac gcc gct gct tcg      1968
Leu Gln Cys Arg Ser Gly Met Ala Arg Arg Arg Asp Ala Ala Ala Ser
                645                 650                 655
agt tcg aga ccg ccg gcg ttc gcc aag gag gac act tgg atg ctc ttc      2016
Ser Ser Arg Pro Pro Ala Phe Ala Lys Glu Asp Thr Trp Met Leu Phe
            660                 665                 670
cac ggt ggc gac gcc gac ggc aag gtg agg gtg gcc agg gag ctg gcc      2064
His Gly Gly Asp Ala Asp Gly Lys Val Arg Val Ala Arg Glu Leu Ala
        675                 680                 685
cgc ctc gtc ttc ggc tcg cgc aag agc ttc gta tcc atc gcc ggc agc      2112
Arg Leu Val Phe Gly Ser Arg Lys Ser Phe Val Ser Ile Ala Gly Ser
    690                 695                 700
ggc acc acc gcg tcg tct ccg gcg cgg tcc gac gac tct tcc aag cag      2160
Gly Thr Thr Ala Ser Ser Pro Ala Arg Ser Asp Asp Ser Ser Lys Gln
705                 710                 715                 720
caa cgc aag cgg ccc cgg ttg atg gag gag gcg agc gac cac ggc tgt      2208
Gln Arg Lys Arg Pro Arg Leu Met Glu Glu Ala Ser Asp His Gly Cys
                725                 730                 735
cac gag agc ctc tac gag gct gta cgc gac aac ccg cac cgc gtc atc      2256
His Glu Ser Leu Tyr Glu Ala Val Arg Asp Asn Pro His Arg Val Ile
            740                 745                 750
ctg gta cag gac gtc gag cag ggt ggc tgg cgg tgc cag agg gac att      2304
Leu Val Gln Asp Val Glu Gln Gly Gly Trp Arg Cys Gln Arg Asp Ile
        755                 760                 765
ttg gag gcc atc caa aga ggc ttg gtc cgg agc cat gcc ggc ggc gac      2352
Leu Glu Ala Ile Gln Arg Gly Leu Val Arg Ser His Ala Gly Gly Asp
    770                 775                 780
gag gca gcg ctc ggc gac gcc atc gtc gtt ctg agc tgc cag agc ttt      2400
Glu Ala Ala Leu Gly Asp Ala Ile Val Val Leu Ser Cys Gln Ser Phe
785                 790                 795                 800
gac gcg tgg tcg aca acc tcc tcg cca cca acg acg acc aag aag gcg      2448
Asp Ala Trp Ser Thr Thr Ser Ser Pro Pro Thr Thr Thr Lys Lys Ala
                805                 810                 815
aag aca gag agc gag gag gag ccg aca ggg gaa gag agc gcc gcg gct      2496
Lys Thr Glu Ser Glu Glu Glu Pro Thr Gly Glu Glu Ser Ala Ala Ala
            820                 825                 830
gca tcg ccg tcg tct tca cct tgc ttt gat ctg aac atg gac gtt gag      2544
Ala Ser Pro Ser Ser Ser Pro Cys Phe Asp Leu Asn Met Asp Val Glu
        835                 840                 845
aac gat gac ttg ctg gag agt tgc ttc acc gat gct agt ctg ctc aag      2592
Asn Asp Asp Leu Leu Glu Ser Cys Phe Thr Asp Ala Ser Leu Leu Lys
    850                 855                 860
gca gtg gat cgg acg ttc ttc ttt aga cga ccc gat gag agg agt gat      2640
Ala Val Asp Arg Thr Phe Phe Phe Arg Arg Pro Asp Glu Arg Ser Asp
865                 870                 875                 880
tag                                                                  2643
```

<210> 36
<211> 880
<212> PRT
<213> Sorghum bicolor

<400> 36

```
Met Arg Ala Gly Gly Cys Thr Val Gln Gln Ala Leu Ala Pro Glu Ala
1               5                   10                  15
```

```
Ala Ala Val Val Lys Gln Ala Val Ser Leu Ala Arg Arg Arg Gly Asn
            20                  25                  30
Ala Gln Val Thr Pro Leu His Val Ala Ser Ala Met Leu His Gln Gln
        35                  40                  45
Val Val Ala Ala Ala Thr Gly Thr Gly Thr Gly Pro Ser Ser Ser Thr
    50                  55                  60
Ala Ala Gly Leu Leu Arg Ala Ala Cys Leu Gln Ser His Ser His Pro
65                  70                  75                  80
Leu Gln Cys Lys Ala Leu Glu Leu Cys Phe Asn Val Ala Leu Asn Arg
                85                  90                  95
Leu Pro Ala Ser Ala Ser Pro Leu Leu Gly Gly His Gly His Val Tyr
            100                 105                 110
Tyr Pro Pro Ser Leu Ser Asn Ala Leu Val Ala Ala Phe Lys Arg Ala
        115                 120                 125
Gln Ala His Gln Arg Arg Gly Ser Val Asp Thr Gln Gln Gln Pro Val
    130                 135                 140
Leu Ala Val Lys Ile Glu Leu Glu Gln Leu Val Ile Ser Ile Leu Asp
145                 150                 155                 160
Asp Pro Ser Val Ser Arg Val Met Arg Glu Ala Gly Phe Ser Ser Thr
                165                 170                 175
Gln Val Lys Ala Asn Val Glu Gln Ala Val Ser Ser Ile Glu Ala Asn
            180                 185                 190
Asn Ser Ala Ser Thr Asn Thr Ala Ala Ala Ala His Gln Asn Pro Asn
        195                 200                 205
Pro Arg Ala Ala Pro His Glu Glu Thr Met Pro Ser Lys Leu Gln Leu
    210                 215                 220
Pro Leu Asp Leu Asp Gln Val Arg Asp Glu Asp Val Ala Val Val Leu
225                 230                 235                 240
Asp Cys Leu Ala Ser Arg Ser Lys Lys Arg Val Met Val Val Ala Glu
                245                 250                 255
Cys Ala Ala Thr Ala Glu Ala Pro Thr Arg Ala Ala Val Glu Lys Ile
            260                 265                 270
Lys Arg Gly Glu Ala Leu Arg Gly Ala Gln Val Val Ser Leu Arg Val
        275                 280                 285
Ser Arg Phe Arg Asp Leu Pro Arg Asp Glu Ala Glu Arg Leu Leu Val
    290                 295                 300
Glu Leu Arg Cys Ala Val Lys Val Gly Gly Arg Ala Gly Gly Val Val
305                 310                 315                 320
Leu Val Val Glu Asp Leu Gly Trp Ala Ala Glu Phe Trp Ala Ala Arg
                325                 330                 335
Ala Glu Ser Gly Arg Ala Arg Trp Pro Ser Ser Cys Cys Tyr Tyr Cys
            340                 345                 350
Ala Val Glu His Ala Val Ala Glu Val Arg Ala Leu Ala Cys Arg Gly
        355                 360                 365
Gly Asp Gly Val Trp Leu Ile Gly Tyr Gly Thr Tyr Gln Ser Tyr Met
    370                 375                 380
Arg Cys Arg Ala Gly Gln Pro Ser Leu Glu Thr Leu Trp Gly Leu Gln
385                 390                 395                 400
Thr Leu Ala Val Pro Ala Gly Ser Leu Ala Leu Ser Leu Asn Phe Val
                405                 410                 415
Gly Asp Ser Ala Thr Ala Met Thr Ile Asn His Leu Ala Lys Cys Asp
            420                 425                 430
Asp Asp Arg Ser Gly Asn Asn Gly Ser Ala Pro Arg Cys Leu Ser Leu
        435                 440                 445
Leu Asp Ala Gly Gly Ser Gly His Leu Thr Ala Val Ser Ser Phe Phe
    450                 455                 460
Ala Asp Asp Cys Ser Ala Thr Ala Thr Lys Cys Glu Pro Ala Leu Lys
465                 470                 475                 480
Ser Ser Ile Pro Pro Trp Leu Gln His Cys Arg Asp Gln Asp Pro Ser
                485                 490                 495
Arg Cys Lys Lys Ser Ser Thr Cys Gly Gly Ser Leu Ser His His His
            500                 505                 510
Arg Thr Ala Leu Asn Phe Ser Thr Ala Val Val Ser Pro Ser Ser Ser
        515                 520                 525
Val Ser Ser Tyr Glu Gln His Tyr His Leu His Gln Pro Ser Tyr Gln
```

```
    530                 535                 540
Pro Trp Val Val Val Ala Asp Ala His Glu Asp Asn His Pro Ser Asn
545                 550                 555                 560
Lys Ala Arg Cys Ala Ala Ala Ala Val Gln Leu His Val Val Asp Asp
                565                 570                 575
Glu Asp Val Lys Leu Leu Ser Ala Ala Ile Lys Val Lys Ser Asp Asp
            580                 585                 590
Ser Ser Ala Ser Asn Asn Ser Ser Val Glu Cys Arg Ser Arg Phe Lys
        595                 600                 605
Glu Leu Ser Ala Glu Asn Leu Lys Val Leu Cys Ser Ala Leu Glu Lys
    610                 615                 620
Glu Val Pro Trp Gln Ala Glu Ile Val Pro Glu Ile Ala Ser Thr Val
625                 630                 635                 640
Leu Gln Cys Arg Ser Gly Met Ala Arg Arg Arg Asp Ala Ala Ala Ser
                645                 650                 655
Ser Ser Arg Pro Pro Ala Phe Ala Lys Glu Asp Thr Trp Met Leu Phe
            660                 665                 670
His Gly Gly Asp Ala Asp Gly Lys Val Arg Val Ala Arg Glu Leu Ala
        675                 680                 685
Arg Leu Val Phe Gly Ser Arg Lys Ser Phe Val Ser Ile Ala Gly Ser
    690                 695                 700
Gly Thr Thr Ala Ser Ser Pro Ala Arg Ser Asp Asp Ser Ser Lys Gln
705                 710                 715                 720
Gln Arg Lys Arg Pro Arg Leu Met Glu Glu Ala Ser Asp His Gly Cys
                725                 730                 735
His Glu Ser Leu Tyr Glu Ala Val Arg Asp Asn Pro His Arg Val Ile
            740                 745                 750
Leu Val Gln Asp Val Glu Gln Gly Gly Trp Arg Cys Gln Arg Asp Ile
        755                 760                 765
Leu Glu Ala Ile Gln Arg Gly Leu Val Arg Ser His Ala Gly Gly Asp
    770                 775                 780
Glu Ala Ala Leu Gly Asp Ala Ile Val Val Leu Ser Cys Gln Ser Phe
785                 790                 795                 800
Asp Ala Trp Ser Thr Thr Ser Ser Pro Pro Thr Thr Thr Lys Lys Ala
                805                 810                 815
Lys Thr Glu Ser Glu Glu Glu Pro Thr Gly Glu Glu Ser Ala Ala Ala
            820                 825                 830
Ala Ser Pro Ser Ser Ser Pro Cys Phe Asp Leu Asn Met Asp Val Glu
        835                 840                 845
Asn Asp Asp Leu Leu Glu Ser Cys Phe Thr Asp Ala Ser Leu Leu Lys
    850                 855                 860
Ala Val Asp Arg Thr Phe Phe Phe Arg Arg Pro Asp Glu Arg Ser Asp
865                 870                 875                 880

<210> 37
<211> 2652
<212> DNA
<213> Sorghum bicolor

<220>
<221> CDS
<222> (1)..(2652)

<400> 37
atg cga gcc ggc ggc tgc gcg gtg cag cag gcg ctg gcg gcg gag gcg       48
Met Arg Ala Gly Gly Cys Ala Val Gln Gln Ala Leu Ala Ala Glu Ala
  1               5                  10                  15
gcg gcc gtg gtg cgg cag gcc gtg gcc ctg gcc cgg agg cgc ggc cac       96
Ala Ala Val Val Arg Gln Ala Val Ala Leu Ala Arg Arg Arg Gly His
             20                  25                  30
gcg cag gtg aca ccg ctc cac gtc gcc agc gcc atg ctc tcg gcg gcg      144
Ala Gln Val Thr Pro Leu His Val Ala Ser Ala Met Leu Ser Ala Ala
         35                  40                  45
ggg ctc ctc cgc gcc gcc tgc ctc cgc tcc cac tcc cac ccg ctg cag      192
Gly Leu Leu Arg Ala Ala Cys Leu Arg Ser His Ser His Pro Leu Gln
```

```
        50                  55                  60
tgc aag gcg ctc gag ctc tgc ttc aac gtc gcg ctc aac cgc ctc ccc      240
Cys Lys Ala Leu Glu Leu Cys Phe Asn Val Ala Leu Asn Arg Leu Pro
65                  70                  75                  80
acc gca ggc ccc gcc gcc gcc gcc gtc atg ttc cac ccc cac cat cac      288
Thr Ala Gly Pro Ala Ala Ala Ala Val Met Phe His Pro His His His
                85                  90                  95
cac cac cac gcc ggg cac ggg cag cag cac gcc gtg ccc gtg ctg tcc      336
His His His Ala Gly His Gly Gln Gln His Ala Val Pro Val Leu Ser
            100                 105                 110
aac gcg ctc gtc gcc gcg ttc aag cgc gcg cag gcg cac cag cgc cgt      384
Asn Ala Leu Val Ala Ala Phe Lys Arg Ala Gln Ala His Gln Arg Arg
        115                 120                 125
ggc gtc gtg gaa gga gtg cag gga cag gga cag gga cag gca ccc gcg      432
Gly Val Val Glu Gly Val Gln Gly Gln Gly Gln Gly Gln Ala Pro Ala
    130                 135                 140
cag ccg ccg ccg cag ccg gtg ctc gcc gcc aag gtc gac atc gag cag      480
Gln Pro Pro Pro Gln Pro Val Leu Ala Ala Lys Val Asp Ile Glu Gln
145                 150                 155                 160
ctc atc atc tcc atc ctc gac gac cct agc gtg agc cgc gtc atg cgc      528
Leu Ile Ile Ser Ile Leu Asp Asp Pro Ser Val Ser Arg Val Met Arg
                165                 170                 175
gag gcc ggc ttc tcc agc tcc cag gtc aag gcc aac gtc gag aag gcc      576
Glu Ala Gly Phe Ser Ser Ser Gln Val Lys Ala Asn Val Glu Lys Ala
            180                 185                 190
gtc tcg ctc tcg tcg tcg tcg tcg tta ccc gac cat caa ccg aac acg      624
Val Ser Leu Ser Ser Ser Ser Ser Leu Pro Asp His Gln Pro Asn Thr
        195                 200                 205
acg ata cca ccc agc ggc gcc cac gcg acg ggc tct cct gca gca ggc      672
Thr Ile Pro Pro Ser Gly Ala His Ala Thr Gly Ser Pro Ala Ala Gly
    210                 215                 220
ggc tcc ggt cac gcg agc agc agg cgc ccc aac gcc ggc ggc aac aag      720
Gly Ser Gly His Ala Ser Ser Arg Arg Pro Asn Ala Gly Gly Asn Lys
225                 230                 235                 240
gcc gac gac gac gac gcc atg cgc gtg ctc gac tgc atg gcg agc ggg      768
Ala Asp Asp Asp Asp Ala Met Arg Val Leu Asp Cys Met Ala Ser Gly
                245                 250                 255
acc aag cgg tgc gtg gtg gtc gtc ggc gag agc gcg gcc acc gcg gag      816
Thr Lys Arg Cys Val Val Val Val Gly Glu Ser Ala Ala Thr Ala Glu
            260                 265                 270
gtg gtg gtg aag tcg gtg atg gac agg gtc agc aaa ggg gag ctc cag      864
Val Val Val Lys Ser Val Met Asp Arg Val Ser Lys Gly Glu Leu Gln
        275                 280                 285
cag cgg cac gag cgg ctc aag aac gtc cag ttc gtg ccc ctc tcg gcc      912
Gln Arg His Glu Arg Leu Lys Asn Val Gln Phe Val Pro Leu Ser Ala
    290                 295                 300
gcg tcc ttc cag cgg atg ccg agg gag gag gtg gag gcc aag gcc ggc      960
Ala Ser Phe Gln Arg Met Pro Arg Glu Glu Val Glu Ala Lys Ala Gly
305                 310                 315                 320
gac ctg cgc gcg ctc gtt cga cag ggc tgc gcc gcc ggg aag ggc gtc      1008
Asp Leu Arg Ala Leu Val Arg Gln Gly Cys Ala Ala Gly Lys Gly Val
                325                 330                 335
gtg ctc gtc ctc gag gac ctc gcg ttc gcc gcc gac gcc tgg gcc gcc      1056
Val Leu Val Leu Glu Asp Leu Ala Phe Ala Ala Asp Ala Trp Ala Ala
            340                 345                 350
gtg tcg gag agg aga agg cac ggc agt gtt ggt cgt gaa cac ggt cag      1104
Val Ser Glu Arg Arg Arg His Gly Ser Val Gly Arg Glu His Gly Gln
        355                 360                 365
tgc ggc tac tgc ccc gtc gag cac gcc gtc atg gaa gtg ggc agc ttg      1152
Cys Gly Tyr Cys Pro Val Glu His Ala Val Met Glu Val Gly Ser Leu
    370                 375                 380
gtg tcc gcc gcc gcc ggt ggc ggt ggc ggt ggc aga ggc ctc gac agg      1200
Val Ser Ala Ala Ala Gly Gly Gly Gly Gly Gly Arg Gly Leu Asp Arg
385                 390                 395                 400
ttc tgg ctg cta ggg ttc ggg aac aac cag gct tac atg aag tcc aga      1248
```

```
Phe Trp Leu Leu Gly Phe Gly Asn Asn Gln Ala Tyr Met Lys Ser Arg
                405                 410                 415
gca ggg cag cca tct ctt gag gcc gtc tgg gag ctg cac ccc gtc gtc      1296
Ala Gly Gln Pro Ser Leu Glu Ala Val Trp Glu Leu His Pro Val Val
            420                 425                 430
gtg ccg gac ggc ggc ctc tcg ttg agc ctc acc tgc aac agt gac gct      1344
Val Pro Asp Gly Gly Leu Ser Leu Ser Leu Thr Cys Asn Ser Asp Ala
        435                 440                 445
gaa caa gct aat caa gat aga gca aca agg cct tgg ccc tcc ttc gtt      1392
Glu Gln Ala Asn Gln Asp Arg Ala Thr Arg Pro Trp Pro Ser Phe Val
    450                 455                 460
aac ggc act gct agc ggc gag agt gag ctc atc act tcc tcc acc aaa      1440
Asn Gly Thr Ala Ser Gly Glu Ser Glu Leu Ile Thr Ser Ser Thr Lys
465                 470                 475                 480
gtg gcg gcg aca act ccg agt gtg ccg cca tgg ttt cgg ggg tac caa      1488
Val Ala Ala Thr Thr Pro Ser Val Pro Pro Trp Phe Arg Gly Tyr Gln
                485                 490                 495
gtt caa gat atg aca aga cca gca agc cgc agc gcc agt ttt cag ttg      1536
Val Gln Asp Met Thr Arg Pro Ala Ser Arg Ser Ala Ser Phe Gln Leu
            500                 505                 510
caa gat tgg aac cac aat ttt aat gga tca cca gca tac cac acg tcg      1584
Gln Asp Trp Asn His Asn Phe Asn Gly Ser Pro Ala Tyr His Thr Ser
        515                 520                 525
gag ctc aca ctg agc ttc tcc tca cag gct act aac tct cca gac gct      1632
Glu Leu Thr Leu Ser Phe Ser Ser Gln Ala Thr Asn Ser Pro Asp Ala
    530                 535                 540
tct tcc atc tcc agc tcc ttt gcc cca agc ttc agt gct gcc agc ttg      1680
Ser Ser Ile Ser Ser Ser Phe Ala Pro Ser Phe Ser Ala Ala Ser Leu
545                 550                 555                 560
atg atg agc tcc gag cca tgg caa ttc aag gtg atg caa cct tgc cca      1728
Met Met Ser Ser Glu Pro Trp Gln Phe Lys Val Met Gln Pro Cys Pro
                565                 570                 575
aac tat cga cgt gat gat cca aac gca ttg gtc agg acc tat gat cat      1776
Asn Tyr Arg Arg Asp Asp Pro Asn Ala Leu Val Arg Thr Tyr Asp His
            580                 585                 590
caa caa ttg cat gga agg cct agt cca gaa aaa tcc tac tcg gtg tcc      1824
Gln Gln Leu His Gly Arg Pro Ser Pro Glu Lys Ser Tyr Ser Val Ser
        595                 600                 605
aac tca tct gaa ggt gcc cca gct gag tcg cca aag ttc acg gag ctc      1872
Asn Ser Ser Glu Gly Ala Pro Ala Glu Ser Pro Lys Phe Thr Glu Leu
    610                 615                 620
acc gct gag aat ctc aag atc ctc tgc aac gcg ctt gag aat cgg gct      1920
Thr Ala Glu Asn Leu Lys Ile Leu Cys Asn Ala Leu Glu Asn Arg Ala
625                 630                 635                 640
cca cat cac aag gac gtg gct aca gag att gca agt gtt gtg ctc cag      1968
Pro His His Lys Asp Val Ala Thr Glu Ile Ala Ser Val Val Leu Gln
                645                 650                 655
tgc cgc tcg ggc atg aca agg agg agg tgg tgg ttc caa gag aag cca      2016
Cys Arg Ser Gly Met Thr Arg Arg Arg Trp Trp Phe Gln Glu Lys Pro
            660                 665                 670
agt gca gtg aca tgg ttg ctc ttc caa gga ggt ggc aat gat ggc aag      2064
Ser Ala Val Thr Trp Leu Leu Phe Gln Gly Gly Gly Asn Asp Gly Lys
        675                 680                 685
aaa gca gtg tgt cag gag ctt gca agg ctt gtc ttt ggc tcc tac agc      2112
Lys Ala Val Cys Gln Glu Leu Ala Arg Leu Val Phe Gly Ser Tyr Ser
    690                 695                 700
aag ttc acc tcc atc tca cta gct gat gat gag ttc act cat cat gtt      2160
Lys Phe Thr Ser Ile Ser Leu Ala Asp Asp Glu Phe Thr His His Val
705                 710                 715                 720
cac tcc gat tcc agc tcc ggc gag ccc atg ttg aag agg caa aga tca      2208
His Ser Asp Ser Ser Ser Gly Glu Pro Met Leu Lys Arg Gln Arg Ser
                725                 730                 735
ctg gac act ggg cat ggc tac att cag aga ttg tac gat gcg ata ctc      2256
Leu Asp Thr Gly His Gly Tyr Ile Gln Arg Leu Tyr Asp Ala Ile Leu
            740                 745                 750
```

```
aaa agc cct cat cgg gtg ata atg atc gac ggt gtt gag caa ctg gac       2304
Lys Ser Pro His Arg Val Ile Met Ile Asp Gly Val Glu Gln Leu Asp
        755                 760                 765
tat gag tcg gag atc ggc atc agg aat gca atc aca aat gga aga att       2352
Tyr Glu Ser Glu Ile Gly Ile Arg Asn Ala Ile Thr Asn Gly Arg Ile
    770                 775                 780
agg ggt tgc aat ggt gat gag atc agt ttg ggg gat gcc atc att gta       2400
Arg Gly Cys Asn Gly Asp Glu Ile Ser Leu Gly Asp Ala Ile Ile Val
785                 790                 795                 800
ttg agt tgt gaa gca ctc gat tca atg tct aac gct tcc tct cct cgg       2448
Leu Ser Cys Glu Ala Leu Asp Ser Met Ser Asn Ala Ser Ser Pro Arg
                805                 810                 815
ctc aag caa agg gtc atc aat aag aat gga aag gaa gga aat ggc atg       2496
Leu Lys Gln Arg Val Ile Asn Lys Asn Gly Lys Glu Gly Asn Gly Met
            820                 825                 830
aac ata gag aat ggg atg gag tca tct ggt ttt acc ttg gat ttg aat       2544
Asn Ile Glu Asn Gly Met Glu Ser Ser Gly Phe Thr Leu Asp Leu Asn
        835                 840                 845
gca tgc gcc gag gat agt gaa ggg aat gaa gag agt gtt tca gat aat       2592
Ala Cys Ala Glu Asp Ser Glu Gly Asn Glu Glu Ser Val Ser Asp Asn
    850                 855                 860
gcg agg atc att aat att gtg gat ggt gtg ttc ttc ttc caa tta atg       2640
Ala Arg Ile Ile Asn Ile Val Asp Gly Val Phe Phe Phe Gln Leu Met
865                 870                 875                 880
gag cac tca tga                                                       2652
Glu His Ser


<210> 38
<211> 883
<212> PRT
<213> Sorghum bicolor

<400> 38
Met Arg Ala Gly Gly Cys Ala Val Gln Gln Ala Leu Ala Ala Glu Ala
1               5                   10                  15
Ala Ala Val Val Arg Gln Ala Val Ala Leu Ala Arg Arg Arg Gly His
            20                  25                  30
Ala Gln Val Thr Pro Leu His Val Ala Ser Ala Met Leu Ser Ala Ala
        35                  40                  45
Gly Leu Leu Arg Ala Ala Cys Leu Arg Ser His Ser His Pro Leu Gln
    50                  55                  60
Cys Lys Ala Leu Glu Leu Cys Phe Asn Val Ala Leu Asn Arg Leu Pro
65                  70                  75                  80
Thr Ala Gly Pro Ala Ala Ala Ala Val Met Phe His Pro His His His
                85                  90                  95
His His His Ala Gly His Gly Gln Gln His Ala Val Pro Val Leu Ser
            100                 105                 110
Asn Ala Leu Val Ala Ala Phe Lys Arg Ala Gln Ala His Gln Arg Arg
        115                 120                 125
Gly Val Val Glu Gly Val Gln Gly Gln Gly Gln Gly Gln Ala Pro Ala
    130                 135                 140
Gln Pro Pro Pro Gln Pro Val Leu Ala Ala Lys Val Asp Ile Glu Gln
145                 150                 155                 160
Leu Ile Ile Ser Ile Leu Asp Asp Pro Ser Val Ser Arg Val Met Arg
                165                 170                 175
Glu Ala Gly Phe Ser Ser Ser Gln Val Lys Ala Asn Val Glu Lys Ala
            180                 185                 190
Val Ser Leu Ser Ser Ser Ser Ser Leu Pro Asp His Gln Pro Asn Thr
        195                 200                 205
Thr Ile Pro Pro Ser Gly Ala His Ala Thr Gly Ser Pro Ala Ala Gly
    210                 215                 220
Gly Ser Gly His Ala Ser Ser Arg Arg Pro Asn Ala Gly Gly Asn Lys
225                 230                 235                 240
Ala Asp Asp Asp Asp Ala Met Arg Val Leu Asp Cys Met Ala Ser Gly
```

```
                245                 250                 255
Thr Lys Arg Cys Val Val Val Val Gly Glu Ser Ala Ala Thr Ala Glu
            260                 265                 270
Val Val Val Lys Ser Val Met Asp Arg Val Ser Lys Gly Glu Leu Gln
        275                 280                 285
Gln Arg His Glu Arg Leu Lys Asn Val Gln Phe Val Pro Leu Ser Ala
    290                 295                 300
Ala Ser Phe Gln Arg Met Pro Arg Glu Glu Val Glu Ala Lys Ala Gly
305                 310                 315                 320
Asp Leu Arg Ala Leu Val Arg Gln Gly Cys Ala Ala Gly Lys Gly Val
                325                 330                 335
Val Leu Val Leu Glu Asp Leu Ala Phe Ala Ala Asp Ala Trp Ala Ala
            340                 345                 350
Val Ser Glu Arg Arg Arg His Gly Ser Val Gly Arg Glu His Gly Gln
        355                 360                 365
Cys Gly Tyr Cys Pro Val Glu His Ala Val Met Glu Val Gly Ser Leu
    370                 375                 380
Val Ser Ala Ala Ala Gly Gly Gly Gly Gly Gly Arg Gly Leu Asp Arg
385                 390                 395                 400
Phe Trp Leu Leu Gly Phe Gly Asn Asn Gln Ala Tyr Met Lys Ser Arg
                405                 410                 415
Ala Gly Gln Pro Ser Leu Glu Ala Val Trp Glu Leu His Pro Val Val
            420                 425                 430
Val Pro Asp Gly Gly Leu Ser Leu Ser Leu Thr Cys Asn Ser Asp Ala
        435                 440                 445
Glu Gln Ala Asn Gln Asp Arg Ala Thr Arg Pro Trp Pro Ser Phe Val
    450                 455                 460
Asn Gly Thr Ala Ser Gly Glu Ser Glu Leu Ile Thr Ser Ser Thr Lys
465                 470                 475                 480
Val Ala Ala Thr Thr Pro Ser Val Pro Pro Trp Phe Arg Gly Tyr Gln
                485                 490                 495
Val Gln Asp Met Thr Arg Pro Ala Ser Arg Ser Ala Ser Phe Gln Leu
            500                 505                 510
Gln Asp Trp Asn His Asn Phe Asn Gly Ser Pro Ala Tyr His Thr Ser
        515                 520                 525
Glu Leu Thr Leu Ser Phe Ser Ser Gln Ala Thr Asn Ser Pro Asp Ala
    530                 535                 540
Ser Ser Ile Ser Ser Ser Phe Ala Pro Ser Phe Ser Ala Ala Ser Leu
545                 550                 555                 560
Met Met Ser Ser Glu Pro Trp Gln Phe Lys Val Met Gln Pro Cys Pro
                565                 570                 575
Asn Tyr Arg Arg Asp Asp Pro Asn Ala Leu Val Arg Thr Tyr Asp His
            580                 585                 590
Gln Gln Leu His Gly Arg Pro Ser Pro Glu Lys Ser Tyr Ser Val Ser
        595                 600                 605
Asn Ser Ser Glu Gly Ala Pro Ala Glu Ser Pro Lys Phe Thr Glu Leu
    610                 615                 620
Thr Ala Glu Asn Leu Lys Ile Leu Cys Asn Ala Leu Glu Asn Arg Ala
625                 630                 635                 640
Pro His His Lys Asp Val Ala Thr Glu Ile Ala Ser Val Val Leu Gln
                645                 650                 655
Cys Arg Ser Gly Met Thr Arg Arg Arg Trp Trp Phe Gln Glu Lys Pro
            660                 665                 670
Ser Ala Val Thr Trp Leu Leu Phe Gln Gly Gly Gly Asn Asp Gly Lys
        675                 680                 685
Lys Ala Val Cys Gln Glu Leu Ala Arg Leu Val Phe Gly Ser Tyr Ser
    690                 695                 700
Lys Phe Thr Ser Ile Ser Leu Ala Asp Asp Glu Phe Thr His His Val
705                 710                 715                 720
His Ser Asp Ser Ser Ser Gly Glu Pro Met Leu Lys Arg Gln Arg Ser
                725                 730                 735
Leu Asp Thr Gly His Gly Tyr Ile Gln Arg Leu Tyr Asp Ala Ile Leu
            740                 745                 750
Lys Ser Pro His Arg Val Ile Met Ile Asp Gly Val Glu Gln Leu Asp
        755                 760                 765
```

```
Tyr Glu Ser Glu Ile Gly Ile Arg Asn Ala Ile Thr Asn Gly Arg Ile
    770                 775                 780
Arg Gly Cys Asn Gly Asp Glu Ile Ser Leu Gly Asp Ala Ile Ile Val
785                 790                 795                 800
Leu Ser Cys Glu Ala Leu Asp Ser Met Ser Asn Ala Ser Ser Pro Arg
                805                 810                 815
Leu Lys Gln Arg Val Ile Asn Lys Asn Gly Lys Glu Gly Asn Gly Met
            820                 825                 830
Asn Ile Glu Asn Gly Met Glu Ser Ser Gly Phe Thr Leu Asp Leu Asn
        835                 840                 845
Ala Cys Ala Glu Asp Ser Glu Gly Asn Glu Glu Ser Val Ser Asp Asn
    850                 855                 860
Ala Arg Ile Ile Asn Ile Val Asp Gly Val Phe Phe Phe Gln Leu Met
865                 870                 875                 880
Glu His Ser


<210> 39
<211> 2625
<212> DNA
<213> Sorghum bicolor

<220>
<221> CDS
<222> (1)..(2625)

<400> 39
atg agg gcc ggg ggg tgc acg gtg cag cag tcg ctg acg gcg gag gcc       48
Met Arg Ala Gly Gly Cys Thr Val Gln Gln Ser Leu Thr Ala Glu Ala
1               5                   10                  15
gcg gcg gtg gtg aag cag gcg gtg agc ctc gcg cgg cgg cgc ggg aac       96
Ala Ala Val Val Lys Gln Ala Val Ser Leu Ala Arg Arg Arg Gly Asn
            20                  25                  30
gcg cag gtg acg ccg ctg cac gtg gcg agc gcg atg ctg gcg gcg ccg      144
Ala Gln Val Thr Pro Leu His Val Ala Ser Ala Met Leu Ala Ala Pro
        35                  40                  45
gcg ggc ctg ctg cgc gcg gcg tgc ctc cgc tcg cac tcc cac ccg ctg      192
Ala Gly Leu Leu Arg Ala Ala Cys Leu Arg Ser His Ser His Pro Leu
    50                  55                  60
cag tgc aag gcg ctg gag ctc tgc ttc aac gtc gcg ctc aac cgc ctc      240
Gln Cys Lys Ala Leu Glu Leu Cys Phe Asn Val Ala Leu Asn Arg Leu
65                  70                  75                  80
ccc gcg tcg gcc gcg gtc gcc tcg tcg ccg ctg ctc ggc gga cac ggg      288
Pro Ala Ser Ala Ala Val Ala Ser Ser Pro Leu Leu Gly Gly His Gly
                85                  90                  95
cac ggc cac cac cac tac tac ccg ccg tcg ctg tcc aac gcg ctc gtc      336
His Gly His His His Tyr Tyr Pro Pro Ser Leu Ser Asn Ala Leu Val
            100                 105                 110
gcg gcg ttc aag cgc gcg cag gcg cac cag cgg cgc ggc tcc gtc gag      384
Ala Ala Phe Lys Arg Ala Gln Ala His Gln Arg Arg Gly Ser Val Glu
        115                 120                 125
agc cag cag cag ccc gtg ctc gcc gtc aag atc gag ctg gag cag ctc      432
Ser Gln Gln Gln Pro Val Leu Ala Val Lys Ile Glu Leu Glu Gln Leu
    130                 135                 140
gtc gtc tcc atc ctc gac gac ccc agc gtc agc cgc gtg atg cgc gag      480
Val Val Ser Ile Leu Asp Asp Pro Ser Val Ser Arg Val Met Arg Glu
145                 150                 155                 160
gct ggc ttc tcc agc acc cag gtc aag gcc aac gtg gag cag gcc gtg      528
Ala Gly Phe Ser Ser Thr Gln Val Lys Ala Asn Val Glu Gln Ala Val
                165                 170                 175
tgc agc aca aca acg acc acc gcg gcc acg gcc gcc gct cca ggc aaa      576
Cys Ser Thr Thr Thr Thr Thr Ala Ala Thr Ala Ala Ala Pro Gly Lys
            180                 185                 190
aac cct aac cct agc agc tct gcc acc acg agc cca aca gct cag gaa      624
Asn Pro Asn Pro Ser Ser Ser Ala Thr Thr Ser Pro Thr Ala Gln Glu
```

```
        195                 200                 205
gcc aaa gcc atc aac aag ctg ccg ctg ccg ctc ccg ctc cac caa gcg      672
Ala Lys Ala Ile Asn Lys Leu Pro Leu Pro Leu Pro Leu His Gln Ala
    210                 215                 220
cgc gac gag gac gtg gcc gcc atc ctg gac tgc ttg gcg tcc cgg agc      720
Arg Asp Glu Asp Val Ala Ala Ile Leu Asp Cys Leu Ala Ser Arg Ser
225                 230                 235                 240
aag agg agg gtc gtc gtc atc gcg gag agc gtg tcc gcg gcc gag gcc      768
Lys Arg Arg Val Val Val Ile Ala Glu Ser Val Ser Ala Ala Glu Ala
                245                 250                 255
atg gcg cat gcg gcc gtg gac aag atc aag aga gcc gag gcg aag cac      816
Met Ala His Ala Ala Val Asp Lys Ile Lys Arg Ala Glu Ala Lys His
            260                 265                 270
gac gcg ctg cgt ggc gcg cag gtc gtc agc ctc cgc gtg tcg tcg ttc      864
Asp Ala Leu Arg Gly Ala Gln Val Val Ser Leu Arg Val Ser Ser Phe
        275                 280                 285
cgc gac atg ccg aga gag gag acc gag cgg cgg ctc ggc gag ctg cgg      912
Arg Asp Met Pro Arg Glu Glu Thr Glu Arg Arg Leu Gly Glu Leu Arg
    290                 295                 300
tgc ctc gtc agg ggc agg agg cag cag gag gtc gtg ctc gtc gtg gag      960
Cys Leu Val Arg Gly Arg Arg Gln Gln Glu Val Val Leu Val Val Glu
305                 310                 315                 320
gac ctc aag tgg gcg gcc gag ttc tgg gcg ggc cac gtc cag agc ggg      1008
Asp Leu Lys Trp Ala Ala Glu Phe Trp Ala Gly His Val Gln Ser Gly
                325                 330                 335
agg aga ggg tac tac agc tcc gtc gag cac gtc gtc acg gag ctg cgc      1056
Arg Arg Gly Tyr Tyr Ser Ser Val Glu His Val Val Thr Glu Leu Arg
            340                 345                 350
gcc ctg ctg gcg agc ggc ggc ggc ggc gac cac ggc ggc ggc agc atg      1104
Ala Leu Leu Ala Ser Gly Gly Gly Gly Asp His Gly Gly Gly Ser Met
        355                 360                 365
tgc tgg ctc ctc ggg ttc ggg acg tac cag gcc tac atg agg tgt cgg      1152
Cys Trp Leu Leu Gly Phe Gly Thr Tyr Gln Ala Tyr Met Arg Cys Arg
    370                 375                 380
gtc ggg cag ccg tcg ctg gag agc ctg tgg ggg ctc cag acg ctc acc      1200
Val Gly Gln Pro Ser Leu Glu Ser Leu Trp Gly Leu Gln Thr Leu Thr
385                 390                 395                 400
gtg ccc gcc ggc agc ctg gtg ctg agc ctc acc tgc gcc ttc gac gac      1248
Val Pro Ala Gly Ser Leu Val Leu Ser Leu Thr Cys Ala Phe Asp Asp
                405                 410                 415
agt gct cta ggc acg gtc aat cag tcc atg aaa gcc ggc tct gac acg      1296
Ser Ala Leu Gly Thr Val Asn Gln Ser Met Lys Ala Gly Ser Asp Thr
            420                 425                 430
gat ggg aac gca ccg gcg tct tgc tgg ccg ctt ttg ggc ggc acc cag      1344
Asp Gly Asn Ala Pro Ala Ser Cys Trp Pro Leu Leu Gly Gly Thr Gln
        435                 440                 445
ctc att tcc aga tgc tgc gcc gat tgc tct gcc gcg agg atc gac acg      1392
Leu Ile Ser Arg Cys Cys Ala Asp Cys Ser Ala Ala Arg Ile Asp Thr
    450                 455                 460
aaa gcg gcg ttg cct cgg ccc ttc gtc tcg tcg tcg tcc acc ctc cct      1440
Lys Ala Ala Leu Pro Arg Pro Phe Val Ser Ser Ser Ser Thr Leu Pro
465                 470                 475                 480
tcc tgg ctc cag cat tgc cgt gat cat cag gag cct act acg acc cat      1488
Ser Trp Leu Gln His Cys Arg Asp His Gln Glu Pro Thr Thr Thr His
                485                 490                 495
ctt acg gac ctt ggc aag aca tgg agc tcc atc tgc agc agg ccg tcg      1536
Leu Thr Asp Leu Gly Lys Thr Trp Ser Ser Ile Cys Ser Arg Pro Ser
            500                 505                 510
tca cag cgg atg acg ctg cat ttc tcc gcg ccg gtg tct ccg gcg tcc      1584
Ser Gln Arg Met Thr Leu His Phe Ser Ala Pro Val Ser Pro Ala Ser
        515                 520                 525
tcc atc tct tcc tat gag cac ggc ggc gat cat cat cat cag tcg cag      1632
Ser Ile Ser Ser Tyr Glu His Gly Gly Asp His His His Gln Ser Gln
    530                 535                 540
cag ccg cgg cac tcg tcg tgg ctc ctc gct ggt ctc gac gcc gcc gcg      1680
```

```
Gln Pro Arg His Ser Ser Trp Leu Leu Ala Gly Leu Asp Ala Ala Ala
545                 550                 555                 560
ccc gcg cac cac cac ccg tgg aga ccc aag cgc gag gcc agc ggc ggg        1728
Pro Ala His His His Pro Trp Arg Pro Lys Arg Glu Ala Ser Gly Gly
                565                 570                 575
aat aag gcc gcc gcc agc agg tcc cac gac tcc ggc ggc tcg aac ggc        1776
Asn Lys Ala Ala Ala Ser Arg Ser His Asp Ser Gly Gly Ser Asn Gly
            580                 585                 590
tcc gtg gag gtg gag tgc cgc cgc gcc aag gcc aag ttc aag gag ctc        1824
Ser Val Glu Val Glu Cys Arg Arg Ala Lys Ala Lys Phe Lys Glu Leu
        595                 600                 605
agc gcc gag aac ctc aag gtg ctc tgc ggc gcg ctg gag aaa gaa gtg        1872
Ser Ala Glu Asn Leu Lys Val Leu Cys Gly Ala Leu Glu Lys Glu Val
    610                 615                 620
ccg tgg cag aag gag atc gtc ccg gag atc gcc agc gcc gtc ctg cag        1920
Pro Trp Gln Lys Glu Ile Val Pro Glu Ile Ala Ser Ala Val Leu Gln
625                 630                 635                 640
tgc cgg tcg ggg atc gcc aag cgg cgc gac aag tcg agg tcg gcg gac        1968
Cys Arg Ser Gly Ile Ala Lys Arg Arg Asp Lys Ser Arg Ser Ala Asp
                645                 650                 655
gcc aag gag gag acg tgg atg ttc ttc ctc ggc ggc gac gcc gac ggc        2016
Ala Lys Glu Glu Thr Trp Met Phe Phe Leu Gly Gly Asp Ala Asp Gly
            660                 665                 670
aag gag aag gtg gcg agg gag ctt gct agt ctc gtc ttc ggg tca cgc        2064
Lys Glu Lys Val Ala Arg Glu Leu Ala Ser Leu Val Phe Gly Ser Arg
        675                 680                 685
aac agc ttc gta tcc atc agg cct ggt ggg ggt gcc tct gcc tcg tcg        2112
Asn Ser Phe Val Ser Ile Arg Pro Gly Gly Gly Ala Ser Ala Ser Ser
    690                 695                 700
ccg ccg ccg cct gcc gcc gcc tcg tcc gag gag cac cac cgg agt aag        2160
Pro Pro Pro Pro Ala Ala Ala Ser Ser Glu Glu His His Arg Ser Lys
705                 710                 715                 720
cgg cca cgg atg gcg gcg gcc tac cta gaa cgg ttg cac gag gct gtc        2208
Arg Pro Arg Met Ala Ala Ala Tyr Leu Glu Arg Leu His Glu Ala Val
                725                 730                 735
tcc gag aac cca cac cgg gtc ata ttc atg gag gac gtc gag cgg gct        2256
Ser Glu Asn Pro His Arg Val Ile Phe Met Glu Asp Val Glu Arg Ala
            740                 745                 750
gac cgg gac tgc cag ctc ggc atc aag gag gcg atc gag agc ggg gtc        2304
Asp Arg Asp Cys Gln Leu Gly Ile Lys Glu Ala Ile Glu Ser Gly Val
        755                 760                 765
gtg cgg aac cat gct ggc gag gag gtc ggc gtg ggc gat gcc atc gtc        2352
Val Arg Asn His Ala Gly Glu Glu Val Gly Val Gly Asp Ala Ile Val
    770                 775                 780
att ctc agc tgc gag agc ttt gac ggc gac agc agg tct aga ggt tgc        2400
Ile Leu Ser Cys Glu Ser Phe Asp Gly Asp Ser Arg Ser Arg Gly Cys
785                 790                 795                 800
tcg ccg ccg agc aag aag gtg aag gta gag atg gag gag acc aag gag        2448
Ser Pro Pro Ser Lys Lys Val Lys Val Glu Met Glu Glu Thr Lys Glu
                805                 810                 815
gag cgc aca ggt gaa cat gaa cac aac gaa gat ggc gct tcc tcg tcg        2496
Glu Arg Thr Gly Glu His Glu His Asn Glu Asp Gly Ala Ser Ser Ser
            820                 825                 830
tct ccg tcg tgc atc gat ttg aac gtg gac atg gag agt gat ccg gcg        2544
Ser Pro Ser Cys Ile Asp Leu Asn Val Asp Met Glu Ser Asp Pro Ala
        835                 840                 845
gac gag cga agt ctc ggt gat ctc tgt ctg ctc acg gcc gtc gac agg        2592
Asp Glu Arg Ser Leu Gly Asp Leu Cys Leu Leu Thr Ala Val Asp Arg
    850                 855                 860
acc cta ttc ttc aga aga caa caa gag aat tag                            2625
Thr Leu Phe Phe Arg Arg Gln Gln Glu Asn
865                 870
```

<210> 40
<211> 874

<212> PRT
<213> Sorghum bicolor

<400> 40

```
Met Arg Ala Gly Gly Cys Thr Val Gln Gln Ser Leu Thr Ala Glu Ala
1               5                   10                  15
Ala Ala Val Val Lys Gln Ala Val Ser Leu Ala Arg Arg Arg Gly Asn
            20                  25                  30
Ala Gln Val Thr Pro Leu His Val Ala Ser Ala Met Leu Ala Ala Pro
        35                  40                  45
Ala Gly Leu Leu Arg Ala Ala Cys Leu Arg Ser His Ser His Pro Leu
    50                  55                  60
Gln Cys Lys Ala Leu Glu Leu Cys Phe Asn Val Ala Leu Asn Arg Leu
65                  70                  75                  80
Pro Ala Ser Ala Ala Val Ala Ser Ser Pro Leu Leu Gly Gly His Gly
                85                  90                  95
His Gly His His His Tyr Tyr Pro Pro Ser Leu Ser Asn Ala Leu Val
            100                 105                 110
Ala Ala Phe Lys Arg Ala Gln Ala His Gln Arg Arg Gly Ser Val Glu
        115                 120                 125
Ser Gln Gln Gln Pro Val Leu Ala Val Lys Ile Glu Leu Glu Gln Leu
    130                 135                 140
Val Val Ser Ile Leu Asp Asp Pro Ser Val Ser Arg Val Met Arg Glu
145                 150                 155                 160
Ala Gly Phe Ser Ser Thr Gln Val Lys Ala Asn Val Glu Gln Ala Val
                165                 170                 175
Cys Ser Thr Thr Thr Thr Thr Ala Ala Thr Ala Ala Ala Pro Gly Lys
            180                 185                 190
Asn Pro Asn Pro Ser Ser Ser Ala Thr Thr Ser Pro Thr Ala Gln Glu
        195                 200                 205
Ala Lys Ala Ile Asn Lys Leu Pro Leu Pro Leu Pro Leu His Gln Ala
    210                 215                 220
Arg Asp Glu Asp Val Ala Ala Ile Leu Asp Cys Leu Ala Ser Arg Ser
225                 230                 235                 240
Lys Arg Arg Val Val Val Ile Ala Glu Ser Val Ser Ala Ala Glu Ala
                245                 250                 255
Met Ala His Ala Ala Val Asp Lys Ile Lys Arg Ala Glu Ala Lys His
            260                 265                 270
Asp Ala Leu Arg Gly Ala Gln Val Val Ser Leu Arg Val Ser Ser Phe
        275                 280                 285
Arg Asp Met Pro Arg Glu Glu Thr Glu Arg Arg Leu Gly Glu Leu Arg
    290                 295                 300
Cys Leu Val Arg Gly Arg Arg Gln Gln Glu Val Val Leu Val Val Glu
305                 310                 315                 320
Asp Leu Lys Trp Ala Ala Glu Phe Trp Ala Gly His Val Gln Ser Gly
                325                 330                 335
Arg Arg Gly Tyr Tyr Ser Ser Val Glu His Val Val Thr Glu Leu Arg
            340                 345                 350
Ala Leu Leu Ala Ser Gly Gly Gly Gly Asp His Gly Gly Gly Ser Met
        355                 360                 365
Cys Trp Leu Leu Gly Phe Gly Thr Tyr Gln Ala Tyr Met Arg Cys Arg
    370                 375                 380
Val Gly Gln Pro Ser Leu Glu Ser Leu Trp Gly Leu Gln Thr Leu Thr
385                 390                 395                 400
Val Pro Ala Gly Ser Leu Val Leu Ser Leu Thr Cys Ala Phe Asp Asp
                405                 410                 415
Ser Ala Leu Gly Thr Val Asn Gln Ser Met Lys Ala Gly Ser Asp Thr
            420                 425                 430
Asp Gly Asn Ala Pro Ala Ser Cys Trp Pro Leu Leu Gly Gly Thr Gln
        435                 440                 445
Leu Ile Ser Arg Cys Cys Ala Asp Cys Ser Ala Ala Arg Ile Asp Thr
    450                 455                 460
Lys Ala Ala Leu Pro Arg Pro Phe Val Ser Ser Ser Ser Thr Leu Pro
465                 470                 475                 480
Ser Trp Leu Gln His Cys Arg Asp His Gln Glu Pro Thr Thr Thr His
```

```
                485                 490                 495
Leu Thr Asp Leu Gly Lys Thr Trp Ser Ser Ile Cys Ser Arg Pro Ser
            500                 505                 510
Ser Gln Arg Met Thr Leu His Phe Ser Ala Pro Val Ser Pro Ala Ser
        515                 520                 525
Ser Ile Ser Ser Tyr Glu His Gly Gly Asp His His His Gln Ser Gln
    530                 535                 540
Gln Pro Arg His Ser Ser Trp Leu Leu Ala Gly Leu Asp Ala Ala Ala
545                 550                 555                 560
Pro Ala His His His Pro Trp Arg Pro Lys Arg Glu Ala Ser Gly Gly
                565                 570                 575
Asn Lys Ala Ala Ala Ser Arg Ser His Asp Ser Gly Gly Ser Asn Gly
            580                 585                 590
Ser Val Glu Val Glu Cys Arg Arg Ala Lys Ala Lys Phe Lys Glu Leu
        595                 600                 605
Ser Ala Glu Asn Leu Lys Val Leu Cys Gly Ala Leu Glu Lys Glu Val
    610                 615                 620
Pro Trp Gln Lys Glu Ile Val Pro Glu Ile Ala Ser Ala Val Leu Gln
625                 630                 635                 640
Cys Arg Ser Gly Ile Ala Lys Arg Arg Asp Lys Ser Arg Ser Ala Asp
                645                 650                 655
Ala Lys Glu Glu Thr Trp Met Phe Phe Leu Gly Gly Asp Ala Asp Gly
            660                 665                 670
Lys Glu Lys Val Ala Arg Glu Leu Ala Ser Leu Val Phe Gly Ser Arg
        675                 680                 685
Asn Ser Phe Val Ser Ile Arg Pro Gly Gly Gly Ala Ser Ala Ser Ser
    690                 695                 700
Pro Pro Pro Pro Ala Ala Ala Ser Ser Glu Glu His His Arg Ser Lys
705                 710                 715                 720
Arg Pro Arg Met Ala Ala Ala Tyr Leu Glu Arg Leu His Glu Ala Val
                725                 730                 735
Ser Glu Asn Pro His Arg Val Ile Phe Met Glu Asp Val Glu Arg Ala
            740                 745                 750
Asp Arg Asp Cys Gln Leu Gly Ile Lys Glu Ala Ile Glu Ser Gly Val
        755                 760                 765
Val Arg Asn His Ala Gly Glu Glu Val Gly Val Gly Asp Ala Ile Val
    770                 775                 780
Ile Leu Ser Cys Glu Ser Phe Asp Gly Asp Ser Arg Ser Arg Gly Cys
785                 790                 795                 800
Ser Pro Pro Ser Lys Lys Val Lys Val Glu Met Glu Glu Thr Lys Glu
                805                 810                 815
Glu Arg Thr Gly Glu His Glu His Asn Glu Asp Gly Ala Ser Ser Ser
            820                 825                 830
Ser Pro Ser Cys Ile Asp Leu Asn Val Asp Met Glu Ser Asp Pro Ala
        835                 840                 845
Asp Glu Arg Ser Leu Gly Asp Leu Cys Leu Leu Thr Ala Val Asp Arg
    850                 855                 860
Thr Leu Phe Phe Arg Arg Gln Gln Glu Asn
865                 870

<210> 41
<211> 2445
<212> DNA
<213> Sorghum bicolor

<220>
<221> CDS
<222> (1)..(2445)

<400> 41
atg cgt agc ggc ggc tgc gcg gtg cag cag gag ctg gcc ggg gac gcg        48
Met Arg Ser Gly Gly Cys Ala Val Gln Gln Glu Leu Ala Gly Asp Ala
  1               5                  10                  15
gcg gcc gtg atg cgg cag gcg gtg agc ctg gcg cgg cgc cgg ggc cac        96
Ala Ala Val Met Arg Gln Ala Val Ser Leu Ala Arg Arg Arg Gly His
```

```
            20                  25                  30
gcg cag gtc acg ccg ctg cac gtc gcc agc gcc gtg ctc tcg gtc tcg        144
Ala Gln Val Thr Pro Leu His Val Ala Ser Ala Val Leu Ser Val Ser
        35                  40                  45
gac gcc ggg gca ctc ctc cgc gcc gcc tgc ctc cgg tcc cgg gcc agc        192
Asp Ala Gly Ala Leu Leu Arg Ala Ala Cys Leu Arg Ser Arg Ala Ser
    50                  55                  60
tcc cac ccg ctc cag tgc aag gcg ctg gag ctc tgc ttc aac gtc gcg        240
Ser His Pro Leu Gln Cys Lys Ala Leu Glu Leu Cys Phe Asn Val Ala
65                  70                  75                  80
ctc aac cgc ctc gcg acg acg gcg ggg atg ccg gga ccg ccg gcc ccg        288
Leu Asn Arg Leu Ala Thr Thr Ala Gly Met Pro Gly Pro Pro Ala Pro
                85                  90                  95
ccg ccg gcc atg ttc cag ttc cac cac gcg ccg gct ggt ggg cac cgc        336
Pro Pro Ala Met Phe Gln Phe His His Ala Pro Ala Gly Gly His Arg
            100                 105                 110
gcg ccg gcg ctg tcc aac gcg ctc gcc gcc gcg ttc aag cgc gcg cag        384
Ala Pro Ala Leu Ser Asn Ala Leu Ala Ala Ala Phe Lys Arg Ala Gln
        115                 120                 125
gcg aac cag cgc cgg ggc ggc ggc ggc ggt ggc ttc ggc gtc cgc cgc        432
Ala Asn Gln Arg Arg Gly Gly Gly Gly Gly Gly Phe Gly Val Arg Arg
    130                 135                 140
gga ggc cgg cca gca cca gca cca gtc gag ctc gag cag ctc gtc atc        480
Gly Gly Arg Pro Ala Pro Ala Pro Val Glu Leu Glu Gln Leu Val Ile
145                 150                 155                 160
tcc atc ctc gac gat ccc agc gtc agc cgc gtc atg cgc gat gcc ggc        528
Ser Ile Leu Asp Asp Pro Ser Val Ser Arg Val Met Arg Asp Ala Gly
                165                 170                 175
ttc gcg agc gcc gag gtc aat gcc aac gtc gag aag gcc gtc tcg tcg        576
Phe Ala Ser Ala Glu Val Asn Ala Asn Val Glu Lys Ala Val Ser Ser
            180                 185                 190
tcg gag cag tcg tcg aac acg gca acc agc agc acc gct tcc ccg aat        624
Ser Glu Gln Ser Ser Asn Thr Ala Thr Ser Ser Thr Ala Ser Pro Asn
        195                 200                 205
act act act aat aat aac ccc acc aag gac aag gag tcc aga gcc aag        672
Thr Thr Thr Asn Asn Asn Pro Thr Lys Asp Lys Glu Ser Arg Ala Lys
    210                 215                 220
gcc gac gac atc gtc ggc gac gcc gtg cgc gtg ctg gac tgc atg gcg        720
Ala Asp Asp Ile Val Gly Asp Ala Val Arg Val Leu Asp Cys Met Ala
225                 230                 235                 240
agc ggg acg aac cgg tgc gtg gtg gtc ctc ggc gag acc gcc gcc gcc        768
Ser Gly Thr Asn Arg Cys Val Val Val Leu Gly Glu Thr Ala Ala Ala
                245                 250                 255
gcg gag agg gtg gtc aag gcg gtg atg gac aag gtg agc aag ggg gag        816
Ala Glu Arg Val Val Lys Ala Val Met Asp Lys Val Ser Lys Gly Glu
            260                 265                 270
ctg cgg cgc cgc cag cac gag cgc ctc aag aac gcc cag ttg gtg ccc        864
Leu Arg Arg Arg Gln His Glu Arg Leu Lys Asn Ala Gln Leu Val Pro
        275                 280                 285
ttc tcg gcc gca tcc ttc cag cgg atg ccg agg gag gag gtc gag gcc        912
Phe Ser Ala Ala Ser Phe Gln Arg Met Pro Arg Glu Glu Val Glu Ala
    290                 295                 300
agg gcc ggc gac ctg tgc gcg ctc gtg cgc gag tgc tgc gcc gcc ggc        960
Arg Ala Gly Asp Leu Cys Ala Leu Val Arg Glu Cys Cys Ala Ala Gly
305                 310                 315                 320
aga ggc gtg gtg ctc gtg ctc gag gac ctc gcc tac gcg gcg gag gcc        1008
Arg Gly Val Val Leu Val Leu Glu Asp Leu Ala Tyr Ala Ala Glu Ala
                325                 330                 335
tgg acc gcc gcg tcg tgg aag aga tca agt ggc cac cgc gcg cat ggc        1056
Trp Thr Ala Ala Ser Trp Lys Arg Ser Ser Gly His Arg Ala His Gly
            340                 345                 350
ctc atc gac tat tgt ccc gtc cag cac gcg gtc atg gag ttg agc agc        1104
Leu Ile Asp Tyr Cys Pro Val Gln His Ala Val Met Glu Leu Ser Ser
        355                 360                 365
ctc gtg cgc ggc gcc ggc ggc cgc ggc cgc gac aag ggc atg ttc tgg        1152
```

```
Leu Val Arg Gly Ala Gly Gly Arg Gly Arg Asp Lys Gly Met Phe Trp
    370                 375                 380
ctg ctg ggg ttc gga gcc tcc gcg tcc tac acg agc tgc agg tcg ggg      1200
Leu Leu Gly Phe Gly Ala Ser Ala Ser Tyr Thr Ser Cys Arg Ser Gly
385                 390                 395                 400
cag cct tcc ctg gag acc gtc ttg gga cta cac ccc gtc gtc gtg ccg      1248
Gln Pro Ser Leu Glu Thr Val Leu Gly Leu His Pro Val Val Val Pro
                405                 410                 415
gac ggc ggc ctc gcg ttg agc ctc ggc ggc gac agt gag gtc aca cac      1296
Asp Gly Gly Leu Ala Leu Ser Leu Gly Gly Asp Ser Glu Val Thr His
            420                 425                 430
tgc agc gcc ggc acg gtc gtg gcg acg gcg gcg gcg gcg agc atc ccc      1344
Cys Ser Ala Gly Thr Val Val Ala Thr Ala Ala Ala Ala Ser Ile Pro
        435                 440                 445
gcg tgg att cgc cgt tgc caa ggt cca gcc ctc act gga tcg gag ctc      1392
Ala Trp Ile Arg Arg Cys Gln Gly Pro Ala Leu Thr Gly Ser Glu Leu
    450                 455                 460
aca ctg agc ttc tcc tct ccg gcc tct tcc tcc cac tgt ggc ttt acc      1440
Thr Leu Ser Phe Ser Ser Pro Ala Ser Ser Ser His Cys Gly Phe Thr
465                 470                 475                 480
cat tat gac acc aac aag agc tgc gag cca tgg cat gat ctc atg gac      1488
His Tyr Asp Thr Asn Lys Ser Cys Glu Pro Trp His Asp Leu Met Asp
                485                 490                 495
cgg cgg caa ccg ttg ctg aac cgc cgg cac gat ggt ccg atg gcc gag      1536
Arg Arg Gln Pro Leu Leu Asn Arg Arg His Asp Gly Pro Met Ala Glu
            500                 505                 510
cca tac gat cag ctg tgg ctc gcc gca aac cct aat tca ggg tcc tcc      1584
Pro Tyr Asp Gln Leu Trp Leu Ala Ala Asn Pro Asn Ser Gly Ser Ser
        515                 520                 525
aac tcg gtg tcc aat aaa tcc aac tca tcg gat ggc acg acg acg acg      1632
Asn Ser Val Ser Asn Lys Ser Asn Ser Ser Asp Gly Thr Thr Thr Thr
    530                 535                 540
gct cgc cgc cgg cgt ccg aag ttc acc gag ctc acc gcg gag aat ctc      1680
Ala Arg Arg Arg Arg Pro Lys Phe Thr Glu Leu Thr Ala Glu Asn Leu
545                 550                 555                 560
aag gtc ctg agc agc gcg ctc gag acg cgc gtc ccg cgc cac aga gac      1728
Lys Val Leu Ser Ser Ala Leu Glu Thr Arg Val Pro Arg His Arg Asp
                565                 570                 575
ata gct ccg ggc atc gcc agc gcc gtg ctc cag cgc cgc tcc ggc gtg      1776
Ile Ala Pro Gly Ile Ala Ser Ala Val Leu Gln Arg Arg Ser Gly Val
            580                 585                 590
acg agg acg acg agg ccg acc ccg gcg acg tgg ctc ctc ttc caa ggg      1824
Thr Arg Thr Thr Arg Pro Thr Pro Ala Thr Trp Leu Leu Phe Gln Gly
        595                 600                 605
agg gac aac gac ggc aag atg gcc atg gct cgg gag ctt gcc agg ctt      1872
Arg Asp Asn Asp Gly Lys Met Ala Met Ala Arg Glu Leu Ala Arg Leu
    610                 615                 620
gtc ttt ggc tcc tac gcc gag ttc acc tgc tgc ttc gcc gca gca gca      1920
Val Phe Gly Ser Tyr Ala Glu Phe Thr Cys Cys Phe Ala Ala Ala Ala
625                 630                 635                 640
tca aag ctc gca ccg gat cac tcc ggc tcc agc agc ccc ggc gat cgc      1968
Ser Lys Leu Ala Pro Asp His Ser Gly Ser Ser Ser Pro Gly Asp Arg
                645                 650                 655
cgc agc cta aag agg cag agg tcg tcg ccg gac aac gag cac ggc ggc      2016
Arg Ser Leu Lys Arg Gln Arg Ser Ser Pro Asp Asn Glu His Gly Gly
            660                 665                 670
tgc atg cag atg ttc tac gag gcg atc cgg gaa aac cct cac cgt gtc      2064
Cys Met Gln Met Phe Tyr Glu Ala Ile Arg Glu Asn Pro His Arg Val
        675                 680                 685
gtg ctg gtc gac ggc ggc gtc gag cac gac tcg gaa cta gaa gtc ggc      2112
Val Leu Val Asp Gly Gly Val Glu His Asp Ser Glu Leu Glu Val Gly
    690                 695                 700
atc atg gat gcc atg gcg agc gga acc gtg agg ggt tgc gat ggt ggt      2160
Ile Met Asp Ala Met Ala Ser Gly Thr Val Arg Gly Cys Asp Gly Gly
705                 710                 715                 720
```

```
gtg gtc agc ttg gag gac tct atc gta gtg tac tgc tgt gaa gtg ttc      2208
Val Val Ser Leu Glu Asp Ser Ile Val Val Tyr Cys Cys Glu Val Phe
                725                 730                 735
gtc gag tcg gtg tca tcg cct agg gtt tcc tca cca cgg cct gtg aag      2256
Val Glu Ser Val Ser Ser Pro Arg Val Ser Ser Pro Arg Pro Val Lys
            740                 745                 750
caa agg atc atc acg agt ggc gac gtt gac agc aag gtg gaa gat gat      2304
Gln Arg Ile Ile Thr Ser Gly Asp Val Asp Ser Lys Val Glu Asp Asp
        755                 760                 765
ggc gca gag aaa gga gta gtt gtg cca cgt ttc agc ttg gat ttg aat      2352
Gly Ala Glu Lys Gly Val Val Val Pro Arg Phe Ser Leu Asp Leu Asn
    770                 775                 780
gcg tgc gcc att gac ggc gaa ggg gag gaa ggg agt tct tcg tat aat      2400
Ala Cys Ala Ile Asp Gly Glu Gly Glu Glu Gly Ser Ser Ser Tyr Asn
785                 790                 795                 800
gcc atg gag atc ctt aat gtt gtg gat ggt gtt ttc ttc ttc tag          2445
Ala Met Glu Ile Leu Asn Val Val Asp Gly Val Phe Phe Phe
                805                 810


<210> 42
<211> 814
<212> PRT
<213> Sorghum bicolor

<400> 42
Met Arg Ser Gly Gly Cys Ala Val Gln Gln Glu Leu Ala Gly Asp Ala
1               5                   10                  15
Ala Ala Val Met Arg Gln Ala Val Ser Leu Ala Arg Arg Arg Gly His
            20                  25                  30
Ala Gln Val Thr Pro Leu His Val Ala Ser Ala Val Leu Ser Val Ser
        35                  40                  45
Asp Ala Gly Ala Leu Leu Arg Ala Ala Cys Leu Arg Ser Arg Ala Ser
    50                  55                  60
Ser His Pro Leu Gln Cys Lys Ala Leu Glu Leu Cys Phe Asn Val Ala
65                  70                  75                  80
Leu Asn Arg Leu Ala Thr Thr Ala Gly Met Pro Gly Pro Pro Ala Pro
                85                  90                  95
Pro Pro Ala Met Phe Gln Phe His His Ala Pro Ala Gly Gly His Arg
            100                 105                 110
Ala Pro Ala Leu Ser Asn Ala Leu Ala Ala Ala Phe Lys Arg Ala Gln
        115                 120                 125
Ala Asn Gln Arg Arg Gly Gly Gly Gly Gly Gly Phe Gly Val Arg Arg
    130                 135                 140
Gly Gly Arg Pro Ala Pro Ala Pro Val Glu Leu Glu Gln Leu Val Ile
145                 150                 155                 160
Ser Ile Leu Asp Asp Pro Ser Val Ser Arg Val Met Arg Asp Ala Gly
                165                 170                 175
Phe Ala Ser Ala Glu Val Asn Ala Asn Val Glu Lys Ala Val Ser Ser
            180                 185                 190
Ser Glu Gln Ser Ser Asn Thr Ala Thr Ser Ser Thr Ala Ser Pro Asn
        195                 200                 205
Thr Thr Thr Asn Asn Asn Pro Thr Lys Asp Lys Glu Ser Arg Ala Lys
    210                 215                 220
Ala Asp Asp Ile Val Gly Asp Ala Val Arg Val Leu Asp Cys Met Ala
225                 230                 235                 240
Ser Gly Thr Asn Arg Cys Val Val Val Leu Gly Glu Thr Ala Ala Ala
                245                 250                 255
Ala Glu Arg Val Val Lys Ala Val Met Asp Lys Val Ser Lys Gly Glu
            260                 265                 270
Leu Arg Arg Arg Gln His Glu Arg Leu Lys Asn Ala Gln Leu Val Pro
        275                 280                 285
Phe Ser Ala Ala Ser Phe Gln Arg Met Pro Arg Glu Glu Val Glu Ala
    290                 295                 300
Arg Ala Gly Asp Leu Cys Ala Leu Val Arg Glu Cys Cys Ala Ala Gly
305                 310                 315                 320
```

```
Arg Gly Val Val Leu Val Leu Glu Asp Leu Ala Tyr Ala Ala Glu Ala
                325                 330                 335
Trp Thr Ala Ala Ser Trp Lys Arg Ser Ser Gly His Arg Ala His Gly
            340                 345                 350
Leu Ile Asp Tyr Cys Pro Val Gln His Ala Val Met Glu Leu Ser Ser
        355                 360                 365
Leu Val Arg Gly Ala Gly Gly Arg Gly Arg Asp Lys Gly Met Phe Trp
    370                 375                 380
Leu Leu Gly Phe Gly Ala Ser Ala Ser Tyr Thr Ser Cys Arg Ser Gly
385                 390                 395                 400
Gln Pro Ser Leu Glu Thr Val Leu Gly Leu His Pro Val Val Val Pro
                405                 410                 415
Asp Gly Gly Leu Ala Leu Ser Leu Gly Gly Asp Ser Glu Val Thr His
            420                 425                 430
Cys Ser Ala Gly Thr Val Val Ala Thr Ala Ala Ala Ala Ser Ile Pro
        435                 440                 445
Ala Trp Ile Arg Arg Cys Gln Gly Pro Ala Leu Thr Gly Ser Glu Leu
    450                 455                 460
Thr Leu Ser Phe Ser Ser Pro Ala Ser Ser Ser His Cys Gly Phe Thr
465                 470                 475                 480
His Tyr Asp Thr Asn Lys Ser Cys Glu Pro Trp His Asp Leu Met Asp
                485                 490                 495
Arg Arg Gln Pro Leu Leu Asn Arg Arg His Asp Gly Pro Met Ala Glu
            500                 505                 510
Pro Tyr Asp Gln Leu Trp Leu Ala Ala Asn Pro Asn Ser Gly Ser Ser
        515                 520                 525
Asn Ser Val Ser Asn Lys Ser Asn Ser Ser Asp Gly Thr Thr Thr Thr
    530                 535                 540
Ala Arg Arg Arg Arg Pro Lys Phe Thr Glu Leu Thr Ala Glu Asn Leu
545                 550                 555                 560
Lys Val Leu Ser Ser Ala Leu Glu Thr Arg Val Pro Arg His Arg Asp
                565                 570                 575
Ile Ala Pro Gly Ile Ala Ser Ala Val Leu Gln Arg Arg Ser Gly Val
            580                 585                 590
Thr Arg Thr Thr Arg Pro Thr Pro Ala Thr Trp Leu Leu Phe Gln Gly
        595                 600                 605
Arg Asp Asn Asp Gly Lys Met Ala Met Ala Arg Glu Leu Ala Arg Leu
    610                 615                 620
Val Phe Gly Ser Tyr Ala Glu Phe Thr Cys Cys Phe Ala Ala Ala Ala
625                 630                 635                 640
Ser Lys Leu Ala Pro Asp His Ser Gly Ser Ser Ser Pro Gly Asp Arg
                645                 650                 655
Arg Ser Leu Lys Arg Gln Arg Ser Ser Pro Asp Asn Glu His Gly Gly
            660                 665                 670
Cys Met Gln Met Phe Tyr Glu Ala Ile Arg Glu Asn Pro His Arg Val
        675                 680                 685
Val Leu Val Asp Gly Gly Val Glu His Asp Ser Glu Leu Glu Val Gly
    690                 695                 700
Ile Met Asp Ala Met Ala Ser Gly Thr Val Arg Gly Cys Asp Gly Gly
705                 710                 715                 720
Val Val Ser Leu Glu Asp Ser Ile Val Val Tyr Cys Cys Glu Val Phe
                725                 730                 735
Val Glu Ser Val Ser Ser Pro Arg Val Ser Ser Pro Arg Pro Val Lys
            740                 745                 750
Gln Arg Ile Ile Thr Ser Gly Asp Val Asp Ser Lys Val Glu Asp Asp
        755                 760                 765
Gly Ala Glu Lys Gly Val Val Val Pro Arg Phe Ser Leu Asp Leu Asn
    770                 775                 780
Ala Cys Ala Ile Asp Gly Glu Gly Glu Glu Gly Ser Ser Ser Tyr Asn
785                 790                 795                 800
Ala Met Glu Ile Leu Asn Val Val Asp Gly Val Phe Phe Phe
                805                 810
```

<210> 43
<211> 2517

<212> DNA
<213> Zea mays

<220>
<221> CDS
<222> (1)..(2517)

<400> 43
atg cga gcc ggc ggc tgc gcg gtg cag cag gcg ctg gcc gcg gag gcg 48
Met Arg Ala Gly Gly Cys Ala Val Gln Gln Ala Leu Ala Ala Glu Ala
1 5 10 15
gcg gcc gtg gtg cgg cag gcc gtg gcc ctg gcc cgg cgg cgc ggc cac 96
Ala Ala Val Val Arg Gln Ala Val Ala Leu Ala Arg Arg Arg Gly His
20 25 30
gcg cag gtg acc ccg ctc cac gtg gcc agc gcc atg ctc tcg gcg gcg 144
Ala Gln Val Thr Pro Leu His Val Ala Ser Ala Met Leu Ser Ala Ala
35 40 45
ggg ctc ctc cgc gcc gcc tgc ctc cgc tcc cac tcc cac ccg ctg cag 192
Gly Leu Leu Arg Ala Ala Cys Leu Arg Ser His Ser His Pro Leu Gln
50 55 60
tgc aag gcc ctg gag ctc tgt ttc aac gtg gcg ctt aac cgc ctc ccc 240
Cys Lys Ala Leu Glu Leu Cys Phe Asn Val Ala Leu Asn Arg Leu Pro
65 70 75 80
acc gcc gcc gcc tct atg ttc cac ccc cat cac cac gcc ggg cag cag 288
Thr Ala Ala Ala Ser Met Phe His Pro His His His Ala Gly Gln Gln
85 90 95
cac gcg ccc gtg ctg tcc aac gcg ctc gtc gcc gcg ttc aag cgc gcg 336
His Ala Pro Val Leu Ser Asn Ala Leu Val Ala Ala Phe Lys Arg Ala
100 105 110
cag gcg cac cag cgc cgg ggc tcc gtg gat gga gca cag gga caa ccg 384
Gln Ala His Gln Arg Arg Gly Ser Val Asp Gly Ala Gln Gly Gln Pro
115 120 125
cca cag ccg gtg ctc gcc ggc aag gtc gac atc gac cag ctc atc atc 432
Pro Gln Pro Val Leu Ala Gly Lys Val Asp Ile Asp Gln Leu Ile Ile
130 135 140
tcc atc ctc gac gac ccc agc gtg agc cgc gtc atg cgc gag gcc ggc 480
Ser Ile Leu Asp Asp Pro Ser Val Ser Arg Val Met Arg Glu Ala Gly
145 150 155 160
ttc tcc agc tcc cag gtc aag gcc aac gtc gag aag gcc gtc tcg gcg 528
Phe Ser Ser Ser Gln Val Lys Ala Asn Val Glu Lys Ala Val Ser Ala
165 170 175
tcg tct tca ccg gag cat cag cag cag aac acg acg ata cca ccc acc 576
Ser Ser Ser Pro Glu His Gln Gln Gln Asn Thr Thr Ile Pro Pro Thr
180 185 190
agc cgc gcc cac gcg gcg atc tct cca ggc gga gca gct tcc ggc gac 624
Ser Arg Ala His Ala Ala Ile Ser Pro Gly Gly Ala Ala Ser Gly Asp
195 200 205
gcc atg cgc gtg ctc gac tgc atg gcg agc gga agc aag cgg agc gtg 672
Ala Met Arg Val Leu Asp Cys Met Ala Ser Gly Ser Lys Arg Ser Val
210 215 220
gtg gtc gtt ggc gag agc gcg gcc acc gcg gag gtg gtg gtg aag gcg 720
Val Val Val Gly Glu Ser Ala Ala Thr Ala Glu Val Val Val Lys Ala
225 230 235 240
gtg atg aac agg gtg agc aaa ggg gag ctg cag cag cgg cac gag cgg 768
Val Met Asn Arg Val Ser Lys Gly Glu Leu Gln Gln Arg His Glu Arg
245 250 255
ctc aag aac gtc cag ttc gtg ccc ctc tcg gcc gcg tcc ttc cag cgg 816
Leu Lys Asn Val Gln Phe Val Pro Leu Ser Ala Ala Ser Phe Gln Arg
260 265 270
atg ccg agg gag gag gtg gag gcc aag gcc gcc gac ctg cgc gcg ctc 864
Met Pro Arg Glu Glu Val Glu Ala Lys Ala Ala Asp Leu Arg Ala Leu
275 280 285
gtc cgc cag ggc tgc gcc gcc ggg aag ggc gtc gtg ctc gtc ctc gag 912
Val Arg Gln Gly Cys Ala Ala Gly Lys Gly Val Val Leu Val Leu Glu
290 295 300

```
gac ctc gcg tac gcc gcc gag gcc tgg gcc gcc gtg tca gag acg aga        960
Asp Leu Ala Tyr Ala Ala Glu Ala Trp Ala Ala Val Ser Glu Thr Arg
305                 310                 315                 320
agg agg agc agc ggc agt ggc ggc cgt gat cac ggc cag ggc tgc tac       1008
Arg Arg Ser Ser Gly Ser Gly Gly Arg Asp His Gly Gln Gly Cys Tyr
                325                 330                 335
tgc ccc gtc gag cac gcc gtc atg gaa gtg ggc agc ttg gtg tcc gct       1056
Cys Pro Val Glu His Ala Val Met Glu Val Gly Ser Leu Val Ser Ala
            340                 345                 350
gct gcc gga ggc ggc ggc aga ggc ctg gac agg ttc tgg ttg cta ggg       1104
Ala Ala Gly Gly Gly Gly Arg Gly Leu Asp Arg Phe Trp Leu Leu Gly
        355                 360                 365
ttt ggg aac aac cag gct tat ttg aag tcc aga gca ggg cag cca tct       1152
Phe Gly Asn Asn Gln Ala Tyr Leu Lys Ser Arg Ala Gly Gln Pro Ser
    370                 375                 380
ctt gag gct gtc tgg gag ctg cac ccc atc gtc gtg ccg gac ggc ggc       1200
Leu Glu Ala Val Trp Glu Leu His Pro Ile Val Val Pro Asp Gly Gly
385                 390                 395                 400
ctc gcg ctg agc ctc agg tgc aac agt gac gcc gaa caa gct aga gca       1248
Leu Ala Leu Ser Leu Arg Cys Asn Ser Asp Ala Glu Gln Ala Arg Ala
                405                 410                 415
aca agg cct tgg ccc ttc gct aac ggc act gca gca acc ggc gac agt       1296
Thr Arg Pro Trp Pro Phe Ala Asn Gly Thr Ala Ala Thr Gly Asp Ser
            420                 425                 430
gag ctc atc act ttg tcc tgc gcc aag gtg gcg gcg aca act cca aat       1344
Glu Leu Ile Thr Leu Ser Cys Ala Lys Val Ala Ala Thr Thr Pro Asn
        435                 440                 445
gtg ccg cca tgg ctt cag ggc tac caa gtt caa gat acg acc aga ctg       1392
Val Pro Pro Trp Leu Gln Gly Tyr Gln Val Gln Asp Thr Thr Arg Leu
    450                 455                 460
gcg agc cgc agc acc agt ttt cag atg caa gat tgg gac ccc aac tct       1440
Ala Ser Arg Ser Thr Ser Phe Gln Met Gln Asp Trp Asp Pro Asn Ser
465                 470                 475                 480
aat gga gca gct tac cac acg tcg gag atc aca ctg agc ttc tcc cca       1488
Asn Gly Ala Ala Tyr His Thr Ser Glu Ile Thr Leu Ser Phe Ser Pro
                485                 490                 495
cag gct act aac tct cca gac gct tct tca atc tcc ggc tcc ttt gcc       1536
Gln Ala Thr Asn Ser Pro Asp Ala Ser Ser Ile Ser Gly Ser Phe Ala
            500                 505                 510
ccc agc ttg atg atg agc tcc gaa cca tgg caa ttc aag ctg atg caa       1584
Pro Ser Leu Met Met Ser Ser Glu Pro Trp Gln Phe Lys Leu Met Gln
        515                 520                 525
ccg tgc cca aac tat gga cgt ggc gat cca ttt gcc agg tcc tct gat       1632
Pro Cys Pro Asn Tyr Gly Arg Gly Asp Pro Phe Ala Arg Ser Ser Asp
    530                 535                 540
cgt caa caa ctg ctt cca agg ccg act agt cca gaa aaa tct tac tcc       1680
Arg Gln Gln Leu Leu Pro Arg Pro Thr Ser Pro Glu Lys Ser Tyr Ser
545                 550                 555                 560
gtg tcc aac tca tcc gaa ggt gcc cct gca gcc gag tcg cca aaa ttc       1728
Val Ser Asn Ser Ser Glu Gly Ala Pro Ala Ala Glu Ser Pro Lys Phe
                565                 570                 575
acg gag ctc acc gct gag aat ctc aag atc ctc tcc aac gcg ctt gag       1776
Thr Glu Leu Thr Ala Glu Asn Leu Lys Ile Leu Ser Asn Ala Leu Glu
            580                 585                 590
aac cga gct cca cgt cac aag gat gtg gtt aca gag atc gca agt gtc       1824
Asn Arg Ala Pro Arg His Lys Asp Val Val Thr Glu Ile Ala Ser Val
        595                 600                 605
gtg ctc cag tgc cgc tcg ggc atg aca aga aag agg agg aga tgg tgc       1872
Val Leu Gln Cys Arg Ser Gly Met Thr Arg Lys Arg Arg Arg Trp Cys
    610                 615                 620
tgc caa gag aag cca agt gca gtg aca tgg ctg ctc ttc caa gga gcg       1920
Cys Gln Glu Lys Pro Ser Ala Val Thr Trp Leu Leu Phe Gln Gly Ala
625                 630                 635                 640
ggc aat gat ggc aag aaa gcc gtg tct aag gag ctc gca agg ctc gtc       1968
Gly Asn Asp Gly Lys Lys Ala Val Ser Lys Glu Leu Ala Arg Leu Val
```

```
                645                 650                 655
ttc ggt tcc tac agc aag ttc acc gcc atc tca cta tcc gag ttc acc      2016
Phe Gly Ser Tyr Ser Lys Phe Thr Ala Ile Ser Leu Ser Glu Phe Thr
            660                 665                 670
cac gtc cac tcc gat tcc agc tcc ggc gag atc acg ttg aag agg caa      2064
His Val His Ser Asp Ser Ser Ser Gly Glu Ile Thr Leu Lys Arg Gln
        675                 680                 685
aga tca ctg gac act ggg cgt agc tac gtt cag aga ttg tat gat gca      2112
Arg Ser Leu Asp Thr Gly Arg Ser Tyr Val Gln Arg Leu Tyr Asp Ala
    690                 695                 700
ata ctc gaa aac cct cat cgg gtg ata atg atc gac ggt gtt gag caa      2160
Ile Leu Glu Asn Pro His Arg Val Ile Met Ile Asp Gly Val Glu Gln
705                 710                 715                 720
ctg gac tac gag tca gag atc ggc atc agg aat gcg atc aca aat gga      2208
Leu Asp Tyr Glu Ser Glu Ile Gly Ile Arg Asn Ala Ile Thr Asn Gly
                725                 730                 735
aga att agg ggt tgc aac ggc gac gag atc agt ctg ggg gat acc atc      2256
Arg Ile Arg Gly Cys Asn Gly Asp Glu Ile Ser Leu Gly Asp Thr Ile
            740                 745                 750
ata gta ctg aat tgt gaa gca ctc cgt tca atg tct aac gct tcc tcc      2304
Ile Val Leu Asn Cys Glu Ala Leu Arg Ser Met Ser Asn Ala Ser Ser
        755                 760                 765
cct cgg ctc aag caa agg gtc atc gag aag gat gga aag gaa gga aat      2352
Pro Arg Leu Lys Gln Arg Val Ile Glu Lys Asp Gly Lys Glu Gly Asn
    770                 775                 780
gac atg aac atg gag aat ggg atg gag tca tct ggt ttt acc ttg gat      2400
Asp Met Asn Met Glu Asn Gly Met Glu Ser Ser Gly Phe Thr Leu Asp
785                 790                 795                 800
ttg aat gca tgt gcc gag gat tgt gaa ggg aac tat gaa gag agt gtt      2448
Leu Asn Ala Cys Ala Glu Asp Cys Glu Gly Asn Tyr Glu Glu Ser Val
                805                 810                 815
tca gat aat gcg agg atc gtt aat att gtg gat ggc gtg ttc ttc ttc      2496
Ser Asp Asn Ala Arg Ile Val Asn Ile Val Asp Gly Val Phe Phe Phe
            820                 825                 830
caa tta atg gag cac tca tga                                          2517
Gln Leu Met Glu His Ser
        835
```

<210> 44
<211> 838
<212> PRT
<213> Zea mays

<400> 44

```
Met Arg Ala Gly Gly Cys Ala Val Gln Gln Ala Leu Ala Ala Glu Ala
1               5                   10                  15
Ala Ala Val Val Arg Gln Ala Val Ala Leu Ala Arg Arg Arg Gly His
            20                  25                  30
Ala Gln Val Thr Pro Leu His Val Ala Ser Ala Met Leu Ser Ala Ala
        35                  40                  45
Gly Leu Leu Arg Ala Ala Cys Leu Arg Ser His Ser His Pro Leu Gln
    50                  55                  60
Cys Lys Ala Leu Glu Leu Cys Phe Asn Val Ala Leu Asn Arg Leu Pro
65                  70                  75                  80
Thr Ala Ala Ala Ser Met Phe His Pro His His His Ala Gly Gln Gln
                85                  90                  95
His Ala Pro Val Leu Ser Asn Ala Leu Val Ala Ala Phe Lys Arg Ala
            100                 105                 110
Gln Ala His Gln Arg Arg Gly Ser Val Asp Gly Ala Gln Gly Gln Pro
        115                 120                 125
Pro Gln Pro Val Leu Ala Gly Lys Val Asp Ile Asp Gln Leu Ile Ile
    130                 135                 140
Ser Ile Leu Asp Asp Pro Ser Val Ser Arg Val Met Arg Glu Ala Gly
145                 150                 155                 160
Phe Ser Ser Ser Gln Val Lys Ala Asn Val Glu Lys Ala Val Ser Ala
```

```
                165                 170                 175
Ser Ser Ser Pro Glu His Gln Gln Gln Asn Thr Thr Ile Pro Pro Thr
            180                 185                 190
Ser Arg Ala His Ala Ala Ile Ser Pro Gly Gly Ala Ala Ser Gly Asp
        195                 200                 205
Ala Met Arg Val Leu Asp Cys Met Ala Ser Gly Ser Lys Arg Ser Val
    210                 215                 220
Val Val Val Gly Glu Ser Ala Ala Thr Ala Glu Val Val Val Lys Ala
225                 230                 235                 240
Val Met Asn Arg Val Ser Lys Gly Glu Leu Gln Gln Arg His Glu Arg
                245                 250                 255
Leu Lys Asn Val Gln Phe Val Pro Leu Ser Ala Ala Ser Phe Gln Arg
            260                 265                 270
Met Pro Arg Glu Glu Val Glu Ala Lys Ala Ala Asp Leu Arg Ala Leu
        275                 280                 285
Val Arg Gln Gly Cys Ala Ala Gly Lys Gly Val Val Leu Val Leu Glu
    290                 295                 300
Asp Leu Ala Tyr Ala Ala Glu Ala Trp Ala Ala Val Ser Glu Thr Arg
305                 310                 315                 320
Arg Arg Ser Ser Gly Ser Gly Gly Arg Asp His Gly Gln Gly Cys Tyr
                325                 330                 335
Cys Pro Val Glu His Ala Val Met Glu Val Gly Ser Leu Val Ser Ala
            340                 345                 350
Ala Ala Gly Gly Gly Gly Arg Gly Leu Asp Arg Phe Trp Leu Leu Gly
        355                 360                 365
Phe Gly Asn Asn Gln Ala Tyr Leu Lys Ser Arg Ala Gly Gln Pro Ser
    370                 375                 380
Leu Glu Ala Val Trp Glu Leu His Pro Ile Val Val Pro Asp Gly Gly
385                 390                 395                 400
Leu Ala Leu Ser Leu Arg Cys Asn Ser Asp Ala Glu Gln Ala Arg Ala
                405                 410                 415
Thr Arg Pro Trp Pro Phe Ala Asn Gly Thr Ala Ala Thr Gly Asp Ser
            420                 425                 430
Glu Leu Ile Thr Leu Ser Cys Ala Lys Val Ala Ala Thr Thr Pro Asn
        435                 440                 445
Val Pro Pro Trp Leu Gln Gly Tyr Gln Val Gln Asp Thr Thr Arg Leu
    450                 455                 460
Ala Ser Arg Ser Thr Ser Phe Gln Met Gln Asp Trp Asp Pro Asn Ser
465                 470                 475                 480
Asn Gly Ala Ala Tyr His Thr Ser Glu Ile Thr Leu Ser Phe Ser Pro
                485                 490                 495
Gln Ala Thr Asn Ser Pro Asp Ala Ser Ser Ile Ser Gly Ser Phe Ala
            500                 505                 510
Pro Ser Leu Met Met Ser Ser Glu Pro Trp Gln Phe Lys Leu Met Gln
        515                 520                 525
Pro Cys Pro Asn Tyr Gly Arg Gly Asp Pro Phe Ala Arg Ser Ser Asp
    530                 535                 540
Arg Gln Gln Leu Leu Pro Arg Pro Thr Ser Pro Glu Lys Ser Tyr Ser
545                 550                 555                 560
Val Ser Asn Ser Ser Glu Gly Ala Pro Ala Ala Glu Ser Pro Lys Phe
                565                 570                 575
Thr Glu Leu Thr Ala Glu Asn Leu Lys Ile Leu Ser Asn Ala Leu Glu
            580                 585                 590
Asn Arg Ala Pro Arg His Lys Asp Val Val Thr Glu Ile Ala Ser Val
        595                 600                 605
Val Leu Gln Cys Arg Ser Gly Met Thr Arg Lys Arg Arg Arg Trp Cys
    610                 615                 620
Cys Gln Glu Lys Pro Ser Ala Val Thr Trp Leu Leu Phe Gln Gly Ala
625                 630                 635                 640
Gly Asn Asp Gly Lys Lys Ala Val Ser Lys Glu Leu Ala Arg Leu Val
                645                 650                 655
Phe Gly Ser Tyr Ser Lys Phe Thr Ala Ile Ser Leu Ser Glu Phe Thr
            660                 665                 670
His Val His Ser Asp Ser Ser Ser Gly Glu Ile Thr Leu Lys Arg Gln
        675                 680                 685
```

```
Arg Ser Leu Asp Thr Gly Arg Ser Tyr Val Gln Arg Leu Tyr Asp Ala
    690                 695                 700
Ile Leu Glu Asn Pro His Arg Val Ile Met Ile Asp Gly Val Glu Gln
705                 710                 715                 720
Leu Asp Tyr Glu Ser Glu Ile Gly Ile Arg Asn Ala Ile Thr Asn Gly
                725                 730                 735
Arg Ile Arg Gly Cys Asn Gly Asp Glu Ile Ser Leu Gly Asp Thr Ile
            740                 745                 750
Ile Val Leu Asn Cys Glu Ala Leu Arg Ser Met Ser Asn Ala Ser Ser
        755                 760                 765
Pro Arg Leu Lys Gln Arg Val Ile Glu Lys Asp Gly Lys Glu Gly Asn
    770                 775                 780
Asp Met Asn Met Glu Asn Gly Met Glu Ser Ser Gly Phe Thr Leu Asp
785                 790                 795                 800
Leu Asn Ala Cys Ala Glu Asp Cys Glu Gly Asn Tyr Glu Glu Ser Val
                805                 810                 815
Ser Asp Asn Ala Arg Ile Val Asn Ile Val Asp Gly Val Phe Phe Phe
            820                 825                 830
Gln Leu Met Glu His Ser
        835

<210> 45
<211> 2508
<212> DNA
<213> Zea mays

<220>
<221> CDS
<222> (1)..(2508)

<400> 45
atg cga gcc ggc ggc tgc gcg gtg cag cag gcg ctg gcc gcg gag gcg       48
Met Arg Ala Gly Gly Cys Ala Val Gln Gln Ala Leu Ala Ala Glu Ala
1               5                   10                  15
gcg gcc gtg gtg cgg cag gcc gtg gcc ctg gcc cgg cgg cgc ggc cac       96
Ala Ala Val Val Arg Gln Ala Val Ala Leu Ala Arg Arg Arg Gly His
            20                  25                  30
gcg cag gtg acc ccg ctc cac gtg gcc agc gcc atg ctc tcg gcg gcg      144
Ala Gln Val Thr Pro Leu His Val Ala Ser Ala Met Leu Ser Ala Ala
        35                  40                  45
ggg ctc ctc cgc gcc gcc tgc ctc cgc tcc cac tcc cac ccg ctg cag      192
Gly Leu Leu Arg Ala Ala Cys Leu Arg Ser His Ser His Pro Leu Gln
    50                  55                  60
tgc aag gcc ctg gag ctc tgt ttc aac gtg gcg ctt aac cgc ctc ccc      240
Cys Lys Ala Leu Glu Leu Cys Phe Asn Val Ala Leu Asn Arg Leu Pro
65                  70                  75                  80
acc gcc gcc gcc tct atg ttc cac ccc cat cac cac gcc ggg cag cag      288
Thr Ala Ala Ala Ser Met Phe His Pro His His His Ala Gly Gln Gln
                85                  90                  95
cac gcg ccc gtg ctg tcc aac gcg ctc gtc gcc gcg ttc aag cgc gcg      336
His Ala Pro Val Leu Ser Asn Ala Leu Val Ala Ala Phe Lys Arg Ala
            100                 105                 110
cag gcg cac cag cgc cgg ggc tcc gtg gat gga gca cag gga caa ccg      384
Gln Ala His Gln Arg Arg Gly Ser Val Asp Gly Ala Gln Gly Gln Pro
        115                 120                 125
cca cag ccg gtg ctc gcc ggc aag gtc gac atc gac cag ctc atc atc      432
Pro Gln Pro Val Leu Ala Gly Lys Val Asp Ile Asp Gln Leu Ile Ile
    130                 135                 140
tcc atc ctc gac gac ccc agc gtg agc cgc gtc atg cgc gag gcc ggc      480
Ser Ile Leu Asp Asp Pro Ser Val Ser Arg Val Met Arg Glu Ala Gly
145                 150                 155                 160
ttc tcc agc tcc cag gtc aag gcc aac gtc gag aag gcc gtc tcg gcg      528
Phe Ser Ser Ser Gln Val Lys Ala Asn Val Glu Lys Ala Val Ser Ala
                165                 170                 175
tcg tct tca ccg gag cat cag cag cag aac acg acg ata cca ccc acc      576
```

```
Ser Ser Ser Pro Glu His Gln Gln Gln Asn Thr Thr Ile Pro Pro Thr
            180                 185                 190
agc cgc gcc cac gcg gcg atc tct cca ggc gga gca gct tcc ggc gac       624
Ser Arg Ala His Ala Ala Ile Ser Pro Gly Gly Ala Ala Ser Gly Asp
        195                 200                 205
gcc atg cgc gtg ctc gac tgc atg gcg agc gga agc aag cgg agc gtg       672
Ala Met Arg Val Leu Asp Cys Met Ala Ser Gly Ser Lys Arg Ser Val
    210                 215                 220
gtg gtc gtt ggc gag agc gcg gcc acc gcg gag gtg gtg gtg aag gcg       720
Val Val Val Gly Glu Ser Ala Ala Thr Ala Glu Val Val Val Lys Ala
225                 230                 235                 240
gtg atg aac agg gtg agc aaa ggg gag ctg cag cag cgg cac gag cgg       768
Val Met Asn Arg Val Ser Lys Gly Glu Leu Gln Gln Arg His Glu Arg
                245                 250                 255
ctc aag aac gtc cag ttc gtg ccc ctc tcg gcc gcg tcc ttc cag cgg       816
Leu Lys Asn Val Gln Phe Val Pro Leu Ser Ala Ala Ser Phe Gln Arg
            260                 265                 270
atg ccg agg gag gag gtg gag gcc aag gcc gcc gac ctg cgc gcg ctc       864
Met Pro Arg Glu Glu Val Glu Ala Lys Ala Ala Asp Leu Arg Ala Leu
        275                 280                 285
gtc cgc cag ggc tgc gcc gcc ggg aag ggc gtc gtg ctc gtc ctc gag       912
Val Arg Gln Gly Cys Ala Ala Gly Lys Gly Val Val Leu Val Leu Glu
    290                 295                 300
gac ctc gcg tac gcc gcc gag gcc tgg gcc gcc gtg tca gag acg aga       960
Asp Leu Ala Tyr Ala Ala Glu Ala Trp Ala Ala Val Ser Glu Thr Arg
305                 310                 315                 320
agg agg agc agc ggc agt ggc ggc cgt gat cac ggc cag ggc tgc tac      1008
Arg Arg Ser Ser Gly Ser Gly Gly Arg Asp His Gly Gln Gly Cys Tyr
                325                 330                 335
tgc ccc gtc gag cac gcc gtc atg gaa gtg ggc agc ttg gtg tcc gct      1056
Cys Pro Val Glu His Ala Val Met Glu Val Gly Ser Leu Val Ser Ala
            340                 345                 350
gct gcc gga ggc ggc ggc aga ggc ctg gac agg ttc tgg ttg cta ggg      1104
Ala Ala Gly Gly Gly Gly Arg Gly Leu Asp Arg Phe Trp Leu Leu Gly
        355                 360                 365
ttt ggg aac aac cag gct tat ttg aag tcc aga gca ggg cag cca tct      1152
Phe Gly Asn Asn Gln Ala Tyr Leu Lys Ser Arg Ala Gly Gln Pro Ser
    370                 375                 380
ctt gag gct gtc tgg gag ctg cac ccc atc gtc gtg ccg gac ggc ggc      1200
Leu Glu Ala Val Trp Glu Leu His Pro Ile Val Val Pro Asp Gly Gly
385                 390                 395                 400
ctc gcg ctg agc ctc agt gac gcc gaa caa gct aga gca aca agg cct      1248
Leu Ala Leu Ser Leu Ser Asp Ala Glu Gln Ala Arg Ala Thr Arg Pro
                405                 410                 415
tgg ccc ttc gct aac ggc act gca gca acc ggc gac agt gag ctc atc      1296
Trp Pro Phe Ala Asn Gly Thr Ala Ala Thr Gly Asp Ser Glu Leu Ile
            420                 425                 430
act ttg tcc tgc gcc aag gtg gcg gcg aca act cca aat gtg ccg cca      1344
Thr Leu Ser Cys Ala Lys Val Ala Ala Thr Thr Pro Asn Val Pro Pro
        435                 440                 445
tgg ctt cag ggc tac caa gtt caa gat acg acc aga ctg gcg agc cgc      1392
Trp Leu Gln Gly Tyr Gln Val Gln Asp Thr Thr Arg Leu Ala Ser Arg
    450                 455                 460
agc acc agt ttt cag atg caa gat tgg gac ccc aac tct aat gga gca      1440
Ser Thr Ser Phe Gln Met Gln Asp Trp Asp Pro Asn Ser Asn Gly Ala
465                 470                 475                 480
gct tac cac acg tcg gag atc aca ctg agc ttc tcc cca cag gct act      1488
Ala Tyr His Thr Ser Glu Ile Thr Leu Ser Phe Ser Pro Gln Ala Thr
                485                 490                 495
aac tct cca gac gct tct tca atc tcc ggc tcc ttt gcc ccc agc ttg      1536
Asn Ser Pro Asp Ala Ser Ser Ile Ser Gly Ser Phe Ala Pro Ser Leu
            500                 505                 510
atg atg agc tcc gaa cca tgg caa ttc aag ctg atg caa ccg tgc cca      1584
Met Met Ser Ser Glu Pro Trp Gln Phe Lys Leu Met Gln Pro Cys Pro
        515                 520                 525
```

```
aac tat gga cgt ggc gat cca ttt gcc agg tcc tct gat cgt caa caa        1632
Asn Tyr Gly Arg Gly Asp Pro Phe Ala Arg Ser Ser Asp Arg Gln Gln
    530                 535                 540
ctg ctt cca agg ccg act agt cca gaa aaa tct tac tcc gtg tcc aac        1680
Leu Leu Pro Arg Pro Thr Ser Pro Glu Lys Ser Tyr Ser Val Ser Asn
545                 550                 555                 560
tca tcc gaa ggt gcc cct gca gcc gag tcg cca aaa ttc acg gag ctc        1728
Ser Ser Glu Gly Ala Pro Ala Ala Glu Ser Pro Lys Phe Thr Glu Leu
                565                 570                 575
acc gct gag aat ctc aag atc ctc tcc aac gcg ctt gag aac cga gct        1776
Thr Ala Glu Asn Leu Lys Ile Leu Ser Asn Ala Leu Glu Asn Arg Ala
            580                 585                 590
cca cgt cac aag gat gtg gtt aca gag atc gca agt gtc gtg ctc cag        1824
Pro Arg His Lys Asp Val Val Thr Glu Ile Ala Ser Val Val Leu Gln
        595                 600                 605
tgc cgc tcg ggc atg aca aga aag agg agg aga tgg tgc tgc caa gag        1872
Cys Arg Ser Gly Met Thr Arg Lys Arg Arg Arg Trp Cys Cys Gln Glu
    610                 615                 620
aag cca agt gca gtg aca tgg ctg ctc ttc caa gga gcg ggc aat gat        1920
Lys Pro Ser Ala Val Thr Trp Leu Leu Phe Gln Gly Ala Gly Asn Asp
625                 630                 635                 640
ggc aag aaa gcc gtg tct aag gag ctc gca agg ctc gtc ttc ggt tcc        1968
Gly Lys Lys Ala Val Ser Lys Glu Leu Ala Arg Leu Val Phe Gly Ser
                645                 650                 655
tac agc aag ttc acc gcc atc tca cta tcc gag ttc acc cac gtc cac        2016
Tyr Ser Lys Phe Thr Ala Ile Ser Leu Ser Glu Phe Thr His Val His
            660                 665                 670
tcc gat tcc agc tcc ggc gag atc acg ttg aag agg caa aga tca ctg        2064
Ser Asp Ser Ser Ser Gly Glu Ile Thr Leu Lys Arg Gln Arg Ser Leu
        675                 680                 685
gac act ggg cgt agc tac gtt cag aga ttg tat gat gca ata ctc gaa        2112
Asp Thr Gly Arg Ser Tyr Val Gln Arg Leu Tyr Asp Ala Ile Leu Glu
    690                 695                 700
aac cct cat cgg gtg ata atg atc gac ggt gtt gag caa ctg gac tac        2160
Asn Pro His Arg Val Ile Met Ile Asp Gly Val Glu Gln Leu Asp Tyr
705                 710                 715                 720
gag tca gag atc ggc atc agg aat gcg atc aca aat gga aga att agg        2208
Glu Ser Glu Ile Gly Ile Arg Asn Ala Ile Thr Asn Gly Arg Ile Arg
                725                 730                 735
ggt tgc aac ggc gac gag atc agt ctg ggg gat acc atc ata gta ctg        2256
Gly Cys Asn Gly Asp Glu Ile Ser Leu Gly Asp Thr Ile Ile Val Leu
            740                 745                 750
aat tgt gaa gca ctc cgt tca atg tct aac gct tcc tcc cct cgg ctc        2304
Asn Cys Glu Ala Leu Arg Ser Met Ser Asn Ala Ser Ser Pro Arg Leu
        755                 760                 765
aag caa agg gtc atc gag aag gat gga aag gaa gga aat gac atg aac        2352
Lys Gln Arg Val Ile Glu Lys Asp Gly Lys Glu Gly Asn Asp Met Asn
    770                 775                 780
atg gag aat ggg atg gag tca tct ggt ttt acc ttg gat ttg aat gca        2400
Met Glu Asn Gly Met Glu Ser Ser Gly Phe Thr Leu Asp Leu Asn Ala
785                 790                 795                 800
tgt gcc gag gat tgt gaa ggg aac tat gaa gag agt gtt tca gat aat        2448
Cys Ala Glu Asp Cys Glu Gly Asn Tyr Glu Glu Ser Val Ser Asp Asn
                805                 810                 815
gcg agg atc gtt aat att gtg gat ggc gtg ttc ttc ttc caa tta atg        2496
Ala Arg Ile Val Asn Ile Val Asp Gly Val Phe Phe Phe Gln Leu Met
            820                 825                 830
gag cac tca tga                                                        2508
Glu His Ser
        835
```

<210> 46
<211> 835
<212> PRT
<213> Zea mays

<400> 46

```
Met Arg Ala Gly Gly Cys Ala Val Gln Gln Ala Leu Ala Ala Glu Ala
1               5                   10                  15
Ala Ala Val Val Arg Gln Ala Val Ala Leu Ala Arg Arg Arg Gly His
            20                  25                  30
Ala Gln Val Thr Pro Leu His Val Ala Ser Ala Met Leu Ser Ala Ala
        35                  40                  45
Gly Leu Leu Arg Ala Ala Cys Leu Arg Ser His Ser His Pro Leu Gln
    50                  55                  60
Cys Lys Ala Leu Glu Leu Cys Phe Asn Val Ala Leu Asn Arg Leu Pro
65                  70                  75                  80
Thr Ala Ala Ala Ser Met Phe His Pro His His His Ala Gly Gln Gln
                85                  90                  95
His Ala Pro Val Leu Ser Asn Ala Leu Val Ala Ala Phe Lys Arg Ala
            100                 105                 110
Gln Ala His Gln Arg Arg Gly Ser Val Asp Gly Ala Gln Gly Gln Pro
        115                 120                 125
Pro Gln Pro Val Leu Ala Gly Lys Val Asp Ile Asp Gln Leu Ile Ile
    130                 135                 140
Ser Ile Leu Asp Asp Pro Ser Val Ser Arg Val Met Arg Glu Ala Gly
145                 150                 155                 160
Phe Ser Ser Ser Gln Val Lys Ala Asn Val Glu Lys Ala Val Ser Ala
                165                 170                 175
Ser Ser Ser Pro Glu His Gln Gln Gln Asn Thr Thr Ile Pro Pro Thr
            180                 185                 190
Ser Arg Ala His Ala Ala Ile Ser Pro Gly Gly Ala Ala Ser Gly Asp
        195                 200                 205
Ala Met Arg Val Leu Asp Cys Met Ala Ser Gly Ser Lys Arg Ser Val
    210                 215                 220
Val Val Val Gly Glu Ser Ala Ala Thr Ala Glu Val Val Val Lys Ala
225                 230                 235                 240
Val Met Asn Arg Val Ser Lys Gly Glu Leu Gln Gln Arg His Glu Arg
                245                 250                 255
Leu Lys Asn Val Gln Phe Val Pro Leu Ser Ala Ala Ser Phe Gln Arg
            260                 265                 270
Met Pro Arg Glu Glu Val Glu Ala Lys Ala Ala Asp Leu Arg Ala Leu
        275                 280                 285
Val Arg Gln Gly Cys Ala Ala Gly Lys Gly Val Val Leu Val Leu Glu
    290                 295                 300
Asp Leu Ala Tyr Ala Ala Glu Ala Trp Ala Ala Val Ser Glu Thr Arg
305                 310                 315                 320
Arg Arg Ser Ser Gly Ser Gly Gly Arg Asp His Gly Gln Gly Cys Tyr
                325                 330                 335
Cys Pro Val Glu His Ala Val Met Glu Val Gly Ser Leu Val Ser Ala
            340                 345                 350
Ala Ala Gly Gly Gly Gly Arg Gly Leu Asp Arg Phe Trp Leu Leu Gly
        355                 360                 365
Phe Gly Asn Asn Gln Ala Tyr Leu Lys Ser Arg Ala Gly Gln Pro Ser
    370                 375                 380
Leu Glu Ala Val Trp Glu Leu His Pro Ile Val Val Pro Asp Gly Gly
385                 390                 395                 400
Leu Ala Leu Ser Leu Ser Asp Ala Glu Gln Ala Arg Ala Thr Arg Pro
                405                 410                 415
Trp Pro Phe Ala Asn Gly Thr Ala Ala Thr Gly Asp Ser Glu Leu Ile
            420                 425                 430
Thr Leu Ser Cys Ala Lys Val Ala Ala Thr Thr Pro Asn Val Pro Pro
        435                 440                 445
Trp Leu Gln Gly Tyr Gln Val Gln Asp Thr Thr Arg Leu Ala Ser Arg
    450                 455                 460
Ser Thr Ser Phe Gln Met Gln Asp Trp Asp Pro Asn Ser Asn Gly Ala
465                 470                 475                 480
Ala Tyr His Thr Ser Glu Ile Thr Leu Ser Phe Ser Pro Gln Ala Thr
                485                 490                 495
Asn Ser Pro Asp Ala Ser Ser Ile Ser Gly Ser Phe Ala Pro Ser Leu
```

```
            500                 505                 510
Met Met Ser Ser Glu Pro Trp Gln Phe Lys Leu Met Gln Pro Cys Pro
        515                 520                 525
Asn Tyr Gly Arg Gly Asp Pro Phe Ala Arg Ser Ser Asp Arg Gln Gln
    530                 535                 540
Leu Leu Pro Arg Pro Thr Ser Pro Glu Lys Ser Tyr Ser Val Ser Asn
545                 550                 555                 560
Ser Ser Glu Gly Ala Pro Ala Ala Glu Ser Pro Lys Phe Thr Glu Leu
                565                 570                 575
Thr Ala Glu Asn Leu Lys Ile Leu Ser Asn Ala Leu Glu Asn Arg Ala
            580                 585                 590
Pro Arg His Lys Asp Val Val Thr Glu Ile Ala Ser Val Val Leu Gln
        595                 600                 605
Cys Arg Ser Gly Met Thr Arg Lys Arg Arg Arg Trp Cys Cys Gln Glu
    610                 615                 620
Lys Pro Ser Ala Val Thr Trp Leu Leu Phe Gln Gly Ala Gly Asn Asp
625                 630                 635                 640
Gly Lys Lys Ala Val Ser Lys Glu Leu Ala Arg Leu Val Phe Gly Ser
                645                 650                 655
Tyr Ser Lys Phe Thr Ala Ile Ser Leu Ser Glu Phe Thr His Val His
            660                 665                 670
Ser Asp Ser Ser Ser Gly Glu Ile Thr Leu Lys Arg Gln Arg Ser Leu
        675                 680                 685
Asp Thr Gly Arg Ser Tyr Val Gln Arg Leu Tyr Asp Ala Ile Leu Glu
    690                 695                 700
Asn Pro His Arg Val Ile Met Ile Asp Gly Val Glu Gln Leu Asp Tyr
705                 710                 715                 720
Glu Ser Glu Ile Gly Ile Arg Asn Ala Ile Thr Asn Gly Arg Ile Arg
                725                 730                 735
Gly Cys Asn Gly Asp Glu Ile Ser Leu Gly Asp Thr Ile Ile Val Leu
            740                 745                 750
Asn Cys Glu Ala Leu Arg Ser Met Ser Asn Ala Ser Ser Pro Arg Leu
        755                 760                 765
Lys Gln Arg Val Ile Glu Lys Asp Gly Lys Glu Gly Asn Asp Met Asn
    770                 775                 780
Met Glu Asn Gly Met Glu Ser Ser Gly Phe Thr Leu Asp Leu Asn Ala
785                 790                 795                 800
Cys Ala Glu Asp Cys Glu Gly Asn Tyr Glu Glu Ser Val Ser Asp Asn
                805                 810                 815
Ala Arg Ile Val Asn Ile Val Asp Gly Val Phe Phe Phe Gln Leu Met
            820                 825                 830
Glu His Ser
        835

<210> 47
<211> 2523
<212> DNA
<213> Vitis vinifera

<220>
<221> CDS
<222> (1)..(2523)

<400> 47
atg aga gca gga gtt tgc agt gta cag cag gtt cta act gct gat gca       48
Met Arg Ala Gly Val Cys Ser Val Gln Gln Val Leu Thr Ala Asp Ala
1               5                   10                  15
gca agc atg gtc aaa caa gca gtt acc ctt gct agg cgg cga ggt cat       96
Ala Ser Met Val Lys Gln Ala Val Thr Leu Ala Arg Arg Arg Gly His
            20                  25                  30
gcc caa gtt act cct ctt cat gtg gcc agt gtc atg ctt gct tcc tcc      144
Ala Gln Val Thr Pro Leu His Val Ala Ser Val Met Leu Ala Ser Ser
        35                  40                  45
tct ggt ctt ctc cgc tct gct tgc cta cga tcc cac tct cat ccc ctg      192
Ser Gly Leu Leu Arg Ser Ala Cys Leu Arg Ser His Ser His Pro Leu
```

```
        50                  55                  60
cag tgc aag gct ctg gag ctc tgc ttc aat gtg gcg ctc aac cgc ctc       240
Gln Cys Lys Ala Leu Glu Leu Cys Phe Asn Val Ala Leu Asn Arg Leu
65                  70                  75                  80
cct gcc tcc tca tca agc ccc cta tta gct cct cat tcc tct cac cct       288
Pro Ala Ser Ser Ser Ser Pro Leu Leu Ala Pro His Ser Ser His Pro
                85                  90                  95
tcc ttg tcc aat gcc ttg gtt gca gcc ttc aag cgt gca cag gct cac       336
Ser Leu Ser Asn Ala Leu Val Ala Ala Phe Lys Arg Ala Gln Ala His
            100                 105                 110
caa cgc cgt gcc tcc atc gaa aac cag caa caa ccc att cta gct ctc       384
Gln Arg Arg Ala Ser Ile Glu Asn Gln Gln Gln Pro Ile Leu Ala Leu
        115                 120                 125
aaa gta gag ata gaa cag ctc ata atc tcc atc cta cat gac cca agt       432
Lys Val Glu Ile Glu Gln Leu Ile Ile Ser Ile Leu His Asp Pro Ser
    130                 135                 140
gtt agt aga gtc atg aga gag gct ggt ttc tct agt acc caa ttg aga       480
Val Ser Arg Val Met Arg Glu Ala Gly Phe Ser Ser Thr Gln Leu Arg
145                 150                 155                 160
acc aac ata gaa caa gct gtc tct ttg gat gtt tgt tct caa agc cct       528
Thr Asn Ile Glu Gln Ala Val Ser Leu Asp Val Cys Ser Gln Ser Pro
                165                 170                 175
gct gtg agt agc ctg tct aag gaa ata act ctg aac aat cca ttt gac       576
Ala Val Ser Ser Leu Ser Lys Glu Ile Thr Leu Asn Asn Pro Phe Asp
            180                 185                 190
gaa gct cag gaa gaa gat gta aag agt ctt ttg gac gca ttt aca agc       624
Glu Ala Gln Glu Glu Asp Val Lys Ser Leu Leu Asp Ala Phe Thr Ser
        195                 200                 205
aaa aga agg aga aac act gtt gtt gta gga gag act ctg gcc agt gct       672
Lys Arg Arg Arg Asn Thr Val Val Val Gly Glu Thr Leu Ala Ser Ala
    210                 215                 220
gag ggt gta gtt aga ggg ctg atg aac aag ttt gag aga gga gat gtc       720
Glu Gly Val Val Arg Gly Leu Met Asn Lys Phe Glu Arg Gly Asp Val
225                 230                 235                 240
cct gga gat ctg agg tat gtg cag ttt ata agc ctt cct ctc ttc tct       768
Pro Gly Asp Leu Arg Tyr Val Gln Phe Ile Ser Leu Pro Leu Phe Ser
                245                 250                 255
tta aag aat ctc tcc aag gag gag gtt gaa cag aag ctt gtg aag ctg       816
Leu Lys Asn Leu Ser Lys Glu Glu Val Glu Gln Lys Leu Val Lys Leu
            260                 265                 270
act tgc ctt ctg aaa agc tat gtg tgt aga ggg gtg gtt ttg tat ttg       864
Thr Cys Leu Leu Lys Ser Tyr Val Cys Arg Gly Val Val Leu Tyr Leu
        275                 280                 285
ggt gat ctc aaa tgg gtc tct gag ttt gag tca aat tat ggt gag agg       912
Gly Asp Leu Lys Trp Val Ser Glu Phe Glu Ser Asn Tyr Gly Glu Arg
    290                 295                 300
aga aac tac tgc tct cct gtg gag cac ata atc atg gag ctc gga aga       960
Arg Asn Tyr Cys Ser Pro Val Glu His Ile Ile Met Glu Leu Gly Arg
305                 310                 315                 320
atg atg tgt gga att ggg gat aga gga cga atg tgg ctt ttg ggg act       1008
Met Met Cys Gly Ile Gly Asp Arg Gly Arg Met Trp Leu Leu Gly Thr
                325                 330                 335
gca acc ttc caa act tac atg aga tgc aaa gca ggc cat cct tct ttg       1056
Ala Thr Phe Gln Thr Tyr Met Arg Cys Lys Ala Gly His Pro Ser Leu
            340                 345                 350
gag act att tgg gaa ctt cat cct ctt act att cct gtt ggc agc ttg       1104
Glu Thr Ile Trp Glu Leu His Pro Leu Thr Ile Pro Val Gly Ser Leu
        355                 360                 365
ggc ctc ggt ctc aat ctt gac agc aat ttg cag ggc cgg ttc caa agt       1152
Gly Leu Gly Leu Asn Leu Asp Ser Asn Leu Gln Gly Arg Phe Gln Ser
    370                 375                 380
aag gca tct gga gat ggg act tct tgg tca cta ctt caa agt gga gat       1200
Lys Ala Ser Gly Asp Gly Thr Ser Trp Ser Leu Leu Gln Ser Gly Asp
385                 390                 395                 400
aag cac ctt act tgc agc aca aat tgc tct gat aat ttt gac aaa gaa       1248
```

```
Lys His Leu Thr Cys Ser Thr Asn Cys Ser Asp Asn Phe Asp Lys Glu
                405                 410                 415
tct caa agt ata gca tgc agt ttt cgc aat ggc gag tcc acc acc acc      1296
Ser Gln Ser Ile Ala Cys Ser Phe Arg Asn Gly Glu Ser Thr Thr Thr
            420                 425                 430
atc acc act tcc acc agc tca agc ttg cct tca tgg ctc caa aaa gag      1344
Ile Thr Thr Ser Thr Ser Ser Ser Leu Pro Ser Trp Leu Gln Lys Glu
        435                 440                 445
aaa aga aga aag atc atg gac gat cag gaa tgt gtc caa gtc aga gat      1392
Lys Arg Arg Lys Ile Met Asp Asp Gln Glu Cys Val Gln Val Arg Asp
    450                 455                 460
ctc tgc aac aaa tgg aac tcg ttt tgc agc tca gtc cac aaa aag gcc      1440
Leu Cys Asn Lys Trp Asn Ser Phe Cys Ser Ser Val His Lys Lys Ala
465                 470                 475                 480
cac agt act gag aaa gcc ctg aat ttt tct tca cca tct cct tcc tcc      1488
His Ser Thr Glu Lys Ala Leu Asn Phe Ser Ser Pro Ser Pro Ser Ser
                485                 490                 495
act tcc atc tcc tcc tat gac caa tgc agc cct aat ctg caa cag aac      1536
Thr Ser Ile Ser Ser Tyr Asp Gln Cys Ser Pro Asn Leu Gln Gln Asn
            500                 505                 510
cac cta agt tgg cca gcc atc att gag ccg aaa cca cct ctg aaa gaa      1584
His Leu Ser Trp Pro Ala Ile Ile Glu Pro Lys Pro Pro Leu Lys Glu
        515                 520                 525
cac cag ttc tgg ata tct gaa aat gtt gat gaa ggc ctt gaa ccc aag      1632
His Gln Phe Trp Ile Ser Glu Asn Val Asp Glu Gly Leu Glu Pro Lys
    530                 535                 540
ttc agt atg cac ata gca gag aga aat ttt cca ata cca gat ctt tta      1680
Phe Ser Met His Ile Ala Glu Arg Asn Phe Pro Ile Pro Asp Leu Leu
545                 550                 555                 560
tca aat cct aac tct tcc cct aat tca gct tct tct agt gag gca ata      1728
Ser Asn Pro Asn Ser Ser Pro Asn Ser Ala Ser Ser Ser Glu Ala Ile
                565                 570                 575
gag gat gga gag ggt ctt tat ggt ttc aaa gag ctt aat gcg gag aat      1776
Glu Asp Gly Glu Gly Leu Tyr Gly Phe Lys Glu Leu Asn Ala Glu Asn
            580                 585                 590
ctg aga atc tta tgc aat gca ttg gag aga agg gtt cca tgg cag aag      1824
Leu Arg Ile Leu Cys Asn Ala Leu Glu Arg Arg Val Pro Trp Gln Lys
        595                 600                 605
gat atc att cct gaa ata gca agc acc att ctt gaa tgc agg tca gga      1872
Asp Ile Ile Pro Glu Ile Ala Ser Thr Ile Leu Glu Cys Arg Ser Gly
    610                 615                 620
aca ctg aga gga aaa aac aag ttg aag cag agg gag gac aag gaa gaa      1920
Thr Leu Arg Gly Lys Asn Lys Leu Lys Gln Arg Glu Asp Lys Glu Glu
625                 630                 635                 640
act tgg ctg ctc ttc tta gga gtt gac ttt caa ggc aaa gac aag att      1968
Thr Trp Leu Leu Phe Leu Gly Val Asp Phe Gln Gly Lys Asp Lys Ile
                645                 650                 655
gca agg gag ata gct aag ctt gtt ttt ggc tct cag agc aag ttc atc      2016
Ala Arg Glu Ile Ala Lys Leu Val Phe Gly Ser Gln Ser Lys Phe Ile
            660                 665                 670
tca att ggt ctg agc agt tta gga tca aca aga gct gat tcc acc gag      2064
Ser Ile Gly Leu Ser Ser Leu Gly Ser Thr Arg Ala Asp Ser Thr Glu
        675                 680                 685
gat ttc cta agc aaa caa gca aga gac gag cct gtt ggt agt tat att      2112
Asp Phe Leu Ser Lys Gln Ala Arg Asp Glu Pro Val Gly Ser Tyr Ile
    690                 695                 700
gag aaa ttc gct gaa gcg gtg cat gag aat cct cac aga gtg ttc ttc      2160
Glu Lys Phe Ala Glu Ala Val His Glu Asn Pro His Arg Val Phe Phe
705                 710                 715                 720
ata gaa gat gtg gag caa cta gat tac tct tct caa atg ggg gtt aag      2208
Ile Glu Asp Val Glu Gln Leu Asp Tyr Ser Ser Gln Met Gly Val Lys
                725                 730                 735
aga gga att gag agt gga aga ata caa att gct gga ggt gaa gca ttt      2256
Arg Gly Ile Glu Ser Gly Arg Ile Gln Ile Ala Gly Gly Glu Ala Phe
            740                 745                 750
```

```
tct ctt gag gat gcc atc atc ata ttc agt tgt gaa agc ttc agc tca        2304
Ser Leu Glu Asp Ala Ile Ile Ile Phe Ser Cys Glu Ser Phe Ser Ser
        755                 760                 765
gta tca aga gcc agc tct cct cca ccc atg ggg cta aaa tct gaa gag        2352
Val Ser Arg Ala Ser Ser Pro Pro Pro Met Gly Leu Lys Ser Glu Glu
    770                 775                 780
aat gag gaa aaa gac cgg gat aat gag ttg gag aag aga agc cca tgt        2400
Asn Glu Glu Lys Asp Arg Asp Asn Glu Leu Glu Lys Arg Ser Pro Cys
785                 790                 795                 800
gtg tct cta gat ctg aat ctt tca gct gaa gat aat caa gaa tat gga        2448
Val Ser Leu Asp Leu Asn Leu Ser Ala Glu Asp Asn Gln Glu Tyr Gly
                805                 810                 815
caa aac tcg gtt gca gac act ggg gtt cta gat tct gtg gac agg cag        2496
Gln Asn Ser Val Ala Asp Thr Gly Val Leu Asp Ser Val Asp Arg Gln
            820                 825                 830
ttt att ttc aaa att caa gag ttg tga                                    2523
Phe Ile Phe Lys Ile Gln Glu Leu
        835                 840
```

<210> 48
<211> 840
<212> PRT
<213> Vitis vinifera

<400> 48

```
Met Arg Ala Gly Val Cys Ser Val Gln Gln Val Leu Thr Ala Asp Ala
1               5                   10                  15
Ala Ser Met Val Lys Gln Ala Val Thr Leu Ala Arg Arg Arg Gly His
            20                  25                  30
Ala Gln Val Thr Pro Leu His Val Ala Ser Val Met Leu Ala Ser Ser
        35                  40                  45
Ser Gly Leu Leu Arg Ser Ala Cys Leu Arg Ser His Ser His Pro Leu
    50                  55                  60
Gln Cys Lys Ala Leu Glu Leu Cys Phe Asn Val Ala Leu Asn Arg Leu
65                  70                  75                  80
Pro Ala Ser Ser Ser Ser Pro Leu Leu Ala Pro His Ser Ser His Pro
                85                  90                  95
Ser Leu Ser Asn Ala Leu Val Ala Ala Phe Lys Arg Ala Gln Ala His
            100                 105                 110
Gln Arg Arg Ala Ser Ile Glu Asn Gln Gln Gln Pro Ile Leu Ala Leu
        115                 120                 125
Lys Val Glu Ile Glu Gln Leu Ile Ile Ser Ile Leu His Asp Pro Ser
    130                 135                 140
Val Ser Arg Val Met Arg Glu Ala Gly Phe Ser Ser Thr Gln Leu Arg
145                 150                 155                 160
Thr Asn Ile Glu Gln Ala Val Ser Leu Asp Val Cys Ser Gln Ser Pro
                165                 170                 175
Ala Val Ser Ser Leu Ser Lys Glu Ile Thr Leu Asn Asn Pro Phe Asp
            180                 185                 190
Glu Ala Gln Glu Glu Asp Val Lys Ser Leu Leu Asp Ala Phe Thr Ser
        195                 200                 205
Lys Arg Arg Arg Asn Thr Val Val Val Gly Glu Thr Leu Ala Ser Ala
    210                 215                 220
Glu Gly Val Val Arg Gly Leu Met Asn Lys Phe Glu Arg Gly Asp Val
225                 230                 235                 240
Pro Gly Asp Leu Arg Tyr Val Gln Phe Ile Ser Leu Pro Leu Phe Ser
                245                 250                 255
Leu Lys Asn Leu Ser Lys Glu Glu Val Glu Gln Lys Leu Val Lys Leu
            260                 265                 270
Thr Cys Leu Leu Lys Ser Tyr Val Cys Arg Gly Val Val Leu Tyr Leu
        275                 280                 285
Gly Asp Leu Lys Trp Val Ser Glu Phe Glu Ser Asn Tyr Gly Glu Arg
    290                 295                 300
Arg Asn Tyr Cys Ser Pro Val Glu His Ile Ile Met Glu Leu Gly Arg
305                 310                 315                 320
```

```
Met Met Cys Gly Ile Gly Asp Arg Gly Arg Met Trp Leu Leu Gly Thr
                325                 330                 335
Ala Thr Phe Gln Thr Tyr Met Arg Cys Lys Ala Gly His Pro Ser Leu
            340                 345                 350
Glu Thr Ile Trp Glu Leu His Pro Leu Thr Ile Pro Val Gly Ser Leu
        355                 360                 365
Gly Leu Gly Leu Asn Leu Asp Ser Asn Leu Gln Gly Arg Phe Gln Ser
    370                 375                 380
Lys Ala Ser Gly Asp Gly Thr Ser Trp Ser Leu Leu Gln Ser Gly Asp
385                 390                 395                 400
Lys His Leu Thr Cys Ser Thr Asn Cys Ser Asp Asn Phe Asp Lys Glu
                405                 410                 415
Ser Gln Ser Ile Ala Cys Ser Phe Arg Asn Gly Glu Ser Thr Thr Thr
            420                 425                 430
Ile Thr Thr Ser Thr Ser Ser Ser Leu Pro Ser Trp Leu Gln Lys Glu
        435                 440                 445
Lys Arg Arg Lys Ile Met Asp Asp Gln Glu Cys Val Gln Val Arg Asp
    450                 455                 460
Leu Cys Asn Lys Trp Asn Ser Phe Cys Ser Ser Val His Lys Lys Ala
465                 470                 475                 480
His Ser Thr Glu Lys Ala Leu Asn Phe Ser Ser Pro Ser Pro Ser Ser
                485                 490                 495
Thr Ser Ile Ser Ser Tyr Asp Gln Cys Ser Pro Asn Leu Gln Gln Asn
            500                 505                 510
His Leu Ser Trp Pro Ala Ile Ile Glu Pro Lys Pro Pro Leu Lys Glu
        515                 520                 525
His Gln Phe Trp Ile Ser Glu Asn Val Asp Glu Gly Leu Glu Pro Lys
    530                 535                 540
Phe Ser Met His Ile Ala Glu Arg Asn Phe Pro Ile Pro Asp Leu Leu
545                 550                 555                 560
Ser Asn Pro Asn Ser Ser Pro Asn Ser Ala Ser Ser Ser Glu Ala Ile
                565                 570                 575
Glu Asp Gly Glu Gly Leu Tyr Gly Phe Lys Glu Leu Asn Ala Glu Asn
            580                 585                 590
Leu Arg Ile Leu Cys Asn Ala Leu Glu Arg Arg Val Pro Trp Gln Lys
        595                 600                 605
Asp Ile Ile Pro Glu Ile Ala Ser Thr Ile Leu Glu Cys Arg Ser Gly
    610                 615                 620
Thr Leu Arg Gly Lys Asn Lys Leu Lys Gln Arg Glu Asp Lys Glu Glu
625                 630                 635                 640
Thr Trp Leu Leu Phe Leu Gly Val Asp Phe Gln Gly Lys Asp Lys Ile
                645                 650                 655
Ala Arg Glu Ile Ala Lys Leu Val Phe Gly Ser Gln Ser Lys Phe Ile
            660                 665                 670
Ser Ile Gly Leu Ser Ser Leu Gly Ser Thr Arg Ala Asp Ser Thr Glu
        675                 680                 685
Asp Phe Leu Ser Lys Gln Ala Arg Asp Glu Pro Val Gly Ser Tyr Ile
    690                 695                 700
Glu Lys Phe Ala Glu Ala Val His Glu Asn Pro His Arg Val Phe Phe
705                 710                 715                 720
Ile Glu Asp Val Glu Gln Leu Asp Tyr Ser Ser Gln Met Gly Val Lys
                725                 730                 735
Arg Gly Ile Glu Ser Gly Arg Ile Gln Ile Ala Gly Gly Glu Ala Phe
            740                 745                 750
Ser Leu Glu Asp Ala Ile Ile Ile Phe Ser Cys Glu Ser Phe Ser Ser
        755                 760                 765
Val Ser Arg Ala Ser Ser Pro Pro Pro Met Gly Leu Lys Ser Glu Glu
    770                 775                 780
Asn Glu Glu Lys Asp Arg Asp Asn Glu Leu Glu Lys Arg Ser Pro Cys
785                 790                 795                 800
Val Ser Leu Asp Leu Asn Leu Ser Ala Glu Asp Asn Gln Glu Tyr Gly
                805                 810                 815
Gln Asn Ser Val Ala Asp Thr Gly Val Leu Asp Ser Val Asp Arg Gln
            820                 825                 830
Phe Ile Phe Lys Ile Gln Glu Leu
```

835 840

<210> 49
<211> 2547
<212> DNA
<213> Vitis vinifera

<220>
<221> CDS
<222> (1)..(2547)

<400> 49
```
atg aga gcc gga ggt tgt aca gtg caa cag gct cta act gcg gag gcg       48
Met Arg Ala Gly Gly Cys Thr Val Gln Gln Ala Leu Thr Ala Glu Ala
1               5                   10                  15
gcc ggc gtg gtg aaa caa gca gta acc ctt gct agg cgg cgt gga cat       96
Ala Gly Val Val Lys Gln Ala Val Thr Leu Ala Arg Arg Arg Gly His
            20                  25                  30
gcc caa gtc act cct cta cat gta gcc aac acc atg ctc gcc gct aca       144
Ala Gln Val Thr Pro Leu His Val Ala Asn Thr Met Leu Ala Ala Thr
        35                  40                  45
aac ggc ctg ctc cga acc gct tgt ctt caa tcc cac tcc cac ccc ctc       192
Asn Gly Leu Leu Arg Thr Ala Cys Leu Gln Ser His Ser His Pro Leu
    50                  55                  60
cag tgc aaa gcc cta gaa ctt tgc ttc aac gtt gcc ctc aac cgc ctt       240
Gln Cys Lys Ala Leu Glu Leu Cys Phe Asn Val Ala Leu Asn Arg Leu
65                  70                  75                  80
cca gcc tcc aca tcc agt ccc atg ctg ggt cct cat tcc caa cat cct       288
Pro Ala Ser Thr Ser Ser Pro Met Leu Gly Pro His Ser Gln His Pro
                85                  90                  95
tcc atc tcc aac gca ttg gtt gct gcc ttc aag cga gct cag gcc cat       336
Ser Ile Ser Asn Ala Leu Val Ala Ala Phe Lys Arg Ala Gln Ala His
            100                 105                 110
cag cga cgc ggc tct att gag aac cag cag caa cct ctt cta gca gtt       384
Gln Arg Arg Gly Ser Ile Glu Asn Gln Gln Gln Pro Leu Leu Ala Val
        115                 120                 125
aaa ata gag cta gag cag ctc ata atc tct att tta gat gat cct agt       432
Lys Ile Glu Leu Glu Gln Leu Ile Ile Ser Ile Leu Asp Asp Pro Ser
    130                 135                 140
gtg agt aga gtc atg aga gaa gct ggg ttc tcc agt acc caa gtg aaa       480
Val Ser Arg Val Met Arg Glu Ala Gly Phe Ser Ser Thr Gln Val Lys
145                 150                 155                 160
agc aat gta gag caa gct gtc tcc ttg gaa atc tgc tct caa gct cca       528
Ser Asn Val Glu Gln Ala Val Ser Leu Glu Ile Cys Ser Gln Ala Pro
                165                 170                 175
tcc gtg agt agt aag tcc aag gaa agt aac ctt cta gtc ctg tcc cag       576
Ser Val Ser Ser Lys Ser Lys Glu Ser Asn Leu Leu Val Leu Ser Gln
            180                 185                 190
tct cct ccc atg ggt caa att gga gta aag cta ggc aaa cca acg gtg       624
Ser Pro Pro Met Gly Gln Ile Gly Val Lys Leu Gly Lys Pro Thr Val
        195                 200                 205
cca gat cca gtc agg aat gag gat gtg atg agt gta ata gag aat ctg       672
Pro Asp Pro Val Arg Asn Glu Asp Val Met Ser Val Ile Glu Asn Leu
    210                 215                 220
atg aat aaa agg agg aaa aat act gtg att gtt gga gag tgt ctg gcg       720
Met Asn Lys Arg Arg Lys Asn Thr Val Ile Val Gly Glu Cys Leu Ala
225                 230                 235                 240
act atc gag ggt gta gtt aga ggg gtg atg gac aaa gtt gac aag gga       768
Thr Ile Glu Gly Val Val Arg Gly Val Met Asp Lys Val Asp Lys Gly
                245                 250                 255
gat gtt cca gag gct ttg aga gat gtt aag ctt ata agc ctt cct ctc       816
Asp Val Pro Glu Ala Leu Arg Asp Val Lys Leu Ile Ser Leu Pro Leu
            260                 265                 270
ttc tct ttt gga cat cac tcc aga gaa gag gtc gaa cag aag ctc ggg       864
Phe Ser Phe Gly His His Ser Arg Glu Glu Val Glu Gln Lys Leu Gly
```

```
        275                 280                 285
gag ctc aag agc ctt gtg aag agt tgc gtg gga aga ggg gtt att ttg        912
Glu Leu Lys Ser Leu Val Lys Ser Cys Val Gly Arg Gly Val Ile Leu
    290                 295                 300
tat ttg gaa gat ctt aaa tgg act acg gat tat aga gct agt tct agt        960
Tyr Leu Glu Asp Leu Lys Trp Thr Thr Asp Tyr Arg Ala Ser Ser Ser
305                 310                 315                 320
gag caa ggg agg aac tat tat tgt ccc gtg gag cac atg atc atg gag        1008
Glu Gln Gly Arg Asn Tyr Tyr Cys Pro Val Glu His Met Ile Met Glu
                325                 330                 335
ctt ggg aaa ttg gtt tgt gga ttt ggg gag aac gga agg ttt tgg ctc        1056
Leu Gly Lys Leu Val Cys Gly Phe Gly Glu Asn Gly Arg Phe Trp Leu
            340                 345                 350
atg gga atc gca act ttc caa act tac tcg aga tgt aga act ggc cat        1104
Met Gly Ile Ala Thr Phe Gln Thr Tyr Ser Arg Cys Arg Thr Gly His
        355                 360                 365
cca tcg ctg gag act att tgg agt cta cat cct ctt aca att cct gca        1152
Pro Ser Leu Glu Thr Ile Trp Ser Leu His Pro Leu Thr Ile Pro Ala
    370                 375                 380
agc agc ttg gcc ttg agt ctc atg cct gac agt cag ttc agt agc aaa        1200
Ser Ser Leu Ala Leu Ser Leu Met Pro Asp Ser Gln Phe Ser Ser Lys
385                 390                 395                 400
aag gct gga agt ggg acc agt aat tgg ctt atg ctt gaa ggt gga gcg        1248
Lys Ala Gly Ser Gly Thr Ser Asn Trp Leu Met Leu Glu Gly Gly Ala
                405                 410                 415
gag aag cag ctt act tgc tgc gct gat tgc tca gcc aat ttc gag aat        1296
Glu Lys Gln Leu Thr Cys Cys Ala Asp Cys Ser Ala Asn Phe Glu Asn
            420                 425                 430
gaa gct cga agc ata cca acc agc act tgt aac agt gat tcc aca act        1344
Glu Ala Arg Ser Ile Pro Thr Ser Thr Cys Asn Ser Asp Ser Thr Thr
        435                 440                 445
tca acc ctt cca aca tgg ctc caa cag tac aaa gat gag aac aaa aaa        1392
Ser Thr Leu Pro Thr Trp Leu Gln Gln Tyr Lys Asp Glu Asn Lys Lys
    450                 455                 460
ctt tcc cgt aat gat cag gac tgt gta gca gtc aga gat ctc tgc aaa        1440
Leu Ser Arg Asn Asp Gln Asp Cys Val Ala Val Arg Asp Leu Cys Lys
465                 470                 475                 480
aaa tgg aat tct att tgc agt tct gcc cac aaa caa ccc cac tca tct        1488
Lys Trp Asn Ser Ile Cys Ser Ser Ala His Lys Gln Pro His Ser Ser
                485                 490                 495
gag aaa acc cta aca ttc tct tct ctt tca cct tct tcc tct act tct        1536
Glu Lys Thr Leu Thr Phe Ser Ser Leu Ser Pro Ser Ser Ser Thr Ser
            500                 505                 510
ggc ttt tca tat gac cag caa tat cct aat ttg cac cag acc cac caa        1584
Gly Phe Ser Tyr Asp Gln Gln Tyr Pro Asn Leu His Gln Thr His Gln
        515                 520                 525
ggt tgg cca gtg gtt gaa cac aaa cag tcc tgg aga gac aat cat ttc        1632
Gly Trp Pro Val Val Glu His Lys Gln Ser Trp Arg Asp Asn His Phe
    530                 535                 540
tgg gtt tcc gaa gct ctt aac aaa acc tat gaa ccc agt ttg aga atg        1680
Trp Val Ser Glu Ala Leu Asn Lys Thr Tyr Glu Pro Ser Leu Arg Met
545                 550                 555                 560
tac att ccg gag cat tct gat cgc aaa tat gcg tcg aac cct aat tct        1728
Tyr Ile Pro Glu His Ser Asp Arg Lys Tyr Ala Ser Asn Pro Asn Ser
                565                 570                 575
acc cct aat tcg gcc tct tca agc gat gtc atg gaa atg gag tac gtt        1776
Thr Pro Asn Ser Ala Ser Ser Ser Asp Val Met Glu Met Glu Tyr Val
            580                 585                 590
caa agg ttc aag gag ctc aac gct gag aac ctg aac act cta tgc aat        1824
Gln Arg Phe Lys Glu Leu Asn Ala Glu Asn Leu Asn Thr Leu Cys Asn
        595                 600                 605
gca ttg gag aaa aag gtc cca tgg cag aaa gat ata att cct gac atc        1872
Ala Leu Glu Lys Lys Val Pro Trp Gln Lys Asp Ile Ile Pro Asp Ile
    610                 615                 620
gct agc acg atc tta caa tgc agg tct gga atg gta aga cgc aaa gga        1920
```

```
Ala Ser Thr Ile Leu Gln Cys Arg Ser Gly Met Val Arg Arg Lys Gly
625                 630                 635                 640
aag gtc aaa aat agc gag acc aaa gaa gaa aca tgg ttt ttc ttc caa      1968
Lys Val Lys Asn Ser Glu Thr Lys Glu Glu Thr Trp Phe Phe Phe Gln
                645                 650                 655
ggc gtt gat atg gat gct aaa gag aag att gcg aga gaa ttg gct aga      2016
Gly Val Asp Met Asp Ala Lys Glu Lys Ile Ala Arg Glu Leu Ala Arg
            660                 665                 670
ctt gtt ttc ggc tcc cag aac aac ttc gtt tca att gct ctg agc agc      2064
Leu Val Phe Gly Ser Gln Asn Asn Phe Val Ser Ile Ala Leu Ser Ser
        675                 680                 685
ttc tca tct aca agg gcg gat tca aca gaa gat ctc cga aat aag aga      2112
Phe Ser Ser Thr Arg Ala Asp Ser Thr Glu Asp Leu Arg Asn Lys Arg
    690                 695                 700
tca aga gat gag caa agc tgc agt tac atc gag agg ttc gct gag gca      2160
Ser Arg Asp Glu Gln Ser Cys Ser Tyr Ile Glu Arg Phe Ala Glu Ala
705                 710                 715                 720
gtg ggc agt aat cca cac cgg gta ttc tta gcg gaa gat gtg gag caa      2208
Val Gly Ser Asn Pro His Arg Val Phe Leu Ala Glu Asp Val Glu Gln
                725                 730                 735
gcg gat tac tgc tcc caa atg ggt atc aaa agg gcc aca gaa aga gga      2256
Ala Asp Tyr Cys Ser Gln Met Gly Ile Lys Arg Ala Thr Glu Arg Gly
            740                 745                 750
aga ata acc aat tcg aat gga gaa gaa atc agc ctg agc gac gca atc      2304
Arg Ile Thr Asn Ser Asn Gly Glu Glu Ile Ser Leu Ser Asp Ala Ile
        755                 760                 765
atc att ttg agc tgt gaa agc ttc agc tcg agg tct aga gcc tgc tca      2352
Ile Ile Leu Ser Cys Glu Ser Phe Ser Ser Arg Ser Arg Ala Cys Ser
    770                 775                 780
cct cca atc aaa caa aaa tca gat gag ttc gaa gag gag aaa ggt gga      2400
Pro Pro Ile Lys Gln Lys Ser Asp Glu Phe Glu Glu Glu Lys Gly Gly
785                 790                 795                 800
ggt ggg ggt gag gag ata agc cca tgt gtg tct ttg gat ttg aat att      2448
Gly Gly Gly Glu Glu Ile Ser Pro Cys Val Ser Leu Asp Leu Asn Ile
                805                 810                 815
tgc att gat gat gac ggc gtt gag gat gaa tca atc gac gac atc gga      2496
Cys Ile Asp Asp Asp Gly Val Glu Asp Glu Ser Ile Asp Asp Ile Gly
            820                 825                 830
ctt ctt gaa tca gtg gat aga agg att act ttc aaa att cag gag tta      2544
Leu Leu Glu Ser Val Asp Arg Arg Ile Thr Phe Lys Ile Gln Glu Leu
        835                 840                 845
taa                                                                  2547



<210> 50
<211> 848
<212> PRT
<213> Vitis vinifera

<400> 50
Met Arg Ala Gly Gly Cys Thr Val Gln Gln Ala Leu Thr Ala Glu Ala
1               5                   10                  15
Ala Gly Val Val Lys Gln Ala Val Thr Leu Ala Arg Arg Arg Gly His
            20                  25                  30
Ala Gln Val Thr Pro Leu His Val Ala Asn Thr Met Leu Ala Ala Thr
        35                  40                  45
Asn Gly Leu Leu Arg Thr Ala Cys Leu Gln Ser His Ser His Pro Leu
    50                  55                  60
Gln Cys Lys Ala Leu Glu Leu Cys Phe Asn Val Ala Leu Asn Arg Leu
65                  70                  75                  80
Pro Ala Ser Thr Ser Ser Pro Met Leu Gly Pro His Ser Gln His Pro
                85                  90                  95
Ser Ile Ser Asn Ala Leu Val Ala Ala Phe Lys Arg Ala Gln Ala His
            100                 105                 110
```

```
Gln Arg Arg Gly Ser Ile Glu Asn Gln Gln Gln Pro Leu Leu Ala Val
        115                 120                 125
Lys Ile Glu Leu Glu Gln Leu Ile Ile Ser Ile Leu Asp Asp Pro Ser
    130                 135                 140
Val Ser Arg Val Met Arg Glu Ala Gly Phe Ser Ser Thr Gln Val Lys
145                 150                 155                 160
Ser Asn Val Glu Gln Ala Val Ser Leu Glu Ile Cys Ser Gln Ala Pro
                165                 170                 175
Ser Val Ser Ser Lys Ser Lys Glu Ser Asn Leu Leu Val Leu Ser Gln
            180                 185                 190
Ser Pro Pro Met Gly Gln Ile Gly Val Lys Leu Gly Lys Pro Thr Val
        195                 200                 205
Pro Asp Pro Val Arg Asn Glu Asp Val Met Ser Val Ile Glu Asn Leu
    210                 215                 220
Met Asn Lys Arg Arg Lys Asn Thr Val Ile Val Gly Glu Cys Leu Ala
225                 230                 235                 240
Thr Ile Glu Gly Val Val Arg Gly Val Met Asp Lys Val Asp Lys Gly
                245                 250                 255
Asp Val Pro Glu Ala Leu Arg Asp Val Lys Leu Ile Ser Leu Pro Leu
            260                 265                 270
Phe Ser Phe Gly His His Ser Arg Glu Glu Val Glu Gln Lys Leu Gly
        275                 280                 285
Glu Leu Lys Ser Leu Val Lys Ser Cys Val Gly Arg Gly Val Ile Leu
    290                 295                 300
Tyr Leu Glu Asp Leu Lys Trp Thr Thr Asp Tyr Arg Ala Ser Ser Ser
305                 310                 315                 320
Glu Gln Gly Arg Asn Tyr Tyr Cys Pro Val Glu His Met Ile Met Glu
                325                 330                 335
Leu Gly Lys Leu Val Cys Gly Phe Gly Glu Asn Gly Arg Phe Trp Leu
            340                 345                 350
Met Gly Ile Ala Thr Phe Gln Thr Tyr Ser Arg Cys Arg Thr Gly His
        355                 360                 365
Pro Ser Leu Glu Thr Ile Trp Ser Leu His Pro Leu Thr Ile Pro Ala
    370                 375                 380
Ser Ser Leu Ala Leu Ser Leu Met Pro Asp Ser Gln Phe Ser Ser Lys
385                 390                 395                 400
Lys Ala Gly Ser Gly Thr Ser Asn Trp Leu Met Leu Glu Gly Gly Ala
                405                 410                 415
Glu Lys Gln Leu Thr Cys Cys Ala Asp Cys Ser Ala Asn Phe Glu Asn
            420                 425                 430
Glu Ala Arg Ser Ile Pro Thr Ser Thr Cys Asn Ser Asp Ser Thr Thr
        435                 440                 445
Ser Thr Leu Pro Thr Trp Leu Gln Gln Tyr Lys Asp Glu Asn Lys Lys
    450                 455                 460
Leu Ser Arg Asn Asp Gln Asp Cys Val Ala Val Arg Asp Leu Cys Lys
465                 470                 475                 480
Lys Trp Asn Ser Ile Cys Ser Ser Ala His Lys Gln Pro His Ser Ser
                485                 490                 495
Glu Lys Thr Leu Thr Phe Ser Ser Leu Ser Pro Ser Ser Ser Thr Ser
            500                 505                 510
Gly Phe Ser Tyr Asp Gln Gln Tyr Pro Asn Leu His Gln Thr His Gln
        515                 520                 525
Gly Trp Pro Val Val Glu His Lys Gln Ser Trp Arg Asp Asn His Phe
    530                 535                 540
Trp Val Ser Glu Ala Leu Asn Lys Thr Tyr Glu Pro Ser Leu Arg Met
545                 550                 555                 560
Tyr Ile Pro Glu His Ser Asp Arg Lys Tyr Ala Ser Asn Pro Asn Ser
                565                 570                 575
Thr Pro Asn Ser Ala Ser Ser Ser Asp Val Met Glu Met Glu Tyr Val
            580                 585                 590
Gln Arg Phe Lys Glu Leu Asn Ala Glu Asn Leu Asn Thr Leu Cys Asn
        595                 600                 605
Ala Leu Glu Lys Lys Val Pro Trp Gln Lys Asp Ile Ile Pro Asp Ile
    610                 615                 620
Ala Ser Thr Ile Leu Gln Cys Arg Ser Gly Met Val Arg Arg Lys Gly
```

```
625                 630                 635                 640
Lys Val Lys Asn Ser Glu Thr Lys Glu Glu Thr Trp Phe Phe Phe Gln
                645                 650                 655
Gly Val Asp Met Asp Ala Lys Glu Lys Ile Ala Arg Glu Leu Ala Arg
            660                 665                 670
Leu Val Phe Gly Ser Gln Asn Asn Phe Val Ser Ile Ala Leu Ser Ser
        675                 680                 685
Phe Ser Ser Thr Arg Ala Asp Ser Thr Glu Asp Leu Arg Asn Lys Arg
    690                 695                 700
Ser Arg Asp Glu Gln Ser Cys Ser Tyr Ile Glu Arg Phe Ala Glu Ala
705                 710                 715                 720
Val Gly Ser Asn Pro His Arg Val Phe Leu Ala Glu Asp Val Glu Gln
                725                 730                 735
Ala Asp Tyr Cys Ser Gln Met Gly Ile Lys Arg Ala Thr Glu Arg Gly
            740                 745                 750
Arg Ile Thr Asn Ser Asn Gly Glu Glu Ile Ser Leu Ser Asp Ala Ile
        755                 760                 765
Ile Ile Leu Ser Cys Glu Ser Phe Ser Ser Arg Ser Arg Ala Cys Ser
    770                 775                 780
Pro Pro Ile Lys Gln Lys Ser Asp Glu Phe Glu Glu Glu Lys Gly Gly
785                 790                 795                 800
Gly Gly Gly Glu Glu Ile Ser Pro Cys Val Ser Leu Asp Leu Asn Ile
                805                 810                 815
Cys Ile Asp Asp Asp Gly Val Glu Asp Glu Ser Ile Asp Asp Ile Gly
            820                 825                 830
Leu Leu Glu Ser Val Asp Arg Arg Ile Thr Phe Lys Ile Gln Glu Leu
        835                 840                 845

<210> 51
<211> 2595
<212> DNA
<213> Ricinus communis

<220>
<221> CDS
<222> (1)..(2595)

<400> 51
atg aga gca gga ata tgc agt gtg cag caa gct ctt act gct gag gct       48
Met Arg Ala Gly Ile Cys Ser Val Gln Gln Ala Leu Thr Ala Glu Ala
1               5                   10                  15
gca aat ata gta aag caa gca gtt agc ctt gct agg cgt cga ggc cat       96
Ala Asn Ile Val Lys Gln Ala Val Ser Leu Ala Arg Arg Arg Gly His
            20                  25                  30
gcc caa gtc act cct ctt cat gtg gca agt gct atg ttg gca tct aca      144
Ala Gln Val Thr Pro Leu His Val Ala Ser Ala Met Leu Ala Ser Thr
        35                  40                  45
aat ggc ctt ctc aga aga gct tgc ctt caa tcc cat tct cat cct ctc      192
Asn Gly Leu Leu Arg Arg Ala Cys Leu Gln Ser His Ser His Pro Leu
    50                  55                  60
caa tgc aag gcc tta gag ctt tgc ttt aat gtt gct cta aat cgc cta      240
Gln Cys Lys Ala Leu Glu Leu Cys Phe Asn Val Ala Leu Asn Arg Leu
65                  70                  75                  80
cct gct tcc act tcc agt gcg ttg tta ggt cct cat tct tct tat cct      288
Pro Ala Ser Thr Ser Ser Ala Leu Leu Gly Pro His Ser Ser Tyr Pro
                85                  90                  95
tcc tta tct aat gcc ttg gtt gca gct ttt aag cgt gct cag gct cac      336
Ser Leu Ser Asn Ala Leu Val Ala Ala Phe Lys Arg Ala Gln Ala His
            100                 105                 110
cag cgt cga ggc tcc att gaa aac cag caa caa ccc att tta gct ttg      384
Gln Arg Arg Gly Ser Ile Glu Asn Gln Gln Gln Pro Ile Leu Ala Leu
        115                 120                 125
aag att gag ata gaa cag ctc ata atc tct att tta gac gac ccg agt      432
Lys Ile Glu Ile Glu Gln Leu Ile Ile Ser Ile Leu Asp Asp Pro Ser
    130                 135                 140
```

```
gtt agt aga gtt atg agg gaa gct ggt ttc tca agt acc caa gtg aag        480
Val Ser Arg Val Met Arg Glu Ala Gly Phe Ser Ser Thr Gln Val Lys
145                 150                 155                 160
aac aag gta gaa cag gca gtt tct ttg gag att tgt tct caa ggc act        528
Asn Lys Val Glu Gln Ala Val Ser Leu Glu Ile Cys Ser Gln Gly Thr
                165                 170                 175
act gct aca agt tgc cag tct aaa gaa atc act aaa cct caa att ttt        576
Thr Ala Thr Ser Cys Gln Ser Lys Glu Ile Thr Lys Pro Gln Ile Phe
            180                 185                 190
agc act aat aat gtg tct ccg tct ctt cct ttt agt cat tat gga gtc        624
Ser Thr Asn Asn Val Ser Pro Ser Leu Pro Phe Ser His Tyr Gly Val
        195                 200                 205
aca cta agc aag cca tta gat cat gag gtt agt aat gat gat gta atg        672
Thr Leu Ser Lys Pro Leu Asp His Glu Val Ser Asn Asp Asp Val Met
    210                 215                 220
agt gtt tta aac aca ttg atg gag aaa aag aga aac aca atc att aca        720
Ser Val Leu Asn Thr Leu Met Glu Lys Lys Arg Asn Thr Ile Ile Thr
225                 230                 235                 240
gga gaa tgt cta gct agt aca gaa agt gta gtt aga cta gtc atg aat        768
Gly Glu Cys Leu Ala Ser Thr Glu Ser Val Val Arg Leu Val Met Asn
                245                 250                 255
aag att gag aga ggc ctt gct cct ggg gag ttg aga gcg atg cgg ttc        816
Lys Ile Glu Arg Gly Leu Ala Pro Gly Glu Leu Arg Ala Met Arg Phe
            260                 265                 270
ata agc ttt cct ctc atc tct ttg aga gat ctt cca caa gag gag gtt        864
Ile Ser Phe Pro Leu Ile Ser Leu Arg Asp Leu Pro Gln Glu Glu Val
        275                 280                 285
gag caa aag ctt gta gag ctt agg tgc aca gtg aag agc tat ttg aat        912
Glu Gln Lys Leu Val Glu Leu Arg Cys Thr Val Lys Ser Tyr Leu Asn
    290                 295                 300
aga ggg gtt ttc tta tat ttg ggt gat atc aaa tgg gtt gct gag ttt        960
Arg Gly Val Phe Leu Tyr Leu Gly Asp Ile Lys Trp Val Ala Glu Phe
305                 310                 315                 320
tgg tcg gaa tac ggt gag caa agg aga agc tat tac tgc tct ggg gag       1008
Trp Ser Glu Tyr Gly Glu Gln Arg Arg Ser Tyr Tyr Cys Ser Gly Glu
                325                 330                 335
tac ata atc atg gag ctg aaa aga tta atc cgc gga att ggt gaa act       1056
Tyr Ile Ile Met Glu Leu Lys Arg Leu Ile Arg Gly Ile Gly Glu Thr
            340                 345                 350
gaa agg ctg tgg ctc atg gga gtt gca act ttc cag aca tac atg aaa       1104
Glu Arg Leu Trp Leu Met Gly Val Ala Thr Phe Gln Thr Tyr Met Lys
        355                 360                 365
tgt aag tca ggc cgc cct tct cta gag act att tgg gaa tta tat cct       1152
Cys Lys Ser Gly Arg Pro Ser Leu Glu Thr Ile Trp Glu Leu Tyr Pro
    370                 375                 380
ctc cca att cca gtt ggt agc ctg agc ctg agt ctc aat ctt gac agt       1200
Leu Pro Ile Pro Val Gly Ser Leu Ser Leu Ser Leu Asn Leu Asp Ser
385                 390                 395                 400
gac cta caa tgt cgg tac cga agc aaa gta tct acc aat gga tat ggt       1248
Asp Leu Gln Cys Arg Tyr Arg Ser Lys Val Ser Thr Asn Gly Tyr Gly
                405                 410                 415
tgg ccg aag ctc gaa tct gca gtt gat aat cac tca act tgt ttc aca       1296
Trp Pro Lys Leu Glu Ser Ala Val Asp Asn His Ser Thr Cys Phe Thr
            420                 425                 430
gac ttc tct gtc aat ttc aat aga gat gct caa agc ata ggt tgc agt       1344
Asp Phe Ser Val Asn Phe Asn Arg Asp Ala Gln Ser Ile Gly Cys Ser
        435                 440                 445
cag agg gag ttc act acc aat ttt aca gtt tcc act agc tca agc ttg       1392
Gln Arg Glu Phe Thr Thr Asn Phe Thr Val Ser Thr Ser Ser Ser Leu
    450                 455                 460
cct tca tgg ctc aag cag cat aaa gta gag aca gaa aga atc act att       1440
Pro Ser Trp Leu Lys Gln His Lys Val Glu Thr Glu Arg Ile Thr Ile
465                 470                 475                 480
gat gat aag gaa tat tgt acg aat aca agc cct ctt ctg aag aaa tgg       1488
Asp Asp Lys Glu Tyr Cys Thr Asn Thr Ser Pro Leu Leu Lys Lys Trp
```

```
                485                 490                 495
aac tca ttt ggc agc tca ttt cat aat aaa gaa tct cac tct cct cca        1536
Asn Ser Phe Gly Ser Ser Phe His Asn Lys Glu Ser His Ser Pro Pro
            500                 505                 510
aaa acc ata aag ttt gct tca tct cct gct tcc cca att tcc att tct        1584
Lys Thr Ile Lys Phe Ala Ser Ser Pro Ala Ser Pro Ile Ser Ile Ser
        515                 520                 525
tcc cat gag tgc aac acc aac ata aat caa gcg cct tta agc tgg cca        1632
Ser His Glu Cys Asn Thr Asn Ile Asn Gln Ala Pro Leu Ser Trp Pro
    530                 535                 540
gtc att ttc gag cca aga cag ttt cag aaa gag cag aag att tgg ttg        1680
Val Ile Phe Glu Pro Arg Gln Phe Gln Lys Glu Gln Lys Ile Trp Leu
545                 550                 555                 560
tct gaa tgt aac aat gcc gaa ggc tct gaa agc aat ttg att agc gtt        1728
Ser Glu Cys Asn Asn Ala Glu Gly Ser Glu Ser Asn Leu Ile Ser Val
                565                 570                 575
acc aaa cca gag ctt tta tca aat cct aac tct agc cca aat tca gct        1776
Thr Lys Pro Glu Leu Leu Ser Asn Pro Asn Ser Ser Pro Asn Ser Ala
            580                 585                 590
tct tca agt gaa gca gtg gac ggc aca gaa ggc tta caa agc ttc aag        1824
Ser Ser Ser Glu Ala Val Asp Gly Thr Glu Gly Leu Gln Ser Phe Lys
        595                 600                 605
gag ctc aat aac caa aac ttg aag att cta tgc agt agc ttg gag aaa        1872
Glu Leu Asn Asn Gln Asn Leu Lys Ile Leu Cys Ser Ser Leu Glu Lys
    610                 615                 620
aag gtt cca tgg cag aaa gat att att ccc gaa atc gcg act gcc att        1920
Lys Val Pro Trp Gln Lys Asp Ile Ile Pro Glu Ile Ala Thr Ala Ile
625                 630                 635                 640
ctt gag tgc agg tcc ggg agg agc aag agc aaa cgc aag tcc aat aac        1968
Leu Glu Cys Arg Ser Gly Arg Ser Lys Ser Lys Arg Lys Ser Asn Asn
                645                 650                 655
cga gca gaa aga gaa gaa act tgg ctc ttc ttc tta gga gtt gat tct        2016
Arg Ala Glu Arg Glu Glu Thr Trp Leu Phe Phe Leu Gly Val Asp Ser
            660                 665                 670
gaa gga aag gaa aag att gca aga gag tta gcc aga ctt gtc tat ggc        2064
Glu Gly Lys Glu Lys Ile Ala Arg Glu Leu Ala Arg Leu Val Tyr Gly
        675                 680                 685
tcc caa gca aac ttt gtt tca att ggt cta agc aac tat tca tca aca        2112
Ser Gln Ala Asn Phe Val Ser Ile Gly Leu Ser Asn Tyr Ser Ser Thr
    690                 695                 700
aga act gat tca act gat gaa tcc aag aat aag agg ggt aga gat gag        2160
Arg Thr Asp Ser Thr Asp Glu Ser Lys Asn Lys Arg Gly Arg Asp Glu
705                 710                 715                 720
ttg ggt tgt ggt tat cat gag agg ttc ggc ctt gca ttg aat gag aat        2208
Leu Gly Cys Gly Tyr His Glu Arg Phe Gly Leu Ala Leu Asn Glu Asn
                725                 730                 735
cct cat cga gta ttc ttc atg gaa gat gtg gag caa gtt gat tac tgt        2256
Pro His Arg Val Phe Phe Met Glu Asp Val Glu Gln Val Asp Tyr Cys
            740                 745                 750
tct caa aag gca atc aag aaa gcc att gaa agt gga aaa gta gca ctt        2304
Ser Gln Lys Ala Ile Lys Lys Ala Ile Glu Ser Gly Lys Val Ala Leu
        755                 760                 765
cct ggt ggc gaa aat gct cct ctg aaa gat gcc att atc att ttc ggt        2352
Pro Gly Gly Glu Asn Ala Pro Leu Lys Asp Ala Ile Ile Ile Phe Gly
    770                 775                 780
agt gaa agt tat agt tca gcg tca aga gct tgt tca cct tca aga aga        2400
Ser Glu Ser Tyr Ser Ser Ala Ser Arg Ala Cys Ser Pro Ser Arg Arg
785                 790                 795                 800
gta aaa agt agt ggg gag aaa gaa gtc aag gat gaa gag gat gaa tca        2448
Val Lys Ser Ser Gly Glu Lys Glu Val Lys Asp Glu Glu Asp Glu Ser
                805                 810                 815
gat gag aag aac aaa gtt ctc tca ctt gac ttg aac att gcc att gat        2496
Asp Glu Lys Asn Lys Val Leu Ser Leu Asp Leu Asn Ile Ala Ile Asp
            820                 825                 830
gtt aat gac gat gat gaa gat gaa tac tca aac att gct gat aat gga        2544
```

```
Val Asn Asp Asp Asp Glu Asp Glu Tyr Ser Asn Ile Ala Asp Asn Gly
        835                 840                 845
atc cta caa tct gtt gat agg caa att ctt ttc aaa att caa gaa ctg       2592
Ile Leu Gln Ser Val Asp Arg Gln Ile Leu Phe Lys Ile Gln Glu Leu
    850                 855                 860
tga                                                                   2595


<210> 52
<211> 864
<212> PRT
<213> Ricinus communis

<400> 52
Met Arg Ala Gly Ile Cys Ser Val Gln Gln Ala Leu Thr Ala Glu Ala
1               5                   10                  15
Ala Asn Ile Val Lys Gln Ala Val Ser Leu Ala Arg Arg Arg Gly His
            20                  25                  30
Ala Gln Val Thr Pro Leu His Val Ala Ser Ala Met Leu Ala Ser Thr
        35                  40                  45
Asn Gly Leu Leu Arg Arg Ala Cys Leu Gln Ser His Ser His Pro Leu
    50                  55                  60
Gln Cys Lys Ala Leu Glu Leu Cys Phe Asn Val Ala Leu Asn Arg Leu
65                  70                  75                  80
Pro Ala Ser Thr Ser Ser Ala Leu Leu Gly Pro His Ser Ser Tyr Pro
                85                  90                  95
Ser Leu Ser Asn Ala Leu Val Ala Ala Phe Lys Arg Ala Gln Ala His
            100                 105                 110
Gln Arg Arg Gly Ser Ile Glu Asn Gln Gln Gln Pro Ile Leu Ala Leu
        115                 120                 125
Lys Ile Glu Ile Glu Gln Leu Ile Ile Ser Ile Leu Asp Asp Pro Ser
    130                 135                 140
Val Ser Arg Val Met Arg Glu Ala Gly Phe Ser Ser Thr Gln Val Lys
145                 150                 155                 160
Asn Lys Val Glu Gln Ala Val Ser Leu Glu Ile Cys Ser Gln Gly Thr
                165                 170                 175
Thr Ala Thr Ser Cys Gln Ser Lys Glu Ile Thr Lys Pro Gln Ile Phe
            180                 185                 190
Ser Thr Asn Asn Val Ser Pro Ser Leu Pro Phe Ser His Tyr Gly Val
        195                 200                 205
Thr Leu Ser Lys Pro Leu Asp His Glu Val Ser Asn Asp Asp Val Met
    210                 215                 220
Ser Val Leu Asn Thr Leu Met Glu Lys Lys Arg Asn Thr Ile Ile Thr
225                 230                 235                 240
Gly Glu Cys Leu Ala Ser Thr Glu Ser Val Val Arg Leu Val Met Asn
                245                 250                 255
Lys Ile Glu Arg Gly Leu Ala Pro Gly Glu Leu Arg Ala Met Arg Phe
            260                 265                 270
Ile Ser Phe Pro Leu Ile Ser Leu Arg Asp Leu Pro Gln Glu Glu Val
        275                 280                 285
Glu Gln Lys Leu Val Glu Leu Arg Cys Thr Val Lys Ser Tyr Leu Asn
    290                 295                 300
Arg Gly Val Phe Leu Tyr Leu Gly Asp Ile Lys Trp Val Ala Glu Phe
305                 310                 315                 320
Trp Ser Glu Tyr Gly Glu Gln Arg Arg Ser Tyr Tyr Cys Ser Gly Glu
                325                 330                 335
Tyr Ile Ile Met Glu Leu Lys Arg Leu Ile Arg Gly Ile Gly Glu Thr
            340                 345                 350
Glu Arg Leu Trp Leu Met Gly Val Ala Thr Phe Gln Thr Tyr Met Lys
        355                 360                 365
Cys Lys Ser Gly Arg Pro Ser Leu Glu Thr Ile Trp Glu Leu Tyr Pro
    370                 375                 380
Leu Pro Ile Pro Val Gly Ser Leu Ser Leu Ser Leu Asn Leu Asp Ser
385                 390                 395                 400
```

```
Asp Leu Gln Cys Arg Tyr Arg Ser Lys Val Ser Thr Asn Gly Tyr Gly
                405                 410                 415
Trp Pro Lys Leu Glu Ser Ala Val Asp Asn His Ser Thr Cys Phe Thr
            420                 425                 430
Asp Phe Ser Val Asn Phe Asn Arg Asp Ala Gln Ser Ile Gly Cys Ser
        435                 440                 445
Gln Arg Glu Phe Thr Thr Asn Phe Thr Val Ser Thr Ser Ser Ser Leu
    450                 455                 460
Pro Ser Trp Leu Lys Gln His Lys Val Glu Thr Glu Arg Ile Thr Ile
465                 470                 475                 480
Asp Asp Lys Glu Tyr Cys Thr Asn Thr Ser Pro Leu Leu Lys Lys Trp
                485                 490                 495
Asn Ser Phe Gly Ser Ser Phe His Asn Lys Glu Ser His Ser Pro Pro
            500                 505                 510
Lys Thr Ile Lys Phe Ala Ser Ser Pro Ala Ser Pro Ile Ser Ile Ser
        515                 520                 525
Ser His Glu Cys Asn Thr Asn Ile Asn Gln Ala Pro Leu Ser Trp Pro
    530                 535                 540
Val Ile Phe Glu Pro Arg Gln Phe Gln Lys Glu Gln Lys Ile Trp Leu
545                 550                 555                 560
Ser Glu Cys Asn Asn Ala Glu Gly Ser Glu Ser Asn Leu Ile Ser Val
                565                 570                 575
Thr Lys Pro Glu Leu Leu Ser Asn Pro Asn Ser Ser Pro Asn Ser Ala
            580                 585                 590
Ser Ser Ser Glu Ala Val Asp Gly Thr Glu Gly Leu Gln Ser Phe Lys
        595                 600                 605
Glu Leu Asn Asn Gln Asn Leu Lys Ile Leu Cys Ser Ser Leu Glu Lys
    610                 615                 620
Lys Val Pro Trp Gln Lys Asp Ile Ile Pro Glu Ile Ala Thr Ala Ile
625                 630                 635                 640
Leu Glu Cys Arg Ser Gly Arg Ser Lys Ser Lys Arg Lys Ser Asn Asn
                645                 650                 655
Arg Ala Glu Arg Glu Glu Thr Trp Leu Phe Phe Leu Gly Val Asp Ser
            660                 665                 670
Glu Gly Lys Glu Lys Ile Ala Arg Glu Leu Ala Arg Leu Val Tyr Gly
        675                 680                 685
Ser Gln Ala Asn Phe Val Ser Ile Gly Leu Ser Asn Tyr Ser Ser Thr
    690                 695                 700
Arg Thr Asp Ser Thr Asp Glu Ser Lys Asn Lys Arg Gly Arg Asp Glu
705                 710                 715                 720
Leu Gly Cys Gly Tyr His Glu Arg Phe Gly Leu Ala Leu Asn Glu Asn
                725                 730                 735
Pro His Arg Val Phe Phe Met Glu Asp Val Glu Gln Val Asp Tyr Cys
            740                 745                 750
Ser Gln Lys Ala Ile Lys Lys Ala Ile Glu Ser Gly Lys Val Ala Leu
        755                 760                 765
Pro Gly Gly Glu Asn Ala Pro Leu Lys Asp Ala Ile Ile Ile Phe Gly
    770                 775                 780
Ser Glu Ser Tyr Ser Ser Ala Ser Arg Ala Cys Ser Pro Ser Arg Arg
785                 790                 795                 800
Val Lys Ser Ser Gly Glu Lys Glu Val Lys Asp Glu Glu Asp Glu Ser
                805                 810                 815
Asp Glu Lys Asn Lys Val Leu Ser Leu Asp Leu Asn Ile Ala Ile Asp
            820                 825                 830
Val Asn Asp Asp Asp Glu Asp Glu Tyr Ser Asn Ile Ala Asp Asn Gly
        835                 840                 845
Ile Leu Gln Ser Val Asp Arg Gln Ile Leu Phe Lys Ile Gln Glu Leu
    850                 855                 860
```

<210> 53
<211> 2649
<212> DNA
<213> Ricinus communis

<220>

<221> CDS
<222> (1)..(2649)

<400> 53
atg aga gca gga ggt tgc aca gtg caa caa gca cta acc aca gag gca 48
Met Arg Ala Gly Gly Cys Thr Val Gln Gln Ala Leu Thr Thr Glu Ala
1 5 10 15
gca acg gtc gta aaa caa gct gta act ttg gct aga cgg cga ggc cat 96
Ala Thr Val Val Lys Gln Ala Val Thr Leu Ala Arg Arg Arg Gly His
20 25 30
gcc caa gtt act cct ctt cat gta gct aac acc atg ctt tct tct tct 144
Ala Gln Val Thr Pro Leu His Val Ala Asn Thr Met Leu Ser Ser Ser
35 40 45
act ggt tta tta aga aca gct tgt ctc caa tct cac tct cat cct ctt 192
Thr Gly Leu Leu Arg Thr Ala Cys Leu Gln Ser His Ser His Pro Leu
50 55 60
cag tgc aaa gcc tta gag ctc tgt ttc aac gtt gcg ctt aat cgt ctt 240
Gln Cys Lys Ala Leu Glu Leu Cys Phe Asn Val Ala Leu Asn Arg Leu
65 70 75 80
cca gca tca acg tca agc cct gtg tta ggt act cac gct caa caa tac 288
Pro Ala Ser Thr Ser Ser Pro Val Leu Gly Thr His Ala Gln Gln Tyr
85 90 95
cct tca atc tct aat gcc tta gtt gca gct ttc aag cgt gct caa gct 336
Pro Ser Ile Ser Asn Ala Leu Val Ala Ala Phe Lys Arg Ala Gln Ala
100 105 110
cat caa cga cga ggc tcg atc gag aat cag cag caa cct ctc tta gct 384
His Gln Arg Arg Gly Ser Ile Glu Asn Gln Gln Gln Pro Leu Leu Ala
115 120 125
gtg aaa ata gag cta gag cag ctt ata ata tcc att tta gat gat cct 432
Val Lys Ile Glu Leu Glu Gln Leu Ile Ile Ser Ile Leu Asp Asp Pro
130 135 140
agt gtt agt aga gtg atg aga gaa gct ggt ttc tca agt act caa gtg 480
Ser Val Ser Arg Val Met Arg Glu Ala Gly Phe Ser Ser Thr Gln Val
145 150 155 160
aaa agc aac gtt gag caa gct gtt tct tta gaa ata tgt tct caa aac 528
Lys Ser Asn Val Glu Gln Ala Val Ser Leu Glu Ile Cys Ser Gln Asn
165 170 175
agt gct cct gta agt agt agc aag tct aaa gaa agc aat aac aat aat 576
Ser Ala Pro Val Ser Ser Ser Lys Ser Lys Glu Ser Asn Asn Asn Asn
180 185 190
agt gtt tta gca ctt tct cat act caa gta gga gct aga aca agc tgt 624
Ser Val Leu Ala Leu Ser His Thr Gln Val Gly Ala Arg Thr Ser Cys
195 200 205
aga tca tca cca aca acc agt act act tct tta gat cca att agg aag 672
Arg Ser Ser Pro Thr Thr Ser Thr Thr Ser Leu Asp Pro Ile Arg Lys
210 215 220
gag gat gtg atg agt gtt ata gag aat tta ata aat aaa aga aaa aga 720
Glu Asp Val Met Ser Val Ile Glu Asn Leu Ile Asn Lys Arg Lys Arg
225 230 235 240
agt gtt gtg att gta ggg gag tgt ctt gtt agt tta gaa ggt gtt gtt 768
Ser Val Val Ile Val Gly Glu Cys Leu Val Ser Leu Glu Gly Val Val
245 250 255
aaa gga gtt atg gac aag gtt att aaa gga gat gtt cct gaa gct tta 816
Lys Gly Val Met Asp Lys Val Ile Lys Gly Asp Val Pro Glu Ala Leu
260 265 270
aaa gaa gtt aag ttt ata tca ttt cca tta tct tct cta ggt cat ctc 864
Lys Glu Val Lys Phe Ile Ser Phe Pro Leu Ser Ser Leu Gly His Leu
275 280 285
tct tct aga gta gag gta gac caa aag ctt gaa gag ctt aaa gtt cat 912
Ser Ser Arg Val Glu Val Asp Gln Lys Leu Glu Glu Leu Lys Val His
290 295 300
att aga agc tat ttg agc aaa ggg gtt gtt ttg aat tta ggg gat ctt 960
Ile Arg Ser Tyr Leu Ser Lys Gly Val Val Leu Asn Leu Gly Asp Leu
305 310 315 320
aag tgg gtt gtc gag tat agg gct aat aac ttg agt cca atg gag cat 1008

```
Lys Trp Val Val Glu Tyr Arg Ala Asn Asn Leu Ser Pro Met Glu His
                325                 330                 335
atg atc atg gag att ggc aag tta gct agt gga atc agt gag aac aat        1056
Met Ile Met Glu Ile Gly Lys Leu Ala Ser Gly Ile Ser Glu Asn Asn
            340                 345                 350
ggg aaa ttt tgg cta acg gga att gca act ttc caa act tac atg aag        1104
Gly Lys Phe Trp Leu Thr Gly Ile Ala Thr Phe Gln Thr Tyr Met Lys
        355                 360                 365
tgc aaa tcg gga aat cca tca ctt gag act gtt tgg ggt ctt cat gct        1152
Cys Lys Ser Gly Asn Pro Ser Leu Glu Thr Val Trp Gly Leu His Ala
    370                 375                 380
ctt aca att cca gca ggc agc tta cgc tta agt ctc atc acc gac agt        1200
Leu Thr Ile Pro Ala Gly Ser Leu Arg Leu Ser Leu Ile Thr Asp Ser
385                 390                 395                 400
aat aaa gtt ggt caa gat ggg tct aga tgt tgg att atg ctt gaa ggt        1248
Asn Lys Val Gly Gln Asp Gly Ser Arg Cys Trp Ile Met Leu Glu Gly
                405                 410                 415
gaa gaa gag aag caa ctt act tgc tgt gtt gat tgt aca tca aag ttt        1296
Glu Glu Glu Lys Gln Leu Thr Cys Cys Val Asp Cys Thr Ser Lys Phe
            420                 425                 430
gag aat gag gct aga agc tta caa agt agc act tct aat agt gac tcc        1344
Glu Asn Glu Ala Arg Ser Leu Gln Ser Ser Thr Ser Asn Ser Asp Ser
        435                 440                 445
aca acc act tct acc ctt cct gca tgg ctt caa caa tac aaa aat gag        1392
Thr Thr Thr Ser Thr Leu Pro Ala Trp Leu Gln Gln Tyr Lys Asn Glu
    450                 455                 460
aac cag ggc gtt aat aac aat aat gat cag gat tgt gtc tcg atc aaa        1440
Asn Gln Gly Val Asn Asn Asn Asn Asp Gln Asp Cys Val Ser Ile Lys
465                 470                 475                 480
gat ctt tgc aaa aag tgg aac tct att tgc agt tcg atc cac caa aaa        1488
Asp Leu Cys Lys Lys Trp Asn Ser Ile Cys Ser Ser Ile His Gln Lys
                485                 490                 495
ccc tat tct tct gag aaa acc att aca ttt tct tct gta tca cct tct        1536
Pro Tyr Ser Ser Glu Lys Thr Ile Thr Phe Ser Ser Val Ser Pro Ser
            500                 505                 510
tct ttt act tct agc ttc tca tat gac cac caa tac cct aat ttt cat        1584
Ser Phe Thr Ser Ser Phe Ser Tyr Asp His Gln Tyr Pro Asn Phe His
        515                 520                 525
cac acc tat cat caa cgc gac tgg cca gtg gtt gaa tcc aag caa tca        1632
His Thr Tyr His Gln Arg Asp Trp Pro Val Val Glu Ser Lys Gln Ser
    530                 535                 540
tgg aga gac cac cac ttc tgg gtt ggt tca gaa act gtc aac aag att        1680
Trp Arg Asp His His Phe Trp Val Gly Ser Glu Thr Val Asn Lys Ile
545                 550                 555                 560
aac agc tgt atc agt att gaa ccc agt ttg aga atg tac atc cca gag        1728
Asn Ser Cys Ile Ser Ile Glu Pro Ser Leu Arg Met Tyr Ile Pro Glu
                565                 570                 575
cat aat aga gat caa tat ccc aaa cca aca ata cca ttt tca tcg aat        1776
His Asn Arg Asp Gln Tyr Pro Lys Pro Thr Ile Pro Phe Ser Ser Asn
            580                 585                 590
ccg aat tct act cct aac tca act tct tcc agt gat gtt atg gaa atg        1824
Pro Asn Ser Thr Pro Asn Ser Thr Ser Ser Ser Asp Val Met Glu Met
        595                 600                 605
gag cat ctt aac aag ttc aaa gaa atg aat gct gaa aac ctg aaa atc        1872
Glu His Leu Asn Lys Phe Lys Glu Met Asn Ala Glu Asn Leu Lys Ile
    610                 615                 620
tta tgc aat gca ttg gag aaa aag gtt aca tgg cag aaa gat ata atc        1920
Leu Cys Asn Ala Leu Glu Lys Lys Val Thr Trp Gln Lys Asp Ile Ile
625                 630                 635                 640
cct gac atc gcg agc aca atc ttg caa tgc aga tca ggc atg gta aga        1968
Pro Asp Ile Ala Ser Thr Ile Leu Gln Cys Arg Ser Gly Met Val Arg
                645                 650                 655
aga aaa ggg aag gtg acg aga aac agt agt acc gag caa gct aaa gaa        2016
Arg Lys Gly Lys Val Thr Arg Asn Ser Ser Thr Glu Gln Ala Lys Glu
            660                 665                 670
```

```
gaa act tgg tta ttg ttt caa gga gtt gat gtg gaa gct aaa gaa aag      2064
Glu Thr Trp Leu Leu Phe Gln Gly Val Asp Val Glu Ala Lys Glu Lys
        675                 680                 685
ata gct aaa gaa ttg gct aag cta atc ttc ggc tcg cag aat aac ttc      2112
Ile Ala Lys Glu Leu Ala Lys Leu Ile Phe Gly Ser Gln Asn Asn Phe
    690                 695                 700
att tca att tct tta agc agt ttt tca tct aca aga gca gac tca acg      2160
Ile Ser Ile Ser Leu Ser Ser Phe Ser Ser Thr Arg Ala Asp Ser Thr
705                 710                 715                 720
gaa gac tgt aga aac aaa aga tca aga gat gaa caa agt tgc agt tat      2208
Glu Asp Cys Arg Asn Lys Arg Ser Arg Asp Glu Gln Ser Cys Ser Tyr
                725                 730                 735
ata gaa aga ttt gct gaa gct gta tca agc aat cca cat agg gtt ttt      2256
Ile Glu Arg Phe Ala Glu Ala Val Ser Ser Asn Pro His Arg Val Phe
            740                 745                 750
ttg gtg gaa gac gtt gag caa gca gat tat tgc tcg caa gtt ggt ttc      2304
Leu Val Glu Asp Val Glu Gln Ala Asp Tyr Cys Ser Gln Val Gly Phe
        755                 760                 765
aaa aga gca ata gaa aga gga aga ata aca aat gtt aag ggt gaa gaa      2352
Lys Arg Ala Ile Glu Arg Gly Arg Ile Thr Asn Val Lys Gly Glu Glu
    770                 775                 780
gtt ggt ctt agt gat gcc att atc att ttg agc tgt gaa agt ttc agt      2400
Val Gly Leu Ser Asp Ala Ile Ile Ile Leu Ser Cys Glu Ser Phe Ser
785                 790                 795                 800
tca aga tca aga gct tgt tct cct cct gtc aag caa aaa aca gat gac      2448
Ser Arg Ser Arg Ala Cys Ser Pro Pro Val Lys Gln Lys Thr Asp Asp
                805                 810                 815
tat att att tct caa gat caa gaa gaa gag aaa ggt caa ggt gct aaa      2496
Tyr Ile Ile Ser Gln Asp Gln Glu Glu Glu Lys Gly Gln Gly Ala Lys
            820                 825                 830
atg gag gag tca agt ccc tgt gta tca ctt gat ctg aat ata tcc att      2544
Met Glu Glu Ser Ser Pro Cys Val Ser Leu Asp Leu Asn Ile Ser Ile
        835                 840                 845
gat gat gac agt att gag gat cgg tca atc gat gat att ggt ctt ctt      2592
Asp Asp Asp Ser Ile Glu Asp Arg Ser Ile Asp Asp Ile Gly Leu Leu
    850                 855                 860
gaa tct gtt gat aga cga att gtt ttc aaa atc caa gag cta cga ttc      2640
Glu Ser Val Asp Arg Arg Ile Val Phe Lys Ile Gln Glu Leu Arg Phe
865                 870                 875                 880
atg tca taa                                                          2649
Met Ser


<210> 54
<211> 882
<212> PRT
<213> Ricinus communis

<400> 54
Met Arg Ala Gly Gly Cys Thr Val Gln Gln Ala Leu Thr Thr Glu Ala
1               5                   10                  15
Ala Thr Val Val Lys Gln Ala Val Thr Leu Ala Arg Arg Arg Gly His
            20                  25                  30
Ala Gln Val Thr Pro Leu His Val Ala Asn Thr Met Leu Ser Ser Ser
        35                  40                  45
Thr Gly Leu Leu Arg Thr Ala Cys Leu Gln Ser His Ser His Pro Leu
    50                  55                  60
Gln Cys Lys Ala Leu Glu Leu Cys Phe Asn Val Ala Leu Asn Arg Leu
65                  70                  75                  80
Pro Ala Ser Thr Ser Ser Pro Val Leu Gly Thr His Ala Gln Gln Tyr
                85                  90                  95
Pro Ser Ile Ser Asn Ala Leu Val Ala Ala Phe Lys Arg Ala Gln Ala
            100                 105                 110
His Gln Arg Arg Gly Ser Ile Glu Asn Gln Gln Gln Pro Leu Leu Ala
        115                 120                 125
```

```
Val Lys Ile Glu Leu Glu Gln Leu Ile Ile Ser Ile Leu Asp Asp Pro
    130                 135                 140
Ser Val Ser Arg Val Met Arg Glu Ala Gly Phe Ser Ser Thr Gln Val
145                 150                 155                 160
Lys Ser Asn Val Glu Gln Ala Val Ser Leu Glu Ile Cys Ser Gln Asn
                165                 170                 175
Ser Ala Pro Val Ser Ser Ser Lys Ser Lys Glu Ser Asn Asn Asn Asn
            180                 185                 190
Ser Val Leu Ala Leu Ser His Thr Gln Val Gly Ala Arg Thr Ser Cys
        195                 200                 205
Arg Ser Ser Pro Thr Thr Ser Thr Thr Ser Leu Asp Pro Ile Arg Lys
    210                 215                 220
Glu Asp Val Met Ser Val Ile Glu Asn Leu Ile Asn Lys Arg Lys Arg
225                 230                 235                 240
Ser Val Val Ile Val Gly Glu Cys Leu Val Ser Leu Glu Gly Val Val
                245                 250                 255
Lys Gly Val Met Asp Lys Val Ile Lys Gly Asp Val Pro Glu Ala Leu
            260                 265                 270
Lys Glu Val Lys Phe Ile Ser Phe Pro Leu Ser Ser Leu Gly His Leu
        275                 280                 285
Ser Ser Arg Val Glu Val Asp Gln Lys Leu Glu Glu Leu Lys Val His
    290                 295                 300
Ile Arg Ser Tyr Leu Ser Lys Gly Val Val Leu Asn Leu Gly Asp Leu
305                 310                 315                 320
Lys Trp Val Val Glu Tyr Arg Ala Asn Asn Leu Ser Pro Met Glu His
                325                 330                 335
Met Ile Met Glu Ile Gly Lys Leu Ala Ser Gly Ile Ser Glu Asn Asn
            340                 345                 350
Gly Lys Phe Trp Leu Thr Gly Ile Ala Thr Phe Gln Thr Tyr Met Lys
        355                 360                 365
Cys Lys Ser Gly Asn Pro Ser Leu Glu Thr Val Trp Gly Leu His Ala
    370                 375                 380
Leu Thr Ile Pro Ala Gly Ser Leu Arg Leu Ser Leu Ile Thr Asp Ser
385                 390                 395                 400
Asn Lys Val Gly Gln Asp Gly Ser Arg Cys Trp Ile Met Leu Glu Gly
                405                 410                 415
Glu Glu Glu Lys Gln Leu Thr Cys Cys Val Asp Cys Thr Ser Lys Phe
            420                 425                 430
Glu Asn Glu Ala Arg Ser Leu Gln Ser Ser Thr Ser Asn Ser Asp Ser
        435                 440                 445
Thr Thr Thr Ser Thr Leu Pro Ala Trp Leu Gln Gln Tyr Lys Asn Glu
    450                 455                 460
Asn Gln Gly Val Asn Asn Asn Asn Asp Gln Asp Cys Val Ser Ile Lys
465                 470                 475                 480
Asp Leu Cys Lys Lys Trp Asn Ser Ile Cys Ser Ser Ile His Gln Lys
                485                 490                 495
Pro Tyr Ser Ser Glu Lys Thr Ile Thr Phe Ser Ser Val Ser Pro Ser
            500                 505                 510
Ser Phe Thr Ser Ser Phe Ser Tyr Asp His Gln Tyr Pro Asn Phe His
        515                 520                 525
His Thr Tyr His Gln Arg Asp Trp Pro Val Val Glu Ser Lys Gln Ser
    530                 535                 540
Trp Arg Asp His His Phe Trp Val Gly Ser Glu Thr Val Asn Lys Ile
545                 550                 555                 560
Asn Ser Cys Ile Ser Ile Glu Pro Ser Leu Arg Met Tyr Ile Pro Glu
                565                 570                 575
His Asn Arg Asp Gln Tyr Pro Lys Pro Thr Ile Pro Phe Ser Ser Asn
            580                 585                 590
Pro Asn Ser Thr Pro Asn Ser Thr Ser Ser Ser Asp Val Met Glu Met
        595                 600                 605
Glu His Leu Asn Lys Phe Lys Glu Met Asn Ala Glu Asn Leu Lys Ile
    610                 615                 620
Leu Cys Asn Ala Leu Glu Lys Lys Val Thr Trp Gln Lys Asp Ile Ile
625                 630                 635                 640
Pro Asp Ile Ala Ser Thr Ile Leu Gln Cys Arg Ser Gly Met Val Arg
```

```
                645                 650                 655
Arg Lys Gly Lys Val Thr Arg Asn Ser Ser Thr Glu Gln Ala Lys Glu
            660                 665                 670
Glu Thr Trp Leu Leu Phe Gln Gly Val Asp Val Glu Ala Lys Glu Lys
        675                 680                 685
Ile Ala Lys Glu Leu Ala Lys Leu Ile Phe Gly Ser Gln Asn Asn Phe
    690                 695                 700
Ile Ser Ile Ser Leu Ser Ser Phe Ser Ser Thr Arg Ala Asp Ser Thr
705                 710                 715                 720
Glu Asp Cys Arg Asn Lys Arg Ser Arg Asp Glu Gln Ser Cys Ser Tyr
                725                 730                 735
Ile Glu Arg Phe Ala Glu Ala Val Ser Ser Asn Pro His Arg Val Phe
            740                 745                 750
Leu Val Glu Asp Val Glu Gln Ala Asp Tyr Cys Ser Gln Val Gly Phe
        755                 760                 765
Lys Arg Ala Ile Glu Arg Gly Arg Ile Thr Asn Val Lys Gly Glu Glu
    770                 775                 780
Val Gly Leu Ser Asp Ala Ile Ile Ile Leu Ser Cys Glu Ser Phe Ser
785                 790                 795                 800
Ser Arg Ser Arg Ala Cys Ser Pro Pro Val Lys Gln Lys Thr Asp Asp
                805                 810                 815
Tyr Ile Ile Ser Gln Asp Gln Glu Glu Glu Lys Gly Gln Gly Ala Lys
            820                 825                 830
Met Glu Glu Ser Ser Pro Cys Val Ser Leu Asp Leu Asn Ile Ser Ile
        835                 840                 845
Asp Asp Asp Ser Ile Glu Asp Arg Ser Ile Asp Asp Ile Gly Leu Leu
    850                 855                 860
Glu Ser Val Asp Arg Arg Ile Val Phe Lys Ile Gln Glu Leu Arg Phe
865                 870                 875                 880
Met Ser


<210> 55
<211> 2487
<212> DNA
<213> Vitis vinifera

<220>
<221> CDS
<222> (1)..(2487)

<220>
<221> misc_feature
<222> (2364)..(2364)
<223> s is g or c

<400> 55
atg aga gcc gga ggt tgt aca gtg caa cag gct cta act gcg gag gcg        48
Met Arg Ala Gly Gly Cys Thr Val Gln Gln Ala Leu Thr Ala Glu Ala
1               5                   10                  15
gcc ggc gtg gtg aaa caa gca gta acc ctt gct agg cgg cgt gga cat        96
Ala Gly Val Val Lys Gln Ala Val Thr Leu Ala Arg Arg Arg Gly His
            20                  25                  30
gcc caa gtc act cct cta cat gta gcc aac acc atg ctc gcc gct aca        144
Ala Gln Val Thr Pro Leu His Val Ala Asn Thr Met Leu Ala Ala Thr
        35                  40                  45
aac ggc ctg ctc cga acc gct tgt ctt caa tcc cac tcc cac ccc ctc        192
Asn Gly Leu Leu Arg Thr Ala Cys Leu Gln Ser His Ser His Pro Leu
    50                  55                  60
cag tgc aaa gcc cta gaa ctt tgc ttc aac gtt gcc ctc aac cgc ctt        240
Gln Cys Lys Ala Leu Glu Leu Cys Phe Asn Val Ala Leu Asn Arg Leu
65                  70                  75                  80
cca gcc tcc aca tcc agt ccc atg ctg ggt cct cat tcc caa cat cct        288
Pro Ala Ser Thr Ser Ser Pro Met Leu Gly Pro His Ser Gln His Pro
                85                  90                  95
```

```
tcc atc tcc aac gca ttg gtt gct gcc ttc aag cga gct cag gcc cat        336
Ser Ile Ser Asn Ala Leu Val Ala Ala Phe Lys Arg Ala Gln Ala His
            100                 105                 110
cag cga cgc ggc tct att gag aac cag cag caa cct ctt cta gca gtt        384
Gln Arg Arg Gly Ser Ile Glu Asn Gln Gln Gln Pro Leu Leu Ala Val
        115                 120                 125
aaa ata gag cta gag cag ctc ata atc tct att tta gat gat cct agt        432
Lys Ile Glu Leu Glu Gln Leu Ile Ile Ser Ile Leu Asp Asp Pro Ser
    130                 135                 140
gtg agt aga gtc atg aga gaa gct ggg ttc tcc agt acc caa gtg aaa        480
Val Ser Arg Val Met Arg Glu Ala Gly Phe Ser Ser Thr Gln Val Lys
145                 150                 155                 160
agc aat gta gag caa gct gtc tcc ttg gaa atc tgc tct caa gct cca        528
Ser Asn Val Glu Gln Ala Val Ser Leu Glu Ile Cys Ser Gln Ala Pro
                165                 170                 175
tcc gtg agt agt aag tcc aag gaa agt aac ctt cta gtc ctg tcc cag        576
Ser Val Ser Ser Lys Ser Lys Glu Ser Asn Leu Leu Val Leu Ser Gln
            180                 185                 190
tct cct ccc atg ggt caa att gga gta aag cta ggc aaa cca acg gtg        624
Ser Pro Pro Met Gly Gln Ile Gly Val Lys Leu Gly Lys Pro Thr Val
        195                 200                 205
cca gat cca gtc agg aat gag gat gtg atg agt gtt aga ggg gtg atg        672
Pro Asp Pro Val Arg Asn Glu Asp Val Met Ser Val Arg Gly Val Met
    210                 215                 220
gac aaa gtt gac aag gga gat gtt cca gag gct ttg aga gat gtt aag        720
Asp Lys Val Asp Lys Gly Asp Val Pro Glu Ala Leu Arg Asp Val Lys
225                 230                 235                 240
ctt ata agc ctt cct cct tct tct gac ttt tgg aca tca ctc cag aga        768
Leu Ile Ser Leu Pro Pro Ser Ser Asp Phe Trp Thr Ser Leu Gln Arg
                245                 250                 255
aga ggt ccg aac aga aag ctc ggg gag ctc aag agc ctt gtg aag agt        816
Arg Gly Pro Asn Arg Lys Leu Gly Glu Leu Lys Ser Leu Val Lys Ser
            260                 265                 270
tgc gtg gga aga ggg gtt att ttg tat ttg gaa gat ctt aaa tgg act        864
Cys Val Gly Arg Gly Val Ile Leu Tyr Leu Glu Asp Leu Lys Trp Thr
        275                 280                 285
acg gat tat aga gct agt tct agt gag caa ggg agg aac tat tat tgt        912
Thr Asp Tyr Arg Ala Ser Ser Ser Glu Gln Gly Arg Asn Tyr Tyr Cys
    290                 295                 300
ccc gtg gag cac atg atc atg gag ctt ggg aaa ttg gtt tgt gga ttt        960
Pro Val Glu His Met Ile Met Glu Leu Gly Lys Leu Val Cys Gly Phe
305                 310                 315                 320
ggg gag aac gga agg ttt tgg ctc atg gga atc gca act ttc caa act        1008
Gly Glu Asn Gly Arg Phe Trp Leu Met Gly Ile Ala Thr Phe Gln Thr
                325                 330                 335
tac tcg aga tgt aga act ggc cat cca tcg ctg gag act att tgg agt        1056
Tyr Ser Arg Cys Arg Thr Gly His Pro Ser Leu Glu Thr Ile Trp Ser
            340                 345                 350
cta cat cct ctt aca att cct gca agc agc ttg gcc ttg agt ctc atg        1104
Leu His Pro Leu Thr Ile Pro Ala Ser Ser Leu Ala Leu Ser Leu Met
        355                 360                 365
cct gac agt gat cta caa agt cag ttc agt agc aaa aag gct gga agt        1152
Pro Asp Ser Asp Leu Gln Ser Gln Phe Ser Ser Lys Lys Ala Gly Ser
    370                 375                 380
ggg acc agt aat tgg ctt atg ctt gaa ggt gga gcg gag aag cag ctt        1200
Gly Thr Ser Asn Trp Leu Met Leu Glu Gly Gly Ala Glu Lys Gln Leu
385                 390                 395                 400
act tgc tgc gct gat tgc tca gcc aat ttc gag aat gaa gct cga agc        1248
Thr Cys Cys Ala Asp Cys Ser Ala Asn Phe Glu Asn Glu Ala Arg Ser
                405                 410                 415
ata cca acc agc act tgt aac agt gat tcc aca act tca acc ctt cca        1296
Ile Pro Thr Ser Thr Cys Asn Ser Asp Ser Thr Thr Ser Thr Leu Pro
            420                 425                 430
aca tgg ctc caa cag tac aaa gat gag aac aaa aaa ctt tcc cgt aat        1344
Thr Trp Leu Gln Gln Tyr Lys Asp Glu Asn Lys Lys Leu Ser Arg Asn
```

```
            435                 440                 445
gat cag gac tgt gta gca gtc aga gat ctc tgc aaa aaa tgg aat tct      1392
Asp Gln Asp Cys Val Ala Val Arg Asp Leu Cys Lys Lys Trp Asn Ser
    450                 455                 460
att tgc agt tct gcc cac aaa caa ccc cac tca tct gag aaa acc cta      1440
Ile Cys Ser Ser Ala His Lys Gln Pro His Ser Ser Glu Lys Thr Leu
465                 470                 475                 480
aca ttc tct tct ctt tca cct tct tcc tct act tct ggc ttt tca tat      1488
Thr Phe Ser Ser Leu Ser Pro Ser Ser Ser Thr Ser Gly Phe Ser Tyr
                485                 490                 495
gac cag caa tat cct aat ttg cac cag acc cac caa ggt tgg cca gtg      1536
Asp Gln Gln Tyr Pro Asn Leu His Gln Thr His Gln Gly Trp Pro Val
            500                 505                 510
gtt gaa cac aaa cag tcc tgg aga gac aat cat ttc tgg gtt tcc gaa      1584
Val Glu His Lys Gln Ser Trp Arg Asp Asn His Phe Trp Val Ser Glu
        515                 520                 525
gct ctt aac aaa acc tat gaa ccc agt ttg aga atg tac att ccg gag      1632
Ala Leu Asn Lys Thr Tyr Glu Pro Ser Leu Arg Met Tyr Ile Pro Glu
    530                 535                 540
cat tct gat cgc aaa tat gcg tcg aac cct aat tct acc cct aat tcg      1680
His Ser Asp Arg Lys Tyr Ala Ser Asn Pro Asn Ser Thr Pro Asn Ser
545                 550                 555                 560
gcc tct tca agc gat gtc atg gaa atg gag tac gtt caa agg ttc aag      1728
Ala Ser Ser Ser Asp Val Met Glu Met Glu Tyr Val Gln Arg Phe Lys
                565                 570                 575
gag ctc aac gct gag aac ctg aac act cta tgc aat gca ttg gag aaa      1776
Glu Leu Asn Ala Glu Asn Leu Asn Thr Leu Cys Asn Ala Leu Glu Lys
            580                 585                 590
aag gtc cca tgg cag aaa gat ata att cct gac atc gct agc acg atc      1824
Lys Val Pro Trp Gln Lys Asp Ile Ile Pro Asp Ile Ala Ser Thr Ile
        595                 600                 605
tta caa tgc agg tct gga atg gta aga cgc aaa gga aag gtc aaa aat      1872
Leu Gln Cys Arg Ser Gly Met Val Arg Arg Lys Gly Lys Val Lys Asn
    610                 615                 620
agc gag acc aaa gaa gaa aca tgg ttt ttc ttc caa ggc gtt gat atg      1920
Ser Glu Thr Lys Glu Glu Thr Trp Phe Phe Phe Gln Gly Val Asp Met
625                 630                 635                 640
gat gct aaa gag aag att gcg aga gaa ttg gct aga ctt gtt ttc ggc      1968
Asp Ala Lys Glu Lys Ile Ala Arg Glu Leu Ala Arg Leu Val Phe Gly
                645                 650                 655
tcc cag aac aac ttc gtt tca att gct ctg agc agc ttc tca tct aca      2016
Ser Gln Asn Asn Phe Val Ser Ile Ala Leu Ser Ser Phe Ser Ser Thr
            660                 665                 670
agg gcg gat tca aca gaa gat ctc cga aat aag aga tca aga gat gag      2064
Arg Ala Asp Ser Thr Glu Asp Leu Arg Asn Lys Arg Ser Arg Asp Glu
        675                 680                 685
caa agc tgc agt tac atc gag agg ttc gct gag gca gtg ggc agt aat      2112
Gln Ser Cys Ser Tyr Ile Glu Arg Phe Ala Glu Ala Val Gly Ser Asn
    690                 695                 700
cca cac cgg gta ttc tta gcg gaa gat gtg gag caa gcg gat tac tgc      2160
Pro His Arg Val Phe Leu Ala Glu Asp Val Glu Gln Ala Asp Tyr Cys
705                 710                 715                 720
tcc caa atg ggt atc aaa agg gcc aca gaa aga gga aga ata acc aat      2208
Ser Gln Met Gly Ile Lys Arg Ala Thr Glu Arg Gly Arg Ile Thr Asn
                725                 730                 735
tcg aat gga gaa gaa atc agc ctg agc gac gca atc atc att ttg agc      2256
Ser Asn Gly Glu Glu Ile Ser Leu Ser Asp Ala Ile Ile Ile Leu Ser
            740                 745                 750
tgt gaa agc ttc agc tcg agg tct aga gcc tgc tca cct cca atc aaa      2304
Cys Glu Ser Phe Ser Ser Arg Ser Arg Ala Cys Ser Pro Pro Ile Lys
        755                 760                 765
caa aaa tca gat gag ttc gaa gag gag aaa ggt gga ggt ggg ggt gag      2352
Gln Lys Ser Asp Glu Phe Glu Glu Glu Lys Gly Gly Gly Gly Gly Glu
    770                 775                 780
gag ata agc ccs tgt gtg tct ttg gat ttg aat att tgc att gat gat      2400
```

```
Glu Ile Ser Pro Cys Val Ser Leu Asp Leu Asn Ile Cys Ile Asp Asp
785                 790                 795                 800
gac ggc gtt gag gat gaa tca atc gac gac atc gga ctt ctt gaa tca       2448
Asp Gly Val Glu Asp Glu Ser Ile Asp Asp Ile Gly Leu Leu Glu Ser
                805                 810                 815
gtg gat aga agg att act ttc aaa att cag gag tta taa                   2487
Val Asp Arg Arg Ile Thr Phe Lys Ile Gln Glu Leu
            820                 825

<210> 56
<211> 828
<212> PRT
<213> Vitis vinifera

<400> 56
Met Arg Ala Gly Gly Cys Thr Val Gln Gln Ala Leu Thr Ala Glu Ala
1               5                   10                  15
Ala Gly Val Val Lys Gln Ala Val Thr Leu Ala Arg Arg Arg Gly His
            20                  25                  30
Ala Gln Val Thr Pro Leu His Val Ala Asn Thr Met Leu Ala Ala Thr
        35                  40                  45
Asn Gly Leu Leu Arg Thr Ala Cys Leu Gln Ser His Ser His Pro Leu
    50                  55                  60
Gln Cys Lys Ala Leu Glu Leu Cys Phe Asn Val Ala Leu Asn Arg Leu
65                  70                  75                  80
Pro Ala Ser Thr Ser Ser Pro Met Leu Gly Pro His Ser Gln His Pro
                85                  90                  95
Ser Ile Ser Asn Ala Leu Val Ala Ala Phe Lys Arg Ala Gln Ala His
            100                 105                 110
Gln Arg Arg Gly Ser Ile Glu Asn Gln Gln Gln Pro Leu Leu Ala Val
        115                 120                 125
Lys Ile Glu Leu Glu Gln Leu Ile Ile Ser Ile Leu Asp Asp Pro Ser
    130                 135                 140
Val Ser Arg Val Met Arg Glu Ala Gly Phe Ser Ser Thr Gln Val Lys
145                 150                 155                 160
Ser Asn Val Glu Gln Ala Val Ser Leu Glu Ile Cys Ser Gln Ala Pro
                165                 170                 175
Ser Val Ser Ser Lys Ser Lys Glu Ser Asn Leu Leu Val Leu Ser Gln
            180                 185                 190
Ser Pro Pro Met Gly Gln Ile Gly Val Lys Leu Gly Lys Pro Thr Val
        195                 200                 205
Pro Asp Pro Val Arg Asn Glu Asp Val Met Ser Val Arg Gly Val Met
    210                 215                 220
Asp Lys Val Asp Lys Gly Asp Val Pro Glu Ala Leu Arg Asp Val Lys
225                 230                 235                 240
Leu Ile Ser Leu Pro Pro Ser Ser Asp Phe Trp Thr Ser Leu Gln Arg
                245                 250                 255
Arg Gly Pro Asn Arg Lys Leu Gly Glu Leu Lys Ser Leu Val Lys Ser
            260                 265                 270
Cys Val Gly Arg Gly Val Ile Leu Tyr Leu Glu Asp Leu Lys Trp Thr
        275                 280                 285
Thr Asp Tyr Arg Ala Ser Ser Ser Glu Gln Gly Arg Asn Tyr Tyr Cys
    290                 295                 300
Pro Val Glu His Met Ile Met Glu Leu Gly Lys Leu Val Cys Gly Phe
305                 310                 315                 320
Gly Glu Asn Gly Arg Phe Trp Leu Met Gly Ile Ala Thr Phe Gln Thr
                325                 330                 335
Tyr Ser Arg Cys Arg Thr Gly His Pro Ser Leu Glu Thr Ile Trp Ser
            340                 345                 350
Leu His Pro Leu Thr Ile Pro Ala Ser Ser Leu Ala Leu Ser Leu Met
        355                 360                 365
Pro Asp Ser Asp Leu Gln Ser Gln Phe Ser Ser Lys Lys Ala Gly Ser
    370                 375                 380
Gly Thr Ser Asn Trp Leu Met Leu Glu Gly Gly Ala Glu Lys Gln Leu
385                 390                 395                 400
```

Thr Cys Cys Ala Asp Cys Ser Ala Asn Phe Glu Asn Glu Ala Arg Ser
405 410 415
Ile Pro Thr Ser Thr Cys Asn Ser Asp Ser Thr Thr Ser Thr Leu Pro
420 425 430
Thr Trp Leu Gln Gln Tyr Lys Asp Glu Asn Lys Lys Leu Ser Arg Asn
435 440 445
Asp Gln Asp Cys Val Ala Val Arg Asp Leu Cys Lys Lys Trp Asn Ser
450 455 460
Ile Cys Ser Ser Ala His Lys Gln Pro His Ser Ser Glu Lys Thr Leu
465 470 475 480
Thr Phe Ser Ser Leu Ser Pro Ser Ser Ser Thr Ser Gly Phe Ser Tyr
485 490 495
Asp Gln Gln Tyr Pro Asn Leu His Gln Thr His Gln Gly Trp Pro Val
500 505 510
Val Glu His Lys Gln Ser Trp Arg Asp Asn His Phe Trp Val Ser Glu
515 520 525
Ala Leu Asn Lys Thr Tyr Glu Pro Ser Leu Arg Met Tyr Ile Pro Glu
530 535 540
His Ser Asp Arg Lys Tyr Ala Ser Asn Pro Asn Ser Thr Pro Asn Ser
545 550 555 560
Ala Ser Ser Ser Asp Val Met Glu Met Glu Tyr Val Gln Arg Phe Lys
565 570 575
Glu Leu Asn Ala Glu Asn Leu Asn Thr Leu Cys Asn Ala Leu Glu Lys
580 585 590
Lys Val Pro Trp Gln Lys Asp Ile Ile Pro Asp Ile Ala Ser Thr Ile
595 600 605
Leu Gln Cys Arg Ser Gly Met Val Arg Arg Lys Gly Lys Val Lys Asn
610 615 620
Ser Glu Thr Lys Glu Glu Thr Trp Phe Phe Phe Gln Gly Val Asp Met
625 630 635 640
Asp Ala Lys Glu Lys Ile Ala Arg Glu Leu Ala Arg Leu Val Phe Gly
645 650 655
Ser Gln Asn Asn Phe Val Ser Ile Ala Leu Ser Ser Phe Ser Ser Thr
660 665 670
Arg Ala Asp Ser Thr Glu Asp Leu Arg Asn Lys Arg Ser Arg Asp Glu
675 680 685
Gln Ser Cys Ser Tyr Ile Glu Arg Phe Ala Glu Ala Val Gly Ser Asn
690 695 700
Pro His Arg Val Phe Leu Ala Glu Asp Val Glu Gln Ala Asp Tyr Cys
705 710 715 720
Ser Gln Met Gly Ile Lys Arg Ala Thr Glu Arg Gly Arg Ile Thr Asn
725 730 735
Ser Asn Gly Glu Glu Ile Ser Leu Ser Asp Ala Ile Ile Ile Leu Ser
740 745 750
Cys Glu Ser Phe Ser Ser Arg Ser Arg Ala Cys Ser Pro Pro Ile Lys
755 760 765
Gln Lys Ser Asp Glu Phe Glu Glu Glu Lys Gly Gly Gly Gly Gly Glu
770 775 780
Glu Ile Ser Pro Cys Val Ser Leu Asp Leu Asn Ile Cys Ile Asp Asp
785 790 795 800
Asp Gly Val Glu Asp Glu Ser Ile Asp Asp Ile Gly Leu Leu Glu Ser
805 810 815
Val Asp Arg Arg Ile Thr Phe Lys Ile Gln Glu Leu
820 825

<210> 57
<211> 2586
<212> DNA
<213> Vitis vinifera

<220>
<221> CDS
<222> (1)..(2586)

<220>

<221> misc_feature
<222> (57)..(57)
<223> k is g or t

<220>
<221> misc_feature
<222> (2066)..(2066)
<223> m is a or c

<220>
<221> misc_feature
<222> (2547)..(2547)
<223> w is a or t

<220>
<221> misc_feature
<222> (2560)..(2560)
<223> w is a or t

<400> 57
```
atg aga gca gga gtt tgc agt gta cag cag gtt cta act gct gat gca        48
Met Arg Ala Gly Val Cys Ser Val Gln Gln Val Leu Thr Ala Asp Ala
 1               5                  10                  15
gca agc atk gtc aaa caa gca gtt acc ctt gct agg cgg cga ggt cat        96
Ala Ser Xaa Val Lys Gln Ala Val Thr Leu Ala Arg Arg Arg Gly His
            20                  25                  30
gcc caa gtt act cct ctt cat gtg gcc agt gtc atg ctt gct tcc tcc       144
Ala Gln Val Thr Pro Leu His Val Ala Ser Val Met Leu Ala Ser Ser
        35                  40                  45
tct ggt ctt ctc cgc tct gct tgc cta cga tcc cac tct cat ccc ctg       192
Ser Gly Leu Leu Arg Ser Ala Cys Leu Arg Ser His Ser His Pro Leu
    50                  55                  60
cag tgc aag gct ctg gag ctc tgc ttc aat gtg gcg ctc aac cgc ctc       240
Gln Cys Lys Ala Leu Glu Leu Cys Phe Asn Val Ala Leu Asn Arg Leu
65                  70                  75                  80
cct gcc tcc tca tca agc ccc cta tta gct cct cat tcc tct cac cct       288
Pro Ala Ser Ser Ser Ser Pro Leu Leu Ala Pro His Ser Ser His Pro
                85                  90                  95
tcc ttg tcc aat gcc ttg gtt gca gcc ttc aag cgt gca cag gct cac       336
Ser Leu Ser Asn Ala Leu Val Ala Ala Phe Lys Arg Ala Gln Ala His
            100                 105                 110
caa cgc cgt gcc tcc atc gaa aac cag caa caa ccc att cta gct ctc       384
Gln Arg Arg Ala Ser Ile Glu Asn Gln Gln Gln Pro Ile Leu Ala Leu
        115                 120                 125
aaa gta gag ata gaa cag ctc ata atc tcc atc cta cat gac cca agt       432
Lys Val Glu Ile Glu Gln Leu Ile Ile Ser Ile Leu His Asp Pro Ser
    130                 135                 140
gtt agt aga gtc atg aga gag gct ggt ttc tct agt acc caa ttg aga       480
Val Ser Arg Val Met Arg Glu Ala Gly Phe Ser Ser Thr Gln Leu Arg
145                 150                 155                 160
acc aac ata gaa caa gct gtc tct ttg gat gtt tgt tct caa agc cct       528
Thr Asn Ile Glu Gln Ala Val Ser Leu Asp Val Cys Ser Gln Ser Pro
                165                 170                 175
gct gtg agt agc ctt tct aag gaa agt aac aac cct cct cta ata ctc       576
Ala Val Ser Ser Leu Ser Lys Glu Ser Asn Asn Pro Pro Leu Ile Leu
            180                 185                 190
ggt act aat gtg tcc caa tct tca aca ttt att cag ttt gga gta act       624
Gly Thr Asn Val Ser Gln Ser Ser Thr Phe Ile Gln Phe Gly Val Thr
        195                 200                 205
ctg aac aat cca ttt gac gaa gct cag gaa gaa gat gta aag agt ctt       672
Leu Asn Asn Pro Phe Asp Glu Ala Gln Glu Glu Asp Val Lys Ser Leu
    210                 215                 220
ttg gac gca ttt aca agc aaa aga agg aga aac act gtt gtt gta gga       720
Leu Asp Ala Phe Thr Ser Lys Arg Arg Arg Asn Thr Val Val Val Gly
225                 230                 235                 240
```

```
gag act ctg gcc agt gct gag ggt gta gtt aga ggg ctg atg aac aag        768
Glu Thr Leu Ala Ser Ala Glu Gly Val Val Arg Gly Leu Met Asn Lys
                245                 250                 255
ttt gag aga gga gat gtc cct gga gat ctg agg tat gtg cag ttt ata        816
Phe Glu Arg Gly Asp Val Pro Gly Asp Leu Arg Tyr Val Gln Phe Ile
            260                 265                 270
agc ctt cct ctc ttc tct tta aag aat ctc tcc aag gag gtt gaa cag        864
Ser Leu Pro Leu Phe Ser Leu Lys Asn Leu Ser Lys Glu Val Glu Gln
        275                 280                 285
aag ctt gtg aag ctg aat tgc ctt ctg aaa agc tat gtg tgt aga ggg        912
Lys Leu Val Lys Leu Asn Cys Leu Leu Lys Ser Tyr Val Cys Arg Gly
    290                 295                 300
gtg gtt ttg tat ttg ggt gat ctc aaa tgg gtc tct gag ttt gag tca        960
Val Val Leu Tyr Leu Gly Asp Leu Lys Trp Val Ser Glu Phe Glu Ser
305                 310                 315                 320
aat tat ggt gag agg aga aac tac tgc tct cct gtg gag cac ata atc        1008
Asn Tyr Gly Glu Arg Arg Asn Tyr Cys Ser Pro Val Glu His Ile Ile
                325                 330                 335
atg gag ctc gga aga atg atg tgt gga att ggg gat aga gga cga atg        1056
Met Glu Leu Gly Arg Met Met Cys Gly Ile Gly Asp Arg Gly Arg Met
            340                 345                 350
tgg ctt ttg ggg act gca acc ttc caa act tac atg aga tgc aaa gca        1104
Trp Leu Leu Gly Thr Ala Thr Phe Gln Thr Tyr Met Arg Cys Lys Ala
        355                 360                 365
ggc cat cct tct ttg gag act att tgg gaa ctt cat cct ctt act att        1152
Gly His Pro Ser Leu Glu Thr Ile Trp Glu Leu His Pro Leu Thr Ile
    370                 375                 380
cct gtt ggc agc ttg ggc ctc ggt ctc aat ctt gac agc aat ttg cag        1200
Pro Val Gly Ser Leu Gly Leu Gly Leu Asn Leu Asp Ser Asn Leu Gln
385                 390                 395                 400
ggc cgg ttc caa agt aag gca tct gga gat ggg act tct tgg tca ctt        1248
Gly Arg Phe Gln Ser Lys Ala Ser Gly Asp Gly Thr Ser Trp Ser Leu
                405                 410                 415
ctt caa agt gga gat aag cac ctt act tgc agc aca aat tgc tct gat        1296
Leu Gln Ser Gly Asp Lys His Leu Thr Cys Ser Thr Asn Cys Ser Asp
            420                 425                 430
aat ttt gac aaa gaa tct caa agt ata gca tgc agt ttt cgc aat ggc        1344
Asn Phe Asp Lys Glu Ser Gln Ser Ile Ala Cys Ser Phe Arg Asn Gly
        435                 440                 445
gag tcc acc acc acc atc acc act tcc acc agc tca agc ttg cct tca        1392
Glu Ser Thr Thr Thr Ile Thr Thr Ser Thr Ser Ser Ser Leu Pro Ser
    450                 455                 460
tgg ctc caa aaa gag aaa aga aga aag atc atg gac gat cag gaa tgt        1440
Trp Leu Gln Lys Glu Lys Arg Arg Lys Ile Met Asp Asp Gln Glu Cys
465                 470                 475                 480
gtc caa gtc aga gat ctc tgc aac aaa tgg aac tcg ttt tgc agc tca        1488
Val Gln Val Arg Asp Leu Cys Asn Lys Trp Asn Ser Phe Cys Ser Ser
                485                 490                 495
gtc cac aaa aag gcc cac agt act gag aaa gcc ctg aat ttt tct tca        1536
Val His Lys Lys Ala His Ser Thr Glu Lys Ala Leu Asn Phe Ser Ser
            500                 505                 510
cca tct cct tcc tcc act tcc atc tcc tcc tat gac caa tgc agc cct        1584
Pro Ser Pro Ser Ser Thr Ser Ile Ser Ser Tyr Asp Gln Cys Ser Pro
        515                 520                 525
aat ctg caa cag aac cac cta agt tgg cca gcc atc att gag ccg aaa        1632
Asn Leu Gln Gln Asn His Leu Ser Trp Pro Ala Ile Ile Glu Pro Lys
    530                 535                 540
cca cct ctg aaa gaa cac cag ttc tgg ata tct gaa aat gtt gat gaa        1680
Pro Pro Leu Lys Glu His Gln Phe Trp Ile Ser Glu Asn Val Asp Glu
545                 550                 555                 560
ggc ctt gaa ccc aag ttc agt atg cac ata gca gag aga aat ttt cca        1728
Gly Leu Glu Pro Lys Phe Ser Met His Ile Ala Glu Arg Asn Phe Pro
                565                 570                 575
ata cca gat ctt tta tca aat cct aac tct tcc cct aat tca gct tct        1776
Ile Pro Asp Leu Leu Ser Asn Pro Asn Ser Ser Pro Asn Ser Ala Ser
```

```
            580                 585                 590
tct agt gag gca ata gag gat gga gag ggt ctt tat ggt ttc aaa gag      1824
Ser Ser Glu Ala Ile Glu Asp Gly Glu Gly Leu Tyr Gly Phe Lys Glu
        595                 600                 605
ctt aat gcg gag aat ctg aga atc tta tgc aat gca ttg gag aga agg      1872
Leu Asn Ala Glu Asn Leu Arg Ile Leu Cys Asn Ala Leu Glu Arg Arg
    610                 615                 620
gtt cca tgg cag aag gat atc att cct gaa ata gca agc acc att ctt      1920
Val Pro Trp Gln Lys Asp Ile Ile Pro Glu Ile Ala Ser Thr Ile Leu
625                 630                 635                 640
gaa tgc agg tca gga aca ctg aga gga aaa aac aag ttg aag cag agg      1968
Glu Cys Arg Ser Gly Thr Leu Arg Gly Lys Asn Lys Leu Lys Gln Arg
                645                 650                 655
gag gac aag gaa gaa act tgg ctg ctc ttc tta gga gtt gac ttt caa      2016
Glu Asp Lys Glu Glu Thr Trp Leu Leu Phe Leu Gly Val Asp Phe Gln
            660                 665                 670
ggc aaa gag aag att gca agg gag ata gct aag ctt gtt ttt ggc tct      2064
Gly Lys Glu Lys Ile Ala Arg Glu Ile Ala Lys Leu Val Phe Gly Ser
        675                 680                 685
cmg agc aag ttc atc tca att ggt ctg agc agt tta gga tca aca aga      2112
Xaa Ser Lys Phe Ile Ser Ile Gly Leu Ser Ser Leu Gly Ser Thr Arg
    690                 695                 700
gct gat tcc acc gag gat ttc cta agc aaa caa gca aga gac gag cct      2160
Ala Asp Ser Thr Glu Asp Phe Leu Ser Lys Gln Ala Arg Asp Glu Pro
705                 710                 715                 720
gtt ggt agt tat att gag aaa ttc gct gaa gcg gtg cat gag aat cct      2208
Val Gly Ser Tyr Ile Glu Lys Phe Ala Glu Ala Val His Glu Asn Pro
                725                 730                 735
cac aga gtg ttc ttc ata gaa gat gtg gag caa cta gat tac tct tct      2256
His Arg Val Phe Phe Ile Glu Asp Val Glu Gln Leu Asp Tyr Ser Ser
            740                 745                 750
caa atg ggg gtt aag aga gga att gag agt gga aga ata caa att gct      2304
Gln Met Gly Val Lys Arg Gly Ile Glu Ser Gly Arg Ile Gln Ile Ala
        755                 760                 765
gga ggt gaa gca ttt tct ctt gag gat gcc atc atc ata ttc agt tgt      2352
Gly Gly Glu Ala Phe Ser Leu Glu Asp Ala Ile Ile Ile Phe Ser Cys
    770                 775                 780
gaa agc ttc agc tca gta tca aga gcc agc tct cct cca ccc atg ggg      2400
Glu Ser Phe Ser Ser Val Ser Arg Ala Ser Ser Pro Pro Pro Met Gly
785                 790                 795                 800
cta aaa tct gaa gag aat gag gaa aaa gac cgg gat aat gag ttg gag      2448
Leu Lys Ser Glu Glu Asn Glu Glu Lys Asp Arg Asp Asn Glu Leu Glu
                805                 810                 815
aag aga agc cca tgt gtg tct cta gat ctg aat ctt tca gct gaa gat      2496
Lys Arg Ser Pro Cys Val Ser Leu Asp Leu Asn Leu Ser Ala Glu Asp
            820                 825                 830
aat caa gaa tat gga caa aac tcg gtt gca gac act ggg gtt ctg gat      2544
Asn Gln Glu Tyr Gly Gln Asn Ser Val Ala Asp Thr Gly Val Leu Asp
        835                 840                 845
tcw gtg gac agg cag wtt att ttc aaa att caa gag ttg tga              2586
Ser Val Asp Arg Gln Xaa Ile Phe Lys Ile Gln Glu Leu
    850                 855                 860
```

<210> 58
<211> 861
<212> PRT
<213> Vitis vinifera

<220>
<221> misc_feature
<222> (19)..(19)
<223> The Xaa at location 19 stands for Ile, or Met.

<220>
<221> misc_feature

<222> (689)..(689)
<223> The Xaa at location 689 stands for Gln, or Pro.

<220>
<221> misc_feature
<222> (854)..(854)
<223> The Xaa at location 854 stands for Ile, or Phe.

<400> 58

```
Met Arg Ala Gly Val Cys Ser Val Gln Gln Val Leu Thr Ala Asp Ala
1               5                   10                  15
Ala Ser Xaa Val Lys Gln Ala Val Thr Leu Ala Arg Arg Arg Gly His
            20                  25                  30
Ala Gln Val Thr Pro Leu His Val Ala Ser Val Met Leu Ala Ser Ser
        35                  40                  45
Ser Gly Leu Leu Arg Ser Ala Cys Leu Arg Ser His Ser His Pro Leu
    50                  55                  60
Gln Cys Lys Ala Leu Glu Leu Cys Phe Asn Val Ala Leu Asn Arg Leu
65                  70                  75                  80
Pro Ala Ser Ser Ser Ser Pro Leu Leu Ala Pro His Ser Ser His Pro
                85                  90                  95
Ser Leu Ser Asn Ala Leu Val Ala Ala Phe Lys Arg Ala Gln Ala His
            100                 105                 110
Gln Arg Arg Ala Ser Ile Glu Asn Gln Gln Gln Pro Ile Leu Ala Leu
        115                 120                 125
Lys Val Glu Ile Glu Gln Leu Ile Ile Ser Ile Leu His Asp Pro Ser
    130                 135                 140
Val Ser Arg Val Met Arg Glu Ala Gly Phe Ser Ser Thr Gln Leu Arg
145                 150                 155                 160
Thr Asn Ile Glu Gln Ala Val Ser Leu Asp Val Cys Ser Gln Ser Pro
                165                 170                 175
Ala Val Ser Ser Leu Ser Lys Glu Ser Asn Asn Pro Pro Leu Ile Leu
            180                 185                 190
Gly Thr Asn Val Ser Gln Ser Ser Thr Phe Ile Gln Phe Gly Val Thr
        195                 200                 205
Leu Asn Asn Pro Phe Asp Glu Ala Gln Glu Glu Asp Val Lys Ser Leu
    210                 215                 220
Leu Asp Ala Phe Thr Ser Lys Arg Arg Arg Asn Thr Val Val Val Gly
225                 230                 235                 240
Glu Thr Leu Ala Ser Ala Glu Gly Val Val Arg Gly Leu Met Asn Lys
                245                 250                 255
Phe Glu Arg Gly Asp Val Pro Gly Asp Leu Arg Tyr Val Gln Phe Ile
            260                 265                 270
Ser Leu Pro Leu Phe Ser Leu Lys Asn Leu Ser Lys Glu Val Glu Gln
        275                 280                 285
Lys Leu Val Lys Leu Asn Cys Leu Leu Lys Ser Tyr Val Cys Arg Gly
    290                 295                 300
Val Val Leu Tyr Leu Gly Asp Leu Lys Trp Val Ser Glu Phe Glu Ser
305                 310                 315                 320
Asn Tyr Gly Glu Arg Arg Asn Tyr Cys Ser Pro Val Glu His Ile Ile
                325                 330                 335
Met Glu Leu Gly Arg Met Met Cys Gly Ile Gly Asp Arg Gly Arg Met
            340                 345                 350
Trp Leu Leu Gly Thr Ala Thr Phe Gln Thr Tyr Met Arg Cys Lys Ala
        355                 360                 365
Gly His Pro Ser Leu Glu Thr Ile Trp Glu Leu His Pro Leu Thr Ile
    370                 375                 380
Pro Val Gly Ser Leu Gly Leu Gly Leu Asn Leu Asp Ser Asn Leu Gln
385                 390                 395                 400
Gly Arg Phe Gln Ser Lys Ala Ser Gly Asp Gly Thr Ser Trp Ser Leu
                405                 410                 415
Leu Gln Ser Gly Asp Lys His Leu Thr Cys Ser Thr Asn Cys Ser Asp
            420                 425                 430
Asn Phe Asp Lys Glu Ser Gln Ser Ile Ala Cys Ser Phe Arg Asn Gly
        435                 440                 445
```

```
Glu Ser Thr Thr Thr Ile Thr Thr Ser Thr Ser Ser Ser Leu Pro Ser
    450                 455                 460
Trp Leu Gln Lys Glu Lys Arg Arg Lys Ile Met Asp Asp Gln Glu Cys
465                 470                 475                 480
Val Gln Val Arg Asp Leu Cys Asn Lys Trp Asn Ser Phe Cys Ser Ser
                485                 490                 495
Val His Lys Lys Ala His Ser Thr Glu Lys Ala Leu Asn Phe Ser Ser
            500                 505                 510
Pro Ser Pro Ser Ser Thr Ser Ile Ser Ser Tyr Asp Gln Cys Ser Pro
        515                 520                 525
Asn Leu Gln Gln Asn His Leu Ser Trp Pro Ala Ile Ile Glu Pro Lys
    530                 535                 540
Pro Pro Leu Lys Glu His Gln Phe Trp Ile Ser Glu Asn Val Asp Glu
545                 550                 555                 560
Gly Leu Glu Pro Lys Phe Ser Met His Ile Ala Glu Arg Asn Phe Pro
                565                 570                 575
Ile Pro Asp Leu Leu Ser Asn Pro Asn Ser Ser Pro Asn Ser Ala Ser
            580                 585                 590
Ser Ser Glu Ala Ile Glu Asp Gly Glu Gly Leu Tyr Gly Phe Lys Glu
        595                 600                 605
Leu Asn Ala Glu Asn Leu Arg Ile Leu Cys Asn Ala Leu Glu Arg Arg
    610                 615                 620
Val Pro Trp Gln Lys Asp Ile Ile Pro Glu Ile Ala Ser Thr Ile Leu
625                 630                 635                 640
Glu Cys Arg Ser Gly Thr Leu Arg Gly Lys Asn Lys Leu Lys Gln Arg
                645                 650                 655
Glu Asp Lys Glu Glu Thr Trp Leu Leu Phe Leu Gly Val Asp Phe Gln
            660                 665                 670
Gly Lys Glu Lys Ile Ala Arg Glu Ile Ala Lys Leu Val Phe Gly Ser
        675                 680                 685
Xaa Ser Lys Phe Ile Ser Ile Gly Leu Ser Ser Leu Gly Ser Thr Arg
    690                 695                 700
Ala Asp Ser Thr Glu Asp Phe Leu Ser Lys Gln Ala Arg Asp Glu Pro
705                 710                 715                 720
Val Gly Ser Tyr Ile Glu Lys Phe Ala Glu Ala Val His Glu Asn Pro
                725                 730                 735
His Arg Val Phe Phe Ile Glu Asp Val Glu Gln Leu Asp Tyr Ser Ser
            740                 745                 750
Gln Met Gly Val Lys Arg Gly Ile Glu Ser Gly Arg Ile Gln Ile Ala
        755                 760                 765
Gly Gly Glu Ala Phe Ser Leu Glu Asp Ala Ile Ile Ile Phe Ser Cys
    770                 775                 780
Glu Ser Phe Ser Ser Val Ser Arg Ala Ser Ser Pro Pro Pro Met Gly
785                 790                 795                 800
Leu Lys Ser Glu Glu Asn Glu Glu Lys Asp Arg Asp Asn Glu Leu Glu
                805                 810                 815
Lys Arg Ser Pro Cys Val Ser Leu Asp Leu Asn Leu Ser Ala Glu Asp
            820                 825                 830
Asn Gln Glu Tyr Gly Gln Asn Ser Val Ala Asp Thr Gly Val Leu Asp
        835                 840                 845
Ser Val Asp Arg Gln Xaa Ile Phe Lys Ile Gln Glu Leu
    850                 855                 860
```

<210> 59
<211> 2538
<212> DNA
<213> Oryza sativa subsp. indica

<220>
<221> CDS
<222> (1)..(2538)

<400> 59

```
atg cgt gcc ggc ggc tgc gca gtg cag cag gcg ctg gcg gcg gag gcg        48
Met Arg Ala Gly Gly Cys Ala Val Gln Gln Ala Leu Ala Ala Glu Ala
```

```
1               5                   10                  15
gcg ggg gtg gtg agg cag gcg gtg acg ctg gcg cgg cgg cgc ggc cac        96
Ala Gly Val Val Arg Gln Ala Val Thr Leu Ala Arg Arg Arg Gly His
            20                  25                  30
gcg cag gtc acg ccg ctc cat gtc gcc agc gcc atg ctc tcc gcc gcc        144
Ala Gln Val Thr Pro Leu His Val Ala Ser Ala Met Leu Ser Ala Ala
        35                  40                  45
ggc ctc ctc cgt gcc gcc tgc ctc cag tcc cac tcc cac ccg ctc cag        192
Gly Leu Leu Arg Ala Ala Cys Leu Gln Ser His Ser His Pro Leu Gln
    50                  55                  60
tgc aag gcc ctc gag ctc tgc ttc aac gtc gcc ctc aac cgc ctc ccc        240
Cys Lys Ala Leu Glu Leu Cys Phe Asn Val Ala Leu Asn Arg Leu Pro
65                  70                  75                  80
acc gcc ggc ccc gcc gcc gcc gcc gcc atc ttc cac cac cac cca cac        288
Thr Ala Gly Pro Ala Ala Ala Ala Ala Ile Phe His His His Pro His
                85                  90                  95
cac ccc ggc ggc ggc ggc ggt cac cac ccc gcg ctg tcg aac gcg ctc        336
His Pro Gly Gly Gly Gly Gly His His Pro Ala Leu Ser Asn Ala Leu
            100                 105                 110
gtc gcc gcg ttc aag cgc gcc cag gcg cac cag cgg cgt ggg tcc gtc        384
Val Ala Ala Phe Lys Arg Ala Gln Ala His Gln Arg Arg Gly Ser Val
        115                 120                 125
gag gga cag ccg ccg ccg cag ccg ccg ccg tcg ccg gtg gtc gcg tcc        432
Glu Gly Gln Pro Pro Pro Gln Pro Pro Pro Ser Pro Val Val Ala Ser
    130                 135                 140
aag gtc gag ctc gag cag ctc atc atc tcc atc ctc gat gac ccc agc        480
Lys Val Glu Leu Glu Gln Leu Ile Ile Ser Ile Leu Asp Asp Pro Ser
145                 150                 155                 160
gtc agc cgc gtc atg cgc gag gcc ggc ttc tcc agc tcg cag gtc aag        528
Val Ser Arg Val Met Arg Glu Ala Gly Phe Ser Ser Ser Gln Val Lys
                165                 170                 175
gcc aac gtc gag aag gcc gtc gtc gcg tcg ttg gac cac gcg aac gcg        576
Ala Asn Val Glu Lys Ala Val Val Ala Ser Leu Asp His Ala Asn Ala
            180                 185                 190
gca ggc ggc ggc ggc ggc cac gcg ggc tct ccc aac tcc ggc cat gga        624
Ala Gly Gly Gly Gly Gly His Ala Gly Ser Pro Asn Ser Gly His Gly
        195                 200                 205
ggg agg cgc aag gag agc tca tca tcc aga gca agg gtc gac gac gac        672
Gly Arg Arg Lys Glu Ser Ser Ser Ser Arg Ala Arg Val Asp Asp Asp
    210                 215                 220
gcc atg cgc gtg ctg gac tgc atg gcg agc ggg acg aaa cgg tgc gtg        720
Ala Met Arg Val Leu Asp Cys Met Ala Ser Gly Thr Lys Arg Cys Val
225                 230                 235                 240
gtg gtc gtc ggc ggc gag ggc gcg gcc gcg gcg gag gcg gtg gtg aag        768
Val Val Val Gly Gly Glu Gly Ala Ala Ala Ala Glu Ala Val Val Lys
                245                 250                 255
gcg gtg atg gac agg gtg agc aag gcg gag ctg cac cac cgg cac gag        816
Ala Val Met Asp Arg Val Ser Lys Ala Glu Leu His His Arg His Glu
            260                 265                 270
cgc ctc aag aac ctc cag ttc gtg ccc ctc tcc atc gcg tcg ttc cac        864
Arg Leu Lys Asn Leu Gln Phe Val Pro Leu Ser Ile Ala Ser Phe His
        275                 280                 285
ggc gcg ccg agg gag gag gtg gag gcc aag gcc agc gac ctc cgc gcg        912
Gly Ala Pro Arg Glu Glu Val Glu Ala Lys Ala Ser Asp Leu Arg Ala
    290                 295                 300
ctc gtc cgc tcc ggc tgc gcc gcc ggc aag ggc gtc gtg ctc gtc ctc        960
Leu Val Arg Ser Gly Cys Ala Ala Gly Lys Gly Val Val Leu Val Leu
305                 310                 315                 320
gag gac ctc gcc tac gcc gcc gac gcg tgg gcc gcc gcg tcg aac acc        1008
Glu Asp Leu Ala Tyr Ala Ala Asp Ala Trp Ala Ala Ala Ser Asn Thr
                325                 330                 335
aga cgc cgc gcc gcc gcc gcc acc ggc ggg cag agc tac tgc ccc atg        1056
Arg Arg Arg Ala Ala Ala Ala Thr Gly Gly Gln Ser Tyr Cys Pro Met
            340                 345                 350
gaa cac gcc gtg atg gag gtg agc agc ttg gtg tcc ggc ggc ggc ggc        1104
```

```
Glu His Ala Val Met Glu Val Ser Ser Leu Val Ser Gly Gly Gly Gly
        355                 360                 365
ggc ggc gag agg ttc tgg gtg cta ggg ttc ggg agc tac cag gtg tac      1152
Gly Gly Glu Arg Phe Trp Val Leu Gly Phe Gly Ser Tyr Gln Val Tyr
    370                 375                 380
atg aag tgc agg gcc gcc ggg cag ccg ccg ctc gag gcc gtc tgg gag      1200
Met Lys Cys Arg Ala Ala Gly Gln Pro Pro Leu Glu Ala Val Trp Glu
385                 390                 395                 400
ctc cac ccc gtc gtc gtc ccc gac ggc ggc ctc gcc ttg agc ctc acc      1248
Leu His Pro Val Val Val Pro Asp Gly Gly Leu Ala Leu Ser Leu Thr
                405                 410                 415
tgc agt gaa gct tca caa gct act cat caa gcg gcg gcg cca aca gct      1296
Cys Ser Glu Ala Ser Gln Ala Thr His Gln Ala Ala Ala Pro Thr Ala
            420                 425                 430
ggg tgg ccc ttc gtc aat ggc gcc ggc gag gcg gcg gcg acg acg gcg      1344
Gly Trp Pro Phe Val Asn Gly Ala Gly Glu Ala Ala Ala Thr Thr Ala
        435                 440                 445
agc ccg acc att ccg ccg tgg ctt cgc cgg tat caa gat cca gat cac      1392
Ser Pro Thr Ile Pro Pro Trp Leu Arg Arg Tyr Gln Asp Pro Asp His
    450                 455                 460
gcc acg ccg gcg agc tgc ggc acc ggt ttg cag ata caa gat ctg tgg      1440
Ala Thr Pro Ala Ser Cys Gly Thr Gly Leu Gln Ile Gln Asp Leu Trp
465                 470                 475                 480
aat ccc atg aga aat gga tca gca cca cac cac aca tcc gag ctt aca      1488
Asn Pro Met Arg Asn Gly Ser Ala Pro His His Thr Ser Glu Leu Thr
                485                 490                 495
ttg agt ttc tcc tct cca tca cct tct tcc atc tca gga tat acc agc      1536
Leu Ser Phe Ser Ser Pro Ser Pro Ser Ser Ile Ser Gly Tyr Thr Ser
            500                 505                 510
tgc tac aac aac aac aac atg atg agc tcc aag cca tgg caa ctc gaa      1584
Cys Tyr Asn Asn Asn Asn Met Met Ser Ser Lys Pro Trp Gln Leu Glu
        515                 520                 525
gcg agg cag cca tgg cca att cat gga cat gaa ggt cag aga atg gcc      1632
Ala Arg Gln Pro Trp Pro Ile His Gly His Glu Gly Gln Arg Met Ala
    530                 535                 540
atg gca tca tat cat gat cat cat ccg ttg gat aca aac cct agc cca      1680
Met Ala Ser Tyr His Asp His His Pro Leu Asp Thr Asn Pro Ser Pro
545                 550                 555                 560
gag tcc aac tcg gtg tcc aat tca ttg gat ggc ggt gag acg agg cgg      1728
Glu Ser Asn Ser Val Ser Asn Ser Leu Asp Gly Gly Glu Thr Arg Arg
                565                 570                 575
ccc aag ttc atc gag ctc aat gca gag aac ctc aag atc ttg tgc aat      1776
Pro Lys Phe Ile Glu Leu Asn Ala Glu Asn Leu Lys Ile Leu Cys Asn
            580                 585                 590
gcg ctc gag agc cgc gtc ccg caa cat agc aac att gtt cca gac att      1824
Ala Leu Glu Ser Arg Val Pro Gln His Ser Asn Ile Val Pro Asp Ile
        595                 600                 605
gcg agc acc gtg ctc caa tgt cga tcc ggc atg aag aag atg aag ttg      1872
Ala Ser Thr Val Leu Gln Cys Arg Ser Gly Met Lys Lys Met Lys Leu
    610                 615                 620
agg cat aag gag atc atc aag gca agc tca act act tgg tta cta ttc      1920
Arg His Lys Glu Ile Ile Lys Ala Ser Ser Thr Thr Trp Leu Leu Phe
625                 630                 635                 640
caa gga aga gat gtt gat ggt aag aag gcc atg gca caa gag ctc gca      1968
Gln Gly Arg Asp Val Asp Gly Lys Lys Ala Met Ala Gln Glu Leu Ala
                645                 650                 655
aag ctt gtc ttt ggt tca tct acc gag ttc tct tcc atc tcc ttc gat      2016
Lys Leu Val Phe Gly Ser Ser Thr Glu Phe Ser Ser Ile Ser Phe Asp
            660                 665                 670
gag ctc aca tcg ccc tac tcc gat tct agc tcc ggt gag ctc acc ctc      2064
Glu Leu Thr Ser Pro Tyr Ser Asp Ser Ser Ser Gly Glu Leu Thr Leu
        675                 680                 685
aag agg caa aga tca gca gac agc aat gag cat agc ttt gcg cag aga      2112
Lys Arg Gln Arg Ser Ala Asp Ser Asn Glu His Ser Phe Ala Gln Arg
    690                 695                 700
```

```
cta tgt gaa att gtg agc aaa aac cca cac cag gtc ata gtg atc aat       2160
Leu Cys Glu Ile Val Ser Lys Asn Pro His Gln Val Ile Val Ile Asn
705                 710                 715                 720
gac att gag caa ctt gat caa gat tca gaa att agt atc aag aaa gcg       2208
Asp Ile Glu Gln Leu Asp Gln Asp Ser Glu Ile Ser Ile Lys Lys Ala
                725                 730                 735
att gcg aac gga aga atg aga gga tgc acc ggt gaa gaa gtt gat ttc       2256
Ile Ala Asn Gly Arg Met Arg Gly Cys Thr Gly Glu Glu Val Asp Phe
            740                 745                 750
gag gat gct atc ata gtg ttg agc tat gaa gaa gaa ttc gat tcg agg       2304
Glu Asp Ala Ile Ile Val Leu Ser Tyr Glu Glu Glu Phe Asp Ser Arg
        755                 760                 765
tct agg gct tcc tcc tcc cct cgg gtc aag cag agg ctc atg aac aat       2352
Ser Arg Ala Ser Ser Ser Pro Arg Val Lys Gln Arg Leu Met Asn Asn
    770                 775                 780
aat gat gat gaa gaa agt agc agc acg gag aag gga gat aat tca cca       2400
Asn Asp Asp Glu Glu Ser Ser Ser Thr Glu Lys Gly Asp Asn Ser Pro
785                 790                 795                 800
caa cgt ttt agc ttg gat ttg aat gca tgt ctc gag gat gaa gaa gag       2448
Gln Arg Phe Ser Leu Asp Leu Asn Ala Cys Leu Glu Asp Glu Glu Glu
                805                 810                 815
gat gag ggg ttt ttg cta att gat aat ggt gtg ggg atg cat gat att       2496
Asp Glu Gly Phe Leu Leu Ile Asp Asn Gly Val Gly Met His Asp Ile
            820                 825                 830
gtg gat ggt gtt ttc ttc ttc ggg ttg atg gca gat ttc tga               2538
Val Asp Gly Val Phe Phe Phe Gly Leu Met Ala Asp Phe
        835                 840                 845
```

<210> 60
<211> 845
<212> PRT
<213> Oryza sativa subsp. indica

<400> 60

```
Met Arg Ala Gly Gly Cys Ala Val Gln Gln Ala Leu Ala Ala Glu Ala
1               5                   10                  15
Ala Gly Val Val Arg Gln Ala Val Thr Leu Ala Arg Arg Arg Gly His
            20                  25                  30
Ala Gln Val Thr Pro Leu His Val Ala Ser Ala Met Leu Ser Ala Ala
        35                  40                  45
Gly Leu Leu Arg Ala Ala Cys Leu Gln Ser His Ser His Pro Leu Gln
    50                  55                  60
Cys Lys Ala Leu Glu Leu Cys Phe Asn Val Ala Leu Asn Arg Leu Pro
65                  70                  75                  80
Thr Ala Gly Pro Ala Ala Ala Ala Ala Ile Phe His His His Pro His
                85                  90                  95
His Pro Gly Gly Gly Gly Gly His His Pro Ala Leu Ser Asn Ala Leu
            100                 105                 110
Val Ala Ala Phe Lys Arg Ala Gln Ala His Gln Arg Arg Gly Ser Val
        115                 120                 125
Glu Gly Gln Pro Pro Pro Gln Pro Pro Pro Ser Pro Val Val Ala Ser
    130                 135                 140
Lys Val Glu Leu Glu Gln Leu Ile Ile Ser Ile Leu Asp Asp Pro Ser
145                 150                 155                 160
Val Ser Arg Val Met Arg Glu Ala Gly Phe Ser Ser Ser Gln Val Lys
                165                 170                 175
Ala Asn Val Glu Lys Ala Val Val Ala Ser Leu Asp His Ala Asn Ala
            180                 185                 190
Ala Gly Gly Gly Gly Gly His Ala Gly Ser Pro Asn Ser Gly His Gly
        195                 200                 205
Gly Arg Arg Lys Glu Ser Ser Ser Ser Arg Ala Arg Val Asp Asp Asp
    210                 215                 220
Ala Met Arg Val Leu Asp Cys Met Ala Ser Gly Thr Lys Arg Cys Val
225                 230                 235                 240
Val Val Val Gly Gly Glu Gly Ala Ala Ala Ala Glu Ala Val Val Lys
```

```
                245                 250                 255
Ala Val Met Asp Arg Val Ser Lys Ala Glu Leu His His Arg His Glu
            260                 265                 270
Arg Leu Lys Asn Leu Gln Phe Val Pro Leu Ser Ile Ala Ser Phe His
        275                 280                 285
Gly Ala Pro Arg Glu Glu Val Glu Ala Lys Ala Ser Asp Leu Arg Ala
    290                 295                 300
Leu Val Arg Ser Gly Cys Ala Ala Gly Lys Gly Val Val Leu Val Leu
305                 310                 315                 320
Glu Asp Leu Ala Tyr Ala Ala Asp Ala Trp Ala Ala Ala Ser Asn Thr
                325                 330                 335
Arg Arg Arg Ala Ala Ala Ala Thr Gly Gly Gln Ser Tyr Cys Pro Met
            340                 345                 350
Glu His Ala Val Met Glu Val Ser Ser Leu Val Ser Gly Gly Gly Gly
        355                 360                 365
Gly Gly Glu Arg Phe Trp Val Leu Gly Phe Gly Ser Tyr Gln Val Tyr
    370                 375                 380
Met Lys Cys Arg Ala Ala Gly Gln Pro Pro Leu Glu Ala Val Trp Glu
385                 390                 395                 400
Leu His Pro Val Val Val Pro Asp Gly Gly Leu Ala Leu Ser Leu Thr
                405                 410                 415
Cys Ser Glu Ala Ser Gln Ala Thr His Gln Ala Ala Ala Pro Thr Ala
            420                 425                 430
Gly Trp Pro Phe Val Asn Gly Ala Gly Glu Ala Ala Ala Thr Thr Ala
        435                 440                 445
Ser Pro Thr Ile Pro Pro Trp Leu Arg Arg Tyr Gln Asp Pro Asp His
    450                 455                 460
Ala Thr Pro Ala Ser Cys Gly Thr Gly Leu Gln Ile Gln Asp Leu Trp
465                 470                 475                 480
Asn Pro Met Arg Asn Gly Ser Ala Pro His His Thr Ser Glu Leu Thr
                485                 490                 495
Leu Ser Phe Ser Ser Pro Ser Pro Ser Ser Ile Ser Gly Tyr Thr Ser
            500                 505                 510
Cys Tyr Asn Asn Asn Asn Met Met Ser Ser Lys Pro Trp Gln Leu Glu
        515                 520                 525
Ala Arg Gln Pro Trp Pro Ile His Gly His Glu Gly Gln Arg Met Ala
    530                 535                 540
Met Ala Ser Tyr His Asp His His Pro Leu Asp Thr Asn Pro Ser Pro
545                 550                 555                 560
Glu Ser Asn Ser Val Ser Asn Ser Leu Asp Gly Gly Glu Thr Arg Arg
                565                 570                 575
Pro Lys Phe Ile Glu Leu Asn Ala Glu Asn Leu Lys Ile Leu Cys Asn
            580                 585                 590
Ala Leu Glu Ser Arg Val Pro Gln His Ser Asn Ile Val Pro Asp Ile
        595                 600                 605
Ala Ser Thr Val Leu Gln Cys Arg Ser Gly Met Lys Lys Met Lys Leu
    610                 615                 620
Arg His Lys Glu Ile Ile Lys Ala Ser Ser Thr Thr Trp Leu Leu Phe
625                 630                 635                 640
Gln Gly Arg Asp Val Asp Gly Lys Lys Ala Met Ala Gln Glu Leu Ala
                645                 650                 655
Lys Leu Val Phe Gly Ser Ser Thr Glu Phe Ser Ser Ile Ser Phe Asp
            660                 665                 670
Glu Leu Thr Ser Pro Tyr Ser Asp Ser Ser Ser Gly Glu Leu Thr Leu
        675                 680                 685
Lys Arg Gln Arg Ser Ala Asp Ser Asn Glu His Ser Phe Ala Gln Arg
    690                 695                 700
Leu Cys Glu Ile Val Ser Lys Asn Pro His Gln Val Ile Val Ile Asn
705                 710                 715                 720
Asp Ile Glu Gln Leu Asp Gln Asp Ser Glu Ile Ser Ile Lys Lys Ala
                725                 730                 735
Ile Ala Asn Gly Arg Met Arg Gly Cys Thr Gly Glu Glu Val Asp Phe
            740                 745                 750
Glu Asp Ala Ile Ile Val Leu Ser Tyr Glu Glu Glu Phe Asp Ser Arg
        755                 760                 765
```

```
Ser Arg Ala Ser Ser Ser Pro Arg Val Lys Gln Arg Leu Met Asn Asn
    770                 775                 780
Asn Asp Asp Glu Glu Ser Ser Ser Thr Glu Lys Gly Asp Asn Ser Pro
785                 790                 795                 800
Gln Arg Phe Ser Leu Asp Leu Asn Ala Cys Leu Glu Asp Glu Glu Glu
                805                 810                 815
Asp Glu Gly Phe Leu Leu Ile Asp Asn Gly Val Gly Met His Asp Ile
            820                 825                 830
Val Asp Gly Val Phe Phe Phe Gly Leu Met Ala Asp Phe
        835                 840                 845

<210> 61
<211> 2562
<212> DNA
<213> Hordeum vulgare var. distichum

<220>
<221> CDS
<222> (1)..(2562)

<400> 61
atg cgt gcc ggc ggc tgc gcg gtg cag cag gcc ctg gcg ccg gag gcg          48
Met Arg Ala Gly Gly Cys Ala Val Gln Gln Ala Leu Ala Pro Glu Ala
1               5                   10                  15
gcg tcc gtt gtg agg cag gcg gtc aca ctg gcg cgg cgg cgc ggc cac          96
Ala Ser Val Val Arg Gln Ala Val Thr Leu Ala Arg Arg Arg Gly His
            20                  25                  30
gcg cag gtc acc ccg ctc cac gtc gcc acc gcc atg ctc ctt ccc ccg         144
Ala Gln Val Thr Pro Leu His Val Ala Thr Ala Met Leu Leu Pro Pro
        35                  40                  45
gcg ccg gcg ggg ctg ctc cgc gcc gcc tgc ctc cgc tcc cac tcc cac         192
Ala Pro Ala Gly Leu Leu Arg Ala Ala Cys Leu Arg Ser His Ser His
    50                  55                  60
ccg ctc cag tgc aag gcc ctc gag ctc tgc ttc aac gtc gcg ctc aac         240
Pro Leu Gln Cys Lys Ala Leu Glu Leu Cys Phe Asn Val Ala Leu Asn
65                  70                  75                  80
cgc ctc ccc acc tgc gga ccc gcc gcc atg ttc cac ggc ggc gcg cac         288
Arg Leu Pro Thr Cys Gly Pro Ala Ala Met Phe His Gly Gly Ala His
                85                  90                  95
atg cag cac cat cac cac cac cgc ggc gcc gcc gaa gca ccc gcg ctg         336
Met Gln His His His His His Arg Gly Ala Ala Glu Ala Pro Ala Leu
            100                 105                 110
tcc aac gcg ctc gtc gcc gcg ttc aag cgc gcg cag gcg cac cag cgc         384
Ser Asn Ala Leu Val Ala Ala Phe Lys Arg Ala Gln Ala His Gln Arg
        115                 120                 125
cgc ggg ggc gcc ggg gac ggc gtc cag cag acg gcc gcg ccg gtg ctc         432
Arg Gly Gly Ala Gly Asp Gly Val Gln Gln Thr Ala Ala Pro Val Leu
    130                 135                 140
gcc gcc aag gtc gag ctg gag cag ctc atc gtc tcc atc ctc gac gac         480
Ala Ala Lys Val Glu Leu Glu Gln Leu Ile Val Ser Ile Leu Asp Asp
145                 150                 155                 160
ccg agc gtg agc cgc gtc atg cgc gag gcc gga ttc tcc agc tcg caa         528
Pro Ser Val Ser Arg Val Met Arg Glu Ala Gly Phe Ser Ser Ser Gln
                165                 170                 175
gtg aag gac aac gtc gag aag gcc gtc tcc tcg tcg tcg cct tcc ttg         576
Val Lys Asp Asn Val Glu Lys Ala Val Ser Ser Ser Ser Pro Ser Leu
            180                 185                 190
gaa cgc gcg gct aac atg aag aca acg ggc tct att aag gag tcc aga         624
Glu Arg Ala Ala Asn Met Lys Thr Thr Gly Ser Ile Lys Glu Ser Arg
        195                 200                 205
gcg aag tcg gct ggc agc gac gac gcc atg cgc gtg cta gag tgc atg         672
Ala Lys Ser Ala Gly Ser Asp Asp Ala Met Arg Val Leu Glu Cys Met
    210                 215                 220
gcg agc ggg act agg cgg tgc ttg gtc gtc gtc ggc gag ggc gcg gag         720
Ala Ser Gly Thr Arg Arg Cys Leu Val Val Val Gly Glu Gly Ala Glu
```

```
225                 230                 235                 240
gcg gcg gtg aag gcc gtg atg gat aag gtg agc aag tcg gag ctt cac       768
Ala Ala Val Lys Ala Val Met Asp Lys Val Ser Lys Ser Glu Leu His
                245                 250                 255
cac cgg cac cac gag cgc ctc aag agc gtc cag ttc gtg ccg ctc tcc       816
His Arg His His Glu Arg Leu Lys Ser Val Gln Phe Val Pro Leu Ser
            260                 265                 270
gtc acg tcg ttc cgg cac gcg gcg agg gag gag gtg gat gcc aag acc       864
Val Thr Ser Phe Arg His Ala Ala Arg Glu Glu Val Asp Ala Lys Thr
        275                 280                 285
agc gac ctc cgc gcg ctc gtt tgc gag gcc cgc gcc gcc ggt aag ggc       912
Ser Asp Leu Arg Ala Leu Val Cys Glu Ala Arg Ala Ala Gly Lys Gly
    290                 295                 300
gtc gcg ctc gtc gta gag gac ctt gcc tac gcc gcc gat gcc tgg cac       960
Val Ala Leu Val Val Glu Asp Leu Ala Tyr Ala Ala Asp Ala Trp His
305                 310                 315                 320
aag aga aga ggc gag cat gtg cac ggc gga cag tca cag tac tac tgc      1008
Lys Arg Arg Gly Glu His Val His Gly Gly Gln Ser Gln Tyr Tyr Cys
                325                 330                 335
ccc gtt gag cac gcc gtc atg gag gtg agc ggc ctg gtg tcc ggc gac      1056
Pro Val Glu His Ala Val Met Glu Val Ser Gly Leu Val Ser Gly Asp
            340                 345                 350
tct ggc tcc ggc ggc gga agg ttc tgg ctg cta ggg ttt gcg agc cgc      1104
Ser Gly Ser Gly Gly Gly Arg Phe Trp Leu Leu Gly Phe Ala Ser Arg
        355                 360                 365
acg gtg ttc atg aag tgc agg ctc ggg cag cca tct ttg gag gcc gtg      1152
Thr Val Phe Met Lys Cys Arg Leu Gly Gln Pro Ser Leu Glu Ala Val
    370                 375                 380
tgg ggg atc cac ccc ctc gtc gtg cca gac ggc ggc agc ctc gcg tta      1200
Trp Gly Ile His Pro Leu Val Val Pro Asp Gly Gly Ser Leu Ala Leu
385                 390                 395                 400
agc ctc agc tgc acc agt gag gca cag ggt agc cag caa gca gga aga      1248
Ser Leu Ser Cys Thr Ser Glu Ala Gln Gly Ser Gln Gln Ala Gly Arg
                405                 410                 415
tcg ata acg gga tgg ccc ttg gtg aac ggc gcg gcc act acc ggc gag      1296
Ser Ile Thr Gly Trp Pro Leu Val Asn Gly Ala Ala Thr Thr Gly Glu
            420                 425                 430
agc gag ctc act tgg tgc gcc agg acg ccg tcc ccg gag cca aac ata      1344
Ser Glu Leu Thr Trp Cys Ala Arg Thr Pro Ser Pro Glu Pro Asn Ile
        435                 440                 445
ccg tca tgg ttt cgc cgg tac cat gac cca tat cag acc aca ccg acg      1392
Pro Ser Trp Phe Arg Arg Tyr His Asp Pro Tyr Gln Thr Thr Pro Thr
    450                 455                 460
agc agc agt acc agt ctt cac cag tta caa gat cta tgg aac ccc gtg      1440
Ser Ser Ser Thr Ser Leu His Gln Leu Gln Asp Leu Trp Asn Pro Val
465                 470                 475                 480
cgc aat tgg tca gcg acg cac cac cat aca tcc gag ctg acg ttg agc      1488
Arg Asn Trp Ser Ala Thr His His His Thr Ser Glu Leu Thr Leu Ser
                485                 490                 495
ttc tca tcc ccc gca tca ccg gca gct tct tcc ctc tcc ggc tac aac      1536
Phe Ser Ser Pro Ala Ser Pro Ala Ala Ser Ser Leu Ser Gly Tyr Asn
            500                 505                 510
aac acc ccg acg acg atg agc tcg tcc aag cca tgt cag ccc gag ccg      1584
Asn Thr Pro Thr Thr Met Ser Ser Ser Lys Pro Cys Gln Pro Glu Pro
        515                 520                 525
agg caa ccg tgg ccg ccg tcg aac caa ggg cac gag gac ggt ccg gcc      1632
Arg Gln Pro Trp Pro Pro Ser Asn Gln Gly His Glu Asp Gly Pro Ala
    530                 535                 540
aca agg gcg gag gcg att cgt gat ctt cct cgg tgg cat agt acc gaa      1680
Thr Arg Ala Glu Ala Ile Arg Asp Leu Pro Arg Trp His Ser Thr Glu
545                 550                 555                 560
atc gct ttc ccg gaa tcc ttc tct gct caa tcc aac tca ccc aac gat      1728
Ile Ala Phe Pro Glu Ser Phe Ser Ala Gln Ser Asn Ser Pro Asn Asp
                565                 570                 575
cat ggc ggc ggt acg gca gat ccg gag cgg cga cgc cag aag ttc acg      1776
```

```
His Gly Gly Gly Thr Ala Asp Pro Glu Arg Arg Arg Gln Lys Phe Thr
            580                 585                 590
gaa ctt act gca gaa aac ctc aag atc atg tgc ggc acg ctc gag gat      1824
Glu Leu Thr Ala Glu Asn Leu Lys Ile Met Cys Gly Thr Leu Glu Asp
        595                 600                 605
tgc gtg ccg ggg cac att aag gat gtg gca gcg ggc gtc gcc agt gct      1872
Cys Val Pro Gly His Ile Lys Asp Val Ala Ala Gly Val Ala Ser Ala
    610                 615                 620
gtg ctc caa cgc cgg tcc ggc atg gtg agg cag cgt gag acg ccc agg      1920
Val Leu Gln Arg Arg Ser Gly Met Val Arg Gln Arg Glu Thr Pro Arg
625                 630                 635                 640
ccg agc tcg acg acg tgg ctg ctc ttc aaa ggg agc gac cgc gac ggc      1968
Pro Ser Ser Thr Thr Trp Leu Leu Phe Lys Gly Ser Asp Arg Asp Gly
                645                 650                 655
aag aaa tcc atg gct ttg gag ctc gca aag ctc gtc ttt gga tct tac      2016
Lys Lys Ser Met Ala Leu Glu Leu Ala Lys Leu Val Phe Gly Ser Tyr
            660                 665                 670
aat gat ttc acg tcc atc tca tca gct ggc tgc acc ccg gtt cac tcc      2064
Asn Asp Phe Thr Ser Ile Ser Ser Ala Gly Cys Thr Pro Val His Ser
        675                 680                 685
agt tcc agc tct ggc gag cgt tcc ggc aag agg cag aga tcg ccg gac      2112
Ser Ser Ser Ser Gly Glu Arg Ser Gly Lys Arg Gln Arg Ser Pro Asp
    690                 695                 700
tac gag cat ggc tgc gca caa agg ttt tac gac acg atc cac gag aac      2160
Tyr Glu His Gly Cys Ala Gln Arg Phe Tyr Asp Thr Ile His Glu Asn
705                 710                 715                 720
cct cgc cgg gtg gtg atg atc gat gat gtc gag caa atg agc ctt gga      2208
Pro Arg Arg Val Val Met Ile Asp Asp Val Glu Gln Met Ser Leu Gly
                725                 730                 735
tct gag atc ggc atc aag aaa gcg att gcc agt gga agg gtg aga ggt      2256
Ser Glu Ile Gly Ile Lys Lys Ala Ile Ala Ser Gly Arg Val Arg Gly
            740                 745                 750
tgc aat ggc gtt gag acc agt ctc gag gat gcc atc ata gtg ctg agc      2304
Cys Asn Gly Val Glu Thr Ser Leu Glu Asp Ala Ile Ile Val Leu Ser
        755                 760                 765
tgc gaa gca ttc gac tcg aga tct agg gct tcc tct cct cgg gtc atc      2352
Cys Glu Ala Phe Asp Ser Arg Ser Arg Ala Ser Ser Pro Arg Val Ile
    770                 775                 780
aag cag aag gtc ata tgc gac gag gaa gat agt ggc ggc gtg gag aag      2400
Lys Gln Lys Val Ile Cys Asp Glu Glu Asp Ser Gly Gly Val Glu Lys
785                 790                 795                 800
ggg gtg acg aag ccg cca tgt ttc agc ttg gat ttg aac gag tgc gcc      2448
Gly Val Thr Lys Pro Pro Cys Phe Ser Leu Asp Leu Asn Glu Cys Ala
                805                 810                 815
tcc ggt gat gat gaa gga cat cag gag gaa gag gga ttg gat agt gac      2496
Ser Gly Asp Asp Glu Gly His Gln Glu Glu Glu Gly Leu Asp Ser Asp
            820                 825                 830
gtg gag atc tgt gat gtt gtg gat ggt gtt ttc ttc ttc cga cta ccg      2544
Val Glu Ile Cys Asp Val Val Asp Gly Val Phe Phe Phe Arg Leu Pro
        835                 840                 845
gga gat ttg tca cat tag                                              2562
Gly Asp Leu Ser His
    850

<210> 62
<211> 853
<212> PRT
<213> Hordeum vulgare var. distichum

<400> 62
Met Arg Ala Gly Gly Cys Ala Val Gln Gln Ala Leu Ala Pro Glu Ala
1               5                   10                  15
Ala Ser Val Val Arg Gln Ala Val Thr Leu Ala Arg Arg Arg Gly His
            20                  25                  30
Ala Gln Val Thr Pro Leu His Val Ala Thr Ala Met Leu Leu Pro Pro
```

```
            35                  40                  45
Ala Pro Ala Gly Leu Leu Arg Ala Ala Cys Leu Arg Ser His Ser His
    50                  55                  60
Pro Leu Gln Cys Lys Ala Leu Glu Leu Cys Phe Asn Val Ala Leu Asn
65                  70                  75                  80
Arg Leu Pro Thr Cys Gly Pro Ala Ala Met Phe His Gly Gly Ala His
                85                  90                  95
Met Gln His His His His His Arg Gly Ala Ala Glu Ala Pro Ala Leu
            100                 105                 110
Ser Asn Ala Leu Val Ala Ala Phe Lys Arg Ala Gln Ala His Gln Arg
        115                 120                 125
Arg Gly Gly Ala Gly Asp Gly Val Gln Gln Thr Ala Ala Pro Val Leu
    130                 135                 140
Ala Ala Lys Val Glu Leu Glu Gln Leu Ile Val Ser Ile Leu Asp Asp
145                 150                 155                 160
Pro Ser Val Ser Arg Val Met Arg Glu Ala Gly Phe Ser Ser Ser Gln
                165                 170                 175
Val Lys Asp Asn Val Glu Lys Ala Val Ser Ser Ser Ser Pro Ser Leu
            180                 185                 190
Glu Arg Ala Ala Asn Met Lys Thr Thr Gly Ser Ile Lys Glu Ser Arg
        195                 200                 205
Ala Lys Ser Ala Gly Ser Asp Asp Ala Met Arg Val Leu Glu Cys Met
    210                 215                 220
Ala Ser Gly Thr Arg Arg Cys Leu Val Val Val Gly Glu Gly Ala Glu
225                 230                 235                 240
Ala Ala Val Lys Ala Val Met Asp Lys Val Ser Lys Ser Glu Leu His
                245                 250                 255
His Arg His His Glu Arg Leu Lys Ser Val Gln Phe Val Pro Leu Ser
            260                 265                 270
Val Thr Ser Phe Arg His Ala Ala Arg Glu Glu Val Asp Ala Lys Thr
        275                 280                 285
Ser Asp Leu Arg Ala Leu Val Cys Glu Ala Arg Ala Ala Gly Lys Gly
    290                 295                 300
Val Ala Leu Val Val Glu Asp Leu Ala Tyr Ala Ala Asp Ala Trp His
305                 310                 315                 320
Lys Arg Arg Gly Glu His Val His Gly Gly Gln Ser Gln Tyr Tyr Cys
                325                 330                 335
Pro Val Glu His Ala Val Met Glu Val Ser Gly Leu Val Ser Gly Asp
            340                 345                 350
Ser Gly Ser Gly Gly Gly Arg Phe Trp Leu Leu Gly Phe Ala Ser Arg
        355                 360                 365
Thr Val Phe Met Lys Cys Arg Leu Gly Gln Pro Ser Leu Glu Ala Val
    370                 375                 380
Trp Gly Ile His Pro Leu Val Val Pro Asp Gly Gly Ser Leu Ala Leu
385                 390                 395                 400
Ser Leu Ser Cys Thr Ser Glu Ala Gln Gly Ser Gln Gln Ala Gly Arg
                405                 410                 415
Ser Ile Thr Gly Trp Pro Leu Val Asn Gly Ala Ala Thr Thr Gly Glu
            420                 425                 430
Ser Glu Leu Thr Trp Cys Ala Arg Thr Pro Ser Pro Glu Pro Asn Ile
        435                 440                 445
Pro Ser Trp Phe Arg Arg Tyr His Asp Pro Tyr Gln Thr Thr Pro Thr
    450                 455                 460
Ser Ser Ser Thr Ser Leu His Gln Leu Gln Asp Leu Trp Asn Pro Val
465                 470                 475                 480
Arg Asn Trp Ser Ala Thr His His His Thr Ser Glu Leu Thr Leu Ser
                485                 490                 495
Phe Ser Ser Pro Ala Ser Pro Ala Ala Ser Ser Leu Ser Gly Tyr Asn
            500                 505                 510
Asn Thr Pro Thr Thr Met Ser Ser Ser Lys Pro Cys Gln Pro Glu Pro
        515                 520                 525
Arg Gln Pro Trp Pro Pro Ser Asn Gln Gly His Glu Asp Gly Pro Ala
    530                 535                 540
Thr Arg Ala Glu Ala Ile Arg Asp Leu Pro Arg Trp His Ser Thr Glu
545                 550                 555                 560
```

```
Ile Ala Phe Pro Glu Ser Phe Ser Ala Gln Ser Asn Ser Pro Asn Asp
                565                 570                 575
His Gly Gly Gly Thr Ala Asp Pro Glu Arg Arg Arg Gln Lys Phe Thr
            580                 585                 590
Glu Leu Thr Ala Glu Asn Leu Lys Ile Met Cys Gly Thr Leu Glu Asp
        595                 600                 605
Cys Val Pro Gly His Ile Lys Asp Val Ala Ala Gly Val Ala Ser Ala
    610                 615                 620
Val Leu Gln Arg Arg Ser Gly Met Val Arg Gln Arg Glu Thr Pro Arg
625                 630                 635                 640
Pro Ser Ser Thr Thr Trp Leu Leu Phe Lys Gly Ser Asp Arg Asp Gly
                645                 650                 655
Lys Lys Ser Met Ala Leu Glu Leu Ala Lys Leu Val Phe Gly Ser Tyr
            660                 665                 670
Asn Asp Phe Thr Ser Ile Ser Ser Ala Gly Cys Thr Pro Val His Ser
        675                 680                 685
Ser Ser Ser Ser Gly Glu Arg Ser Gly Lys Arg Gln Arg Ser Pro Asp
    690                 695                 700
Tyr Glu His Gly Cys Ala Gln Arg Phe Tyr Asp Thr Ile His Glu Asn
705                 710                 715                 720
Pro Arg Arg Val Val Met Ile Asp Asp Val Glu Gln Met Ser Leu Gly
                725                 730                 735
Ser Glu Ile Gly Ile Lys Lys Ala Ile Ala Ser Gly Arg Val Arg Gly
            740                 745                 750
Cys Asn Gly Val Glu Thr Ser Leu Glu Asp Ala Ile Ile Val Leu Ser
        755                 760                 765
Cys Glu Ala Phe Asp Ser Arg Ser Arg Ala Ser Ser Pro Arg Val Ile
    770                 775                 780
Lys Gln Lys Val Ile Cys Asp Glu Glu Asp Ser Gly Gly Val Glu Lys
785                 790                 795                 800
Gly Val Thr Lys Pro Pro Cys Phe Ser Leu Asp Leu Asn Glu Cys Ala
                805                 810                 815
Ser Gly Asp Asp Glu Gly His Gln Glu Glu Glu Gly Leu Asp Ser Asp
            820                 825                 830
Val Glu Ile Cys Asp Val Val Asp Gly Val Phe Phe Phe Arg Leu Pro
        835                 840                 845
Gly Asp Leu Ser His
    850

<210> 63
<211> 2589
<212> DNA
<213> Vitis vinifera

<220>
<221> CDS
<222> (1)..(2589)

<400> 63
atg aga gca gga gtt tgc agt gta cag cag gtt cta act gct gat gca       48
Met Arg Ala Gly Val Cys Ser Val Gln Gln Val Leu Thr Ala Asp Ala
1               5                   10                  15
gca agc atg gtc aaa caa gca gtt acc ctt gct agg cgg cga ggt cat       96
Ala Ser Met Val Lys Gln Ala Val Thr Leu Ala Arg Arg Arg Gly His
            20                  25                  30
gcc caa gtt act cct ctt cat gtg gcc agt gtc atg ctt gct tcc tcc      144
Ala Gln Val Thr Pro Leu His Val Ala Ser Val Met Leu Ala Ser Ser
        35                  40                  45
tct ggt ctt ctc cgc tct gct tgc cta cga tcc cac tct cat ccc ctg      192
Ser Gly Leu Leu Arg Ser Ala Cys Leu Arg Ser His Ser His Pro Leu
    50                  55                  60
cag tgc aag gct ctg gag ctc tgc ttc aat gtg gcg ctc aac cgc ctc      240
Gln Cys Lys Ala Leu Glu Leu Cys Phe Asn Val Ala Leu Asn Arg Leu
65                  70                  75                  80
cct gcc tcc tca tca agc ccc cta tta gct cct cat tcc tct cac cct      288
```

```
Pro Ala Ser Ser Ser Ser Pro Leu Leu Ala Pro His Ser Ser His Pro
                    85                  90                  95
tcc ttg tcc aat gcc ttg gtt gca gcc ttc aag cgt gca cag gct cac        336
Ser Leu Ser Asn Ala Leu Val Ala Ala Phe Lys Arg Ala Gln Ala His
                100                 105                 110
caa cgc cgt gcc tcc atc gaa aac cag caa caa ccc att cta gct ctc        384
Gln Arg Arg Ala Ser Ile Glu Asn Gln Gln Gln Pro Ile Leu Ala Leu
        115                 120                 125
aaa gta gag ata gaa cag ctc ata atc tcc atc cta cat gac cca agt        432
Lys Val Glu Ile Glu Gln Leu Ile Ile Ser Ile Leu His Asp Pro Ser
    130                 135                 140
gtt agt aga gtc atg aga gag gct ggt ttc tct agt acc caa ttg aga        480
Val Ser Arg Val Met Arg Glu Ala Gly Phe Ser Ser Thr Gln Leu Arg
145                 150                 155                 160
acc aac ata gaa caa gct gtc tct ttg gat gtt tgt tct caa agc cct        528
Thr Asn Ile Glu Gln Ala Val Ser Leu Asp Val Cys Ser Gln Ser Pro
                165                 170                 175
gct gtg agt agc ctg tct aag gaa agt aac aac cct cct cta ata ctc        576
Ala Val Ser Ser Leu Ser Lys Glu Ser Asn Asn Pro Pro Leu Ile Leu
            180                 185                 190
ggt act aat gtg tcc caa tct tca aca ttt att cag ttt gga gta act        624
Gly Thr Asn Val Ser Gln Ser Ser Thr Phe Ile Gln Phe Gly Val Thr
        195                 200                 205
ctg aac aat cca ttt gac gaa gct cag gaa gaa gat gta aag agt ctt        672
Leu Asn Asn Pro Phe Asp Glu Ala Gln Glu Glu Asp Val Lys Ser Leu
    210                 215                 220
ttg gac gca ttt aca agc aaa aga agg aga aac act gtt gtt gta gga        720
Leu Asp Ala Phe Thr Ser Lys Arg Arg Arg Asn Thr Val Val Val Gly
225                 230                 235                 240
gag act ctg gcc agt gct gag ggt gta gtt aga ggg ctg atg aac aag        768
Glu Thr Leu Ala Ser Ala Glu Gly Val Val Arg Gly Leu Met Asn Lys
                245                 250                 255
ttt gag aga gga gat gtc cct gga gat ctg agg tat gtg cag ttt ata        816
Phe Glu Arg Gly Asp Val Pro Gly Asp Leu Arg Tyr Val Gln Phe Ile
            260                 265                 270
agc ctt cct ctc ttc tct tta aag aat ctc tcc aag gag gag gtt gaa        864
Ser Leu Pro Leu Phe Ser Leu Lys Asn Leu Ser Lys Glu Glu Val Glu
        275                 280                 285
cag aag ctt gtg aag ctg act tgc ctt ctg aaa agc tat gtg tgt aga        912
Gln Lys Leu Val Lys Leu Thr Cys Leu Leu Lys Ser Tyr Val Cys Arg
    290                 295                 300
ggg gtg gtt ttg tat ttg ggt gat ctc aaa tgg gtc tct gag ttt gag        960
Gly Val Val Leu Tyr Leu Gly Asp Leu Lys Trp Val Ser Glu Phe Glu
305                 310                 315                 320
tca aat tat ggt gag agg aga aac tac tgc tct cct gtg gag cac ata        1008
Ser Asn Tyr Gly Glu Arg Arg Asn Tyr Cys Ser Pro Val Glu His Ile
                325                 330                 335
atc atg gag ctc gga aga atg atg tgt gga att ggg gat aga gga cga        1056
Ile Met Glu Leu Gly Arg Met Met Cys Gly Ile Gly Asp Arg Gly Arg
            340                 345                 350
atg tgg ctt ttg ggg act gca acc ttc caa act tac atg aga tgc aaa        1104
Met Trp Leu Leu Gly Thr Ala Thr Phe Gln Thr Tyr Met Arg Cys Lys
        355                 360                 365
gca ggc cat cct tct ttg gag act att tgg gaa ctt cat cct ctt act        1152
Ala Gly His Pro Ser Leu Glu Thr Ile Trp Glu Leu His Pro Leu Thr
    370                 375                 380
att cct gtt ggc agc ttg ggc ctc ggt ctc aat ctt gac agc aat ttg        1200
Ile Pro Val Gly Ser Leu Gly Leu Gly Leu Asn Leu Asp Ser Asn Leu
385                 390                 395                 400
cag ggc cgg ttc caa agt aag gca tct gga gat ggg act tct tgg tca        1248
Gln Gly Arg Phe Gln Ser Lys Ala Ser Gly Asp Gly Thr Ser Trp Ser
                405                 410                 415
cta ctt caa agt gga gat aag cac ctt act tgc agc aca aat tgc tct        1296
Leu Leu Gln Ser Gly Asp Lys His Leu Thr Cys Ser Thr Asn Cys Ser
            420                 425                 430
```

```
gat aat ttt gac aaa gaa tct caa agt ata gca tgc agt ttt cgc aat        1344
Asp Asn Phe Asp Lys Glu Ser Gln Ser Ile Ala Cys Ser Phe Arg Asn
            435                 440                 445
ggc gag tcc acc acc acc atc acc act tcc acc agc tca agc ttg cct        1392
Gly Glu Ser Thr Thr Thr Ile Thr Thr Ser Thr Ser Ser Ser Leu Pro
    450                 455                 460
tca tgg ctc caa aaa gag aaa aga aga aag atc atg gac gat cag gaa        1440
Ser Trp Leu Gln Lys Glu Lys Arg Arg Lys Ile Met Asp Asp Gln Glu
465                 470                 475                 480
tgt gtc caa gtc aga gat ctc tgc aac aaa tgg aac tcg ttt tgc agc        1488
Cys Val Gln Val Arg Asp Leu Cys Asn Lys Trp Asn Ser Phe Cys Ser
                485                 490                 495
tca gtc cac aaa aag gcc cac agt act gag aaa gcc ctg aat ttt tct        1536
Ser Val His Lys Lys Ala His Ser Thr Glu Lys Ala Leu Asn Phe Ser
            500                 505                 510
tca cca tct cct tcc tcc act tcc atc tcc tcc tat gac caa tgc agc        1584
Ser Pro Ser Pro Ser Ser Thr Ser Ile Ser Ser Tyr Asp Gln Cys Ser
        515                 520                 525
cct aat ctg caa cag aac cac cta agt tgg cca gcc atc att gag ccg        1632
Pro Asn Leu Gln Gln Asn His Leu Ser Trp Pro Ala Ile Ile Glu Pro
    530                 535                 540
aaa cca cct ctg aaa gaa cac cag ttc tgg ata tct gaa aat gtt gat        1680
Lys Pro Pro Leu Lys Glu His Gln Phe Trp Ile Ser Glu Asn Val Asp
545                 550                 555                 560
gaa ggc ctt gaa ccc aag ttc agt atg cac ata gca gag aga aat ttt        1728
Glu Gly Leu Glu Pro Lys Phe Ser Met His Ile Ala Glu Arg Asn Phe
                565                 570                 575
cca ata cca gat ctt tta tca aat cct aac tct tcc cct aat tca gct        1776
Pro Ile Pro Asp Leu Leu Ser Asn Pro Asn Ser Ser Pro Asn Ser Ala
            580                 585                 590
tct tct agt gag gca ata gag gat gga gag ggt ctt tat ggt ttc aaa        1824
Ser Ser Ser Glu Ala Ile Glu Asp Gly Glu Gly Leu Tyr Gly Phe Lys
        595                 600                 605
gag ctt aat gcg gag aat ctg aga atc tta tgc aat gca ttg gag aga        1872
Glu Leu Asn Ala Glu Asn Leu Arg Ile Leu Cys Asn Ala Leu Glu Arg
    610                 615                 620
agg gtt cca tgg cag aag gat atc att cct gaa ata gca agc acc att        1920
Arg Val Pro Trp Gln Lys Asp Ile Ile Pro Glu Ile Ala Ser Thr Ile
625                 630                 635                 640
ctt gaa tgc agg tca gga aca ctg aga gga aaa aac aag ttg aag cag        1968
Leu Glu Cys Arg Ser Gly Thr Leu Arg Gly Lys Asn Lys Leu Lys Gln
                645                 650                 655
agg gag gac aag gaa gaa act tgg ctg ctc ttc tta gga gtt gac ttt        2016
Arg Glu Asp Lys Glu Glu Thr Trp Leu Leu Phe Leu Gly Val Asp Phe
            660                 665                 670
caa ggc aaa gac aag att gca agg gag ata gct aag ctt gtt ttt ggc        2064
Gln Gly Lys Asp Lys Ile Ala Arg Glu Ile Ala Lys Leu Val Phe Gly
        675                 680                 685
tct cag agc aag ttc atc tca att ggt ctg agc agt tta gga tca aca        2112
Ser Gln Ser Lys Phe Ile Ser Ile Gly Leu Ser Ser Leu Gly Ser Thr
    690                 695                 700
aga gct gat tcc acc gag gat ttc cta agc aaa caa gca aga gac gag        2160
Arg Ala Asp Ser Thr Glu Asp Phe Leu Ser Lys Gln Ala Arg Asp Glu
705                 710                 715                 720
cct gtt ggt agt tat att gag aaa ttc gct gaa gcg gtg cat gag aat        2208
Pro Val Gly Ser Tyr Ile Glu Lys Phe Ala Glu Ala Val His Glu Asn
                725                 730                 735
cct cac aga gtg ttc ttc ata gaa gat gtg gag caa cta gat tac tct        2256
Pro His Arg Val Phe Phe Ile Glu Asp Val Glu Gln Leu Asp Tyr Ser
            740                 745                 750
tct caa atg ggg gtt aag aga gga att gag agt gga aga ata caa att        2304
Ser Gln Met Gly Val Lys Arg Gly Ile Glu Ser Gly Arg Ile Gln Ile
        755                 760                 765
gct gga ggt gaa gca ttt tct ctt gag gat gcc atc atc ata ttc agt        2352
Ala Gly Gly Glu Ala Phe Ser Leu Glu Asp Ala Ile Ile Ile Phe Ser
```

```
    770                 775                 780
tgt gaa agc ttc agc tca gta tca aga gcc agc tct cct cca ccc atg       2400
Cys Glu Ser Phe Ser Ser Val Ser Arg Ala Ser Ser Pro Pro Pro Met
785                 790                 795                 800
ggg cta aaa tct gaa gag aat gag gaa aaa gac cgg gat aat gag ttg       2448
Gly Leu Lys Ser Glu Glu Asn Glu Glu Lys Asp Arg Asp Asn Glu Leu
                805                 810                 815
gag aag aga agc cca tgt gtg tct cta gat ctg aat ctt tca gct gaa       2496
Glu Lys Arg Ser Pro Cys Val Ser Leu Asp Leu Asn Leu Ser Ala Glu
            820                 825                 830
gat aat caa gaa tat gga caa aac tcg gtt gca gac act ggg gtt cta       2544
Asp Asn Gln Glu Tyr Gly Gln Asn Ser Val Ala Asp Thr Gly Val Leu
        835                 840                 845
gat tct gtg gac agg cag ttt att ttc aaa att caa gag ttg tga           2589
Asp Ser Val Asp Arg Gln Phe Ile Phe Lys Ile Gln Glu Leu
    850                 855                 860
```

<210> 64
<211> 862
<212> PRT
<213> Vitis vinifera

<400> 64

```
Met Arg Ala Gly Val Cys Ser Val Gln Gln Val Leu Thr Ala Asp Ala
1               5                   10                  15
Ala Ser Met Val Lys Gln Ala Val Thr Leu Ala Arg Arg Arg Gly His
            20                  25                  30
Ala Gln Val Thr Pro Leu His Val Ala Ser Val Met Leu Ala Ser Ser
        35                  40                  45
Ser Gly Leu Leu Arg Ser Ala Cys Leu Arg Ser His Ser His Pro Leu
    50                  55                  60
Gln Cys Lys Ala Leu Glu Leu Cys Phe Asn Val Ala Leu Asn Arg Leu
65                  70                  75                  80
Pro Ala Ser Ser Ser Ser Pro Leu Leu Ala Pro His Ser Ser His Pro
                85                  90                  95
Ser Leu Ser Asn Ala Leu Val Ala Ala Phe Lys Arg Ala Gln Ala His
            100                 105                 110
Gln Arg Arg Ala Ser Ile Glu Asn Gln Gln Gln Pro Ile Leu Ala Leu
        115                 120                 125
Lys Val Glu Ile Glu Gln Leu Ile Ile Ser Ile Leu His Asp Pro Ser
    130                 135                 140
Val Ser Arg Val Met Arg Glu Ala Gly Phe Ser Ser Thr Gln Leu Arg
145                 150                 155                 160
Thr Asn Ile Glu Gln Ala Val Ser Leu Asp Val Cys Ser Gln Ser Pro
                165                 170                 175
Ala Val Ser Ser Leu Ser Lys Glu Ser Asn Asn Pro Pro Leu Ile Leu
            180                 185                 190
Gly Thr Asn Val Ser Gln Ser Ser Thr Phe Ile Gln Phe Gly Val Thr
        195                 200                 205
Leu Asn Asn Pro Phe Asp Glu Ala Gln Glu Glu Asp Val Lys Ser Leu
    210                 215                 220
Leu Asp Ala Phe Thr Ser Lys Arg Arg Arg Asn Thr Val Val Val Gly
225                 230                 235                 240
Glu Thr Leu Ala Ser Ala Glu Gly Val Val Arg Gly Leu Met Asn Lys
                245                 250                 255
Phe Glu Arg Gly Asp Val Pro Gly Asp Leu Arg Tyr Val Gln Phe Ile
            260                 265                 270
Ser Leu Pro Leu Phe Ser Leu Lys Asn Leu Ser Lys Glu Glu Val Glu
        275                 280                 285
Gln Lys Leu Val Lys Leu Thr Cys Leu Leu Lys Ser Tyr Val Cys Arg
    290                 295                 300
Gly Val Val Leu Tyr Leu Gly Asp Leu Lys Trp Val Ser Glu Phe Glu
305                 310                 315                 320
Ser Asn Tyr Gly Glu Arg Arg Asn Tyr Cys Ser Pro Val Glu His Ile
                325                 330                 335
```

Ile Met Glu Leu Gly Arg Met Met Cys Gly Ile Gly Asp Arg Gly Arg
340 345 350
Met Trp Leu Leu Gly Thr Ala Thr Phe Gln Thr Tyr Met Arg Cys Lys
355 360 365
Ala Gly His Pro Ser Leu Glu Thr Ile Trp Glu Leu His Pro Leu Thr
370 375 380
Ile Pro Val Gly Ser Leu Gly Leu Gly Leu Asn Leu Asp Ser Asn Leu
385 390 395 400
Gln Gly Arg Phe Gln Ser Lys Ala Ser Gly Asp Gly Thr Ser Trp Ser
405 410 415
Leu Leu Gln Ser Gly Asp Lys His Leu Thr Cys Ser Thr Asn Cys Ser
420 425 430
Asp Asn Phe Asp Lys Glu Ser Gln Ser Ile Ala Cys Ser Phe Arg Asn
435 440 445
Gly Glu Ser Thr Thr Thr Ile Thr Thr Ser Thr Ser Ser Ser Leu Pro
450 455 460
Ser Trp Leu Gln Lys Glu Lys Arg Arg Lys Ile Met Asp Asp Gln Glu
465 470 475 480
Cys Val Gln Val Arg Asp Leu Cys Asn Lys Trp Asn Ser Phe Cys Ser
485 490 495
Ser Val His Lys Lys Ala His Ser Thr Glu Lys Ala Leu Asn Phe Ser
500 505 510
Ser Pro Ser Pro Ser Ser Thr Ser Ile Ser Ser Tyr Asp Gln Cys Ser
515 520 525
Pro Asn Leu Gln Gln Asn His Leu Ser Trp Pro Ala Ile Ile Glu Pro
530 535 540
Lys Pro Pro Leu Lys Glu His Gln Phe Trp Ile Ser Glu Asn Val Asp
545 550 555 560
Glu Gly Leu Glu Pro Lys Phe Ser Met His Ile Ala Glu Arg Asn Phe
565 570 575
Pro Ile Pro Asp Leu Leu Ser Asn Pro Asn Ser Ser Pro Asn Ser Ala
580 585 590
Ser Ser Ser Glu Ala Ile Glu Asp Gly Glu Gly Leu Tyr Gly Phe Lys
595 600 605
Glu Leu Asn Ala Glu Asn Leu Arg Ile Leu Cys Asn Ala Leu Glu Arg
610 615 620
Arg Val Pro Trp Gln Lys Asp Ile Ile Pro Glu Ile Ala Ser Thr Ile
625 630 635 640
Leu Glu Cys Arg Ser Gly Thr Leu Arg Gly Lys Asn Lys Leu Lys Gln
645 650 655
Arg Glu Asp Lys Glu Glu Thr Trp Leu Leu Phe Leu Gly Val Asp Phe
660 665 670
Gln Gly Lys Asp Lys Ile Ala Arg Glu Ile Ala Lys Leu Val Phe Gly
675 680 685
Ser Gln Ser Lys Phe Ile Ser Ile Gly Leu Ser Ser Leu Gly Ser Thr
690 695 700
Arg Ala Asp Ser Thr Glu Asp Phe Leu Ser Lys Gln Ala Arg Asp Glu
705 710 715 720
Pro Val Gly Ser Tyr Ile Glu Lys Phe Ala Glu Ala Val His Glu Asn
725 730 735
Pro His Arg Val Phe Phe Ile Glu Asp Val Glu Gln Leu Asp Tyr Ser
740 745 750
Ser Gln Met Gly Val Lys Arg Gly Ile Glu Ser Gly Arg Ile Gln Ile
755 760 765
Ala Gly Gly Glu Ala Phe Ser Leu Glu Asp Ala Ile Ile Ile Phe Ser
770 775 780
Cys Glu Ser Phe Ser Ser Val Ser Arg Ala Ser Ser Pro Pro Pro Met
785 790 795 800
Gly Leu Lys Ser Glu Glu Asn Glu Glu Lys Asp Arg Asp Asn Glu Leu
805 810 815
Glu Lys Arg Ser Pro Cys Val Ser Leu Asp Leu Asn Leu Ser Ala Glu
820 825 830
Asp Asn Gln Glu Tyr Gly Gln Asn Ser Val Ala Asp Thr Gly Val Leu
835 840 845
Asp Ser Val Asp Arg Gln Phe Ile Phe Lys Ile Gln Glu Leu

850 855 860

<210> 65
<211> 2484
<212> DNA
<213> Vitis vinifera

<220>
<221> CDS
<222> (1)..(2484)

<400> 65
```
atg aga gcc gga ggt tgt aca gtg caa cag gct cta act gcg gag gcg       48
Met Arg Ala Gly Gly Cys Thr Val Gln Gln Ala Leu Thr Ala Glu Ala
1               5                   10                  15
gcc ggc gtg gtg aaa caa gca gta acc ctt gct agg cgg cgt gga cat       96
Ala Gly Val Val Lys Gln Ala Val Thr Leu Ala Arg Arg Arg Gly His
            20                  25                  30
gcc caa gtc act cct cta cat gta gcc aac acc atg ctc gcc gct aca       144
Ala Gln Val Thr Pro Leu His Val Ala Asn Thr Met Leu Ala Ala Thr
        35                  40                  45
aac ggc ctg ctc cga acc gct tgt ctt caa tcc cac tcc cac ccc ctc       192
Asn Gly Leu Leu Arg Thr Ala Cys Leu Gln Ser His Ser His Pro Leu
    50                  55                  60
cag tgc aaa gcc cta gaa ctt tgc ttc aac gtt gcc ctc aac cgc ctt       240
Gln Cys Lys Ala Leu Glu Leu Cys Phe Asn Val Ala Leu Asn Arg Leu
65                  70                  75                  80
cca gcc tcc aca tcc agt ccc atg ctg ggt cct cat tcc caa cat cct       288
Pro Ala Ser Thr Ser Ser Pro Met Leu Gly Pro His Ser Gln His Pro
                85                  90                  95
tcc atc tcc aac gca ttg gtt gct gcc ttc aag cga gct cag gcc cat       336
Ser Ile Ser Asn Ala Leu Val Ala Ala Phe Lys Arg Ala Gln Ala His
            100                 105                 110
cag cga cgc ggc tct att gag aac cag cag caa cct ctt cta gca gtt       384
Gln Arg Arg Gly Ser Ile Glu Asn Gln Gln Gln Pro Leu Leu Ala Val
        115                 120                 125
aaa ata gag cta gag cag ctc ata atc tct att tta gat gat cct agt       432
Lys Ile Glu Leu Glu Gln Leu Ile Ile Ser Ile Leu Asp Asp Pro Ser
    130                 135                 140
gtg agt aga gtc atg aga gaa gct ggg ttc tcc agt acc caa gtg aaa       480
Val Ser Arg Val Met Arg Glu Ala Gly Phe Ser Ser Thr Gln Val Lys
145                 150                 155                 160
agc aat gta gag caa gct gtc tcc ttg gaa atc tgc tct caa gct cca       528
Ser Asn Val Glu Gln Ala Val Ser Leu Glu Ile Cys Ser Gln Ala Pro
                165                 170                 175
tcc gtg agt agt aag tcc aag gaa aat cca gtc agg aat gag gat gtg       576
Ser Val Ser Ser Lys Ser Lys Glu Asn Pro Val Arg Asn Glu Asp Val
            180                 185                 190
atg agt gta ata gag aat ctg atg aat aaa agg agg aaa aat act gtg       624
Met Ser Val Ile Glu Asn Leu Met Asn Lys Arg Arg Lys Asn Thr Val
        195                 200                 205
att gtt gga gag tgt ctg gcg act atc gag ggt gta gtt aga ggg gtg       672
Ile Val Gly Glu Cys Leu Ala Thr Ile Glu Gly Val Val Arg Gly Val
    210                 215                 220
atg gac aaa gtt gac aag gga gat gtt cca gag gct ttg aga gat gtt       720
Met Asp Lys Val Asp Lys Gly Asp Val Pro Glu Ala Leu Arg Asp Val
225                 230                 235                 240
aag ctt ata agc ctt cct ctc ttc tct ttt gga cat cac tcc aga gaa       768
Lys Leu Ile Ser Leu Pro Leu Phe Ser Phe Gly His His Ser Arg Glu
                245                 250                 255
gag gtc gaa cag aag ctc ggg gag ctc aag agc ctt gtg aag agt tgc       816
Glu Val Glu Gln Lys Leu Gly Glu Leu Lys Ser Leu Val Lys Ser Cys
            260                 265                 270
gtg gga aga ggg gtt att ttg tat ttg gaa gat ctt aaa tgg act acg       864
Val Gly Arg Gly Val Ile Leu Tyr Leu Glu Asp Leu Lys Trp Thr Thr
```

```
        275                 280                 285
gat tat aga gct agt tct agt gag caa ggg agg aac tat tat tgt ccc       912
Asp Tyr Arg Ala Ser Ser Ser Glu Gln Gly Arg Asn Tyr Tyr Cys Pro
    290                 295                 300
gtg gag cac atg atc atg gag ctt ggg aaa ttg gtt tgt gga ttt ggg       960
Val Glu His Met Ile Met Glu Leu Gly Lys Leu Val Cys Gly Phe Gly
305                 310                 315                 320
gag aac gga agg ttt tgg ctc atg gga atc gca act ttc caa act tac      1008
Glu Asn Gly Arg Phe Trp Leu Met Gly Ile Ala Thr Phe Gln Thr Tyr
                325                 330                 335
tcg aga tgt aga act ggc cat cca tcg ctg gag act att tgg agt cta      1056
Ser Arg Cys Arg Thr Gly His Pro Ser Leu Glu Thr Ile Trp Ser Leu
            340                 345                 350
cat cct ctt aca att cct gca agc agc ttg gcc ttg agt ctc atg cct      1104
His Pro Leu Thr Ile Pro Ala Ser Ser Leu Ala Leu Ser Leu Met Pro
        355                 360                 365
gac agt gat cta caa agt cag ttc agt agc aaa aag gct gga agt ggg      1152
Asp Ser Asp Leu Gln Ser Gln Phe Ser Ser Lys Lys Ala Gly Ser Gly
    370                 375                 380
acc agt aat tgg ctt atg ctt gaa ggt gga gcg gag aag cag ctt act      1200
Thr Ser Asn Trp Leu Met Leu Glu Gly Gly Ala Glu Lys Gln Leu Thr
385                 390                 395                 400
tgc tgc gct gat tgc tca gcc aat ttc gag aat gaa gct cga agc ata      1248
Cys Cys Ala Asp Cys Ser Ala Asn Phe Glu Asn Glu Ala Arg Ser Ile
                405                 410                 415
cca acc agc act tgt aac agt gat tcc aca act tca acc ctt cca aca      1296
Pro Thr Ser Thr Cys Asn Ser Asp Ser Thr Thr Ser Thr Leu Pro Thr
            420                 425                 430
tgg ctc caa cag tac aaa gat gag aac aaa aaa ctt tcc cgt aat gat      1344
Trp Leu Gln Gln Tyr Lys Asp Glu Asn Lys Lys Leu Ser Arg Asn Asp
        435                 440                 445
cag gac tgt gta gca gtc aga gat ctc tgc aaa aaa tgg aat tct att      1392
Gln Asp Cys Val Ala Val Arg Asp Leu Cys Lys Lys Trp Asn Ser Ile
    450                 455                 460
tgc agt tct gcc cac aaa caa ccc cac tca tct gag aaa acc cta aca      1440
Cys Ser Ser Ala His Lys Gln Pro His Ser Ser Glu Lys Thr Leu Thr
465                 470                 475                 480
ttc tct tct ctt tca cct tct tcc tct act tct ggc ttt tca tat gac      1488
Phe Ser Ser Leu Ser Pro Ser Ser Ser Thr Ser Gly Phe Ser Tyr Asp
                485                 490                 495
cag caa tat cct aat ttg cac cag acc cac caa ggt tgg cca gtg gtt      1536
Gln Gln Tyr Pro Asn Leu His Gln Thr His Gln Gly Trp Pro Val Val
            500                 505                 510
gaa cac aaa cag tcc tgg aga gac aat cat ttc tgg gtt tcc gaa gct      1584
Glu His Lys Gln Ser Trp Arg Asp Asn His Phe Trp Val Ser Glu Ala
        515                 520                 525
ctt aac aaa acc tat gaa ccc agt ttg aga atg tac att ccg gag cat      1632
Leu Asn Lys Thr Tyr Glu Pro Ser Leu Arg Met Tyr Ile Pro Glu His
    530                 535                 540
tct gat cgc aaa tat gcg tcg aac cct aat tct acc cct aat tcg gcc      1680
Ser Asp Arg Lys Tyr Ala Ser Asn Pro Asn Ser Thr Pro Asn Ser Ala
545                 550                 555                 560
tct tca agc gat gtc atg gaa atg gag tac gtt caa agg ttc aag gag      1728
Ser Ser Ser Asp Val Met Glu Met Glu Tyr Val Gln Arg Phe Lys Glu
                565                 570                 575
ctc aac gct gag aac ctg aac act cta tgc aat gca ttg gag aaa aag      1776
Leu Asn Ala Glu Asn Leu Asn Thr Leu Cys Asn Ala Leu Glu Lys Lys
            580                 585                 590
gtc cca tgg cag aaa gat ata att cct gac atc gct agc acg atc tta      1824
Val Pro Trp Gln Lys Asp Ile Ile Pro Asp Ile Ala Ser Thr Ile Leu
        595                 600                 605
caa tgc agg tct gga atg gta aga cgc aaa gga aag gtc aaa aat agc      1872
Gln Cys Arg Ser Gly Met Val Arg Arg Lys Gly Lys Val Lys Asn Ser
    610                 615                 620
gag acc aaa gaa gaa aca tgg ttt ttc ttc caa ggc gtt gat atg gat      1920
```

```
Glu Thr Lys Glu Glu Thr Trp Phe Phe Phe Gln Gly Val Asp Met Asp
625                 630                 635                 640
gct aaa gag aag att gcg aga gaa ttg gct aga ctt gtt ttc ggc tcc        1968
Ala Lys Glu Lys Ile Ala Arg Glu Leu Ala Arg Leu Val Phe Gly Ser
                645                 650                 655
cag aac aac ttc gtt tca att gct ctg agc agc ttc tca tct aca agg        2016
Gln Asn Asn Phe Val Ser Ile Ala Leu Ser Ser Phe Ser Ser Thr Arg
            660                 665                 670
gcg gat tca aca gaa gat ctc cga aat aag aga tca aga gat gag caa        2064
Ala Asp Ser Thr Glu Asp Leu Arg Asn Lys Arg Ser Arg Asp Glu Gln
        675                 680                 685
agc tgc agt tac atc gag agg ttc gct gag gca gtg ggc agt aat cca        2112
Ser Cys Ser Tyr Ile Glu Arg Phe Ala Glu Ala Val Gly Ser Asn Pro
    690                 695                 700
cac cgg gta ttc tta gcg gaa gat gtg gag caa gcg gat tac tgc tcc        2160
His Arg Val Phe Leu Ala Glu Asp Val Glu Gln Ala Asp Tyr Cys Ser
705                 710                 715                 720
caa atg ggt atc aaa agg gcc aca gaa aga gga aga ata acc aat tcg        2208
Gln Met Gly Ile Lys Arg Ala Thr Glu Arg Gly Arg Ile Thr Asn Ser
                725                 730                 735
aat gga gaa gaa atc agc ctg agc gac gca atc atc att ttg agc tgt        2256
Asn Gly Glu Glu Ile Ser Leu Ser Asp Ala Ile Ile Ile Leu Ser Cys
            740                 745                 750
gaa agc ttc agc tcg agg tct aga gcc tgc tca cct cca atc aaa caa        2304
Glu Ser Phe Ser Ser Arg Ser Arg Ala Cys Ser Pro Pro Ile Lys Gln
        755                 760                 765
aaa tca gat gag ttc gaa gag gag aaa ggt gga ggt ggg ggt gag gag        2352
Lys Ser Asp Glu Phe Glu Glu Glu Lys Gly Gly Gly Gly Gly Glu Glu
    770                 775                 780
ata agc cca tgt gtg tct ttg gat ttg aat att tgc att gat gat gac        2400
Ile Ser Pro Cys Val Ser Leu Asp Leu Asn Ile Cys Ile Asp Asp Asp
785                 790                 795                 800
ggc gtt gag gat gaa tca atc gac gac atc gga ctt ctt gaa tca gtg        2448
Gly Val Glu Asp Glu Ser Ile Asp Asp Ile Gly Leu Leu Glu Ser Val
                805                 810                 815
gat aga agg att act ttc aaa att cag gag tta taa                        2484
Asp Arg Arg Ile Thr Phe Lys Ile Gln Glu Leu
            820                 825
```

<210> 66
<211> 827
<212> PRT
<213> Vitis vinifera

<400> 66

```
Met Arg Ala Gly Gly Cys Thr Val Gln Gln Ala Leu Thr Ala Glu Ala
1               5                   10                  15
Ala Gly Val Val Lys Gln Ala Val Thr Leu Ala Arg Arg Arg Gly His
            20                  25                  30
Ala Gln Val Thr Pro Leu His Val Ala Asn Thr Met Leu Ala Ala Thr
        35                  40                  45
Asn Gly Leu Leu Arg Thr Ala Cys Leu Gln Ser His Ser His Pro Leu
    50                  55                  60
Gln Cys Lys Ala Leu Glu Leu Cys Phe Asn Val Ala Leu Asn Arg Leu
65                  70                  75                  80
Pro Ala Ser Thr Ser Ser Pro Met Leu Gly Pro His Ser Gln His Pro
                85                  90                  95
Ser Ile Ser Asn Ala Leu Val Ala Ala Phe Lys Arg Ala Gln Ala His
            100                 105                 110
Gln Arg Arg Gly Ser Ile Glu Asn Gln Gln Gln Pro Leu Leu Ala Val
        115                 120                 125
Lys Ile Glu Leu Glu Gln Leu Ile Ile Ser Ile Leu Asp Asp Pro Ser
    130                 135                 140
Val Ser Arg Val Met Arg Glu Ala Gly Phe Ser Ser Thr Gln Val Lys
145                 150                 155                 160
```

Ser Asn Val Glu Gln Ala Val Ser Leu Glu Ile Cys Ser Gln Ala Pro
165 170 175
Ser Val Ser Ser Lys Ser Lys Glu Asn Pro Val Arg Asn Glu Asp Val
180 185 190
Met Ser Val Ile Glu Asn Leu Met Asn Lys Arg Arg Lys Asn Thr Val
195 200 205
Ile Val Gly Glu Cys Leu Ala Thr Ile Glu Gly Val Val Arg Gly Val
210 215 220
Met Asp Lys Val Asp Lys Gly Asp Val Pro Glu Ala Leu Arg Asp Val
225 230 235 240
Lys Leu Ile Ser Leu Pro Leu Phe Ser Phe Gly His His Ser Arg Glu
245 250 255
Glu Val Glu Gln Lys Leu Gly Glu Leu Lys Ser Leu Val Lys Ser Cys
260 265 270
Val Gly Arg Gly Val Ile Leu Tyr Leu Glu Asp Leu Lys Trp Thr Thr
275 280 285
Asp Tyr Arg Ala Ser Ser Ser Glu Gln Gly Arg Asn Tyr Tyr Cys Pro
290 295 300
Val Glu His Met Ile Met Glu Leu Gly Lys Leu Val Cys Gly Phe Gly
305 310 315 320
Glu Asn Gly Arg Phe Trp Leu Met Gly Ile Ala Thr Phe Gln Thr Tyr
325 330 335
Ser Arg Cys Arg Thr Gly His Pro Ser Leu Glu Thr Ile Trp Ser Leu
340 345 350
His Pro Leu Thr Ile Pro Ala Ser Ser Leu Ala Leu Ser Leu Met Pro
355 360 365
Asp Ser Asp Leu Gln Ser Gln Phe Ser Ser Lys Lys Ala Gly Ser Gly
370 375 380
Thr Ser Asn Trp Leu Met Leu Glu Gly Gly Ala Glu Lys Gln Leu Thr
385 390 395 400
Cys Cys Ala Asp Cys Ser Ala Asn Phe Glu Asn Glu Ala Arg Ser Ile
405 410 415
Pro Thr Ser Thr Cys Asn Ser Asp Ser Thr Thr Ser Thr Leu Pro Thr
420 425 430
Trp Leu Gln Gln Tyr Lys Asp Glu Asn Lys Lys Leu Ser Arg Asn Asp
435 440 445
Gln Asp Cys Val Ala Val Arg Asp Leu Cys Lys Lys Trp Asn Ser Ile
450 455 460
Cys Ser Ser Ala His Lys Gln Pro His Ser Ser Glu Lys Thr Leu Thr
465 470 475 480
Phe Ser Ser Leu Ser Pro Ser Ser Ser Thr Ser Gly Phe Ser Tyr Asp
485 490 495
Gln Gln Tyr Pro Asn Leu His Gln Thr His Gln Gly Trp Pro Val Val
500 505 510
Glu His Lys Gln Ser Trp Arg Asp Asn His Phe Trp Val Ser Glu Ala
515 520 525
Leu Asn Lys Thr Tyr Glu Pro Ser Leu Arg Met Tyr Ile Pro Glu His
530 535 540
Ser Asp Arg Lys Tyr Ala Ser Asn Pro Asn Ser Thr Pro Asn Ser Ala
545 550 555 560
Ser Ser Ser Asp Val Met Glu Met Glu Tyr Val Gln Arg Phe Lys Glu
565 570 575
Leu Asn Ala Glu Asn Leu Asn Thr Leu Cys Asn Ala Leu Glu Lys Lys
580 585 590
Val Pro Trp Gln Lys Asp Ile Ile Pro Asp Ile Ala Ser Thr Ile Leu
595 600 605
Gln Cys Arg Ser Gly Met Val Arg Arg Lys Gly Lys Val Lys Asn Ser
610 615 620
Glu Thr Lys Glu Glu Thr Trp Phe Phe Phe Gln Gly Val Asp Met Asp
625 630 635 640
Ala Lys Glu Lys Ile Ala Arg Glu Leu Ala Arg Leu Val Phe Gly Ser
645 650 655
Gln Asn Asn Phe Val Ser Ile Ala Leu Ser Ser Phe Ser Ser Thr Arg
660 665 670
Ala Asp Ser Thr Glu Asp Leu Arg Asn Lys Arg Ser Arg Asp Glu Gln

```
        675                 680                 685
Ser Cys Ser Tyr Ile Glu Arg Phe Ala Glu Ala Val Gly Ser Asn Pro
    690                 695                 700
His Arg Val Phe Leu Ala Glu Asp Val Glu Gln Ala Asp Tyr Cys Ser
705                 710                 715                 720
Gln Met Gly Ile Lys Arg Ala Thr Glu Arg Gly Arg Ile Thr Asn Ser
                725                 730                 735
Asn Gly Glu Glu Ile Ser Leu Ser Asp Ala Ile Ile Ile Leu Ser Cys
            740                 745                 750
Glu Ser Phe Ser Ser Arg Ser Arg Ala Cys Ser Pro Pro Ile Lys Gln
        755                 760                 765
Lys Ser Asp Glu Phe Glu Glu Glu Lys Gly Gly Gly Gly Gly Glu Glu
    770                 775                 780
Ile Ser Pro Cys Val Ser Leu Asp Leu Asn Ile Cys Ile Asp Asp Asp
785                 790                 795                 800
Gly Val Glu Asp Glu Ser Ile Asp Asp Ile Gly Leu Leu Glu Ser Val
                805                 810                 815
Asp Arg Arg Ile Thr Phe Lys Ile Gln Glu Leu
            820                 825

<210> 67
<211> 2631
<212> DNA
<213> Oryza sativa

<220>
<221> CDS
<222> (1)..(2631)

<400> 67
atg agg gcc ggg ggg tgc acg gtg cag cag gcg ctg acg gcg gag gcg       48
Met Arg Ala Gly Gly Cys Thr Val Gln Gln Ala Leu Thr Ala Glu Ala
1               5                   10                  15
gcc gcc gtg gtg aag cag gcg gtg acc ctg gcg cgg cgg agg ggc aac       96
Ala Ala Val Val Lys Gln Ala Val Thr Leu Ala Arg Arg Arg Gly Asn
            20                  25                  30
gcg cag gtg acg ccg ctg cac gtg gcg agc gcg atg ctg gcg ccg ccg      144
Ala Gln Val Thr Pro Leu His Val Ala Ser Ala Met Leu Ala Pro Pro
        35                  40                  45
ggg ggg ctc ctc cgc gcg gcg tgc ctc cgg tcg cac tcc cac ccg ctg      192
Gly Gly Leu Leu Arg Ala Ala Cys Leu Arg Ser His Ser His Pro Leu
    50                  55                  60
cag tgc aag gcc ctg gag ctc tgc ttc aac gtg gcg ctc aac cgc ctc      240
Gln Cys Lys Ala Leu Glu Leu Cys Phe Asn Val Ala Leu Asn Arg Leu
65                  70                  75                  80
ccg gcc tcc gcc gcc gtc gcc tcg tcg ccg ctg ctc ggc ggc cac ggg      288
Pro Ala Ser Ala Ala Val Ala Ser Ser Pro Leu Leu Gly Gly His Gly
                85                  90                  95
cac ggc cac cac ggc cac tac tac ccg ccg tcg ctc tcc aac gcg ctc      336
His Gly His His Gly His Tyr Tyr Pro Pro Ser Leu Ser Asn Ala Leu
            100                 105                 110
gtc gcg gcg ttc aag cgg gcg cag gcg cac cag cgg cgc ggg tcc gtg      384
Val Ala Ala Phe Lys Arg Ala Gln Ala His Gln Arg Arg Gly Ser Val
        115                 120                 125
gag acc cag cag cag ccg gtg ctc gcc gtg aag atc gag ctg gag cag      432
Glu Thr Gln Gln Gln Pro Val Leu Ala Val Lys Ile Glu Leu Glu Gln
    130                 135                 140
ctc gtc gtc tcc atc ctc gac gac ccc agc gtc agc cgc gtc atg cgg      480
Leu Val Val Ser Ile Leu Asp Asp Pro Ser Val Ser Arg Val Met Arg
145                 150                 155                 160
gag gcc ggc ttc tcc agc acc cag gtc aag gcc aac gtg gag cag gcg      528
Glu Ala Gly Phe Ser Ser Thr Gln Val Lys Ala Asn Val Glu Gln Ala
                165                 170                 175
tgc agc acc acc acg gcc acc agc gcg cca ccg aac caa aac cct aac      576
Cys Ser Thr Thr Thr Ala Thr Ser Ala Pro Pro Asn Gln Asn Pro Asn
```

180 185 190
cct agc tgc acc ggc gcc gca gcc acc gcc acc gcc acc acc agc ccg 624
Pro Ser Cys Thr Gly Ala Ala Ala Thr Ala Thr Ala Thr Thr Ser Pro
195 200 205
gct cat cct ccg gag atc aaa gcc aaa ctg cca ctg ctc gac atg ctg 672
Ala His Pro Pro Glu Ile Lys Ala Lys Leu Pro Leu Leu Asp Met Leu
210 215 220
gcg cgc gac gag gac atc gcc gcg gtg ctc gac tgc ctt gcc ccg gca 720
Ala Arg Asp Glu Asp Ile Ala Ala Val Leu Asp Cys Leu Ala Pro Ala
225 230 235 240
gcg gct ggc ggc cgg ggc ggc ggc ggc agc agg agg agg gtc gtc gtc 768
Ala Ala Gly Gly Arg Gly Gly Gly Gly Ser Arg Arg Arg Val Val Val
245 250 255
gtc gcc gag agc acg gcc gcc gcg gag gcc acg gcg cgc gcc gcc gtg 816
Val Ala Glu Ser Thr Ala Ala Ala Glu Ala Thr Ala Arg Ala Ala Val
260 265 270
gac agg gtc agg aga ggg gag gcg aag cag cac gac gcg ctg cgt ggc 864
Asp Arg Val Arg Arg Gly Glu Ala Lys Gln His Asp Ala Leu Arg Gly
275 280 285
gcg cag gtg gtc aac ctc cgc gtg tcg tcg ttc cgc gac atg ccg agg 912
Ala Gln Val Val Asn Leu Arg Val Ser Ser Phe Arg Asp Met Pro Arg
290 295 300
gag gag gcg gag cgg cgg ctc gcc gag ctc cgg tgc ctc gtg aag agc 960
Glu Glu Ala Glu Arg Arg Leu Ala Glu Leu Arg Cys Leu Val Lys Ser
305 310 315 320
cgc ggc gcg cgg gtg ctc ctc gtc gtc gag gac ctc aag tgg gcg gcc 1008
Arg Gly Ala Arg Val Leu Leu Val Val Glu Asp Leu Lys Trp Ala Ala
325 330 335
gac ttc tgg gcc gcc gcg cac acc ggc gcg agg agg gtc ggc agc ggc 1056
Asp Phe Trp Ala Ala Ala His Thr Gly Ala Arg Arg Val Gly Ser Gly
340 345 350
ggc ggc ggc tac tac tgc tct gtc gag cac gtc gtc acc gag gtg cgc 1104
Gly Gly Gly Tyr Tyr Cys Ser Val Glu His Val Val Thr Glu Val Arg
355 360 365
gcc ctg gcg tcg tgc gac ggc ggc atc tgg ctc gtc ggg ttc ggg acg 1152
Ala Leu Ala Ser Cys Asp Gly Gly Ile Trp Leu Val Gly Phe Gly Thr
370 375 380
tac cag acg tac atg aag tgc agg gcg ggg cat ccg tcg ctg gag agc 1200
Tyr Gln Thr Tyr Met Lys Cys Arg Ala Gly His Pro Ser Leu Glu Ser
385 390 395 400
atg tgg ggg ctc cag acg ctc gcc gtc ccc gcc ggc agc ctc gcg ctg 1248
Met Trp Gly Leu Gln Thr Leu Ala Val Pro Ala Gly Ser Leu Ala Leu
405 410 415
agc ctc acc tgc gcc ttc gac gac agc gcg ctg ggc gca gtc aat cag 1296
Ser Leu Thr Cys Ala Phe Asp Asp Ser Ala Leu Gly Ala Val Asn Gln
420 425 430
tcc atg aaa gcg agc cct cac acc acc gat ggg aac cgc ccg gcg ccg 1344
Ser Met Lys Ala Ser Pro His Thr Thr Asp Gly Asn Arg Pro Ala Pro
435 440 445
tct tgc tgg ccg ctt ttg ggc ggc agc cac ctc ctc tcc aga tgc tgc 1392
Ser Cys Trp Pro Leu Leu Gly Gly Ser His Leu Leu Ser Arg Cys Cys
450 455 460
ggc ggc gac tgc tcc gcc gcg acg acg acg cac gag cac gac acc aag 1440
Gly Gly Asp Cys Ser Ala Ala Thr Thr Thr His Glu His Asp Thr Lys
465 470 475 480
gcg tcg ttg ccc cgc tcc ttc gtg tcg tcg tcg agc ctc cct tct tgg 1488
Ala Ser Leu Pro Arg Ser Phe Val Ser Ser Ser Ser Leu Pro Ser Trp
485 490 495
ctc cag cat tgc cgc gac cag cag ctg cag gag tcg aca cat ttc gcg 1536
Leu Gln His Cys Arg Asp Gln Gln Leu Gln Glu Ser Thr His Phe Ala
500 505 510
gat ctt ggc aag aca tgg gga tcc atc tgc ggc aag ccg tct cag cgg 1584
Asp Leu Gly Lys Thr Trp Gly Ser Ile Cys Gly Lys Pro Ser Gln Arg
515 520 525
atg acg ctg cat ttc tcg gcg ccg gtg tcg ccg gcg tcc tcc atc tcc 1632

```
Met Thr Leu His Phe Ser Ala Pro Val Ser Pro Ala Ser Ser Ile Ser
    530                 535                 540
tcc tac gag cac ggc cac ggc cac cag cag cag cag cac cag cct cac      1680
Ser Tyr Glu His Gly His Gly His Gln Gln Gln Gln His Gln Pro His
545                 550                 555                 560
cac tcg tgg ctt ctc gcc gac ctc gac gcc aag cac cca tgg aag ccc      1728
His Ser Trp Leu Leu Ala Asp Leu Asp Ala Lys His Pro Trp Lys Pro
                565                 570                 575
aag cgc gag gac gac gac gac gag aag gcc aag tcg cac gac gac tgc      1776
Lys Arg Glu Asp Asp Asp Asp Glu Lys Ala Lys Ser His Asp Asp Cys
            580                 585                 590
tcc ggc gcc tcc aat ggc tcg gtg gag gtg gag tgc cgt tcc agg ttc      1824
Ser Gly Ala Ser Asn Gly Ser Val Glu Val Glu Cys Arg Ser Arg Phe
        595                 600                 605
aag gag ctc aac gcc gag aac ctg aag ctc ctg tgc gcc gcg ctg gag      1872
Lys Glu Leu Asn Ala Glu Asn Leu Lys Leu Leu Cys Ala Ala Leu Glu
    610                 615                 620
aag gag gtg ccg tgg cag aag gag atc gtc ccg gag gtg gcg agc gcc      1920
Lys Glu Val Pro Trp Gln Lys Glu Ile Val Pro Glu Val Ala Ser Ala
625                 630                 635                 640
gtc ctc cag tgc agg tcc ggg atc gcc aag cgg cgg gac agg tcg agg      1968
Val Leu Gln Cys Arg Ser Gly Ile Ala Lys Arg Arg Asp Arg Ser Arg
                645                 650                 655
tcg acg gag gcg aag gag gag acg tgg ctc ttc ttc ctc gga ggc gac      2016
Ser Thr Glu Ala Lys Glu Glu Thr Trp Leu Phe Phe Leu Gly Gly Asp
            660                 665                 670
gcg cac ggc aag gag agg gtg gcg agg gag ctc gcc ggc ctc gtc ttc      2064
Ala His Gly Lys Glu Arg Val Ala Arg Glu Leu Ala Gly Leu Val Phe
        675                 680                 685
ggg tca cgc aag agc ttc ctc tcc gtc aag ctc ggc gcc tcc tcc tcc      2112
Gly Ser Arg Lys Ser Phe Leu Ser Val Lys Leu Gly Ala Ser Ser Ser
    690                 695                 700
tcg ccg tcc gcg tcg ggc tcc acc gag gat cac cac cgg agc aag cgg      2160
Ser Pro Ser Ala Ser Gly Ser Thr Glu Asp His His Arg Ser Lys Arg
705                 710                 715                 720
ccg cgg acg acg acg agg tcg tcg gcg agc gag gcc tac ctc gag cgg      2208
Pro Arg Thr Thr Thr Arg Ser Ser Ala Ser Glu Ala Tyr Leu Glu Arg
                725                 730                 735
ctc tac gac gcc gtc tcg gag aac ccg cac cgc gtc atc ctc atc gag      2256
Leu Tyr Asp Ala Val Ser Glu Asn Pro His Arg Val Ile Leu Ile Glu
            740                 745                 750
gac gtc gag cag ggc gac cat cgc tgg cag gtg ggc gtc aag gag gcc      2304
Asp Val Glu Gln Gly Asp His Arg Trp Gln Val Gly Val Lys Glu Ala
        755                 760                 765
atc gac aga ggg gtt ctc cgg agc cag gcc ggc gac gag gtc ggc gtg      2352
Ile Asp Arg Gly Val Leu Arg Ser Gln Ala Gly Asp Glu Val Gly Val
    770                 775                 780
ggc gac gcc atc atc atc ctg agc tgc gag agc ttc gag gcg agg tct      2400
Gly Asp Ala Ile Ile Ile Leu Ser Cys Glu Ser Phe Glu Ala Arg Ser
785                 790                 795                 800
aga gct ggc tcg ccg ctg atg aac aag aag atg aag gtg gag aaa gag      2448
Arg Ala Gly Ser Pro Leu Met Asn Lys Lys Met Lys Val Glu Lys Glu
                805                 810                 815
gaa gcc aac aca agt gat cat gat cac aaa tta gaa atc gaa tca ggc      2496
Glu Ala Asn Thr Ser Asp His Asp His Lys Leu Glu Ile Glu Ser Gly
            820                 825                 830
gtc ccc tct tct tgc ttc gat ttg aac ctt gac atg gag agc gat caa      2544
Val Pro Ser Ser Cys Phe Asp Leu Asn Leu Asp Met Glu Ser Asp Gln
        835                 840                 845
gca gca gat gag ctc agc tcc ggt gat gtc tgt ctg ctc acg gcg gtc      2592
Ala Ala Asp Glu Leu Ser Ser Gly Asp Val Cys Leu Leu Thr Ala Val
    850                 855                 860
gac cgg gtg ctg ctc ttc aga agg cag gat gaa atg taa                  2631
Asp Arg Val Leu Leu Phe Arg Arg Gln Asp Glu Met
865                 870                 875
```

<210> 68
<211> 876
<212> PRT
<213> Oryza sativa

<400> 68
Met Arg Ala Gly Gly Cys Thr Val Gln Gln Ala Leu Thr Ala Glu Ala
1               5                   10                  15
Ala Ala Val Val Lys Gln Ala Val Thr Leu Ala Arg Arg Arg Gly Asn
            20                  25                  30
Ala Gln Val Thr Pro Leu His Val Ala Ser Ala Met Leu Ala Pro Pro
        35                  40                  45
Gly Gly Leu Leu Arg Ala Ala Cys Leu Arg Ser His Ser His Pro Leu
    50                  55                  60
Gln Cys Lys Ala Leu Glu Leu Cys Phe Asn Val Ala Leu Asn Arg Leu
65                  70                  75                  80
Pro Ala Ser Ala Ala Val Ala Ser Ser Pro Leu Leu Gly Gly His Gly
                85                  90                  95
His Gly His His Gly His Tyr Tyr Pro Pro Ser Leu Ser Asn Ala Leu
            100                 105                 110
Val Ala Ala Phe Lys Arg Ala Gln Ala His Gln Arg Arg Gly Ser Val
        115                 120                 125
Glu Thr Gln Gln Gln Pro Val Leu Ala Val Lys Ile Glu Leu Glu Gln
    130                 135                 140
Leu Val Val Ser Ile Leu Asp Asp Pro Ser Val Ser Arg Val Met Arg
145                 150                 155                 160
Glu Ala Gly Phe Ser Ser Thr Gln Val Lys Ala Asn Val Glu Gln Ala
                165                 170                 175
Cys Ser Thr Thr Thr Ala Thr Ser Ala Pro Pro Asn Gln Asn Pro Asn
            180                 185                 190
Pro Ser Cys Thr Gly Ala Ala Ala Thr Ala Thr Ala Thr Thr Ser Pro
        195                 200                 205
Ala His Pro Pro Glu Ile Lys Ala Lys Leu Pro Leu Leu Asp Met Leu
    210                 215                 220
Ala Arg Asp Glu Asp Ile Ala Ala Val Leu Asp Cys Leu Ala Pro Ala
225                 230                 235                 240
Ala Ala Gly Gly Arg Gly Gly Gly Gly Ser Arg Arg Arg Val Val Val
                245                 250                 255
Val Ala Glu Ser Thr Ala Ala Ala Glu Ala Thr Ala Arg Ala Ala Val
            260                 265                 270
Asp Arg Val Arg Arg Gly Glu Ala Lys Gln His Asp Ala Leu Arg Gly
        275                 280                 285
Ala Gln Val Val Asn Leu Arg Val Ser Ser Phe Arg Asp Met Pro Arg
    290                 295                 300
Glu Glu Ala Glu Arg Arg Leu Ala Glu Leu Arg Cys Leu Val Lys Ser
305                 310                 315                 320
Arg Gly Ala Arg Val Leu Leu Val Val Glu Asp Leu Lys Trp Ala Ala
                325                 330                 335
Asp Phe Trp Ala Ala Ala His Thr Gly Ala Arg Arg Val Gly Ser Gly
            340                 345                 350
Gly Gly Gly Tyr Tyr Cys Ser Val Glu His Val Val Thr Glu Val Arg
        355                 360                 365
Ala Leu Ala Ser Cys Asp Gly Gly Ile Trp Leu Val Gly Phe Gly Thr
    370                 375                 380
Tyr Gln Thr Tyr Met Lys Cys Arg Ala Gly His Pro Ser Leu Glu Ser
385                 390                 395                 400
Met Trp Gly Leu Gln Thr Leu Ala Val Pro Ala Gly Ser Leu Ala Leu
                405                 410                 415
Ser Leu Thr Cys Ala Phe Asp Asp Ser Ala Leu Gly Ala Val Asn Gln
            420                 425                 430
Ser Met Lys Ala Ser Pro His Thr Thr Asp Gly Asn Arg Pro Ala Pro
        435                 440                 445
Ser Cys Trp Pro Leu Leu Gly Gly Ser His Leu Leu Ser Arg Cys Cys
    450                 455                 460

```
Gly Gly Asp Cys Ser Ala Ala Thr Thr Thr His Glu His Asp Thr Lys
465                 470                 475                 480
Ala Ser Leu Pro Arg Ser Phe Val Ser Ser Ser Ser Leu Pro Ser Trp
                485                 490                 495
Leu Gln His Cys Arg Asp Gln Gln Leu Gln Glu Ser Thr His Phe Ala
            500                 505                 510
Asp Leu Gly Lys Thr Trp Gly Ser Ile Cys Gly Lys Pro Ser Gln Arg
        515                 520                 525
Met Thr Leu His Phe Ser Ala Pro Val Ser Pro Ala Ser Ser Ile Ser
    530                 535                 540
Ser Tyr Glu His Gly His Gly His Gln Gln Gln Gln His Gln Pro His
545                 550                 555                 560
His Ser Trp Leu Leu Ala Asp Leu Asp Ala Lys His Pro Trp Lys Pro
                565                 570                 575
Lys Arg Glu Asp Asp Asp Asp Glu Lys Ala Lys Ser His Asp Asp Cys
            580                 585                 590
Ser Gly Ala Ser Asn Gly Ser Val Glu Val Glu Cys Arg Ser Arg Phe
        595                 600                 605
Lys Glu Leu Asn Ala Glu Asn Leu Lys Leu Leu Cys Ala Ala Leu Glu
    610                 615                 620
Lys Glu Val Pro Trp Gln Lys Glu Ile Val Pro Glu Val Ala Ser Ala
625                 630                 635                 640
Val Leu Gln Cys Arg Ser Gly Ile Ala Lys Arg Arg Asp Arg Ser Arg
                645                 650                 655
Ser Thr Glu Ala Lys Glu Glu Thr Trp Leu Phe Phe Leu Gly Gly Asp
            660                 665                 670
Ala His Gly Lys Glu Arg Val Ala Arg Glu Leu Ala Gly Leu Val Phe
        675                 680                 685
Gly Ser Arg Lys Ser Phe Leu Ser Val Lys Leu Gly Ala Ser Ser Ser
    690                 695                 700
Ser Pro Ser Ala Ser Gly Ser Thr Glu Asp His His Arg Ser Lys Arg
705                 710                 715                 720
Pro Arg Thr Thr Thr Arg Ser Ser Ala Ser Glu Ala Tyr Leu Glu Arg
                725                 730                 735
Leu Tyr Asp Ala Val Ser Glu Asn Pro His Arg Val Ile Leu Ile Glu
            740                 745                 750
Asp Val Glu Gln Gly Asp His Arg Trp Gln Val Gly Val Lys Glu Ala
        755                 760                 765
Ile Asp Arg Gly Val Leu Arg Ser Gln Ala Gly Asp Glu Val Gly Val
    770                 775                 780
Gly Asp Ala Ile Ile Ile Leu Ser Cys Glu Ser Phe Glu Ala Arg Ser
785                 790                 795                 800
Arg Ala Gly Ser Pro Leu Met Asn Lys Lys Met Lys Val Glu Lys Glu
                805                 810                 815
Glu Ala Asn Thr Ser Asp His Asp His Lys Leu Glu Ile Glu Ser Gly
            820                 825                 830
Val Pro Ser Ser Cys Phe Asp Leu Asn Leu Asp Met Glu Ser Asp Gln
        835                 840                 845
Ala Ala Asp Glu Leu Ser Ser Gly Asp Val Cys Leu Leu Thr Ala Val
    850                 855                 860
Asp Arg Val Leu Leu Phe Arg Arg Gln Asp Glu Met
865                 870                 875
```

<210> 69
<211> 2514
<212> DNA
<213> Arabidopsis thaliana

<220>
<221> CDS
<222> (1)..(2514)

<400> 69

```
atg aga gct gga ggc tgc acg gtg gag caa gcc ctc acg gcg gat gct       48
Met Arg Ala Gly Gly Cys Thr Val Glu Gln Ala Leu Thr Ala Asp Ala
```

1 5 10 15
gca aac gtg gtg aaa caa gcc atg ggg ttg gct aga cgg aga gga cat 96
Ala Asn Val Val Lys Gln Ala Met Gly Leu Ala Arg Arg Arg Gly His
20 25 30
gct cag gtc aca cct ctc cat gta gct agc acc atg ctc tct gct ccc 144
Ala Gln Val Thr Pro Leu His Val Ala Ser Thr Met Leu Ser Ala Pro
35 40 45
acg ggt tta ctt aga aca gct tgt ctc caa tct cac act cac cct ctt 192
Thr Gly Leu Leu Arg Thr Ala Cys Leu Gln Ser His Thr His Pro Leu
50 55 60
caa tgc aga gcc ctc gag ctc tgc ttc aac gta gct ttg aac cgc ctc 240
Gln Cys Arg Ala Leu Glu Leu Cys Phe Asn Val Ala Leu Asn Arg Leu
65 70 75 80
ccg acc tct aca ggg agt cct atg tta ggg gtt cca act tcc cct ttc 288
Pro Thr Ser Thr Gly Ser Pro Met Leu Gly Val Pro Thr Ser Pro Phe
85 90 95
cct tct atc tcc aac gct ctc ggt gca gct ttc aaa cgc gca caa gct 336
Pro Ser Ile Ser Asn Ala Leu Gly Ala Ala Phe Lys Arg Ala Gln Ala
100 105 110
cac caa cga cgt ggt tct ata gag agc cag caa caa ccg atc tta gca 384
His Gln Arg Arg Gly Ser Ile Glu Ser Gln Gln Gln Pro Ile Leu Ala
115 120 125
gtc aag atc gaa gtg gag cag ctc ata ata tct atc ctc gat gat cct 432
Val Lys Ile Glu Val Glu Gln Leu Ile Ile Ser Ile Leu Asp Asp Pro
130 135 140
agc gtg agt aga gtg atg aga gaa gct ggt ttc tcg agt cct caa gtg 480
Ser Val Ser Arg Val Met Arg Glu Ala Gly Phe Ser Ser Pro Gln Val
145 150 155 160
aag act aaa gtc gag caa gct gtt tct tta gag att tgc tct aaa aca 528
Lys Thr Lys Val Glu Gln Ala Val Ser Leu Glu Ile Cys Ser Lys Thr
165 170 175
acc tct tcg agt aaa ccc aaa gag ggg aag tta tta act cct gtc agg 576
Thr Ser Ser Ser Lys Pro Lys Glu Gly Lys Leu Leu Thr Pro Val Arg
180 185 190
aac gag gat gtt atg aac gtt atc aac aat ctt gtc gac aaa aag aga 624
Asn Glu Asp Val Met Asn Val Ile Asn Asn Leu Val Asp Lys Lys Arg
195 200 205
aga aat ttc gtg atc gta gga gag tgt tta gcc act att gac gga gtt 672
Arg Asn Phe Val Ile Val Gly Glu Cys Leu Ala Thr Ile Asp Gly Val
210 215 220
gtg aag acg gtg atg gaa aaa gtc gac aaa aaa gac gtg ccc gag gtt 720
Val Lys Thr Val Met Glu Lys Val Asp Lys Lys Asp Val Pro Glu Val
225 230 235 240
tta aaa gac gta aag ttc ata act tta tcg ttc tca tcg ttc ggg caa 768
Leu Lys Asp Val Lys Phe Ile Thr Leu Ser Phe Ser Ser Phe Gly Gln
245 250 255
cca agt aga gcc gat gtg gaa cgt aag cta gag gag cta gag gcc gat 816
Pro Ser Arg Ala Asp Val Glu Arg Lys Leu Glu Glu Leu Glu Ala Asp
260 265 270
gtg gaa cgt aag cta gag gag cta gag gcc gat gtg gaa cgt aag cta 864
Val Glu Arg Lys Leu Glu Glu Leu Glu Ala Asp Val Glu Arg Lys Leu
275 280 285
gag gag cta gag aca ctc gtg aag agc tgt gta ggg aaa gga gtg att 912
Glu Glu Leu Glu Thr Leu Val Lys Ser Cys Val Gly Lys Gly Val Ile
290 295 300
cta aat ctg gga gat cta aac tgg ttc gta gag tca aga acc aga ggt 960
Leu Asn Leu Gly Asp Leu Asn Trp Phe Val Glu Ser Arg Thr Arg Gly
305 310 315 320
tct tca ctg tat aac aac aac gat agc tac tgt gtt gtg gag cat atg 1008
Ser Ser Leu Tyr Asn Asn Asn Asp Ser Tyr Cys Val Val Glu His Met
325 330 335
ata atg gag att ggg aag ttg gct tgt ggg tta gtc atg gga gat cat 1056
Ile Met Glu Ile Gly Lys Leu Ala Cys Gly Leu Val Met Gly Asp His
340 345 350
gga agg ttt tgg tta atg ggt ctt gcg act tca cag act tac gtg aga 1104

```
Gly Arg Phe Trp Leu Met Gly Leu Ala Thr Ser Gln Thr Tyr Val Arg
        355                 360                 365
tgc aag tct ggt caa ccc tcg ctt gaa tct ctc tgg tgt ctc act act      1152
Cys Lys Ser Gly Gln Pro Ser Leu Glu Ser Leu Trp Cys Leu Thr Thr
    370                 375                 380
ctt act att ccg gcg act agt aat agt ctc cgg ttg agt ctc gtc tcc      1200
Leu Thr Ile Pro Ala Thr Ser Asn Ser Leu Arg Leu Ser Leu Val Ser
385                 390                 395                 400
gag agt gag ctt gaa gtc aag aaa tca gag aac gtg tct ctc cag ctg      1248
Glu Ser Glu Leu Glu Val Lys Lys Ser Glu Asn Val Ser Leu Gln Leu
                405                 410                 415
cag caa tca tcg gat cag ctc agt ttc tgt gag gaa tgt tcc gtc aag      1296
Gln Gln Ser Ser Asp Gln Leu Ser Phe Cys Glu Glu Cys Ser Val Lys
            420                 425                 430
ttt gaa tcg gaa gct cga ttc tta aag agt agc aat agc aat gta acc      1344
Phe Glu Ser Glu Ala Arg Phe Leu Lys Ser Ser Asn Ser Asn Val Thr
        435                 440                 445
act gta gca tta ccg gca tgg ctt cag cag tat aag aag gag aat caa      1392
Thr Val Ala Leu Pro Ala Trp Leu Gln Gln Tyr Lys Lys Glu Asn Gln
    450                 455                 460
aat agt cac act gat tca gat tct att aag gaa ctt gtg gtg aag tgg      1440
Asn Ser His Thr Asp Ser Asp Ser Ile Lys Glu Leu Val Val Lys Trp
465                 470                 475                 480
aac tca atc tgt gat tca atc cac aaa aga cca tct cta aaa aca ctc      1488
Asn Ser Ile Cys Asp Ser Ile His Lys Arg Pro Ser Leu Lys Thr Leu
                485                 490                 495
aca ctc tct tct cct act tct tcc ttt tct ggt tcc act caa cct tca      1536
Thr Leu Ser Ser Pro Thr Ser Ser Phe Ser Gly Ser Thr Gln Pro Ser
            500                 505                 510
atc agt act ctt cat cat ctt caa acc aac ggt gat tgg ccc gtg atc      1584
Ile Ser Thr Leu His His Leu Gln Thr Asn Gly Asp Trp Pro Val Ile
        515                 520                 525
gag aca aac acg cat cgt cat cat tct gtt gtc cat gag aca tct cac      1632
Glu Thr Asn Thr His Arg His His Ser Val Val His Glu Thr Ser His
    530                 535                 540
tta aga ctg ttc att cca gaa cat gac agc gag caa aag act gag cta      1680
Leu Arg Leu Phe Ile Pro Glu His Asp Ser Glu Gln Lys Thr Glu Leu
545                 550                 555                 560
gtc tgt tcg aat cct aat tct aca atg aac tct gaa gcc tct tca agc      1728
Val Cys Ser Asn Pro Asn Ser Thr Met Asn Ser Glu Ala Ser Ser Ser
                565                 570                 575
gat gca atg gag ttg gaa cat gcc tct tca agg ttt aaa gag atg aat      1776
Asp Ala Met Glu Leu Glu His Ala Ser Ser Arg Phe Lys Glu Met Asn
            580                 585                 590
gca gaa aac cta gca act tta tgt gct gcc ttg gaa agc aag gta ccg      1824
Ala Glu Asn Leu Ala Thr Leu Cys Ala Ala Leu Glu Ser Lys Val Pro
        595                 600                 605
tgg caa aag gat tta gtc ccg gag tta gcc aaa aca gtc ttg aaa tgc      1872
Trp Gln Lys Asp Leu Val Pro Glu Leu Ala Lys Thr Val Leu Lys Cys
    610                 615                 620
agg tca gga tcg agt acg agg aag att aac gga aac gag gat aaa aaa      1920
Arg Ser Gly Ser Ser Thr Arg Lys Ile Asn Gly Asn Glu Asp Lys Lys
625                 630                 635                 640
gaa gat aca tgg atg ttc ttc caa ggt ctt gat gta gat gct aaa gag      1968
Glu Asp Thr Trp Met Phe Phe Gln Gly Leu Asp Val Asp Ala Lys Glu
                645                 650                 655
aag atc gct aga gaa ttg gcc aaa ctc gtg ttt gga tcg caa gac agc      2016
Lys Ile Ala Arg Glu Leu Ala Lys Leu Val Phe Gly Ser Gln Asp Ser
            660                 665                 670
ttt gtc tct atc tgt ttg agt agt ttc tcg tcg aca aga tcg gat tct      2064
Phe Val Ser Ile Cys Leu Ser Ser Phe Ser Ser Thr Arg Ser Asp Ser
        675                 680                 685
gca gaa gat ttg agg aac aag agg ttg aga gat gaa caa agc ctg agt      2112
Ala Glu Asp Leu Arg Asn Lys Arg Leu Arg Asp Glu Gln Ser Leu Ser
    690                 695                 700
```

```
tac ata gag aga ttc tcg gaa gct gtt tcg ttg gat cca aat aga gtg        2160
Tyr Ile Glu Arg Phe Ser Glu Ala Val Ser Leu Asp Pro Asn Arg Val
705                 710                 715                 720
atc tta gtt gaa gat att gag caa gct gat tat ttg tct caa gtg ggt        2208
Ile Leu Val Glu Asp Ile Glu Gln Ala Asp Tyr Leu Ser Gln Val Gly
                725                 730                 735
ttc aag aga gct gtt gag aga gga aga gtt tgt aat tcg agt ggt gaa        2256
Phe Lys Arg Ala Val Glu Arg Gly Arg Val Cys Asn Ser Ser Gly Glu
            740                 745                 750
gaa gct tcg ctt aaa gac gcg att gtg atc ttg agc tgt gaa aga ttt        2304
Glu Ala Ser Leu Lys Asp Ala Ile Val Ile Leu Ser Cys Glu Arg Phe
        755                 760                 765
cgt tca aga tca aga gct tgt tct cct cct agt aac cag aaa tcg gac        2352
Arg Ser Arg Ser Arg Ala Cys Ser Pro Pro Ser Asn Gln Lys Ser Asp
    770                 775                 780
ggt tca gat caa cct gag gac aag aat gtt gct act tgt gtt gca ctt        2400
Gly Ser Asp Gln Pro Glu Asp Lys Asn Val Ala Thr Cys Val Ala Leu
785                 790                 795                 800
gat cta aac cta tct att gat agt gcg tac gtt tgt gaa gaa gaa tca        2448
Asp Leu Asn Leu Ser Ile Asp Ser Ala Tyr Val Cys Glu Glu Glu Ser
                805                 810                 815
tgt gat gag att ggc tta ctc gaa gcg gta gat gca cgg ttt cat ttc        2496
Cys Asp Glu Ile Gly Leu Leu Glu Ala Val Asp Ala Arg Phe His Phe
            820                 825                 830
aag tgt tca tca aca taa                                                2514
Lys Cys Ser Ser Thr
        835

<210> 70
<211> 837
<212> PRT
<213> Arabidopsis thaliana

<400> 70
Met Arg Ala Gly Gly Cys Thr Val Glu Gln Ala Leu Thr Ala Asp Ala
1               5                   10                  15
Ala Asn Val Val Lys Gln Ala Met Gly Leu Ala Arg Arg Arg Gly His
            20                  25                  30
Ala Gln Val Thr Pro Leu His Val Ala Ser Thr Met Leu Ser Ala Pro
        35                  40                  45
Thr Gly Leu Leu Arg Thr Ala Cys Leu Gln Ser His Thr His Pro Leu
    50                  55                  60
Gln Cys Arg Ala Leu Glu Leu Cys Phe Asn Val Ala Leu Asn Arg Leu
65                  70                  75                  80
Pro Thr Ser Thr Gly Ser Pro Met Leu Gly Val Pro Thr Ser Pro Phe
                85                  90                  95
Pro Ser Ile Ser Asn Ala Leu Gly Ala Ala Phe Lys Arg Ala Gln Ala
            100                 105                 110
His Gln Arg Arg Gly Ser Ile Glu Ser Gln Gln Gln Pro Ile Leu Ala
        115                 120                 125
Val Lys Ile Glu Val Glu Gln Leu Ile Ile Ser Ile Leu Asp Asp Pro
    130                 135                 140
Ser Val Ser Arg Val Met Arg Glu Ala Gly Phe Ser Ser Pro Gln Val
145                 150                 155                 160
Lys Thr Lys Val Glu Gln Ala Val Ser Leu Glu Ile Cys Ser Lys Thr
                165                 170                 175
Thr Ser Ser Ser Lys Pro Lys Glu Gly Lys Leu Leu Thr Pro Val Arg
            180                 185                 190
Asn Glu Asp Val Met Asn Val Ile Asn Asn Leu Val Asp Lys Lys Arg
        195                 200                 205
Arg Asn Phe Val Ile Val Gly Glu Cys Leu Ala Thr Ile Asp Gly Val
    210                 215                 220
Val Lys Thr Val Met Glu Lys Val Asp Lys Lys Asp Val Pro Glu Val
225                 230                 235                 240
Leu Lys Asp Val Lys Phe Ile Thr Leu Ser Phe Ser Ser Phe Gly Gln
```

```
                245                 250                 255
Pro Ser Arg Ala Asp Val Glu Arg Lys Leu Glu Glu Leu Glu Ala Asp
            260                 265                 270
Val Glu Arg Lys Leu Glu Glu Leu Glu Ala Asp Val Glu Arg Lys Leu
        275                 280                 285
Glu Glu Leu Glu Thr Leu Val Lys Ser Cys Val Gly Lys Gly Val Ile
    290                 295                 300
Leu Asn Leu Gly Asp Leu Asn Trp Phe Val Glu Ser Arg Thr Arg Gly
305                 310                 315                 320
Ser Ser Leu Tyr Asn Asn Asn Asp Ser Tyr Cys Val Val Glu His Met
                325                 330                 335
Ile Met Glu Ile Gly Lys Leu Ala Cys Gly Leu Val Met Gly Asp His
            340                 345                 350
Gly Arg Phe Trp Leu Met Gly Leu Ala Thr Ser Gln Thr Tyr Val Arg
        355                 360                 365
Cys Lys Ser Gly Gln Pro Ser Leu Glu Ser Leu Trp Cys Leu Thr Thr
    370                 375                 380
Leu Thr Ile Pro Ala Thr Ser Asn Ser Leu Arg Leu Ser Leu Val Ser
385                 390                 395                 400
Glu Ser Glu Leu Glu Val Lys Lys Ser Glu Asn Val Ser Leu Gln Leu
                405                 410                 415
Gln Gln Ser Ser Asp Gln Leu Ser Phe Cys Glu Glu Cys Ser Val Lys
            420                 425                 430
Phe Glu Ser Glu Ala Arg Phe Leu Lys Ser Ser Asn Ser Asn Val Thr
        435                 440                 445
Thr Val Ala Leu Pro Ala Trp Leu Gln Gln Tyr Lys Lys Glu Asn Gln
    450                 455                 460
Asn Ser His Thr Asp Ser Asp Ser Ile Lys Glu Leu Val Val Lys Trp
465                 470                 475                 480
Asn Ser Ile Cys Asp Ser Ile His Lys Arg Pro Ser Leu Lys Thr Leu
                485                 490                 495
Thr Leu Ser Ser Pro Thr Ser Ser Phe Ser Gly Ser Thr Gln Pro Ser
            500                 505                 510
Ile Ser Thr Leu His His Leu Gln Thr Asn Gly Asp Trp Pro Val Ile
        515                 520                 525
Glu Thr Asn Thr His Arg His His Ser Val Val His Glu Thr Ser His
    530                 535                 540
Leu Arg Leu Phe Ile Pro Glu His Asp Ser Glu Gln Lys Thr Glu Leu
545                 550                 555                 560
Val Cys Ser Asn Pro Asn Ser Thr Met Asn Ser Glu Ala Ser Ser Ser
                565                 570                 575
Asp Ala Met Glu Leu Glu His Ala Ser Ser Arg Phe Lys Glu Met Asn
            580                 585                 590
Ala Glu Asn Leu Ala Thr Leu Cys Ala Ala Leu Glu Ser Lys Val Pro
        595                 600                 605
Trp Gln Lys Asp Leu Val Pro Glu Leu Ala Lys Thr Val Leu Lys Cys
    610                 615                 620
Arg Ser Gly Ser Ser Thr Arg Lys Ile Asn Gly Asn Glu Asp Lys Lys
625                 630                 635                 640
Glu Asp Thr Trp Met Phe Phe Gln Gly Leu Asp Val Asp Ala Lys Glu
                645                 650                 655
Lys Ile Ala Arg Glu Leu Ala Lys Leu Val Phe Gly Ser Gln Asp Ser
            660                 665                 670
Phe Val Ser Ile Cys Leu Ser Ser Phe Ser Ser Thr Arg Ser Asp Ser
        675                 680                 685
Ala Glu Asp Leu Arg Asn Lys Arg Leu Arg Asp Glu Gln Ser Leu Ser
    690                 695                 700
Tyr Ile Glu Arg Phe Ser Glu Ala Val Ser Leu Asp Pro Asn Arg Val
705                 710                 715                 720
Ile Leu Val Glu Asp Ile Glu Gln Ala Asp Tyr Leu Ser Gln Val Gly
                725                 730                 735
Phe Lys Arg Ala Val Glu Arg Gly Arg Val Cys Asn Ser Ser Gly Glu
            740                 745                 750
Glu Ala Ser Leu Lys Asp Ala Ile Val Ile Leu Ser Cys Glu Arg Phe
        755                 760                 765
```

```
Arg Ser Arg Ser Arg Ala Cys Ser Pro Pro Ser Asn Gln Lys Ser Asp
    770                 775                 780
Gly Ser Asp Gln Pro Glu Asp Lys Asn Val Ala Thr Cys Val Ala Leu
785                 790                 795                 800
Asp Leu Asn Leu Ser Ile Asp Ser Ala Tyr Val Cys Glu Glu Glu Ser
                805                 810                 815
Cys Asp Glu Ile Gly Leu Leu Glu Ala Val Asp Ala Arg Phe His Phe
            820                 825                 830
Lys Cys Ser Ser Thr
        835

<210> 71
<211> 2754
<212> DNA
<213> Oryza sativa subsp. japonica

<220>
<221> CDS
<222> (1)..(2754)

<400> 71
atg aga gct ggg ggg tgc acg gtg cag cag gcg ctg acg gcg gag gcg      48
Met Arg Ala Gly Gly Cys Thr Val Gln Gln Ala Leu Thr Ala Glu Ala
1               5                   10                  15
gcg gct gtg gtg aag cag gcg gtg agc ctg gcg cgg cgg cgc ggg aac      96
Ala Ala Val Val Lys Gln Ala Val Ser Leu Ala Arg Arg Arg Gly Asn
            20                  25                  30
gcg cag gtg acg ccg ctg cac gtg gcg agc gcg atg ctg cag gcg gcg      144
Ala Gln Val Thr Pro Leu His Val Ala Ser Ala Met Leu Gln Ala Ala
        35                  40                  45
ggg gcg gcg ccg gcg ccg ggg ctc ctc cgc gcg gcg tgc ctc cgg tcg      192
Gly Ala Ala Pro Ala Pro Gly Leu Leu Arg Ala Ala Cys Leu Arg Ser
    50                  55                  60
cac tcc cac ccg ctg cag tgc aag gcc ctc gag ctc tgc ttc aac gtc      240
His Ser His Pro Leu Gln Cys Lys Ala Leu Glu Leu Cys Phe Asn Val
65                  70                  75                  80
gcc ctc aac cgc ctc ccg gcg tcg gcg ggg gcg tcg ccg ctg ctc ggc      288
Ala Leu Asn Arg Leu Pro Ala Ser Ala Gly Ala Ser Pro Leu Leu Gly
                85                  90                  95
cat ggc cac ggc gtc ggc gtc tac tac ccg ccg tcg ctg tcg aac gcg      336
His Gly His Gly Val Gly Val Tyr Tyr Pro Pro Ser Leu Ser Asn Ala
            100                 105                 110
ctc gtc gcc gcg ttc aag cgc gcc cag gcg cac cag cgg cgg ggc tcc      384
Leu Val Ala Ala Phe Lys Arg Ala Gln Ala His Gln Arg Arg Gly Ser
        115                 120                 125
gtc gag acg cag cag cag ccg gtg ctc gcc gtc aag atc gag ctc gag      432
Val Glu Thr Gln Gln Gln Pro Val Leu Ala Val Lys Ile Glu Leu Glu
    130                 135                 140
cag ctc gtc atc tcc atc ctc gac gac ccc agc gtc agc cgc gtc atg      480
Gln Leu Val Ile Ser Ile Leu Asp Asp Pro Ser Val Ser Arg Val Met
145                 150                 155                 160
cgc gag gcc ggc ttc tcc agc acc cag gtc aag gcc aac gtc gag caa      528
Arg Glu Ala Gly Phe Ser Ser Thr Gln Val Lys Ala Asn Val Glu Gln
                165                 170                 175
gcc gtc tct tcc tcc atg gaa gcc gcc acc acc aag ccc caa aac cct      576
Ala Val Ser Ser Ser Met Glu Ala Ala Thr Thr Lys Pro Gln Asn Pro
            180                 185                 190
aac cct agc agc agc tcg cct ccg ccg gcg gct cat caa gag gcg aaa      624
Asn Pro Ser Ser Ser Ser Pro Pro Pro Ala Ala His Gln Glu Ala Lys
        195                 200                 205
cct agt aga tgc atc gat cag gtg gtg gtg cgg gag gag gac gtc gcg      672
Pro Ser Arg Cys Ile Asp Gln Val Val Val Arg Glu Glu Asp Val Ala
    210                 215                 220
gcc atc ctg gac tgc ctg gcg acg cgg agc aag aag cgg gtc atg gtg      720
Ala Ile Leu Asp Cys Leu Ala Thr Arg Ser Lys Lys Arg Val Met Val
```

```
225                 230                 235                 240
gtc gcc gag tgc gcc gcc gcg gcc gag gcg gcg gcg cgg gcg gcc gtg      768
Val Ala Glu Cys Ala Ala Ala Ala Glu Ala Ala Ala Arg Ala Ala Val
                245                 250                 255
gac agg atc agg cgc ggc gag gcg agg cag cac gcg cag gcg cag gtg      816
Asp Arg Ile Arg Arg Gly Glu Ala Arg Gln His Ala Gln Ala Gln Val
            260                 265                 270
gtc acc ctc gcc gtg tcc cgg ttc cgc ggc gcg ccg agg gag gag gcg      864
Val Thr Leu Ala Val Ser Arg Phe Arg Gly Ala Pro Arg Glu Glu Ala
        275                 280                 285
gag cgg cgg ctg gcg gag ctc cgg tgc gcc gtc agg ggc ggc ggc ggc      912
Glu Arg Arg Leu Ala Glu Leu Arg Cys Ala Val Arg Gly Gly Gly Gly
    290                 295                 300
cgc gcg gtg gtg ctc gtc gtg gag gac ctc gcg tgg gcg gcc gag ttc      960
Arg Ala Val Val Leu Val Val Glu Asp Leu Ala Trp Ala Ala Glu Phe
305                 310                 315                 320
tgg gcc ggg agg agg ccg ccg cca tcc tcc tgc ggc gca ggc gcc ggc      1008
Trp Ala Gly Arg Arg Pro Pro Pro Ser Ser Cys Gly Ala Gly Ala Gly
                325                 330                 335
ggc tac tac tac tgc gcc gtg gag cac gcc gtc gcc gag gtg cgc gcc      1056
Gly Tyr Tyr Tyr Cys Ala Val Glu His Ala Val Ala Glu Val Arg Ala
            340                 345                 350
ctg gcg tgc ggc ggc ggc ggc ggc ggc ggc ggc gtt tgg ctc gtc ggg      1104
Leu Ala Cys Gly Gly Gly Gly Gly Gly Gly Gly Val Trp Leu Val Gly
        355                 360                 365
cac ggc acg tac cag acg aac atc cgg tgc cgg acg ggg cac ccc tcg      1152
His Gly Thr Tyr Gln Thr Asn Ile Arg Cys Arg Thr Gly His Pro Ser
    370                 375                 380
ctg gag act ctc tgg ggc ctc cac acg ctc gcc gtc ccc gcc ggc agc      1200
Leu Glu Thr Leu Trp Gly Leu His Thr Leu Ala Val Pro Ala Gly Ser
385                 390                 395                 400
ctc gcc ctc agc ctc acc tgc gcc gac gcc gac gcc gcc gcc gac gac      1248
Leu Ala Leu Ser Leu Thr Cys Ala Asp Ala Asp Ala Ala Ala Asp Asp
                405                 410                 415
gac gac agt ggt gca atg gca gca gca gca gtc aat cac cag tca gcc      1296
Asp Asp Ser Gly Ala Met Ala Ala Ala Ala Val Asn His Gln Ser Ala
            420                 425                 430
aaa ggc gcg aac gga tcg acg tcg ccg tcg cct tgc ctg tca ctt ttg      1344
Lys Gly Ala Asn Gly Ser Thr Ser Pro Ser Pro Cys Leu Ser Leu Leu
        435                 440                 445
gac gct gct gca gca ggt ggc gcc tgc agc tcc ggg cag ctg gcg gtg      1392
Asp Ala Ala Ala Ala Gly Gly Ala Cys Ser Ser Gly Gln Leu Ala Val
    450                 455                 460
atg gcc gcc gcc gtc tcc ggc gcc tac tgc ggc ggc gat tgc gcc gcc      1440
Met Ala Ala Ala Val Ser Gly Ala Tyr Cys Gly Gly Asp Cys Ala Ala
465                 470                 475                 480
gcg acg aaa gca ctg ctg cca cag tca gtc gtc ttc atg ccg ccg tcg      1488
Ala Thr Lys Ala Leu Leu Pro Gln Ser Val Val Phe Met Pro Pro Ser
                485                 490                 495
gcg acg acg acc acc acc acc atc cct cca tgg ctg cac cat tgc cgc      1536
Ala Thr Thr Thr Thr Thr Thr Ile Pro Pro Trp Leu His His Cys Arg
            500                 505                 510
gat cag gag cct gca gct cat atg aag aaa tgg atg tcg gcg cat ggc      1584
Asp Gln Glu Pro Ala Ala His Met Lys Lys Trp Met Ser Ala His Gly
        515                 520                 525
ggc tcg ccg tcg cgc agg acg gcg ctg aac atc tcg tcc acg gcg gtg      1632
Gly Ser Pro Ser Arg Arg Thr Ala Leu Asn Ile Ser Ser Thr Ala Val
    530                 535                 540
tcg ccg tgc tcc tcc gtc tcg tcc tac gag cag tac acc cgg ttg cac      1680
Ser Pro Cys Ser Ser Val Ser Ser Tyr Glu Gln Tyr Thr Arg Leu His
545                 550                 555                 560
cag ccg tac cag cca tgg ctc gtc gcc gac gac gac gac gaa gcc gaa      1728
Gln Pro Tyr Gln Pro Trp Leu Val Ala Asp Asp Asp Asp Glu Ala Glu
                565                 570                 575
gag acg aag cat cca tac atc gcc ggc gat ggc ggc gct ggt agg ctt      1776
```

```
Glu Thr Lys His Pro Tyr Ile Ala Gly Asp Gly Gly Ala Gly Arg Leu
            580                 585                 590
gtg ccg gcg gcg gcc aag gtg gtg atc aag tcc gac gac tcg agc gcc      1824
Val Pro Ala Ala Ala Lys Val Val Ile Lys Ser Asp Asp Ser Ser Ala
        595                 600                 605
tcc aat ggc tcc gtg gag gtg gag tgg cgg cgg ccc agg ttc aag gag      1872
Ser Asn Gly Ser Val Glu Val Glu Trp Arg Arg Pro Arg Phe Lys Glu
    610                 615                 620
gtg agc gcc gag aac ctc aag gtg ctc tgc ggc gcg ctg gag aag gag      1920
Val Ser Ala Glu Asn Leu Lys Val Leu Cys Gly Ala Leu Glu Lys Glu
625                 630                 635                 640
gtg ccg tgg cag aag gtg atc gtg ccg gag atc gcc agc acg gtg ctc      1968
Val Pro Trp Gln Lys Val Ile Val Pro Glu Ile Ala Ser Thr Val Leu
                645                 650                 655
cgg tgc cgg tcg ggc atg gcg gcg ccc gcc atg gcg agg agg tcg agc      2016
Arg Cys Arg Ser Gly Met Ala Ala Pro Ala Met Ala Arg Arg Ser Ser
            660                 665                 670
tct tgc tcg agc tcc aag gag cac acg tgg atg ctc ttc ctc ggc ggc      2064
Ser Cys Ser Ser Ser Lys Glu His Thr Trp Met Leu Phe Leu Gly Gly
        675                 680                 685
gac gcg gat ggc aag ttg agg gtg gcg agg gag ctg gcg agc ctc gtc      2112
Asp Ala Asp Gly Lys Leu Arg Val Ala Arg Glu Leu Ala Ser Leu Val
    690                 695                 700
ttc ggc tcg tcc aag agc ttc gtg tcc atc ggc ggc gcc gcc aac gcg      2160
Phe Gly Ser Ser Lys Ser Phe Val Ser Ile Gly Gly Ala Ala Asn Ala
705                 710                 715                 720
tcg ccg ccg ccg tcg tca tcg tcg tcg tct ccg gcg cgg tcg tcg ggc      2208
Ser Pro Pro Pro Ser Ser Ser Ser Ser Ser Pro Ala Arg Ser Ser Gly
                725                 730                 735
tcc acc gag cag ccg cac cgg agc aag cgg cca tgg gcg gag acg acg      2256
Ser Thr Glu Gln Pro His Arg Ser Lys Arg Pro Trp Ala Glu Thr Thr
            740                 745                 750
acg acg acg acg agc ggg cgg gac cag gac cac ctc gag gcg ctc tac      2304
Thr Thr Thr Thr Ser Gly Arg Asp Gln Asp His Leu Glu Ala Leu Tyr
        755                 760                 765
gac gcc gtg cgc gac aac ccg cgg cgc gtc att ctg atg gag cgc gtc      2352
Asp Ala Val Arg Asp Asn Pro Arg Arg Val Ile Leu Met Glu Arg Val
    770                 775                 780
gac cgg gcc gac gcg cgg tgc cac gac ggc atc agg gac gcc atc gag      2400
Asp Arg Ala Asp Ala Arg Cys His Asp Gly Ile Arg Asp Ala Ile Glu
785                 790                 795                 800
cgc ggc gtg gtg cgg agc cgc ggc ggc ggc ggc gag gag gcc ttc ctc      2448
Arg Gly Val Val Arg Ser Arg Gly Gly Gly Gly Glu Glu Ala Phe Leu
                805                 810                 815
ggc gac gcc atc gtc gtc ctc agc tgc gag agc ctc aac cca agt tcg      2496
Gly Asp Ala Ile Val Val Leu Ser Cys Glu Ser Leu Asn Pro Ser Ser
            820                 825                 830
acg acg ccg gcg aag aag gcc aag acg gag tac agc gtg gag aag ctc      2544
Thr Thr Pro Ala Lys Lys Ala Lys Thr Glu Tyr Ser Val Glu Lys Leu
        835                 840                 845
gac cag gat ggt gat gat cac cat ggc aag gaa gcc gtc gcg gcg gcg      2592
Asp Gln Asp Gly Asp Asp His His Gly Lys Glu Ala Val Ala Ala Ala
    850                 855                 860
gcg tcg ccg tct tgc ttt gat ctg aac atg agc atg gac gac gac gac      2640
Ala Ser Pro Ser Cys Phe Asp Leu Asn Met Ser Met Asp Asp Asp Asp
865                 870                 875                 880
gag gcg gcg gag gag cgt tgc acc ggc gaa gaa gaa gaa gca ggc cat      2688
Glu Ala Ala Glu Glu Arg Cys Thr Gly Glu Glu Glu Glu Ala Gly His
                885                 890                 895
cat cat cat cag ctg ctg ctc aag gca gta gac agg gtg ctg ttc ttc      2736
His His His Gln Leu Leu Leu Lys Ala Val Asp Arg Val Leu Phe Phe
            900                 905                 910
aga tca att ggg gaa taa                                              2754
Arg Ser Ile Gly Glu
        915
```

<210> 72
<211> 917
<212> PRT
<213> Oryza sativa subsp. japonica

<400> 72
Met Arg Ala Gly Gly Cys Thr Val Gln Gln Ala Leu Thr Ala Glu Ala
1               5                   10                  15
Ala Ala Val Val Lys Gln Ala Val Ser Leu Ala Arg Arg Arg Gly Asn
            20                  25                  30
Ala Gln Val Thr Pro Leu His Val Ala Ser Ala Met Leu Gln Ala Ala
        35                  40                  45
Gly Ala Ala Pro Ala Pro Gly Leu Leu Arg Ala Ala Cys Leu Arg Ser
    50                  55                  60
His Ser His Pro Leu Gln Cys Lys Ala Leu Glu Leu Cys Phe Asn Val
65                  70                  75                  80
Ala Leu Asn Arg Leu Pro Ala Ser Ala Gly Ala Ser Pro Leu Leu Gly
                85                  90                  95
His Gly His Gly Val Gly Val Tyr Tyr Pro Pro Ser Leu Ser Asn Ala
            100                 105                 110
Leu Val Ala Ala Phe Lys Arg Ala Gln Ala His Gln Arg Arg Gly Ser
        115                 120                 125
Val Glu Thr Gln Gln Gln Pro Val Leu Ala Val Lys Ile Glu Leu Glu
    130                 135                 140
Gln Leu Val Ile Ser Ile Leu Asp Asp Pro Ser Val Ser Arg Val Met
145                 150                 155                 160
Arg Glu Ala Gly Phe Ser Ser Thr Gln Val Lys Ala Asn Val Glu Gln
                165                 170                 175
Ala Val Ser Ser Ser Met Glu Ala Ala Thr Thr Lys Pro Gln Asn Pro
            180                 185                 190
Asn Pro Ser Ser Ser Ser Pro Pro Pro Ala Ala His Gln Glu Ala Lys
        195                 200                 205
Pro Ser Arg Cys Ile Asp Gln Val Val Val Arg Glu Glu Asp Val Ala
    210                 215                 220
Ala Ile Leu Asp Cys Leu Ala Thr Arg Ser Lys Lys Arg Val Met Val
225                 230                 235                 240
Val Ala Glu Cys Ala Ala Ala Ala Glu Ala Ala Ala Arg Ala Ala Val
                245                 250                 255
Asp Arg Ile Arg Arg Gly Glu Ala Arg Gln His Ala Gln Ala Gln Val
            260                 265                 270
Val Thr Leu Ala Val Ser Arg Phe Arg Gly Ala Pro Arg Glu Glu Ala
        275                 280                 285
Glu Arg Arg Leu Ala Glu Leu Arg Cys Ala Val Arg Gly Gly Gly Gly
    290                 295                 300
Arg Ala Val Val Leu Val Val Glu Asp Leu Ala Trp Ala Ala Glu Phe
305                 310                 315                 320
Trp Ala Gly Arg Arg Pro Pro Pro Ser Ser Cys Gly Ala Gly Ala Gly
                325                 330                 335
Gly Tyr Tyr Tyr Cys Ala Val Glu His Ala Val Ala Glu Val Arg Ala
            340                 345                 350
Leu Ala Cys Gly Gly Gly Gly Gly Gly Gly Gly Val Trp Leu Val Gly
        355                 360                 365
His Gly Thr Tyr Gln Thr Asn Ile Arg Cys Arg Thr Gly His Pro Ser
    370                 375                 380
Leu Glu Thr Leu Trp Gly Leu His Thr Leu Ala Val Pro Ala Gly Ser
385                 390                 395                 400
Leu Ala Leu Ser Leu Thr Cys Ala Asp Ala Asp Ala Ala Ala Asp Asp
                405                 410                 415
Asp Asp Ser Gly Ala Met Ala Ala Ala Ala Val Asn His Gln Ser Ala
            420                 425                 430
Lys Gly Ala Asn Gly Ser Thr Ser Pro Ser Pro Cys Leu Ser Leu Leu
        435                 440                 445
Asp Ala Ala Ala Ala Gly Gly Ala Cys Ser Ser Gly Gln Leu Ala Val
    450                 455                 460

Met Ala Ala Ala Val Ser Gly Ala Tyr Cys Gly Gly Asp Cys Ala Ala
465 470 475 480
Ala Thr Lys Ala Leu Leu Pro Gln Ser Val Val Phe Met Pro Pro Ser
485 490 495
Ala Thr Thr Thr Thr Thr Thr Ile Pro Pro Trp Leu His His Cys Arg
500 505 510
Asp Gln Glu Pro Ala Ala His Met Lys Lys Trp Met Ser Ala His Gly
515 520 525
Gly Ser Pro Ser Arg Arg Thr Ala Leu Asn Ile Ser Ser Thr Ala Val
530 535 540
Ser Pro Cys Ser Ser Val Ser Ser Tyr Glu Gln Tyr Thr Arg Leu His
545 550 555 560
Gln Pro Tyr Gln Pro Trp Leu Val Ala Asp Asp Asp Asp Glu Ala Glu
565 570 575
Glu Thr Lys His Pro Tyr Ile Ala Gly Asp Gly Gly Ala Gly Arg Leu
580 585 590
Val Pro Ala Ala Ala Lys Val Val Ile Lys Ser Asp Asp Ser Ser Ala
595 600 605
Ser Asn Gly Ser Val Glu Val Glu Trp Arg Arg Pro Arg Phe Lys Glu
610 615 620
Val Ser Ala Glu Asn Leu Lys Val Leu Cys Gly Ala Leu Glu Lys Glu
625 630 635 640
Val Pro Trp Gln Lys Val Ile Val Pro Glu Ile Ala Ser Thr Val Leu
645 650 655
Arg Cys Arg Ser Gly Met Ala Ala Pro Ala Met Ala Arg Arg Ser Ser
660 665 670
Ser Cys Ser Ser Ser Lys Glu His Thr Trp Met Leu Phe Leu Gly Gly
675 680 685
Asp Ala Asp Gly Lys Leu Arg Val Ala Arg Glu Leu Ala Ser Leu Val
690 695 700
Phe Gly Ser Ser Lys Ser Phe Val Ser Ile Gly Gly Ala Ala Asn Ala
705 710 715 720
Ser Pro Pro Pro Ser Ser Ser Ser Ser Ser Pro Ala Arg Ser Ser Gly
725 730 735
Ser Thr Glu Gln Pro His Arg Ser Lys Arg Pro Trp Ala Glu Thr Thr
740 745 750
Thr Thr Thr Thr Ser Gly Arg Asp Gln Asp His Leu Glu Ala Leu Tyr
755 760 765
Asp Ala Val Arg Asp Asn Pro Arg Arg Val Ile Leu Met Glu Arg Val
770 775 780
Asp Arg Ala Asp Ala Arg Cys His Asp Gly Ile Arg Asp Ala Ile Glu
785 790 795 800
Arg Gly Val Val Arg Ser Arg Gly Gly Gly Gly Glu Glu Ala Phe Leu
805 810 815
Gly Asp Ala Ile Val Val Leu Ser Cys Glu Ser Leu Asn Pro Ser Ser
820 825 830
Thr Thr Pro Ala Lys Lys Ala Lys Thr Glu Tyr Ser Val Glu Lys Leu
835 840 845
Asp Gln Asp Gly Asp Asp His His Gly Lys Glu Ala Val Ala Ala Ala
850 855 860
Ala Ser Pro Ser Cys Phe Asp Leu Asn Met Ser Met Asp Asp Asp Asp
865 870 875 880
Glu Ala Ala Glu Glu Arg Cys Thr Gly Glu Glu Glu Glu Ala Gly His
885 890 895
His His His Gln Leu Leu Leu Lys Ala Val Asp Arg Val Leu Phe Phe
900 905 910
Arg Ser Ile Gly Glu
915

<210> 73
<211> 53
<212> DNA
<213> Artificial sequence

<220>

<223> primer

<400> 73
ggggacaagt ttgtacaaaa aagcaggctt aaacaatgag agctggaggc tgc 53

<210> 74
<211> 50
<212> DNA
<213> Artificial sequence

<220>
<223> primer

<400> 74
ggggaccact ttgtacaaga aagctgggtg catacgtgca tgctgttagt 50

<210> 75
<211> 1086
<212> PRT
<213> Artificial sequence

<220>
<223> consensus sequence

<220>
<221> Variant
<222> (3)..(3)
<223> Xaa in position 3 is any amino acid

<220>
<221> Variant
<222> (5)..(5)
<223> Xaa in position 5 is any amino acid

<220>
<221> Variant
<222> (7)..(7)
<223> Xaa in position 7 is any amino acid

<220>
<221> Variant
<222> (10)..(10)
<223> Xaa in position 10 is any amino acid

<220>
<221> Variant
<222> (12)..(12)
<223> Xaa in position 12 is any amino acid

<220>
<221> Variant
<222> (14)..(16)
<223> Xaa in position 14 to 16 is any amino acid

<220>
<221> Variant
<222> (19)..(20)
<223> Xaa in position 19 to 20 is any amino acid

<220>
<221> Variant
<222> (22)..(22)
<223> Xaa in position 22 is any amino acid

<220>
<221> Variant
<222> (26)..(26)
<223> Xaa in position 26 is any amino acid

<220>
<221> Variant
<222> (29)..(29)
<223> Xaa in position 29 is any amino acid

<220>
<221> Variant
<222> (43)..(44)
<223> Xaa in position 43 to 44 is any amino acid

<220>
<221> Variant
<222> (47)..(49)
<223> Xaa in position 47 to 49 is any amino acid

<220>
<221> Variant
<222> (50)..(67)
<223> Xaa in position 50 to 67 is any or no amino acid

<220>
<221> Variant
<222> (72)..(72)
<223> Xaa in position 72 is any amino acid

<220>
<221> Variant
<222> (76)..(76)
<223> Xaa in position 76 is any amino acid

<220>
<221> Variant
<222> (78)..(79)
<223> Xaa in position 78 to 79 is any amino acid

<220>
<221> Variant
<222> (102)..(115)
<223> Xaa in position 102 to 115 is any amino acid

<220>
<221> Variant
<222> (116)..(129)
<223> Xaa in position 116 to 129 is any or no amino acid

<220>
<221> Variant
<222> (131)..(132)
<223> Xaa in position 131 to 132 is any amino acid

<220>
<221> Variant
<222> (151)..(152)
<223> Xaa in position 151 to 152 is any amino acid

<220>
<221> Variant
<222> (153)..(155)
<223> Xaa in position 153 to 155 is any or no amino acid

<220>
<221> Variant
<222> (157)..(157)
<223> Xaa in position 157 is any amino acid

<220>
<221> Variant
<222> (159)..(160)
<223> Xaa in position 159 to 160 is any amino acid

<220>
<221> Variant
<222> (161)..(172)
<223> Xaa in position 161 to 172 is any or no amino acid

<220>
<221> Variant
<222> (175)..(175)
<223> Xaa in position 175 is any amino acid

<220>
<221> Variant
<222> (177)..(177)
<223> Xaa in position 177 is any amino acid

<220>
<221> Variant
<222> (179)..(179)
<223> Xaa in position 179 is any amino acid

<220>
<221> Variant
<222> (183)..(183)
<223> Xaa in position 183 is any amino acid

<220>
<221> Variant
<222> (204)..(205)
<223> Xaa in position 204 to 205 is any amino acid

<220>
<221> Variant
<222> (208)..(209)
<223> Xaa in position 208 to 209 is any amino acid

<220>
<221> Variant
<222> (210)..(211)
<223> Xaa in position 210 to 211 is any or no amino acid

<220>
<221> Variant
<222> (214)..(214)
<223> Xaa in position 214 is any amino acid

<220>
<221> Variant
<222> (218)..(239)
<223> Xaa in position 218 to 239 is any amino acid

<220>
<221> Variant
<222> (240)..(276)
<223> Xaa in position 240 to 276 is any or no amino acid

<220>
<221> Variant
<222> (278)..(280)
<223> Xaa in position 278 to 280 is any amino acid

<220>
<221> Variant
<222> (282)..(291)
<223> Xaa in position 282 to 291 is any amino acid

<220>
<221> Variant
<222> (292)..(303)
<223> Xaa in position 292 to 303 is any or no amino acid

<220>
<221> Variant
<222> (305)..(306)
<223> Xaa in position 305 to 306 is any amino acid

<220>
<221> Variant
<222> (307)..(307)
<223> Xaa in position 307 is any or no amino acid

<220>
<221> Variant
<222> (309)..(311)
<223> Xaa in position 309 to 311 is any amino acid

<220>
<221> Variant
<222> (312)..(313)
<223> Xaa in position 312 to 313 is any or no amino acid

<220>
<221> Variant
<222> (315)..(324)
<223> Xaa in position 315 to 324 is any amino acid

<220>
<221> Variant
<222> (325)..(326)
<223> Xaa in position 325 to 326 is any or no amino acid

<220>
<221> Variant
<222> (328)..(331)
<223> Xaa in position 328 to 331 is any amino acid

<220>
<221> Variant
<222> (332)..(336)
<223> Xaa in position 332 to 336 is any or no amino acid

<220>
<221> Variant
<222> (338)..(338)
<223> Xaa in position 338 is any amino acid

<220>
<221> Variant
<222> (339)..(348)
<223> Xaa in position 339 to 348 is any or no amino acid

<220>
<221> Variant
<222> (350)..(351)
<223> Xaa in position 350 to 351 is any amino acid

<220>
<221> Variant
<222> (353)..(357)
<223> Xaa in position 353 to 357 is any amino acid

<220>
<221> Variant
<222> (358)..(359)
<223> Xaa in position 358 to 359 is any or no amino acid

<220>
<221> Variant
<222> (361)..(361)
<223> Xaa in position 361 is any amino acid

<220>
<221> Variant
<222> (363)..(367)
<223> Xaa in position 363 to 367 is any amino acid

<220>
<221> Variant
<222> (368)..(389)
<223> Xaa in position 368 to 389 is any or no amino acid

<220>
<221> Variant
<222> (391)..(392)
<223> Xaa in position 391 to 392 is any amino acid

<220>
<221> Variant
<222> (395)..(399)
<223> Xaa in position 395 to 399 is any amino acid

<220>
<221> Variant
<222> (400)..(402)
<223> Xaa in position 400 to 402 is any or no amino acid

<220>
<221> Variant
<222> (405)..(405)
<223> Xaa in position 405 is any amino acid

<220>
<221> Variant
<222> (407)..(409)
<223> Xaa in position 407 to 409 is any amino acid

<220>
<221> Variant
<222> (412)..(423)
<223> Xaa in position 412 to 423 is any amino acid

<220>
<221> Variant
<222> (424)..(440)
<223> Xaa in position 424 to 440 is any or no amino acid

<220>
<221> Variant
<222> (443)..(444)
<223> Xaa in position 443 to 444 is any amino acid

<220>
<221> Variant
<222> (447)..(449)
<223> Xaa in position 447 to 449 is any amino acid

<220>
<221> Variant
<222> (451)..(453)
<223> Xaa in position 451 to 453 is any amino acid

<220>
<221> Variant
<222> (455)..(461)
<223> Xaa in position 455 to 461 is any amino acid

<220>
<221> Variant
<222> (462)..(471)
<223> Xaa in position 462 to 471 is any or no amino acid

<220>
<221> Variant
<222> (474)..(474)
<223> Xaa in position 474 is any amino acid

<220>
<221> Variant
<222> (476)..(481)
<223> Xaa in position 476 to 481 is any amino acid

<220>
<221> Variant
<222> (483)..(484)
<223> Xaa in position 483 to 484 is any amino acid

<220>
<221> Variant
<222> (486)..(487)
<223> Xaa in position 486 to 487 is any amino acid

<220>
<221> Variant
<222> (488)..(488)
<223> Xaa in position 488 is any or no amino acid

<220>
<221> Variant
<222> (490)..(490)
<223> Xaa in position 490 is any amino acid

<220>
<221> Variant
<222> (495)..(496)
<223> Xaa in position 495 to 496 is any amino acid

<220>
<221> Variant
<222> (498)..(498)
<223> Xaa in position 498 is any amino acid

<220>
<221> Variant
<222> (500)..(504)
<223> Xaa in position 500 to 504 is any amino acid

<220>
<221> Variant
<222> (506)..(506)
<223> Xaa in position 506 is any amino acid

<220>
<221> Variant
<222> (508)..(508)
<223> Xaa in position 508 is any amino acid

<220>
<221> Variant
<222> (509)..(510)
<223> Xaa in position 509 to 510 is any or no amino acid

<220>
<221> Variant
<222> (512)..(512)
<223> Xaa in position 512 is any amino acid

<220>
<221> Variant
<222> (516)..(535)
<223> Xaa in position 516 to 535 is any amino acid

<220>
<221> Variant
<222> (536)..(614)
<223> Xaa in position 536 to 614 is any or no amino acid

<220>
<221> Variant
<222> (616)..(616)
<223> Xaa in position 616 is any amino acid

<220>
<221> Variant
<222> (619)..(629)
<223> Xaa in position 619 to 629 is any amino acid

<220>
<221> Variant
<222> (630)..(645)
<223> Xaa in position 630 to 645 is any or no amino acid

<220>
<221> Variant
<222> (647)..(658)
<223> Xaa in position 647 to 658 is any amino acid

<220>
<221> Variant
<222> (659)..(677)
<223> Xaa in position 659 to 677 is any or no amino acid

<220>
<221> Variant
<222> (679)..(680)
<223> Xaa in position 679 to 680 is any amino acid

<220>
<221> Variant
<222> (682)..(693)
<223> Xaa in position 682 to 693 is any amino acid

<220>
<221> Variant
<222> (694)..(699)
<223> Xaa in position 694 to 699 is any or no amino acid

<220>
<221> Variant
<222> (701)..(727)
<223> Xaa in position 701 to 727 is any amino acid

<220>
<221> Variant
<222> (728)..(764)
<223> Xaa in position 728 to 764 is any or no amino acid

<220>
<221> Variant
<222> (766)..(775)
<223> Xaa in position 766 to 775 is any amino acid

<220>
<221> Variant
<222> (776)..(786)
<223> Xaa in position 776 to 786 is any or no amino acid

<220>
<221> Variant
<222> (788)..(788)
<223> Xaa in position 788 is any amino acid

<220>
<221> Variant
<222> (790)..(791)
<223> Xaa in position 790 to 791 is any amino acid

<220>
<221> Variant
<222> (796)..(797)
<223> Xaa in position 796 to 797 is any amino acid

<220>
<221> Variant
<222> (800)..(800)
<223> Xaa in position 800 is any amino acid

<220>
<221> Variant
<222> (804)..(805)
<223> Xaa in position 804 to 805 is any amino acid

<220>
<221> Variant
<222> (808)..(809)
<223> Xaa in position 808 to 809 is any amino acid

<220>
<221> Variant
<222> (811)..(811)
<223> Xaa in position 811 is any amino acid

<220>
<221> Variant
<222> (812)..(812)
<223> Xaa in position 812 is any or no amino acid

<220>
<221> Variant
<222> (814)..(815)
<223> Xaa in position 814 to 815 is any amino acid

<220>
<221> Variant
<222> (816)..(816)
<223> Xaa in position 816 is any or no amino acid

<220>
<221> Variant
<222> (820)..(821)
<223> Xaa in position 820 to 821 is any amino acid

<220>
<221> Variant
<222> (823)..(823)
<223> Xaa in position 823 is any amino acid

<220>
<221> Variant
<222> (828)..(837)
<223> Xaa in position 828 to 837 is any amino acid

<220>
<221> Variant
<222> (838)..(848)
<223> Xaa in position 838 to 848 is any or no amino acid

<220>
<221> Variant
<222> (851)..(852)
<223> Xaa in position 851 to 852 is any amino acid

<220>
<221> Variant
<222> (854)..(854)
<223> Xaa in position 854 is any amino acid

<220>
<221> Variant
<222> (856)..(856)
<223> Xaa in position 856 is any amino acid

<220>
<221> Variant
<222> (858)..(860)
<223> Xaa in position 858 to 860 is any amino acid

<220>
<221> Variant
<222> (862)..(866)
<223> Xaa in position 862 to 866 is any amino acid

<220>
<221> Variant
<222> (870)..(870)
<223> Xaa in position 870 is any amino acid

<220>
<221> Variant
<222> (876)..(878)
<223> Xaa in position 876 to 878 is any amino acid

<220>
<221> Variant
<222> (880)..(880)
<223> Xaa in position 880 is any amino acid

<220>
<221> Variant
<222> (883)..(889)
<223> Xaa in position 883 to 889 is any amino acid

<220>
<221> Variant
<222> (890)..(904)
<223> Xaa in position 890 to 904 is any or no amino acid

<220>
<221> Variant
<222> (906)..(906)
<223> Xaa in position 906 is any amino acid

<220>
<221> Variant
<222> (908)..(910)
<223> Xaa in position 908 to 910 is any amino acid

<220>
<221> Variant
<222> (911)..(912)
<223> Xaa in position 911 to 912 is any or no amino acid

<220>
<221> Variant
<222> (915)..(915)
<223> Xaa in position 915 is any amino acid

<220>
<221> Variant
<222> (917)..(923)
<223> Xaa in position 917 to 923 is any amino acid

<220>
<221> Variant
<222> (924)..(933)
<223> Xaa in position 924 to 933 is any or no amino acid

<220>
<221> Variant
<222> (935)..(937)
<223> Xaa in position 935 to 937 is any amino acid

<220>
<221> Variant
<222> (939)..(941)
<223> Xaa in position 939 to 941 is any amino acid

<220>
<221> Variant
<222> (947)..(950)
<223> Xaa in position 947 to 950 is any amino acid

<220>
<221> Variant
<222> (952)..(952)
<223> Xaa in position 952 is any amino acid

<220>
<221> Variant
<222> (953)..(954)
<223> Xaa in position 953 to 954 is any or no amino acid

<220>
<221> Variant
<222> (956)..(956)
<223> Xaa in position 956 is any amino acid

<220>
<221> Variant
<222> (958)..(962)
<223> Xaa in position 958 to 962 is any amino acid

<220>
<221> Variant
<222> (964)..(966)
<223> Xaa in position 964 to 966 is any amino acid

<220>
<221> Variant
<222> (969)..(970)
<223> Xaa in position 969 to 970 is any amino acid

<220>
<221> Variant
<222> (972)..(977)
<223> Xaa in position 972 to 977 is any amino acid

<220>
<221> Variant
<222> (979)..(982)
<223> Xaa in position 979 to 982 is any amino acid

<220>
<221> Variant
<222> (983)..(984)
<223> Xaa in position 983 to 984 is any or no amino acid

<220>
<221> Variant
<222> (986)..(986)
<223> Xaa in position 986 is any amino acid

<220>
<221> Variant
<222> (990)..(991)
<223> Xaa in position 990 to 991 is any amino acid

<220>
<221> Variant
<222> (992)..(992)
<223> Xaa in position 992 is any or no amino acid

<220>
<221> Variant
<222> (994)..(994)
<223> Xaa in position 994 is any amino acid

<220>
<221> Variant
<222> (997)..(998)
<223> Xaa in position 997 to 998 is any amino acid

<220>
<221> Variant
<222> (999)..(999)
<223> Xaa in position 999 is any or no amino acid

<220>
<221> Variant
<222> (1001)..(1026)
<223> Xaa in position 1001 to 1026 is any amino acid

<220>
<221> Variant
<222> (1027)..(1043)
<223> Xaa in position 1027 to 1043 is any or no amino acid

<220>
<221> Variant
<222> (1048)..(1060)
<223> Xaa in position 1048 to 1060 is any amino acid

<220>
<221> Variant
<222> (1061)..(1076)
<223> Xaa in position 1061 to 1076 is any or no amino acid

<220>
<221> Variant
<222> (1078)..(1079)
<223> Xaa in position 1078 to 1079 is any amino acid

<220>
<221> Variant
<222> (1082)..(1085)
<223> Xaa in position 1082 to 1085 is any amino acid

<400> 75
```
Met Arg Xaa Gly Xaa Cys Xaa Val Gln Xaa Gln Xaa Leu Xaa Xaa Xaa
1               5                   10                  15
Ala Ala Xaa Xaa Val Xaa Gln Ala Val Xaa Leu Ala Xaa Arg Arg Gly
            20                  25                  30
His Ala Gln Val Thr Pro Leu His Val Ala Xaa Xaa Met Leu Xaa Xaa
        35                  40                  45
Xaa Xaa Xaa Xaa Xaa Xaa Xaa Xaa Xaa Xaa Xaa Xaa Xaa Xaa Xaa Xaa
    50                  55                  60
Xaa Xaa Xaa Gly Leu Leu Arg Xaa Ala Cys Leu Xaa Ser Xaa Xaa His
65                  70                  75                  80
Ser His Pro Leu Gln Cys Lys Ala Leu Glu Leu Cys Phe Asn Val Ala
                85                  90                  95
Leu Asn Arg Leu Pro Xaa Xaa Xaa Xaa Xaa Xaa Xaa Xaa Xaa Xaa Xaa
            100                 105                 110
Xaa Xaa Xaa Xaa Xaa Xaa Xaa Xaa Xaa Xaa Xaa Xaa Xaa Xaa Xaa Xaa
        115                 120                 125
Xaa Pro Xaa Xaa Ser Asn Ala Leu Val Ala Ala Phe Lys Arg Ala Gln
    130                 135                 140
Ala His Gln Arg Arg Gly Xaa Xaa Xaa Xaa Xaa Gln Xaa Gln Xaa Xaa
145                 150                 155                 160
Xaa Xaa Xaa Xaa Xaa Xaa Xaa Xaa Xaa Xaa Xaa Xaa Leu Ala Xaa Lys
                165                 170                 175
Xaa Glu Xaa Glu Gln Leu Xaa Ile Ser Ile Leu Asp Asp Pro Ser Val
            180                 185                 190
```

Ser Arg Val Met Arg Glu Ala Gly Phe Ser Ser Xaa Xaa Val Lys Xaa
195 200 205
Xaa Xaa Xaa Val Glu Xaa Ala Val Ser Xaa Xaa Xaa Xaa Xaa Xaa Xaa
210 215 220
Xaa Xaa Xaa Xaa Xaa Xaa Xaa Xaa Xaa Xaa Xaa Xaa Xaa Xaa Xaa Xaa
225 230 235 240
Xaa Xaa Xaa Xaa Xaa Xaa Xaa Xaa Xaa Xaa Xaa Xaa Xaa Xaa Xaa Xaa
245 250 255
Xaa Xaa Xaa Xaa Xaa Xaa Xaa Xaa Xaa Xaa Xaa Xaa Xaa Xaa Xaa Xaa
260 265 270
Xaa Xaa Xaa Xaa Asp Xaa Xaa Xaa Val Xaa Xaa Xaa Xaa Xaa Xaa Xaa
275 280 285
Xaa Xaa Xaa Xaa Xaa Xaa Xaa Xaa Xaa Xaa Xaa Xaa Xaa Xaa Xaa Val
290 295 300
Xaa Xaa Xaa Glu Xaa Xaa Xaa Xaa Xaa Glu Xaa Xaa Xaa Xaa Xaa Xaa
305 310 315 320
Xaa Xaa Xaa Xaa Xaa Xaa Gly Xaa Xaa Xaa Xaa Xaa Xaa Xaa Xaa Xaa
325 330 335
Leu Xaa Xaa Xaa Xaa Xaa Xaa Xaa Xaa Xaa Xaa Xaa Ser Xaa Xaa Phe
340 345 350
Xaa Xaa Xaa Xaa Xaa Xaa Xaa Glu Xaa Glu Xaa Xaa Xaa Xaa Xaa Xaa
355 360 365
Xaa Xaa Xaa Xaa Xaa Xaa Xaa Xaa Xaa Xaa Xaa Xaa Xaa Xaa Xaa Xaa
370 375 380
Xaa Xaa Xaa Xaa Xaa Leu Xaa Xaa Leu Val Xaa Xaa Xaa Xaa Xaa Xaa
385 390 395 400
Xaa Xaa Gly Val Xaa Leu Xaa Xaa Xaa Asp Leu Xaa Xaa Xaa Xaa Xaa
405 410 415
Xaa Xaa Xaa Xaa Xaa Xaa Xaa Xaa Xaa Xaa Xaa Xaa Xaa Xaa Xaa Xaa
420 425 430
Xaa Xaa Xaa Xaa Xaa Xaa Xaa Xaa Tyr Cys Xaa Xaa Glu His Xaa Xaa
435 440 445
Xaa Glu Xaa Xaa Xaa Leu Xaa Xaa Xaa Xaa Xaa Xaa Xaa Xaa Xaa Xaa
450 455 460
Xaa Xaa Xaa Xaa Xaa Xaa Xaa Trp Leu Xaa Gly Xaa Xaa Xaa Xaa Xaa
465 470 475 480
Xaa Tyr Xaa Xaa Cys Xaa Xaa Xaa Gly Xaa Pro Ser Leu Glu Xaa Xaa
485 490 495
Trp Xaa Leu Xaa Xaa Xaa Xaa Xaa Pro Xaa Gly Xaa Xaa Xaa Leu Xaa
500 505 510
Leu Ser Leu Xaa Xaa Xaa Xaa Xaa Xaa Xaa Xaa Xaa Xaa Xaa Xaa Xaa
515 520 525
Xaa Xaa Xaa Xaa Xaa Xaa Xaa Xaa Xaa Xaa Xaa Xaa Xaa Xaa Xaa Xaa
530 535 540
Xaa Xaa Xaa Xaa Xaa Xaa Xaa Xaa Xaa Xaa Xaa Xaa Xaa Xaa Xaa Xaa
545 550 555 560
Xaa Xaa Xaa Xaa Xaa Xaa Xaa Xaa Xaa Xaa Xaa Xaa Xaa Xaa Xaa Xaa
565 570 575
Xaa Xaa Xaa Xaa Xaa Xaa Xaa Xaa Xaa Xaa Xaa Xaa Xaa Xaa Xaa Xaa
580 585 590
Xaa Xaa Xaa Xaa Xaa Xaa Xaa Xaa Xaa Xaa Xaa Xaa Xaa Xaa Xaa Xaa
595 600 605
Xaa Xaa Xaa Xaa Xaa Xaa Pro Xaa Trp Leu Xaa Xaa Xaa Xaa Xaa Xaa
610 615 620
Xaa Xaa Xaa Xaa Xaa Xaa Xaa Xaa Xaa Xaa Xaa Xaa Xaa Xaa Xaa Xaa
625 630 635 640
Xaa Xaa Xaa Xaa Xaa Trp Xaa Xaa Xaa Xaa Xaa Xaa Xaa Xaa Xaa Xaa
645 650 655
Xaa Xaa Xaa Xaa Xaa Xaa Xaa Xaa Xaa Xaa Xaa Xaa Xaa Xaa Xaa Xaa
660 665 670
Xaa Xaa Xaa Xaa Xaa Ser Xaa Xaa Ser Xaa Xaa Xaa Xaa Xaa Xaa Xaa
675 680 685
Xaa Xaa Xaa Xaa Xaa Xaa Xaa Xaa Xaa Xaa Xaa Trp Xaa Xaa Xaa Xaa
690 695 700
Xaa Xaa Xaa Xaa Xaa Xaa Xaa Xaa Xaa Xaa Xaa Xaa Xaa Xaa Xaa Xaa

```
705                 710                 715                 720
Xaa Xaa Xaa Xaa Xaa Xaa Xaa Xaa Xaa Xaa Xaa Xaa Xaa Xaa Xaa Xaa
                725                 730                 735
Xaa Xaa Xaa Xaa Xaa Xaa Xaa Xaa Xaa Xaa Xaa Xaa Xaa Xaa Xaa Xaa
            740                 745                 750
Xaa Xaa Xaa Xaa Xaa Xaa Xaa Xaa Xaa Xaa Xaa Xaa Ser Xaa Xaa Xaa
        755                 760                 765
Xaa Xaa Xaa Xaa Xaa Xaa Xaa Xaa Xaa Xaa Xaa Xaa Xaa Xaa Xaa Xaa
    770                 775                 780
Xaa Xaa Phe Xaa Glu Xaa Xaa Ala Glu Asn Leu Xaa Xaa Leu Cys Xaa
785                 790                 795                 800
Ala Leu Glu Xaa Xaa Val Pro Xaa Xaa Lys Xaa Xaa Ile Xaa Xaa Xaa
                805                 810                 815
Ile Ala Ser Xaa Xaa Leu Xaa Cys Arg Ser Gly Xaa Xaa Xaa Xaa Xaa
            820                 825                 830
Xaa Xaa Xaa Xaa Xaa Xaa Xaa Xaa Xaa Xaa Xaa Xaa Xaa Xaa Xaa Xaa
        835                 840                 845
Thr Trp Xaa Xaa Phe Xaa Gly Xaa Asp Xaa Xaa Xaa Lys Xaa Xaa Xaa
    850                 855                 860
Xaa Xaa Glu Leu Ala Xaa Leu Val Phe Gly Ser Xaa Xaa Xaa Phe Xaa
865                 870                 875                 880
Ser Ile Xaa Xaa Xaa Xaa Xaa Xaa Xaa Xaa Xaa Xaa Xaa Xaa Xaa Xaa
                885                 890                 895
Xaa Xaa Xaa Xaa Xaa Xaa Xaa Xaa Ser Xaa Glu Xaa Xaa Xaa Xaa Xaa
            900                 905                 910
Lys Arg Xaa Arg Xaa Xaa Xaa Xaa Xaa Xaa Xaa Xaa Xaa Xaa Xaa Xaa
        915                 920                 925
Xaa Xaa Xaa Xaa Xaa Arg Xaa Xaa Xaa Ala Xaa Xaa Xaa Asn Pro His
    930                 935                 940
Arg Val Xaa Xaa Xaa Xaa Asp Xaa Xaa Xaa Gln Xaa Asp Xaa Xaa Xaa
945                 950                 955                 960
Xaa Xaa Gly Xaa Xaa Xaa Ala Ile Xaa Xaa Gly Xaa Xaa Xaa Xaa Xaa
                965                 970                 975
Xaa Gly Xaa Xaa Xaa Xaa Xaa Xaa Leu Xaa Asp Ala Ile Xaa Xaa Xaa
            980                 985                 990
Ser Xaa Cys Glu Xaa Xaa Xaa Ser Xaa Xaa Xaa Xaa Xaa Xaa Xaa Xaa
        995                 1000                1005
Xaa Xaa Xaa Xaa Xaa Xaa Xaa Xaa Xaa Xaa Xaa Xaa Xaa Xaa Xaa Xaa
   1010                1015                1020
Xaa Xaa Xaa Xaa Xaa Xaa Xaa Xaa Xaa Xaa Xaa Xaa Xaa Xaa Xaa Xaa
1025                1030                1035                1040
Xaa Xaa Xaa Leu Asp Leu Asn Xaa Xaa Xaa Xaa Xaa Xaa Xaa Xaa Xaa
               1045                1050                1055
Xaa Xaa Xaa Xaa Xaa Xaa Xaa Xaa Xaa Xaa Xaa Xaa Xaa Xaa Xaa Xaa
           1060                1065                1070
Xaa Xaa Xaa Xaa Leu Xaa Xaa Val Asp Xaa Xaa Xaa Xaa Phe
       1075                1080                1085
```

<210> 76
<211> 42
<212> PRT
<213> Artificial sequence

<220>
<223> protein pattern

<220>
<221> Variant
<222> (2)..(2)
<223> Xaa in position 2 is Ala, Gly, Leu or Val

<220>
<221> Variant
<222> (4)..(5)

<223> Xaa in position 4 to 5 is any or no amino acid

<220>
<221> Variant
<222> (7)..(7)
<223> Xaa in position 7 is any amino acid

<220>
<221> Variant
<222> (8)..(9)
<223> Xaa in position 8 to 9 is any or no amino acid

<220>
<221> Variant
<222> (12)..(12)
<223> Xaa in position 12 is Ile, Met or Val

<220>
<221> Variant
<222> (13)..(13)
<223> Xaa in position 13 is Ile or Val

<220>
<221> Variant
<222> (17)..(17)
<223> Xaa in position 17 is Asp or His

<220>
<221> Variant
<222> (27)..(27)
<223> Xaa in position 27 is Asp or Glu

<220>
<221> Variant
<222> (29)..(29)
<223> Xaa in position 29 is Asp or Gly

<220>
<221> Variant
<222> (31)..(31)
<223> Xaa in position 31 is Asn or Ser

<220>
<221> Variant
<222> (33)..(33)
<223> Xaa in position 33 is Pro, Ser or Thr

<220>
<221> Variant
<222> (34)..(34)
<223> Xaa in position 34 is any amino acid

<220>
<221> Variant
<222> (35)..(35)
<223> Xaa in position 35 is Leu or Val

<220>
<221> Variant
<222> (36)..(36)
<223> Xaa in position 36 is Lys or Arg

<220>
<221> Variant
<222> (37)..(37)

<223> Xaa in position 37 is Ala, Asn, Ser or Thr

<220>
<221> Variant
<222> (38)..(38)
<223> Xaa in position 38 is any amino acid

<220>
<221> Variant
<222> (39)..(39)
<223> Xaa in position 39 is Ile or Val

<220>
<221> Variant
<222> (41)..(41)
<223> Xaa in position 41 is any amino acid

<220>
<221> Variant
<222> (42)..(42)
<223> Xaa in position 42 is Ala or Glu

<400> 76

```
Ala Xaa Lys Xaa Xaa Ile Xaa Xaa Xaa Gln Leu Xaa Xaa Ser Ile Leu
1               5                   10                  15
Xaa Asp Pro Ser Val Ser Arg Val Met Arg Xaa Ala Xaa Phe Xaa Ser
            20                  25                  30
Xaa Xaa Xaa Xaa Xaa Xaa Xaa Glu Xaa Xaa
        35                  40
```

<210> 77
<211> 21
<212> PRT
<213> Artificial sequence

<220>
<223> protein pattern

<220>
<221> Variant
<222> (2)..(2)
<223> Xaa in position 2 is any amino acid

<220>
<221> Variant
<222> (4)..(4)
<223> Xaa in position 4 is any amino acid

<220>
<221> Variant
<222> (5)..(5)
<223> Xaa in position 5 is Asn, Ser or Thr

<220>
<221> Variant
<222> (6)..(6)
<223> Xaa in position 6 is Ala, Asp, Glu, Ser or Thr

<220>
<221> Variant
<222> (10)..(11)
<223> Xaa in position 10 to 11 is any amino acid

<220>

<221> Variant
<222> (13)..(13)
<223> Xaa in position 13 is Cys or Ser

<220>
<221> Variant
<222> (14)..(14)
<223> Xaa in position 14 is any amino acid

<220>
<221> Variant
<222> (17)..(18)
<223> Xaa in position 17 to 18 is any amino acid

<220>
<221> Variant
<222> (19)..(19)
<223> Xaa in position 19 is Glu, Lys or Arg

<220>
<221> Variant
<222> (20)..(20)
<223> Xaa in position 20 is any amino acid

<220>
<221> Variant
<222> (21)..(21)
<223> Xaa in position 21 is Pro, Ser or Thr

<400> 77
```
Phe Xaa Glu Xaa Xaa Xaa Glu Asn Leu Xaa Xaa Leu Xaa Xaa Ala Leu
1               5                   10                  15
Xaa Xaa Xaa Xaa Xaa
            20
```

<210> 78
<211> 38
<212> PRT
<213> Artificial sequence

<220>
<223> protein pattern

<220>
<221> Variant
<222> (3)..(3)
<223> Xaa in position 3 is Phe, Leu or Met

<220>
<221> Variant
<222> (4)..(4)
<223> Xaa in position 4 is Phe or Leu

<220>
<221> Variant
<222> (6)..(6)
<223> Xaa in position 6 is any amino acid

<220>
<221> Variant
<222> (8)..(8)
<223> Xaa in position 8 is any amino acid

<220>

<221> Variant
<222> (9)..(9)
<223> Xaa in position 9 is Asp, Gly or Asn

<220>
<221> Variant
<222> (10)..(11)
<223> Xaa in position 10 to 11 is any amino acid

<220>
<221> Variant
<222> (12)..(12)
<223> Xaa in position 12 is Ala, Asp or Gly

<220>
<221> Variant
<222> (13)..(13)
<223> Xaa in position 13 is Lys or Arg

<220>
<221> Variant
<222> (14)..(15)
<223> Xaa in position 14 to 15 is any amino acid

<220>
<221> Variant
<222> (16)..(16)
<223> Xaa in position 16 is Ile, Met or Val

<220>
<221> Variant
<222> (17)..(17)
<223> Xaa in position 17 is Ala, Cys or Ser

<220>
<221> Variant
<222> (18)..(18)
<223> Xaa in position 18 is any amino acid

<220>
<221> Variant
<222> (22)..(22)
<223> Xaa in position 22 is any amino acid

<220>
<221> Variant
<222> (23)..(23)
<223> Xaa in position 23 is Leu or Val

<220>
<221> Variant
<222> (28)..(30)
<223> Xaa in position 28 to 30 is any amino acid

<220>
<221> Variant
<222> (31)..(31)
<223> Xaa in position 31 is Phe or Val

<220>
<221> Variant
<222> (32)..(32)
<223> Xaa in position 32 is any amino acid

<220>

<221> Variant
<222> (33)..(33)
<223> Xaa in position 33 is Ala, Cys, Ser or Thr

<220>
<221> Variant
<222> (34)..(36)
<223> Xaa in position 34 to 36 is any amino acid

<220>
<221> Variant
<222> (37)..(37)
<223> Xaa in position 37 is Ala, Asp, Gly or Ser

<220>
<221> Variant
<222> (38)..(38)
<223> Xaa in position 38 is Ala, Asp, Glu, Gly, Asn, Ser or Thr

<400> 78
```
Thr Trp Xaa Xaa Phe Xaa Gly Xaa Xaa Xaa Xaa Xaa Xaa Xaa Xaa Xaa
1               5                   10                  15
Xaa Xaa Glu Leu Ala Xaa Xaa Val Phe Gly Ser Xaa Xaa Xaa Xaa Xaa
            20                  25                  30
Xaa Xaa Xaa Xaa Xaa Xaa
        35
```

<210> 79
<211> 19
<212> PRT
<213> Artificial sequence

<220>
<223> protein pattern

<220>
<221> Variant
<222> (2)..(2)
<223> Xaa in position 2 is any amino acid

<220>
<221> Variant
<222> (3)..(3)
<223> Xaa in position 3 is any or no amino acid

<220>
<221> Variant
<222> (5)..(5)
<223> Xaa in position 5 is any or no amino acid

<220>
<221> Variant
<222> (7)..(7)
<223> Xaa in position 7 is any amino acid

<220>
<221> Variant
<222> (8)..(8)
<223> Xaa in position 8 is Leu or Val

<220>
<221> Variant
<222> (9)..(9)
<223> Xaa in position 9 is Glu or Gly

<220>
<221> Variant
<222> (12)..(12)
<223> Xaa in position 12 is any amino acid

<220>
<221> Variant
<222> (13)..(13)
<223> Xaa in position 13 is Trp or Tyr

<220>
<221> Variant
<222> (14)..(15)
<223> Xaa in position 14 to 15 is any amino acid

<220>
<221> Variant
<222> (16)..(16)
<223> Xaa in position 16 is Asp or Glu

<220>
<221> Variant
<222> (17)..(18)
<223> Xaa in position 17 to 18 is any amino acid

<220>
<221> Variant
<222> (19)..(19)
<223> Xaa in position 19 is Ala, Ser or Thr

<400> 79
Gly Xaa Xaa Val Xaa Leu Xaa Xaa Xaa Asp Leu Xaa Xaa Xaa Xaa Xaa
1               5                   10                  15
Xaa Xaa Xaa

<210> 80
<211> 14
<212> PRT
<213> Artificial sequence

<220>
<223> protein pattern

<220>
<221> Variant
<222> (2)..(5)
<223> Xaa in position 2 to 5 is any or no amino acid

<220>
<221> Variant
<222> (7)..(7)
<223> Xaa in position 7 is any amino acid

<220>
<221> Variant
<222> (8)..(8)
<223> Xaa in position 8 is Pro or Thr

<220>
<221> Variant
<222> (9)..(9)
<223> Xaa in position 9 is Phe, Ile, Leu or Val

<220>
<221> Variant
<222> (10)..(10)
<223> Xaa in position 10 is Ala, Thr or Val

<220>
<221> Variant
<222> (11)..(11)
<223> Xaa in position 11 is Ile or Val

<220>
<221> Variant
<222> (13)..(13)
<223> Xaa in position 13 is Ala, Asp or Val

<220>
<221> Variant
<222> (14)..(14)
<223> Xaa in position 14 is Gly, Ser or Thr

<400> 80
Glu Xaa Xaa Xaa Xaa Leu Xaa Xaa Xaa Xaa Xaa Pro Xaa Xaa
1               5                   10

<210> 81
<211> 11
<212> PRT
<213> Artificial sequence

<220>
<223> protein pattern

<220>
<221> Variant
<222> (2)..(2)
<223> Xaa in position 2 is any amino acid

<220>
<221> Variant
<222> (4)..(4)
<223> Xaa in position 4 is Ala, Ser or Thr

<220>
<221> Variant
<222> (6)..(7)
<223> Xaa in position 6 to 7 is Ile or Val

<220>
<221> Variant
<222> (8)..(8)
<223> Xaa in position 8 is any amino acid

<220>
<221> Variant
<222> (9)..(9)
<223> Xaa in position 9 is Cys, Gly, Asn or Ser

<220>
<221> Variant
<222> (10)..(10)
<223> Xaa in position 10 is any amino acid

<220>

<221> Variant
<222> (11)..(11)
<223> Xaa in position 11 is Ala, Glu or Gln

<400> 81
Leu Xaa Asp Xaa Ile Xaa Xaa Xaa Xaa Xaa Xaa
1 5 10

<210> 82
<211> 2194
<212> DNA
<213> Oryza sativa

<400> 82
aatccgaaaa gtttctgcac cgttttcacc ccctaactaa caatataggg aacgtgtgct 60

aaatataaaa tgagacctta tatatgtagc gctgataact agaactatgc aagaaaaact 120

catccaccta ctttagtggc aatcgggcta aataaaaaag agtcgctaca ctagtttcgt 180

tttccttagt aattaagtgg gaaaatgaaa tcattattgc ttagaatata cgttcacatc 240

tctgtcatga agttaaatta ttcgaggtag ccataattgt catcaaactc ttcttgaata 300

aaaaaatctt tctagctgaa ctcaatgggt aaagagagag atttttttta aaaaaataga 360

atgaagatat tctgaacgta ttggcaaaga tttaaacata taattatata attttatagt 420

ttgtgcattc gtcatatcgc acatcattaa ggacatgtct tactccatcc caatttttat 480

ttagtaatta aagacaattg acttattttt attatttatc ttttttcgat tagatgcaag 540

gtacttacgc acacactttg tgctcatgtg catgtgtgag tgcacctcct caatacacgt 600

tcaactagca acacatctct aatatcactc gcctatttaa tacatttagg tagcaatatc 660

tgaattcaag cactccacca tcaccagacc acttttaata atatctaaaa tacaaaaaat 720

aattttacag aatagcatga aaagtatgaa acgaactatt taggtttttc acatacaaaa 780

aaaaaaagaa ttttgctcgt gcgcgagcgc caatctccca tattgggcac acaggcaaca 840

acagagtggc tgcccacaga acaacccaca aaaaacgatg atctaacgga ggacagcaag 900

tccgcaacaa ccttttaaca gcaggctttg cggccaggag agaggaggag aggcaaagaa 960

aaccaagcat cctccttctc ccatctataa attcctcccc ccttttcccc tctctatata 1020

ggaggcatcc aagccaagaa gagggagagc accaaggaca cgcgactagc agaagccgag 1080

cgaccgcctt ctcgatccat atcttccggt cgagttcttg gtcgatctct tccctcctcc 1140

acctcctcct cacagggtat gtgcctccct tcggttgttc ttggatttat tgttctaggt 1200

tgtgtagtac gggcgttgat gttaggaaag gggatctgta tctgtgatga ttcctgttct 1260

tggatttggg atagaggggt tcttgatgtt gcatgttatc ggttcggttt gattagtagt 1320

atggttttca atcgtctgga gagctctatg gaaatgaaat ggtttaggga tcggaatctt 1380

gcgattttgt gagtaccttt tgtttgaggt aaaatcagag caccggtgat tttgcttggt 1440

gtaataaagt acggttgttt ggtcctcgat tctggtagtg atgcttctcg atttgacgaa 1500

gctatccttt gtttattccc tattgaacaa aaataatcca actttgaaga cggtcccgtt 1560

gatgagattg aatgattgat tcttaagcct gtccaaaatt tcgcagctgg cttgtttaga 1620

tacagtagtc cccatcacga aattcatgga aacagttata atcctcagga acaggggatt 1680

ccctgttctt ccgatttgct ttagtcccag aatttttttt cccaaatatc ttaaaaagtc 1740

actttctggt tcagttcaat gaattgattg ctacaaataa tgcttttata gcgttatcct 1800

agctgtagtt cagttaatag gtaatacccc tatagtttag tcaggagaag aacttatccg 1860

atttctgatc tccattttta attatatgaa atgaactgta gcataagcag tattcatttg 1920

gattattttt tttattagct ctcacccctt cattattctg agctgaaagt ctggcatgaa 1980

ctgtcctcaa ttttgttttc aaattcacat cgattatcta tgcattatcc tcttgtatct 2040

acctgtagaa gtttcttttt ggttattcct tgactgcttg attacagaaa gaaatttatg 2100

aagctgtaat cgggatagtt atactgcttg ttcttatgat tcatttcctt tgtgcagttc 2160

ttggtgtagc ttgccacttt caccagcaaa gttc 2194

<210> 83
<211> 15
<212> PRT
<213> Artificial sequence

<220>
<223> protein pattern

<220>
<221> Variant
<222> (6)..(6)
<223> Xaa in position 6 is Lys or Arg

<220>
<221> Variant
<222> (13)..(13)
<223> Xaa in position 13 is Asn or Ser

<400> 83
His Pro Leu Gln Cys Xaa Ala Leu Glu Leu Cys Phe Xaa Val Ala
1 5 10 15

<210> 84
<211> 15
<212> PRT
<213> Artificial sequence

<220>
<223> protein pattern

<220>
<221> Variant
<222> (4)..(4)
<223> Xaa in position 4 is Gly or Val

<220>
<221> Variant
<222> (7)..(7)
<223> Xaa in position 7 is Leu or Phe

```
<400> 84
Asn Ala Leu Xaa Ala Ala Xaa Lys Arg Ala Gln Ala His Gln Arg
1               5                   10                  15


<210> 85
<211> 15
<212> PRT
<213> Artificial sequence

<220>
<223> protein pattern

<220>
<221> Variant
<222> (14)..(14)
<223> Xaa in position 14 is Gly or Ser

<400> 85
Leu Asp Asp Pro Ser Val Ser Arg Val Met Arg Glu Ala Xaa Phe
1               5                   10                  15
```

## Description

## Background

The present invention relates generally to the field of molecular biology and concerns a method for enhancing one or more yield-related traits in plants by modulating expression in a plant of a nucleic acid encoding a POI (Protein Of Interest) polypeptide. The present invention also concerns plants having modulated expression of a nucleic acid encoding a POI polypeptides, which plants have one or more one or more enhanced yield-related traits relative to corresponding wild type plants or other control plants. The invention also provides constructs useful in the methods of the invention.

The ever-increasing world population and the dwindling supply of arable land available for agriculture fuels research towards increasing the efficiency of agriculture. Conventional means for crop and horticultural improvements utilise selective breeding techniques to identify plants having desirable characteristics. However, such selective breeding techniques have several drawbacks, namely that these techniques are typically labour intensive and result in plants that often contain heterogeneous genetic components that may not always result in the desirable trait being passed on from parent plants. Advances in molecular biology have allowed mankind to modify the germplasm of animals and plants. Genetic engineering of plants entails the isolation and manipulation of genetic material (typically in the form of DNA or RNA) and the subsequent introduction of that genetic material into a plant. Such technology has the capacity to deliver crops or plants having various improved economic, agronomic or horticultural traits.

A trait of economic interest is increased yield. Yield is normally defined as the measurable produce of economic value from a crop. This may be defined in terms of quantity and/or quality. Yield is directly dependent on several factors, for example, the number and size of the organs, plant architecture (for example, the number of branches), seed production, leaf senescence and more. Root development, nutrient uptake, stress tolerance and early vigour may also be important factors in determining yield. Optimizing the abovementioned factors may therefore contribute to increasing crop yield.

Seed yield is an important trait, since the seeds of many plants are important for human and animal nutrition. Crops such as corn, rice, wheat, canola and soybean account for over half the total human caloric intake, whether through direct consumption of the seeds themselves or through consumption of meat products raised on processed seeds. They are also a source of sugars, oils and many kinds of metabolites used in industrial processes. Seeds contain an embryo (the source of new shoots and roots) and an endosperm (the source of nutrients for embryo growth during germination and during early growth of seedlings). The development of a seed involves many genes, and requires the transfer of metabolites from the roots, leaves and stems into the growing seed. The endosperm, in particular, assimilates the metabolic precursors of carbohydrates, oils and proteins and synthesizes them into storage macromolecules to fill out the grain.

Another important trait for many crops is early vigour. Improving early vigour is an important objective of modern rice breeding programs in both temperate and tropical rice cultivars. Long roots are important for proper soil anchorage in water-seeded rice. Where rice is sown directly into flooded fields, and where plants must emerge rapidly through water, longer shoots are associated with vigour. Where drill-seeding is practiced, longer mesocotyls and coleoptiles are important for good seedling emergence. The ability to engineer early vigour into plants would be of great importance in agriculture. For example, poor early vigour has been a limitation to the introduction of maize (Zea mays L.) hybrids based on Corn Belt germplasm in the European Atlantic.

A further important trait is that of improved abiotic stress tolerance. Abiotic stress is a primary cause of crop loss worldwide, reducing average yields for most major crop plants by more than 50% (Wang et al., Planta 218, 1-14, 2003). Abiotic stresses may be caused by drought, salinity, extremes of temperature, chemical toxicity and oxidative stress. The ability to improve plant tolerance to abiotic stress would be of great economic advantage to farmers worldwide and would allow for the cultivation of crops during adverse conditions and in territories where cultivation of crops may not otherwise be possible.

Crop yield may therefore be increased by optimising one of the above-mentioned factors.

ATP-dependent chaperons like proteins ClpA (At3g52490) are part of the ClpAP protease that participates in regulatory protein degradation and the dissolution and degradation of protein aggregates. For example ClpA recognises sequences in specific proteins, which it then unfolds in an ATP-dependent manner and transports into the degradation chamber of the associated ClpP protein. A small adaptor-like protein, ClpS, modulates the activity of ClpA and is an important regulatory factor for this protein. It protects ClpA from autodegradation and appears to redirect its activity away from soluble proteins and toward aggregated proteins.

Depending on the end use, the modification of certain yield traits may be favoured over others. For example for applications such as forage or wood production, or bio-fuel resource, an increase in the vegetative parts of a plant may be desirable, and for applications such as flour, starch or oil production, an increase in seed parameters may be particularly desirable. Even amongst the seed parameters, some may be favoured over others, depending on the application. Various mechanisms may contribute to increasing seed yield, whether that is in the form of increased seed size or increased seed number.

It has now been found that various yield-related traits may be improved in plants by modulating expression in a plant of a nucleic acid encoding a POI (Protein Of Interest) polypeptide in a plant under non stress conditions but also under conditions of environmental stress,

## Detailed description of the invention

The present invention shows that modulating expression in a plant of a nucleic acid encoding a POI polypeptide gives plants having one or more enhanced yield-related traits relative to control plants.

According to a first embodiment, the present invention provides a method for enhancing one or more yield-related traits in plants relative to control plants, comprising modulating expression in a plant of a nucleic acid encoding a POI polypeptide and optionally selecting for plants having one or more enhanced yield-related traits. According to another embodiment, the present invention provides a method for producing plants having one or more enhanced yield-related traits relative to control plants, wherein said method comprises the steps of modulating expression in said plant of a nucleic acid encoding a POI polypeptide as described herein and optionally selecting for plants having one or more enhanced yield-related traits.

A preferred method for modulating (preferably, increasing) expression of a nucleic acid encoding a POI polypeptide is by introducing and expressing in a plant a nucleic acid encoding a POI polypeptide.

Any reference hereinafter to a "protein useful in the methods of the invention" is taken to mean a POI polypeptide as defined herein. Any reference hereinafter to a "nucleic acid useful in the methods of the invention" is taken to mean a nucleic acid capable of encoding such a POI polypeptide. In one embodiment any reference to a protein or nucleic acid "useful in the methods of the invention" is to be understood to mean proteins or nucleic acids "useful in the methods, constructs, plants, harvestable parts and products of the invention". The nucleic acid to be introduced into a plant (and therefore useful in performing the methods of the invention) is any nucleic acid encoding the type of protein which will now be described, hereafter also named "POI nucleic acid" or "POI gene". The POI gene is encoding a DDL-like polypeptide.

A "POI polypeptide" as defined herein refers to any chaperone-like polypeptide (CLP) preferably a double Clp-N motif-containing P-loop nucleoside triphosphate hydrolases superfamily protein, more preferably comprising the Interpro motifs IPR004176 (Clp, N-terminal) and /or IPR023150 (Double Clp-N motif), preferably both. In a preferred embodiment the POI polypeptide refers to a CLP polypeptide comprising in addition ATP-dependent protease activity, more preferably the CLP polypeptide comprises a PFAM domain PF02861when analysed with the Interproscan software (see example 4 for details on this software), and even more preferably comprises the PFAM domain PF02861 as a split domain in the CLP wherein the N-terminal part of the PFAM domain PF02861 finds a match at or near the N-terminus, preferably starting within in order of preference 100, 90, 80, 70, 60, 55, 50, 45, 40, 35, 30, 29, 28, 27, 26, 25, 24, 23, 22, 21, 20, 19, 18, 17, 16, 15, 14, 13, 12, 11, 10, 9, 8, 7, 6, 5, 4, 3, 2 or 1 amino acid of the N-terminus of the CLP polypeptide, and the C-terminal part of the PFAM domain PF02861 finds a match C-terminally of this, wherein both matching amino acid stretches are located in the N-terminal half, preferably in the N-terminal quarter of the CLP polypeptide. In one embodiment, the N-terminal part of the split PFAM domain matches the amino acid residues of the CLP polypeptide starting from residue 20, 21, 22, 23, 24, 25, 26, 27, 28, 29 or 30 of the CLP polypeptide, preferably from residue 23 or 24, most preferably from amino acid residue 23 when any pre-sequence, N-terminal tag, signal peptide or transit peptide to the CLP polypeptide is ignored.

According one embodiment, there is provided a method for improving yield-related traits as provided herein in plants relative to control plants, comprising modulating expression in a plant of a nucleic acid encoding a CLP polypeptides as defined herein.

In one embodiment the nucleic acid sequences employed in the methods, constructs, plants, harvestable parts and products of the invention are nucleic acid molecule selected from the group consisting of:

(i) a nucleic acid represented by SEQ ID NO: 1;

(ii) the complement of a nucleic acid represented by SEQ ID NO: 1;

(iii) a nucleic acid encoding a CLP polypeptide having in increasing order of preference at least 35%, 40%, 45%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to the amino acid sequence represented by SEQ ID NO: 2, and additionally or alternatively comprising one or more motifs having in increasing order of preference at least 50%, 55%, 60%, 65%, 70%, 75%, 80%, 85%, 90%, 95%, 96%, 97%, 98%, 99% or more sequence identity to any one or more of the motifs given in SEQ ID NO: 76 to SEQ ID NO: 81 and/or one or more of the consensus motifs 1 to 3 as defined above (SEQ ID NO: 83 to 85), and further preferably conferring one or more enhanced yield-related traits relative to control plants; and

(iv) a nucleic acid molecule which hybridizes with a nucleic acid molecule of (i) to (iii) under high stringency hybridization conditions and preferably confers one or more enhanced yield-related traits relative to control plants;

Or encoding a polypeptide selected from the group consisting of:

(i) an amino acid sequence represented by SEQ ID NO: 2;
(ii) an amino acid sequence having, in increasing order of preference, at least 35%, 40%, 45%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to the amino acid sequence represented by SEQ ID NO: 2, and additionally or alternatively comprising one or more motifs having in increasing order of preference at least 50%, 55%, 60%, 65%, 70%, 75%, 80%, 85%, 90%, 95%, 96%, 97%, 98%, 99% or more sequence identity to any one or more of the motifs given in SEQ ID NO: 76 to SEQ ID NO: 81 and/or one or more of the consensus motifs 1 to 3 as defined above (SEQ ID NO: 83 to 85), and further preferably conferring one or more enhanced yield-related traits relative to control plants; and
(iii) derivatives of any of the amino acid sequences given in (i) or (ii) above.

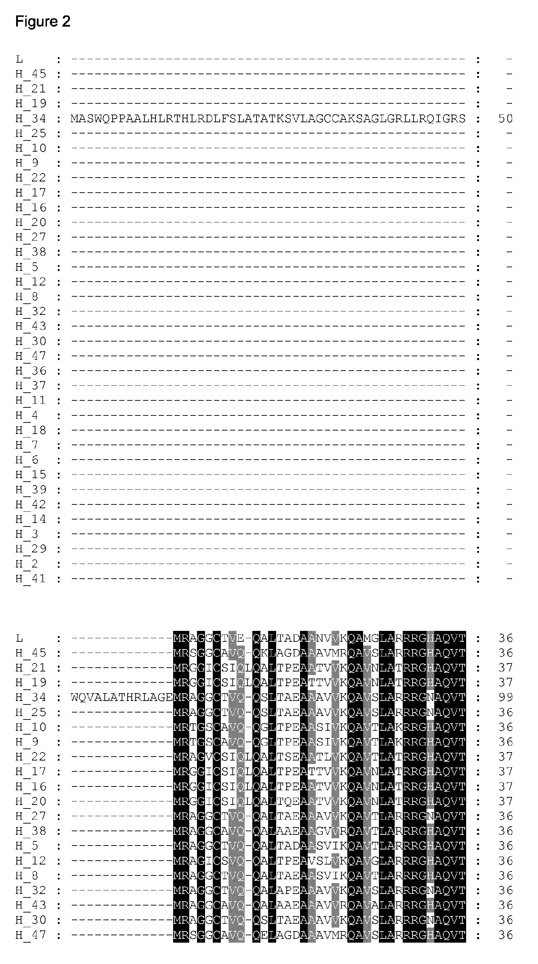

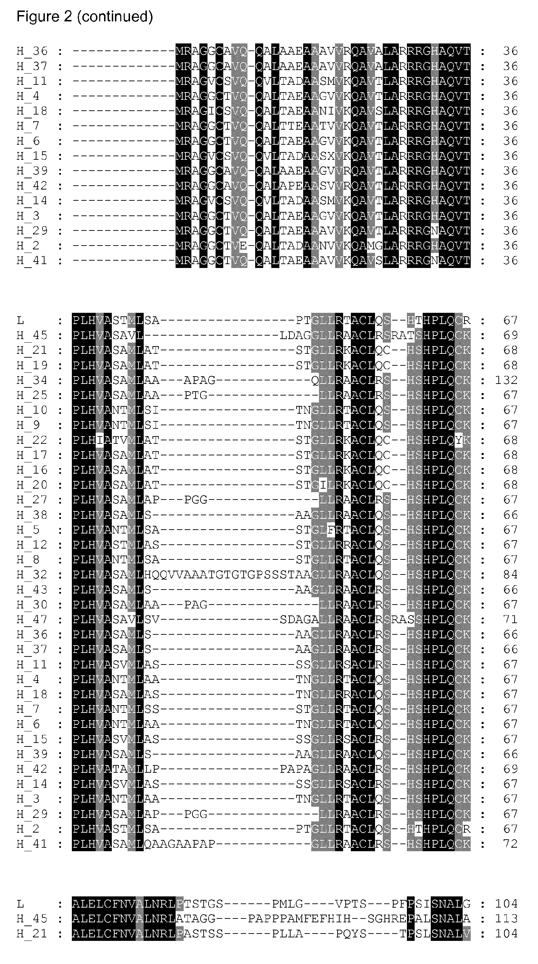

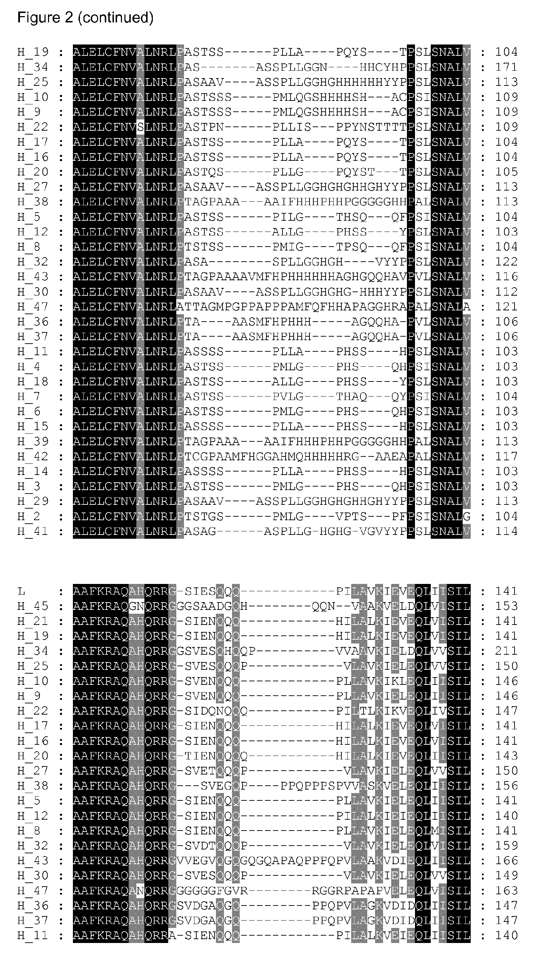

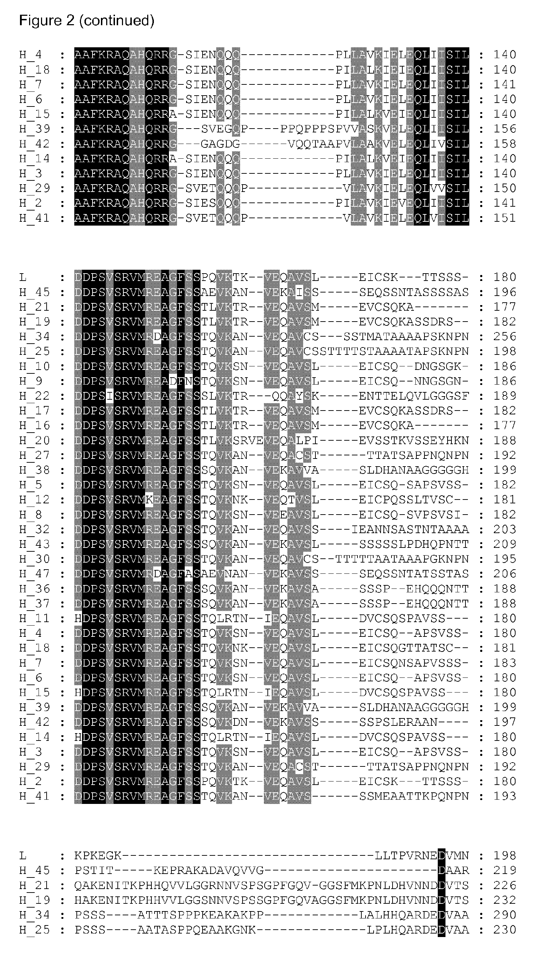

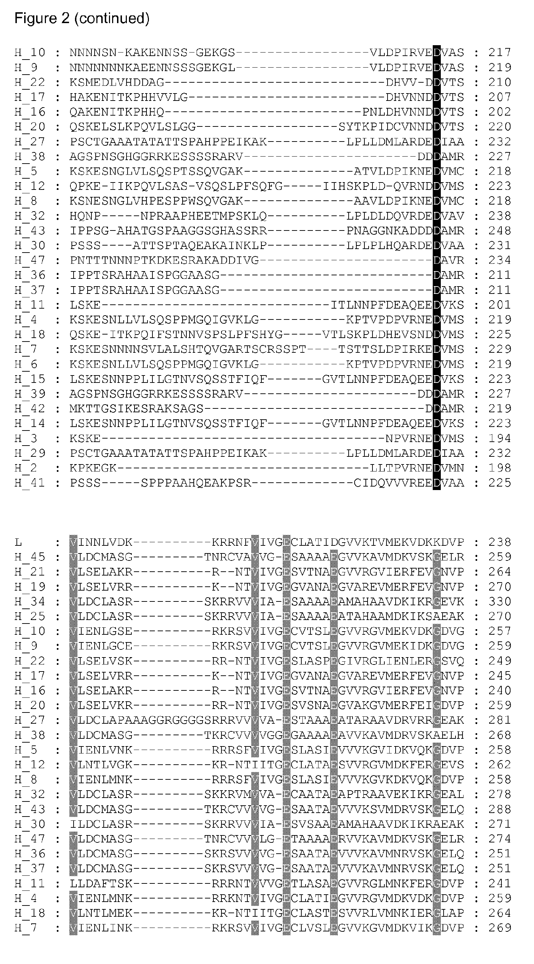

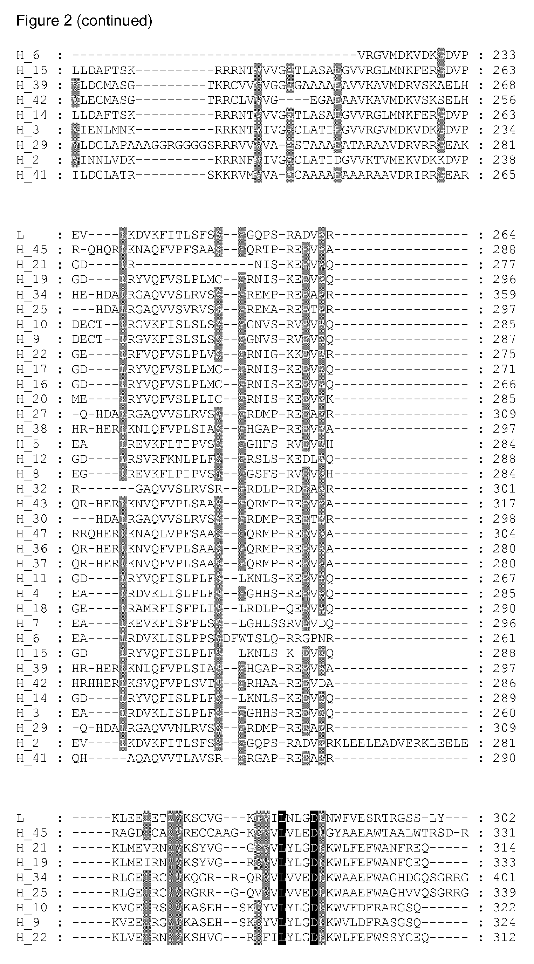

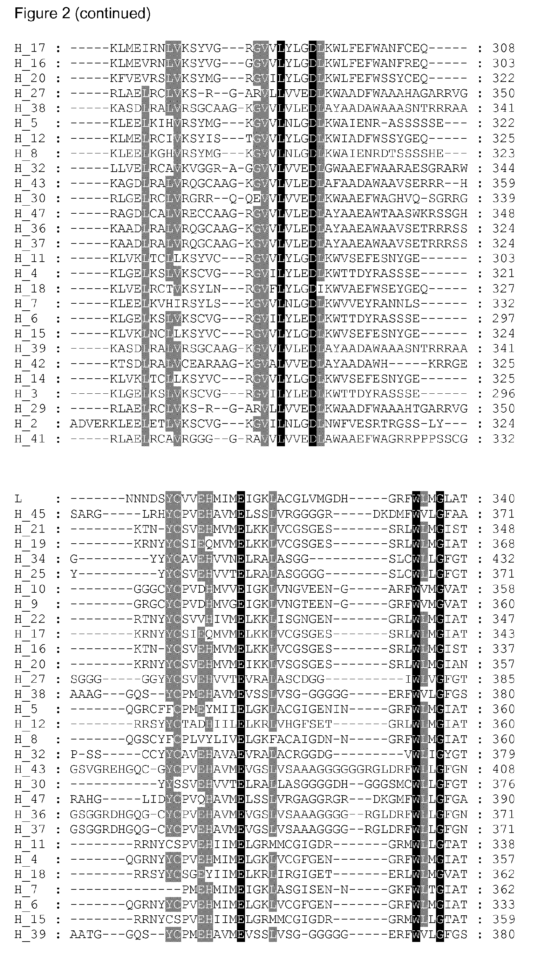

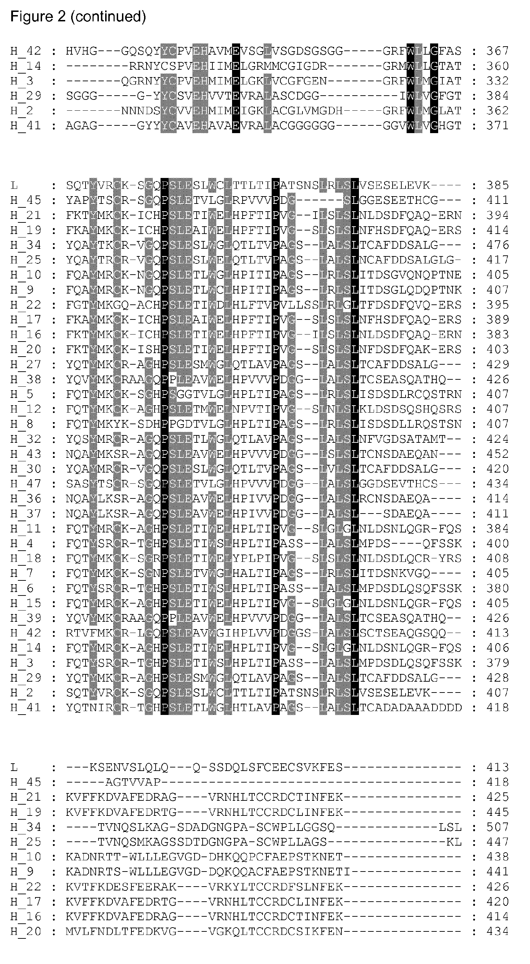

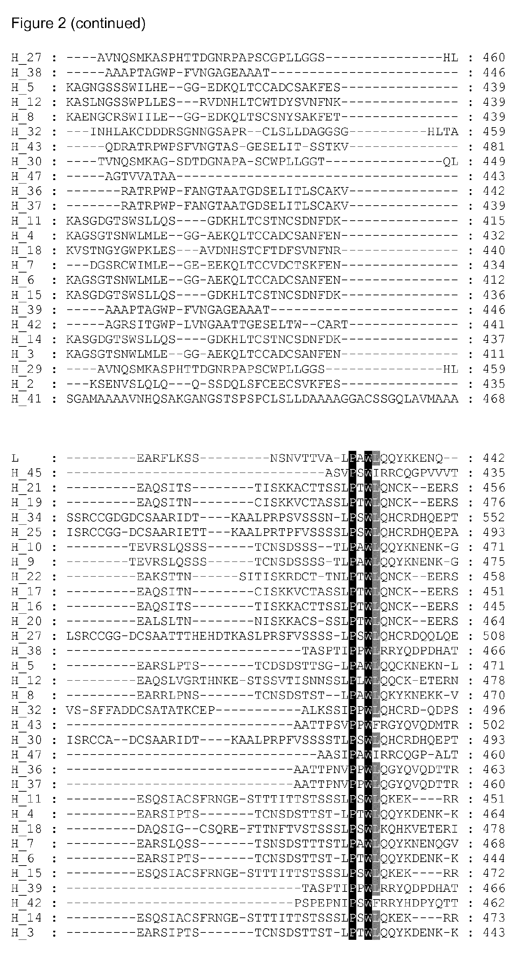

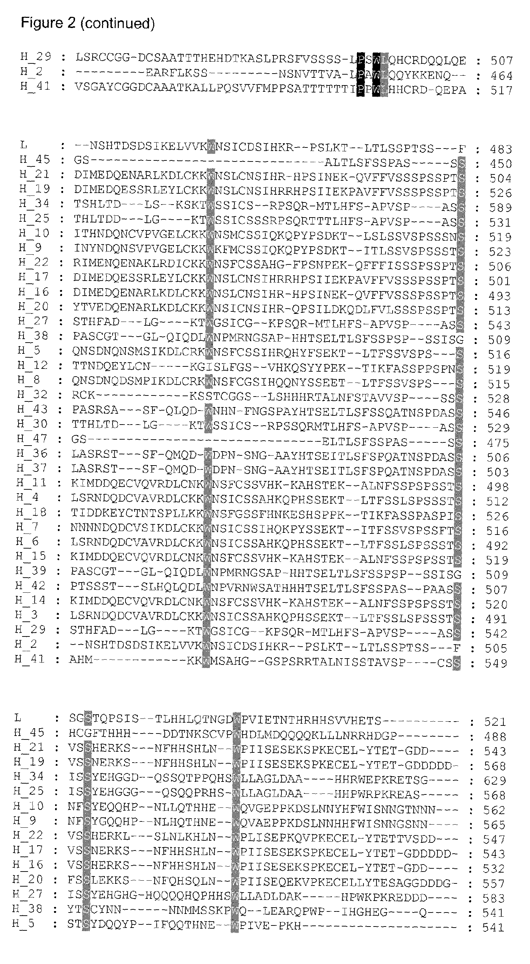

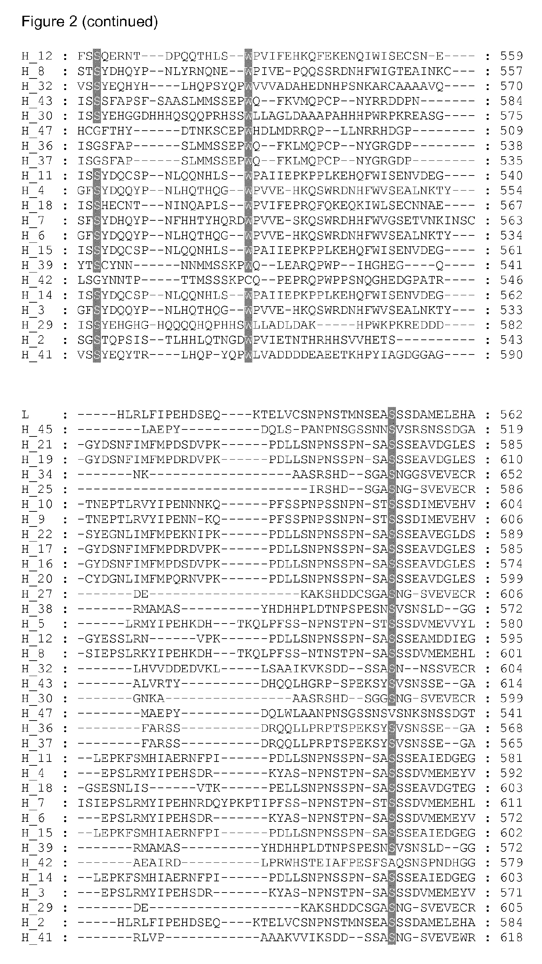

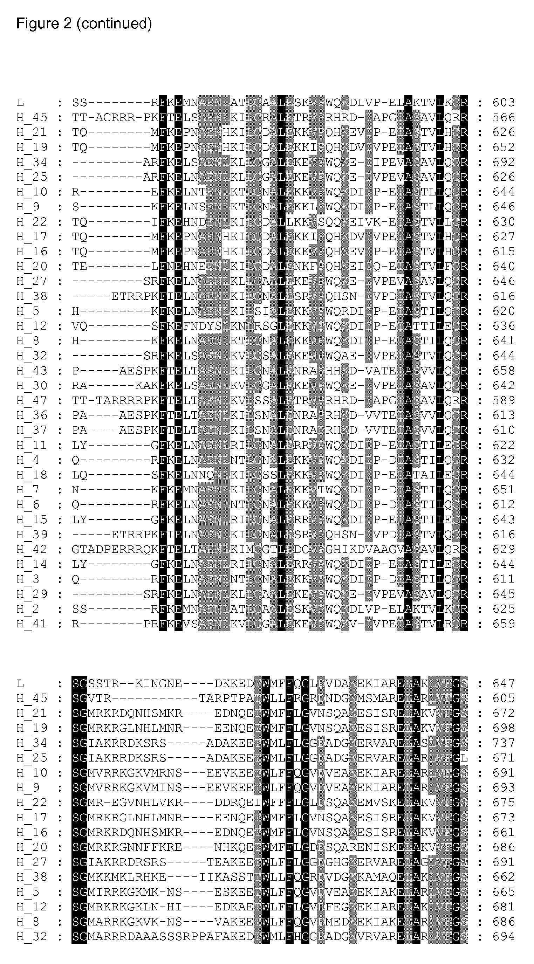

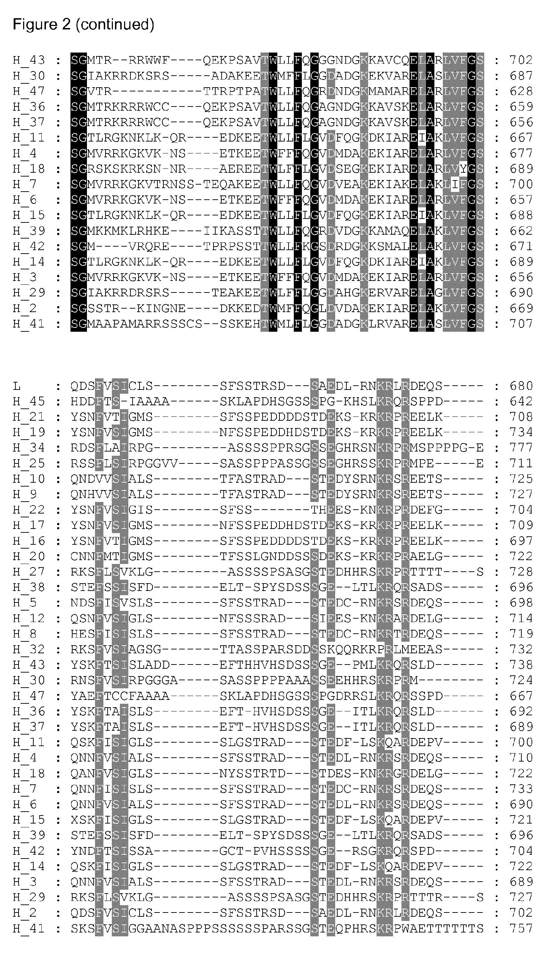

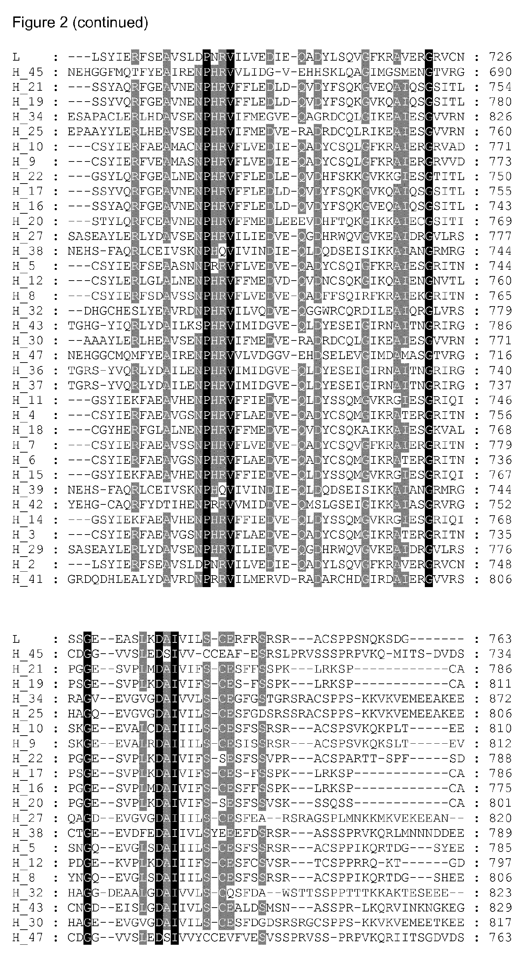

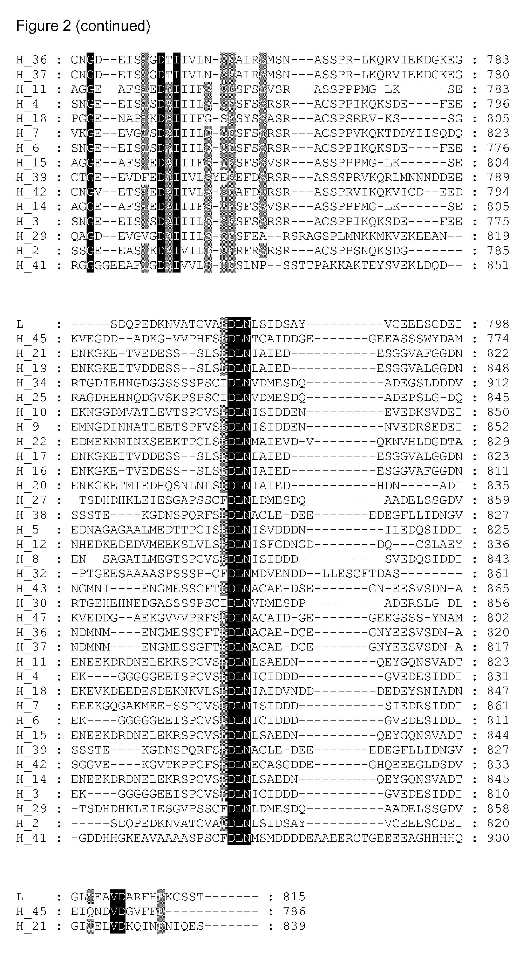

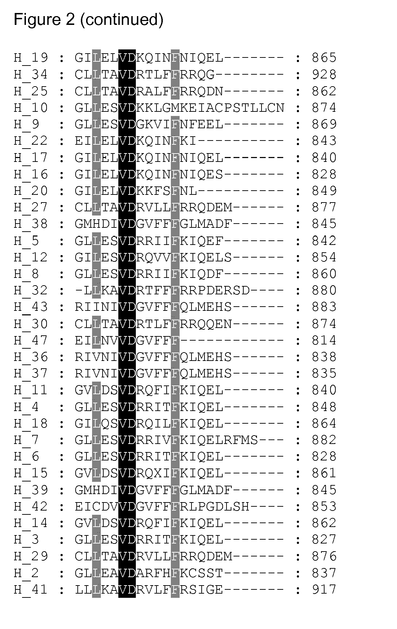

Preferably the polypeptides comprises one or more motifs and/ or domains as defined elsewhere herein.
The consensus sequence was derived using an alignment as shown in figure 2 and example 2 and deducing the consensus sequence.
In one embodiment the CLP polypeptide comprises the consensus sequence as given in SEQ ID NO: 75.
Motifs 1 to 6 as shown herein below were generated as described in examples 2 and 4.
In a further embodiment, the CLP polypeptide as used herein comprises at least one of the motifs 1, 2, 3, 4, 5 or 6 as defined herein below, wherein in the letter-numbers combination

—x(a,b)-

of any motif the letter x stands for Xaa, i.e. any amino acid, and the integer numbers a and b give the minimum and the maximum number of Xaa that may be found after the amino acid preceding the x. For example, S-x(0,3)-P indicates that following the amino acid Serine either one, two or three amino acids of any choice may be included before a Proline residue, or that no amino acid is to be found between the Serine and the Proline residue of this motif.
Consequently, the letters

-x(2)

indicate that exactly two amino acids of any type are found at this position of the motif. A single -x without a number in brackets- indicates that one amino acid residue of any type is present at this position of the motif.
Moreover any amino acid residue(s) replacing -x may be identical to or different from the amino acid residue preceding or succeeding it, or any other amino acid inserted instead of the -x at the same or any other position.
Residues within square brackets represent alternatives.
In a preferred embodiment the POI polypeptide comprises

1) all of the following motifs:

Motif 1 (SEQ ID NO: 76):

A-[AGLV]-K-x(0,2)-I-x(1,3)-Q-L-[IMV]-[IV]-S-I-L-[DH]-D-P-S-V-S-R-V-M-R-[DE]-A-[DG]-F-[NS]-S-[PST]-x-[LV]-[KR]-[ANST]-x-[IV]-E-x-[AE];

Motif 2 (SEQ ID NO: 77):
F-x-E-x-[NST]-[ADEST]-E-N-L-x(2)-L-[CS]-x-A-L-x(2)-[EKR]-x-[PST];

Motif 3 (SEQ ID NO: 78):

T-W-[FLM]-[FL]-F-x-G-x-[DGN]-x(2)-[ADG]-[KR]-x(2)-[IMV]-[ACS]-x-E-L-A-x-[LV]-V-F-G-S-x(3)-[FV]-x-[ACST]-x(3)-[ADGS]-[ADEGNST];

Motif 4(SEQ ID NO: 79):
G-x(1,2)-V-x(0,1)-L-x-[LV]-[EG]-D-L-x-[WY]-x(2)-[DE]-x(2)-[AST];

Motif 5 (SEQ ID NO: 80):
E-x(0,4)-L-x-[PT]-[FILV]-[ATV]-[IV]-P-[ADV]-[GST]
and

Motif 6 (SEQ ID NO: 81):
L-x-D-[AST]-I-[IV]-[IV]-x-[CGNS]-x-[AEQ];
or

2) the motifs according to 1) and in addition the consensus sequence as represented by the sequence listed under SEQ ID NO: 75; or
3) the motifs according to 1) or 2) above and in addition comprising all of the following consensus motifs
Consensus motif 1 (SEQ. ID NO: 83): HPLQC[KR]ALELCF[NS]VA
Consensus motif 2 (SEQ ID NO: 84): NAL[GV]AA[LF]KRAQAHQR
and
Consensus motif 3 (SEQ ID NO: 85) LDDPSVSRVMREA[GS]F;
or
4) any 5 of the motifs listed under 1) and all three consensus motifs listed under 3); or
5) any 4 of the motifs listed under 1) and all three consensus motifs listed under 3); or
6) any 4 of the motifs listed under 1) and any one of the consensus motifs listed under 3); or
7) any 3 of the motifs listed under 1); or
8) all of the motifs listed under 1) above, all three consensus motifs as listed under 3) above and in addition the Interpro motifs IPR004176 (Clp, N-terminal) and IPR023150 (Double Clp-N motif) and also the PFAM domain PF02861 as a split domain as described herein above; or
9) One of the motifs as described herein above.

In still another embodiment, the CLP polypeptide comprises in increasing order of preference, at least 2, at least 3, at least 4, at least 5 or all 6 motifs in addition to the consensus sequence as defined above.

Additionally or alternatively, the CLP protein has in increasing order of preference at least 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% overall sequence identity to the amino acid sequence represented by SEQ ID NO: 2, provided that the homologous protein comprises any one or more of the conserved motifs as outlined above. The overall sequence identity is determined using a global alignment algorithm, such as the Needleman Wunsch algorithm in the program GAP (GCG Wisconsin Package, Accelrys), preferably with default parameters and preferably with sequences of mature proteins (i.e. without taking into account secretion signals or transit peptides). In one embodiment the sequence identity level is determined by comparison of the polypeptide sequences over the entire length of the sequence of SEQ ID NO: 2. In another embodiment the sequence identity level of a nucleic acid sequence is determined by comparison of the nucleic acid sequence over the entire length of the coding sequence of the sequence of SEQ ID NO: 1.

In another embodiment, the sequence identity level is determined by comparison of one or more conserved domains or motifs in SEQ ID NO: 2 with corresponding conserved domains or motifs in other CLP polypeptides. Compared to overall sequence identity, the sequence identity will generally be higher when only conserved domains or motifs are considered. Preferably the motifs in a CLP polypeptide have, in increasing order of preference, at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to any one or more of the motifs represented by SEQ ID NO: 76 to SEQ ID NO: 81 (Motifs 1 to 6), one or more of the consensus motifs 1 to 3 as defined above (SEQ ID NO: 83 to 85) and/or the consensus sequence as represented by SEQ ID NO: 75. In other words, in another embodiment a method for enhancing one or more yield-related traits in plants is provided wherein said CLP polypeptide comprises a conserved domain with at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to the conserved domain starting with amino acid 11 up to amino acid 166 in SEQ ID NO:2.

The terms "domain", "signature" and "motif" are defined in the "definitions" section herein.

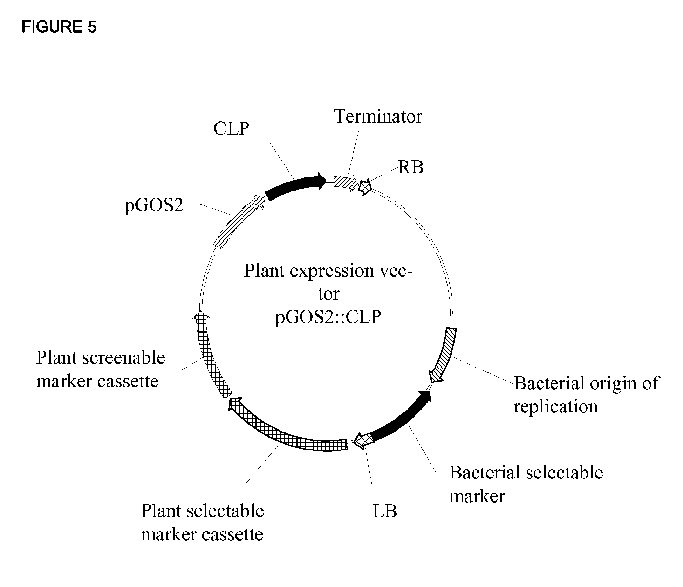

Preferably, the polypeptide sequence which when used in the construction of a phylogenetic tree, such as the one depicted in Figure 5, clusters with the group of CLP polypeptide comprising the amino acid sequence represented by SEQ ID NO: 2 rather than with any other group.

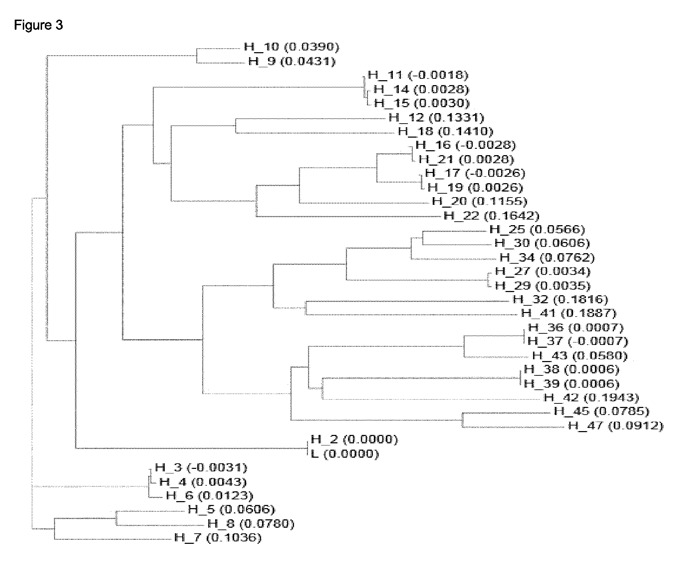

In another embodiment the polypeptides of the invention when used in the construction of a phylogenetic tree, such as the one depicted in Figure 3 cluster not more than 4, 3, or 2 hierarchical branch Points away from the amino acid sequence of SEQ ID NO: 2.

In one embodiment, CLP polypeptides (at least in their native form) typically possess ATP binding and /or ATP-dependent protease activity. Tools and techniques for measuring ATP binding or ATP-dependent protease activity are well known in the art. In addition, nucleic acids encoding CLP polypeptides, when expressed in rice according to the methods of the present invention as outlined in Examples 7 and 9, give plants having increased yield related traits under normal or drought conditions, in particular increased aboveground biomass, increased height of the centre of gravity of the plant and particularly increased seed yield. Another function of the nucleic acid sequences encoding CLP polypeptides is to confer information for synthesis of the CLP protein that increases yield or yield related traits as described herein, when such a nucleic acid sequence of the invention is transcribed and translated in a living plant cell.

The present invention is illustrated by transforming plants with the nucleic acid sequence represented by SEQ ID NO: 1, encoding the polypeptide sequence of SEQ ID NO: 2. However, performance of the invention is not restricted to these sequences; the methods of the invention may advantageously be performed using any DDLLP-encoding nucleic acid or CLP polypeptide as defined herein. The term "CLP" or "CLP polypeptide" as used herein also intends to include homologues as defined hereunder of SEQ ID NO: 2.

Examples of nucleic acids encoding CLP polypeptides are given in Table A of the Examples section herein. Such nucleic acids are useful in performing the methods of the invention. The amino acid sequences given in Table A of the Examples section are example sequences of orthologues and paralogues of the CLP polypeptide represented by SEQ ID NO: 2, the terms "orthologues" and "paralogues" being as defined herein. Further orthologues and paralogues may readily be identified by performing a so-called reciprocal blast search as described in the definitions section; where the query sequence is SEQ ID NO: 1 or SEQ ID NO: 2, the second BLAST (back-BLAST) would be against tomato sequences.

The invention also provides hitherto unknown DDLLP-encoding nucleic acids and CLP polypeptides useful for conferring one or more enhanced yield-related traits in plants relative to control plants.

According to a further embodiment of the present invention, there is therefore provided an isolated nucleic acid molecule selected from the group consisting of:

(i) a nucleic acid represented by any SEQ ID NO shown in table A;
(ii) the complement of a nucleic acid represented by any SEQ ID NO shown in table A;
(iii) a nucleic acid encoding a CLP polypeptide having in increasing order of preference at least 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to any amino acid sequence of table A and additionally or alternatively comprising one or more motifs having in increasing order of preference at least 50%, 55%, 60%, 65%, 70%, 75%, 80%, 85%, 90%, 95%, 96%, 97%, 98%, 99% or more sequence identity to any one or more of the motifs given in SEQ ID NO: 76 to SEQ ID NO: 81 and/or one or more of the consensus motifs 1 to 3 as defined above (SEQ ID NO: 83 to 85), and further preferably conferring one or more enhanced yield-related traits relative to control plants; and
(iv) a nucleic acid molecule which hybridizes with a nucleic acid molecule of (i) to (iii) under high stringency hybridization conditions and preferably confers one or more enhanced yield-related traits relative to control plants.

According to a further embodiment of the present invention, there is also provided an isolated polypeptide selected from the group consisting of:

(i) an amino acid sequence represented by any SEQ ID NO shown in table A;
(ii) an amino acid sequence having, in increasing order of preference, at least 50%, 51 %, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%. 78%. 79%, 80%, 81 %, 82%, 83%, 84%, 85%. 86%, 87%, 88%, 89%, 90%, 91%, 92%, 93%, 94%, 95%, 96%, 97%, 98%, or 99% sequence identity to the amino acid sequence represented by any SEQ ID NO shown in table A, and additionally or alternatively comprising one or more motifs having in increasing order of preference at least 50%, 55%, 60%, 65%, 70%, 75%, 80%, 85%, 90%, 95%, 96%, 97%, 98%, 99% or more sequence identity to any one or more of the motifs given in SEQ. ID NO: 76 to SEQ ID NO: 81 and/or one or more of the consensus motifs 1 to 3 as defined above (SEQ ID NO: 83 to 85), and further preferably conferring one or more enhanced yield-related traits relative to control plants, and

(iii) derivatives of any of the amino acid sequences given in (i) or (ii) above.

In one embodiment the nucleic acids useful in the methods of the invention are those listed in Tables I as lead or homologue, or those encoding the protein sequences listed in tables II as lead or homologues, or those comprising the consensus sequence and the patterns shown in table IV.

Nucleic add variants may also be useful in practising the methods of the invention. Examples of such variants include nucleic acids encoding homologues and derivatives of any one of the amino acid sequences given in Table A of the Examples section, the terms "homologue" and "derivative" being as defined herein. Also useful in the methods, constructs, plants, harvestable parts and products of the invention are nucleic acids encoding homologues and derivatives of orthologues or paralogues of any one of the amino acid sequences given in Table A of the Examples section. Homologues and derivatives useful in the methods of the present invention have substantially the same biological and functional activity as the unmodified protein from which they are derived. Further variants useful in practising the methods of the invention are variants in which codon usage is optimised or in which miRNA target sites are removed.

Further nucleic acid variants useful in practising the methods of the invention include portions of nucleic acids encoding CLP polypeptides, nucleic acids hybridising to nucleic acids encoding CLP polypeptides, splice variants of nucleic acids encoding CLP polypeptides, allelic variants of nucleic acids encoding CLP polypeptides and variants of nucleic acids encoding CLP polypeptides obtained by gene shuffling, The terms hybridising sequence, splice variant, allelic variant and gene shuffling are as described herein.

Nucleic acids encoding CLP polypeptides need not be full-length nucleic acids, since performance of the methods of the invention does not rely on the use of full-length nucleic acid sequences. According to the present invention, there is provided a method for enhancing one or more yield-related traits in plants, comprising introducing, preferably by recombinant methods, and expressing in a plant a portion of any one of the nucleic acid sequences given in Table A of the Examples section, or a portion of a nucleic acid encoding an orthologue, paralogue or homologue of any of the amino acid sequences given in Table A of the Examples section.

A portion of a nucleic acid may be prepared, for example, by making one or more deletions to the nucleic acids. The portions may be used in isolated form or they may be fused to other coding (or non-coding) sequences in order to, for example, produce a protein that combines several activities. When fused to other coding sequences, the resultant polypeptide produced upon translation may be bigger than that predicted for the protein portion.

Portions useful in the methods, constructs, plants, harvestable parts and products of the invention, encode a CLP polypeptide as defined herein or at least part thereof, and have substantial the same biological activity as the amino acid sequences given in Table A of the Examples section. Preferably, the portion is a portion of any one of the nucleic acids given in Table A of the Examples section, or is a portion of a nucleic acid encoding an orthologue or paralogue of any one of the amino acid sequences given in Table A of the Examples section. Preferably the portion is at least 500, 550, 600, 650, 700, 750, 800, 850, 900,950,1000,1050.1100,1150,1200,1250,1300.1350,1400,1450,1500,1550,1600, 1650,1700,1750,1800,1850,1900,1950,2000,2050,2100,2150,2200,2250,2300, 2350, 2400 or 2048 consecutive nucleotides in length, the consecutive nucleotides being of any one of the nucleic acid sequences given in Table A of the Examples section, or of a nucleic acid encoding an orthologue or paralogue of any one of the amino acid sequences given in Table A of the Examples section. Most preferably the portion is a portion of the nucleic acid of SEQ ID NO_ 1. Preferably, the portion encodes a fragment of an amino acid sequence which comprises motifs 1 to 6, and more preferably in addition the consensus motifs 1 to 3 and the consensus sequence as defined herein above and/or has biological activity of ATP-dependent protease, and/or has at least 50%, 60 %, 70%, 75%, 80%, 85%, 90%, 93%, 95%, 97% or 99% sequence identity to SEQ ID NO: 2.

Another nucleic acid variant useful in the methods, constructs, plants, harvestable parts and products of the invention is a nucleic acid capable of hybridising, under reduced stringency conditions, preferably under stringent conditions, with a nucleic acid encoding a CLP polypeptide as defined herein, or with a portion as defined herein. According to the present invention, there is provided a method for enhancing one or more yield-related traits in plants, comprising introducing, preferably by recombinant methods, and expressing in a plant a nucleic acid capable of hybridizing to the complement of a nucleic acid encoding any one of the proteins given in Table A of the Examples section, or to the complement of a nucleic acid encoding an orthologue, paralogue or homologue of any one of the proteins given in Table A.

Hybridising sequences useful in the methods, constructs, plants, harvestable parts and products of the invention encode a CLP polypeptide as defined herein, having substantially the same biological activity as the amino acid sequences given in Table A of the Examples section. Preferably, the hybridising sequence is capable of hybridising to the complement of a nucleic acid encoding any one of the proteins given in Table A of the Examples section, or to a portion of any of these sequences, a portion being as defined herein, or the hybridising sequence is capable of hybridising to the complement of a nucleic encoding an orthologue or paralogue of any one of the amino acid sequences given in Table A of the Examples section. Most preferably, the hybridising sequence is capable of hybridising to the complement of a nucleic acid encoding the polypeptide as represented by SEQ ID NO: 2 or to a portion thereof. In one embodiment, the hybridization conditions are of medium stringency, preferably of high stringency, as defined herein.

Preferably, the hybridising sequence encodes a polypeptide with an amino acid sequence which comprises motifs to 6, and more preferably the consensus motifs 1 to 3 and the consensus sequence as defined herein above and/or has biological activity of ATP-dependent protease, and/or has at least 50%, 60 % , 70%, 75%, 80%, 85%, 90%, 93%, 95%, 97% or 99% sequence identity to SEQ ID NO: 2.

In another embodiment, there is provided a method for enhancing one or more yield-related traits in plants, comprising introducing, preferably by recombinant methods, and expressing traits in plants, comprising introducing, preferably by recombinant methods, and expressing in a plant a splice variant of a nucleic acid encoding any one of the proteins given in Table A of the Examples section, or a splice variant of a nucleic acid encoding an orthologue, paralogue or homologue of any of the amino acid sequences given in Table A of the Examples section.

Preferred splice variants are splice variants of a nucleic acid represented by SEQ ID NO: 1, or a splice variant of a nucleic acid encoding an orthologue or paralogue of SEQ ID NO: 2. Preferably, the amino acid sequence encoded by the splice variant comprises motifs 1 to 6, and more preferably the consensus motifs 1 to 3 and the consensus sequence as defined herein above and/or has biological activity of ATP-dependent protease, and/or has at least 50%, 60 % , 70%, 75%, 80%, 85%, 90%, 93%, 95%, 97% or 99% sequence identity to SEQ ID NO: 2.

In yet another embodiment, there is provided a method for enhancing one or more yield-related traits in plants, comprising introducing, preferably by recombinant methods, and expressing in a plant an allelic variant of a nucleic acid encoding any one of the proteins given in Table A of the Examples section, or comprising introducing, preferably by recombinant methods, and expressing in a plant an allelic variant of a nucleic acid encoding an orthologue, paralogue or homologue of any of the amino acid sequences given in Table A of the Examples section.

The polypeptides encoded by allelic variants useful in the methods of the present invention have substantially the same biological activity as the CLP polypeptide of SEQ ID NO: 2 and any of the amino acid sequences depicted in Table A of the Examples section. Allelic variants exist in nature, and encompassed within the methods of the present invention is the use of these natural alleles. Preferably, the allelic variant is an allelic variant of SEQ ID NO: 1 or an allelic variant of a nucleic acid encoding an orthologue or paralogue of SEQ ID NO: 2. Preferably, the amino acid sequence encoded by the allelic variant comprises motifs 1 to 6, and more preferably the consensus motifs 1 to 3 and the consensus sequence as defined herein above and/or has biological activity of ATP-dependent protease, and/or has at least 50%, 60 % , 70%, 75%, 80%, 85%, 90%, 93%, 95%, 97% or 99% sequence identity to SEQ ID NO: 2.

In yet another embodiment, there is provided a method for enhancing one or more yield-related traits in plants, comprising introducing, preferably by recombinant methods, and expressing in a plant a variant of a nucleic acid encoding any one of the proteins given in Table A of the Examples section, or comprising introducing, preferably by recombinant methods, and expressing in a plant a variant of a nucleic acid encoding an orthologue, paralogue or homologue of any of the amino acid sequences given in Table A of the Examples section, which variant nucleic acid is obtained by gene shuffling.

Preferably, the amino acid sequence encoded by the variant nucleic acid obtained by gene shuffling comprises motifs 1 to 6, and more preferably the consensus motifs 1 to 3 and the consensus sequence as defined herein above and/or has biological activity of ATP-dependent protease, and/or has at least 50%, 60 %, 70%, 75%, 80%, 85%, 90%, 93%, 95%, 97% or 99% sequence identity to SEQ ID NO: 2.

Furthermore, nucleic acid variants may also be obtained by site-directed, mutagenesis. Several methods are available to achieve site-directed mutagenesis, the most common being PCR based methods (Current Protocols in Molecular Biology. Wiley Eds.). CLP polypeptides differing from the sequence of SEQ ID NO- 2 by one or several amino acids (substitutions), insertion(s) and/or deletion(s) as defined herein) may equally be useful to increase the yield of plants in the methods and constructs and plants of the invention..

Nucleic acids encoding CLP polypeptides may be derived from any natural or artificial source. The nucleic acid may be modified from its native form in composition and/or genomic environment through deliberate human manipulation. Preferably the CLP polypeptide-encoding nucleic acid is from a plant, further preferably from a dicotyledonous plant, more preferably from the family Brassicaceae, most preferably the nucleic acid is from *Arabidopsis* species, in particular from *Arabidopsis thaliana.*

In another embodiment the present invention extends to recombinant chromosomal DNA comprising a nucleic acid sequence useful in the methods of the invention, wherein said nucleic acid is present in the chromosomal DNA as a result of recombinant methods, but is not in its natural genetic environment. In a further embodiment the recombinant chromosomal DNA of the invention is comprised in a plant cell. DNA comprised within a cell, particularly a cell with cell walls like a plant cell, is better protected from degradation than a bare nucleic acid sequence. The same holds true for a DNA construct comprised in a host cell, for example a plant cell.

Performance of the methods of the invention gives plants having one or more enhanced yield-related traits. In particular performance of the methods of the invention gives plants having increased early growth and/or increased yield, especially increased biomass and/or increased seed yield relative to control plants. The terms "early vigour" "yield" and "seed yield" are described in more detail in the "definitions" section herein.

The present invention thus provides a method, for increasing yield-related traits, especially biomass and/or seed yield of plants, relative to control plants, which method comprises modulating expression in a plant of a nucleic acid encoding a CLP polypeptide as defined herein.

According to a preferred feature of the present invention, performance of the methods of the invention gives plants having an increased growth rate relative to control plants. Therefore, according to the present invention, there is provided a method for increasing the growth rate of plants, which method comprises modulating expression in a. plant of a nucleic acid encoding a CLP polypeptide as defined herein.

Performance of the methods of the invention gives plants grown under non-stress conditions or under drought conditions increased yield-related traits, preferably biomass and/or seed yield, relative to control plants grown under comparable conditions. Therefore, according to the present invention, there is provided a method for increasing yield-related traits, preferably biomass and/or seed yield, in plants grown under non-stress conditions or under drought conditions, which method comprises modulating expression in a plant of a nucleic acid encoding a CLP polypeptide.

In one embodiment of the invention, root biomass is increased, preferably and/or taproot biomass, more preferably in sugar beet plants, but seed yield and/or above ground biomass are not increased.

In another embodiment of the invention, above ground biomass is increased, preferably stem, stalk and/or sett biomass, more preferably in Poaceae, even more preferably in a Saccharum species, most preferably in sugarcane, yet seed yield and/or root growth is not increased.

In a further embodiment the total harvestable sugar, preferably glucose, fructose and/or sucrose, is increased, preferably in addition to increased other yield-related traits as defined herein, for example biomass, and more preferably also in addition to an increase in sugar content, preferably glucose, fructose and/or sucrose content.

The invention also provides genetic constructs and vectors to facilitate introduction and/or expression in plants of nucleic acids encoding CLP polypeptides. The gene constructs may be inserted into vectors, which may be commercially available, suitable for transforming into plants or host cells and suitable for expression of the gene of interest in the transformed cells. The invention also provides use of a gene construct as defined herein in the methods of the invention.

More specifically, the present invention provides a construct comprising:

(a) a nucleic acid encoding a CLP polypeptide as defined above;
(b) one or more control sequences capable of driving expression of the nucleic acid sequence of (a); and optionally
(c) a transcription termination sequence.

Preferably, the nucleic acid encoding a CLP polypeptide is as defined above. The term "control sequence" and "termination sequence" are as defined herein.

In particular the genetic construct of the invention is a plant expression construct, i.e. a genetic construct that allows for the expression of the nucleic acid encoding a CLP in a plant, plant cell or plant tissue after the construct has been introduced, preferably by recombinant means. The plant expression construct may for example comprise said nucleic acid encoding a CLP in functional linkage to a promoter and optionally other control sequences controlling the expression of said nucleic acid in one or more plant cells, wherein the promoter and optional the other control sequences are not natively found in functional linkage to said nucleic acid. In a preferred embodiment the control sequence(s) including the promoter result in overexpression of said nucleic acid when the construct of the invention has been introduced into a plant, plant cell or plant tissue.

The genetic construct of the invention may be comprised in a host cell - for example a plant cell - seed, agricultural product or plant. Plants or host cells are transformed with a genetic construct such as a vector or an expression cassette comprising any of the nucleic acids described above. Thus the invention furthermore provides plants or host cells transformed with a construct as described above. In particular, the invention provides plants transformed with a construct as described above, which plants have increased yield-related traits as described herein.

In one embodiment the genetic construct of the invention confers increased yield or yield related traits(s) to a plant when it has been introduced into said plant, which plant expresses the nucleic acid encoding the CLP polypeptide comprised in the genetic construct and preferably resulting in increased abundance of the CLP polypeptide. In another embodiment the genetic construct of the invention confers increased yield or yield related traits(s) to a plant comprising plant cells in which the construct has been introduced, which plant cells the nucleic acid encoding the CLP comprised in the genetic construct.

The promoter in such an genetic construct may be a non-native promoter to the nucleic acid described above, i,e, a promoter not regulating the expression of said nucleic acid in its native surrounding.

In a preferred embodiment the nucleic acid encoding the CLP polypeptide useful in the methods, constructs, plants, harvestable parts and products of the invention is in functional linkage to a promoter resulting in the expression of said nucleic acid encoding a CLP polypeptide in

- aboveground biomass preferably the leaves and shoot, more preferably the stem, of monocot plants, preferably Poaceae plants, more preferably Saccharum species plants, AND/OR
- leaves, belowground biomass and/or root biomass, preferably tubers, taproots and/or beet organs, more preferably taproot and beet organs of dicot plants, more preferably Solanaceae and/or Beta species plants.

The expression cassettes or the genetic construct of the invention may be comprised in a host cell, plant cell, seed, agricultural product or plant.

The skilled artisan is well aware of the genetic elements that must be present on the genetic construct in order to successfully transform, select and propagate host cells containing the sequence of interest. The sequence of interest is operably linked to one or more control sequences (at least to a promoter).

Advantageously, any type of promoter, whether natural or synthetic, may be used to drive expression of the nucleic acid sequence, but preferably the promoter is of plant origin. A constitutive promoter is particularly useful in the methods. See the "Definitions" section herein for definitions of the various promoter types.

The constitutive promoter is preferably a ubiquitous constitutive promoter of medium strength. More preferably it is a plant derived promoter, e.g. a promoter of plant chromosomal origin, such as a GOS2 promoter or a promoter of substantially the same strength and having substantially the same expression pattern (a functionally equivalent promoter), more preferably the promoter is the promoter GOS2 promoter from rice. Further preferably the constitutive promoter is represented by a nucleic acid sequence substantially similar to SEQ ID NO: 82, most preferably the constitutive promoter is as represented by SEQ ID NO: 82. See the "Definitions" section herein for further examples of constitutive promoters.

It should be clear that the applicability of the present invention is not restricted to the CLP polypeptide-encoding nucleic acid represented by SEQ ID NO: 1, nor is the applicability of the invention restricted to the rice GOS2 promoter when expression of a CLP polypeptide-encoding nucleic acid is driven by a constitutive promoter.

Yet another embodiment relates to the nucleic acid sequences useful in the methods, constructs, plants, harvestable parts and products of the invention and encoding CLP polypeptides of the invention functionally linked a promoter as disclosed herein above and further functionally linked to one or more

1) nucleic acid expression enhancing nucleic acids (NEENAs):

a) as disclosed in the international patent application published as WO2011/023537 in table 1 on page 27 to page 28 and/or SEQ ID NO: 1 to 19 and/or as defined in items i) to vi) of claim 1 of said international application which NEENAs are herewith incorporated by reference; and/or
b) as disclosed in the international patent application published as WO2011/023539 in table 1 on page 27 and/or SEQ ID NO: 1 to 19 and/or as defined in items i) to vi) of claim 1 of said international application which NEENAs are herewith incorporated by reference; and/or
c) and/or as contained in or disclosed in:

i) the European priority application filed on 05 July 2011 as EP 11172672.5 in table 1 on page 27 and/or SEQ ID NO: 1 to 14937, preferably SEQ ID NO: 1 to 5, 14936 or 14937, and/or as defined in items i) to v) of claim 1 of said European priority application which NEENAs are herewith incorporated by reference; and/or
ii) the European priority application filed on 06 July 2011 as EP 11172825.9 in table 1 on page 27 and/or SEQ ID NO: 1 to 65560, preferably SEQ ID NO: 1 to 3, and/or as defined in items i) to v) of claim 1 of said European priority application which NEENAs are herewith incorporated by reference;

d) or equivalents having substantially the enhancing effect;

2) and/or functionally linked to one or more Reliability Enhancing Nucleic Acid (RENA) molecule

a) as contained in or disclosed in the European priority application filed on 15 September 2011 as EP 11181420.8 in table 1 on page 26 and/or SEQ ID NO: 1 to 16 or 94 to 116666, preferably SEQ ID NO: 1 to 16, and/or as defined in point i) to v) of item a) of claim 1 of said European priority application which RENA molecule(s) are herewith incorporated by reference;
b) or equivalents having substantially the enhancing effect.

The term "functional linkage" or "functionally linked" is to be understood as meaning, for example, the sequential arrangement of a regulatory element (e.g. a promoter) with a nucleic acid sequence to be expressed and, if appropriate, further regulatory elements (such as e.g., a terminator, NEENA or a RENA) in such a way that each of the regulatory elements can fulfil its intended function to allow, modify, facilitate or otherwise influence expression of said nucleic acid sequence. As a synonym the wording "operable linkage" or "operably linked" may be used. The expression may result depending on the arrangement of the nucleic acid sequences in relation to sense or antisense RNA. To this end, direct linkage in the chemical sense is not necessarily required. Genetic control sequences such as, for example, enhancer sequences, can also exert their function on the target sequence from positions which are further away, or indeed from other DNA molecules. Preferred arrangements are those is which the nucleic acid sequence to be expressed recombinantly is positioned behind the sequence acting as promoter, so that the two sequences are linked covalently to each other. The distance between the promoter sequence and the nucleic acid sequence to be expressed recombinantly is preferably less than 200 base pairs, especially preferably less than 100 base pairs, very especially preferably less than 50 base pairs. In a preferred embodiment, the nucleic acid sequence to be transcribed is located behind the promoter in such a way that the transcription start is identical with the desired beginning of the chimeric RNA of the invention. Functional linkage, and an expression construct, can be generated by means of customary recombination and cloning techniques as described (e.g., in Maniatis T, Fritsch EF and Sambrook J (1989) Molecular Cloning: A Laboratory Manual, 2nd Ed., Cold Spring Harbor Laboratory, Cold Spring Harbor (NY); Silhavy et al. (1984) Experiments with Gene Fusions, Cold Spring Harbor Laboratory, Cold Spring Harbor (NY); Ausubel et al. (1987) Current Protocols in Molecular Biology, Greene Publishing Assoc. and Wiley Interscience; Gelvin et al. (Eds) (1990) Plant Molecular Biology Manual; Kluwer Academic Publisher, Dordrecht, The Netherlands). However, further sequences, which, for example, act as a linker with specific cleavage sites for restriction enzymes, or as a signal peptide, may also be positioned between the two sequences. The insertion of sequences may also lead to the expression of fusion proteins. Preferably, the expression construct, consisting of a linkage of a regulatory region for example a promoter and nucleic acid sequence to be expressed, can exist in a vector-integrated form and be inserted into a plant genome, for example by transformation.

A preferred embodiment of the invention relates to a nucleic acid molecule useful in the methods, constructs, plants, harvestable parts and products of the invention and encoding a CLP polypeptide of the invention under the control of a promoter as described herein above, wherein the NEENA, RENA and/or the promoter is heterologous to said nucleic acid molecule encoding a CLP polypeptide of the invention.

Optionally, one or more terminator sequences may be used in the construct introduced into a plant. Those skilled in the art will be aware of terminator sequences that may be suitable for use in performing the invention. Preferably, the construct comprises an expression cassette comprising a GOS2 promoter, substantially similar to SEQ ID NO: 82, operably linked to the nucleic acid encoding the CLP polypeptide. Furthermore, one or more sequences encoding selectable markers may be present on the construct introduced into a plant.

According to a preferred feature of the invention, the modulated expression is increased expression. Methods for increasing expression of nucleic acids or genes, or gene products, are well documented in the art and examples are provided in the definitions section.

As mentioned above, a preferred method for modulating expression of a nucleic acid encoding a CLP polypeptide is by introducing, preferably by recombinant methods, and expressing in a plant a nucleic acid encoding a CLP polypeptide; however the effects of performing the method, i.e. enhancing one or more yield-related traits may also be achieved using other well-known techniques, including but not limited to T-DNA activation tagging, TILLING, homologous recombination. A description of these techniques is provided in the definitions section.

The invention also provides a method for the production of transgenic plants having one or more enhanced yield-related traits relative to control plants, comprising introduction and expression in a plant of any nucleic acid encoding a CLP polypeptide as defined herein.

More specifically, the present invention provides a method for the production of transgenic plants having one or more enhanced yield-related traits, particularly increased biomass, preferably aboveground and/or root biomass, and/or seed yield, which method comprises:

(i) introducing, preferably by recombinant methods, and expressing in a plant or plant cell a CLP polypeptide-encoding nucleic acid or a genetic construct comprising a CLP polypeptide-encoding nucleic acid; and
(ii) cultivating the plant cell under conditions promoting plant growth and development.

The nucleic acid of (i) may be any of the nucleic acids capable of encoding a CLP polypeptide as defined herein.

Cultivating the plant cell under conditions promoting plant growth and development, may or may not include regeneration and/or growth to maturity. Accordingly, in a particular embodiment of the invention, the plant cell transformed by the method according to the invention is regenerate into a transformed plant. In another particular embodiment, the plant cell transformed by the method according to the invention is not regenerate into a transformed plant, i.e. cells that are not capable to regenerate into a plant using cell culture techniques known in the art. While plants cells generally have the characteristic of totipotency, some plant cells can not be used to regenerate or propagate intact plants from said cells. In one embodiment of the invention the plant cells of the invention are such cells. In another embodiment the plant cells of the invention are plant cells that do not sustain themselves in an autotrophic way. One example are plant cells that do not sustain themselves through photosynthesis by synthesizing carbohydrate and protein from such inorganic substances as water, carbon dioxide and mineral salt.

The nucleic acid may be introduced directly into a plant cell or into the plant itself (including introduction into a tissue, organ or any other part of a plant). According to a preferred feature of the present invention, the nucleic acid is preferably introduced into a plant or plant cell by transformation. The term "transformation" is described in more detail in the "definitions" section herein.

In one embodiment the method for the production of a transgenic plant, transgenic plant part, or transgenic plant cell having one or more enhanced yield-related traits relative to control plants, comprises the step of harvesting setts from the transgenic plant and planting the setts and growing the setts to plants, wherein the setts comprises the exogenous nucleic acid encoding the DDLL polypeptide and the promoter sequence operably linked thereto.

In one embodiment the present invention extends to any plant cell or plant produced by any of the methods described herein, and to all plant parts and propagules thereof.

The present invention encompasses plants or parts thereof (including seeds) obtainable by the methods according to the present invention. The plants or plant parts or plant cells comprise a nucleic acid transgene encoding a CLP polypeptide as defined above, preferably in a genetic construct such as an expression cassette. The present invention extends further to encompass the progeny of a primary transformed or transfected cell, tissue, organ or whole plant that has been produced by any of the aforementioned methods, the only requirement being that progeny exhibit the same genotypic and/or phenotypic characteristic(s) as those produced by the parent in the methods according to the invention.

In a further embodiment the invention extends to seeds recombinantly comprising the expression cassettes of the invention, the genetic constructs of the invention, or the nucleic acids encoding the CLP and/or the CLP polypeptides as described above.

The invention also includes host cells containing an isolated nucleic acid encoding a CLP polypeptide as defined above. In one embodiment host cells according to the invention are plant cells, yeasts, bacteria or fungi. Host plants for the nucleic acids, construct, expression cassette or the vector used in the method according to the invention are, in principle, advantageously all plants which are capable of synthesizing the polypeptides used in the inventive method. In a particular embodiment the plant cells of the invention overexpress the nucleic acid molecule of the invention.

The methods of the invention are advantageously applicable to any plant, in particular to any plant as defined herein. Plants that are particularly useful in the methods of the invention include all plants which belong to the superfamily Viridiplantae, in particular monocotyledonous and dicotyledonous plants inducing fodder or forage legumes, ornamental plants, food crops, trees or shrubs. According to an embodiment of the present invention, the plant is a crop plant. Examples of crop plants include but are not limited to chicory, carrot, cassava, trefoil, soybean, beet, sugar beet, sunflower, canola, alfalfa, rapeseed, linseed, cotton, tomato, potato and tobacco. According to another embodiment of the present invention, the plant is a monocotyledonous plant. Examples of monocotyledonous plants include sugarcane. According to another embodiment of the present invention, the plant is a cereal. Examples of cereals include rice, maize, wheat, barley, millet, rye, triticale, sorghum, emmer, spelt, einkorn, teff, milo and oats. In a particular embodiment the plants of the invention or used in the methods of the invention are selected from the group consisting of maize, wheat, rice, soybean, cotton, oilseed rape including canola, sugarcane, sugar beet and alfalfa. Advantageously the methods of the invention are more efficient than the known methods, because the plants of the invention have increased yield and/or tolerance to an environmental stress compared to control plants used in comparable methods.

The invention also extends to harvestable parts of a plant such as, but not limited to seeds, leaves, fruits, flowers, stems, roots, rhizomes, tubers and bulbs, which harvestable parts comprise a recombinant nucleic acid encoding a CLP polypeptide. . In particular, such harvestable parts are roots such as taproots, rhizomes, fruits, stems, beets, tubers, bulbs, leaves, flowers and/or seeds. In one embodiment harvestable parts are stem cuttings (like setts of sugar cane).

The invention furthermore relates to products derived or produced, preferably directly derived or directly produced, from a harvestable part of such a plant, such as dry pellets, pressed stems, meal or powders, oil, fat and fatty acids, starch, sap, juice or proteins. Preferred carbohydrates are sugars, preferably sucrose. Also preferred products are residual dry fibers, e.g., of the stem (like bagasse from sugar cane after cane juice removal), molasse, or filtercake, preferably from sugar cane. In one embodiment the product comprises a recombinant nucleic acid encoding a CLP polypeptide and/or a recombinant CLP polypeptide for example as an indicator of the particular quality of the product.

The invention also includes methods for manufacturing a product comprising a) growing the plants of the invention and b) producing said product from or by the plants of the invention or parts thereof, including stem, root, beet and/or seeds. In a further embodiment the methods comprise the steps of a) growing the plants of the invention, b) removing the harvestable parts as described herein from the plants and c) producing said product from, or with the harvestable parts of plants according to the invention. In one embodiment, the product is produced from the stem of the transgenic plant.

The present invention is also directed to a product obtained by a method for manufacturing a product, as described herein. In one embodiment the products produced by the methods of the invention are plant products such as, but not limited to, a foodstuff, feedstuff, a food supplement, feed supplement, fiber, cosmetic or pharmaceutical. In another embodiment the methods for production are used to make agricultural products such as, but not limited to, plant extracts, proteins, amino acids, carbohydrates, fats, oils, polymers, vitamins, and the like.

In yet another embodiment the polynucleotides or the polypeptides or the constructs of the invention are comprised in an agricultural product. In a particular embodiment the nucleic acid sequences and protein sequences of the invention may be used as product markers, for example where an agricultural product was produced by the methods of the invention. Such a marker can be used to identify a product to have been produced by an advantageous process resulting not only in a greater efficiency of the process but also improved quality of the product due to increased quality of the plant material and harvestable parts used in the process. Such markers can be detected by a variety of methods known in the art, for example but not limited to PCR based methods for nucleic acid detection or antibody based methods for protein detection.

The present invention also encompasses use of nucleic acids encoding CLP polypeptides as described herein and use of these CLP polypeptides in enhancing any of the aforementioned yield-related traits in plants. For example, nucleic acids encoding CLP polypeptide described herein, or the CLP polypeptides themselves, may find use in breeding programmes in which a DNA marker is identified which may be genetically linked to a CLP polypeptide-encoding gene. The nucleic acids/genes, or the CLP polypeptides themselves may be to define a molecular marker. This DNA or protein marker may then be used in breeding programmes to select plants having one or more enhanced yield-related traits as defined herein in the methods of the invention. Furthermore, allelic variants of a CLP polypeptide-encoding nucleic acid/gene may find use in marker-assisted breeding programmes. Nucleic acids encoding CLP polypeptides may also be used as probes for genetically and physically mapping the genes that they are a part of, and as markers for traits linked to those genes. Such information may be useful in plant breeding in order to develop lines with desired phenotypes.

In the following, the expression "as defined in claim/item X" is meant to direct the artisan to apply the definition as disclosed in item/claim X. For example, "a nucleic acid as defined in item 1" has to be understood so that the definition of the nucleic acid as in item 1 is to be applied to the nucleic acid. In consequence the term " as defined in item" or "as defined in claim" may be replaced with the corresponding definition of that item or claim, respectively.

Moreover, the present invention relates to the following specific embodiments:

1. A method for enhancing one or more yield-related traits in plants relative to control plants, comprising modulating, preferably increasing expression, in a plant of a nucleic acid encoding a CLP polypeptide, wherein said CLP polypeptide comprising the Interpro motifs IPR004176 (Clp, N-terminal) and /or IPR023150 (Double Clp-N motif), preferably both, and optionally comprising a PFAM domain PF02861 preferably as a split domain wherein the N-terminal part of the PFAM domain PF02861 finds a match at or near the N-terminus of the CLP polypeptide and the C-terminal part of the PFAM domain PF02861 finds a match C-terminally of this, wherein both matching amino acid stretches are located in the N-terminal half, preferably in the N-terminal quarter of the CLP polypeptide, and wherein said CLP polypeptide is selected from the group consisting of:

   (i) an amino acid sequence of any SEQ ID NO listed in table A, preferably SEQ ID NO: 2; and
   (ii) an amino acid sequence having, in increasing order of preference, at least 35%, 40%, 45%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to the amino acid sequence of any SEQ ID NO listed in table A, preferably SEQ ID NO: 2, over the entire length of the sequences of said SEQ ID NO listed in table A, preferably SEQ ID NO: 2, and further preferably conferring one or more enhanced yield-related traits relative to control plants.

2. Method according to embodiment 1 wherein the CLP polypeptide has in increasing order of preference at least 70%, 75%, 80%, 85%, 90%, 95 %, 96%, 97%, 98 % or 99% sequence identity to the polypeptide sequence of SEQ ID NO: 2 over the entire length of the amino acid sequence of SEQ ID NO: 2.

3. Method according to embodiment 1 or 2, wherein said modulated expression is effected by introducing and expressing in a plant said nucleic acid encoding said CLP polypeptide.

4. Method according to embodiment 1, 2 or 3, wherein said nucleic acid is selected from the group consisting of:

   (i) a nucleic acid represented by SEQ ID NO: 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69 or 71; preferably SEQ ID NO: 1;
   (ii) the complement of a nucleic acid represented by SEQ ID NO: 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69 or 71; preferably SEQ ID NO: 1;

(iii) a nucleic acid encoding a POI polypeptide having in increasing order of preference at least 25%, 30%, 35%, 40%, 45%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to the entire amino acid sequence of any SEQ ID NO: listed in table A, preferably SEQ ID NO: 2, and further preferably conferring one or more enhanced yield-related traits relative to control plants; and
(iv) a nucleic acid molecule which hybridizes with a nucleic acid molecule of (i) to (iii) under high stringency hybridization conditions and preferably confers one or more enhanced yield-related traits relative to control plants.

5. Method according to any of embodiments 1 to 4, wherein said one or more enhanced yield-related traits comprise increased yield relative to control plants, and preferably comprise increased biomass and/or increased seed yield relative to control plants.

6. Method according to any one of embodiments 1 to 5, wherein said one or more enhanced yield-related traits are obtained under non-stress conditions.

7. Method according to any one of embodiments 1 to 5, wherein said one or more enhanced yield-related traits are obtained under conditions of environmental stress.

8. Method according to any of embodiments 1 to 7, wherein said CLP polypeptide comprises

i) all of the following motifs:

Motif 1 (SEQ ID NO: 76):

A-[AGLV]-K-x(0,2)-I-x(1,3)-Q-L-[IMV]-[IV]-S-I-L-[DH]-D-P-S-V-S-R-V-M-R-[DE]-A-[DG]-F-[NS]-S-[PST]-x-[LV]-[KR]-[ANST]-x-[IV]-E-x-[AE];

Motif 2 (SEQ ID NO: 77):
F-x-E-x-[NST]-[ADEST]-E-N-L-x(2)-L-[CS]-x-A-L-x(2)-[EKR]-x-[PST];

Motif 3 (SEQ ID NO: 78):

T-W-[FLM]-[FL]-F-x-G-x-[DGN]-x(2)-[ADG]-[KR]-x(2)-[IMV]-[ACS]-x-E-L-A-x-[LV]-V-F-G-S-x(3)-[FV]-x-[ACST]-x(3)-[ADGS]-[ADEGNST];

Motif 4(SEQ ID NO: 79):
G-x(1,2)-V-x(0,1)-L-x-[LV]-[EG]-D-L-x-[WY]-x(2)-[DE]-x(2)-[AST];

Motif 5 (SEQ ID NO: 80):
E-x(0,4)-L-x-[PT]-[FILV]-[ATV]-[IV]-P-[ADV]-[GST]
and

Motif 6 (SEQ ID NO: 81):
L-x-D-[AST]-I-[IV]-[IV]-x-[CGNS]-x-[AEQ];
or

ii) the motifs according to i) and in addition the consensus sequence as represented by the sequence listed under SEQ ID NO: 75; or
iii) the motifs according to i) or ii) above and in addition comprising all of the following consensus motifs
Consensus motif 1 (SEQ ID NO: 83): HPLQC[KR]ALELCF[NS]VA
Consensus motif 2 (SEQ ID NO: 84): NAL[GV]AA[LF]KRAQAHQR
and

Consensus motif 3 (SEQ ID NO: 85) LDDPSVSRVMREA[GS]F;
or
iv) any 5 of the motifs listed under i) and all three consensus motifs listed under iii); or
v) any 4 of the motifs listed under i) and all three consensus motifs listed under iii); or
vi) any 4 of the motifs listed under i) and any one of the consensus motifs listed under iii); or
vii) any 3 of the motifs listed under i); or
viii) One of the motifs as described herein above;
wherein -x represents in any motif position the presence of an amino acid residue of any type as often as the lowest integer number or the highest integer number in brackets following the -x indicate, or any of the integer numbers in between the lowest and the highest number, wherein the lowest integer number and the highest integer number might be identical and hence only one integer number is found within the brackets following -x, and wherein -x(1) is shortened to -x and any amino acid residue inserted at the position of -x does not need to be of the same type as the preceding one or another one inserted.

9. Method according to any one of embodiments 1 to 8, wherein said nucleic acid encoding a CLP is of plant origin, preferably from a dicotyledonous plant, further preferably from the family Brassicaceae, most preferably from Arabidopsis species.

10. Method according to any one of embodiments 1 to 9, wherein said nucleic acid encoding a CLP encodes any one of the polypeptides listed in Table A or is a portion of such a nucleic acid, or a nucleic acid capable of hybridising with a complementary sequence of such a nucleic acid.

11. Method according to any one of embodiments 1 to 10, wherein said nucleic acid sequence encodes an orthologue or paralogue of any of the polypeptides given in Table A.

12. Method according to any one of embodiments 1 to 11, wherein said nucleic acid encodes the polypeptide represented by SEQ ID NO: 2.

13. Method according to any one of embodiments 1 to 12, wherein said nucleic acid is operably linked to a constitutive promoter of plant origin, preferably to a medium strength constitutive promoter of plant origin, more preferably to a GOS2 promoter, most preferably to a GOS2 promoter from rice.

14. Plant, or part thereof, or plant cell, obtainable by a method according to any one of embodiments 1 to 14, wherein said plant, plant part or plant cell comprises a recombinant nucleic acid encoding a CLP polypeptide as defined in any of embodiments 1, 2, 4 and 8 to 12.

15. Construct comprising:

(i) nucleic acid encoding an CLP as defined in any of embodiments 1, 2, 4 and 8 to 12;
(ii) one or more control sequences capable of driving expression of the nucleic acid sequence of (i); and optionally
(iii) a transcription termination sequence.

16. Construct according to embodiment 15, wherein one of said control sequences is a constitutive promoter of plant origin, preferably to a medium strength constitutive promoter of plant origin, more preferably to a GOS2 promoter, most preferably to a GOS2 promoter from rice.

17. A host cell, preferably a bacterial host cell, more preferably an Agrobacterium species host cell comprising the construct according to any of embodiments 15 or 16 or the nucleic acid as defined in embodiment 4.

18. Use of a construct according to embodiment 15 or 16 in a method for making plants having one or more enhanced yield-related traits, preferably increased yield relative to control plants, and more preferably increased seed yield and/or increased biomass relative to control plants.

19. Plant, plant part or plant cell transformed with a construct according to embodiment 15 or 16.

20. Method for the production of a transgenic plant having one or more enhanced yield-related traits to control plants, preferably increased yield relative to control plants, and more preferably increased seed yield and/or increased biomass relative to control plants, comprising:

(i) introducing and expressing in a plant cell or plant a nucleic acid encoding an CLP polypeptide as defined in any of embodiments 1, 2, 4 and 8 to 12 ; and
(ii) cultivating said plant cell or plant under conditions promoting plant growth and development.

21. Transgenic plant having one or more enhanced yield-related traits relative to control plants, preferably increased yield relative to control plants, and more preferably increased seed yield and/or increased biomass, resulting from modulated expression of a nucleic acid encoding an CLP polypeptide as defined in any of embodiments 1, 2, 4 and 8 to 12 or a transgenic plant cell derived from said transgenic plant.

22. Transgenic plant according to embodiment 14, 19 or 21, or a transgenic plant cell derived therefrom, wherein said plant is a crop plant, such as beet, sugarbeet or alfalfa; or a monocotyledonous plant such as sugarcane; or a cereal, such as rice, maize, wheat, barley, millet, rye, triticale, sorghum, emmer, spelt, einkorn, teff, milo or oats.

23. Harvestable part of a plant according to embodiment 22, wherein said harvestable parts are preferably shoot biomass and/or root biomass and/or seeds.

24. A products derived from a plant according to embodiment 22 and/or from harvestable parts of a plant according to embodiment 23.

25. Use of a nucleic acid encoding an CLP polypeptide as defined in any of embodiments 1, 2, 4 and 8 to 12 for enhancing one or more yield-related traits in plants relative to control plants, preferably for increasing yield, and more preferably for increasing seed yield and/or for increasing biomass in plants relative to control plants.

26. A method for manufacturing a product comprising the steps of growing the plants according to embodiment 14,19, 21 or 22 and producing said product from or by said plants; or parts thereof, including seeds.

27. Recombinant chromosomal DNA comprising the construct according to embodiment 15 or 16.

28. Construct according to embodiment 15 or 16 or recombinant chromosomal DNA according to embodiment 27 comprised in a plant cell, preferably a crop plant cell.

Additional or alternative embodiments:

A. Method according to any one of embodiments 1 to 13, wherein said polypeptide is encoded by a nucleic acid molecule comprising a nucleic acid molecule selected from the group consisting of:

(i) a nucleic acid represented by (any one of) SEQ IDNO: 1 ;
(ii) the complement of a nucleic acid represented by (any one of) SEQ IDNO: 1;
(iii) a nucleic acid encoding the polypeptide as represented by (any one of) SEQ ID NO: 2, and further preferably confers one or more enhanced yield-related traits relative to control plants;
(iv) a nucleic acid having, in increasing order of preference at least 30 %, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity with (any of) the nucleic acid sequences of SEQ IDNO: 1, and further preferably conferring one or more enhanced yield-related traits relative to control plants;
(v) a nucleic acid molecule which hybridizes to the complement of a nucleic acid molecule of (i) to (iv) under stringent hybridization conditions and preferably confers one or more enhanced yield-related traits relative to control plants, ;
(vi) a nucleic acid encoding said polypeptide having, in increasing order of preference, at least 30 %, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to the amino acid sequence represented by (any one of) SEQ ID NO: 2 and preferably conferring one or more enhanced yield-related traits relative to control plants; or
(vii) a nucleic acid comprising any combination(s) of features of (i) to (vi) above.

B. Products produced from a plant according to embodiment 14, 19, 20 or 22 and/or from harvestable parts of a plant according to embodiment 23.

C. Construct according to embodiment 15 or 16 comprised in a plant cell.

D. Recombinant chromosomal DNA comprising the construct according to embodiment 15 or 16.

**Definitions**

The following definitions will be used throughout the present application. The section captions and headings in this application are for convenience and reference purpose only and should not affect in any way the meaning or interpretation of this application. The technical terms and expressions used within the scope of this application are generally to be given the meaning commonly applied to them in the pertinent art of plant biology, molecular biology, bioinformatics and plant breeding. All of the following term definitions apply to the complete content of this application. The term "essentially", "about", "approximately" and the like in connection with an attribute or a value, particularly also define exactly the attribute or exactly the value, respectively. The term "about" in the context of a given numeric value or range relates in particular to a value or range that is within 20%, within 10%, or within 5% of the value or range given. As used herein, the term "comprising" also encompasses the term "consisting of".

Peptide(s)/Protein(s)

The terms "peptides", "oligopeptides", "polypeptide" and "protein" are used interchangeably herein and refer to amino acids in a polymeric form of any length, linked together by peptide bonds, unless mentioned herein otherwise.

Polynucleotide(s)/Nucleic acid(s)/Nucleic acid sequence(s)/nucleotide sequence(s)

The terms "polynucleotide(s)", "nucleic acid sequence(s)", "nucleotide sequence(s)", "nucleic acid(s)", "nucleic acid molecule" are used interchangeably herein and refer to nucleotides, either ribonucleotides or deoxyribonucleotides or a combination of both, in a polymeric unbranched form of any length.

Homologue(s)

"Homologues" of a protein encompass peptides, oligopeptides, polypeptides, proteins and enzymes having amino acid substitutions, deletions and/or insertions relative to the unmodified protein in question and having similar biological and functional activity as the unmodified protein from which they are derived.

"Homologues" of a gene encompass genes having a nucleic acid sequence with nucleotide substitutions, deletions and/or insertions relative to the unmodified gene in question and having similar biological and functional activity as the unmodified gene from which they are derived, or encoding polypeptides having substantially the same biological and functional activity as the polypeptide encoded by the unmodified nucleic sequence

Orthologues and paralogues are two different forms of homologues and encompass evolutionary concepts used to describe the ancestral relationships of genes. Paralogues are genes within the same species that have originated through duplication of an ancestral gene; orthologues are genes from different organisms that have originated through speciation, and are also derived from a common ancestral gene.

A "deletion" refers to removal of one or more amino acids from a protein.

An "insertion" refers to one or more amino acid residues being introduced into a predetermined site in a protein. Insertions may comprise N-terminal and/or C-terminal fusions as well as intra-sequence insertions of single or multiple amino acids. Generally, insertions within the amino acid sequence will be smaller than N- or C-terminal fusions, of the order of about 1 to 10 residues. Examples of N- or C-terminal fusion proteins or peptides include the binding domain or activation domain of a transcriptional activator as used in the yeast two-hybrid system, phage coat proteins, (histidine)-6-tag, glutathione S-transferase-tag, protein A, maltose-binding protein, dihydrofolate reductase, Tag•100 epitope, c-myc epitope, FLAG®-epitope, lacZ, CMP (calmodulin-binding peptide), HA epitope, protein C epitope and VSV epitope.

A "substitution" refers to replacement of amino acids of the protein with other amino acids having similar properties (such as similar hydrophobicity, hydrophilicity, antigenicity, prodensity to form or break $\alpha$-helical structures or $\beta$-sheet structures). Amino acid substitutions are typically of single residues, but may be clustered depending upon functional constraints placed upon the polypeptide and may range from 1 to 10 amino acids. The amino acid substitutions are preferably conservative amino acid substitutions. Conservative substitution tables are well known in the art (see for example Creighton (1984) Proteins. W.H. Freeman and Company (Eds) and Table 1 below).

**Table 1** Examples of conserved amino acid substitutions

| Residue | Conservative Substitutions | Residue | Conservative Substitutions |
|---|---|---|---|
| Ala | Ser | Leu | Ile; Val |
| Arg | Lys | Lys | Arg; Gln |
| Asn | Gln; His | Met | Leu; Ile |
| Asp | Glu | Phe | Met; Leu; Tyr |
| Gln | Asn | Ser | Thr; Gly |
| Cys | Ser | Thr | Ser; Val |
| Glu | Asp | Trp | Tyr |
| Gly | Pro | Tyr | Phe |
| His | Asn; Gln | Val | Ile; Leu |
| Ile | Leu, Val | | |

Amino acid substitutions, deletions and/or insertions may readily be made using peptide synthetic techniques known in the art, such as solid phase peptide synthesis and the like, or by recombinant DNA manipulation. Methods for the manipulation of DNA sequences to produce substitution, insertion or deletion variants of a protein are well known in the art. For example, techniques for making substitution mutations at predetermined sites in DNA are well known to those skilled in the art and include M13 mutagenesis, T7-Gen in vitro mutagenesis (USB, Cleveland, OH), QuickChange Site Directed mutagenesis (Stratagene, San Diego, CA), PCR-mediated site-directed mutagenesis or other site-directed mutagenesis protocols (see Current Protocols in Molecular Biology, John Wiley & Sons, N.Y. (1989 and yearly update)).

<u>Derivatives</u>

"Derivatives" include peptides, oligopeptides, polypeptides which may, compared to the amino acid sequence of the naturally-occurring form of the protein, such as the protein of interest, comprise substitutions of amino acids with non-naturally occurring amino acid residues, or additions of non-naturally occurring amino acid residues. "Derivatives" of a protein also encompass peptides, oligopeptides, polypeptides which comprise naturally occurring altered (glycosylated, acylated, prenylated, phosphorylated, myristoylated, sulphated etc.) or non-naturally altered amino acid residues compared to the amino acid sequence of a naturally-occurring form of the polypeptide. A derivative may also comprise one or more non-amino acid substituents or additions compared to the amino acid sequence from which it is derived, for example a reporter molecule or other ligand, covalently or non-covalently bound to the amino acid sequence, such as a reporter molecule which is bound to facilitate its detection, and non-naturally occurring amino acid residues relative to the amino acid sequence of a naturally-occurring protein. Furthermore, "derivatives" also include fusions of the naturally-occurring form of the protein with tagging peptides such as FLAG, HIS6 or thioredoxin (for a review of tagging peptides, see Terpe, Appl. Microbiol. Biotechnol. 60, 523-533,2003).

"Derivatives" of nucleic acids include nucleic acids which may, compared to the nucleotide sequence of the naturally-occurring form of the nucleic acid comprise deletions, alterations, or additions with non-naturally occurring nucleotides. "Derivatives" of a nucleic acid also encompass nucleic acids which comprise naturally occurring altered or non-naturally altered nucleotides as compared to the nucleotide sequence of a naturally-occurring form of the nucleic acid. A derivative of a protein or nucleic acid still provides substantially the same function, e.g., enhanced yield-related trait, when expressed or repressed in a plant respectively.

Functional fragments

The term "functional fragment" refers to any nucleic acid or protein which comprises merely a part of the fulllength nucleic acid or fulllength protein, respectively, but still provides the same function, e.g., enhanced yield-related trait, when expressed or repressed in a plant respectively.

In cases where overexpression of nucleic acid is desired, the term "similar functional activity" or "similar function" means that any homologue and/or fragment provide increased / enhanced yield-related trait when expressed in a plant. Preferably similar functional activity means at least 50%, at 60%, at least 70%, at least 80 %, at least 90%, at least 95%, at least 98 %, at least 99% or 100% or higher increased / enhanced yield-related trait compared with functional activity provided by the exogenous expression of the full-length CLP encoding nucleotide sequence or the CLP amino acid sequence.

Domain, Motif/consensus sequence/Signature

The term "domain" refers to a set of amino acids conserved at specific positions along an alignment of sequences of evolutionarily related proteins. While amino acids at other positions can vary between homologues, amino acids that are highly conserved at specific positions indicate amino acids that are likely essential in the structure, stability or function of a protein. Identified by their high degree of conservation in aligned sequences of a family of protein homologues, they can be used as identifiers to determine if any polypeptide in question belongs to a previously identified polypeptide family.
The term "motif" or "consensus sequence" or "signature" refers to a short conserved region in the sequence of evolutionarily related proteins. Motifs are frequency highly conserved parts of domains, but may also include only part of the domain, or be located outside of conserved domain (if all of the amino acids of the motif fall outside of a defined domain).
Specialist databases exist for the identification of domains, for example, SMART (Schultz et al. (1998) Proc. Natl. Acad. Sci. USA 95, 5857-5864; Letunic et al. (2002) Nucleic Acids Res 30, 242-244), InterPro (Mulder et al., (2003) Nucl. Acids. Res. 31, 315-318), Prosite (Bucher and Bairoch (1994), A generalized profile syntax for biomolecular sequences motifs and its function in automatic sequence interpretation. (In) ISMB-94; Proceedings 2nd International Conference on Intelligent Systems for Molecular Biology. Altman R., Brutlag D., Karp P., Lathrop R., Searls D., Eds., pp53-61, AAAI Press, Menlo Park; Hulo et al., Nucl. Acids. Res. 32:D134-D137, (2004)), or Pfam (Bateman et al., Nucleic Acids Research 30(1): 276-280 (2002) ) & The Pfam protein families database: R.D. Finn, J. Mistry, J. Tate, P. Coggill, A. Heger, J.E. Pollington, O.L. Gavin, P. Gunesekaran, G. Ceric, K. Forslund, L. Holm, E.L. Sonnhammer, S.R. Eddy, A. Bateman Nucleic Acids Research (2010) Database Issue 38:211-222). A set of tools for *in silico* analysis of protein sequences is available on the ExPASy proteomics server (Swiss Institute of Bioinformatics (Gasteiger et al., ExPASy: the proteomics server for in-depth protein knowledge and analysis, Nucleic Acids Res. 31:3784-3788(2003)). Domains or motifs may also be identified using routine techniques, such as by sequence alignment.
Methods for the alignment of sequences for comparison are well known in the art, such methods include GAP, BESTFIT, BLAST, FASTA and TFASTA. GAP uses the algorithm of Needleman and Wunsch ((1970) J Mol Biol 48: 443-453) to find the global (i.e. spanning the complete sequences) alignment of two sequences that maximizes the number of matches and minimizes the number of gaps. The BLAST algorithm (Altschul et al. (1990) J Mol Biol 215: 403-10) calculates percent sequence identity and performs a statistical analysis of the similarity between the two sequences. The software for performing BLAST analysis is publicly available through the National Centre for Biotechnology Information (NCBI). Homologues may readily be identified using, for example, the ClustalW multiple sequence alignment algorithm (version 1.83), with the default pairwise alignment parameters, and a scoring method in percentage. Global percentages of similarity and identity may also be determined using one of the methods available in the MatGAT Software package (Campanella et al., BMC Bioinformatics. 2003 Jul 10;4:29. MatGAT: an application that generates similarity/ identity matrices using protein or DNA sequences.). Minor manual editing may be performed to optimise alignment between conserved motifs, as would be apparent to a person skilled in the art. Furthermore, instead of using full-length sequences for the identification of homologues, specific domains may also be used. The sequence identity values may be determined over the entire nucleic acid or amino acid sequence or over selected domains or conserved motif(s), using the programs mentioned above using the default parameters. For local alignments, the Smith-Waterman algorithm is particularly useful (Smith TF, Waterman MS (1981) J. Mol. Biol 147(1);195-7).

Reciprocal BLAST

Typically, this involves a first BLAST involving BLASTing a query sequence (for example using any of the sequences listed in Table A of the Examples section) against any sequence database, such as the publicly available NCBI database. BLASTN or TBLASTX (using standard default values) are generally used when starting from a nucleotide sequence, and BLASTP or TBLASTN (using standard default values) when starting from a protein sequence. The BLAST results may optionally be filtered. The full-length sequences of either the filtered results or non-filtered results are then BLASTed back (second BLAST) against sequences from the organism from which the query sequence is derived. The results of the first and second BLASTs are then compared. A paralogue is identified if a high-ranking hit from the first blast is from the same species as from which the query sequence is derived, a BLAST back then ideally results in the query sequence amongst the highest hits; an orthologue is identified if a high-ranking hit in the first BLAST is not from the same species as from which the query sequence is derived, and preferably results upon BLAST back in the query sequence being among the highest hits.
High-ranking hits are those having a low E-value. The lower the E-value, the more significant the score (or in other words the lower the chance that the hit was found by chance). Computation of the E-value is well known in the art. In addition to E-values, comparisons are also scored by percentage identity. Percentage identity refers to the number of identical nucleotides (or amino acids) between the two compared nucleic acid (or polypeptide) sequences over a particular length. In the case of large families, ClustalW may be used, followed by a neighbour joining tree, to help visualize clustering of related genes and to identify orthologues and paralogues.

Transit peptide

A "transit peptide" is a short (3-60 amino acids long) peptide chain that directs the transport of a protein, preferably to organelles within the cell or to certain subcellular locations or for the secretion of a protein. Transit peptides may also be called transit signal, signal peptide, signal sequence, targeting signals, or (subcellular) localization signals.

Hybridisation

The term "hybridisation" as defined herein is a process wherein substantially homologous complementary nucleotide sequences anneal to each other. The hybridisation process can occur entirely in solution, i.e. both complementary nucleic acids are in solution. The hybridisation process can also occur with one of the complementary nucleic acids immobilised to a matrix such as magnetic beads, Sepharose beads or any other resin. The hybridisation process can furthermore occur with one of the complementary nucleic acids immobilised to a solid support such as a nitrocellulose or nylon membrane or immobilised by e.g. photolithography to, for example, a siliceous glass support (the latter known as nucleic acid arrays or microarrays or as nucleic acid chips). In order to allow hybridisation to occur, the nucleic acid molecules are generally thermally or chemically denatured to melt a double strand into two single strands and/or to remove hairpins or other secondary structures from single stranded nucleic acids.

The term "stringency" refers to the conditions under which a hybridisation takes place. The stringency of hybridisation is influenced by conditions such as temperature, salt concentration, ionic strength and hybridisation buffer composition. Generally, low stringency conditions are selected to be about 30°C lower than the thermal melting Point ($T_m$) for the specific sequence at a defined ionic strength and pH. Medium stringency conditions are when the temperature is 20°C below $T_m$, and high stringency conditions are when the temperature is 10°C below $T_m$. High stringency hybridisation conditions are typically used for isolating hybridising sequences that have high sequence similarity to the target nucleic acid sequence. However, nucleic acids may deviate in sequence and still encode a substantially identical polypeptide, due to the degeneracy of the genetic code. Therefore medium stringency hybridisation conditions may sometimes be needed to identify such nucleic acid molecules.

The $T_m$ is the temperature under defined ionic strength and pH, at which 50% of the target sequence hybridises to a perfectly matched probe. The $T_m$ is dependent upon the solution conditions and the base composition and length of the probe. For example, longer sequences hybridise Specifically at higher temperatures. The maximum rate of hybridisation is obtained from about 16°C up to 32°C below Tm. The presence of monovalent cations in the hybridisation solution reduce the electrostatic repulsion between the two nucleic acid strands thereby promoting hybrid formation; this effect is visible for sodium concentrations of up to 0.4M (for higher concentrations, this effect may be ignored). Formamide reduces the melting temperature of DNA-DNA and DNA-RNA duplexes with 0.6 to 0.7°C for each percent formamide, and addition of 50% formamide allows hybridisation to be performed at 30 to 45°C, though the rate of hybridisation will be lowered. Base pair mismatches reduce the hybridisation rate and the thermal stability of the duplexes. On average and for large probes, the Tm decreases about 1°C per % base mismatch. The $T_m$ may be calculated using the following equations, depending on the types of hybrids:

1) DNA-DNA hybrids (Meinkoth and Wahl, Anal. Biochem., 138: 267-284, 1984):

$$T_m = 81.5°C + 16.6 x \log_{10}[Na^+]^a + 0.41 x \%[G/C^b] - 500 x [L^c]^{-1} - 0.61 x \% \text{ formamide}$$

2) DNA-RNA or RNA-RNA hybrids:

$$T_m = 79.8°C + 18.5 (\log_{10}[Na^+]^a) + 0.58 (\%G/C^b) + 11.8 (\%G/C^b)^2 - 820/L^c$$

3) oligo-DNA or oligo-RNA[d] hybrids:

For <20 nucleotides: $T_m$= 2 (In)
For 20—35 nucleotides: $T_m$= 22 + 1.46 ($I_n$)

[a] or for other monovalent cation, but only accurate in the 0.01—0.4 M range.
[b] only accurate for %GC in the 30% to 75% range.
[c] L = length of duplex in base pairs.
[d] oligo, oligonucleotide; $I_n$, = effective length of primer = 2×(no. of G/C)+(no. of A/T).

Non-specific binding may be controlled using any one of a number of known techniques such as, for example, blocking the membrane with protein containing solutions, additions of heterologous RNA, DNA, and SDS to the hybridisation buffer, and treatment with Rnase. For non-homologous probes, a series of hybridizations may be performed by varying one of (i) progressively lowering the annealing temperature (for example from 68°C to 42°C) or (ii) progressively lowering the formamide concentration (for example from 50% to 0%). The skilled artisan is aware of various parameters which may be altered during hybridisation and which will either maintain or change the stringency conditions.

Besides the hybridisation conditions, specificity of hybridisation typically also depends on the function of post-hybridisation washes. To remove background resulting from non-specific hybridisation, samples are washed with dilute salt solutions. Critical factors of such washes include the ionic strength and temperature of the final wash solution: the lower the salt concentration and the higher the wash temperature, the higher the stringency of the wash. Wash conditions are typically performed at or below hybridisation stringency. A positive hybridisation gives a signal that is at least twice of that of the background. Generally, suitable stringent conditions for nucleic acid hybridisation assays or gene amplification detection procedures are as set forth above. More or less stringent conditions may also be selected. The skilled artisan is aware of various parameters which may be altered during washing and which will either maintain or change the stringency conditions.

For example, typical high stringency hybridisation conditions for DNA hybrids longer than 50 nucleotides encompass hybridisation at 65°C in 1x SSC or at 42°C in 1x SSC and 50% formamide, followed by washing at 65°C in 0.3x SSC. Examples of medium stringency hybridisation conditions for DNA hybrids longer than 50 nucleotides encompass hybridisation at 50°C in 4x SSC or at 40°C in 6x SSC and 50% formamide, followed by washing at 50°C in 2x SSC. The length of the hybrid is the anticipated length for the hybridising nucleic acid. When nucleic acids of known sequence are hybridised, the hybrid length may be determined by aligning the sequences and identifying the conserved regions described herein. 1×SSC is 0.15M NaCl and 15mM sodium citrate; the hybridisation solution and wash solutions may additionally include 5x Denhardt's reagent, 0.5-1.0% SDS, 100 μg/ml denatured, fragmented salmon sperm DNA, 0.5% sodium pyrophosphate.

For the purposes of defining the level of stringency, reference can be made to Sambrook et al. (2001) Molecular Cloning: a laboratory manual, 3rd Edition, Cold Spring Harbor Laboratory Press, CSH, New York or to Current Protocols in Molecular Biology, John Wiley & Sons, N.Y. (1989 and yearly updates

<u>Splice variant</u>

The term "splice variant" as used herein encompasses variants of a nucleic acid sequence in which selected introns and/or exons have been excised, replaced, displaced or added, or in which introns have been shortened or lengthened. Such variants will be ones in which the biological activity of the protein is substantially retained; this may be achieved by selectively retaining functional segments of the protein. Such splice variants may be found in nature or may be manmade. Methods for predicting and isolating such splice variants are well known in the art (see for example Foissac and Schiex (2005) BMC Bioinformatics 6: 25).

<u>Allelic variant</u>

"Alleles" or "allelic variants" are alternative forms of a given gene, located at the same chromosomal position. Allelic variants encompass Single Nucleotide Polymorphisms (SNPs), as well as Small Insertion/Deletion Polymorphisms (INDELs). The size of INDELs is usually less than 100 bp. SNPs and INDELs form the largest set of sequence variants in naturally occurring polymorphic strains of most organisms.

<u>Endogenous</u>

Reference herein to an "endogenous" nucleic acid and / or protein refers to the nucleic acid and / or protein in question as found in a plant in its natural form (i.e., without there being any human intervention like recombinant DNA technology), but also refers to that same gene (or a substantially homologous nucleic acid/gene) in an isolated form subsequently (re)introduced into a plant (a transgene). For example, a transgenic plant containing such a transgene may encounter a substantial reduction of the transgene expression and/or substantial reduction of expression of the endogenous gene. The isolated gene may be isolated from an organism or may be manmade, for example by chemical synthesis.

Exogenous

The term "exogenous" (in contrast to "endogenous") nucleic acid or gene refers to a nucleic acid that has been introduced in a plant by means of recombinant DNA technology. An "exogenous" nucleic acid can either not occur in a plant in its natural form, be different from the nucleic acid in question as found in a plant in its natural form, or can be identical to a nucleic acid found in a plant in its natural form, but integrated not within its natural genetic environment. The corresponding meaning of "exogenous" is applied in the context of protein expression. For example, a transgenic plant containing a transgene, i.e., an exogenous nucleic acid, may, when compared to the expression of the endogenous gene, encounter a substantial increase of the expression of the respective gene or protein in total. A transgenic plant according to the present invention includes an exogenous CLP nucleic acid integrated at any genetic loci and optionally the plant may also include the endogenous gene within the natural genetic back-ground.

Gene shuffling/Directed evolution

"Gene shuffling" or "directed evolution" consists of iterations of DNA shuffling followed by appropriate screening and/or selection to generate variants of nucleic acids or portions thereof encoding proteins having a modified biological activity (Castle et al., (2004) Science 304(5674): 1151-4; US patents 5,811,238 and 6,395,547).

Construct

Artificial DNA (such as but, not limited to plasmids or viral DNA) capable of replication in a host cell and used for introduction of a DNA sequence of interest into a host cell or host organism. Host cells of the invention may be any cell selected from bacterial cells, such as Escherichia coli or Agrobacterium species cells, yeast cells, fungal, algal or cyanobacterial cells or plant cells. The skilled artisan is well aware of the genetic elements that must be present on the genetic construct in order to successfully transform, select and propagate host cells containing the sequence of interest. The sequence of interest is operably linked to one or more control sequences (at least to a promoter) as described herein. Additional regulatory elements may include transcriptional as well as translational enhancers. Those skilled in the art will be aware of terminator and enhancer sequences that may be suitable for use in performing the invention. An intron sequence may also be added to the 5' untranslated region (UTR) or in the coding sequence to increase the amount of the mature message that accumulates in the cytosol, as described in the definitions section. Other control sequences (besides promoter, enhancer, silencer, intron sequences, 3'UTR and/or 5'UTR regions) may be protein and/or RNA stabilizing elements. Such sequences would be known or may readily be obtained by a person skilled in the art.

The genetic constructs of the invention may further include an origin of replication sequence that is required for maintenance and/or replication in a specific cell type. One example is when a genetic construct is required to be maintained in a bacterial cell as an episomal genetic element (e.g. plasmid or cosmid molecule). Preferred origins of replication include, but are not limited to, the f1-ori and colE1.

For the detection of the successful transfer of the nucleic acid sequences as used in the methods of the invention and/or selection of transgenic plants comprising these nucleic acids, it is advantageous to use marker genes (or reporter genes). Therefore, the genetic construct may optionally comprise a selectable marker gene. Selectable markers are described in more detail in the "definitions" section herein. The marker genes may be removed or excised from the transgenic cell once they are no longer needed. Techniques for marker removal are known in the art, useful techniques are described above in the definitions section.

Vector construct

Artificial DNA (such as but, not limited to plasmids or viral DNA) capable of replication in a host cell and used for introduction of a DNA sequence of interest into a host cell or host organism. Host cells of the invention may be any cell selected from bacterial cells, such as Escherichia coli or Agrobacterium species cells, yeast cells, fungal, algal or cyanobacterial cells or plant cells. The skilled artisan is well aware of the genetic elements that must be present on the genetic construct in order to successfully transform, select and propagate host cells containing the sequence of interest. The one or more sequence(s) of interest is operably linked to one or more control sequences (at least to a promoter) as described herein. Additional regulatory elements may include transcriptional as well as translational enhancers. Those skilled in the art will be aware of terminator and enhancer sequences that may be suitable for use in performing the invention. An intron sequence may also be added to the 5' untranslated region (UTR) or in the coding sequence to increase the amount of the mature message that accumulates in the cytosol, as described in the definitions section. Other control sequences (besides promoter, enhancer, silencer, intron sequences, 3'UTR and/or 5'UTR regions) may be protein and/or RNA stabilizing elements. Such sequences would be known or may readily be obtained by a person skilled in the art.

Regulatory element/Control sequence/Promoter

The terms "regulatory element, "control sequence" and "promoter" are all used interchangeably herein and are to be taken in a broad context to refer to regulatory nucleic acid sequences capable of effecting expression of the sequences to which they are ligated. The term "promoter" typically refers to a nucleic acid control sequence located upstream from the transcriptional start of a gene and which is involved in recognising and binding of RNA polymerase and other proteins, thereby directing transcription of an operably linked nucleic acid. Encompassed by the aforementioned terms are transcriptional regulatory sequences derived from a classical eukaryotic genomic gene (including the TATA box which is required for accurate transcription initiation, with or without a CCAAT box sequence) and additional regulatory elements (i.e. upstream activating sequences, enhancers and silencers) which alter gene expression in response to developmental and/or external stimuli, or in a tissue-specific manner. Also included within the term is a transcriptional regulatory sequence of a classical prokaryotic gene, in which case it may induce a -35 box sequence and/or -10 box transcriptional regulatory sequences. The term "regulatory element" also encompasses a synthetic fusion molecule or derivative that confers, activates or enhances expression of a nucleic acid molecule in a cell, tissue or organ.

A "plant promoter" comprises regulatory elements, which mediate the expression of a coding sequence segment in plant cells. Accordingly, a plant promoter need not be of plant origin, but may originate from viruses or micro-organisms, for example from viruses which attack plant cells. The "plant promoter" can also originate from a plant cell, e.g. from the plant which is transformed with the nucleic acid sequence to be expressed in the inventive process and described herein. This also applies to other "plant" regulatory signals, such as "plant" terminators. The promoters upstream of the nucleotide sequences useful in the methods of the present invention can be modified by one or more nucleotide substitution(s), insertion(s) and/or deletion(s) without interfering with the functionality or activity of either the promoters, the open reading frame (ORF) or the 3'-regulatory region such as terminators or other 3' regulatory regions which are located away from the ORF. It is furthermore possible that the activity of the promoters is increased by modification of their sequence, or that they are replaced completely by more active promoters, even promoters from heterologous organisms. For expression in plants, the nucleic acid molecule must, as described above, be linked operably to or comprise a suitable promoter which expresses the gene at the right Point in time and with the required spatial expression pattern.

For the identification of functionally equivalent promoters, the promoter strength and/or expression pattern of a promoter may be analysed for example by operably linking the promoter to a reporter gene and assaying the expression level and pattern of the reporter gene in various tissues of the plant. Suitable well-known reporter genes include for example beta-glucuronidase or beta-galactosidase. The promoter activity is assayed by measuring the enzymatic activity of the beta-glucuronidase or beta-galactosidase. The promoter strength and/or expression pattern may then be compared to that of a reference promoter (such as the one used in the methods of the present invention). Alternatively, promoter strength may be assayed by quantifying mRNA levels or by comparing mRNA levels of the nucleic acid used in the methods of the present invention, with mRNA levels of housekeeping genes such as 18S rRNA, using methods known in the art, such as Northern blotting with densitometric analysis of autoradiograms, quantitative real-time PCR or RT-PCR (Heid et al., 1996 Genome Methods 6: 986-994). Generally by "weak promoter" is intended a promoter that drives expression of a coding sequence at a low level. By "low level" is intended at levels of about 1/10,000 transcripts to about 1/100,000 transcripts, to about 1/500,0000 transcripts per cell. Conversely, a "strong promoter" drives expression of a coding sequence at high level, or at about 1/10 transcripts to about 1/100 transcripts to about 1/1000 transcripts per cell. Generally, by "medium strength promoter" is intended a promoter that drives expression of a coding sequence at a lower level than a strong promoter, in particular at a level that is in all instances below that obtained when under the control of a 35S CaMV promoter.

Operably linked

The term "operably linked" or "functionally linked" as used herein refers to a functional linkage between the promoter sequence and the gene of interest, such that the promoter sequence is able to initiate transcription of the gene of interest.

Constitutive promoter,

A "constitutive promoter" refers to a promoter that is transcriptionally active during most, but not necessarily all, phases of growth and development and under most environmental conditions, in at least one cell, tissue or organ. Table 2a below gives examples of constitutive promoters.

**Table 2a:** Examples of constitutive promoters

| Gene Source | Reference |
|---|---|
| Actin | McElroy et al, Plant Cell, 2: 163-171, 1990 |
| HMGP | WO 2004/070039 |
| CAMV 35S | Odell et al, Nature, 313: 810-812, 1985 |
| CaMV 19S | Nilsson et al., Physiol. Plant, 100:456-462, 1997 |
| GOS2 | de Pater et al, Plant J Nov;2(6):837-44, WO 2004/065596 |
| Ubiquitin | Christensen et al, Plant Mol. Biol. 18: 675-689, 1992 |
| Rice cyclophilin | Buchholz et al, Plant Mol Biol. 25(5): 837-43, 1994 |
| Maize H3 histone | Lepetit et al, Mol. Gen. Genet. 231:276-285, 1992 |
| Alfalfa H3 histone | Wu et al. Plant Mol. Biol. 11:641-649, 1988 |
| Actin 2 | An et al, Plant J. 10(1); 107-121, 1996 |
| 34S FMV | Sanger et al., Plant. Mol. Biol., 14, 1990: 433-443 |
| Rubisco subunit | US 4,962,028 |
| OCS | Leisner (1988) Proc Natl Acad Sci USA 85(5): 2553 |
| SAD1 | Jain et al., Crop Science, 39 (6), 1999: 1696 |
| SAD2 | Jain et al., Crop Science, 39 (6), 1999: 1696 |
| nos | Shaw et al. (1984) Nucleic Acids Res. 12(20):7831-7846 |
| V-ATPase | WO 01/14572 |
| Super promoter | WO 95/14098 |
| G-box proteins | WO 94/12015 |

Ubiquitous promoter

A "ubiquitous promoter" is active in substantially all tissues or cells of an organism.

Developmentally-regulated promoter

A "developmentally-regulated promoter" is active during certain developmental stages or in parts of the plant that undergo developmental changes.

Inducible promoter

An "inducible promoter" has induced or increased transcription initiation in response to a chemical (for a review see Gatz 1997, Annu. Rev. Plant Physiol. Plant Mol. Biol., 48:89-108), environmental or physical stimulus, or may be "stress-inducible", i.e. activated when a plant is exposed to various stress conditions, or a "pathogen-inducible" i.e. activated when a plant is exposed to exposure to various pathogens.

Organ-specific/Tissue-specific promoter

An "organ-specific" or "tissue-specific promoter" is one that is capable of preferentially initiating transcription in certain organs or tissues, such as the leaves, roots, seed tissue etc. For example, a "root-specific promoter" is a promoter that is transcriptionally active predominantly in plant roots, substantially to the exclusion of any other parts of a plant, whilst still allowing for any leaky expression in these other plant parts. Promoters able to initiate transcription in certain cells only are referred to herein as "cell-specific".

Examples of root-specific promoters are listed in Table 2b below:

**Table 2b:** Examples of root-specific promoters

| Gene Source | Reference |
|---|---|
| RCc3 | Plant Mol Biol. 1995 Jan;27(2):237-48 |
| Arabidopsis PHT1 | Koyama et al. J Biosci Bioeng. 2005 Jan;99(1):38-42.; Mudge et al. (2002, Plant J. 31:341) |
| Medicago phosphate transporter | Xiao et al., 2006, Plant Biol (Stuttg). 2006 Jul;8(4):439-49 |
| Arabidopsis Pyk10 | Nitz et al. (2001) Plant Sci 161(2): 337-346 |
| root-expressibte genes | Tingey et al., EMBO J. 6: 1, 1987. |
| tobacco auxin-inducible gene | Van der Zaal et al., Plant Mol. Biol. 16, 983, 1991. |
| β-tubulin | Oppenheimer, et al., Gene 63: 87, 1988. |
| tobacco root-specific genes | Conkling, et al., Plant Physiol. 93: 1203, 1990. |
| B. napus G1-3b gene | United States No. 5, 401, 836 |
| SbPRP1 | Suzuki et al., Plant Mol. Biol. 21: 109-119, 1993. |
| LRX1 | Baumberger et al. 2001, Genes & Dev. 15:1128 |
| BTG-26 Brassica napus | US 20050044585 |
| LeAMT1 (tomato) | Lauter et al. (1996, PNAS 3:8139) |
| The LeNRT1-1 (tomato) | Lauter et al. (1996, PNAS 3:8139) |
| class I patatin gene (potato) | Liu et al., Plant Mol. Biol. 17 (6): 1139-1154 |
| KDC1 (Daucus carota) | Downey et al. (2000, J. Biol. Chem. 275:39420) |
| TobRB7 gene | W Song (1997) PhD Thesis, North Carolina State University, Raleigh, NC USA |
| OsRAB5a (rice) | Wang et al. 2002, Plant Sci. 163:273 |
| ALF5 (Arabidopsis) | Diener et al. (2001, Plant Cell 13:1625) |
| NRT2;1Np (N. plumbaginifolia) | Quesada et al. (1997, Plant Mol. Biol. 34:265) |

A "seed-specific promoter" is transcriptionally active predominantly in seed tissue, but not necessarily exclusively in seed tissue (in cases of leaky expression). The seed-specific promoter may be active during seed development and/or during germination. The seed specific promoter may be endosperm/aleurone/embryo specific. Examples of seed-specific promoters (endosperm/aleurone/embryo specific) are shown in Table 2c to Table 2f below.

Further examples of seed-specific promoters are given in Qing Qu and Takaiwa (Plant Biotechnol. J. 2,113-125, 2004), which disclosure is incorporated by reference herein as if fully set forth.

**Table 2c:** Examples of seed-specific promoters

| Gene source | Reference |
|---|---|
| seed-specific genes | Simon et al., Plant Mol. Biol. 5: 191, 1985; |
| | Scofield et al., J. Biol. Chem. 262: 12202, 1987.; |
| | Baszczynski et al., Plant Mol. Biol. 14: 633, 1990. |
| Brazil Nut albumin | Pearson et al., Plant Mol. Biol. 18: 235-245, 1992. |
| legumin | Ellis et al., Plant Mol. Biol. 10: 203-214, 1988. |
| glutelin (rice) | Takaiwa et al., Mol. Gen. Genet. 208: 15-22. 1986; |
| | Takaiwa et al., FEBS Letts. 221: 43-47, 1987. |
| zein | Matzke et al Plant Mol Biol, 14(3):323-32 1990 |
| napA | Stalberg et al, Planta 199: 515-519, 1996. |
| wheat LMW and HMW glutenin-1 | Mol Gen Genet 216:81-90, 1989; NAR 17:461-2, 1989 |

(continued)

| Gene source | Reference |
|---|---|
| wheat SPA | Albani et al, Plant Cell, 9: 171-184, 1997 |
| wheat $\alpha$, $\beta$, $\gamma$-gliadins | EMBO J. 3:1409-15, 1984 |
| barley ltr1 promoter | Diaz et al. (1995) Mol Gen Genet 248(5):592-8 |
| barley B1, C, D, hordein | Theor Appl Gen 98:1253-62, 1999; Plant J 4:343-55, 1993; Mol Gen Genet 250:750-60, 1996 |
| barley DOF | Mena et al, The Plant Journal, 116(1): 53-62, 1998 |
| blz2 | EP99106056.7 |
| synthetic promoter | Vicente-Carbajosa et al., Plant J. 13: 629-640, 1998. |
| rice prolamin NRP33 | Wu et al, Plant Cell Physiology 39(8) 885-889, 1998 |
| rice a-globulin Glb-1 | Wu et al, Plant Cell Physiology 39(8) 885-889, 1998 |
| rice OSH1 | Sato et al, Proc. Natl. Acad. Sci. USA, 93: 8117-8122, 1996 |
| rice $\alpha$-globulin REB/OHP-1 | Nakase et al. Plant Mol. Biol. 33: 513-522, 1997 |
| rice ADP-glucose pyrophosphorylase | Trans Res 6:157-68, 1997 |
| maize ESR gene family | Plant J 12:235-46, 1997 |
| sorghum $\alpha$-kafirin | DeRose et al., Plant Mol. Biol 32:1029-35, 1996 |
| KNOX | Postma-Haarsma et al, Plant Mol. Biol. 39:257-71, 1999 |
| rice oleosin | Wu et al, J. Biochem. 123:386, 1998 |
| sunflower oleosin | Cummins et al., Plant Mol. Biol. 19: 873-876, 1992 |
| PRO0117, putative rice 40S ribosomal protein | WO 2004/070039 |
| PRO0136, rice alanine aminotransferase | unpublished |
| PRO0147, trypsin inhibitor ITR1 (barley) | unpublished |
| PRO0151, rice WSI18 | WO 2004/070039 |
| PRO0175, rice RAB21 | WO 2004/070039 |
| PRO005 | WO 2004/070039 |
| PRO0095 | WO 2004/070039 |
| $\alpha$-amylase (Amy32b) | Lanahan et al, Plant Cell 4:203-211, 1992; Skriver et al, Proc Natl Acad Sci USA 88:7266-7270, 1991 |
| cathepsin $\beta$-like gene | Cejudo et al, Plant Mol Biol 20:849-856, 1992 |
| Barley Ltp2 | Kalla et al., Plant J. 6:849-60, 1994 |
| Chi26 | Leah et al., Plant J. 4:579-89, 1994 |
| Maize B-Peru | Selinger et al., Genetics 149; 1125-38, 1998 |

**Table 2d:** examples of endosperm-specific promoters

| Gene source | Reference |
|---|---|
| glutelin (rice) | Takaiwa et al. (1986) Mol Gen Genet 208:15-22; Takaiwa et al. (1987) FEBS Letts. 221:43-47 |
| zein | Matzke et al., (1990) Plant Mol Biol 14(3): 323-32 |
| wheat LMW and HMW glutenin-1 | Colot et al. (1989) Mol Gen Genet 216:81-90, Anderson et al. (1989) NAR 17:461-2 |

(continued)

| Gene source | Reference |
|---|---|
| wheat SPA | Albani et al. (1997) Plant Cell 9:171-184 |
| wheat gliadins | Rafalski et al. (1984) EMBO 3:1409-15 |
| barley Itr1 promoter | Diaz et al. (1995) Mol Gen Genet 248(5):592-8 |
| barley B1, C, D, hordein | Cho et al. (1999) Theor Appl Genet 98:1253-62; Muller et al. (1993) Plant J 4:343-55; Sorenson et al. (1996) Mol Gen Genet 250:750-60 |
| barley DOF | Mena et al, (1998) Plant J 116(1): 53-62 |
| blz2 | Onate et al. (1999) J Biol Chem 274(14):9175-82 |
| synthetic promoter | Vicente-Carbajosa et al. (1998) Plant J 13:629-640 |
| rice prolamin NRP33 | Wu et al, (1998) Plant Cell Physiol 39(8) 885-889 |
| rice globulin Gtb-1 | Wu et al. (1998) Plant Cell Physiol 39(8) 885-889 |
| rice globulin REB/OHP-1 | Nakase et al. (1997) Plant Molec Biol 33: 513-522 |
| rice ADP-glucose pyrophosphorylase | Russell et al. (1997) Trans Res 6:157-68 |
| maize ESR gene family | Opsahl-Ferstad et al. (1997) Plant J 12:235-46 |
| sorghum kafirin | DeRose et al. (1996) Plant Mol Biol 32:1029-35 |

**Table 2e:** Examples of embryo specific promoters:

| Gene source | Reference |
|---|---|
| rice OSH1 | Sato et al, Proc. Natl. Acad. Sci. USA, 93: 8117-8122, 1996 |
| KNOX | Postma-Haarsma et al, Plant Mol. Biol. 39:257-71, 1999 |
| PRO0151 | WO 2004/070039 |
| PRO0175 | WO 2004/070039 |
| PRO005 | WO 2004/070039 |
| PRO0095 | WO 2004/070039 |

**Table 2f:** Examples of aleurone-specific promoters:

| Gene source | Reference |
|---|---|
| α-amylase (Amy32b) | Lanahan et al, Plant Cell 4:203-211, 1992; Skiver et al, Proc Natl Acad Sci USA 88: 7266-7270, 1991 |
| cathepsin β-like gene | Cejudo et al, Plant Mol Biol 20:849-856, 1992 |
| Barley Ltp2 | Kalla et al., Plant J. 6:849-60, 1994 |
| Chi26 | Leah et al., Plant J. 4:579-89, 1994 |
| Maize B-Peru | Selinger et al., Genetics 149;1125-38, 1998 |

A "green tissue-specific promoter" as defined herein is a promoter that is transcriptionally active predominantly in green tissue, substantially to the exclusion of any other parts of a plant, whilst still allowing for any leaky expression in these other plant parts.

Examples of green tissue-specific promoters which may be used to perform the methods of the invention are shown in Table 2g below.

**Table 2g:** Examples of green tissue-specific promoters

| Gene | Expression | Reference |
|---|---|---|
| Maize Orthophosphate dikinase | Leaf specific | Fukavama et 21., Plant Physiol. 2001 Nov;127(3): 1136-46 |
| Maize Phosphoenolpyruvate carboxylase | Leaf specific | Kausch et al., Plant Mol Biol. 2001 Jan;45(1):1-15 |
| Rice Phosphoenolpyruvate carboxylase | Leaf specific | Lin et al., 2004 DNA Seq. 2004 Aug;15(4):269-76 |
| Rice small subunit Rubisco | Leaf specific | Nomura et al., Plant Mol Biol. 2000 Sep;44(1):99-106 |
| rice beta expansin EXBP9 | Shoot specific | WO 2004/070039 |
| Pigeonpea small subunit Rubisco | Leaf specific | Panguluri et al., Indian J Exp Biol. 2005 Apr;43(4): 369-72 |
| Pea RBCS3A | Leaf specific | |

Another example of a tissue-specific promoter is a meristem-specific promoter, which is transcriptionally active predominantly in meristematic tissue, substantially to the exclusion of any other parts of a plant, whilst still allowing for any leaky expression in these other plant parts. Examples of green meristem-specific promoters which may be used to perform the methods of the invention are shown in Table 2h below.

**Table 2h:** Examples of meristem-specific promoters

| **Gene source** | **Expression pattern** | **Reference** |
|---|---|---|
| rice OSH1 | Shoot apical meristem, from embryo globular stage to seedling stage | Sato et al. (1996) Proc. Natl. Acad. Sci. USA, 93: 8117-8122 |
| Rice metallothionein | Meristem specific | BAD87835.1 |
| WAK1 & WAK 2 | Shoot and root apical meristems, and in expanding leaves and sepals | Wagner & Kohorn (2001) Plant Cell 13(2): 303-318 |

Terminator

The term "terminator" encompasses a control sequence which is a DNA sequence at the end of a transcriptional unit which signals 3' processing and polyadenylation of a. primary transcript and termination of transcription. The terminator can be derived from the natural gene, from a variety of other plant genes, or from T-DNA. The terminator to be added May be derived from, for example, the nopaline synthase or octopine synthase genes, or alternatively from another plant gene, or less preferably from any other eukaryotic gene.

Selectable marker (gene)/Reporter gene

"Selectable marker", "selectable marker gene" or "reporter gene" includes any gene that confers a phenotype on a cell in which it is expressed to facilitate the identification and/or selection of cells that are transfected or transformed with a nucleic acid construct of the invention. These marker genes enable the identification of a successful transfer of the nucleic acid molecules via a series of different principles. Suitable markers may be selected from markers that confer antibiotic or herbicide resistance, that introduce a new metabolic trait or that allow visual selection. Examples of selectable marker genes include genes conferring resistance to antibiotics (such as nptll that phosphorylates neomycin and kanamycin, or hpt, phosphorylating hygromycin, or genes conferring resistance to, for example, bleomycin, streptomycin, tetracyclin, chloramphenicol, ampicillin, gentamycin, geneticin (G418), spectinomycin or blasticidin), to herbicides (for example bar which provides resistance to Basta®; aroA or gox providing resistance against glyphosate, or the genes conferring resistance to, for example, imidazolinone, phosphinothricin or sulfonylurea), or genes that provide a metabolic trait (such as manA that allows plants to use mannose as sole carbon source or xylose isomerase for the utilisation of xylose, or antinutritive markers such as the resistance to 2-deoxyglucose). Expression of visual marker genes results in the formation of colour (for example β-glucuronidase, GUS or β-galactosidase with its coloured substrates, for example X-Gal), luminescence (such as the luciferin/luceferase system) or fluorescence (Green Fluorescent Protein, GFP, and derivatives thereof). This list represents only a small number of possible markers. The skilled worker is familiar with such markers. Different markers are preferred, depending on the organism and the selection method.

It is known that upon stable or transient integration of nucleic acids into plant cells, only a minority of the cells takes up the foreign DNA and, if desired, integrates it into its genome, depending on the expression vector used and the transfection technique used. To identify and select these integrants, a gene coding for a selectable marker (such as the ones described above) is usually introduced into the host cells together with the gene of interest. These markers can for example be used in mutants in which these genes are not functional by, for example, deletion by conventional methods. Furthermore, nucleic acid molecule encoding a selectable marker can be introduced into a host cell on the same vector that comprises the sequence encoding the polypeptides of the invention or used in the methods of the invention, or else in a separate vector. Cells which have been stably transfected with the introduced nucleic acid can be identified for example by selection (for example, cells which have integrated the selectable marker survive whereas the other cells die).

Since the marker genes, particularly genes for resistance to antibiotics and herbicides, are no longer required or are undesired in the transgenic host cell once the nucleic acids have been introduced successfully, the process according to the invention for introducing the nucleic acids advantageously employs techniques which enable the removal or excision of these marker genes. One such a method is what is known as co-transformation. The co-transformation method employs two vectors simultaneously for the transformation, one vector bearing the nucleic acid according to the invention and a second bearing the marker gene(s). A large proportion of transformants receives or, in the case of plants, comprises (up to 40% or more of the transformants), both vectors. In of transformation with Agrobacteria, the transformants usually receive only a part of the vector, i.e. the sequence flanked by the T-DNA, which usually represents the expression cassette. The marker genes can subsequently be removed from the transformed plant by performing crosses. In another method, marker genes integrated into a transposon are used for the transformation together with desired nucleic acid (known as the Ac/Ds technology). The transformants can be crossed with a transposase source or the transformants are transformed with a nucleic acid construct conferring expression of a transposase, transiently or stable. In some cases (approx. 10%), the transposon jumps out of the genome of the host cell once transformation has taken place successfully and is lost. In a further number of cases, the transposon jumps to a different location. In these cases the marker gene must be eliminated by performing crosses. In microbiology, techniques were developed which make possible, or facilitate, the detection of such events. A further advantageous method relies on what is known as recombination systems; whose advantage is that elimination by crossing can be dispensed with. The best-known system of this type is what is known as the Cre/lox system. Cre1 is a recombinase that removes the sequences located between the loxP sequences. If the marker gene is integrated between the loxP sequences, it is removed once transformation has taken place successfully, by expression of the recombinase. Further recombination systems are the HIN/HIX, FLP/FRT and REP/STB system (Tribble et al., J. Biol. Chem., 275, 2000: 22255-22267; Velmurugan et al., J. Cell Biol., 149, 2000: 553-566), A site-specific integration into the plant genome of the nucleic acid sequences according to the invention is possible. Naturally, these methods can also be applied to microorganisms such as yeast, fungi or bacteria.

<u>Transgenic/Transgene/Recombinant</u>

For the purposes of the invention, "transgenic", "transgene" or "recombinant" means with regard to, for example, a nucleic acid sequence, an expression cassette, gene construct or a vector comprising the nucleic acid sequence or an organism transformed with the nucleic acid sequences, expression cassettes or vectors according to the invention, all those constructions brought about by recombinant methods in which either

(a) the nucleic acid sequences encoding proteins useful in the methods of the invention, or
(b) genetic control sequence(s) which is operably linked with the nucleic acid sequence according to the invention, for example a promoter, or
(c) a) and b)

are not located in their natural genetic environment or have been modified by recombinant methods, it being possible for the modification to take the form of, for example, a substitution, addition, deletion, inversion or insertion of one or more nucleotide residues. The natural genetic environment is understood as meaning the natural genomic or chromosomal locus in the original plant or the presence in a genomic library. In the case of a genomic library, the natural genetic environment of the nucleic acid sequence is preferably retained, at least in part. The environment flanks the nucleic acid sequence at least on one side and has a sequence length of at least 50 bp, preferably at least 500 bp, especially preferably at least 1000 bp, most preferably at least 5000 bp. A naturally occurring expression cassette - for example the naturally occurring combination of the natural promoter of the nucleic acid sequences with the corresponding nucleic acid sequence encoding a polypeptide useful in the methods of the present invention, as defined above - becomes a transgenic expression cassette when this expression cassette is modified by non-natural, synthetic ("artificial") methods such as, for example, mutagenic treatment. Suitable methods are described, for example, in US 5,565,350 or WO 00/15815.

A transgenic plant for the purposes of the invention is thus understood as meaning, as above, that the nucleic acids used in the method of the invention are not present in, or originating from, the genome of said plant, or are present in the genome of said plant but not at their natural locus in the genome of said plant, it being for the nucleic acids to be expressed homologously or heterologously, However, as mentioned, transgenic also means that, while the nucleic acids according to the invention or used in the inventive method are at their natural position in the genome of a plant, the sequence has modified with regard to the natural sequence, and/or that the regulatory sequences of the natural sequences have been modified. Transgenic is preferably understood as meaning the expression of the nucleic acids according to the invention at an unnatural locus in the genome, i.e. homologous or, preferably, heterologous expression of the nucleic acids takes place. Preferred transgenic plants are mentioned herein.

It shall further be noted that in the context of the present invention, the term "isolated nucleic acid" or "isolated polypeptide" may in some instances be considered as a synonym for a "recombinant nucleic acid" or a "recombinant polypeptide", respectively and refers to a nucleic or polypeptide that is not located in its natural genetic environment and/or that has been modified by recombinant methods.

In one embodiment an isolated nucleic acid sequence or isolated nucleic acid molecule is one that is not in its native surrounding or it native nucleic acid neighbourhood, yet is physically and functionally connected to other nucleic acid sequences or nucleic acid molecules and is found as part of a nucleic acid construct, vector sequence or chromosome.

Transgenic

As used herein, the term "transgenic" refers to an organism, e.g., a plant, plant cell, callus, plant tissue, or plant part that exogenously contains the nucleic acid, recombinant construct, vector or expression cassette described herein or a part thereof which is preferably introduced by non-essentially biological processes that are not essentially biological, preferably by Agrobacteria transformation. A transgenic plant for the purposes of the invention is thus understood as meaning, as above, that the nucleic acids described herein are not present in, or not originating from the genome of said plant, or are present in the genome of said plant but not at their natural genetic environment in the genome of said plant, it being possible for the nucleic acids to be expressed homologously or heterologously

Modulation

The term "modulation" means in relation to expression or gene expression, a process in which the expression level is changed by said gene expression in comparison to the control plant, the expression level may be increased or decreased. The original, unmodulated expression may be of any kind of expression of a structural RNA (rRNA, tRNA) or mRNA with subsequent translation. For the purposes of this invention, the original unmodulated expression may also be absence of any expression. The term "modulating the activity" or the term "modulating expression" shall mean any change of the expression of the inventive nucleic acid sequences or encoded proteins, which leads to increased yield and/or increased growth of the plants. The expression can increase from zero (absence of, or immeasurable expression) to a certain amount, or can decrease from a certain amount to immeasurable small amounts or zero.

Expression

The term "expression" or "gene expression" means the transcription of a specific gene or specific genes or specific genetic construct. The term "expression" or "gene expression" in particular means the transcription of a gene or genes or genetic construct into structural RNA (rRNA, tRNA) or mRNA with or without subsequent translation of the latter into a protein. The process includes transcription of DNA and processing of the resulting mRNA product.

Increased expression/overexpression

The term "increased expression" or "overexpression" as used herein means any form of expression that is additional to the original wild-type expression level. For the purposes of this invention, the original wild-type expression level might also be zero, i.e. absence of expression or immeasurable expression. Reference herein to "increased expression" is taken to mean an increase in gene expression and/or as far as referring to polypeptides polypeptide levels and/or polypeptide activity relative to control plants. The increase in expression is in increasing order of preference at least 10%, 20%, 30%, 40% or 50%, 60%, 70%, 80%, 85%, 90%, or 95%, 96%, 97%, 98%, 99% or even more compared to that of control plants. Methods for increasing of genes or gene products are well documented in the art and include, for example, overexpression driven by appropriate promoters, the use of transcription enhancers or translation enhances. Isolated nucleic acids which serve us promoter or enhancer elements may be introduced in an appropriate position (typically upstream) of a non-heterologous form of a polynucleotide so as to upregulate expression of a nucleic acid encoding the polypeptide of interest. For example, endogenous promoters may be altered in vivo by mutation, deletion, and/or substitution (see, Kmiec, US 5,565,350; Zarling et al., WO9322443), or isolated promoters may be introduced into a plant cell in the proper orientation and distance from a gene of the present invention so as to control the expression of the gene.

If polypeptide expression is desired, it is generally desirable to include a polyadenylation region at the 3'-end of a polynucleotide coding region. The polyadenylation region can be derived from the natural gene, from a variety of other plant genes, or from T-DNA. The 3' end sequence to be added may be derived from, for example, the nopaline synthase or octopine synthase genes, or alternatively from another plant gene, or less preferably from any other eukaryotic gene.

An intron sequence may also be added to the 5' untranslated region (UTR) or the coding sequence of the partial coding sequence to increase the amount of the mature message that accumulates in the cytosol. Inclusion of a spliceable intron in the transcription unit in both plant and animal expression constructs has been shown to increase gene expression at both the mRNA and protein levels up to 1000-fold (Buchman and Berg (1988) Mol. Cell biol. 8: 4395-4405; Callis et al. (1987) Genes Dev 1:1183-1200). Such intron enhancement of gene expression is typically greatest when placed near the 5' end of the transcription unit. Use of the maize introns Adh1-S intron 1, 2, and 6, the Bronze-1 intron are known in the art. For general information see: The Maize Handbook, Chapter 116, Freeling and Walbot, Eds., Springer, N.Y. (1994).

For increased expression or overexpression of the polypeptide most commonly the nucleic acid encoding said polypeptide is overexpressed in sense orientation and with a polyadenylation signal. Introns or other enhancing elements may be used in addition to a promoter suitable for the desired overexpression in the spatial and local distribution intended.

In contrast to this, overexpression of the same nucleic acid sequence as antisense construct will not result in increased expression of the protein, but decreased expression of the protein.

Decreased expression

Reference herein to "decreased expression" or "reduction or substantial elimination" of expression is taken to mean a decrease in endogenous gene expression and/or polypeptides levels and/or polypeptide activity relative to control plants. The reduction or substantial elimination is in increasing order of preference at least 10%, 20%, 30%, 40% or 50%, 60%, 70%, 80%, 85%, 90%, or 95%, 96%, 97%, 98%, 99% or more reduced compared to that of control plants.

For the reduction or substantial elimination of expression an endogenous gene in a plant, a sufficient length of substantially contiguous nucleotides of a nucleic acid sequence is required. In order to perform gene silencing, this may be as little as 20, 19, 18,17, 16, 15, 14, 13, 12, 11, 10 or fewer nucleotides, alternatively this may be as much as the entire gene (including the 5' and/or 3' UTR, either in part or in whole). The stretch of substantially contiguous nucleotides may be derived from the nucleic acid encoding the protein of interest (target gene), or from any nucleic acid capable of encoding an orthologue, paralogue or homologue of the protein of interest. Preferably, the stretch of substantially contiguous nucleotides is capable of forming hydrogen bonds with the target gene (either sense or antisense strand), more preferably, the stretch of substantially contiguous nucleotides has, in increasing order of preference, 50%, 60%, 70%, 80%, 85%, 90%, 95%, 96%, 97%, 98%, 99%, 100% sequence identity to the target gene (either sense or antisense strand). A nucleic acid sequence encoding a (functional) polypeptide is not a requirement for the various methods discussed herein for the reduction or substantial elimination of expression of an endogenous gene.

This reduction or substantial elimination of expression may be achieved using routine tools and techniques. A preferred method for the reduction or substantial elimination of endogenous gene expression is by introducing, preferably by recombinant methods, and expressing in a plant a genetic construct into which the nucleic acid (in this case a stretch of substantially contiguous nucleotides derived from the gene of interest, or from any nucleic acid capable of encoding an orthologue, paralogue or homologue of any one of the protein of interest) is cloned as an inverted repeat (in part or completely), separated by a spacer (non-coding DNA).

In such a preferred method, expression of the endogenous gene is reduced or substantially eliminated through RNA-mediated silencing using an inverted repeat of a nucleic acid or a part thereof (in this case a stretch of substantially contiguous nucleotides derived from the gene of interest, or from any nucleic acid capable of encoding an orthologue, paralogue or homologue of the protein of interest), preferably capable of forming a hairpin structure. The inverted repeat is cloned in an expression vector comprising control sequences. A non-coding DNA nucleic acid sequence (a spacer, for example a matrix attachment region fragment (MAR), an intron, a polylinker, etc.) is located between the two inverted nucleic acids forming the inverted repeat. After transcription of the inverted repeat, a chimeric RNA with a self-complementary structure is formed (partial or complete) This double-stranded RNA structure is referred to as the hairpin RNA (hpRNA). The hpRNA is processed by the plant into siRNAs that are incorporated into an RNA-induced silencing complex (RISC). The RISC further cleaves the mRNA transcripts, thereby substantially reducing the number of mRNA transcripts to be translated into polypeptides. For further general details see for example, Grierson et al. (1998) WO 98/53083; Waterhouse et al. (1999) WO 99/53050).

Performance of the methods of the invention does not rely on introducing and expressing in a plant a genetic construct into which the nucleic acid is cloned as an inverted repeat, but any one or more of several well-known "gene silencing" methods may be used to achieve the same effects.

One such method for the reduction of endogenous gene expression is RNA-mediated silencing of gene expression (downregulation). Silencing in this case is triggered in a plant by a double stranded RNA sequence (dsRNA) that is substantially similar to the target endogenous gene. This dsRNA is further processed by the plant into about 20 to about 26 nucleotides called short interfering RNAs (siRNAs). The siRNAs are incorporated into an DNA-induced silencing complex (RISC) that cleaves the mRNA transcript of the endogenous target gene, thereby substantially reducing the number of mRNA transcripts to be translated into a polypeptide. Preferably, the double stranded RNA sequence corresponds to a target gene.

Another example of an RNA silencing method involves the introduction of nucleic acid sequences or parts thereof (in this case a stretch of substantially contiguous nucleotides derived from the gene of interest, or from any nucleic acid capable of encoding an orthologue, paralogue or homologue of the protein of interest) in a sense orientation into a plant. "Sense orientation" refers to a DNA sequence that is homologous to an mRNA transcript thereof. Introduced into a plant would therefore be at least one copy of the nucleic acid sequence. The additional nucleic acid sequence will reduce expression of the endogenous gene, giving rise to a phenomenon known as co-suppression. The reduction of gene expression will be more pronounced if several additional copies of a nucleic acid sequence are introduced into the plant, as there is a positive correlation between high transcript levels and the triggering of co-suppression.

Another example of an RNA silencing method involves the use of antisense nucleic acid sequences. An "antisense" nucleic acid sequence comprises a nucleotide sequence that is complementary to a "sense" nucleic acid sequence encoding a protein, i.e. complementary to the coding strand of a double-stranded cDNA molecule or complementary to an mRNA transcript sequence. The antisense nucleic acid sequence is preferably complementary to the endogenous gene to be silenced. The complementarity may be located in the "coding region" and/or in the "non-coding region" of a gene. The term "coding region" refers to a region of the nucleotide sequence comprising codons that are translated into amino acid residues. The term "non-coding region" refers to 5' and 3' sequences that flank the coding region that are transcribed but not translated into amino acids (also referred to as 5' and 3' untranslated regions).

Antisense nucleic acid sequences can be designed according to the rules of Watson and Crick base pairing. The antisense nucleic acid sequence may be complementary to the entire nucleic acid sequence (in this case a stretch of substantially contiguous nucleotides derived from the gene of interest, or from any nucleic acid capable of encoding an orthologue, paralogue or homologue of the protein of interest), but may also be an oligonucleotide that is antisense to only a part of the nucleic acid sequence (including the mRNA 5' and 3' UTR). For example, the antisense oligonucleotide sequence may be complementary to the region surrounding the translation start site of an mRNA transcript encoding a polypeptide. The length of a suitable antisense oligonucleotide sequence is known in the art and may start from about 50, 45, 40, 35, 30, 25, 20, 15 or 10 nucleotides in length or less. An antisense nucleic acid sequence according to the invention may be constructed using chemical synthesis and enzymatic ligation reactions using methods known in the art. For example, an antisense nucleic acid sequence (e.g., an antisense oligonucleotide sequence) may be chemically synthesized using naturally occurring nucleotides or variously modified nucleotides designed to increase the biological stability of the molecules or to increase the physical stability of the duplex formed between the antisense and sense nucleic acid sequences, e.g., phosphorothioate derivatives and acridine substituted nucleotides may be used. Examples of modified nucleotides that may be used to generate the antisense nucleic acid sequences are well known in the art. Known nucleotide modifications include methylation, cyclization and 'caps' and substitution of one or more of the naturally occurring nucleotides with an analogue such as inosine. Other modifications of nucleotides are well known in the art.

The antisense nucleic acid sequence can be produced biologically using an expression vector into which a nucleic acid sequence has been subcloned in an antisense orientation (i.e., RNA transcribed from the inserted nucleic acid will be of an antisense orientation to a target nucleic acid of interest). Preferably, production of antisense nucleic acid sequences in plants occurs by means of a stably integrated nucleic acid construct comprising a promoter, an operably linked antisense oligonucleotide, and a terminator.

The nucleic acid molecules used for silencing in the methods of the invention (whether introduced into a plant or generated in situ) hybridize with or bind to mRNA transcripts and/or genomic DNA encoding a polypeptide to thereby inhibit expression of the protein, e.g., by inhibiting transcription and/or translation. The hybridization can be by conventional nucleotide complementarity to form a stable duplex, or, for example, in the case of an antisense nucleic acid sequence which binds to DNA duplexes, through specific interactions in the major groove of the double helix. Antisense nucleic acid sequences may be introduced into a plant by transformation or direct injection at a specific tissue site. Alternatively, antisense nucleic acid sequences can be modified to target selected cells and then administered systemically. For example, for systemic administration, antisense nucleic acid sequences can be modified such that they specifically bind to receptors or antigens expressed on a selected cell surface, e.g., by linking the antisense nucleic acid sequence to peptides or antibodies which bind to cell surface receptors or antigens. The antisense nucleic acid sequences can also be delivered to cells using the vectors described herein.

According to a further aspect, the antisense nucleic acid sequence is an a-anomeric nucleic acid sequence. An a-anomeric nucleic acid sequence forms specific double-stranded hybrids with complementary RNA in which, contrary to the usual b-units, the strands run parallel to each other (Gaultier et al. (1987) Nucl Ac Res 15: 6625-6641), The antisense nucleic acid sequence may also comprise a 2'-o-methylribonucleotide (Inoue et al. (1987) Nucl Ac Res 15, 6131-6148) or a chimeric RNA-DNA analogue (Inoue et al. (1987) FEBS Lett. 215, 327-330).

The reduction or substantial elimination of endogenous gene expression may also be performed using ribozymes. Ribozymes are catalytic RNA molecules with ribonuclease activity that are capable of cleaving a single-stranded nucleic acid sequence, such as an mRNA, to which they have a complementary region. Thus, ribozymes (e.g., hammerhead ribozymes (described in Haselhoff and Gerlach (1988) Nature 334, 585-591) can be used to catalytically cleave mRNA transcripts encoding a polypeptide, thereby substantially reducing the number of mRNA transcripts to be translated into a polypeptide. A ribozyme having specificity for a nucleic acid sequence can be designed (see for example: Cech et al. U.S. Patent No. 4,987,071; and Cech et al. U.S. Patent No. 5,116,742). Alternatively, mRNA transcripts corresponding to a nucleic acid sequence can be used to select a catalytic DNA having a specific ribonuclease activity from a pool of RNA molecules (Bartel and Szostak (1993) Science 261, 1411-1418). The use of ribozymes for gene silencing in plants is known in the art (e.g., Atkins et al. (1994) WO 94/00012; Lenne et al. (1995) WO 95/03404; Lutziger et al. (2000) WO 00/00619; Prinsen et al. (1997) WO 97/13865 and Scott et al. (1997) WO 97/38116).

Gene silencing may also be achieved by insertion mutagenesis (for example, T-DNA insertion or transposon insertion) or by strategies as described by, among others, Angell and Baulcombe ((1999) Plant J 20(3): 357-62), (Amplicon VIGS WO 98/36083), or Baulcombe (WO 99/15682).

Gene silencing may also occur if there is a mutation on an endogenous gene and/or a mutation on an isolated gene/nucleic acid subsequently introduced into a plant. The reduction or substantial elimination may be caused by a non-functional polypeptide. For example, the polypeptide may bind to various interacting proteins; one or more mutation (s) and/or truncation(s) may therefore provide for a polypeptide that is still able to bind interacting proteins (such as receptor proteins) but that cannot exhibit its normal function (such as signalling ligand).

A further approach to gene silencing is by targeting nucleic acid sequences complementary to the regulatory region of the gene (e.g., the promoter and/or enhancers) to form triple helical structures that prevent transcription of the gene in target cells. See Helene, C., Anticancer Drug Res. 6, 569-84, 1991; Helene et al., Ann. N.Y. Acad. Sci. 660, 27-36 1992; and Maher, L.J. Bioassays 14, 807-15, 1992.

Other methods, such as the use of antibodies directed to an endogenous polypeptide for inhibiting its function in planta, or interference in the signalling pathway in which a polypeptide is involved, will be well known to the skilled man. In particular, it can be envisaged that manmade molecules may be useful for inhibiting the biological function of a target polypeptide, or for interfering with the signalling pathway in which the target polypeptide is involved.

Alternatively, a screening program may be set up to identify in a plant population natural variants of a gene, which variants encode polypeptides with reduced activity. Such natural variants may also be used for example, to perform homologous recombination.

Artificial and/or natural microRNAs (miRNAs) may be used to knock out gene expression and/or mRNA translation. Endogenous miRNAs are single stranded small RNAs of typically 19-24 nucleotides long. They function primarily to regulate gene expression and/ or mRNA translation. Most plant microRNAs (miRNAs) have perfect or near-perfect complementarity with their target sequences. However, there are natural targets with up to five mismatches. They are processed from longer non-coding RNAs with characteristic fold-back structures by double-strand specific RNases of the Dicer family. Upon processing, they are incorporated in the RNA-induced silencing complex (RISC) by binding to its main component, an Argonaute protein. MiRNAs serve as the specificity components of RISC, since they base-pair to target nucleic acids, mostly mRNAs, in the cytoplasm. Subsequent regulatory events include target mRNA cleavage and destruction and/or translational inhibition. Effects of miRNA overexpression are thus often reflected in decreased mRNA levels of target genes.

Artificial microRNAs (amiRNAs), which are typically 21 nucleotides in length, can be genetically engineered specifically to negatively regulate gene expression of single or multiple genes of interest. Determinants of plant microRNA target selection are well known in the art. Empirical parameters for target recognition have been defined and can be used to aid in the design of specific amiRNAs, (Schwab et al., Dev. Cell 8, 517-527, 2005). Convenient tools for design and generation of amiRNAs and their precursors are also available to the public (Schwab et al., Plant Cell 18, 1121-1133, 2006).

For optimal performance, the gene silencing techniques used for reducing expression in a plant of an endogenous gene requires the use of nucleic acid sequences from monocotyledonous plants for transformation of monocotyledonous plants, and from dicotyledonous plants for transformation of dicotyledonous plants. Preferably, a nucleic acid sequence from any given plant species is introduced into that same species. For example, a nucleic acid sequence from rice is transformed into a rice plant. However, it is not an absolute requirement that the nucleic acid sequence to be introduced originates from the same plant species as the plant in which it will be introduced. It is sufficient that there is substantial homology between the endogenous target gene and the nucleic acid to be introduced.

Described above are examples of various methods for the reduction or substantial elimination of expression in a plant of an endogenous gene. A person skilled in the art would readily be able to adapt the aforementioned methods for silencing so as to achieve reduction of expression of an endogenous gene in a whole plant or in parts thereof through the use of an appropriate promoter, for example.

Transformation

The term "introduction" or "transformation" as referred to herein encompasses the transfer of an exogenous polynucleotide into a host cell, irrespective of the method used for transfer. Plant tissue capable of subsequent clonal propagation, whether by organogenesis or embryogenesis, may be transformed with a genetic construct of the present invention and a whole plant regenerated there from. The particular tissue chosen will vary depending on the clonal propagation systems available for, and best suited to, the particular species being transformed. Exemplary tissue targets include leaf disks, pollen, embryos, cotyledons, hypocotyls, megagametophytes, callus tissue, existing meristematic tissue (e.g., apical meristem, axillary buds, and root meristems), and induced meristem tissue (e.g., cotyledon meristem and hypocotyl meristem). The polynucleotide may be transiently or stably introduced into a host cell and may be maintained non-integrated, for example, as a plasmid. Alternatively, it may be integrated into the host genome. The resulting transformed plant cell may then be used to regenerate a transformed plant in a manner known to persons skilled in the art. Alternatively, a plant cell that cannot be regenerated into a plant may be chosen as host cell, i.e. the resulting transformed plant cell does not have the capacity to regenerate into a (whole) plant.

The transfer of foreign genes into the genome of a plant is called transformation. Transformation of plant species is now a fairly routine technique. Advantageously, any of several transformation methods may be used to introduce the gene of interest into a suitable ancestor cell. The methods described for the transformation and regeneration of plants from plant tissues or plant cells may be utilized for transient or for transformation. Transformation methods include the use of liposomes, electroporation, chemicals that increase free DNA uptake, injection of the DNA directly into the plant, particle gun bombardment, transformation using viruses or pollen and microprojection. Methods may be selected from the calcium/polyethylene glycol method for protoplasts (Krens, F.A. et al., (1982) Nature 296, 72-74; Negrutiu I et al. (1987) Plant Mol Biol 8: 363-373); electroporation of protoplasts (Shillito R.D. et al. (1985) Bio/Technol 3, 1099-1102); microinjection into plant material (Crossway A et al., (1986) Mol. Gen Genet 202: 179-185); DNA or RNA-coated particle bombardment (Klein TM et al., (1987) Nature 327: 70) infection with (non-integrative) viruses and the like. Transgenic plants, including transgenic crop plants, are preferably produced via *Agrobacterium*-mediated transformation. An advantageous transformation method is the transformation *in planta.* To this end, it is possible, for example, to allow the agrobacteria to act on plant seeds or to inoculate the plant meristem with agrobacteria. It has proved particularly expedient in accordance with the invention to allow a suspension of transformed agrobacteria to act on the intact plant or at least on the flower primordia. The plant is subsequently grown on until the seeds of the treated plant are obtained (Clough and Bent, Plant J. (1998) 16, 735-743). Methods for *Agrobacterium*-mediated transformation of rice include well known methods for rice transformation, such as those described in any of the following: European patent application EP 1198985 A1, Aldemita and Hodges (Planta 199: 612-617, 1996); Chan et al. (Plant Mol Biol 22 (3): 491-506, 1993), Hiei et al. (Plant J 6 (2): 271-282, 1994), which disclosures are incorporated by reference herein as if fully set forth. In the case of corn transformation, the preferred method is as described in either Ishida et al. (Nat. Biotechnol 14(6): 745-50, 1996) or Frame et al. (Plant Physiol 129(1): 13-22, 2002), which disclosures are incorporated by reference herein as if fully set forth. Said methods are further described by way of example in B. Jenes et al., Techniques for Gene Transfer, in: Transgenic Plants, Vol. 1, Engineering and Utilization, eds. S.D. Kung and R. Wu, Academic Press (1993) 128-143 and in Potrykus Annu. Rev. Plant Physiol. Plant Molec. Biol. 42 (1991) 205-225). The nucleic acids or the construct to be expressed is preferably cloned into a vector, which is suitable for transforming *Agrobacterium tumefaciens,* for example pBin19 (Bevan et al., Nucl. Acids Res. 12 (1984) 8711). Agrobacteria transformed by such a vector can then be used in known manner for the transformation of plants, such as plants used as a model, like *Arabidopsis* (*Arabidopsis thaliana* is within the scope of the present invention not considered as a crop plant), or crop plants such as, by way of example, tobacco plants, for example by immersing bruised leaves or chopped leaves in an agrobacterial solution and then culturing them in suitable media. The transformation of plants by means of *Agrobacterium tumefaciens* is described, for example, by Höfgen and Willmitzer in Nucl. Acid Res. (1988) 16, 9877 or is known inter alia from F.F. White, Vectors for Gene Transfer in Higher Plants; in Transgenic Plants, Vol. 1, Engineering and Utilization, eds. S.D. Kung and R. Wu, Academic Press, 1993, pp. 15-38.

In addition to the transformation of somatic cells, which then have to be regenerated into intact plants, it is also possible to transform the cells of plant meristems and in particular those cells which develop into gametes. In this case, the transformed gametes follow the natural plant development, giving rise to transgenic plants. Thus, for example, seeds of *Arabidopsis* are treated with agrobacteria and seeds are obtained from the developing plants of which a certain proportion is transformed and thus transgenic [Feldman, KA and Marks MD (1987). Mol Gen Genet 208:1-9; Feldmann K (1992). In: C Koncz, N-H Chua and J Shell, eds, Methods in Arabidopsis Research. Word Scientific, Singapore, pp.

274-289]. Alternative methods are based on the repeated removal of the inflorescences and incubation of the excision site in the center of the rosette with transformed agrobacteria, whereby transformed seeds can likewise be obtained at a later Point in time (Chang (1994). Plant J. 5: 551-558; Katavic (1994). Mol Gen Genet, 245: 363-370). However, an especially effective method is the vacuum infiltration method with its modifications such as the "floral dip" method. In the case of vacuum infiltration of *Arabidopsis,* intact plants under reduced pressure are treated with an agrobacterial suspension [Bechthold, N (1993). C R Acad Sci Paris Life Sci, 316:1194-1199], while in the case of the "floral dip" method the developing floral tissue is incubated briefly with a surfactant-treated agrobacterial suspension [Clough, SJ and Bent AF (1998) The Plant J. 16, 735-743]. A certain proportion of transgenic seeds are harvested in both cases, and these seeds can be distinguished from non-transgenic seeds by growing under the above-described selective conditions. In addition the stable transformation of plastids is of advantages because plastids are inherited maternally is most crops reducing or eliminating the risk of transgene flow through pollen. The transformation of the chloroplast genome is generally achieved by a process which has been schematically displayed in Klaus et al., 2004 [Nature Biotechnology 22 (2), 225-229]. Briefly the sequences to be transformed are cloned together with a selectable marker gene between flanking sequences homologous to the chloroplast genome. These homologous flanking sequences direct site specific integration into the plastome. Plastidal transformation been described for many different plant species and an overview is given in Bock (2001) Transgenic plastids in basic research and plant biotechnology. J Mol Biol. 2001 Sep 21; 312 (3):425-38 or Maliga, P (2003) Progress towards commercialization of plastid transformation technology. Trends Biotechnol. 21, 20-28. Further biotechnological progress has recently been reported in form of marker free plastid transformants, which can be produced by a transient co-integrated maker gene (Klaus et al., 2004, Nature Biotechnology 22(2), 225-229).

The genetically modified plant cells can be regenerated via all methods with which the skilled worker is familiar. Suitable methods can be found in the abovementioned publications by S.D. Kung and R. Wu, Potrykus or Höfgen and Willmitzer. Alternatively, the genetically modified plant cells are non-regenerable into a whole plant.

Generally after transformation, plant cells or cell groupings are selected for the presence of one or more markers which are encoded by plant-expressible genes co-transferred with the gene of interest, following which the transformed material is regenerated into a whole plant. To select transformed plants, the plant material obtained in the transformation is, as a rule, subjected to selective conditions so that transformed plants can be distinguished from untransformed plants. For example, the seeds obtained in the above-described manner can be planted and, after an initial growing period, subjected to a suitable selection by spraying. A further possibility consists in growing the seeds, if appropriate after sterilization, on agar plates using a suitable selection agent so that only the transformed seeds can grow into plants. Alternatively, the transformed plants are screened for the presence of a selectable marker such as the ones described above.

Following DMA transfer and regeneration, putatively transformed plants may also be evaluated, for instance using Southern analysis, for the presence of the gene of interest, copy number and/or genomic organisation. Alternatively or additionally, expression levels of the newly introduced DNA may be monitored using Northern and/or Western analysis, both techniques being well known to persons having ordinary skill in the art.

The generated transformed plants may be propagated by a variety of means, such as by clonal propagation or classical breeding techniques. For example, a first generation (or T1) transformed plant may be selfed and homozygous second-generation (or T2) transformants selected, and the T2 plants may then further be propagated through classical breeding techniques. The generated transformed organisms may take a variety of forms. For example, they may be chimeras of transformed cells and non-transformed cells; clonal transformants (e.g., all cells transformed to contain the expression cassette); grafts of transformed and untransformed tissues (e.g., in plants, a transformed rootstock grafted to an untransformed scion).

Throughout this application a plant, plant part, seed or plant cell transformed with - or interchangeably transformed by - a construct or transformed with or by a nucleic acid is to be understood as meaning a plant, plant part, or plant cell that carries said construct or said nucleic acid as a transgene due the result of an introduction of said construct or said nucleic acid by biotechnological means. The plant, plant part, seed or plant cell therefore comprises said recombinant construct or said recombinant nucleic acid. Any plant, plant part, seed or plant cell that no longer contains said recombinant construct or said recombinant nucleic acid after introduction in the past, is termed null-segregant, nullizygote or null control, but is not considered a plant, plant part, seed or plant cell transformed with said construct or with said nucleic acid within the meaning of this application.

<u>T-DNA activation tagging</u>

"T-DNA activation" tagging (Hayashi et al. Science (1992) 1350-1353), involves insertion of T-DNA, usually containing a promoter (may also be a translation enhancer or an intron), in the genomic region of the gene of interest or 10 kb up- or downstream of the coding region of a gene in a configuration such that the promoter directs expression of the targeted gene. Typically, regulation of expression of the targeted gene by its natural promoter is disrupted and the gene falls under the control of the newly introduced promoter. The promoter is typically embedded in a T-DNA. This T-DNA is randomly inserted into the plant genome, for example, through *Agrobacterium* infection and leads to modified expression of genes near the inserted T-DNA. The resulting transgenic plants show dominant phenotypes due to modified expression of genes close to the introduced promoter.

TILLING

The term "TILLING" is an abbreviation of "Targeted Induced Local Lesions In Genomes" and refers to a mutagenesis technology useful to generate and/or identify nucleic acids encoding proteins with modified expression and/or activity. TILLING also allows selection of plants carrying such mutant variants. These mutant variants may exhibit modified expression, either in strength or in location or in timing (if the mutations affect the promoter for example). These mutant variants may exhibit higher activity than that exhibited by the gene in its natural form. TILLING combines high-density mutagenesis with high-throughput screening methods. The steps typically followed in TILLING are: (a) EMS mutagenesis (Redei GP and Koncz C (1992) In Methods in Arabidopsis Research, Koncz C, Chua NH, Schell J, eds. Singapore, World Scientific Publishing Co, pp. 16-82; Feldmann et al., (1994) In Meyerowitz EM, Somerville CR, eds, Arabidopsis. Cold Spring Harbor Laboratory Press, Cold Spring Harbor, NY, pp 137-172; Lightner J and Caspar T (1998) In J Martinez-Zapater, J Salinas, eds, Methods on Molecular Biology, Vol. 82. Humana Press, Totowa, NJ, pp 91-104); (b) DNA preparation and pooling of individuals; (c) PCR amplification of a region of interest; (d) denaturation and annealing to allow formation of heteroduplexes; (e) DHPLC, where the presence of a heteroduplex in a pool is detected as an extra peak in the chromatogram; (f) identification of the mutant individual; and (g) sequencing of the mutant PCR product. Methods for TILLING are well known in the art (McCallum et al., (2000) Nat Biotechnol 18: 455-457; reviewed by Stemple (2004) Nat Rev Genet 5(2): 145-50).

Homologous recombination

"Homologous recombination" allows introduction in a genome of a selected nucleic acid at a defined selected position. Homologous recombination is a standard technology used routinely in biological sciences for lower organisms such as yeast or the moss *Physcomitrella.* Methods for performing homologous recombination in plants have been described not only for model plants (Offringa et al. (1990) EMBO J 9(10): 3077-84) but also for crop plants, for example rice (Terada et al. (2002) Nat Biotech 20(10): 1030-4; Iida and Terada (2004) Curr Opin Biotech 15(2): 132-8), and approaches exist that are generally applicable regardless of the target organism (Miller et al, Nature Biotechnol. 25, 778-785, 2007).

Yield related Trait(s)

A "Yield related trait" is a trait or feature which is related to plant yield. Yield-related traits may comprise one or more of the following non-limitative list of features: early flowering time, yield, biomass, seed yield, early vigour, greenness index, growth rate, agronomic traits, such as e.g. tolerance to submergence (which to yield in rice), Water Use Efficiency (WUE), Nitrogen Use Efficiency (NUE), etc.

The term "one or more yield related traits" is to be understood to refer to one yield related trait, or two, or three, or four, or five, or six or seven or eight or nine or ten, or more than ten yield related traits of one plant compared with a control plant.

Reference herein to "enhanced yield-related trait" is taken to mean an increase relative to control plants in a yield-related trait, for instance in early vigour and/or in biomass, of a whole plant or of one or more parts of a plant, which may include (i) aboveground parts, preferably aboveground harvestable parts, and/or (ii) parts below ground, preferably harvestable parts below ground.

one or more enhanced yield-related traitsIn particular, such harvestable parts are roots such as taproots, stems, beets, tubers, leaves, flowers or seeds, and performance of the of the invention results in plants having increased seed yield relative to the seed yield of control plants, and/or increased aboveground biomass, in particular stem biomass relative to the aboveground biomass, and in particular stem biomass of control plants, and/or increased root biomass relative to the root biomass of control plants and/or increased beet biomass relative to the beet biomass of control plants. Moreover, it is particularly contemplated that the sugar content (in particular the sucrose content) in the above ground parts, particularly stem (in particular of sugar cane plants) and/or in the belowground parts, in particular in roots including taproots and tubers, and/or in (in particular in sugar beets) is increased relative to the sugar content (in particular the sucrose content) in corresponding part(s) of the control plant.

Yield

The term "yield" in general means a measurable produce of economic value, typically related to a specified crop, to an area, and to a period of time. Individual plant parts directly contribute to yield based on their number, size and/or weight, or the actual yield is the yield per square meter for a crop and year, which is determined by dividing total production (incudes both harvested and appraised production) by planted square meters.

The terms "yield" of a plant and "plant yield" are used interchangeably herein and are meant to refer to vegetative biomass such as root and/or shoot biomass, to reproductive organs, and/or to propagules such as seeds of that plant.

Flowers in maize are unisexual; male inflorescences (tassels) originate from the apical stem and female inflorescences (ears) arise from axillary bud apices, The female inflorescence produces pairs of spikelets on the surface of a central axis (cob). Each of the female spikelets encloses two fertile florets, one of them will usually mature into a maize kernel once fertilized. Hence a yield increase in maize may be manifested as one or more of the following: increase in the number of plants established per square meter, an increase in the number of ears plant, an increase in the number of rows, number of kernels per row, kernel weight, thousand kernel weight, ear length/diameter, increase in the seed filling rate, which is the number of filled florets (i.e. florets containing seed) divided by the total number of florets and multiplied by 100), among others.

Inflorescences in rice plants are named panicles. The panicle bears spikelets, which are the basic units of the panicles, and which consist of a pedicel and a floret. The floret is borne on the pedicel and includes a flower that is covered by two protective glumes: a larger glume (the lemma) and a shorter glume (the palea), Hence, taking rice as an example, a yield increase may manifest itself as an increase in one or more of the following: number of plants per square meter, number of panicles per plant, panicle length, number of spikelets per panicle, number of flowers (or florets) per panicle; an increase in the seed filling rate which is the number of filled florets (i.e. florets containing seeds) divided by the total number of florets and multiplied by 100; an increase in thousand kernel weight, among others.

Early flowering time

Plants having an "early flowering time" as used herein are plants which start to flower earlier than control plants. Hence this term refers to plants that show an earlier start of flowering. Flowering time of plants can be assessed by counting the number of days ("time to flower") between sowing and the emergence of a first inflorescence. The "flowering time" of a plant can for instance be determined using the method as described in WO 2007/093444.

Early vigour

"Early vigour" refers to active healthy well-balanced growth especially during early stages of plant growth, and may result from increased plant fitness due to, for example, the plants being better adapted to their environment (i.e. optimizing the use of energy resources and partitioning between shoot and root). Plants having early vigour also show increased seedling survival and a better establishment of the crop, which often results in highly uniform fields (with the crop growing in uniform manner, i.e. with the majority of plants reaching the various stages of development at substantially the same time), and often better and higher yield. Therefore, early vigour may be determined by measuring various factors, such as thousand kernel weight, percentage germination, percentage emergence, seedling growth, seedling height, root length, root and shoot biomass and many more.

Increased growth rate

The increased growth rate may be specific to one or more parts of a plant (including seeds), or may be throughout substantially the whole plant. Plants having an increased growth rate may have a shorter life cycle. The life cycle of a plant may be taken to mean the time needed to grow from a mature seed up to the stage where the plant has produced mature seeds, similar to the starting material. This life cycle may be influenced by factors such as speed of germination, early vigour, growth rate, greenness index, flowering time and speed of seed maturation. The increase in growth rate may take place at one or more stages in the life cycle of a plant or during substantially the whole plant life cycle. Increased growth rate during the early stages in the life cycle of a plant may reflect enhanced vigour. The increase in growth rate may alter the harvest cycle of a plant allowing plants to be sown later and/or harvested sooner than would otherwise be possible (a similar effect may be obtained with earlier flowering time). If the growth rate is sufficiently increased, it may allow for the further sowing of seeds of the same plant species (for example sowing and harvesting of rice plants followed by sowing and harvesting of further rice plants all within one conventional growing period). Similarly, if the growth rate is sufficiently increased, it may allow for the further sowing of seeds of different plants species (for example the sowing and harvesting of corn plants followed by, for example, the sowing and optional harvesting of soybean, potato or any other suitable plant). Harvesting additional times from the same rootstock in the case of some crop plants may also be possible. Altering the harvest cycle of a plant may lead to an increase in annual biomass production per square meter (due to an increase in the number of times (say in a year) that any particular plant may be grown and harvested). An increase in growth rate may also allow for the cultivation of transgenic plants in a wider geographical area than their wild-type counterparts, since the territorial limitations for growing a crop are often determined by adverse environmental conditions either at the time of planting (early season) or at the time of harvesting (late season). Such adverse conditions may be avoided if the harvest cycle is shortened. The growth rate may be determined by deriving various parameters from growth curves, such parameters may be: T-Mid (the time taken for plants to reach 50% of their maximal size) and T-90 (time taken for plants to reach 90% of their maximal size), amongst others.

Stress resistance

An increase in yield and/or growth rate occurs whether the plant is under non-stress conditions or whether the plant is exposed to various stresses compared to control plants. Plants typically respond to exposure to stress by growing more slowly. In conditions of severe stress, the plant may even stop growing altogether. Mild stress on the other hand is defined herein as being any stress to which a plant is exposed which does not result in the plant ceasing to grow altogether without the capacity to resume growth. Mild stress in the sense of the invention leads to a reduction in the growth of the stressed plants of less than 40%, 35%, 30% or 25%, more preferably less than 20% or 15% in comparison to the control plant under non-stress conditions. Due to advances in agricultural practices (irrigation, fertilization, pesticide treatments) severe stresses are not often encountered in cultivated crop plants. As a consequence, the compromised growth induced by mild stress is often an undesirable feature for agriculture. Abiotic stresses or interchangeably environmental stresses may be due to drought or excess water, anaerobic stress, salt stress, chemical toxicity, oxidative stress and hot, cold or freezing temperatures.

"Biotic stresses" are typically those stresses caused by pathogens, such as bacteria, viruses, fungi, nematodes and insects. "Biotic stress" is understood as the negative impact done to plants by other living organisms, such as bacteria, viruses, fungi, nematodes, insects, other animals or other plants.

The "abiotic stress" may be an osmotic stress caused by a water stress, e.g. due to drought, salt stress, or freezing stress, Abiotic stress may also be an oxidative stress or a cold stress. "Freezing stress" is intended to refer to stress due to freezing temperatures, i.e. temperatures at which available water molecules freeze and turn into ice. "Cold stress", also called "chilling stress", is intended to refer to cold temperatures, e.g. temperatures below 10°, or preferably below 5°C, but at which water molecules do not freeze. As reported in Wang et al. (Planta (2003) 218: 1-14), abiotic stress leads to a series of morphological, physiological, biochemical and molecular changes that adversely affect plant growth and productivity. Drought, salinity, extreme temperatures and oxidative stress are known to be interconnected and may induce growth and cellular damage through similar mechanisms. Rabbani et al. (Plant Physiol (2003) 133: 1755-1767) describes a particularly high degree of "cross talk" between drought stress and high-salinity stress. For example, drought and/or salinisation are manifested primarily as osmotic stress, resulting in the disruption of homeostasis and ion distribution in the cell. Oxidative stress, which frequently accompanies high or low temperature, salinity or drought stress, may cause denaturing of functional and structural proteins. As a consequence, these diverse environmental stresses often activate similar cell signalling pathways and cellular responses, such as the production of stress proteins, up-regutation of anti-oxidants, accumulation of compatible solutes and growth arrest. The term "non-stress" conditions as used herein are those environmental conditions that allow optimal growth of plants. Persons skilled in the art are aware of normal soil conditions and climatic conditions for a given location. Plants with optimal growth conditions, (grown under non-stress conditions) typically yield in increasing order of preference at least 97%, 95%, 92%, 90%, 87%, 85%, 83%, 80%, 77% or 75% of the average production of such plant in a given environment. Average production may be calculated on harvest and/or season basis. Persons skilled in the art are aware of average yield productions of a crop.

In particular, the methods of the present invention may be performed under non-stress conditions. In an example, the methods of the present invention may be performed under non-stress conditions such as mild drought to give plants having increased yield relative to control plants.

In another embodiment, the methods of the present invention may be performed under stress conditions, preferably under abiotic stress conditions.

In an example, the methods of the present invention may be performed under stress conditions such as drought to give plants having increased yield relative to control plants.

In another example, the methods of the present invention may be performed under stress conditions such as nutrient deficiency to give plants having increased yield relative to control plants.

Nutrient deficiency may result from a lack of nutrients such as nitrogen, phosphates and other phosphorous-containing compounds, potassium, calcium, magnesium, manganese, iron and boron, amongst others.

In yet another example, the methods of the present invention may be performed under stress conditions such as salt stress to give plants having increased yield relative to control plants. The term salt stress is not restricted to common salt (NaCl), but may be any one or more of: NaCl), KCl, LiCl, $MgCl_2$, $CaCl_2$, amongst others.

In yet another example, the methods of the present invention may be performed under stress conditions such as cold stress or freezing stress to give plants having increased yield relative to control plants.

Increase/improve/Enhance

The terms "increase", "improve" or "enhance" in the context of a yield-related trait are interchangeable and shall mean in the sense of the application at least a 3%, 4%, 5%, 6%, 7%, 8%, 9% or 10%, preferably at least 15% or 20%, more preferably 25%, 30%, 35% or 40% increase in the yield-related trait in comparison to control plants as defined herein.

Seed yield

Increased seed yield may manifest itself as one or more of the following:

a) an increase in seed biomass (total seed weight) which may be on an individual seed basis and/or per plant and/or per square meter;
b) increased number of flowers per plant;
c) increased number of seeds;
d) increased seed filling rate (which is expressed as the ratio between the number of filled florets divided by the total number of florets);
e) increased harvest index, which is expressed as a ratio of the yield of harvestable parts, such as seeds, divided by the biomass of aboveground plant parts; and
f) increased thousand kernel weight (TKW), which is extrapolated from the number of seeds counted and their total weight. An increased TKW may result from an increased seed size and/or seed weight, and may also result from an increase in embryo and/or endosperm size.

The terms "filled florets" and "filled seeds" may be considered synonyms.
An increase in seed yield may also be manifested as an increase in seed size and/or seed volume. Furthermore, an increase in seed yield may also manifest itself as an increase in seed area and/or seed length and/or seed width and/or seed perimeter.

Greenness Index

The "greenness index" as used herein is calculated from digital images of plants. For each pixel belonging to the plant object on the image, the ratio of the green value versus the red value (in the RGB model for encoding color) is calculated. The greenness index is expressed as the percentage of pixels for which the green-to-red ratio exceeds a given threshold. Under normal growth conditions, under salt stress growth conditions, and under reduced nutrient availability growth conditions, the greenness index of plants is measured in the last imaging before flowering. In contrast, under drought stress growth conditions, the greenness index of plants is measured in the first imaging after drought.

Biomass

The term "biomass" as used herein is intended to refer to the total weight of a plant or plant part. Total weight can be measured as dry weight, fresh weight or wet weight. Within the definition of biomass, a distinction may be made between the biomass of one or more parts of a plant, which may include any one or more of the following:

- aboveground parts such as but not limited to shoot biomass, seed biomass, leaf biomass, etc.;
- aboveground harvestable parts such as but not limited to shoot biomass, seed biomass, leaf biomass, stem biomass, setts etc.;
- parts below ground, such as but not limited to root biomass, tubers, bulbs, etc.;
- harvestable parts below ground, such as but not limited to root biomass, tubers, bulbs, etc.;
- harvestable parts partially below ground such as but not limited to beets and other hypocotyl areas of a plant, rhizomes, stolons or creeping rootstalks;
- vegetative biomass such as root biomass, shoot biomass, etc.;
- reproductive organs; and
- propagules such as seed.

In a preferred embodiment throughout this application any reference to "root" as biomass or harvestable parts or as organ of increased sugar content is to be understood as a reference to harvestable parts partly inserted in or in physical contact with the ground such as but not limited to beets and other hypocotyl areas of a plant, rhizomes, stolons or creeping rootstalks, but not including leaves, as well as harvestable parts belowground, such as but not limited to root, taproot, tubers or bulbs.

Marker assisted breeding

Such breeding programmes sometimes require introduction of allelic variation by mutagenic treatment of the plants, using for example EMS mutagenesis; alternatively, the programme may start with a collection of allelic variants of so called "natural" origin caused unintentionally. Identification of allelic variants then takes place, for example, by PCR. This is followed by a step for selection of superior allelic variants of the sequence in question and which give increased yield. Selection is typically carried out by monitoring growth performance of plants containing different allelic variants of the sequence in question. Growth performance may be monitored in a greenhouse or in the field. Further optional steps include crossing plants in which the superior allelic variant was identified with another plant. This could be used, for example, to make a combination of interesting phenotypic features.

Use as probes in (gene mapping)

Use of nucleic acids encoding the protein of interest for genetically and physically mapping the genes requires only a nucleic acid sequence of at least 15 nucleotides in length. These nucleic acids may be used as restriction fragment length polymorphism (RFLP) markers. Southern blots (Sambrook J, Fritsch EF and Maniatis T (1989) Molecular Cloning, A Laboratory Manual) of restriction-digested plant genomic DNA may be probed with the nucleic acids encoding the protein of interest. The resulting banding patterns may then be subjected to genetic analyses using computer programs such as MapMaker (Lander et al. (1987) Genomics 1: 174-181) in order to construct a genetic map. In addition, the nucleic acids may be used to probe Southern blots containing restriction endonuclease-treated genomic DNAs of a set of individuals representing parent and progeny of a defined genetic cross. Segregation of the DNA polymorphisms is noted and used to calculate the position of the nucleic acid encoding the protein of interest in the genetic map previously obtained using this population (Botstein et al. (1980) Am. J. Hum. Genet. 32:314-331).

The production and use of plant gene-derived probes for use in genetic mapping is described in Bernatzky and Tanksley (1986) Plant Mol. Biol. Reporter 4: 37-41. Numerous publications describe genetic mapping of specific cDNA clones using the methodology outlined above or variations thereof. For example, F2 intercross populations, backcross populations, randomly mated populations, near isogenic lines, and other sets of individuals may be used for mapping. Such methodologies are well known to those skilled in the art.

The nucleic acid probes may also be used for physical mapping (i.e., placement of sequences on physical maps; see Hoheisel et al. In: Non-mammalian Genomic Analysis: A Practical Guide, Academic press 1996, pp. 319-346, and references cited therein).

In another embodiment, the nucleic acid probes may be used in direct fluorescence in situ hybridisation (FISH) mapping (Trask (1991) Trends Genet. 7:149-154). Although current methods of FISH mapping favour use of large clones (several kb to several hundred kb; see Laan et al. (1995) Genome Res. 5:13-20), improvements in sensitivity may allow performance of FISH mapping using shorter probes.

A variety of nucleic acid amplification-based methods for genetic and physical mapping may be carried out using the nucleic acids. Examples include allele-specific amplification (Kazazian (1989) J. Lab. Clin. Med 11:95-96), polymorphism of PCR-amplified fragments (CAPS; Sheffield et al. (1993) Genomics 16:325-332), allele-specific ligation (Landegren et al. (1988) Science 241:1077-1080), nucleotide extension reactions (Sokolov (1990) Nucleic Acid Res. 18:3671), Radiation Hybrid Mapping (Walter et al. (1997) Nat. Genet. 7:22-28) and Happy Mapping (Dear and Cook (1989) Nucleic Acid Res. 17:6795-6807). For these methods, the sequence of a nucleic acid is used to design and produce primer pairs for use in the amplification reaction or in primer extension reactions. The design of such primers is well known to those skilled in the art. In methods employing PCR-based genetic mapping, it may be necessary to identify DNA sequence differences between the parents of the mapping cross in the region corresponding to the instant nucleic acid sequence. This, however, is generally not necessary for mapping methods.

Plant

The term "plant" as used herein encompasses whole plants, ancestors and progeny of the plants and plant parts, including seeds, shoots, stems, leaves, roots (including tubers), flowers, and tissues and organs, wherein each of the aforementioned comprise the gene/nucleic acid of interest. The term "plant" also encompasses plant cells, suspension cultures, callus tissue, embryos, meristematic regions, gametophytes, sporophytes, pollen and microspores, again wherein each of the aforementioned comprises the gene/nucleic acid of interest.

Plants that are particularly useful in the methods of the invention include all plants which belong to the superfamily Viridiplantae, in particular monocotyledonous and dicotyledonous plants including fodder or forage legumes, ornamental plants, food crops, trees or shrubs selected from the list comprising *Acer* spp., *Actinidia* spp., *Abelmoschus* spp., *Agave*

*sisalana, Agropyron spp., Agrostis stolonifera, Allium* spp., *Amaranthus* spp., *Ammophila arenaria, Ananas comosus, Annona* spp., *Apium graveolens, Arachis spp, Artocarpus* spp., *Asparagus officinalis, Avena* spp. *(e.g. Avena sativa, Avena fatua, Avena byzantina, Avena fatua var. sativa, Avena hybrida), Averrhoa carambola, Bambusa sp., Benincasa hispida, Bertholletia excelsea, Beta vulgaris, Brassica* spp. *(e.g. Brassica napus, Brassica rapa* ssp. [canola, oilseed rape, turnip rape]), *Cadaba farinosa, Camellia sinensis, Canna indica, Cannabis sativa, Capsicum* spp., *Carex elata, Carica papaya, Carissa macrocarpa, Carya* spp., *Carthamus tinctorius, Castanea* spp., *Ceiba pentandra, Cichorium endivia, Cinnamomum* spp., *Citrullus lanatus, Citrus* spp., *Cocos* spp., *Coffea* spp., *Colocasia esculenta*, *Cola* spp., *Corchorus sp., Coriandrum sativum, Corylus* spp., *Crataegus* spp., *Crocus sativus, Cucurbita* spp., *Cucumis* spp., *Cynara* spp., *Daucus carota, Desmodium* spp., *Dimocarpus longan, Dioscorea* spp., *Diospyros* spp., *Echinochloa* spp., *Elaeis (e.g. Elaeis guineensis, Elaeis oleifera), Eleusine coracana, Eragrostis tef, Erianthus* sp., *Eriobotrya japonica, Eucalyptus sp., Eugenia uniflora, Fagopyrum* spp., *Fagus* spp., *Festuca arundinacea, Ficus carica, Fortunella* spp., *Fragaria* spp., *Ginkgo biloba, Glycine* spp. *(e.g. Glycine max, Soja hispida* or *Soja max), Gossypium hirsutum, Helianthus* spp. *(e.g. Helianthus annuus), Hemerocallis fulva, Hibiscus* spp., Hordeum spp. *(e.g. Hordeum vulgare), Ipomoea batatas, Juglans* spp., *Lactuca sativa, Lathyrus* spp., *Lens culinaris, Linum usitatissimum, Litchi chinensis, Lotus* spp., *Luffa acutangula, Lupinus* spp., *Luzula sylvatica, Lycopersicon* spp. *(e.g. Lycopersicon esculentum, Lycopersicon lycopersicum, Lycopersicon pyriforme), Macrotyloma* spp., *Malus* spp., *Malpighia emarginata, Mammea americana, Mangifera indica, Manihot* spp., *Manilkara zapota, Medicago sativa, Melilotus* spp., *Mentha* spp., *Miscanthus sinensis, Momordica* spp., *Morus nigra, Musa* spp., *Nicotiana* spp., *Olea* spp., *Opuntia spp., Ornithopus* spp., *Oryza* spp. *(e.g. Oryza sativa, Oryza latifolia), Panicum miliaceum, Panicum virgatum, Passiflora edulis, Pastinaca sativa, Pennisetum sp., Persea* spp., *Petroselinum crispum, Phalaris arundinacea, Phaseolus* spp., *Phleum pratense, Phoenix* spp., *Phragmites australis, Physalis* spp., *Pinus* spp., *Pistacia vera, Pisum* spp., *Poa* spp., *Populus* spp., *Prosopis* spp., *Prunus* spp., *Psidium* spp., *Punica granatum, Pyrus communis*, *Quercus* spp., *Raphanus sativus, Rheum rhabarbarum*, *Ribes* spp., *Ricinus communis, Rubus* spp., *Saccharum* spp., *Salix sp., Sambucus* spp., *Secale cereale, Sesamum* spp., *Sinapis sp*., *Solanum* spp. *(e.g. Solanum tuberosum, Solanum integrifolium or Solanum lycopersicum), Sorghum bicolor, Spinacia* spp., *Syzygium* spp., *Tagetes* spp., *Tamarindus indica, Theobroma cacao, Trifolium* spp., *Tripsacum dactyloides, Triticosecale rimpaui, Triticum* spp. *(e.g. Triticum aestivum, Triticum durum, Triticum turgidum, Triticum hybernum, Triticum macha, Triticum sativum, Triticum monococcum or Triticum vulgare), Tropaeolum minus, Tropaeolum majus, Vaccinium* spp., *Vicia* spp., *Vigna* spp., *Viola odorata, Vitis* spp., *Zea mays, Zizania palustris, Ziziphus* spp., amongst others.

With respect to the sequences of the invention, a nucleic acid or a polypeptide sequence of plant origin has the characteristic of a codon usage optimised for expression in plants, and of the use of amino acids and regulatory sites common in plants, respectively. The plant of origin may be any plant, but preferably those plants as described in the previous paragraph. Control plant(s)

The choice of suitable control plants is a routine part of an experimental setup and may include corresponding wild type plants or corresponding plants without the gene of interest. The control plant is typically of the same plant species or even of the same variety as the plant to be assessed. The control plant may also be a nullizygote of the plant to be assessed. Nullizygotes (or null control plants) are individuals missing the transgene by segregation. Further, control plants are grown under equal growing conditions to the growing conditions of the plants of the invention, i.e. in the vicinity of, and simultaneously with, the plants of the invention. A "control plant" as used herein refers not only to whole plants, but also to plant parts, including seeds and seed parts.

Propagation material / Propagule

"Propagation material" and interchangeably "propagule" is any kind of organ, tissue, or cell of a plant capable of developing into a complete plant. "Propagation material" can be based on vegetative reproduction (also known as vegetative propagation, vegetative multiplication, or vegetative cloning) or sexual reproduction. Propagation material can therefore be seeds or parts of the non-reproductive organs, like stem or leave. In particular, with respect to poaceae, suitable propagation material can also be sections of the stem, i.e., stem cuttings (like setts).

Stalk

A "stalk" is the stem of a poaceae and is also known as the "millable cane". In the context of poaceae "stalk", "stem", "shoot", or "tiller" are used interchangeably.

Sett

A "sett" is a section of the stem of a poaceae, which is suitable to be used as propagation material. Synonymous expressions to "sett" are "seed-cane", "stem cutting", "section of the stalk", and "seed piece".

## Description of figures

Throughout the figures, for each sequence of table A the shortname given in table A below is used to represent the sequence.

The present invention will now be described with reference to the following figures in which:

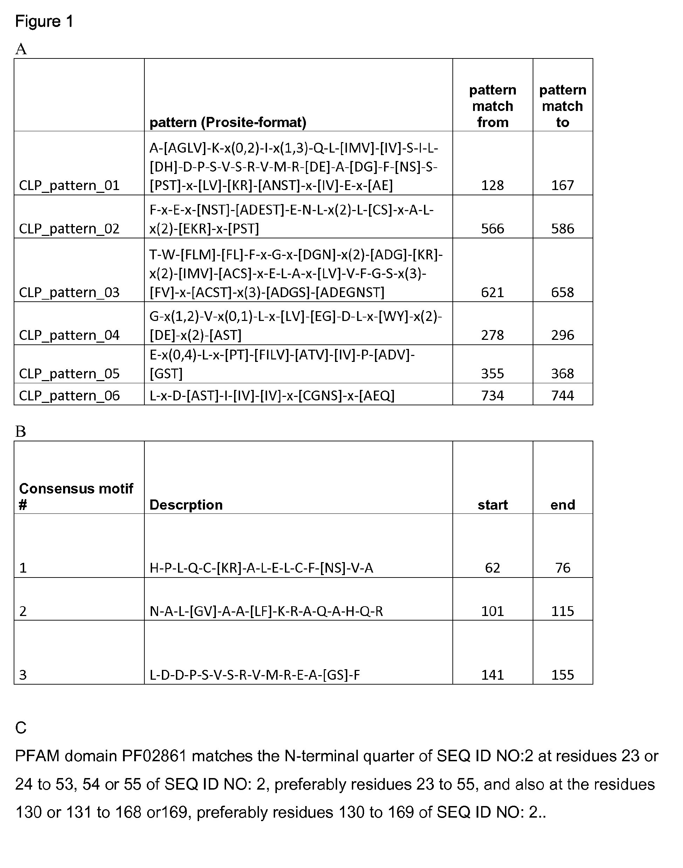

**Fig. 1** summarises the positions within the sequence of SEQ ID NO: 2 of A. the identified motifs 1 to 6 (CLP_ PATTERN_01 to CLP_PATTERN_06), and their corresponding SEQ ID Nos.; and B. of the three consensus motifs with their starting and ending amino acid residue in SEQ ID NO: 2; and C. of the PFAM domain PF02861 in the amino acid sequence as provided in SEQ ID NO: 2. Interestingly, the N-terminal part of the domain PF02861 finds a match near the N-terminus of this CLP protein while the C-terminal part of the domain PF02861 finds a match C-terminally to this as indicated by the given positions for SEQ ID NO:2

**Fig. 2** represents a multiple alignment of various POI polypeptides. Black shading indicates identical amino acids among the various protein sequences, grey shading represent highly conserved amino acid substitutions, i.e. at least 80% conserved residue, on other positions there is no sequence conservation. These alignments can be used for defining further motifs or signature sequences, when using conserved amino acids.

**Fig. 3** shows phylogenetic tree of CLP polypeptides, using AlignX from the VectorNTI suite software from Invirtrogen..

**Fig. 4** shows the seqeunce similarity and identity table of Example 3.

**Fig. 5** represents the binary vector used for increased expression in Oryza sativa of a POI-encoding nucleic acid under the control of a rice GOS2 promoter (pGOS2).

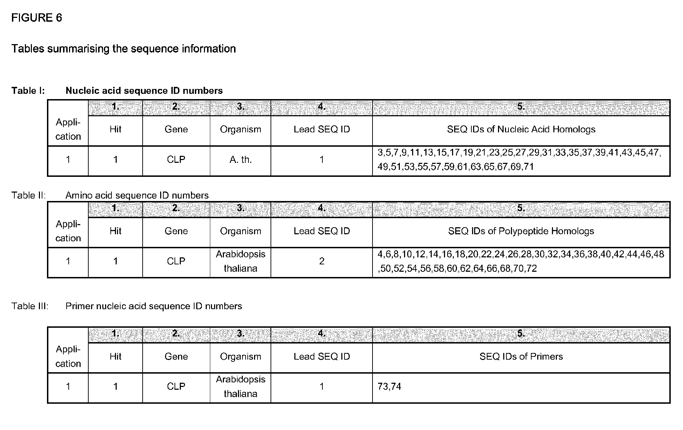

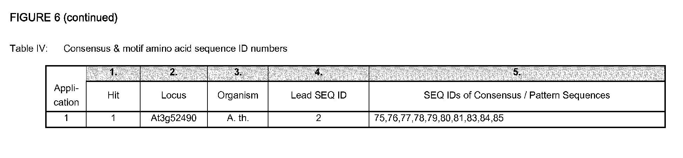

**Fig. 6** CLP summarises the relations of the different SEQ ID NOs. to the lead sequence.

## Examples

The present invention will now be described with reference to the following examples, which are by way of illustration only. The following examples are not intended to limit the scope of the invention. Unless otherwise indicated, the present invention employs conventional techniques and methods of plant biology, molecular biology, bioinformatics and plant breedings. DNA manipulation: unless otherwise stated, recombinant DNA techniques are performed according to standard protocols described in (Sambrook (2001) Molecular Cloning: a laboratory manual, 3rd Edition Cold Spring Harbor Laboratory Press, CSH, New York) or in Volumes 1 and 2 of Ausubel et al. (1994), Current Protocols in Molecular Biology, Current Protocols. Standard materials and methods for plant molecular work are described in Plant Molecular Biology Labfax (1993) by R.D.D. Croy, published by BIOS Scientific Publications Ltd (UK) and Blackwell Scientific Publications (UK).

Example 1: Identification of sequences related to SEQ ID NO: 1 and SEQ ID NO: 2

Sequences (full length cDNA, ESTs or genomic) related to SEQ ID NO: 1 and SEQ ID NO: 2 were identified amongst those maintained in the Entrez Nucleotides database at the National Center for Biotechnology Information (NCBI) using database sequence search tools, such as the Basic Local Alignment Tool (BLAST) (Altschul et al. (1990) J. Mol. Biol. 215:403-410; and Altschul et al. (1997) Nucleic Acids Res. 25:3389-3402). The program is used to find regions of local similarity between sequences by comparing nucleic acid or polypeptide sequences to sequence databases and by calculating the statistical significance of matches. For example, the polypeptide encoded by the nucleic acid of SEQ ID NO: 1 was used for the TBLASTN algorithm, with default settings and the filter to ignore low complexity sequences set off. The output of the analysis was viewed by pairwise comparison, and ranked according to the probability score (E-value), where the score reflect the probability that a particular alignment occurs by chance (the lower the E-value, the more significant the hit). In addition to E-values, comparisons were also scored by percentage identity. Percentage identity refers to the number of identical nucleotides (or amino acids) between the two compared nucleic acid (or polypeptide) sequences over a particular length. In some instances, the default parameters may be adjusted to modify the stringency of the search. For example the E-value may be increased to show less stringent matches. This way, short nearly exact matches may be identified.

Table A provides a list of nucleic acid sequences related to SEQ ID NO: 1 and SEQ ID NO: 2.

**Table A:** Examples of CLP encoding nucleic acids and polypeptides:

| Organism | Nucleic acid SEQ ID NO: | Protein SEQ ID NO: | Short name in figures |
|---|---|---|---|
| Arabidopsis thaliana | 1 | 2 | L |
| Zea mays | 3 | 4 | H_45 |

(continued)

| Organism | Nucleic acid SEQ ID NO: | Protein SEQ ID NO: | Short name in figures |
|---|---|---|---|
| Glycine max | 5 | 6 | H_21 |
| Glycine max | 7 | 8 | H_19 |
| Zea mays | 9 | 10 | H_34 |
| Zea mays | 11 | 12 | H_25 |
| Glycine max | 13 | 14 | H_10 |
| Glycine max | 15 | 16 | H_9 |
| Glycine max | 17 | 18 | H_22 |
| Glycine max | 19 | 20 | H_17 |
| Glycine max | 21 | 22 | H_16 |
| Medicago truncatula | 23 | 24 | H_20 |
| Glycine max | 25 | 26 | H_27 |
| Oryza sativa | 27 | 28 | H_38 |
| Populus trichocarpa | 29 | 30 | H_5 |
| Populus trichocarpa | 31 | 32 | H_12 |
| Populus trichocarpa | 33 | 34 | H_8 |
| Sorghum bicolor | 35 | 36 | H_32 |
| Sorghum bicolor | 37 | 38 | H_43 |
| Sorghum bicolor | 39 | 40 | H_30 |
| Sorghum bicolor | 41 | 42 | H_47 |
| Zea mays | 43 | 44 | H_36 |
| Zea mays | 45 | 46 | H_37 |
| Vitis vinifera | 47 | 48 | H_11 |
| Vitis vinifera | 49 | 50 | H_4 |
| Ricinus communis | 51 | 52 | H_18 |
| Ricinus communis | 53 | 54 | H_7 |
| Vitis vinifera | 55 | 56 | H_6 |
| Vitis vinifera | 57 | 58 | H_15 |
| Oryza sativa subsp. indica | 59 | 60 | H_39 |
| Hordeum vulgare var. distichum | 61 | 62 | H_42 |
| Vitis vinifera | 63 | 64 | H_14 |
| Vitis vinifera | 65 | 66 | H_3 |
| Oryza sativa | 67 | 68 | H_29 |
| Arabidopsis thaliana | 69 | 70 | H_2 |
| Oryza sativa subsp. japonica | 71 | 72 | H_41 |

Sequences have been tentatively assembled and publicly disclosed by research institutions, such as The Institute for Genomic Research (TIGR; beginning with TA). For instance, the Eukaryotic Gene Orthologs (EGO) database may be used to identify such related sequences, either by keyword search or by using the BLAST algorithm with the nucleic acid sequence or polypeptide sequence of interest. Special nucleic acid sequence databases have been created for particular organisms, e.g. for certain prokaryotic organisms, such as by the Joint Genome Institute. Furthermore, access to proprietary databases, has allowed the identification of novel nucleic acid and polypeptide sequences.

Example 2: Alignment of POI polypeptide sequences

The alignment was performed with the software ClustalW (version 1.83) and is described by Thompson et al. (Nucleic Acids Research 22, 4673 (1994)). The source code for the stand-alone program is publicly available from the European Molecular Biology Laboratory; Heidelberg, Germany. The analysis was performed using the default parameters of ClustalW v1.83 (gap open penalty: 10.0; gap extension penalty: 0.2; protein matrix: Gonnet; protein/DNA endgap: -1; protein/DNA gapdist: 4).

Minor manual editing was done to further optimise the alignment. The POI polypeptides are aligned in Figure 2.

A phylogenetic tree of CLP polypeptides (Figure 3) was constructed by aligning POI sequences using AlignX of the VectorNTI Software suite (Invitrogen, part of Life Technologies GmbH, Frankfurter Straße 129B, 64293 Darmstadt, Germany)

Example 3: Calculation of global percentage identity between polypeptide sequences

Global percentages of similarity and identity between full length polypeptide sequences useful in performing the methods of the invention were determined program "needle" from the EMBOSS software collection (The European Molecular Biology Open Software Suite ; http://www.ebi.ac.uk/Tools/psa/);

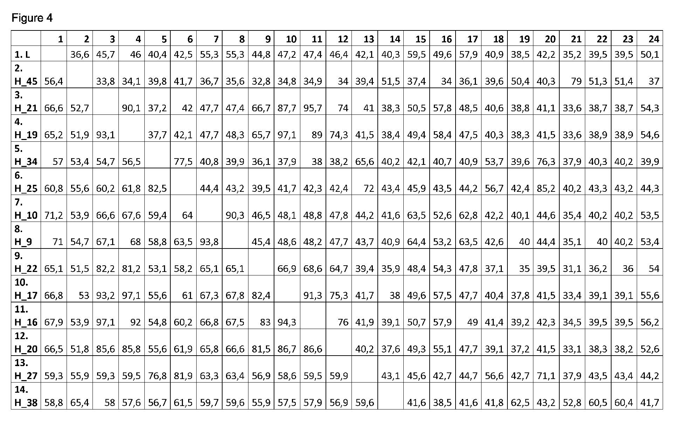

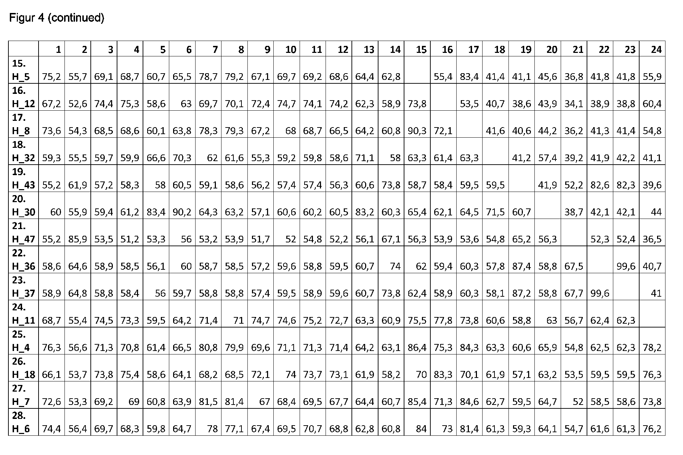

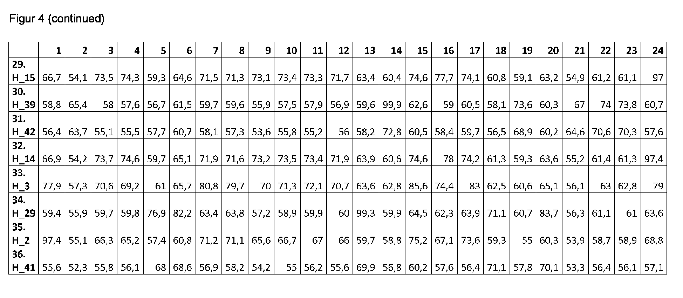

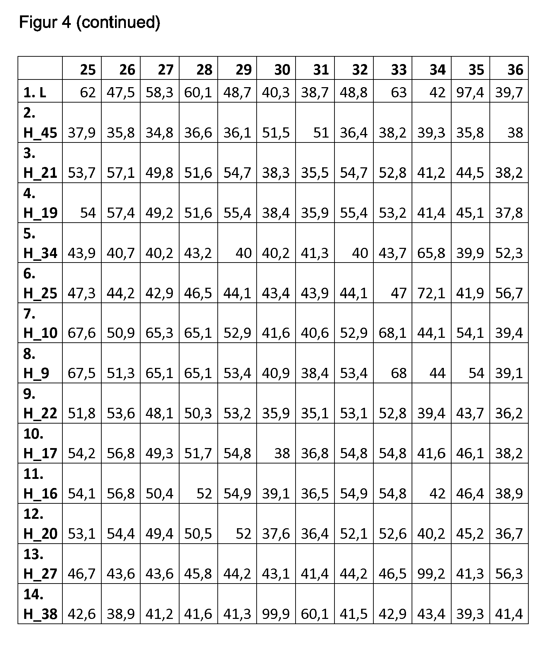

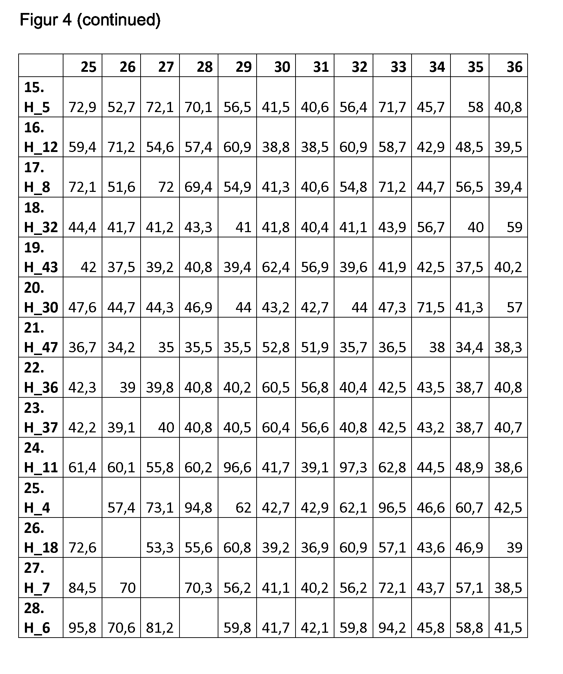

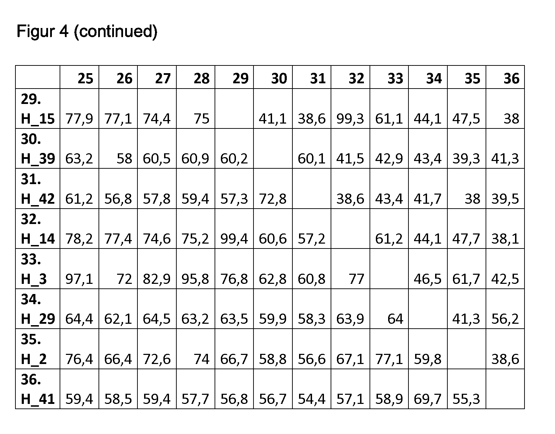

Results of the analysis are shown in Figure 4 with global similarity and identity percentages over the full length of the polypeptide sequences. Sequence similarity is shown in the bottom half of the dividing line and sequence identity is shown in the top half of the diagonal dividing line. Parameters used in the analysis were: -gapopen 10.0, -gapextend 0.5, matrix: BLOSUM62. The sequence identity (in %) between the CLP polypeptide sequences useful in performing the methods of the invention can be as low as 35 %, but is generally higher than 40%) compared to SEQ ID NO: 2.

Based on a multiple alignment of CLP polypeptides, such as for example the one of Example 2, a skilled person may select conserved sequences and submit as input for a similarity/identity analysis. This approach is useful where overall sequence conservation among CLP proteins is rather low.

Example 4: Identification of motifs and domains comprised in polypeptide sequences useful in performing the methods of the invention

The integrated Resource of Protein Families, Domains and Sites (InterPro) database is an integrated interface for the commonly used signature databases for text- and sequence-based searches. The InterPro database combines these databases, which use different methodologies and varying degrees of biological information about well-characterized proteins to derive protein signatures. Collaborating databases include SWISS-PROT, PROSITE, TrEMBL, PRINTS, ProDom and Pfam, Smart and TIGRFAMs. Pfam is a large collection of multiple sequence alignments and hidden Markov models covering many common protein domains and families. Pfam is hosted at the Sanger Institute server in the United Kingdom. Interpro is hosted at the European Bioinformatics Institute in the United Kingdom.

The results of the InterPro scan ((see Zdobnov E.M. and Apweiler R.; "InterProScan - an integration platform for the signature-recognition methods in InterPro."; Bioinformatics, 2001, 17(9): 847-8; InterPro database, release 37.0, 30 April, 2012) of the polypeptide sequence as represented by SEQ ID NO: 2 are presented in Table B.

**Table B:** InterPro scan results (major accession numbers) of the polypeptide sequence as represented by SEQ ID NO: 2. Default parameters (DB genetic code = standard; transcript length = 20) were used.

| Database | Accession number | Accession name | Amino acid coordinates on SEQ ID NO 2 |
|---|---|---|---|
| PFAM | PF02861 | ClpA domain, N-terminal | 23 or 24 to 53, 54 or 55, preferably residues 23 to 55, and 130 or 131 to 168 or169, preferably residues 130 to 169 |
| Interpro | IPR004176 | Clp, N-terminal | 23 or 24 to 53, 54 or 55, preferably residues 23 to 55, and 130 or 131 to 168 or169, preferably residues 130 to 169 |

(continued)

| Database | Accession number | Accession name | Amino acid coordinates on SEQ ID NO 2 |
|---|---|---|---|
| Interpro | IPR023150 | Double Clp-N motif | 11-166 |

In one embodiment a POI polypeptide comprises a conserved domain with at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to a conserved domain from amino acid 11 to 166 in SEQ ID NO:2.

identification of conserved motifs

Conserved patterns were identified with the Software tool MEME version 3.5. MEME was developed by Timothy L. Bailey and Charles Elkan, Dept. of Computer Science and Engeneering, University of California, San Diego, USA and is described by Timothy L. Bailey and Charles Elkan (Fitting a mixture model by expectation maximization to discover motifs in biopolymers, Pro-ceedings of the Second International Conference on Intelligent Systems for Mo-lecular Biology, pp. 28-36, AAAI Press, Menlo Park, California, 1994). The source code for the stand-alone program is public available from the San Diego Supercomputer centercentre (http://meme.sdsc.edu).

For identifying common motifs in all sequences with the software tool MEME, the following settings were used: -maxsize 500000, -nmotifs 15, -evt 0.001, -maxw 60, -distance 1e-3, -minsites number of sequences used for the analysis. Input sequences for MEME were non-aligned sequences in Fasta format. Other parameters were used in the default settings in this software version.

Prosite patterns for conserved domains were generated with the software tool Pratt version 2.1 or manually. Pratt was developed by Inge Jonassen, Dept. of of Informatics, University of Bergen, Norway and is described by Jonassen et al. (I.Jonassen, J.F.Collins and D.G.Higgins, Finding flexible patterns in unaligned protein sequences, Protein Science 4 (1995), pp. 1587-1595; I.Jonassen, Effi-cient discovery of conserved patterns using a pattern graph, Submitted to CABIOS Febr. 1997]. The source code (ANSI C) for the stand-alone program is public available, e.g. at establisched Bioinformatic centers like EBI (European Bioinformatics Institute).

For generating patterns with the software tool Pratt, following settings were used: PL (max Pattern Length): 100, PN (max Nr of Pattern Symbols): 100, PX (max Nr of consecutive x's): 30, FN (max Nr of flexible spacers): 5, FL (max Flexibility): 30, FP (max Flex.Product): 10, ON (max number patterns): 50. Input sequences for Pratt were distinct regions of the protein sequences exhibiting high similarity as identified from software tool MEME. The minimum number of sequences, which have to match the generated patterns (CM, min Nr of Seqs to Match) was set to at least 80% of the provided sequences.

The pattern identified via PROSITE and/or MEME were further processed with progam Fuzzpro, as implemented in the "The European Molecular Biology Open Software Suite" (EMBOSS), version 6.3.1.2 (Trends in Genetics 16 (6), 276 (2000)), to arrive at the motifs 1 to 6 as provided above.

Using the alignment as described in example 3, highly conserved consensus motifs 1 to 3 were identified.

In one embodiment a CLP polypeptide comprises a motif with at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to any of the six conserved motifs and/or the three consensus motifs contained in SEQ ID NO: 2 as shown by their starting and end positions in figure 1 A and B.

Example 5: Topology prediction of the CLP POI polypeptide sequences

TargetP 1.1 predicts the subcellular location of eukaryotic proteins. The location assignment is based on the predicted presence of any of the N-terminal pre-sequences: chloroplast transit peptide (cTP), mitochondrial targeting peptide (mTP) or secretory pathway signal peptide (SP). Scores on which the final prediction is based are not really probabilities, and they do not necessarily add to one. However, the location with the highest score is the most likely according to TargetP, and the relationship between the scores (the reliability class) may be an indication of how certain the prediction is. The reliability class (RC) ranges from 1 to 5, where 1 indicates the strongest prediction. For the sequences predicted to contain an N-terminal presequence a potential cleavage site can also be predicted. TargetP is maintained at the server of the Technical University of Denmark (see http://www.cbs.dtu.dk/services/TargetP/ & "Locating proteins in the cell using TargetP, SignalP, and related tools", Olof Emanuelsson, Søren Brunak, Gunnar von Heijne, Henrik Nielsen, Nature Protocols 2, 953-971 (2007)).

A number of parameters must be selected before analysing a sequence, such as organism group (non-plant or plant), cutoff sets (none, predefined set of cutoffs, or user-specified set of cutoffs), and the calculation of prediction of cleavage sites (yes or no). TargetP settings were: "plant"; cutoff cTP =0; cutoff mTP =0; cutoff SP = 0; cutoff other = 0. Cleavage site predictions included.

The results of TargetP 1.1 analysis of the polypeptide sequence as represented by SEQ ID NO: 2 are presented Table C. The "plant" organism group has been selected, no cutoffs defined, and the predicted length of the transit peptide requested. The subcellular localization of the polypeptide sequence as represented by SEQ ID NO: 2 is may most likely be in the cytoplasm and /or the nucleus.

**Table C:** TargetP 1.1 analysis of the polypeptide sequence as represented by SEQ ID NO: 2

| | |
|---|---|
| Length (AA) | 815 |
| Chloroplastic transit peptide | 0,24 |
| Mitochondrial transit peptide | 0,194 |
| Secretory pathway signal peptide | 0,006 |
| Other subcellular targeting | 0,444 |
| Predicted Location | - |
| Reliability class | 4 |
| Predicted transit peptide length | 0 |

Many other algorithms can be used to perform such analyses, including:

- ChloroP 1.1 hosted on the server of the Technical University of Denmark;
- Protein Prowler Subcellular Localisation Predictor version 1.2 hosted on the server of the Institute for Molecular Bioscience, University of Queensland, Brisbane, Australia;
- PENCE Proteome Analyst PA-GOSUB 2.5 hosted on the server of the University of Alberta, Edmonton, Alberta, Canada;
- TMHMM, hosted on the server of the Technical University of Denmark
- PSORT (URL: psort-org)
- PLOC (Park and Kanehisa, Bioinformatics, 19, 1656-1663, 2003).

Example 6: Cloning of the CLP encoding nucleic acid sequence

The nucleic acid sequence was amplified by PCR using as template a custom-made tomato cDNA library.

The nucleic acid sequence was amplified by PCR using as template a custom-made Arabidopsis thaliana seedlings cDNA library.

The cDNA library used for cloning was custom made from different tissues (e.g. leaves, roots) of Arabidopsis thaliana Col-0 seedlings grown from seeds obtained in Belgium. PCR was performed using a commercially available proofreading Taq DNA polymerase in standard conditions, using 200 ng of template in a 50 μl PCR mix. The primers used were prm17897 (SEQ ID NO: 73; sense, start codon in bold):

5' ggggacaagtttgtacaaaaaagcaggcttaaaca**atg**agagctggaggctgc 3'
and prm 17898 (SEQ ID NO: 74; reverse, complementary):
5' ggggaccactttgtacaagaaagctgggtgcatacgtgcatgctgttagt 3',
which include the AttB sites for Gateway recombination. The amplified PCR fragment was purified also using standard methods. The first step of the Gateway procedure, the BP reaction, was then performed, during which the PCR fragment recombined in vivo with the pDONR201 plasmid to produce, according to the Gateway terminology, an "entry clone", $_p$POI. Plasmid pDONR201 was purchased from Invitrogen (Life Technologies GmbH, Frankfurter Straße 129B, 64293 Darmstadt, Germany), as part of the Gateway® technology.

The entry clone comprising SEQ ID NO: 1 was then used in an LR reaction with a destination vector used for *Oryza sativa* transformation. This vector contained as functional elements within the T-DNA borders: a plant selectable marker; a screenable marker expression cassette; and a Gateway cassette intended for LR *in vivo* recombination with the nucleic acid sequence of interest already cloned in the entry clone. A rice GOS2 promoter (SEQ ID NO: 82) for constitutive expression was located upstream of this Gateway cassette.

After the LR recombination step, the resulting expression vector pGOS2::POI (Figure 5) was transformed into *Agrobacterium* strain LBA4044 according to methods well known in the art.

Example 7: Plant transformation

*Rice transformation*

The *Agrobacterium* containing the expression vector was used to transform *Oryza sativa* plants. Mature dry seeds of the rice japonica cultivar Nipponbare were dehusked. Sterilization was carried out by incubating for one minute in 70% ethanol, followed by 30 to 60 minutes, preferably 30 minutes in sodium hypochlorite solution (depending on the grade of contamination), followed by a 3 to 6 times, preferably 4 time wash with sterile distilled water. The sterile seeds were then germinated on a medium containing 2,4-D (callus induction medium). After incubation in light for 6 days scutellum-derived calli is transformed with Agrobacterium as described herein below.

*Agrobacterium* strain LBA4404 containing the expression vector was used for co-cultivation. *Agrobacterium* was inoculated on AB medium with the appropriate antibiotics and cultured for 3 days at 28°C. The bacteria were then collected and suspended in liquid co-cultivation medium to a density ($OD_{600}$) of about 1. The calli were immersed in the suspension for 1 to 15 minutes. The callus tissues were then blotted dry on a filter paper and transferred to solidified, co-cultivation medium and incubated for 3 days in the dark at 25°C. After washing away the *Agrobacterium,* the calli were grown on 2,4-D-containing medium for 10 to 14 days (growth time for indica: 3 weeks) under light at 28°C - 32°C in the presence of a selection agent. During this period, rapidly growing resistant callus developed. After transfer of this material to regeneration media, the embryogenic potential was released and shoots developed in the next four to six weeks. Shoots were excised from the calli and incubated for 2 to 3 weeks on an auxin-containing medium from which they were transferred to soil. Hardened shoots were grown under high humidity and short days in a greenhouse.

Transformation of rice cultivar indica can also be done in a similar way as give above according to techniques well known to a skilled person.

35 to 90 independent T0 rice transformants were generated for one construct. The primary transformants were transferred from a tissue culture chamber to a greenhouse. After a quantitative PCR analysis to verify copy number of the T-DNA insert, only single copy transgenic plants that exhibit tolerance to the selection agent were kept for harvest of T1 seed. Seeds were then harvested three to five months after transplanting. The method yielded single locus transformants at a rate of over 50 % (Aldemita and Hodges1996, Chan et al. 1993, Hiei et al. 1994).

**Example 8: Transformation of other crops**

*Corn transformation*

Transformation of maize (Zea mays) is performed with a modification of the method described by Ishida et al. (1996) Nature Biotech 14(6): 745-50. Transformation is genotype-dependent in corn and only specific genotypes are amenable to transformation and regeneration. The inbred line A188 (University of Minnesota) or hybrids with A188 as a parent are good sources of donor material for transformation, but other genotypes can be used successfully as well. Ears are harvested from corn plant approximately 11 days after pollination (DAP) when the length of the immature embryo is about 1 to 1.2 mm. Immature embryos are cocultivated with *Agrobacterium tumefaciens* containing the expression vector, and transgenic plants are recovered through organogenesis. Excised embryos are grown on callus induction medium, then maize regeneration medium, containing the selection agent (for example imidazolinone but various selection markers can be used). The Petri plates are incubated in the light at 25 °C for 2-3 weeks, or until shoots develop- The green shoots are transferred from each embryo to maize rooting medium and incubated at 25 °C for 2-3 weeks, until roots develop. The rooted shoots are transplanted to soil in the greenhouse. T1 seeds are produced from plants that exhibit tolerance to the selection agent and that contain a single copy of the T-DNA insert.

*Wheat transformation*

Transformation of wheat is performed with the method described by Ishida et al. (1996) Nature Biotech 14(6): 745-50. The cultivar Bobwhite (available from CIMMYT, Mexico) is commonly used in transformation, immature embryos are co-cultivated With *Agrobacterium tumefaciens* containing the expression vector, and transgenic plants are recovered through organogenesis. After incubation with *Agrobacterium,* the embryo are grown in vitro on callus induction medium, then regeneration medium, containing the selection agent (for example imidazolinone but various selection markers can be used). The Petri plates are incubated in the light at 25 °C for 2-3 weeks, or until shoots develop. The green shoots are transferred from each embryo to rooting medium and incubated at 25 °C for 2-3 weeks, until roots develop. The rooted shoots are transplanted to soil in the greenhouse. T1 seeds are produced from plants that exhibit tolerance to the selection agent and that contain a single copy of the T-DNA insert.

*Soybean transformation*

Soybean is transformed according to a modification of the method described in the Texas A&M patent US 5,164,310. Several commercial soybean varieties are amenable to transformation by this method. The cultivar Jack (available from the Illinois foundation) is commonly used for transformation. Soybean are sterilised for *in vitro* sowing. The hypocotyl, the radicle and one cotyledon are excised from seven-day old young seedlings, The epicotyl and the remaining cotyledon are further grown to develop axillary nodes. These axillary nodes are excised and incubated with *Agrobacterium tumefaciens* containing the expression vector. After the cocultivation treatment, the explants are washed and transferred to selection media. Regenerated shoots are excised and placed on a shoot elongation medium. Shoots no longer than 1 cm are placed on rooting medium until roots develop. The rooted shoots are transplanted to soil in the greenhouse. T1 seeds are produced from plants that exhibit tolerance to the selection agent and that contain a single copy of the T-DNA insert.

*Rapeseed/canola transformation*

Cotyledonary petioles and hypocotyls of 5-6 day old young seedling are used as explants for tissue culture and transformed according to Babic et al. (1998, Plant Cell Rep 17: 183-188). The commercial cultivar Westar (Agriculture Canada) is the standard variety used for transformation, but other varieties can also be used. Canola seeds are surface-sterilized for in vitro sowing. The cotyledon petiole explants with the cotyledon attached are excised from the in vitro seedlings, and inoculated with *Agrobacterium* (containing the expression vector) by dipping the cut end of the petiole explant into the bacterial suspension. The explants are then cultured for 2 days on MSBAP-3 medium containing 3 mg/l BAP, 3 % sucrose, 0.7 % Phytagar at 23 °C, 16 hr light. After two days of co-cultivation with *Agrobacterium,* the petiole explants are transferred to MSBAP-3 medium containing 3 mg/l BAP, cefotaxime, carbenicillin, or timentin (300 mg/l) for 7 days, and then cultured on MSBAP-3 medium with cefotaxime, carbenicillin, or timentin and selection agent until shoot regeneration. When the shoots are 5-10 mm in length, they are cut and transferred to shoot elongation medium (MSBAP-0.5, containing 0.5 mg/l BAP). Shoots of about 2 cm in length are transferred to the rooting medium (MS0) for root induction. The rooted shoots are transplanted to soil in the greenhouse. T1 seeds are produced from plants that exhibit tolerance to the selection agent and that contain a single copy of the T-DNA insert.

*Alfalfa Transformation*

A regenerating clone of alfalfa (*Medicago sativa*) is transformed using the method of (McKersie et al., 1999 Plant Physiol 119: 839-847). Regeneration and transformation of alfalfa is genotype dependent and therefore a regenerating plant is required. Methods to obtain regenerating plants have been described. For example, these can be selected from the cultivar Rangelander (Agriculture Canada) or any other commercial alfalfa variety as described by Brown DCW and A Atanassov (1985. Plant Cell Tissue Organ Culture 4: 111-112). Alternatively, the RA3 variety (University of Wisconsin) has been selected for use in tissue culture (Walker et al., 1978 Am J Bot 65:654-659). Petiole explants are cocultivated with an overnight culture of *Agrobacterium tumefaciens* C58C1 pMP90 (McKersie et al., 1999 Plant Physiol 119: 839-847) or LBA4404 containing the expression vector. The explants are cocultivated for 3 d in the dark on SH induction medium containing 288 mg/ L Pro, 53 mg/ L thioproline, 4.35 g/L K2SO4, and 100 μm acetosyringinone. The explants are washed in half-strength Murashige-Skoog medium (Murashige and Skoog, 1962) and plated on the same SH induction medium without acetosyringinone but with a suitable selection agent and suitable antibiotic to inhibit *Agrobacterium* growth. After several weeks, somatic embryos are transferred to BOi2Y development medium containing no growth regulators, no antibiotics, and 50 g/ L sucrose. Somatic embryos are subsequently germinated on half-strength Murashige-Skoog medium. Rooted seedlings were transplanted into pots and grown in a greenhouse. T1 seeds are produced from plants that exhibit tolerance to the selection agent and that contain a single copy of the T-DNA insert.

*Cotton transformation*

Cotton is transformed using *Agrobacterium tumefaciens* according to the method described in US 5,159,135. Cotton seeds are surface sterilised in 3% sodium hypochlorite solution during 20 minutes and washed in distilled water with 500 μg/ml cefotaxime. The seeds are then transferred to SH-medium with 50μg/m) benomyl for germination. Hypocotyls of 4 to 6 days old seedlings are removed, cut into 0.5 cm pieces and are placed on 0.8% agar. An *Agrobacterium* suspension (approx. 108 cells per ml, diluted from an overnight culture transformed with the gene of interest and suitable selection markers) is used for inoculation of the hypocotyl explants. After 3 days at room temperature and lighting, the tissues are transferred to a solid medium (1.6 g/l Gelrite) with Murashige and Skoog salts with B5 vitamins (Gamborg et al., Exp. Cell Res. 50:151-158 (1968)), 0.1 mg/l 2,4-D, 0.1 mg/l 6-furfurylaminopurine and 750 μg/ml MgCL2, and with 50 to 100 μg/ml cefotaxime and 400-500 μg/m) carbenicillin to kill residual bacteria. Individual cell lines are isolated after two to three months (with subcultures every four to six weeks) and are further cultivated on selective medium for tissue amplification (30°C, 16 hr photoperiod). Transformed tissues are subsequently further cultivated on non-selective medium during 2 to 3 months to give rise to somatic embryos, Healthy looking embryos of at least 4 mm length are transferred to tubes with SH medium in fine vermiculite, supplemented with 0.1 mg/l indole acetic acid, 6 furfurytaminopurine and gibberellic acid. The embryos are cultivated at 30°C with a photoperiod of 16 hrs, and plantlets at the 2 to 3 leaf stage are transferred to with vermiculite and nutrients. The plants are hardened and subsequently moved to the greenhouse for further cultivation.

*Sugarbeet transformation*

Seeds of sugarbeet (Beta vulgaris L.) are sterilized in 70% ethanol for one minute followed by 20 min. shaking in 20% Hypochlorite bleach e,g. Clorox® regular bleach (commercially available from Clorox, 1221 Broadway, Oakland, CA 94612, USA). Seeds are rinsed with sterile water and air dried followed by plating onto germinating medium (Murashige and Skoog (MS) based medium (Murashige, T., and Skoog, ., 1962. Physiol. Plant, vol. 15, 473-497) including B5 vitamins (Gamborg et al.; Exp. Cell Res., vol. 50, 151-8.) supplemented with 10 g/l sucrose and 0,8% agar). Hypocotyl tissue is used essentially for the initiation of shoot cultures according to Hussey and Hepher (Hussey, G., and Hepher, A., 1978. Annals of Botany, 42, 477-9) and are maintained on MS based medium supplemented with 30g/l sucrose plus 0,25mg/l benzylamino purine and 0,75% agar, pH 5,8 at 23-25°C with a 16-hour photoperiod. Agrobacterium tumefaciens strain carrying a binary plasmid harbouring a selectable marker gene, for example nptll, is used in transformation experiments. One day before transformation, a liquid LB culture including antibiotics is grown on a shaker (28°C, 150rpm) until an optical density (O.D.) at 600 nm of~1 is reached. Overnight-grown bacterial cultures are centrifuged and resuspended in inoculation medium (O.D. ~1) including Acetosyringone, pH 5,5. Shoot base tissue is cut into slices (1.0 cm x 1.0 cm x 2.0 mm approximately), Tissue is immersed for 30s in liquid bacterial inoculation medium. Excess liquid is removed by filter paper blotting. Co-cultivation occurred for 24-72 hours on MS based medium incl. 30g/l sucrose followed by a non-selective period including MS based medium, 30g/l sucrose with 1 mg/l BAP to induce shoot development and cefotaxim for eliminating the Agrobacterium. After 3-10 days explants are transferred to similar selective medium harbouring for example kanamycin or G418 (50-100 mg/l genotype dependent). Tissues are transferred to fresh medium every 2-3 weeks to maintain selection pressure. The very rapid initiation of shoots (after 3-4 days) indicates regeneration of existing meristems rather than organogenesis of newly developed transgenic meristems. Small shoots are transferred after several rounds of subculture to root induction medium containing 5 mg/l NAA and kanamycin or G418. Additional steps are taken to reduce the potential of generating transformed plants that are chimeric (partially transgenic). Tissue samples from regenerated shoots are used for DNA analysis. Other transformation methods for sugarbeet are known in the art, for example those by Linsey & Gallois (Linsey, K., and Gallois, P., 1990. Journal of Experimental Botany; vol. 41, No. 226; 529-36) or the methods published in the international application published as WO9623891A.

*Sugarcane transformation*

Spindles are isolated from 6-month-old field grown sugarcane plants (Arencibia et al., 1998. Transgenic Research, vol. 7, 213-22; Enriquez-Obregon et al., 1998. Planta, vol. 206, 20-27). Material is sterilized by immersion in a 20% Hypochlorite bleach e.g. Clorox® regular bleach (commercially available from Clorox, 1221 Broadway, Oakland, CA 94612, USA) for 20 minutes. Transverse sections around 0,5cm are placed on the medium in the top-up direction. Plant material is cultivated for 4 weeks on MS (Murashige, T., and Skoog, ., 1962. Physiol. Plant, vol. 15, 473-497) based medium incl. B5 vitamins (Gamborg, O., et al., 1968. Exp. Cell Res., vol. 50, 151-8) supplemented with 20g/l sucrose, 500 mg/l casein hydrolysate, 0,8% agar and 5mg/l 2,4-D at 23°C in the dark. Cultures are transferred after 4 weeks onto identical fresh medium. Agrobacterium tumefaciens strain carrying a binary plasmid harbouring a selectable marker gene, for example hpt, is used in transformation experiments. One day before transformation, a liquid LB culture including antibiotics is grown on a shaker (28°C, 150rpm) until an optical density (O.D.) at 600 nm of ~0,6 is reached. Overnight-grown bacterial cultures are centrifuged and resuspended in MS based inoculation medium (O.D. ~0,4) including acetosyringone, pH 5,5. Sugarcane embryogenic callus pieces (2-4 mm) are isolated based on morphological characteristics as compact structure and yellow colour and dried for 20 min. in the flow hood followed by immersion in a liquid bacterial inoculation medium for 10-20 minutes. Excess liquid is removed by filter paper blotting. Co-cultivation occurred for 3-5 days in the dark on filter paper which is placed on top of MS based medium incl. B5 vitamins containing 1 mg/l 2,4-D. After co-cultivation calli are washed with sterile water followed by a non-selective cultivation period on similar medium containing 500 mg/l cefotaxime for eliminating remaining Agrobacterium cells. After 3-10 days explants are transferred to MS based selective medium incl. B5 vitamins containing 1 mg/l 2,4-D for another 3 weeks harbouring 25 mg/l of hygromycin (genotype dependent). All treatments are made at 23°C under dark conditions. Resistant calli are further cultivated on medium lacking 2,4-D including 1 mg/l BA and 25 mg/l hygromycin under 16 h light photoperiod resulting in the development of shoot structures. Shoots are isolated and cultivated on selective rooting medium (MS based including, 20g/l sucrose, 20 mg/l hygromycin and 500 mg/l cefotaxime). Tissue samples from regenerated shoots are used for DNA analysis, Other transformation methods for sugarcane are known in the art, for example from the international application published as WO2010/151634A and the granted European patent EP1831378.

For transformation by particle bombardment the induction of callus and the transformation of sugarcane can be carried out by the method of Snyman et al. (Snyman et al., 1996, S. Afr. J. Bot 62, 151-154). The construct can be cotransformed with the vector pEmuKN, which expressed the npt[pi] gene (Beck et al. Gene 19, 1982, 327-336; GenBank Accession No. V00618) under the control of the pEmu promoter (Last et al. (1991) Theor. Appl. Genet. 81, 581-588). Plants are regenerated by the method of Snyman et al. 2001 (Acta Horticulturae 560, (2001), 105-108).

Example 9: Phenotypic evaluation procedure

9.1 Evaluation setup

35 to 90 independent T0 rice transformants were generated. The primary transformants were transferred from a tissue culture chamber to a greenhouse for growing and harvest of T1 seed. Six events, of which the T1 progeny segregated 3:1 for presence/absence of the transgene, were retained, For each of these events, approximately 10 T1 seedlings containing the transgene (hetero- and homo-zygotes) and approximately 10 T1 seedlings lacking the transgene (nullizygotes) were selected by monitoring visual marker expression. The transgenic plants and the corresponding nullizygotes were grown side-by-side at random positions. Greenhouse conditions were of shorts days (12 hours light), 28°C in the light and 22°C in the dark, and a relative humidity of 70%. Plants grown under non-stress conditions were watered at regular intervals to ensure that water and nutrients were not limiting and to satisfy plant needs to complete growth and development, unless they were used in a stress screen.

From the stage of sowing until the stage of maturity the plants were passed several times through a digital imaging cabinet. At each time point digital images (2048x1536 pixels, 16 million colours) were taken of each plant from at least 6 different angles.

T1 events can be further evaluated in the T2 generation following the same evaluation procedure as for the T1 generation, e.g. with less events and/or with more individuals per event.

*Drought screen*

T1 or T2 plants were grown in potting soil under normal conditions until they approached the heading stage. They were then transferred to a "dry" section where irrigation is withheld. Soil moisture probes were inserted in randomly chosen pots to monitor the soil water content (SWC). When SWC went below certain thresholds, the plants were automatically re-watered continuously until a normal level is reached again. The plants were then retransferred again to normal conditions. The of the cultivation (plant maturation, seed harvest) was the same as for plants not grown under abiotic stress conditions. Growth and yield parameters recorded as detailed for growth under normal conditions.

*Nitrogen use efficiency screen*

T1 or T2 plants are grown in potting soil under normal conditions except for the nutrient solution. The are watered from transplantation to maturation with a specific nutrient solution containing reduced N nitrogen (N) content, usually between 7 to 8 times less. The rest of the cultivation (plant maturation, seed harvest) is the same as for plants not grown under abiotic stress. Growth and yield parameters are recorded as detailed for growth under normal conditions.

*Salt stress screen*

T1 or T2 plants are grown on a substrate made of coco fibers and particles of baked clay (Argex) (3 to 1 ratio). A normal nutrient solution is used during the first two weeks after transplanting the plantlets in the greenhouse. After the first two weeks, 25 mM of salt (Nacl) is added to the nutrient solution, until the plants are harvested. Growth and yield parameters are recorded as detailed for growth under normal conditions.

9.2 Statistical analysis: F test

A two factor ANOVA (analysis of variants) was used as a statistical model for the overall evaluation of plant phenotypic characteristics. An F test was carried out on all the parameters measured of all the plants of all the events transformed with the gene of the present invention. The F test was carried out to check for an effect of the gene over all the transformation events and to verify for an overall effect of the gene, also known as a global gene effect. The threshold for significance for a true global gene effect was set at a 5% probability level for the F test. A significant F test value Points to a gene effect, meaning that it is not only the mere presence or position of the gene that is causing the differences in phenotype.

9.3 Parameters measured

From the stage of sowing until the of maturity the plants were passed several times through a digital imaging cabinet. At each time point digital images (2048x1536 pixels, 16 million colours) were taken of each plant from at least 6 different angles as described in W02010/031780. These measurements were used to determine different parameters.

*Biomass-related parameter measurement*

The plant aboveground area (or leafy biomass) was determined by counting the total number of pixels on the digital images from aboveground plant parts discriminated from the background. This value was averaged for the pictures taken on the same time point from the different angles and was converted to a physical surface value expressed in square mm by calibration. Experiments show that the aboveground plant area measured this way correlates with the biomass of plant parts above ground. The above ground area is the area measured at the time point at which the plant had reached its maximal leafy biomass.

increase in root biomass is expressed as an increase in total root biomass (measured as maximum biomass of roots observed during the lifespan of a plant); or as an increase in the root/shoot index, measured as the ratio between root mass and shoot mass in the period of active growth of root and shoot. In other words, the root/shoot index is defined as the ratio of the rapidity of root growth to the rapidity of shoot growth in the period of active growth of root and shoot. Root biomass can be determined using a method as described in WO 2006/029987.

The absolute height of a plant can be measured. A robust indication of the height of the plant is the measurement of the location of the centre of gravity, i.e. determining the height (in mm) of the gravity centre of the leafy biomass. This avoids influence by a single erect leaf, based on the asymptote of curve fitting or, if the fit is not satisfactory, based on the absolute maximum.

The greenness before flowering (GNbfFlow) can be measured from digital images as well. It is an indication of the greenness of a plant before flowering. Proportion (expressed as %)of green and dark green pixels in the last imaging before flowering. It is both a development time related parameter and a biomass related parameter.

*Parameters related to development time*

The early vigour is the plant aboveground area three weeks post-germination. Early vigour was determined by counting the total number of pixels from aboveground plant parts discriminated from the background. This value was averaged for the pictures taken on the same time Point from different angles and was converted to a physical surface value expressed in square mm by calibration.

Increase in root biomass is expressed as an increase in total root biomass (measured as maximum biomass of roots observed during the lifespan of a plant). Also, the diameter of the root, the amount of roots above a certain thickness level and below a certain thinness level can be measured. Root biomass can be determined using a method as described in WO 2006/029987. The amounts of thick and/or thin roots above or below a threshold can be measured as well.

AreaEmer is an indication of quick early development when this value is decreased compared to control plants. It is the ratio (expressed in %) between the time a plant needs to make 30 % of the final biomass and the time needs to make 90 % of its final biomass.

The "time to flower" or "flowering time" of the plant can be determined using the method as described in WO 2007/093444.

The relative growth rate (RGR) as the the natural logarithm of the above ground biomass measured (called 'TotalArea') at a second time point, minus the natural logarithm of the above ground biomass at a first time point, divided by the number of days between those two time points ([log(TotalArea2)-log(TotalArea1)]/ndays). The time points are the same for all plants in one experiment. The first time point is chosen as the earliest measurement taken between 25 and 41 days after planting. If the number of measurements (plants) at that time point in that experiment is less than one third of the maximum number of measurements taken per time point for that experiment, then the next time point is taken (again with the same restriction on the number of measurements). The second time point is simply the next time point (with the same restriction on the number of measurements).

Measuring the greenness of plants

The greenness index is calculated as one minus the number of pixels that are light green (bins 2-21 in the spectrum) divided by the total number of pixels, multiplied by 100 (100 * [1-(nLGpixels/npixels)]).

Early greenness:

The greenness index at the time point before the flowering time point, when the maximum mean greenness for null plants is reached for that experiment. The flowering time point is defined as the time point where more than 3 plants with panicles are detected.

Time points are the same for all plants in an experiment. If the number of valid observations on that time point is 30 or less, the time point with the second highest mean greenness for null plants, before flowering, is chosen. The first time point is never chosen as flowering time point.

Late greenness:

The greenness index at the time point after or at the flowering time point, when the minimum mean greenness for null plants is reached for that experiment. The flowering time point is defined as the time point where more than 3 plants with panicles are detected.

Time points are the same for all plants in an experiment. If the number of valid observations on that time point is 30 or less, the time point with the second lowest mean greenness for null plants, after or at flowering, is chosen.

Greenness after drought

The greenness of a plant after drought stress can be measured as the proportion (expressed as %) of green and dark green pixels in the first imaging after the drought treatment.

In addition, the growth of the plants under drought conditions can be measured.

*Seed-related parameter measurements*

The mature primary panicles were harvested, counted, bagged, barcode-labelled and then dried for three days in an oven at 37°C. The panicles were then threshed and all the seeds were collected and counted. The seeds are usually covered by a dry outer covering, the husk. The filled husks (herein also named filled florets) were separated from the empty ones using an air-blowing device. The empty husks were discarded and the remaining fraction was counted again. The filled husks were weighed on an analytical balance.

The total number of seeds was determined by counting the number of filled husks that remained after the separation step. The total seed weight was measured by weighing all filled husks harvested from a plant.

The total number of seeds (or florets) per plant was determined by counting the number of husks (whether filled or not) harvested from a plant.

Thousand Kernel Weight (TKW) is extrapolated from the number of seeds counted and their total weight.

The Harvest index (HI) in the present invention is defined as the ratio between the total seed weight and the above ground area ($mm^2$), by a factor $10^6$.

The number of flowers per panicle as defined in the present invention is the ratio between the total number of seeds over the number of mature primary panicles.

The "seed fill rate" or "seed filling rate" as defined in the present invention is the proportion (expressed as a %) of the number of filled seeds (i.e. florets containing seeds) over the total number of seeds (i.e. total number of florets). In other words, the seed filling rate is the percentage of florets that are filled with seed.

Also, the number of panicles in the first flush and the flowers per panicle, a calculated parameter (the number of florets of a plant/ number of panicles in the first flush) estimating the average number of florets per panicle on a plant were measured.

Example 10: Results of the phenotypic evaluation of the transgenic plants

The results of the evaluation of transgenic rice plants in the T1 generation and expressing a nucleic acid encoding the CLP polypeptide of SEQ ID NO: 2 under non-stress conditions are presented below in Table D1. When grown under non-stress conditions, an increase of at least 5 % was observed for the height of the centre of gravity (GravityYmax) in at least 3 of the 6 events, and for the seed yield parameters total number of seed (nrtotalseed), number of filled seed (nrfilledseed), the filtrate, the total weight of seeds (totalwgseeds) and the harvest index (harvestindex). Also, the number of panicles in the first flush and the flowers per panicle, a calculated parameter (the number of florets of a plant/ number of panicles in the first flush) estimating the average number of florets per panicle on a plant were higher in at least one event than in the control plants. In addition the thousand kernel weight was increased in at least two events, just as the fillrate and the number of filled seed. Interestingly, the seed yield parameters TKW and fillrate or the number of filled seed show increases sometimes in different events indicating that increases in seed yield are common but the events may differ in individual parameters. An event may show increased TKW, but not the highest fillrate, while the event with the highest fillrate may not show an increase in TKW Yet a third event may show both increased TKW and increased fillrate.

Also increased were the aboveground biomass (AreaMax) and the maximum height of the plants in at least one event. The aboveground biomass was increased in at least 2 out of the 6 events significantly and up to an increase of 9 % in two events.

**Table D1:** Data summary for transgenic rice plants under normal growth conditions; for each parameter, the overall percent increase is shown for T1 generation plants, for each parameter the p-value is <0.05.

| Parameter | Overall |
|---|---|
| totalwgseeds | 16.0 |
| nrtotalseed | 6.7 |
| fillrate | 8.9 |
| harvestindex | 11.7 |
| nrfilledseed | 14.5 |
| GravityYMax | 5.2 |

The results of the evaluation of transgenic rice plants in the T1 generation and expressing a nucleic acid encoding the CLP polypeptide of SEQ ID NO: 2 under drought conditions are presented below in Table D2. When grown under drought conditions, an increase of at least 5 % was observed the seed yield parameters number of filled seed (nrfilledseed), the fillrate, the total weight of seeds (totalwgseeds) and the harvest index (harvestindex). All this were increased overall above 20%. Further seed yield parameters like the number of total seed, TKW and the number of panicles in the first flush were increased. Also increased was the growth of the plants under drought conditions.

The aboveground biomass was increased in 2 out of the 6 events significantly. Further at least two events showed an increase in root biomass (Rootmax). Also, the emergence vigour was increase in at least one event, as was the greenness after drought. The centr of gravity of at least one event was increased simultaneously with an increase in root thickness (RootThickMax). In at least one event the ealy greenness, early height, late greenness or relative growth rate (RGR) were increased.

**Table D2:** Data summary for transgenic rice plants under drougth growth conditions; for each parameter, the overall percent increase is shown for T1 generation plants, for each parameter the p-value is <0.05.

| Parameter | Overall |
|---|---|
| totalwgseeds | 27.0 |
| fillrate | 21.6 |
| harvestindex | 21.0 |
| nrfilledseed | 23.4 |

Example 11: Functional assay for the CLP polypeptide

Suitable assay to test ATP-dependent protease, as well as materials and methods for doing such experiments are well known in the art.

Example 12: Sugarcane phenotypic evaluation procedure

12.1 The transgenic sugarcane plants generated are grown for 10 to 15 months, either in the greenhouse or the field. Standard conditions for growth of the plants are used.

12.2 Sugar extraction method

Stalks of sugarcane plants which are 10 to 15 months old and have more than 10 internodes are harvested. After all of the leaves have been removed, the internodes of the stalk are numbered from top (= 1) to bottom (for example = 36). A stalk disc approximately 1-2 g in weight is excised from the middle of each internode. The stalk discs of 3 internodes are then combined to give one sample and frozen in liquid nitrogen.

For the sugar extraction, the stalk discs are first comminuted in a Waring blender (from Waring, New Hartford, Connecticut, USA). The sugars are extracted by shaking for one hour at 95°C in 10 mM sodium phosphate buffer pH 7.0. Thereafter, the solids are removed by filtration through a 30 μm sieve. The resulting solution is subsequently employed for the sugar determination (see herein below).

12.3 Fresh weight and biomass

The transgenic sugarcane plants expressing the CLP polypeptide are grown for 10 to 15 months. In each case a sugarcane stalk of the transgenic line and a wild-type sugarcane plant is defoliated, the stalk is divided into segments of 3 internodes, and these internode segments are frozen in liquid nitrogen in a sealed 50 ml plastic container. The fresh weight of the samples is determined. The extraction for the purposes of the sugar determination is done as described below.

The stem biomass is increased in the transgenic plant.

12.4 Sugar determination (glucose, fructose and sucrose)

The glucose, fructose and sucrose contents in the extract obtained in accordance with the sugar extraction method described above is determined photometrically in an enzyme assay via the conversion of NAD+ (nicotinamide adenine dinucleotide) into NADH (reduced nicotinamide adenine dinucleotide). During the reduction, the aromatic character at the nicotinamide ring is lost, and the absorption spectrum thus changes. This change in the absorption spectrum can be detected photometrically. The glucose and fructose present in the extract is converted into glucose-6-phosphate and fructose-6-phosphate by means of the enzyme hexokinase and adenosin triphosphate (ATP). The glucose- 6-phosphate is subsequently oxidized by the enzyme glucose-6-phosphate dehydrogenase to give 6-phosphogluconate. In this reaction, NAD+ is reduced to give NADH, and the amount of NADH formed is determined photometrically. The ratio between the NADH formed and the glucose present in the extract is 1:1, so that the glucose content can be calculated from the NADH content using the molar absorption coefficient of NADH (6.3 1 per mmol and per cm lightpath). Following the complete oxidation of glucose-6-phosphate, fructose-6-phosphate, which has likewise formed in the solution, is converted by the enzyme phosphoglucoisomerase to give glucose-6-phosphate which, in turn, is oxidized to give 6-phosphogluconate. Again, the ratio between fructose and the amount of NADH formed is 1 :1. Thereafter, the sucrose present in the extract is cleaved by the enzyme sucrase (Megazyme) to give glucose and fructose. The glucose and fructose molecules liberated are then converted with the abovementioned enzymes in the NAD+-dependent reaction to give 6- phosphogluconate. The conversion of one sucrose molecule into 6-phosphogluconate results in two NADH molecules. The amount of NADH formed is likewise determined photometrically and used for calculating the sucrose content, using the molar absorption coefficient of NADH.

The sugarcane stalks are divided into segments of in each case three internodes, as specified above. The internodes are numbered from top to bottom (top = internode 1, bottom = internode 21). In the sugarcane wild-type plant, the sucrose contents rises from internode 1-3 up to internode 10-12. The sucrose contents of all subsequent internodes are similarly high.

In the transgenic lines, which comprises the CLP encoding gene the storage carbohydrate content in the stalk likewise climbs. The mean storage carbohydrate content is higher than the sucrose content in the sugarcane wild-type plants.

In total, it can be observed that, surprisingly, the sucrose content in the internodes of the transgenic sugarcane line is higher than in the wild type.

## Claims

**1.** A method for enhancing one or more yield-related traits in plants relative to control plants, comprising modulating, preferably increasing expression, in a plant of a nucleic acid encoding a CLP polypeptide, wherein said CLP polypeptide comprises the Interpro motifs IPR004176 (Clp, N-terminal) and /or IPR023150 (Double Clp-N motif), preferably both, and comprises a PFAM domain PF02861 as a split domain wherein the N-terminal part of the PFAM domain PF02861 finds a match at or near the N-terminus of the CLP polypeptide and the C-terminal part of the PFAM domain PF02861 finds a match C-terminally of this, wherein both matching amino acid stretches are located in the N-terminal half of the CLP polypeptide, preferably in the N-terminal quarter of the CLP polypeptide, and wherein said CLP polypeptide is selected from the group consisting of:

(i) an amino acid sequence of any SEQ ID NO listed in table A, preferably SEQ ID NO: 2; and
(ii) an amino acid sequence having, in increasing order of preference, at least 35%, 40%, 45%, 50%, 51 %, 52%, 53%, 54%, 55%, 56%, 57%, 58%, 59%, 60%, 61 %, 62%, 63%, 64%, 65%, 66%, 67%, 68%, 69%, 70%, 71 %, 72%, 73%, 74%, 75%, 76%, 77%, 78%, 79%, 80%, 81 %, 82%, 83%, 84%, 85%, 86%, 87%, 88%, 89%, 90%, 91 %, 92%, 93%, 94%, 95%, 96%, 97%, 98%, or 99% sequence identity to the amino acid sequence of any SEQ ID NO listed in table A, preferably SEQ ID NO: 2, over the entire length of the sequences of said SEQ ID NO listed in table A, preferably SEQ ID NO: 2, and further preferably conferring one or more enhanced yield-related traits relative to control plants.

**2.** Method according to claim 1, wherein said nucleic acid is selected from the group consisting of:

(i) a nucleic acid represented by SEQ ID NO: 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69 or 71; preferably SEQ ID NO: 1;
(ii) the complement of a nucleic acid represented by SEQ ID NO: 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69 or 71; preferably SEQ ID NO: 1;
(iii) a nucleic acid encoding a POI polypeptide having in increasing order of preference at least 25%, 30%, 35%, 40%, 45%, 50%, 51%, 52%, 53%, 54%, 55%, 56%, 57%, 58%, 59%, 60%, 61 %, 62%, 63%, 64%, 65%, 66%, 67%, 68%, 69%, 70%, 71 %, 72%, 73%, 74%, 75%, 76%, 77%, 78%, 79%, 80%, 81 %, 82%, 83%, 84%, 85%, 86%, 87%, 88%, 89%, 90%, 91 %, 92%, 93%, 94%, 95%, 96%, 97%, 98%, or 99% sequence identity to the entire amino acid sequence of any SEQ ID NO: listed in table A, preferably SEQ ID NO: 2, and further preferably conferring one or more enhanced yield-related traits relative to control plants; and
(iv) a nucleic acid molecule which hybridizes with a nucleic acid molecule of (i) to (iii) under high stringency hybridization conditions and preferably confers one or more enhanced yield-related traits relative to control plants.

**3.** Method according to claim 1 or 2, wherein said modulated expression is effected by introducing and expressing in a plant said nucleic acid encoding said CLP polypeptide.

**4.** Method according to any of claims 1 to 3, wherein said CLP polypeptide comprises

i) all of the following motifs:

Motif 1 (SEQ ID NO: 76):

A-[AGLV]-K-x(0,2)-I-x(1,3)-Q-L-[IMV]-[IV]-S-I-L-[DH]-D-P-S-V-S-R-V-M-R-[DE]-A-[DG]-F-[NS]-S-[PST]-x-[LV]-[KR]-[ANST]-x-[IV]-E-x-[AE];

Motif 2 (SEQ ID NO: 77):
F-x-E-x-[NST]-[ADEST]-E-N-L-x(2)-L-[CS]-x-A-L-x(2)-[EKR]-x-[PST] ;
Motif 3 (SEQ ID NO: 78):

T-W-[FLM]-[FL]-F-x-G-x-[DGN]-x(2)-[ADG]-[KR]-x(2)-[IMV]-[ACS]-x-E-L-A-x-[LV]-V-F-G-S-x(3)-[FV]-x-[ACST]-x(3)-[ADGS]-[ADEGNST];

Motif 4(SEQ ID NO: 79):
G-x(1,2)-V-x(0,1)-L-x-[LV]-[EG]-D-L-x-[WY]-x(2)-[DE]-x(2)-[AST];
Motif 5 (SEQ ID NO: 80):
E-x(0,4)-L-x-[PT]-[FILV]-[ATV]-[IV]-P-[ADV]-[GST]
and
Motif 6 (SEQ ID NO: 81):
L-x-D-[AST]-I-[IV]-[IV]-x-[CGNS]-x-[AEQ];
or

ii) the motifs according to i) and in addition the consensus sequence as represented by the sequence listed under SEQ ID NO: 75; or
iii) the motifs according to i) or ii) above and in addition comprising all of the following consensus motifs
Consensus motif 1 (SEQ ID NO: 83): HPLQC[KR]ALELCF[NS]VA
Consensus motif 2 (SEQ ID NO: 84): NAL[GV]AA[LF]KRAQAHQR
and
Consensus motif 3 (SEQ ID NO: 85) LDDPSVSRVMREA[GS]F;
or
iv) any 5 of the motifs listed under i) and all three consensus motifs listed under iii); or
v) any 4 of the motifs listed under i) and all three consensus motifs listed under iii); or
vi) any 4 of the motifs listed under i) and any one of the consensus motifs listed under iii); or
vii) any 3 of the motifs listed under i); or
viii)One of the motifs as described herein above;

wherein -x represents in any motif position the presence of an amino acid residue of any type as often as the lowest integer number or the highest integer number in brackets following the -x indicate, or any of the integer numbers in between the lowest and the highest number, wherein the lowest integer number and the highest integer number might be identical and hence only one integer number is found within the brackets following -x, and wherein -x(1) is shortened to -x and any amino acid residue inserted at the position of -x does not need to be of the same type as the preceding one or another one inserted.

**5.** Method according to any of claims 1 to 4, wherein said one or more enhanced yield-related traits comprise increased yield relative to control plants, and preferably comprise increased biomass and/or increased seed yield relative to control plants.

**6.** Method according to any one of embodiments 1 to 5, wherein said nucleic acid encoding a CLP is of plant origin, preferably from a dicotyledonous plant, further preferably from the family Brassicaceae, most preferably from Arabidopsis species.

**7.** Method according to any one of claims 1 to 6, wherein said nucleic acid is operably linked to a constitutive promoter of plant origin, preferably to a medium strength constitutive promoter of plant origin, more preferably to a GOS2 promoter, most preferably to a GOS2 promoter from rice.

**8.** Construct comprising:

(i) nucleic acid encoding an CLP as defined in any of claims 1, 2, 4, 6 and 7;
(ii) one or more control sequences capable of driving expression of the nucleic acid sequence of (i); and optionally
(iii) a transcription termination sequence.

**9.** A host cell, preferably a bacterial host cell, more preferably an Agrobacterium species host cell comprising the construct according to any of claim 8 or the nucleic acid as defined in claim 2.

**10.** Use of a construct according to claim 8 in a method for making plants having one or more enhanced yield-related traits, preferably increased yield relative to control plants, and more preferably increased seed yield and/or increased biomass relative to control plants.

**11.** Method for the production of a transgenic plant having one or more enhanced yield-related traits relative to control plants, preferably increased yield relative to control plants, and more preferably increased seed yield and/or increased biomass relative to control plants, comprising:

(i) introducing and expressing in a plant cell or plant a nucleic acid encoding an CLP polypeptide as defined in any of claims 1, 2, 4, 6 and 7; and
(ii) cultivating said plant cell or plant under conditions promoting plant growth and development.

**12.** Plant, or part thereof, or plant cell, obtainable by a method according to any one of claims 1 to 7 or 11, wherein said plant, plant part or plant cell comprises a recombinant nucleic acid encoding a CLP polypeptide as defined in any of claims 1, 2, 4, 6 and 7.

**13.** Transgenic plant having one or more enhanced yield-related traits relative to control plants, preferably increased yield relative to control plants, and more preferably increased seed yield and/or increased biomass, resulting from modulated expression of a nucleic acid encoding an CLP polypeptide as defined in any of claims 1, 2, 4, 6 and 7 or a transgenic plant cell derived from said transgenic plant.

**14.** Harvestable part of a plant according to claim 12 or 13, wherein said harvestable parts are preferably shoot biomass and/or root biomass, preferably taproot or tuber biomass, and/or seeds.

**15.** Transgenic plant according to claim 12 or 13, or a transgenic plant cell derived therefrom, wherein said plant is a crop plant, such as beet, sugarbeet or alfalfa; or a monocotyledonous plant such as sugarcane; or a cereal, such as rice, maize, wheat, barley, millet, rye, triticale, sorghum, emmer, spelt, einkorn, teff, milo or oats.

Figure 1

A

| | pattern (Prosite-format) | pattern match from | pattern match to |
|---|---|---|---|
| CLP_pattern_01 | A-[AGLV]-K-x(0,2)-I-x(1,3)-Q-L-[IMV]-[IV]-S-I-L-[DH]-D-P-S-V-S-R-V-M-R-[DE]-A-[DG]-F-[NS]-S-[PST]-x-[LV]-[KR]-[ANST]-x-[IV]-E-x-[AE] | 128 | 167 |
| CLP_pattern_02 | F-x-E-x-[NST]-[ADEST]-E-N-L-x(2)-L-[CS]-x-A-L-x(2)-[EKR]-x-[PST] | 566 | 586 |
| CLP_pattern_03 | T-W-[FLM]-[FL]-F-x-G-x-[DGN]-x(2)-[ADG]-[KR]-x(2)-[IMV]-[ACS]-x-E-L-A-x-[LV]-V-F-G-S-x(3)-[FV]-x-[ACST]-x(3)-[ADGS]-[ADEGNST] | 621 | 658 |
| CLP_pattern_04 | G-x(1,2)-V-x(0,1)-L-x-[LV]-[EG]-D-L-x-[WY]-x(2)-[DE]-x(2)-[AST] | 278 | 296 |
| CLP_pattern_05 | E-x(0,4)-L-x-[PT]-[FILV]-[ATV]-[IV]-P-[ADV]-[GST] | 355 | 368 |
| CLP_pattern_06 | L-x-D-[AST]-I-[IV]-[IV]-x-[CGNS]-x-[AEQ] | 734 | 744 |

B

| Consensus motif # | Descrption | start | end |
|---|---|---|---|
| 1 | H-P-L-Q-C-[KR]-A-L-E-L-C-F-[NS]-V-A | 62 | 76 |
| 2 | N-A-L-[GV]-A-A-[LF]-K-R-A-Q-A-H-Q-R | 101 | 115 |
| 3 | L-D-D-P-S-V-S-R-V-M-R-E-A-[GS]-F | 141 | 155 |

C

PFAM domain PF02861 matches the N-terminal quarter of SEQ ID NO:2 at residues 23 or 24 to 53, 54 or 55 of SEQ ID NO: 2, preferably residues 23 to 55, and also at the residues 130 or 131 to 168 or169, preferably residues 130 to 169 of SEQ ID NO: 2..

## Figure 2

```
L     : -------------------------------------------------- :   -
H_45  : -------------------------------------------------- :   -
H_21  : -------------------------------------------------- :   -
H_19  : -------------------------------------------------- :   -
H_34  : MASWQPPAALHLRTHLRDLFSLATATKSVLAGCCAKSAGLGRLLRQIGRS :  50
H_25  : -------------------------------------------------- :   -
H_10  : -------------------------------------------------- :   -
H_9   : -------------------------------------------------- :   -
H_22  : -------------------------------------------------- :   -
H_17  : -------------------------------------------------- :   -
H_16  : -------------------------------------------------- :   -
H_20  : -------------------------------------------------- :   -
H_27  : -------------------------------------------------- :   -
H_38  : -------------------------------------------------- :   -
H_5   : -------------------------------------------------- :   -
H_12  : -------------------------------------------------- :   -
H_8   : -------------------------------------------------- :   -
H_32  : -------------------------------------------------- :   -
H_43  : -------------------------------------------------- :   -
H_30  : -------------------------------------------------- :   -
H_47  : -------------------------------------------------- :   -
H_36  : -------------------------------------------------- :   -
H_37  : -------------------------------------------------- :   -
H_11  : -------------------------------------------------- :   -
H_4   : -------------------------------------------------- :   -
H_18  : -------------------------------------------------- :   -
H_7   : -------------------------------------------------- :   -
H_6   : -------------------------------------------------- :   -
H_15  : -------------------------------------------------- :   -
H_39  : -------------------------------------------------- :   -
H_42  : -------------------------------------------------- :   -
H_14  : -------------------------------------------------- :   -
H_3   : -------------------------------------------------- :   -
H_29  : -------------------------------------------------- :   -
H_2   : -------------------------------------------------- :   -
H_41  : -------------------------------------------------- :   -
```

```
L     : -------------MRAGGCTVE-QALTADAANVVKQAMGLARRRGHAQVT :  36
H_45  : -------------MRSGGCAVQ-QKLAGDAAAVMRQAVSLARRRGHAQVT :  36
H_21  : -------------MRGGICSIQLQALTPEAATVVKQAVNLATRRGHAQVT :  37
H_19  : -------------MRGGICSIQLQALTPEATTVVKQAVNLATRRGHAQVT :  37
H_34  : WQVALATHRLAGEMRAGGCTVQ-QSLTAEAAAVVKQAVSLARRRGNAQVT :  99
H_25  : -------------MRAGGCTVQ-QSLTAEAAAVVKQAVSLARRRGNAQVT :  36
H_10  : -------------MRTGSCAVQ-QGLTPEAASIVKQAVTLAKRRGHAQVT :  36
H_9   : -------------MRTGSCAVQ-QGLTPEAASIVKQAVTLAKRRGHAQVT :  36
H_22  : -------------MRAGVCSIQLQALTSEAATLVKQAVTLATRRGHAQVT :  37
H_17  : -------------MRGGICSIQLQALTPEATTVVKQAVNLATRRGHAQVT :  37
H_16  : -------------MRGGICSIQLQALTPEAATVVKQAVNLATRRGHAQVT :  37
H_20  : -------------MRGGICSIQLQALTQEAATVVKQAVNLATRRGHAQVT :  37
H_27  : -------------MRAGGCTVQ-QALTAEAAAVVKQAVTLARRRGNAQVT :  36
H_38  : -------------MRAGGCAVQ-QALAAEAAGVVRQAVTLARRRGHAQVT :  36
H_5   : -------------MRAGGCTVQ-QALTADAASVIKQAVTLARRRGHAQVT :  36
H_12  : -------------MRAGICSVQ-QALTPEAVSLVKQAVGLARRRGHAQVT :  36
H_8   : -------------MRAGGCTVQ-QALTAEAASVIKQAVTLARRRGHAQVT :  36
H_32  : -------------MRAGGCTVQ-QALAPEAAAVVKQAVSLARRRGNAQVT :  36
H_43  : -------------MRAGGCAVQ-QALAAEAAAVVRQAVALARRRGHAQVT :  36
H_30  : -------------MRAGGCTVQ-QSLTAEAAAVVKQAVSLARRRGNAQVT :  36
H_47  : -------------MRSGGCAVQ-QELAGDAAAVMRQAVSLARRRGHAQVT :  36
```

Figure 2 (continued)

```
H_36 : -------------MRAGGCAVQ-QALAAEAAAVVRQAVALARRRGHAQVT :  36
H_37 : -------------MRAGGCAVQ-QALAAEAAAVVRQAVALARRRGHAQVT :  36
H_11 : -------------MRAGVCSVQ-QVLTADAASMVKQAVTLARRRGHAQVT :  36
H_4  : -------------MRAGGCTVQ-QALTAEAAGVVKQAVTLARRRGHAQVT :  36
H_18 : -------------MRAGICSVQ-QALTAEAANIVKQAVSLARRRGHAQVT :  36
H_7  : -------------MRAGGCTVQ-QALTTEAATVVKQAVTLARRRGHAQVT :  36
H_6  : -------------MRAGGCTVQ-QALTAEAAGVVKQAVTLARRRGHAQVT :  36
H_15 : -------------MRAGVCSVQ-QVLTADAASXVKQAVTLARRRGHAQVT :  36
H_39 : -------------MRAGGCAVQ-QALAAEAAGVVRQAVTLARRRGHAQVT :  36
H_42 : -------------MRAGGCAVQ-QALAPEAASVVRQAVTLARRRGHAQVT :  36
H_14 : -------------MRAGVCSVQ-QVLTADAASMVKQAVTLARRRGHAQVT :  36
H_3  : -------------MRAGGCTVQ-QALTAEAAGVVKQAVTLARRRGHAQVT :  36
H_29 : -------------MRAGGCTVQ-QALTAEAAAVVKQAVTLARRRGNAQVT :  36
H_2  : -------------MRAGGCTVE-QALTADAANVVKQAMGLARRRGHAQVT :  36
H_41 : -------------MRAGGCTVQ-QALTAEAAAVVKQAVSLARRRGNAQVT :  36


L    : PLHVASTMLSA-----------------PTGLLRTACLQS--HTHPLQCR :  67
H_45 : PLHVASAVL-----------------LDAGGLLRAACLRSRATSHPLQCK :  69
H_21 : PLHVASAMLAT-----------------STGLLRKACLQC--HSHPLQCK :  68
H_19 : PLHVASAMLAT-----------------STGLLRKACLQC--HSHPLQCK :  68
H_34 : PLHVASAMLAA---APAG------------QLLRAACLRS--HSHPLQCK : 132
H_25 : PLHVASAMLAA---PTG--------------LLRAACLRS--HSHPLQCK :  67
H_10 : PLHVANTMLSI-----------------TNGLLRTACLQS--HSHPLQCK :  67
H_9  : PLHVANTMLSI-----------------TNGLLRTACLQS--HSHPLQCK :  67
H_22 : PLHIATVMLAT-----------------STGLLRKACLQC--HSHPLQYK :  68
H_17 : PLHVASAMLAT-----------------STGLLRKACLQC--HSHPLQCK :  68
H_16 : PLHVASAMLAT-----------------STGLLRKACLQC--HSHPLQCK :  68
H_20 : PLHVASAMLAT-----------------STGILRKACLQC--HSHPLQCK :  68
H_27 : PLHVASAMLAP---PGG--------------LLRAACLRS--HSHPLQCK :  67
H_38 : PLHVASAMLS------------------AAGLLRAACLQS--HSHPLQCK :  66
H_5  : PLHVANTMLSA-----------------STGLFRTACLQS--HSHPLQCK :  67
H_12 : PLHVASTMLAS-----------------STGLLRRACLQS--HSHPLQCK :  67
H_8  : PLHVANTMLSA-----------------STGLLRTACLQS--HSHPLQCK :  67
H_32 : PLHVASAMLHQQVVAAATGTGTGPSSSTAAGLLRAACLQS--HSHPLQCK :  84
H_43 : PLHVASAMLS------------------AAGLLRAACLRS--HSHPLQCK :  66
H_30 : PLHVASAMLAA---PAG--------------LLRAACLRS--HSHPLQCK :  67
H_47 : PLHVASAVLSV---------------SDAGALLRAACLRSRASSHPLQCK :  71
H_36 : PLHVASAMLS------------------AAGLLRAACLRS--HSHPLQCK :  66
H_37 : PLHVASAMLS------------------AAGLLRAACLRS--HSHPLQCK :  66
H_11 : PLHVASVMLAS-----------------SSGLLRSACLRS--HSHPLQCK :  67
H_4  : PLHVANTMLAA-----------------TNGLLRTACLQS--HSHPLQCK :  67
H_18 : PLHVASAMLAS-----------------TNGLLRRACLQS--HSHPLQCK :  67
H_7  : PLHVANTMLSS-----------------STGLLRTACLQS--HSHPLQCK :  67
H_6  : PLHVANTMLAA-----------------TNGLLRTACLQS--HSHPLQCK :  67
H_15 : PLHVASVMLAS-----------------SSGLLRSACLRS--HSHPLQCK :  67
H_39 : PLHVASAMLS------------------AAGLLRAACLQS--HSHPLQCK :  66
H_42 : PLHVATAMLLP---------------PAPAGLLRAACLRS--HSHPLQCK :  69
H_14 : PLHVASVMLAS-----------------SSGLLRSACLRS--HSHPLQCK :  67
H_3  : PLHVANTMLAA-----------------TNGLLRTACLQS--HSHPLQCK :  67
H_29 : PLHVASAMLAP---PGG--------------LLRAACLRS--HSHPLQCK :  67
H_2  : PLHVASTMLSA-----------------PTGLLRTACLQS--HTHPLQCR :  67
H_41 : PLHVASAMLQAAGAAPAP------------GLLRAACLRS--HSHPLQCK :  72


L    : ALELCFNVALNRLPTSTGS------PMLG----VPTS---PFPSISNALG : 104
H_45 : ALELCFNVALNRLATAGG----PAPPPAMFEFHIH--SGHREPALSNALA : 113
H_21 : ALELCFNVALNRLPASTSS------PLLA----PQYS----TPSLSNALV : 104
```

Figure 2 (continued)

```
H_19 : ALELCFNVALNRLPASTSS------PLLA----PQYS----TPSLSNALV : 104
H_34 : ALELCFNVALNRLPAS-------ASSPLLGGN----HHCYHPPSLSNALV : 171
H_25 : ALELCFNVALNRLPASAAV----ASSPLLGGHGHHHHHHYYPPSLSNALV : 113
H_10 : ALELCFNVALNRLPASTSSS-----PMLQGSHHHHSH---ACPSISNALV : 109
H_9  : ALELCFNVALNRLPASTSSS-----PMLQGSHHHHSH---ACPSISNALV : 109
H_22 : ALELCFNVSLNRLPASTPN------PLLIS---PPYNSTTTTPSLSNALV : 109
H_17 : ALELCFNVALNRLPASTSS------PLLA----PQYS----TPSLSNALV : 104
H_16 : ALELCFNVALNRLPASTSS------PLLA----PQYS----TPSLSNALV : 104
H_20 : ALELCFNVALNRLPASTQS------PLLG----PQYST---TPSLSNALV : 105
H_27 : ALELCFNVALNRLPASAAV----ASSPLLGGHGHGHHGHYYPPSLSNALV : 113
H_38 : ALELCFNVALNRLPTAGPAAA---AAIFHHHPHHPGGGGGHHPALSNALV : 113
H_5  : ALELCFNVALNRLPASTSS------PILG----THSQ---QFPSISNALV : 104
H_12 : ALELCFNVALNRLPASTSS------ALLG----PHSS----YPSLSNALV : 103
H_8  : ALELCFNVALNRLPTSTSS------PMIG----TPSQ---QFPSISNALV : 104
H_32 : ALELCFNVALNRLPASA--------SPLLGGHGH----VYYPPSLSNALV : 122
H_43 : ALELCFNVALNRLPTAGPAAAAVMFHPHHHHHHAGHGQQHAVPVLSNALV : 116
H_30 : ALELCFNVALNRLPASAAV----ASSPLLGGHGHG-HHHYYPPSLSNALV : 112
H_47 : ALELCFNVALNRLATTAGMPGPPAPPPAMFQFHHAPAGGHRAPALSNALA : 121
H_36 : ALELCFNVALNRLPTA----AASMFHPHHH-----AGQQHA-PVLSNALV : 106
H_37 : ALELCFNVALNRLPTA----AASMFHPHHH-----AGQQHA-PVLSNALV : 106
H_11 : ALELCFNVALNRLPASSSS------PLLA----PHSS----HPSLSNALV : 103
H_4  : ALELCFNVALNRLPASTSS------PMLG----PHS----QHPSISNALV : 103
H_18 : ALELCFNVALNRLPASTSS------ALLG----PHSS----YPSLSNALV : 103
H_7  : ALELCFNVALNRLPASTSS------PVLG----THAQ---QYPSISNALV : 104
H_6  : ALELCFNVALNRLPASTSS------PMLG----PHS----QHPSISNALV : 103
H_15 : ALELCFNVALNRLPASSSS------PLLA----PHSS----HPSLSNALV : 103
H_39 : ALELCFNVALNRLPTAGPAAA---AAIFHHHPHHPGGGGGHHPALSNALV : 113
H_42 : ALELCFNVALNRLPTCGPAAMFHGGAHMQHHHHHRG--AAEAPALSNALV : 117
H_14 : ALELCFNVALNRLPASSSS------PLLA----PHSS----HPSLSNALV : 103
H_3  : ALELCFNVALNRLPASTSS------PMLG----PHS----QHPSISNALV : 103
H_29 : ALELCFNVALNRLPASAAV----ASSPLLGGHGHGHHGHYYPPSLSNALV : 113
H_2  : ALELCFNVALNRLPTSTGS------PMLG----VPTS---PFPSISNALG : 104
H_41 : ALELCFNVALNRLPASAG------ASPLLG-HGHG-VGVYYPPSLSNALV : 114


L    : AAFKRAQAHQRRG-SIESQQQ------------PILAVKIEVEQLIISIL : 141
H_45 : AAFKRAQGNQRRGGGSAADGQH--------QQN--VAAKVELDQLVISIL : 153
H_21 : AAFKRAQAHQRRG-SIENQQQ------------HILALKIEVEQLVISIL : 141
H_19 : AAFKRAQAHQRRG-SIENQQQ------------HILALKIEVEQLVISIL : 141
H_34 : AAFKRAQAHQRRGGSVESQHQQP----------VVAAVKIELDQLVVSIL : 211
H_25 : AAFKRAQAHQRRG-SVESQQQP------------VLAVKIELEQLVVSIL : 150
H_10 : AAFKRAQAHQRRG-SVENQQQ------------PLLAVKIKLEQLIISIL : 146
H_9  : AAFKRAQAHQRRG-SVENQQQ------------PLLAVKIELEQLIISIL : 146
H_22 : AAFKRAQAHQRRG-SIDQNQQQ-----------PIITLKIKVEQLIVSIL : 147
H_17 : AAFKRAQAHQRRG-SIENQQQ------------HILALKIEVEQLVISIL : 141
H_16 : AAFKRAQAHQRRG-SIENQQQ------------HILALKIEVEQLVISIL : 141
H_20 : AAFKRAQAHQRRG-TIENQQQQ-----------HILALKIEVEQLIISIL : 143
H_27 : AAFKRAQAHQRRG-SVETQQQP------------VLAVKIELEQLVVSIL : 150
H_38 : AAFKRAQAHQRRG---SVEGQP----PPQPPPSPVVASKVELEQLIISIL : 156
H_5  : AAFKRAQAHQRRG-SIENQQQ------------PLLAVKIELEQLIISIL : 141
H_12 : AAFKRAQAHQRRG-SIENQQQ------------PILALKIEIEQLIISIL : 140
H_8  : AAFKRAQAHQRRG-SIENQQQ------------PLLAVKIELEQLMISIL : 141
H_32 : AAFKRAQAHQRRG-SVDTQQQP------------VLAVKIELEQLVISIL : 159
H_43 : AAFKRAQAHQRRGVVEGVQGQGQGQAPAQPPPQPVLAAKVDIEQLIISIL : 166
H_30 : AAFKRAQAHQRRG-SVESQQQP------------VLAVKIELEQLVVSIL : 149
H_47 : AAFKRAQANQRRGGGGGGFGVR--------RGGRPAPAPVELEQLVISIL : 163
H_36 : AAFKRAQAHQRRGSVDGAQGQ---------PPQPVLAGKVDIDQLIISIL : 147
H_37 : AAFKRAQAHQRRGSVDGAQGQ---------PPQPVLAGKVDIDQLIISIL : 147
H_11 : AAFKRAQAHQRRA-SIENQQQ------------PILALKVEIEQLIISIL : 140
```

Figure 2 (continued)

```
H_4  : AAFKRAQAHQRRG-SIENQQQ------------PLLAVKIELEQLIISIL : 140
H_18 : AAFKRAQAHQRRG-SIENQQQ------------PILALKIEIEQLIISIL : 140
H_7  : AAFKRAQAHQRRG-SIENQQQ------------PLLAVKIELEQLIISIL : 141
H_6  : AAFKRAQAHQRRG-SIENQQQ------------PLLAVKIELEQLIISIL : 140
H_15 : AAFKRAQAHQRRA-SIENQQQ------------PILALKVEIEQLIISIL : 140
H_39 : AAFKRAQAHQRRG---SVEGQP----PPQPPPSPVVASKVELEQLIISIL : 156
H_42 : AAFKRAQAHQRRG---GAGDG------VQQTAAPVLAAKVELEQLIVSIL : 158
H_14 : AAFKRAQAHQRRA-SIENQQQ------------PILALKVEIEQLIISIL : 140
H_3  : AAFKRAQAHQRRG-SIENQQQ------------PLLAVKIELEQLIISIL : 140
H_29 : AAFKRAQAHQRRG-SVETQQQP------------VLAVKIELEQLVVSIL : 150
H_2  : AAFKRAQAHQRRG-SIESQQQ------------PILAVKIEVEQLIISIL : 141
H_41 : AAFKRAQAHQRRG-SVETQQQP------------VLAVKIELEQLVISIL : 151


L    : DDPSVSRVMREAGFSSPQVKTK--VEQAVSL-----EICSK---TTSSS- : 180
H_45 : DDPSVSRVMREAGFSSAEVKAN--VEKAISS-----SEQSSNTASSSSAS : 196
H_21 : DDPSVSRVMREAGFSSTLVKTR--VEQAVSM-----EVCSQKA------- : 177
H_19 : DDPSVSRVMREAGFSSTLVKTR--VEQAVSM-----EVCSQKASSDRS-- : 182
H_34 : DDPSVSRVMRDAGFSSTQVKAN--VEQAVCS---SSTMATAAAAPSKNPN : 256
H_25 : DDPSVSRVMREAGFSSTQVKAN--VEQAVCSSTTTTSTAAAATAPSKNPN : 198
H_10 : DDPSVSRVMREAGFSSTQVKSN--VEQAVSL-----EICSQ--DNGSGK- : 186
H_9  : DDPSVSRVMREADFNSTQVKSN--VEQAVSL-----EICSQ--NNGSGN- : 186
H_22 : DDPSISRVMREAGFSSSLVKTR---QQAYSK-----ENTTELQVLGGGSF : 189
H_17 : DDPSVSRVMREAGFSSTLVKTR--VEQAVSM-----EVCSQKASSDRS-- : 182
H_16 : DDPSVSRVMREAGFSSTLVKTR--VEQAVSM-----EVCSQKA------- : 177
H_20 : DDPSVSRVMREAGFSSTLVKSRVEVEQALPI-----EVSSTKVSSEYHKN : 188
H_27 : DDPSVSRVMREAGFSSTQVKAN--VEQACST------TTATSAPPNQNPN : 192
H_38 : DDPSVSRVMREAGFSSSQVKAN--VEKAVVA-----SLDHANAAGGGGGH : 199
H_5  : DDPSVSRVMREAGFSSTQVKSN--VEQAVSL-----EICSQ-SAPSVSS- : 182
H_12 : DDPSVSRVMKEAGFSSTQVKNK--VEQTVSL-----EICPQSSLTVSC-- : 181
H_8  : DDPSVSRVMREAGFSSTQVKSN--VEEAVSL-----EICSQ-SVPSVSI- : 182
H_32 : DDPSVSRVMREAGFSSTQVKAN--VEQAVSS----IEANNSASTNTAAAA : 203
H_43 : DDPSVSRVMREAGFSSSQVKAN--VEKAVSL-----SSSSSLPDHQPNTT : 209
H_30 : DDPSVSRVMREAGFSSTQVKAN--VEQAVCS--TTTTTAATAAAPGKNPN : 195
H_47 : DDPSVSRVMRDAGFASAEVNAN--VEKAVSS-----SEQSSNTATSSTAS : 206
H_36 : DDPSVSRVMREAGFSSSQVKAN--VEKAVSA-----SSSP--EHQQQNTT : 188
H_37 : DDPSVSRVMREAGFSSSQVKAN--VEKAVSA-----SSSP--EHQQQNTT : 188
H_11 : HDPSVSRVMREAGFSSTQLRTN--IEQAVSL-----DVCSQSPAVSS--- : 180
H_4  : DDPSVSRVMREAGFSSTQVKSN--VEQAVSL-----EICSQ--APSVSS- : 180
H_18 : DDPSVSRVMREAGFSSTQVKNK--VEQAVSL-----EICSQGTTATSC-- : 181
H_7  : DDPSVSRVMREAGFSSTQVKSN--VEQAVSL-----EICSQNSAPVSSS- : 183
H_6  : DDPSVSRVMREAGFSSTQVKSN--VEQAVSL-----EICSQ--APSVSS- : 180
H_15 : HDPSVSRVMREAGFSSTQLRTN--IEQAVSL-----DVCSQSPAVSS--- : 180
H_39 : DDPSVSRVMREAGFSSSQVKAN--VEKAVVA-----SLDHANAAGGGGGH : 199
H_42 : DDPSVSRVMREAGFSSSQVKDN--VEKAVSS-----SSPSLERAAN---- : 197
H_14 : HDPSVSRVMREAGFSSTQLRTN--IEQAVSL-----DVCSQSPAVSS--- : 180
H_3  : DDPSVSRVMREAGFSSTQVKSN--VEQAVSL-----EICSQ--APSVSS- : 180
H_29 : DDPSVSRVMREAGFSSTQVKAN--VEQACST------TTATSAPPNQNPN : 192
H_2  : DDPSVSRVMREAGFSSPQVKTK--VEQAVSL-----EICSK---TTSSS- : 180
H_41 : DDPSVSRVMREAGFSSTQVKAN--VEQAVS------SSMEAATTKPQNPN : 193


L    : KPKEGK--------------------------------LLTPVRNEDVMN : 198
H_45 : PSTIT-----KEPRAKADAVQVVG----------------------DAAR : 219
H_21 : QAKENITKPHHQVVLGGRNNVSPSGPFGQV-GGSFMKPNLDHVNNDDVTS : 226
H_19 : HAKENITKPHHVVLGGSNNVSPSSGPFGQVAGGSFMKPNLDHVNNDDVTS : 232
H_34 : PSSS----ATTTSPPPKEAKAKPP------------LALHHQARDEDVAA : 290
H_25 : PSSS----AATASPPQEAAKGNK--------------LPLHQARDEDVAA : 230
```

Figure 2 (continued)

```
H_10  : NNNNSN-KAKENNSS-GEKGS-----------------VLDPIRVEDVAS : 217
H_9   : NNNNNNNNKAEENNSSSGEKGL-----------------VLDPIRVEDVAS : 219
H_22  : KSMEDLVHDDAG----------------------------DHVV-DDVTS : 210
H_17  : HAKENITKPHHVVLG-------------------------DHVNNDDVTS : 207
H_16  : QAKENITKPHHQ-------------------------PNLDHVNNDDVTS : 202
H_20  : QSKELSLKPQVLSLGG------------------SYTKPIDCVNNDDVTS : 220
H_27  : PSCTGAAATATATTSPAHPPEIKAK----------LPLLDMLARDEDIAA : 232
H_38  : AGSPNSGHGGRRKESSSSRARV-----------------------DDDAMR : 227
H_5   : KSKESNGLVLSQSPTSSQVGAK--------------ATVLDPIKNEDVMC : 218
H_12  : QPKE-IIKPQVLSAS-VSQSLPFSQFG-----IIHSKPLD-QVRNDDVMS : 223
H_8   : KSNESNGLVHPESPPWSQVGAK--------------AAVLDPIKNEDVMC : 218
H_32  : HQNP-----NPRAAPHEETMPSKLQ----------LPLDLDQVRDEDVAV : 238
H_43  : IPPSG-AHATGSPAAGGSGHASSRR----------PNAGGNKADDDDAMR : 248
H_30  : PSSS----ATTSPTAQEAKAINKLP----------LPLPLHQARDEDVAA : 231
H_47  : PNTTTNNNPTKDKESRAKADDIVG----------------------DAVR : 234
H_36  : IPPTSRAHAAISPGGAASG---------------------------DAMR : 211
H_37  : IPPTSRAHAAISPGGAASG---------------------------DAMR : 211
H_11  : LSKE-----------------------------ITLNNPFDEAQEEDVKS : 201
H_4   : KSKESNLLVLSQSPPMGQIGVKLG-----------KPTVPDPVRNEDVMS : 219
H_18  : QSKE-ITKPQIFSTNNVSPSLPFSHYG-----VTLSKPLDHEVSNDDVMS : 225
H_7   : KSKESNNNNSVLALSHTQVGARTSCRSSPT----TSTTSLDPIRKEDVMS : 229
H_6   : KSKESNLLVLSQSPPMGQIGVKLG-----------KPTVPDPVRNEDVMS : 219
H_15  : LSKESNNPPLILGTNVSQSSTFIQF-------GVTLNNPFDEAQEEDVKS : 223
H_39  : AGSPNSGHGGRRKESSSSRARV-----------------------DDDAMR : 227
H_42  : MKTTGSIKESRAKSAGS----------------------------DDAMR : 219
H_14  : LSKESNNPPLILGTNVSQSSTFIQF-------GVTLNNPFDEAQEEDVKS : 223
H_3   : KSKE------------------------------------NPVRNEDVMS : 194
H_29  : PSCTGAAATATATTSPAHPPEIKAK----------LPLLDMLARDEDIAA : 232
H_2   : KPKEGK-------------------------------LLTPVRNEDVMN : 198
H_41  : PSSS-----SPPPAAHQEAKPSR-------------CIDQVVVREEDVAA : 225

L     : VINNLVDK----------KRRNFVIVGECLATIDGVVKTVMEKVDKKDVP : 238
H_45  : VLDCMASG---------TNRCVAVVG-ESAAAAEGVVKAVMDKVSKGELR : 259
H_21  : VLSELAKR----------R--NTVIVGESVTNAEGVVRGVIERFEVGNVP : 264
H_19  : VLSELVRR----------K--NTVIVGEGVANAEGVAREVMERFEVGNVP : 270
H_34  : VLDCLASR---------SKRRVVVIA-ESAAAAEAMAHAAVDKIKRGEVK : 330
H_25  : VLDCLASR---------SKRRVVVIA-ESAAAAEATAHAAMDKIKSAEAK : 270
H_10  : VIENLGSE----------RKRSVVIVGECVTSLEGVVRGVMEKVDKGDVG : 257
H_9   : VIENLGCE----------RKRSVVIVGECVTSLEGVVRGVMEKIDKGDVG : 259
H_22  : VLSELVSK----------RR-NTVIVGESLASPEGIVRGLIENLERGSVQ : 249
H_17  : VLSELVRR----------K--NTVIVGEGVANAEGVAREVMERFEVGNVP : 245
H_16  : VLSELAKR----------R--NTVIVGESVTNAEGVVRGVIERFEVGNVP : 240
H_20  : VLSELVKR----------RR-NTVIVGESVSNAEGVAKGVMERFEIGDVP : 259
H_27  : VLDCLAPAAAGGRGGGGSRRRVVVVA-ESTAAAEATARAAVDRVRRGEAK : 281
H_38  : VLDCMASG---------TKRCVVVVGGEGAAAAEAVVKAVMDRVSKAELH : 268
H_5   : VIENLVNK----------RRRSFVIVGESLASIEVVVKGVIDKVQKGDVP : 258
H_12  : VLNTLVGK----------KR-NTIITGECLATAESVVRGVMDKFERGEVS : 262
H_8   : VIENLMNK----------RRRSFVIVGESLASIEVVVKGVKDKVQKGDVP : 258
H_32  : VLDCLASR---------SKKRVMVVA-ECAATAEAPTRAAVEKIKRGEAL : 278
H_43  : VLDCMASG---------TKRCVVVVG-ESAATAEVVVKSVMDRVSKGELQ : 288
H_30  : ILDCLASR---------SKRRVVVIA-ESVSAAEAMAHAAVDKIKRAEAK : 271
H_47  : VLDCMASG---------TNRCVVVLG-ETAAAAERVVKAVMDKVSKGELR : 274
H_36  : VLDCMASG---------SKRSVVVVG-ESAATAEVVVKAVMNRVSKGELQ : 251
H_37  : VLDCMASG---------SKRSVVVVG-ESAATAEVVVKAVMNRVSKGELQ : 251
H_11  : LLDAFTSK----------RRRNTVVVGETLASAEGVVRGLMNKFERGDVP : 241
H_4   : VIENLMNK----------RRKNTVIVGECLATIEGVVRGVMDKVDKGDVP : 259
H_18  : VLNTLMEK----------KR-NTIITGECLASTESVVRLVMNKIERGLAP : 264
H_7   : VIENLINK----------RKRSVVIVGECLVSLEGVVKGVMDKVIKGDVP : 269
```

Figure 2 (continued)

```
H_6  : ------------------------------------VRGVMDKVDKGDVP : 233
H_15 : LLDAFTSK----------RRRNTVVVGETLASAEGVVRGLMNKFERGDVP : 263
H_39 : VLDCMASG---------TKRCVVVVGGEGAAAAEAVVKAVMDRVSKAELH : 268
H_42 : VLECMASG---------TRRCLVVVG----EGAEAAVKAVMDKVSKSELH : 256
H_14 : LLDAFTSK----------RRRNTVVVGETLASAEGVVRGLMNKFERGDVP : 263
H_3  : VIENLMNK----------RRKNTVIVGECLATIEGVVRGVMDKVDKGDVP : 234
H_29 : VLDCLAPAAAGGRGGGGSRRRVVVVA-ESTAAAEATARAAVDRVRRGEAK : 281
H_2  : VINNLVDK----------KRRNFVIVGECLATIDGVVKTVMEKVDKKDVP : 238
H_41 : ILDCLATR---------SKKRVMVVA-ECAAAAEAAARAAVDRIRRGEAR : 265


L    : EV----LKDVKFITLSFSS--FGQPS-RADVER----------------- : 264
H_45 : R-QHQRLKNAQFVPFSAAS--FQRTP-REEVEA----------------- : 288
H_21 : GD----LR---------------NIS-KEEVEQ----------------- : 277
H_19 : GD----LRYVQFVSLPLMC--FRNIS-KEEVEQ----------------- : 296
H_34 : HE-HDALRGAQVVSLRVSS--FREMP-REEAER----------------- : 359
H_25 : ---HDALRGAQVVSVRVSS--FREMA-REETER----------------- : 297
H_10 : DECT--LRGVKFISLSLSS--FGNVS-RVEVEQ----------------- : 285
H_9  : DECT--LRGVKFISLSLSS--FGNVS-RVEVEQ----------------- : 287
H_22 : GE----LRFVQFVSLPLVS--FRNIG-KKEVER----------------- : 275
H_17 : GD----LRYVQFVSLPLMC--FRNIS-KEEVEQ----------------- : 271
H_16 : GD----LRYVQFVSLPLMC--FRNIS-KEEVEQ----------------- : 266
H_20 : ME----LRYVQFVSLPLIC--FRNIS-KEEVEK----------------- : 285
H_27 : -Q-HDALRGAQVVSLRVSS--FRDMP-REEAER----------------- : 309
H_38 : HR-HERLKNLQFVPLSIAS--FHGAP-REEVEA----------------- : 297
H_5  : EA----LREVKFLTIPVSS--FGHFS-RVEVEH----------------- : 284
H_12 : GD----LRSVRFKNLPLFS--FRSLS-KEDLEQ----------------- : 288
H_8  : EG----LREVKFLPIPVSS--FGSFS-RVEVEH----------------- : 284
H_32 : R-------GAQVVSLRVSR--FRDLP-RDEAER----------------- : 301
H_43 : QR-HERLKNVQFVPLSAAS--FQRMP-REEVEA----------------- : 317
H_30 : ---HDALRGAQVVSLRVSS--FRDMP-REETER----------------- : 298
H_47 : RRQHERLKNAQLVPFSAAS--FQRMP-REEVEA----------------- : 304
H_36 : QR-HERLKNVQFVPLSAAS--FQRMP-REEVEA----------------- : 280
H_37 : QR-HERLKNVQFVPLSAAS--FQRMP-REEVEA----------------- : 280
H_11 : GD----LRYVQFISLPLFS--LKNLS-KEEVEQ----------------- : 267
H_4  : EA----LRDVKLISLPLFS--FGHHS-REEVEQ----------------- : 285
H_18 : GE----LRAMRFISFPLIS--LRDLP-QEEVEQ----------------- : 290
H_7  : EA----LKEVKFISFPLSS--LGHLSSRVEVDQ----------------- : 296
H_6  : EA----LRDVKLISLPPSSDFWTSLQ-RRGPNR----------------- : 261
H_15 : GD----LRYVQFISLPLFS--LKNLS-K-EVEQ----------------- : 288
H_39 : HR-HERLKNLQFVPLSIAS--FHGAP-REEVEA----------------- : 297
H_42 : HRHHERLKSVQFVPLSVTS--FRHAA-REEVDA----------------- : 286
H_14 : GD----LRYVQFISLPLFS--LKNLS-KEEVEQ----------------- : 289
H_3  : EA----LRDVKLISLPLFS--FGHHS-REEVEQ----------------- : 260
H_29 : -Q-HDALRGAQVVNLRVSS--FRDMP-REEAER----------------- : 309
H_2  : EV----LKDVKFITLSFSS--FGQPS-RADVERKLEELEADVERKLEELE : 281
H_41 : QH-----AQAQVVTLAVSR--FRGAP-REEAER----------------- : 290


L    : -----KLEELETLVKSCVG---KGVILNLGDLNWFVESRTRGSS-LY--- : 302
H_45 : -----RAGDLCALVRECCAAG-KGVVLVLEDLGYAAEAWTAALWTRSD-R : 331
H_21 : -----KLMEVRNLVKSYVG---GGVVLYLGDLKWLFEFWANFREQ----- : 314
H_19 : -----KLMEIRNLVKSYVG---RGVVLYLGDLKWLFEFWANFCEQ----- : 333
H_34 : -----RLGELRCLVKQGR--R-QRVVLVVEDLKWAAEFWAGHDGQSGRRG : 401
H_25 : -----RLGELRCLVRGRR--G-QVVVLVVEDLKWAAEFWAGHVVQSGRRG : 339
H_10 : -----KVGELRSLVKASEH--SKGYVLYLGDLKWVFDFRARGSQ------ : 322
H_9  : -----KVEELRGLVKASEH--SKGYVLYLGDLKWVLDFRASGSQ------ : 324
H_22 : -----KLVELRNLVKSHVG---RGFILYLGDLKWLFEFWSSYCEQ----- : 312
```

Figure 2 (continued)

```
H_17 : -----KLMEIRNLVKSYVG---RGVVLYLGDLKWLFEFWANFCEQ----- : 308
H_16 : -----KLMEVRNLVKSYVG---GGVVLYLGDLKWLFEFWANFREQ----- : 303
H_20 : -----KFVEVRSLVKSYMG---RGVILYLGDLKWLFEFWSSYCEQ----- : 322
H_27 : -----RLAELRCLVKS-R--G-ARVLLVVEDLKWAADFWAAAHAGARRVG : 350
H_38 : -----KASDLRALVRSGCAAG-KGVVLVLEDLAYAADAWAAASNTRRRAA : 341
H_5  : -----KLEELKIHVRSYMG---KGVVLNLGDLKWAIENR-ASSSSSE--- : 322
H_12 : -----KLMELRCIVKSYIS---TGVVLYLGDLKWIADFWSSYGEQ----- : 325
H_8  : -----KLEELKGHVRSYMG---KGVVLNLGDLKWAIENRDTSSSSHE--- : 323
H_32 : -----LLVELRCAVKVGGR-A-GGVVLVVEDLGWAAEFWAARAESGRARW : 344
H_43 : -----KAGDLRALVRQGCAAG-KGVVLVLEDLAFAADAWAAVSERRR--H : 359
H_30 : -----RLGELRCLVRGRR--Q-QEVVLVVEDLKWAAEFWAGHVQ-SGRRG : 339
H_47 : -----RAGDLCALVRECCAAG-RGVVLVLEDLAYAAEAWTAASWKRSSGH : 348
H_36 : -----KAADLRALVRQGCAAG-KGVVLVLEDLAYAAEAWAAVSETRRRSS : 324
H_37 : -----KAADLRALVRQGCAAG-KGVVLVLEDLAYAAEAWAAVSETRRRSS : 324
H_11 : -----KLVKLTCLLKSYVC---RGVVLYLGDLKWVSEFESNYGE------ : 303
H_4  : -----KLGELKSLVKSCVG---RGVILYLEDLKWTTDYRASSSE------ : 321
H_18 : -----KLVELRCTVKSYLN---RGVFLYLGDIKWVAEFWSEYGEQ----- : 327
H_7  : -----KLEELKVHIRSYLS---KGVVLNLGDLKWVVEYRANNLS------ : 332
H_6  : -----KLGELKSLVKSCVG---RGVILYLEDLKWTTDYRASSSE------ : 297
H_15 : -----KLVKLNCLLKSYVC---RGVVLYLGDLKWVSEFESNYGE------ : 324
H_39 : -----KASDLRALVRSGCAAG-KGVVLVLEDLAYAADAWAAASNTRRRAA : 341
H_42 : -----KTSDLRALVCEARAAG-KGVALVVEDLAYAADAWH-----KRRGE : 325
H_14 : -----KLVKLTCLLKSYVC---RGVALYLGDLKWVSEFESNYGE------ : 325
H_3  : -----KLGELKSLVKSCVG---RGVILYLEDLKWTTDYRASSSE------ : 296
H_29 : -----RLAELRCLVKS-R--G-ARVLLVVEDLKWAADFWAAAHTGARRVG : 350
H_2  : ADVERKLEELETLVKSCVG---KGVILNLGDLNWFVESRTRGSS-LY--- : 324
H_41 : -----RLAELRCAVRGGG--G-RAVVLVVEDLAWAAEFWAGRRPPPSSCG : 332
```

```
L    : -------NNNDSYCVVEHMIMEIGKLACGLVMGDH-----GRFWLMGLAT : 340
H_45 : SARG-----LRHYCPVEHAVMELSSLVRGGGGR-----DKDMFWVLGFAA : 371
H_21 : --------KTN-YCSVEHMVMELKKLVCGSGES-------SRLWLMGIST : 348
H_19 : --------KRNYYCSIEQMVMELKKLVCGSGES-------SRLWLMGIAT : 368
H_34 : G---------YYYCAVEHVVNELRALASGG----------SLCWLLGFGT : 432
H_25 : Y----------YYCSVEHVVTELRALASGGGGG--------SLCWLLGFGT : 371
H_10 : --------GGGCYCPVDHMVVEIGKLVNGVEEN-G-----ARFWVMGVAT : 358
H_9  : --------GRGCYCPVDHMVGEIGKLVNGTEEN-G-----GRFWVMGVAT : 360
H_22 : --------RTNYYCSVVHIVMELKKLISGNGEN-------GRLWLMGIAT : 347
H_17 : --------KRNYYCSIEQMVMELKKLVCGSGES-------SRLWLMGIAT : 343
H_16 : --------KTN-YCSVEHMVMELKKLVCGSGES-------SRLWLMGIST : 337
H_20 : --------KRNYYCSVEHMVMEIKKLVSGSGES-------SRLWLMGIAN : 357
H_27 : SGGG-----GGYYCSVEHVVTEVRALASCDGG----------IWLVGFGT : 385
H_38 : AAAG---GQS--YCPMEHAVMEVSSLVSG-GGGGG-----ERFWVLGFGS : 380
H_5  : -------QGRCFFCPMEYMIIELGKLACGIGENIN-----GRFWLMGIAT : 360
H_12 : --------RRSYYCTADHIILELKRLVHGFSET-------GRLWLMGIAT : 360
H_8  : -------QGSCYFCPLVYLIVELGKFACAIGDN-N-----GRFWLMGIAT : 360
H_32 : P-SS-----CCYYCAVEHAVAEVRALACRGGDG---------VWLIGYGT : 379
H_43 : GSVGREHGQC-GYCPVEHAVMEVGSLVSAAAGGGGGGRGLDRFWLLGFGN : 408
H_30 : -----------YYSSVEHVVTELRALLASGGGGDH--GGGSMCWLLGFGT : 376
H_47 : RAHG-----LIDYCPVQHAVMELSSLVRGAGGRGR---DKGMFWLLGFGA : 390
H_36 : GSGGRDHGQG-CYCPVEHAVMEVGSLVSAAAGGGG--RGLDRFWLLGFGN : 371
H_37 : GSGGRDHGQG-CYCPVEHAVMEVGSLVSAAAGGGG--RGLDRFWLLGFGN : 371
H_11 : --------RRNYCSPVEHIIMELGRMMCGIGDR-------GRMWLLGTAT : 338
H_4  : -------QGRNYYCPVEHMIMELGKLVCGFGEN-------GRFWLMGIAT : 357
H_18 : --------RRSYYCSGEYIIMELKRLIRGIGET-------ERLWLMGVAT : 362
H_7  : --------------PMEHMIMEIGKLASGISEN-N-----GKFWLTGIAT : 362
H_6  : -------QGRNYYCPVEHMIMELGKLVCGFGEN-------GRFWLMGIAT : 333
H_15 : --------RRNYCSPVEHIIMELGRMMCGIGDR-------GRMWLLGTAT : 359
H_39 : AATG---GQS--YCPMEHAVMEVSSLVSG-GGGGG-----ERFWVLGFGS : 380
```

Figure 2 (continued)

```
H_42 : HVHG---GQSQYYCPVEHAVMEVSGLVSGDSGSGG-----GRFWLLGFAS : 367
H_14 : --------RRNYCSPVEHIIMELGRMMCGIGDR-------GRMWLLGTAT : 360
H_3  : -------QGRNYYCPVEHMIMELGKLVCGFGEN-------GRFWLMGIAT : 332
H_29 : SGGG-----G-YYCSVEHVVTEVRALASCDGG----------IWLVGFGT : 384
H_2  : -------NNNDSYCVVEHMIMEIGKLACGLVMGDH-----GRFWLMGLAT : 362
H_41 : AGAG-----GYYYCAVEHAVAEVRALACGGGGGG------GGVWLVGHGT : 371


L    : SQTYVRCK-SGQPSLESLWCLTTLTIPATSNSLRLSLVSESELEVK---- : 385
H_45 : YAPYTSCR-SGQPSLETVLGLRPVVVPDG------SLGGESEETHCG--- : 411
H_21 : FKTYMKCK-ICHPSLETIWELHPFTIPVG--ILSLSLNLDSDFQAQ-ERN : 394
H_19 : FKAYMKCK-ICHPSLEAIWELHPFTIPVG--SLSLSLNFHSDFQAQ-ERS : 414
H_34 : YQAYTKCR-VGQPSLESLWGLQTLTVPAGS--LALSLTCAFDDSALG--- : 476
H_25 : YQAYTRCR-VGQPSLESLWELQTLTVPAGS--LALSLNCAFDDSALGLG- : 417
H_10 : FQAYMRCK-NGQPSLETLWGLHPITIPAGS--LRLSLITDSGVQNQPTNE : 405
H_9  : FQAYMRCK-NGQPSLETLWCLHPITIPAGS--LRLSLITDSGLQDQPTNK : 407
H_22 : FGTYMKGQ-ACHPSLETIWDLHLFTVPVLLSSLRLGLTFDSDFQVQ-ERS : 395
H_17 : FKAYMKCK-ICHPSLEAIWELHPFTIPVG--SLSLSLNFHSDFQAQ-ERS : 389
H_16 : FKTYMKCK-ICHPSLETIWELHPFTIPVG--ILSLSLNLDSDFQAQ-ERN : 383
H_20 : FKTYMKCK-ISHPSLETIWELHPFTIPVG--SLSLSLNFDSDFQAK-ERS : 403
H_27 : YQTYMKCR-AGHPSLESMWGLQTLAVPAGS--LALSLTCAFDDSALG--- : 429
H_38 : YQVYMKCRAAGQPPLEAVWELHPVVVPDGG--LALSLTCSEASQATHQ-- : 426
H_5  : FQTYMKCK-SGHPSGGTVLGLHPLTIPAGS--LRLSLISDSDLRCQSTRN : 407
H_12 : FQTYMKCK-AGHPSLETMWELNPVTIPVG--SLNLSLKLDSDSQSHQSRS : 407
H_8  : FQTYMKYK-SDHPPGDTVLGLHPLTIPAGS--LRLSLISDSDLLRQSTSN : 407
H_32 : YQSYMRCR-AGQPSLETLWGLQTLAVPAGS--LALSLNFVGDSATAMT-- : 424
H_43 : NQAYMKSR-AGQPSLEAVWELHPVVVPDGG--LSLSLTCNSDAEQAN--- : 452
H_30 : YQAYMRCR-VGQPSLESLWGLQTLTVPAGS--LVLSLTCAFDDSALG--- : 420
H_47 : SASYTSCR-SGQPSLETVLGLHPVVVPDGG--LALSLGGDSEVTHCS--- : 434
H_36 : NQAYLKSR-AGQPSLEAVWELHPIVVPDGG--LALSLRCNSDAEQA---- : 414
H_37 : NQAYLKSR-AGQPSLEAVWELHPIVVPDGG--LALSL---SDAEQA---- : 411
H_11 : FQTYMRCK-AGHPSLETIWELHPLTIPVG--SLGLGLNLDSNLQGR-FQS : 384
H_4  : FQTYSRCR-TGHPSLETIWSLHPLTIPASS--LALSLMPDS----QFSSK : 400
H_18 : FQTYMKCK-SGRPSLETIWELYPLPIPVG--SLSLSLNLDSDLQCR-YRS : 408
H_7  : FQTYMKCK-SGNPSLETVWGLHALTIPAGS--LRLSLITDSNKVGQ---- : 405
H_6  : FQTYSRCR-TGHPSLETIWSLHPLTIPASS--LALSLMPDSDLQSQFSSK : 380
H_15 : FQTYMRCK-AGHPSLETIWELHPLTIPVG--SLGLGLNLDSNLQGR-FQS : 405
H_39 : YQVYMKCRAAGQPPLEAVWELHPVVVPDGG--LALSLTCSEASQATHQ-- : 426
H_42 : RTVFMKCR-LGQPSLEAVWGIHPLVVPDGGS-LALSLSCTSEAQGSQQ-- : 413
H_14 : FQTYMRCK-AGHPSLETIWELHPLTIPVG--SLGLGLNLDSNLQGR-FQS : 406
H_3  : FQTYSRCR-TGHPSLETIWSLHPLTIPASS--LALSLMPDSDLQSQFSSK : 379
H_29 : YQTYMKCR-AGHPSLESMWGLQTLAVPAGS--LALSLTCAFDDSALG--- : 428
H_2  : SQTYVRCK-SGQPSLESLWCLTTLTIPATSNSLRLSLVSESELEVK---- : 407
H_41 : YQTNIRCR-TGHPSLETLWGLHTLAVPAGS--LALSLTCADADAAADDDD : 418


L    : ---KSENVSLQLQ---Q-SSDQLSFCEECSVKFES--------------- : 413
H_45 : -----AGTVVAP-------------------------------------- : 418
H_21 : KVFFKDVAFEDRAG----VRNHLTCCRDCTINFEK--------------- : 425
H_19 : KVFFKDVAFEDRTG----VRNHLTCCRDCLINFEK--------------- : 445
H_34 : ----TVNQSLKAG-SDADGNGPA-SCWPLLGGSQ-------------LSL : 507
H_25 : ----TVNQSMKAGSSDTDGNGPA-SCWPLLAGS---------------KL : 447
H_10 : KADNRTT-WLLLEGVGD-DHKQQPCFAEPSTKNET--------------- : 438
H_9  : KADNRTS-WLLLEGVGD-DQKQQACFAEPSTKNETI-------------- : 441
H_22 : KVTFKDESFEERAK----VRKYLTCCRDFSLNFEK--------------- : 426
H_17 : KVFFKDVAFEDRTG----VRNHLTCCRDCLINFEK--------------- : 420
H_16 : KVFFKDVAFEDRAG----VRNHLTCCRDCTINFEK--------------- : 414
H_20 : MVLFNDLTFEDKVG----VGKQLTCCRDCSIKFEN--------------- : 434
```

Figure 2 (continued)

```
H_27 : ----AVNQSMKASPHTTDGNRPAPSCGPLLGGS---------------HL : 460
H_38 : -----AAAPTAGWP-FVNGAGEAAAT------------------------ : 446
H_5  : KAGNGSSSWILHE--GG-EDKQLTCCADCSAKFES--------------- : 439
H_12 : KASLNGSSWPLLES---RVDNHLTCWTDYSVNFNK--------------- : 439
H_8  : KAENGCRSWIILE--GG-EDKQLTSCSNYSAKFET--------------- : 439
H_32 : ---INHLAKCDDDRSGNNGSAPR--CLSLLDAGGSG----------HLTA : 459
H_43 : -----QDRATRPWPSFVNGTAS-GESELIT-SSTKV-------------- : 481
H_30 : ----TVNQSMKAG-SDTDGNAPA-SCWPLLGGT---------------QL : 449
H_47 : -----AGTVVATAA------------------------------------ : 443
H_36 : -------RATRPWP-FANGTAATGDSELITLSCAKV-------------- : 442
H_37 : -------RATRPWP-FANGTAATGDSELITLSCAKV-------------- : 439
H_11 : KASGDGTSWSLLQS----GDKHLTCSTNCSDNFDK--------------- : 415
H_4  : KAGSGTSNWLMLE--GG-AEKQLTCCADCSANFEN--------------- : 432
H_18 : KVSTNGYGWPKLES---AVDNHSTCFTDFSVNFNR--------------- : 440
H_7  : ---DGSRCWIMLE--GE-EEKQLTCCVDCTSKFEN--------------- : 434
H_6  : KAGSGTSNWLMLE--GG-AEKQLTCCADCSANFEN--------------- : 412
H_15 : KASGDGTSWSLLQS----GDKHLTCSTNCSDNFDK--------------- : 436
H_39 : -----AAAPTAGWP-FVNGAGEAAAT------------------------ : 446
H_42 : -----AGRSITGWP-LVNGAATTGESELTW--CART-------------- : 441
H_14 : KASGDGTSWSLLQS----GDKHLTCSTNCSDNFDK--------------- : 437
H_3  : KAGSGTSNWLMLE--GG-AEKQLTCCADCSANFEN--------------- : 411
H_29 : ----AVNQSMKASPHTTDGNRPAPSCWPLLGGS---------------HL : 459
H_2  : ---KSENVSLQLQ---Q-SSDQLSFCEECSVKFES--------------- : 435
H_41 : SGAMAAAAVNHQSAKGANGSTSPSPCLSLLDAAAAGGACSSGQLAVMAAA : 468
```

```
L    : ---------EARFLKSS---------NSNVTTVA-LPAWLQQYKKENQ-- : 442
H_45 : ---------------------------------ASVPSWIRRCQGPVVVT : 435
H_21 : ---------EAQSITS--------TISKKACTTSSLPTWLQNCK--EERS : 456
H_19 : ---------EAQSITN--------CISKKVCTASSLPTWLQNCK--EERS : 476
H_34 : SSRCCGDGDCSAARIDT----KAALPRPSVSSSN-LPSWLQHCRDHQEPT : 552
H_25 : ISRCCGG-DCSAARIETT---KAALPRTPFVSSSSLPSWLQHCRDHQEPA : 493
H_10 : --------TEVRSLQSSS------TCNSDSSS--TLPAWLQQYKNENK-G : 471
H_9  : --------TEVRSLQSSS------TCNSDSSSS-TLPAWLQQYKNENK-G : 475
H_22 : ---------EAKSTTN------SITISKRDCT-TNLPTWLQNCK--EERS : 458
H_17 : ---------EAQSITN--------CISKKVCTASSLPTWLQNCK--EERS : 451
H_16 : ---------EAQSITS--------TISKKACTTSSLPTWLQNCK--EERS : 445
H_20 : ---------EALSLTN--------NISKKACS-SSLPTWLQNCK--EERS : 464
H_27 : LSRCCGG-DCSAATTTHEHDTKASLPRSFVSSSS-LPSWLQHCRDQQLQE : 508
H_38 : ------------------------------TASPTIPPWLRRYQDPDHAT : 466
H_5  : ---------EARSLPTS-------TCDSDSTTSG-LPAWLQQCKNEKN-L : 471
H_12 : ---------EAQSLVGRTHNKE-STSSVTISNNSSLPLWLQQCK-ETERN : 478
H_8  : ---------EARRLPNS-------TCNSDSTST--LPAWLQKYKNEKK-V : 470
H_32 : VS-SFFADDCSATATKCEP-----------ALKSSIPPWLQHCRD-QDPS : 496
H_43 : -----------------------------AATTPSVPPWFRGYQVQDMTR : 502
H_30 : ISRCCA--DCSAARIDT----KAALPRPFVSSSSTLPSWLQHCRDHQEPT : 493
H_47 : --------------------------------AASIPAWIRRCQGP-ALT : 460
H_36 : -----------------------------AATTPNVPPWLQGYQVQDTTR : 463
H_37 : -----------------------------AATTPNVPPWLQGYQVQDTTR : 460
H_11 : ---------ESQSIACSFRNGE-STTTITTSTSSSLPSWLQKEK----RR : 451
H_4  : ---------EARSIPTS-------TCNSDSTTST-LPTWLQQYKDENK-K : 464
H_18 : ---------DAQSIG--CSQRE-FTTNFTVSTSSSLPSWLKQHKVETERI : 478
H_7  : ---------EARSLQSS-------TSNSDSTTTSTLPAWLQQYKNENQGV : 468
H_6  : ---------EARSIPTS-------TCNSDSTTST-LPTWLQQYKDENK-K : 444
H_15 : ---------ESQSIACSFRNGE-STTTITTSTSSSLPSWLQKEK----RR : 472
H_39 : ------------------------------TASPTIPPWLRRYQDPDHAT : 466
H_42 : -----------------------------PSPEPNIPSWFRRYHDPYQTT : 462
H_14 : ---------ESQSIACSFRNGE-STTTITTSTSSSLPSWLQKEK----RR : 473
H_3  : ---------EARSIPTS-------TCNSDSTTST-LPTWLQQYKDENK-K : 443
```

Figure 2 (continued)

```
H_29 : LSRCCGG-DCSAATTTHEHDTKASLPRSFVSSSS-LPSWLQHCRDQQLQE : 507
H_2  : ---------EARFLKSS---------NSNVTTVA-LPAWLQQYKKENQ-- : 464
H_41 : VSGAYCGGDCAAATKALLPQSVVFMPPSATTTTTTIPPWLHHCRD-QEPA : 517

L    : --NSHTDSDSIKELVVKWNSICDSIHKR--PSLKT--LTLSSPTSS---F : 483
H_45 : GS-----------------------------ALTLSFSSPAS-----SS : 450
H_21 : DIMEDQENARLKDLCKKWNSLCNSIHR-HPSINEK-QVFFVSSSPSSPTS : 504
H_19 : DIMEDQESSRLEYLCKKWNSLCNSIHRRHPSIIEKPAVFFVSSSPSSPTS : 526
H_34 : TSHLTD---LS--KSKTWSSICS--RPSQR-MTLHFS-APVSP----ASS : 589
H_25 : THLTDD---LG----KTWSSICSSSRPSQRTTTLHFS-APVSP----ASS : 531
H_10 : ITHNDQNCVPVGELCKKWNSMCSSIQKQPYPSDKT--LSLSSVSPSSSNS : 519
H_9  : INYNDQNSVPVGELCKKWKFMCSSIQKQPYPSDKT--ITLSSVSPSSSTS : 523
H_22 : RIMENQENAKLRDICKKWNSFCSSAHG-FPSNPEK-QFFFISSSPSSPTS : 506
H_17 : DIMEDQESSRLEYLCKKWNSLCNSIHRRHPSIIEKPAVFFVSSSPSSPTS : 501
H_16 : DIMEDQENARLKDLCKKWNSLCNSIHR-HPSINEK-QVFFVSSSPSSPTS : 493
H_20 : YTVEDQENARLKDLCKKWNSICNSIHR-QPSILDKQDLFVLSSSPSSPTS : 513
H_27 : STHFAD---LG----KTWGSICG--KPSQR-MTLHFS-APVSP----ASS : 543
H_38 : PASCGT---GL-QIQDLWNPMRNGSAP-HHTSELTLSFSSPSP--SSISG : 509
H_5  : QNSDNQNSMSIKDLCRKWNSFCSSIHRQHYFSEKT--LTFSSVSPS---S : 516
H_12 : TTNDQEYLCN-----KGISLFGS--VHKQSYYPEK--TIKFASSPPSPNS : 519
H_8  : QNSDNQDSMPIKDLCRKWNSFCGSIHQQNYSSEET--LTFSSVSPS---S : 515
H_32 : RCK------------KSSTCGGS--LSHHHRTALNFSTAVVSP----SSS : 528
H_43 : PASRSA---SF-QLQD-WNHN-FNGSPAYHTSELTLSFSSQATNSPDASS : 546
H_30 : TTHLTD---LG----KTWSSICS--RPSSQRMTLHFS-APVSP----ASS : 529
H_47 : GS-----------------------------ELTLSFSSPAS-----SS : 475
H_36 : LASRST---SF-QMQD-WDPN-SNG-AAYHTSEITLSFSPQATNSPDASS : 506
H_37 : LASRST---SF-QMQD-WDPN-SNG-AAYHTSEITLSFSPQATNSPDASS : 503
H_11 : KIMDDQECVQVRDLCNKWNSFCSSVHK-KAHSTEK--ALNFSSPSPSSTS : 498
H_4  : LSRNDQDCVAVRDLCKKWNSICSSAHKQPHSSEKT--LTFSSLSPSSSTS : 512
H_18 : TIDDKEYCTNTSPLLKKWNSFGSSFHNKESHSPPK--TIKFASSPASPIS : 526
H_7  : NNNNDQDCVSIKDLCKKWNSICSSIHQKPYSSEKT--ITFSSVSPSSFTS : 516
H_6  : LSRNDQDCVAVRDLCKKWNSICSSAHKQPHSSEKT--LTFSSLSPSSSTS : 492
H_15 : KIMDDQECVQVRDLCNKWNSFCSSVHK-KAHSTEK--ALNFSSPSPSSTS : 519
H_39 : PASCGT---GL-QIQDLWNPMRNGSAP-HHTSELTLSFSSPSP--SSISG : 509
H_42 : PTSSST---SLHQLQDLWNPVRNWSATHHHTSELTLSFSSPAS--PAASS : 507
H_14 : KIMDDQECVQVRDLCNKWNSFCSSVHK-KAHSTEK--ALNFSSPSPSSTS : 520
H_3  : LSRNDQDCVAVRDLCKKWNSICSSAHKQPHSSEKT--LTFSSLSPSSSTS : 491
H_29 : STHFAD---LG----KTWGSICG--KPSQR-MTLHFS-APVSP----ASS : 542
H_2  : --NSHTDSDSIKELVVKWNSICDSIHKR--PSLKT--LTLSSPTSS---F : 505
H_41 : AHM------------KKWMSAHG--GSPSRRTALNISSTAVSP----CSS : 549

L    : SGSTQPSIS--TLHHLQTNGDWPVIETNTHRHHSVVHETS---------- : 521
H_45 : HCGFTHHH----DDTNKSCVPWHDLMDQQQQKLLLNRRHDGP-------- : 488
H_21 : VSSHERKS---NFHHSHLN--WPIISESEKSPKECEL-YTET-GDD---- : 543
H_19 : VSSNERKS---NFHHSHLN--WPIISESEKSPKECEL-YTET-GDDDDD- : 568
H_34 : ISSYEHGGD--QSSQTPPQHSWLLAGLDAA----HHRWEPKRETSG---- : 629
H_25 : ISSYEHGGG---QSQQPRHS-WLLAGLDAA----HHPWRPKREAS----- : 568
H_10 : NFSYEQQHP--NLLQTHHE--WQVGEPPKDSLNNYHFWISNNGTNNN--- : 562
H_9  : NFSYGQQHP--NLHQTHNE--WQVAEPPKDSLNNHHFWISNNGSNN---- : 565
H_22 : VSSHERKL---SLNLKHLN--WPLISEPKQVPKECEL-YTETTVSDD--- : 547
H_17 : VSSNERKS---NFHHSHLN--WPIISESEKSPKECEL-YTET-GDDDDD- : 543
H_16 : VSSHERKS---NFHHSHLN--WPIISESEKSPKECEL-YTET-GDD---- : 532
H_20 : FSSLEKKS---NFQHSQLN--WPIISEQEKVPKECELLYTESAGGDDDG- : 557
H_27 : ISSYEHGHG-HQQQQHQPHHSWLLADLDAK-----HPWKPKREDDD---- : 583
H_38 : YTSCYNN------NNMMSSKPWQ--LEARQPWP--IHGHEG----Q---- : 541
H_5  : STSYDQQYP--IFQQTHNE--WPIVE-PKH-------------------- : 541
```

Figure 2 (continued)

```
H_12 : FSSQERNT---DPQQTHLS--WPVIFEHKQFEKENQIWISECSN-E---- : 559
H_8  : STSYDHQYP--NLYRNQNE--WPIVE-PQQSSRDNHFWIGTEAINKC--- : 557
H_32 : VSSYEQHYH----LHQPSYQPWVVVADAHEDNHPSNKARCAAAAVQ---- : 570
H_43 : ISSSFAPSF-SAASLMMSSEPWQ--FKVMQPCP--NYRRDDPN------- : 584
H_30 : ISSYEHGGDHHHQSQQPRHSSWLLAGLDAAAPAHHHPWRPKREASG---- : 575
H_47 : HCGFTHY------DTNKSCEPWHDLMDRRQP--LLNRRHDGP-------- : 509
H_36 : ISGSFAP------SLMMSSEPWQ--FKLMQPCP--NYGRGDP-------- : 538
H_37 : ISGSFAP------SLMMSSEPWQ--FKLMQPCP--NYGRGDP-------- : 535
H_11 : ISSYDQCSP--NLQQNHLS--WPAIIEPKPPLKEHQFWISENVDEG---- : 540
H_4  : GFSYDQQYP--NLHQTHQG--WPVVE-HKQSWRDNHFWVSEALNKTY--- : 554
H_18 : ISSHECNT---NINQAPLS--WPVIFEPRQFQKEQKIWLSECNNAE---- : 567
H_7  : SFSYDHQYP--NFHHTYHQRDWPVVE-SKQSWRDHHFWVGSETVNKINSC : 563
H_6  : GFSYDQQYP--NLHQTHQG--WPVVE-HKQSWRDNHFWVSEALNKTY--- : 534
H_15 : ISSYDQCSP--NLQQNHLS--WPAIIEPKPPLKEHQFWISENVDEG---- : 561
H_39 : YTSCYNN------NNMMSSKPWQ--LEARQPWP--IHGHEG----Q---- : 541
H_42 : LSGYNNTP-----TTMSSSKPCQ--PEPRQPWPPSNQGHEDGPATR---- : 546
H_14 : ISSYDQCSP--NLQQNHLS--WPAIIEPKPPLKEHQFWISENVDEG---- : 562
H_3  : GFSYDQQYP--NLHQTHQG--WPVVE-HKQSWRDNHFWVSEALNKTY--- : 533
H_29 : ISSYEHGHG-HQQQQHQPHHSWLLADLDAK-----HPWKPKREDDD---- : 582
H_2  : SGSTQPSIS--TLHHLQTNGDWPVIETNTHRHHSVVHETS---------- : 543
H_41 : VSSYEQYTR----LHQP-YQPWLVADDDDEAEETKHPYIAGDGGAG---- : 590
```

```
L    : -----HLRLFIPEHDSEQ----KTELVCSNPNSTMNSEASSSDAMELEHA : 562
H_45 : --------LAEPY----------DQLS-PANPNSGSSNNSVSRSNSSDGA : 519
H_21 : -GYDSNFIMFMPDSDVPK------PDLLSNPNSSPN-SASSSEAVDGLES : 585
H_19 : -GYDSNFIMFMPDRDVPK------PDLLSNPNSSPN-SASSSEAVDGLES : 610
H_34 : -------NK------------------AASRSHD--SGASNGGSVEVECR : 652
H_25 : ----------------------------IRSHD--SGASNG-SVEVECR : 586
H_10 : -TNEPTLRVYIPENNNKQ------PFSSPNPSSNPN-STSSSDIMEVEHV : 604
H_9  : -TNEPTLRVYIPENN-KQ------PFSSPNPSSNPN-STSSSDIMEVEHV : 606
H_22 : -SYEGNLIMFMPEKNIPK------PDLLSNPNSSPN-SASSSEAVEGLDS : 589
H_17 : -GYDSNFIMFMPDRDVPK------PDLLSNPNSSPN-SASSSEAVDGLES : 585
H_16 : -GYDSNFIMFMPDSDVPK------PDLLSNPNSSPN-SASSSEAVDGLES : 574
H_20 : -CYDGNLIMFMPQRNVPK------PDLLSNPNSSPN-SASSSEAVDGLES : 599
H_27 : -------DE-------------------KAKSHDDCSGASNG-SVEVECR : 606
H_38 : -------RMAMAS----------YHDHHPLDTNPSPESNSVSNSLD--GG : 572
H_5  : ------LRMYIPEHKDH---TKQLPFSS-NPNSTPN-STSSSDVMEVVYL : 580
H_12 : -GYESSLRN------VPK------PDLLSNPNSSPN-SASSSEAMDDIEG : 595
H_8  : -SIEPSLRKYIPEHKDH---TKQLPFSS-NTNSTPN-SASSSDVMEMEHL : 601
H_32 : -------LHVVDDEDVKL-----LSAAIKVKSDD--SSASN--NSSVECR : 604
H_43 : -------ALVRTY----------DHQQLHGRP-SPEKSYSVSNSSE--GA : 614
H_30 : -------GNKA----------------AASRSHD--SGGSNG-SVEVECR : 599
H_47 : --------MAEPY----------DQLWLAANPNSGSSNSVSNKSNSSDGT : 541
H_36 : --------FARSS----------DRQQLLPRPTSPEKSYSVSNSSE--GA : 568
H_37 : --------FARSS----------DRQQLLPRPTSPEKSYSVSNSSE--GA : 565
H_11 : --LEPKFSMHIAERNFPI------PDLLSNPNSSPN-SASSSEAIEDGEG : 581
H_4  : ---EPSLRMYIPEHSDR-------KYAS-NPNSTPN-SASSSDVMEMEYV : 592
H_18 : -GSESNLIS------VTK------PELLSNPNSSPN-SASSSEAVDGTEG : 603
H_7  : ISIEPSLRMYIPEHNRDQYPKPTIPFSS-NPNSTPN-STSSSDVMEMEHL : 611
H_6  : ---EPSLRMYIPEHSDR-------KYAS-NPNSTPN-SASSSDVMEMEYV : 572
H_15 : --LEPKFSMHIAERNFPI------PDLLSNPNSSPN-SASSSEAIEDGEG : 602
H_39 : -------RMAMAS----------YHDHHPLDTNPSPESNSVSNSLD--GG : 572
H_42 : -------AEAIRD----------LPRWHSTEIAFPESFSAQSNSPNDHGG : 579
H_14 : --LEPKFSMHIAERNFPI------PDLLSNPNSSPN-SASSSEAIEDGEG : 603
H_3  : ---EPSLRMYIPEHSDR-------KYAS-NPNSTPN-SASSSDVMEMEYV : 571
H_29 : -------DE-------------------KAKSHDDCSGASNG-SVEVECR : 605
H_2  : -----HLRLFIPEHDSEQ----KTELVCSNPNSTMNSEASSSDAMELEHA : 584
H_41 : -------RLVP------------AAAKVVIKSDD--SSASNG-SVEVEWR : 618
```

Figure 2 (continued)

```
L     : SS--------RFKEMNAENLATLCAALESKVPWQKDLVP-ELAKTVLKCR : 603
H_45  : TT-ACRRR-PKFTELSAENLKILCRALETRVPRHRD-IAPGIASAVLQRR : 566
H_21  : TQ--------MFKEPNAENHKILCDALEKKVPQHKEVIP-EIASTVLHCR : 626
H_19  : TQ--------MFKEPNAENHKILCDALEKKIPQHKDVIVPEIASTVLHCR : 652
H_34  : ---------ARFKELSAENLKLLCGALEKEVPWQKE-IIPEVASAVLQCR : 692
H_25  : ---------ARFKELNAENLKLLCGALEKEVPWQKE-IVPEVASAVLQCR : 626
H_10  : R---------EFKELNTENLKTLCNALEKKVPWQKDIIP-EIASTLLQCR : 644
H_9   : S---------KFKELNSENLKTLCNALEKKLPWQKDIIP-EIASTLLQCR : 646
H_22  : TQ--------IFKEHNDENLKILCDALLKKVSQQKEIVK-EIASTVLLCR : 630
H_17  : TQ--------MFKEPNAENHKILCDALEKKIPQHKDVIVPEIASTVLHCR : 627
H_16  : TQ--------MFKEPNAENHKILCDALEKKVPQHKEVIP-EIASTVLHCR : 615
H_20  : TE--------LFNEHNEENLKILCDALENKFPQHKEIIQ-EIASTVLFCR : 640
H_27  : ---------SRFKELNAENLKLLCAALEKEVPWQKE-IVPEVASAVLQCR : 646
H_38  : -----ETRRPKFIELNAENLKILCNALESRVPQHSN-IVPDIASTVLQCR : 616
H_5   : H---------KFKELNAENLKILSIALEKKVPWQRDIIP-EIASTILQCR : 620
H_12  : VQ--------SFKEFNDYSLKNLRSGLEKKVPWQKDIIP-EIATTILECR : 636
H_8   : H---------KFKELNAENLKTLCNALEKKVPWQKDIIP-EIASTILQCR : 641
H_32  : ---------SRFKELSAENLKVLCSALEKEVPWQAE-IVPEIASTVLQCR : 644
H_43  : P-----AESPKFTELTAENLKILCNALENRAPHHKD-VATEIASVVLQCR : 658
H_30  : RA------KAKFKELSAENLKVLCGALEKEVPWQKE-IVPEIASAVLQCR : 642
H_47  : TT-TARRRRPKFTELTAENLKVLSSALETRVPRHRD-IAPGIASAVLQRR : 589
H_36  : PA----AESPKFTELTAENLKILSNALENRAPRHKD-VVTEIASVVLQCR : 613
H_37  : PA----AESPKFTELTAENLKILSNALENRAPRHKD-VVTEIASVVLQCR : 610
H_11  : LY--------GFKELNAENLRILCNALERRVPWQKDIIP-EIASTILECR : 622
H_4   : Q---------RFKELNAENLNTLCNALEKKVPWQKDIIP-DIASTILQCR : 632
H_18  : LQ--------SFKELNNQNLKILCSSLEKKVPWQKDIIP-EIATAILECR : 644
H_7   : N---------KFKEMNAENLKILCNALEKKVTWQKDIIP-DIASTILQCR : 651
H_6   : Q---------RFKELNAENLNTLCNALEKKVPWQKDIIP-DIASTILQCR : 612
H_15  : LY--------GFKELNAENLRILCNALERRVPWQKDIIP-EIASTILECR : 643
H_39  : -----ETRRPKFIELNAENLKILCNALESRVPQHSN-IVPDIASTVLQCR : 616
H_42  : GTADPERRRQKFTELTAENLKIMCGTLEDCVPGHIKDVAAGVASAVLQRR : 629
H_14  : LY--------GFKELNAENLRILCNALERRVPWQKDIIP-EIASTILECR : 644
H_3   : Q---------RFKELNAENLNTLCNALEKKVPWQKDIIP-DIASTILQCR : 611
H_29  : ---------SRFKELNAENLKLLCAALEKEVPWQKE-IVPEVASAVLQCR : 645
H_2   : SS--------RFKEMNAENLATLCAALESKVPWQKDLVP-ELAKTVLKCR : 625
H_41  : R--------PRFKEVSAENLKVLCGALEKEVPWQKV-IVPEIASTVLRCR : 659


L     : SGSSTR--KINGNE----DKKEDTWMFFQGLDVDAKEKIARELAKLVFGS : 647
H_45  : SGVTR-----------TARPTPATWLLFRGRDNDGKMSMARELARLVFGS : 605
H_21  : SGMRKRDQNHSMKR----EDNQETWMFFLGVNSQAKESISRELAKVVFGS : 672
H_19  : SGMRKRGLNHLMNR----EENQETWMFFLGVNSQAKESISRELAKVVFGS : 698
H_34  : SGIAKRRDKSRS-----ADAKEETWMLFLGGDADGKERVARELASLVFGS : 737
H_25  : SGIAKRRDKSRS-----ADAKEETWMLFLGGDADGKERVARELARLVFGL : 671
H_10  : SGMVRRKGKVMRNS---EEVKEETWLFFQGVDVEAKEKIARELARLVFGS : 691
H_9   : SGMVRRKGKVMINS---EEVKEETWLFFQGVDVEAKEKIARELARLVFGS : 693
H_22  : SGMR-EGVNHLVKR----DDRQEIWFFFLGLDSQAKEMVSKELAKVVFGS : 675
H_17  : SGMRKRGLNHLMNR----EENQETWMFFLGVNSQAKESISRELAKVVFGS : 673
H_16  : SGMRKRDQNHSMKR----EDNQETWMFFLGVNSQAKESISRELAKVVFGS : 661
H_20  : SGMRKRGNNFFKRE----NHKQETWMFFLGDDSQARENISKELAKVVFGS : 686
H_27  : SGIAKRRDRSRS-----TEAKEETWLFFLGGDGHGKERVARELAGLVFGS : 691
H_38  : SGMKKMKLRHKE----IIKASSTTWLLFQGRDVDGKKAMAQELAKLVFGS : 662
H_5   : SGMIRRKGKMK-NS----ESKEETWLFFQGVDVEAKEKIAKELARLVFGS : 665
H_12  : SGMRKRKGKLN-HI----EDKAETWLFFLGVDFEGKEKIARELAKLVFGS : 681
H_8   : SGMARRKGKVK-NS----VAKEETWLFFQGVDMEDKEKIAKELARLVFGS : 686
H_32  : SGMARRRDAAASSSRPPAFAKEDTWMLFHGGDADGKVRVARELARLVFGS : 694
```

Figure 2 (continued)

```
H_43 : SGMTR--RRWWF----QEKPSAVTWLLFQGGGNDGKKAVCQELARLVFGS : 702
H_30 : SGIAKRRDKSRS-----ADAKEETWMFFLGGDADGKEKVARELASLVFGS : 687
H_47 : SGVTR-----------TTRPTPATWLLFQGRDNDGKMAMARELARLVFGS : 628
H_36 : SGMTRKRRRWCC----QEKPSAVTWLLFQGAGNDGKKAVSKELARLVFGS : 659
H_37 : SGMTRKRRRWCC----QEKPSAVTWLLFQGAGNDGKKAVSKELARLVFGS : 656
H_11 : SGTLRGKNKLK-QR----EDKEETWLLFLGVDFQGKDKIAREIAKLVFGS : 667
H_4  : SGMVRRKGKVK-NS----ETKEETWFFFQGVDMDAKEKIARELARLVFGS : 677
H_18 : SGRSKSKRKSN-NR----AEREETWLFFLGVDSEGKEKIARELARLVYGS : 689
H_7  : SGMVRRKGKVTRNSS-TEQAKEETWLLFQGVDVEAKEKIAKELAKLIFGS : 700
H_6  : SGMVRRKGKVK-NS----ETKEETWFFFQGVDMDAKEKIARELARLVFGS : 657
H_15 : SGTLRGKNKLK-QR----EDKEETWLLFLGVDFQGKEKIAREIAKLVFGS : 688
H_39 : SGMKKMKLRHKE----IIKASSTTWLLFQGRDVDGKKAMAQELAKLVFGS : 662
H_42 : SGM----VRQRE----TPRPSSTTWLLFKGSDRDGKKSMALELAKLVFGS : 671
H_14 : SGTLRGKNKLK-QR----EDKEETWLLFLGVDFQGKDKIAREIAKLVFGS : 689
H_3  : SGMVRRKGKVK-NS----ETKEETWFFFQGVDMDAKEKIARELARLVFGS : 656
H_29 : SGIAKRRDRSRS-----TEAKEETWLFFLGGDAHGKERVARELAGLVFGS : 690
H_2  : SGSSTR--KINGNE----DKKEDTWMFFQGLDVDAKEKIARELAKLVFGS : 669
H_41 : SGMAAPAMARRSSSCS--SSKEHTWMLFLGGDADGKLRVARELASLVFGS : 707
```

```
L    : QDSFVSICLS--------SFSSTRSD---SAEDL-RNKRLRDEQS----- : 680
H_45 : HDDFTS-IAAAA------SKLAPDHSGSSSPG-KHSLKRQRSPPD----- : 642
H_21 : YSNFVTIGMS--------SFSSPEDDDDSTDEKS-KRKRPREELK----- : 708
H_19 : YSNFVSIGMS--------NFSSPEDDHDSTDEKS-KRKRPREELK----- : 734
H_34 : RDSFLAIRPG---------ASSSSPPRSGSSEGHRSNKRPRMSPPPPG-E : 777
H_25 : RSSFLSIRPGGVV-----SASSPPPASSGSSEGHRSSKRPRMPE-----E : 711
H_10 : QNDVVSIALS--------TFASTRAD---STEDYSRNKRSREETS----- : 725
H_9  : QNHVVSIALS--------TFASTRAD---STEDYSRNKRSREETS----- : 727
H_22 : YSNFVSIGIS--------SFSS-------THEES-KNKRPRDEFG----- : 704
H_17 : YSNFVSIGMS--------NFSSPEDDHDSTDEKS-KRKRPREELK----- : 709
H_16 : YSNFVTIGMS--------SFSSPEDDDDSTDEKS-KRKRPREELK----- : 697
H_20 : CNNFMTIGMS--------TFSSLGNDDSSSDEKS-KRKRPRAELG----- : 722
H_27 : RKSFLSVKLG---------ASSSSPSASGSTEDHHRSKRPRTTTT----S : 728
H_38 : STEFSSISFD--------ELT-SPYSDSSSGE--LTLKRQRSADS----- : 696
H_5  : NDSFISVSLS--------SFSSTRAD---STEDC-RNKRSRDEQS----- : 698
H_12 : QSNFVSIGLS--------NFSSSRAD---SIEES-KNKRARDELG----- : 714
H_8  : HESFISISLS--------SFSSTRAD---STEDC-RNKRTRDEQS----- : 719
H_32 : RKSFVSIAGSG-------TTASSPARSDDSSKQQRKRPRLMEEAS----- : 732
H_43 : YSKFTSISLADD------EFTHHVHSDSSSGE--PMLKRQRSLD------ : 738
H_30 : RNSFVSIRPGGGA-----SASSPPPPAAASSEEHHRSKRPRM-------- : 724
H_47 : YAEFTCCFAAAA------SKLAPDHSGSSSPGDRRSLKRQRSSPD----- : 667
H_36 : YSKFTAISLS--------EFT-HVHSDSSSGE--ITLKRQRSLD------ : 692
H_37 : YSKFTAISLS--------EFT-HVHSDSSSGE--ITLKRQRSLD------ : 689
H_11 : QSKFISIGLS--------SLGSTRAD---STEDF-LSKQARDEPV----- : 700
H_4  : QNNFVSIALS--------SFSSTRAD---STEDL-RNKRSRDEQS----- : 710
H_18 : QANFVSIGLS--------NYSSTRTD---STDES-KNKRGRDELG----- : 722
H_7  : QNNFISISLS--------SFSSTRAD---STEDC-RNKRSRDEQS----- : 733
H_6  : QNNFVSIALS--------SFSSTRAD---STEDL-RNKRSRDEQS----- : 690
H_15 : XSKFISIGLS--------SLGSTRAD---STEDF-LSKQARDEPV----- : 721
H_39 : STEFSSISFD--------ELT-SPYSDSSSGE--LTLKRQRSADS----- : 696
H_42 : YNDFTSISSA--------GCT-PVHSSSSSGE--RSGKRQRSPD------ : 704
H_14 : QSKFISIGLS--------SLGSTRAD---STEDF-LSKQARDEPV----- : 722
H_3  : QNNFVSIALS--------SFSSTRAD---STEDL-RNKRSRDEQS----- : 689
H_29 : RKSFLSVKLG---------ASSSSPSASGSTEDHHRSKRPRTTTR----S : 727
H_2  : QDSFVSICLS--------SFSSTRSD---SAEDL-RNKRLRDEQS----- : 702
H_41 : SKSFVSIGGAANASPPPSSSSSSPARSSGSTEQPHRSKRPWAETTTTTTS : 757
```

Figure 2 (continued)

```
L     : ---LSYIERFSEAVSLDPNRVILVEDIE-QADYLSQVGFKRAVERGRVCN : 726
H_45 : NEHGGFMQTFYEAIRENPHRVVLIDG-V-EHHSKLQAGIMGSMENGTVRG : 690
H_21 : ---SSYAQRFGEAVNENPHRVFFLEDLD-QVDYFSQKGVEQAIQSGSITL : 754
H_19 : ---SSYVQRFGEAVNENPHRVFFLEDLD-QVDYFSQKGVKQAIQSGSITL : 780
H_34 : ESAPACLERLHDAVSENPHRVIFMEGVE-QAGRDCQLGIKEAIESGVVRN : 826
H_25 : EPAAYYLERLHEAVSENPHRVIFMEDVE-RADRDCQLRIKEAIESGVVRN : 760
H_10 : ---CSYIERFAEAMACNPHRVFLVEDIE-QADYCSQLGFKRAIERGRVAD : 771
H_9   : ---CSYIERFVEAMASNPHRVFLVEDIE-QADYCSQLGFKRAIERGRVVD : 773
H_22 : ---GSYLQRFGEALNENPHRVFFLEDLE-QVDHFSKKGVKKGIESGTITL : 750
H_17 : ---SSYVQRFGEAVNENPHRVFFLEDLD-QVDYFSQKGVKQAIQSGSITL : 755
H_16 : ---SSYAQRFGEAVNENPHRVFFLEDLD-QVDYFSQKGVEQAIQSGSITL : 743
H_20 : ---STYLQRFCEAVNENPHRVFFMEDLEEEVDHFTQKGIKKAIECGSITI : 769
H_27 : SASEAYLERLYDAVSENPHRVILIEDVE-QGDHRWQVGVKEAIDRGVLRS : 777
H_38 : NEHS-FAQRLCEIVSKNPHQVIVINDIE-QLDQDSEISIKKAIANGRMRG : 744
H_5   : ---CSYIERFSEAASNNPRRVFLVEDVE-QADYCSQIGFKRAIESGRITN : 744
H_12 : ---CSYLERLGLALNENPHRVFFMEDVD-QVDNCSQKGIKQAIENGNVTL : 760
H_8   : ---CSYIERFSDAVSSNPHRVFLVEDVE-QADFFSQIRFKRAIEKGRITN : 765
H_32 : --DHGCHESLYEAVRDNPHRVILVQDVE-QGGWRCQRDILEAIQRGLVRS : 779
H_43 : TGHG-YIQRLYDAILKSPHRVIMIDGVE-QLDYESEIGIRNAITNGRIRG : 786
H_30 : --AAAYLERLHEAVSENPHRVIFMEDVE-RADRDCQLGIKEAIESGVVRN : 771
H_47 : NEHGGCMQMFYEAIRENPHRVVLVDGGV-EHDSELEVGIMDAMASGTVRG : 716
H_36 : TGRS-YVQRLYDAILENPHRVIMIDGVE-QLDYESEIGIRNAITNGRIRG : 740
H_37 : TGRS-YVQRLYDAILENPHRVIMIDGVE-QLDYESEIGIRNAITNGRIRG : 737
H_11 : ---GSYIEKFAEAVHENPHRVFFIEDVE-QLDYSSQMGVKRGIESGRIQI : 746
H_4   : ---CSYIERFAEAVGSNPHRVFLAEDVE-QADYCSQMGIKRATERGRITN : 756
H_18 : ---CGYHERFGLALNENPHRVFFMEDVE-QVDYCSQKAIKKAIESGKVAL : 768
H_7   : ---CSYIERFAEAVSSNPHRVFLVEDVE-QADYCSQVGFKRAIERGRITN : 779
H_6   : ---CSYIERFAEAVGSNPHRVFLAEDVE-QADYCSQMGIKRATERGRITN : 736
H_15 : ---GSYIEKFAEAVHENPHRVFFIEDVE-QLDYSSQMGVKRGIESGRIQI : 767
H_39 : NEHS-FAQRLCEIVSKNPHQVIVINDIE-QLDQDSEISIKKAIANGRMRG : 744
H_42 : YEHG-CAQRFYDTIHENPRRVVMIDDVE-QMSLGSEIGIKKAIASGRVRG : 752
H_14 : ---GSYIEKFAEAVHENPHRVFFIEDVE-QLDYSSQMGVKRGIESGRIQI : 768
H_3   : ---CSYIERFAEAVGSNPHRVFLAEDVE-QADYCSQMGIKRATERGRITN : 735
H_29 : SASEAYLERLYDAVSENPHRVILIEDVE-QGDHRWQVGVKEAIDRGVLRS : 776
H_2   : ---LSYIERFSEAVSLDPNRVILVEDIE-QADYLSQVGFKRAVERGRVCN : 748
H_41 : GRDQDHLEALYDAVRDNPRRVILMERVD-RADARCHDGIRDAIERGVVRS : 806
```

```
L     : SSGE--EASLKDAIVILS-CERFRSRSR---ACSPPSNQKSDG------- : 763
H_45 : CDGG--VVSLEDSIVV-CCEAF-ESRSLPRVSSSPRPVKQ-MITS-DVDS : 734
H_21 : PGGE--SVPLMDAIVIFS-CESFFSSPK---LRKSP------------CA : 786
H_19 : PSGE--SVPLKDAIVIFS-CES-FSSPK---LRKSP------------CA : 811
H_34 : RAGV--EVGVGDAIVVLS-CEGFGSTGRSRACSPPS-KKVKVEMEEAKEE : 872
H_25 : HAGQ--EVGVGDAIVILS-CESFGDSRSSRACSPPS-KKVKVEMEEAKEE : 806
H_10 : SKGE--EVALCDAIIILS-CESFSSRSR---ACSPSVKQKPLT-----EE : 810
H_9   : SKGE--EVALRDAIIILS-CESISSRSR---ACSPSVKQKSLT-----EV : 812
H_22 : PGGE--SVPLKDAIVIFS-SESFSSVPR---ACSPARTT-SPF-----SD : 788
H_17 : PSGE--SVPLKDAIVIFS-CES-FSSPK---LRKSP------------CA : 786
H_16 : PGGE--SVPLMDAIVIFS-CESFFSSPK---LRKSP------------CA : 775
H_20 : PGGE--SVPLKDAIVIFS-SESFSSVSK---SSQSS------------CA : 801
H_27 : QAGD--EVGVGDAIIILS-CESFEA--RSRAGSPLMNKKMKVEKEEAN-- : 820
H_38 : CTGE--EVDFEDAIIVLSYEEEFDSRSR---ASSSPRVKQRLMNNNDDEE : 789
H_5   : SNGQ--EVGLSDAIIILS-CESFSSRSR---ACSPPIKQRTDG---SYEE : 785
H_12 : PDGE--KVPLKDAIIIFS-CESFCSVSR---TCSPPRRQ-KT------GD : 797
H_8   : YNGQ--EVGLSDAIIILS-CESFSSRSR---ACSPPIKQRTDG---SHEE : 806
H_32 : HAGG-DEAALGDAIVVLS-CQSFDA--WSTTSSPPTTTKKAKTESEEE-- : 823
H_43 : CNGD--EISLGDAIIVLS-CEALDSMSN---ASSPR-LKQRVINKNGKEG : 829
H_30 : HAGE--EVGVGDAIVILS-CESFDGDSRSRGCSPPS-KKVKVEMEETKEE : 817
H_47 : CDGG--VVSLEDSIVVYCCEVFVESVSSPRVSS-PRPVKQRIITSGDVDS : 763
```

Figure 2 (continued)

```
H_36 : CNGD--EISLGDTIIVLN-CEALRSMSN---ASSPR-LKQRVIEKDGKEG : 783
H_37 : CNGD--EISLGDTIIVLN-CEALRSMSN---ASSPR-LKQRVIEKDGKEG : 780
H_11 : AGGE--AFSLEDAIIIFS-CESFSSVSR---ASSPPPMG-LK------SE : 783
H_4  : SNGE--EISLSDAIIILS-CESFSSRSR---ACSPPIKQKSDE----FEE : 796
H_18 : PGGE--NAPLKDAIIIFG-SESYSSASR---ACSPSRRV-KS------SG : 805
H_7  : VKGE--EVGLSDAIIILS-CESFSSRSR---ACSPPVKQKTDDYIISQDQ : 823
H_6  : SNGE--EISLSDAIIILS-CESFSSRSR---ACSPPIKQKSDE----FEE : 776
H_15 : AGGE--AFSLEDAIIIFS-CESFSSVSR---ASSPPPMG-LK------SE : 804
H_39 : CTGE--EVDFEDAIIVLSYEEEFDSRSR---ASSSPRVKQRLMNNNDDEE : 789
H_42 : CNGV--ETSLEDAIIVLS-CEAFDSRSR---ASSPRVIKQKVICD--EED : 794
H_14 : AGGE--AFSLEDAIIIFS-CESFSSVSR---ASSPPPMG-LK------SE : 805
H_3  : SNGE--EISLSDAIIILS-CESFSSRSR---ACSPPIKQKSDE----FEE : 775
H_29 : QAGD--EVGVGDAIIILS-CESFEA--RSRAGSPLMNKKMKVEKEEAN-- : 819
H_2  : SSGE--EASLKDAIVILS-CERFRSRSR---ACSPPSNQKSDG------- : 785
H_41 : RGGGGEEAFLGDAIVVLS-CESLNP--SSTTPAKKAKTEYSVEKLDQD-- : 851
```

```
L    : -----SDQPEDKNVATCVALDLNLSIDSAY----------VCEEESCDEI : 798
H_45 : KVEGDD--ADKG-VVPHFSLDLNTCAIDDGE-------GEEASSSWYDAM : 774
H_21 : ENKGKE-TVEDESS--SLSLDLNIAIED-----------ESGGVAFGGDN : 822
H_19 : ENKGKEITVDDESS--SLSLDLNLAIED-----------ESGGVALGGDN : 848
H_34 : RTGDIEHNGDGGSSSSPSCIDLNVDMESDQ----------ADEGSLDDDV : 912
H_25 : RAGDHEHNQDGVSKPSPSCIDLNVDMESDQ----------ADEPSLG-DQ : 845
H_10 : EKNGGDMVATLEVTSPCVSLDLNISIDDEN----------EVEDKSVDEI : 850
H_9  : EMNGDINNATLEETSPFVSLDLNISIDDEN----------NVEDRSEDEI : 852
H_22 : EDMEKNNINKSEEKTPCLSLDLNMAIEVD-V--------QKNVHLDGDTA : 829
H_17 : ENKGKEITVDDESS--SLSLDLNLAIED-----------ESGGVALGGDN : 823
H_16 : ENKGKE-TVEDESS--SLSLDLNIAIED-----------ESGGVAFGGDN : 811
H_20 : ENKGKETMIEDHQSNLNLSLDLNIAIED-----------HDN-----ADI : 835
H_27 : -TSDHDHKLEIESGAPSSCFDLNLDMESDQ----------AADELSSGDV : 859
H_38 : SSSTE----KGDNSPQRFSLDLNACLE-DEE-------EDEGFLLIDNGV : 827
H_5  : EDNAGAGAALMEDTTPCISLDLNISVDDDN----------ILEDQSIDDI : 825
H_12 : NHEDKEDEDVMEEKSLVLSLDLNISFGDNGD--------DQ---CSLAEY : 836
H_8  : EN--SAGATLMEGTSPCVSLDLNISIDDD-----------SVEDQSIDDI : 843
H_32 : -PTGEESAAAASPSSSP-CFDLNMDVENDD--LLESCFTDAS-------- : 861
H_43 : NGMNI----ENGMESSGFTLDLNACAE-DSE-------GN-EESVSDN-A : 865
H_30 : RTGEHEHNEDGASSSSPSCIDLNVDMESDP----------ADERSLG-DL : 856
H_47 : KVEDDG--AEKGVVVPRFSLDLNACAID-GE-------GEEGSSS-YNAM : 802
H_36 : NDMNM----ENGMESSGFTLDLNACAE-DCE-------GNYEESVSDN-A : 820
H_37 : NDMNM----ENGMESSGFTLDLNACAE-DCE-------GNYEESVSDN-A : 817
H_11 : ENEEKDRDNELEKRSPCVSLDLNLSAEDN----------QEYGQNSVADT : 823
H_4  : EK----GGGGGEEISPCVSLDLNICIDDD-----------GVEDESIDDI : 831
H_18 : EKEVKDEEDESDEKNKVLSLDLNIAIDVNDD--------DEDEYSNIADN : 847
H_7  : EEEKGQGAKMEE-SSPCVSLDLNISIDDD-----------SIEDRSIDDI : 861
H_6  : EK----GGGGGEEISPCVSLDLNICIDDD-----------GVEDESIDDI : 811
H_15 : ENEEKDRDNELEKRSPCVSLDLNLSAEDN----------QEYGQNSVADT : 844
H_39 : SSSTE----KGDNSPQRFSLDLNACLE-DEE-------EDEGFLLIDNGV : 827
H_42 : SGGVE----KGVTKPPCFSLDLNECASGDDE-------GHQEEEGLDSDV : 833
H_14 : ENEEKDRDNELEKRSPCVSLDLNLSAEDN----------QEYGQNSVADT : 845
H_3  : EK----GGGGGEEISPCVSLDLNICIDDD-----------GVEDESIDDI : 810
H_29 : -TSDHDHKLEIESGVPSSCFDLNLDMESDQ----------AADELSSGDV : 858
H_2  : -----SDQPEDKNVATCVALDLNLSIDSAY----------VCEEESCDEI : 820
H_41 : -GDDHHGKEAVAAAASPSCFDLNMSMDDDDEAAEERCTGEEEEAGHHHHQ : 900
```

```
L    : GLLEAVDARFHFKCSST------- : 815
H_45 : EIQNDVDGVFFF------------ : 786
H_21 : GILELVDKQINFNIQES------- : 839
```

Figure 2 (continued)

```
H_19 : GILELVDKQINFNIQEL------- : 865
H_34 : CLLTAVDRTLFFRRQG-------- : 928
H_25 : CLLTAVDRALFFRRQDN------- : 862
H_10 : GLLESVDKKLGMKEIACPSTLLCN : 874
H_9  : GLLESVDGKVIFNFEEL------- : 869
H_22 : EILELVDKQINFKI---------- : 843
H_17 : GILELVDKQINFNIQEL------- : 840
H_16 : GILELVDKQINFNIQES------- : 828
H_20 : GILELVDKKFSFNL---------- : 849
H_27 : CLLTAVDRVLLFRRQDEM------ : 877
H_38 : GMHDIVDGVFFFGLMADF------ : 845
H_5  : GLLESVDRRIIFKIQEF------- : 842
H_12 : GILESVDRQVVFKIQELS------ : 854
H_8  : GLLESVDRRIIFKIQDF------- : 860
H_32 : -LLKAVDRTFFFRRPDERSD---- : 880
H_43 : RIINIVDGVFFFQLMEHS------ : 883
H_30 : CLLTAVDRTLFFRRQQEN------ : 874
H_47 : EILNVVDGVFFF------------ : 814
H_36 : RIVNIVDGVFFFQLMEHS------ : 838
H_37 : RIVNIVDGVFFFQLMEHS------ : 835
H_11 : GVLDSVDRQFIFKIQEL------- : 840
H_4  : GLLESVDRRITFKIQEL------- : 848
H_18 : GILQSVDRQILFKIQEL------- : 864
H_7  : GLLESVDRRIVFKIQELRFMS--- : 882
H_6  : GLLESVDRRITFKIQEL------- : 828
H_15 : GVLDSVDRQXIFKIQEL------- : 861
H_39 : GMHDIVDGVFFFGLMADF------ : 845
H_42 : EICDVVDGVFFFRLPGDLSH---- : 853
H_14 : GVLDSVDRQFIFKIQEL------- : 862
H_3  : GLLESVDRRITFKIQEL------- : 827
H_29 : CLLTAVDRVLLFRRQDEM------ : 876
H_2  : GLLEAVDARFHFKCSST------- : 837
H_41 : LLLKAVDRVLFFRSIGE------- : 917
```

Figure 3

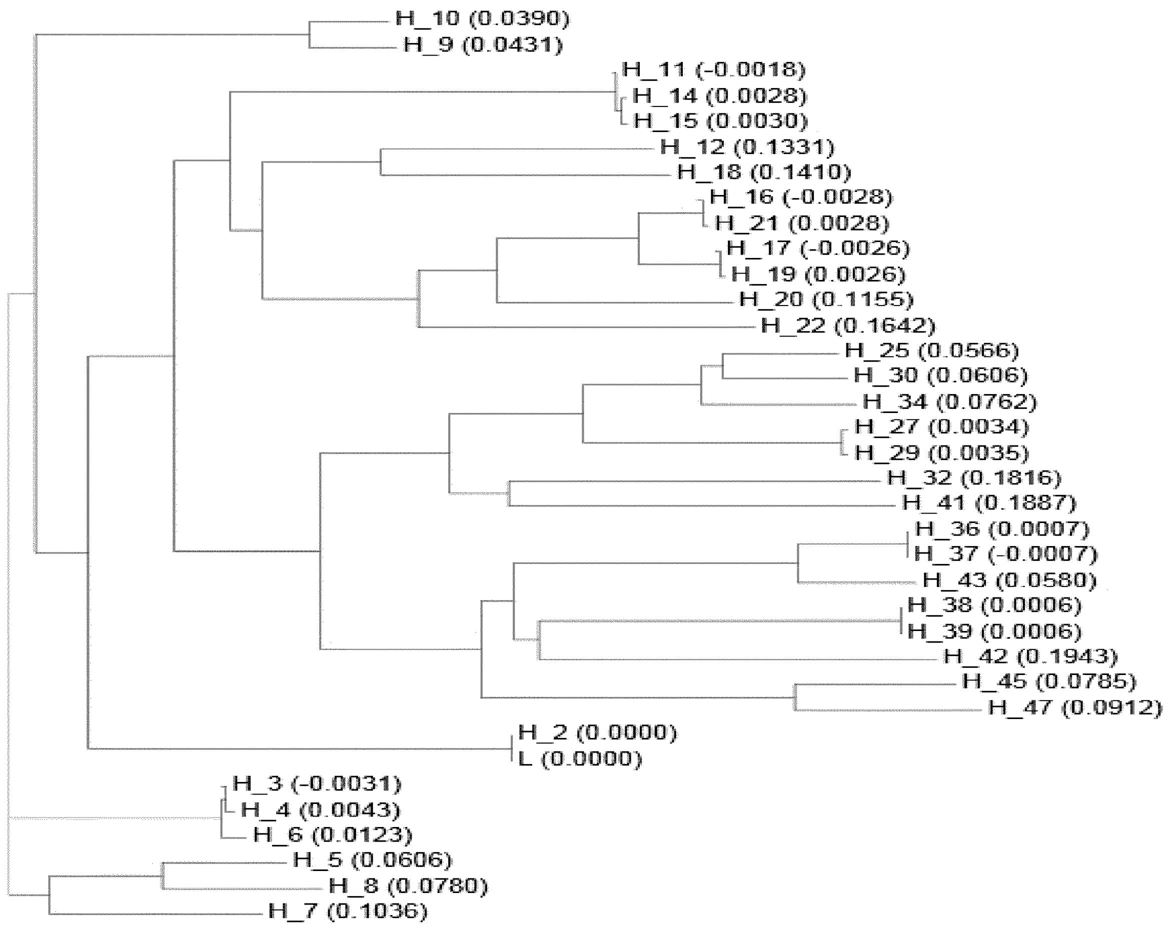
H_10 (0.0390)
H_9 (0.0431)
H_11 (-0.0018)
H_14 (0.0028)
H_15 (0.0030)
H_12 (0.1331)
H_18 (0.1410)
H_16 (-0.0028)
H_21 (0.0028)
H_17 (-0.0026)
H_19 (0.0026)
H_20 (0.1155)
H_22 (0.1642)
H_25 (0.0566)
H_30 (0.0606)
H_34 (0.0762)
H_27 (0.0034)
H_29 (0.0035)
H_32 (0.1816)
H_41 (0.1887)
H_36 (0.0007)
H_37 (-0.0007)
H_43 (0.0580)
H_38 (0.0006)
H_39 (0.0006)
H_42 (0.1943)
H_45 (0.0785)
H_47 (0.0912)
H_2 (0.0000)
L (0.0000)
H_3 (-0.0031)
H_4 (0.0043)
H_6 (0.0123)
H_5 (0.0606)
H_8 (0.0780)
H_7 (0.1036)

Figure 4

| | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 |
|---|---|---|---|---|---|---|---|---|---|---|---|---|---|---|---|---|---|---|---|---|---|---|---|---|
| **1. L** | | 36,6 | 45,7 | 46 | 40,4 | 42,5 | 55,3 | 55,3 | 44,8 | 47,2 | 47,4 | 46,4 | 42,1 | 40,3 | 59,5 | 49,6 | 57,9 | 40,9 | 38,5 | 42,2 | 35,2 | 39,5 | 39,5 | 50,1 |
| **2. H_45** | 56,4 | | 33,8 | 34,1 | 39,8 | 41,7 | 36,7 | 35,6 | 32,8 | 34,8 | 34,9 | 34 | 39,4 | 51,5 | 37,4 | 34 | 36,1 | 39,6 | 50,4 | 40,3 | 79 | 51,3 | 51,4 | 37 |
| **3. H_21** | 66,6 | 52,7 | | 90,1 | 37,2 | 42 | 47,7 | 47,4 | 66,7 | 87,7 | 95,7 | 74 | 41 | 38,3 | 50,5 | 57,8 | 48,5 | 40,6 | 38,8 | 41,1 | 33,6 | 38,7 | 38,7 | 54,3 |
| **4. H_19** | 65,2 | 51,9 | 93,1 | | 37,7 | 42,1 | 47,7 | 48,3 | 65,7 | 97,1 | 89 | 74,3 | 41,5 | 38,4 | 49,4 | 58,4 | 47,5 | 40,3 | 38,3 | 41,5 | 33,6 | 38,9 | 38,9 | 54,6 |
| **5. H_34** | 57 | 53,4 | 54,7 | 56,5 | | 77,5 | 40,8 | 39,9 | 36,1 | 37,9 | 38 | 38,2 | 65,6 | 40,2 | 42,1 | 40,7 | 40,9 | 53,7 | 39,6 | 76,3 | 37,9 | 40,3 | 40,2 | 39,9 |
| **6. H_25** | 60,8 | 55,6 | 60,2 | 61,8 | 82,5 | | 44,4 | 43,2 | 39,5 | 41,7 | 42,3 | 42,4 | 72 | 43,4 | 45,9 | 43,5 | 44,2 | 56,7 | 42,4 | 85,2 | 40,2 | 43,3 | 43,2 | 44,3 |
| **7. H_10** | 71,2 | 53,9 | 66,6 | 67,6 | 59,4 | 64 | | 90,3 | 46,5 | 48,1 | 48,8 | 47,8 | 44,2 | 41,6 | 63,5 | 52,6 | 62,8 | 42,2 | 40,1 | 44,6 | 35,4 | 40,2 | 40,2 | 53,5 |
| **8. H_9** | 71 | 54,7 | 67,1 | 68 | 58,8 | 63,5 | 93,8 | | 45,4 | 48,6 | 48,2 | 47,7 | 43,7 | 40,9 | 64,4 | 53,2 | 63,5 | 42,6 | 40 | 44,4 | 35,1 | 40 | 40,2 | 53,4 |
| **9. H_22** | 65,1 | 51,5 | 82,2 | 81,2 | 53,1 | 58,2 | 65,1 | 65,1 | | 66,9 | 68,6 | 64,7 | 39,4 | 35,9 | 48,4 | 54,3 | 47,8 | 37,1 | 35 | 39,5 | 31,1 | 36,2 | 36 | 54 |
| **10. H_17** | 66,8 | 53 | 93,2 | 97,1 | 55,6 | 61 | 67,3 | 67,8 | 82,4 | | 91,3 | 75,3 | 41,7 | 38 | 49,6 | 57,5 | 47,7 | 40,4 | 37,8 | 41,5 | 33,4 | 39,1 | 39,1 | 55,6 |
| **11. H_16** | 67,9 | 53,9 | 97,1 | 92 | 54,8 | 60,2 | 66,8 | 67,5 | 83 | 94,3 | | 76 | 41,9 | 39,1 | 50,7 | 57,9 | 49 | 41,4 | 39,2 | 42,3 | 34,5 | 39,5 | 39,5 | 56,2 |
| **12. H_20** | 66,5 | 51,8 | 85,6 | 85,8 | 55,6 | 61,9 | 65,8 | 66,6 | 81,5 | 86,7 | 86,6 | | 40,2 | 37,6 | 49,3 | 55,1 | 47,7 | 39,1 | 37,2 | 41,5 | 33,1 | 38,3 | 38,2 | 52,6 |
| **13. H_27** | 59,3 | 55,9 | 59,3 | 59,5 | 76,8 | 81,9 | 63,3 | 63,4 | 56,9 | 58,6 | 59,5 | 59,9 | | 43,1 | 45,6 | 42,7 | 44,7 | 56,6 | 42,7 | 71,1 | 37,9 | 43,5 | 43,4 | 44,2 |
| **14. H_38** | 58,8 | 65,4 | 58 | 57,6 | 56,7 | 61,5 | 59,7 | 59,6 | 55,9 | 57,5 | 57,9 | 56,9 | 59,6 | | 41,6 | 38,5 | 41,6 | 41,8 | 62,5 | 43,2 | 52,8 | 60,5 | 60,4 | 41,7 |

Figur 4 (continued)

| | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 |
|---|---|---|---|---|---|---|---|---|---|---|---|---|---|---|---|---|---|---|---|---|---|---|---|---|
| 15. H_5 | 75,2 | 55,7 | 69,1 | 68,7 | 60,7 | 65,5 | 78,7 | 79,2 | 67,1 | 69,7 | 69,2 | 68,6 | 64,4 | 62,8 | | 55,4 | 83,4 | 41,4 | 41,1 | 45,6 | 36,8 | 41,8 | 41,8 | 55,9 |
| 16. H_12 | 67,2 | 52,6 | 74,4 | 75,3 | 58,6 | 63 | 69,7 | 70,1 | 72,4 | 74,7 | 74,1 | 74,2 | 62,3 | 58,9 | 73,8 | | 53,5 | 40,7 | 38,6 | 43,9 | 34,1 | 38,9 | 38,8 | 60,4 |
| 17. H_8 | 73,6 | 54,3 | 68,5 | 68,6 | 60,1 | 63,8 | 78,3 | 79,3 | 67,2 | 68 | 68,7 | 66,5 | 64,2 | 60,8 | 90,3 | 72,1 | | 41,6 | 40,6 | 44,2 | 36,2 | 41,3 | 41,4 | 54,8 |
| 18. H_32 | 59,3 | 55,5 | 59,7 | 59,9 | 66,6 | 70,3 | 62 | 61,6 | 55,3 | 59,2 | 59,8 | 58,6 | 71,1 | 58 | 63,3 | 61,4 | 63,3 | | 41,2 | 57,4 | 39,2 | 41,9 | 42,2 | 41,1 |
| 19. H_43 | 55,2 | 61,9 | 57,2 | 58,3 | 58 | 60,5 | 59,1 | 58,6 | 56,2 | 57,4 | 57,4 | 56,3 | 60,6 | 73,8 | 58,7 | 58,4 | 59,5 | 59,5 | | 41,9 | 52,2 | 82,6 | 82,3 | 39,6 |
| 20. H_30 | 60 | 55,9 | 59,4 | 61,2 | 83,4 | 90,2 | 64,3 | 63,2 | 57,1 | 60,6 | 60,2 | 60,5 | 83,2 | 60,3 | 65,4 | 62,1 | 64,5 | 71,5 | 60,7 | | 38,7 | 42,1 | 42,1 | 44 |
| 21. H_47 | 55,2 | 85,9 | 53,5 | 51,2 | 53,3 | 56 | 53,2 | 53,9 | 51,7 | 52 | 54,8 | 52,2 | 56,1 | 67,1 | 56,3 | 53,9 | 53,6 | 54,8 | 65,2 | 56,3 | | 52,3 | 52,4 | 36,5 |
| 22. H_36 | 58,6 | 64,6 | 58,9 | 58,5 | 56,1 | 60 | 58,7 | 58,5 | 57,2 | 59,6 | 58,8 | 59,5 | 60,7 | 74 | 62 | 59,4 | 60,3 | 57,8 | 87,4 | 58,8 | 67,5 | | 99,6 | 40,7 |
| 23. H_37 | 58,9 | 64,8 | 58,8 | 58,4 | 56 | 59,7 | 58,8 | 58,8 | 57,4 | 59,5 | 58,9 | 59,6 | 60,7 | 73,8 | 62,4 | 58,9 | 60,3 | 58,1 | 87,2 | 58,8 | 67,7 | 99,6 | | 41 |
| 24. H_11 | 68,7 | 55,4 | 74,5 | 73,3 | 59,5 | 64,2 | 71,4 | 71 | 74,7 | 74,6 | 75,2 | 72,7 | 63,3 | 60,9 | 75,5 | 77,8 | 73,8 | 60,6 | 58,8 | 63 | 56,7 | 62,4 | 62,3 | |
| 25. H_4 | 76,3 | 56,6 | 71,3 | 70,8 | 61,4 | 66,5 | 80,8 | 79,9 | 69,6 | 71,1 | 71,3 | 71,4 | 64,2 | 63,1 | 86,4 | 75,3 | 84,3 | 63,3 | 60,6 | 65,9 | 54,8 | 62,5 | 62,3 | 78,2 |
| 26. H_18 | 66,1 | 53,7 | 73,8 | 75,4 | 58,6 | 64,1 | 68,2 | 68,5 | 72,1 | 74 | 73,7 | 73,1 | 61,9 | 58,2 | 70 | 83,3 | 70,1 | 61,9 | 57,1 | 63,2 | 53,5 | 59,5 | 59,5 | 76,3 |
| 27. H_7 | 72,6 | 53,3 | 69,2 | 69 | 60,8 | 63,9 | 81,5 | 81,4 | 67 | 68,4 | 69,5 | 67,7 | 64,4 | 60,7 | 85,4 | 71,3 | 84,6 | 62,7 | 59,5 | 64,7 | 52 | 58,5 | 58,6 | 73,8 |
| 28. H_6 | 74,4 | 56,4 | 69,7 | 68,3 | 59,8 | 64,7 | 78 | 77,1 | 67,4 | 69,5 | 70,7 | 68,8 | 62,8 | 60,8 | 84 | 73 | 81,4 | 61,3 | 59,3 | 64,1 | 54,7 | 61,6 | 61,3 | 76,2 |

Figur 4 (continued)

| | **1** | **2** | **3** | **4** | **5** | **6** | **7** | **8** | **9** | **10** | **11** | **12** | **13** | **14** | **15** | **16** | **17** | **18** | **19** | **20** | **21** | **22** | **23** | **24** |
|---|---|---|---|---|---|---|---|---|---|---|---|---|---|---|---|---|---|---|---|---|---|---|---|---|
| **29. H_15** | 66,7 | 54,1 | 73,5 | 74,3 | 59,3 | 64,6 | 71,5 | 71,3 | 73,1 | 73,4 | 73,3 | 71,7 | 63,4 | 60,4 | 74,6 | 77,7 | 74,1 | 60,8 | 59,1 | 63,2 | 54,9 | 61,2 | 61,1 | 97 |
| **30. H_39** | 58,8 | 65,4 | 58 | 57,6 | 56,7 | 61,5 | 59,7 | 59,6 | 55,9 | 57,5 | 57,9 | 56,9 | 59,6 | 99,9 | 62,6 | 59 | 60,5 | 58,1 | 73,6 | 60,3 | 67 | 74 | 73,8 | 60,7 |
| **31. H_42** | 56,4 | 63,7 | 55,1 | 55,5 | 57,7 | 60,7 | 58,1 | 57,3 | 53,6 | 55,8 | 55,2 | 56 | 58,2 | 72,8 | 60,5 | 58,4 | 59,7 | 56,5 | 68,9 | 60,2 | 64,6 | 70,6 | 70,3 | 57,6 |
| **32. H_14** | 66,9 | 54,2 | 73,7 | 74,6 | 59,7 | 65,1 | 71,9 | 71,6 | 73,2 | 73,5 | 73,4 | 71,9 | 63,9 | 60,6 | 74,6 | 78 | 74,2 | 61,3 | 59,3 | 63,6 | 55,2 | 61,4 | 61,3 | 97,4 |
| **33. H_3** | 77,9 | 57,3 | 70,6 | 69,2 | 61 | 65,7 | 80,8 | 79,7 | 70 | 71,3 | 72,1 | 70,7 | 63,6 | 62,8 | 85,6 | 74,4 | 83 | 62,5 | 60,6 | 65,1 | 56,1 | 63 | 62,8 | 79 |
| **34. H_29** | 59,4 | 55,9 | 59,7 | 59,8 | 76,9 | 82,2 | 63,4 | 63,8 | 57,2 | 58,9 | 59,9 | 60 | 99,3 | 59,9 | 64,5 | 62,3 | 63,9 | 71,1 | 60,7 | 83,7 | 56,3 | 61,1 | 61 | 63,6 |
| **35. H_2** | 97,4 | 55,1 | 66,3 | 65,2 | 57,4 | 60,8 | 71,2 | 71,1 | 65,6 | 66,7 | 67 | 66 | 59,7 | 58,8 | 75,2 | 67,1 | 73,6 | 59,3 | 55 | 60,3 | 53,9 | 58,7 | 58,9 | 68,8 |
| **36. H_41** | 55,6 | 52,3 | 55,8 | 56,1 | 68 | 68,6 | 56,9 | 58,2 | 54,2 | 55 | 56,2 | 55,6 | 69,9 | 56,8 | 60,2 | 57,6 | 56,4 | 71,1 | 57,8 | 70,1 | 53,3 | 56,4 | 56,1 | 57,1 |

Figur 4 (continued)

| | 25 | 26 | 27 | 28 | 29 | 30 | 31 | 32 | 33 | 34 | 35 | 36 |
|---|---|---|---|---|---|---|---|---|---|---|---|---|
| **1. L** | 62 | 47,5 | 58,3 | 60,1 | 48,7 | 40,3 | 38,7 | 48,8 | 63 | 42 | 97,4 | 39,7 |
| **2. H_45** | 37,9 | 35,8 | 34,8 | 36,6 | 36,1 | 51,5 | 51 | 36,4 | 38,2 | 39,3 | 35,8 | 38 |
| **3. H_21** | 53,7 | 57,1 | 49,8 | 51,6 | 54,7 | 38,3 | 35,5 | 54,7 | 52,8 | 41,2 | 44,5 | 38,2 |
| **4. H_19** | 54 | 57,4 | 49,2 | 51,6 | 55,4 | 38,4 | 35,9 | 55,4 | 53,2 | 41,4 | 45,1 | 37,8 |
| **5. H_34** | 43,9 | 40,7 | 40,2 | 43,2 | 40 | 40,2 | 41,3 | 40 | 43,7 | 65,8 | 39,9 | 52,3 |
| **6. H_25** | 47,3 | 44,2 | 42,9 | 46,5 | 44,1 | 43,4 | 43,9 | 44,1 | 47 | 72,1 | 41,9 | 56,7 |
| **7. H_10** | 67,6 | 50,9 | 65,3 | 65,1 | 52,9 | 41,6 | 40,6 | 52,9 | 68,1 | 44,1 | 54,1 | 39,4 |
| **8. H_9** | 67,5 | 51,3 | 65,1 | 65,1 | 53,4 | 40,9 | 38,4 | 53,4 | 68 | 44 | 54 | 39,1 |
| **9. H_22** | 51,8 | 53,6 | 48,1 | 50,3 | 53,2 | 35,9 | 35,1 | 53,1 | 52,8 | 39,4 | 43,7 | 36,2 |
| **10. H_17** | 54,2 | 56,8 | 49,3 | 51,7 | 54,8 | 38 | 36,8 | 54,8 | 54,8 | 41,6 | 46,1 | 38,2 |
| **11. H_16** | 54,1 | 56,8 | 50,4 | 52 | 54,9 | 39,1 | 36,5 | 54,9 | 54,8 | 42 | 46,4 | 38,9 |
| **12. H_20** | 53,1 | 54,4 | 49,4 | 50,5 | 52 | 37,6 | 36,4 | 52,1 | 52,6 | 40,2 | 45,2 | 36,7 |
| **13. H_27** | 46,7 | 43,6 | 43,6 | 45,8 | 44,2 | 43,1 | 41,4 | 44,2 | 46,5 | 99,2 | 41,3 | 56,3 |
| **14. H_38** | 42,6 | 38,9 | 41,2 | 41,6 | 41,3 | 99,9 | 60,1 | 41,5 | 42,9 | 43,4 | 39,3 | 41,4 |

Figur 4 (continued)

| | 25 | 26 | 27 | 28 | 29 | 30 | 31 | 32 | 33 | 34 | 35 | 36 |
|---|---|---|---|---|---|---|---|---|---|---|---|---|
| 15. H_5 | 72,9 | 52,7 | 72,1 | 70,1 | 56,5 | 41,5 | 40,6 | 56,4 | 71,7 | 45,7 | 58 | 40,8 |
| 16. H_12 | 59,4 | 71,2 | 54,6 | 57,4 | 60,9 | 38,8 | 38,5 | 60,9 | 58,7 | 42,9 | 48,5 | 39,5 |
| 17. H_8 | 72,1 | 51,6 | 72 | 69,4 | 54,9 | 41,3 | 40,6 | 54,8 | 71,2 | 44,7 | 56,5 | 39,4 |
| 18. H_32 | 44,4 | 41,7 | 41,2 | 43,3 | 41 | 41,8 | 40,4 | 41,1 | 43,9 | 56,7 | 40 | 59 |
| 19. H_43 | 42 | 37,5 | 39,2 | 40,8 | 39,4 | 62,4 | 56,9 | 39,6 | 41,9 | 42,5 | 37,5 | 40,2 |
| 20. H_30 | 47,6 | 44,7 | 44,3 | 46,9 | 44 | 43,2 | 42,7 | 44 | 47,3 | 71,5 | 41,3 | 57 |
| 21. H_47 | 36,7 | 34,2 | 35 | 35,5 | 35,5 | 52,8 | 51,9 | 35,7 | 36,5 | 38 | 34,4 | 38,3 |
| 22. H_36 | 42,3 | 39 | 39,8 | 40,8 | 40,2 | 60,5 | 56,8 | 40,4 | 42,5 | 43,5 | 38,7 | 40,8 |
| 23. H_37 | 42,2 | 39,1 | 40 | 40,8 | 40,5 | 60,4 | 56,6 | 40,8 | 42,5 | 43,2 | 38,7 | 40,7 |
| 24. H_11 | 61,4 | 60,1 | 55,8 | 60,2 | 96,6 | 41,7 | 39,1 | 97,3 | 62,8 | 44,5 | 48,9 | 38,6 |
| 25. H_4 | | 57,4 | 73,1 | 94,8 | 62 | 42,7 | 42,9 | 62,1 | 96,5 | 46,6 | 60,7 | 42,5 |
| 26. H_18 | 72,6 | | 53,3 | 55,6 | 60,8 | 39,2 | 36,9 | 60,9 | 57,1 | 43,6 | 46,9 | 39 |
| 27. H_7 | 84,5 | 70 | | 70,3 | 56,2 | 41,1 | 40,2 | 56,2 | 72,1 | 43,7 | 57,1 | 38,5 |
| 28. H_6 | 95,8 | 70,6 | 81,2 | | 59,8 | 41,7 | 42,1 | 59,8 | 94,2 | 45,8 | 58,8 | 41,5 |

Figur 4 (continued)

| | 25 | 26 | 27 | 28 | 29 | 30 | 31 | 32 | 33 | 34 | 35 | 36 |
|---|---|---|---|---|---|---|---|---|---|---|---|---|
| **29. H_15** | 77,9 | 77,1 | 74,4 | 75 | | 41,1 | 38,6 | 99,3 | 61,1 | 44,1 | 47,5 | 38 |
| **30. H_39** | 63,2 | 58 | 60,5 | 60,9 | 60,2 | | 60,1 | 41,5 | 42,9 | 43,4 | 39,3 | 41,3 |
| **31. H_42** | 61,2 | 56,8 | 57,8 | 59,4 | 57,3 | 72,8 | | 38,6 | 43,4 | 41,7 | 38 | 39,5 |
| **32. H_14** | 78,2 | 77,4 | 74,6 | 75,2 | 99,4 | 60,6 | 57,2 | | 61,2 | 44,1 | 47,7 | 38,1 |
| **33. H_3** | 97,1 | 72 | 82,9 | 95,8 | 76,8 | 62,8 | 60,8 | 77 | | 46,5 | 61,7 | 42,5 |
| **34. H_29** | 64,4 | 62,1 | 64,5 | 63,2 | 63,5 | 59,9 | 58,3 | 63,9 | 64 | | 41,3 | 56,2 |
| **35. H_2** | 76,4 | 66,4 | 72,6 | 74 | 66,7 | 58,8 | 56,6 | 67,1 | 77,1 | 59,8 | | 38,6 |
| **36. H_41** | 59,4 | 58,5 | 59,4 | 57,7 | 56,8 | 56,7 | 54,4 | 57,1 | 58,9 | 69,7 | 55,3 | |

FIGURE 5

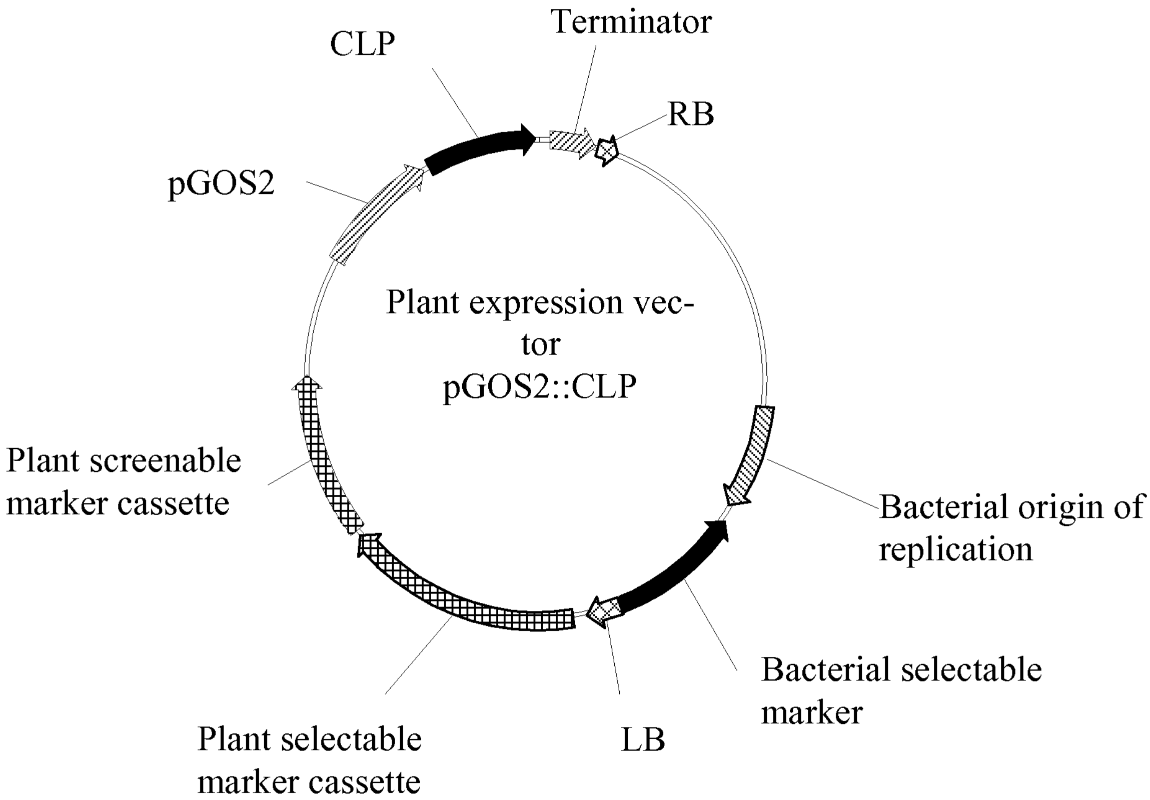
Terminator
CLP
RB
pGOS2
Plant expression vector
pGOS2::CLP
Plant screenable marker cassette
Bacterial origin of replication
Bacterial selectable marker
Plant selectable marker cassette
LB

FIGURE 6

Tables summarising the sequence information

**Table I: Nucleic acid sequence ID numbers**

| Application | 1. Hit | 2. Gene | 3. Organism | 4. Lead SEQ ID | 5. SEQ IDs of Nucleic Acid Homologs |
|---|---|---|---|---|---|
| 1 | 1 | CLP | A. th. | 1 | 3,5,7,9,11,13,15,17,19,21,23,25,27,29,31,33,35,37,39,41,43,45,47,49,51,53,55,57,59,61,63,65,67,69,71 |

Table II: Amino acid sequence ID numbers

| Application | 1. Hit | 2. Gene | 3. Organism | 4. Lead SEQ ID | 5. SEQ IDs of Polypeptide Homologs |
|---|---|---|---|---|---|
| 1 | 1 | CLP | Arabidopsis thaliana | 2 | 4,6,8,10,12,14,16,18,20,22,24,26,28,30,32,34,36,38,40,42,44,46,48,50,52,54,56,58,60,62,64,66,68,70,72 |

Table III: Primer nucleic acid sequence ID numbers

| Application | 1. Hit | 2. Gene | 3. Organism | 4. Lead SEQ ID | 5. SEQ IDs of Primers |
|---|---|---|---|---|---|
| 1 | 1 | CLP | Arabidopsis thaliana | 1 | 73,74 |

FIGURE 6 (continued)

Table IV: Consensus & motif amino acid sequence ID numbers

| Appli-cation | 1. Hit | 2. Locus | 3. Organism | 4. Lead SEQ ID | 5. SEQ IDs of Consensus / Pattern Sequences |
|---|---|---|---|---|---|
| 1 | 1 | At3g52490 | A. th. | 2 | 75,76,77,78,79,80,81,83,84,85 |

Europäisches Patentamt
European Patent Office
Office européen des brevets

# EUROPEAN SEARCH REPORT

Application Number
EP 12 17 3160

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DATABASE UniProt [Online]<br><br>1 May 2000 (2000-05-01), "SubName: Full=At3g52490; SubName: Full=Heat shock protein-like protein; SubName: Full=Putative uncharacterized protein F22O6_130;", XP002684596, retrieved from EBI accession no. UNIPROT:Q9SVD0<br>Database accession no. Q9SVD0<br>* sequence * | 8,9, 12-15 | INV. C12N15/82 |
| X | US 2009/100536 A1 (ADAMS THOMAS [US] ET AL) 16 April 2009 (2009-04-16)<br>* PEP SEQ ID NO: 425-427; page 9 * | 8,9, 12-15 | |
| A | WO 2010/046221 A1 (BASF PLANT SCIENCE GMBH [DE]; SCHOEN HARDY [DE]; THIMM OLIVER [DE]; RI) 29 April 2010 (2010-04-29)<br>* abstract * | 1-15 | TECHNICAL FIELDS SEARCHED (IPC) |
| A | M. E. BARNETT: "Structure and Activity of ClpB from Escherichia coli. ROLE OF THE AMINO- AND CARBOXYL-TERMINAL DOMAINS", JOURNAL OF BIOLOGICAL CHEMISTRY, vol. 275, no. 48, 22 November 2000 (2000-11-22), pages 37565-37571, XP55039792, ISSN: 0021-9258, DOI: 10.1074/jbc.M005211200<br>* the whole document * | 1-15 | C12N |

-/--

The present search report has been drawn up for all claims

| Place of search | Date of completion of the search | Examiner |
|---|---|---|
| Munich | 12 October 2012 | Bilang, Jürg |

4

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 12 17 3160

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| A | BO ZHENG ET AL: "A nuclear-encoded ClpP subunit of the chloroplast ATP-dependent Clp protease is essential for early development in Arabidopsis thaliana", PLANTA ; AN INTERNATIONAL JOURNAL OF PLANT BIOLOGY, SPRINGER, BERLIN, DE, vol. 224, no. 5, 17 May 2006 (2006-05-17), pages 1103-1115, XP019443310, ISSN: 1432-2048, DOI: 10.1007/S00425-006-0292-2<br>* abstract *<br>----- | 1-15 | |
| A | SHAI KOUSSEVITZKY ET AL: "An Arabidopsis thaliana virescent mutant reveals a role for ClpR1 in plastid development", PLANT MOLECULAR BIOLOGY, KLUWER ACADEMIC PUBLISHERS, DORDRECHT, NL, vol. 63, no. 1, 29 September 2006 (2006-09-29), pages 85-96, XP019453463, ISSN: 1573-5028, DOI: 10.1007/S11103-006-9074-2<br>* abstract *<br>----- | 1-15 | TECHNICAL FIELDS SEARCHED (IPC) |
| A | AMANJOT SINGH ET AL: "Plant Hsp100/ClpB-like proteins: poorly-analyzed cousins of yeast ClpB machine", PLANT MOLECULAR BIOLOGY, KLUWER ACADEMIC PUBLISHERS, DORDRECHT, NL, vol. 74, no. 4-5, 2 September 2010 (2010-09-02), pages 395-404, XP019828390, ISSN: 1573-5028<br>* the whole document *<br>-----<br>-/-- | 1-15 | |

The present search report has been drawn up for all claims

| Place of search | Date of completion of the search | Examiner |
|---|---|---|
| Munich | 12 October 2012 | Bilang, Jürg |

4

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 12 17 3160

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | SINGH AMANJOT ET AL: "Genome-wide analysis of rice ClpB/HSP100, ClpC and ClpD genes", BMC GENOMICS, BIOMED CENTRAL LTD, LONDON, UK, vol. 11, no. 1, 8 February 2010 (2010-02-08), page 95, XP021066224, ISSN: 1471-2164 * the whole document * ----- | 1-15 | |

TECHNICAL FIELDS SEARCHED (IPC)

The present search report has been drawn up for all claims

| Place of search | Date of completion of the search | Examiner |
|---|---|---|
| Munich | 12 October 2012 | Bilang, Jürg |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

4

EPO FORM 1503 03.82 (P04C01)

Europäisches Patentamt
European Patent Office
Office européen des brevets

Application Number
EP 12 17 3160

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☒ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches
Patentamt
European
Patent Office
Office européen
des brevets

# LACK OF UNITY OF INVENTION
# SHEET B

Application Number
EP 12 17 3160

The Search Division considers that the present European patent application does not comply with the requirements of unity of invention and relates to several inventions or groups of inventions, namely:

1. claims: 1-7, 10, 11(completely); 12-15(partially)

   Method of increasing yield in plants, comprising modulation of a CLP

   ---

2. claims: 8, 9, 12-15(all partially)

   Construct comprising a CLP having SEQ ID NO: 2

   ---

3-37. claims: 8, 9, 12-15(all partially)

   Construct comprising a CLP having SEQ ID NO: 4 to 72 (even numbers)

   ---

**ANNEX TO THE EUROPEAN SEARCH REPORT ON EUROPEAN PATENT APPLICATION NO.** EP 12 17 3160

This annex lists the patent family members relating to the patent documents cited in the above-mentioned European search report.
The members are as contained in the European Patent Office EDP file on
The European Patent Office is in no way liable for these particulars which are merely given for the purpose of information.

12-10-2012

| Patent document cited in search report | Publication date | Patent family member(s) | Publication date |
|---|---|---|---|
| US 2009100536 A1 | 16-04-2009 | US 2009100536 A1 | 16-04-2009 |
| | | US 2011258734 A1 | 20-10-2011 |
| WO 2010046221 A1 | 29-04-2010 | AR 076157 A1 | 26-05-2011 |
| | | AU 2009306575 A1 | 29-04-2010 |
| | | CA 2740257 A1 | 29-04-2010 |
| | | CN 102264907 A | 30-11-2011 |
| | | DE 112009002577 T5 | 21-06-2012 |
| | | EP 2350289 A1 | 03-08-2011 |
| | | US 2011321197 A1 | 29-12-2011 |
| | | WO 2010046221 A1 | 29-04-2010 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2011023537 A
- WO 2011023539 A
- EP 11172672 A
- EP 11172825 A
- EP 11181420 A
- US 5811238 A
- US 6395547 A
- WO 2004070039 A
- WO 2004065596 A
- US 4962028 A
- WO 0114572 A
- WO 9514098 A
- WO 9412015 A
- US 5401836 A
- US 20050044585 A
- EP 99106056 A
- US 5565350 A
- WO 0015815 A
- WO 9322443 A, Zarling
- WO 9853083 A, Grierson
- WO 9953050 A, Waterhouse
- US 4987071 A, Cech
- US 5116742 A, Cech
- WO 9400012 A, Atkins
- WO 9503404 A, Lenne
- WO 0000619 A, Lutziger
- WO 9713865 A, Prinsen
- WO 9738116 A, Scott
- WO 9836083 A
- WO 9915682 A, Baulcombe
- EP 1198985 A1
- WO 2007093444 A
- US 5164310 A
- US 5159135 A
- WO 9623891 A
- WO 2010151634 A
- EP 1831378 A
- WO 2010031780 A
- WO 2006029987 A


### Non-patent literature cited in the description


- **WANG et al.** *Planta,* 2003, vol. 218, 1-14
- Current Protocols in Molecular Biology
- **MANIATIS T ; FRITSCH EF ; SAMBROOK J.** Molecular Cloning: A Laboratory Manual. Cold Spring Harbor Laboratory, 1989
- **SILHAVY et al.** Experiments with Gene Fusions. Cold Spring Harbor Laboratory, 1984
- **AUSUBEL et al.** Current Protocols in Molecular Biology. Greene Publishing Assoc. and Wiley Interscience, 1987
- Plant Molecular Biology Manual. Kluwer Academic Publisher, 1990
- **CREIGHTON.** Proteins. W.H. Freeman and Company, 1984
- Current Protocols in Molecular Biology. John Wiley & Sons, 1989
- **TERPE.** *Appl. Microbiol. Biotechnol.,* 2003, vol. 60, 523-533
- **SCHULTZ et al.** *Proc. Natl. Acad. Sci. USA,* 1998, vol. 95, 5857-5864
- **LETUNIC et al.** *Nucleic Acids Res,* 2002, vol. 30, 242-244
- **MULDER et al.** *Nucl. Acids. Res.,* 2003, vol. 31, 315-318
- A generalized profile syntax for biomolecular sequences motifs and its function in automatic sequence interpretation. **BUCHER ; BAIROCH.** ISMB-94; Proceedings 2nd International Conference on Intelligent Systems for Molecular Biology. AAAI Press, 1994, 53-61
- **HULO et al.** *Nucl. Acids. Res.,* 2004, vol. 32, D134-D137
- **BATEMAN et al.** *Nucleic Acids Research,* 2002, vol. 30 (1), 276-280
- **R.D. FINN ; J. MISTRY ; J. TATE ; P. COGGILL ; A. HEGER ; J.E. POLLINGTON ; O.L. GAVIN ; P. GUNESEKARAN ; G. CERIC ; K. FORSLUND.** *Nucleic Acids Research,* 2010, 211-222
- **GASTEIGER et al.** ExPASy: the proteomics server for in-depth protein knowledge and analysis. *Nucleic Acids Res.,* 2003, vol. 31, 3784-3788
- **NEEDLEMAN ; WUNSCH.** *J Mol Biol,* 1970, vol. 48, 443-453
- **ALTSCHUL et al.** *J Mol Biol,* 1990, vol. 215, 403-10
- **CAMPANELLA et al.** *BMC Bioinformatics,* 10 July 2003, vol. 4, 29
- **SMITH TF ; WATERMAN MS.** *J. Mol. Biol,* 1981, vol. 147 (1), 195-7

- **MEINKOTH ; WAHL.** *Anal. Biochem.,* 1984, vol. 138, 267-284
- **SAMBROOK et al.** Molecular Cloning: a laboratory manual. Cold Spring Harbor Laboratory Press, 2001
- **FOISSAC ; SCHIEX.** *BMC Bioinformatics,* 2005, vol. 6, 25
- **CASTLE et al.** *Science,* 2004, vol. 304 (5674), 1151-4
- **HEID et al.** *Genome Methods,* 1996, vol. 6, 986-994
- **MCELROY et al.** *Plant Cell,* 1990, vol. 2, 163-171
- **ODELL et al.** *Nature,* 1985, vol. 313, 810-812
- **NILSSON et al.** *Physiol. Plant,* 1997, vol. 100, 456-462
- **DE PATER et al.** *Plant J Nov,* vol. 2 (6), 837-44
- **CHRISTENSEN et al.** *Plant Mol. Biol.,* 1992, vol. 18, 675-689
- **BUCHHOLZ et al.** *Plant Mol Biol.,* 1994, vol. 25 (5), 837-43
- **LEPETIT et al.** *Mol. Gen. Genet.,* 1992, vol. 231, 276-285
- **WU et al.** *Plant Mol. Biol.,* 1988, vol. 11, 641-649
- **AN et al.** *Plant J.,* 1996, vol. 10 (1), 107-121
- **SANGER et al.** *Plant. Mol. Biol.,* 1990, vol. 14, 433-443
- **LEISNER.** *Proc Natl Acad Sci USA,* 1988, vol. 85 (5), 2553
- **JAIN et al.** *Crop Science,* 1999, vol. 39 (6), 1696
- **SHAW et al.** *Nucleic Acids Res.,* 1984, vol. 12 (20), 7831-7846
- **GATZ.** *Annu. Rev. Plant Physiol. Plant Mol. Biol.,* 1997, vol. 48, 89-108
- *Plant Mol Biol.,* January 1995, vol. 27 (2), 237-48
- **KOYAMA et al.** *J Biosci Bioeng.,* January 2005, vol. 99 (1), 38-42
- **MUDGE et al.** *Plant J.,* 2002, vol. 31, 341
- **XIAO et al.** *Plant Biol (Stuttg,* July 2006, vol. 8 (4), 439-49
- **NITZ et al.** *Plant Sci,* 2001, vol. 161 (2), 337-346
- **TINGEY et al.** *EMBO J.,* 1987, vol. 6, 1
- **VAN DER ZAAL et al.** *Plant Mol. Biol.,* 1991, vol. 16, 983
- **OPPENHEIMER et al.** *Gene,* 1988, vol. 63, 87
- **CONKLING et al.** *Plant Physiol.,* 1990, vol. 93, 1203
- **SUZUKI et al.** *Plant Mol. Biol.,* 1993, vol. 21, 109-119
- **BAUMBERGER et al.** *Genes & Dev.,* 2001, vol. 15, 1128
- **LAUTER et al.** *PNAS,* 1996, vol. 3, 8139
- **LIU et al.** *Plant Mol. Biol.,* vol. 17 (6), 1139-1154
- **DOWNEY et al.** *J. Biol. Chem.,* 2000, vol. 275, 39420
- **W SONG.** PhD Thesis. North Carolina State University, 1997
- **WANG et al.** *Plant Sci.,* 2002, vol. 163, 273
- **DIENER et al.** *Plant Cell,* 2001, vol. 13, 1625
- **QUESADA et al.** *Plant Mol. Biol.,* 1997, vol. 34, 265
- **QING QU ; TAKAIWA.** *Plant Biotechnol. J.,* 2004, vol. 2, 113-125
- **SIMON et al.** *Plant Mol. Biol.,* 1985, vol. 5, 191
- **SCOFIELD et al.** *J. Biol. Chem.,* 1987, vol. 262, 12202
- **BASZCZYNSKI et al.** *Plant Mol. Biol.,* 1990, vol. 14, 633
- **PEARSON et al.** *Plant Mol. Biol.,* 1992, vol. 18, 235-245
- **ELLIS et al.** *Plant Mol. Biol.,* 1988, vol. 10, 203-214
- **TAKAIWA et al.** *Mol. Gen. Genet.,* 1986, vol. 208, 15-22
- **TAKAIWA et al.** *FEBS Letts.,* 1987, vol. 221, 43-47
- **MATZKE et al.** *Plant Mol Biol,* 1990, vol. 14 (3), 323-32
- **STALBERG et al.** *Planta,* 1996, vol. 199, 515-519
- *Mol Gen Genet,* 1989, vol. 216, 81-90
- *NAR,* 1989, vol. 17, 461-2
- **ALBANI et al.** *Plant Cell,* 1997, vol. 9, 171-184
- *EMBO J.,* 1984, vol. 3, 1409-15
- **DIAZ et al.** *Mol Gen Genet,* 1995, vol. 248 (5), 592-8
- *Theor Appl Gen,* 1999, vol. 98, 1253-62
- *Plant J,* 1993, vol. 4, 343-55
- *Mol Gen Genet,* 1996, vol. 250, 750-60
- **MENA et al.** *The Plant Journal,* 1998, vol. 116 (1), 53-62
- **VICENTE-CARBAJOSA et al.** *Plant J.,* 1998, vol. 13, 629-640
- **WU et al.** *Plant Cell Physiology,* 1998, vol. 39 (8), 885-889
- **SATO et al.** *Proc. Natl. Acad. Sci. USA,* 1996, vol. 93, 8117-8122
- **NAKASE et al.** *Plant Mol. Biol.,* 1997, vol. 33, 513-522
- *Trans Res,* 1997, vol. 6, 157-68
- *Plant J,* 1997, vol. 12, 235-46
- **DEROSE et al.** *Plant Mol. Biol,* 1996, vol. 32, 1029-35
- **POSTMA-HAARSMA et al.** *Plant Mol. Biol.,* 1999, vol. 39, 257-71
- **WU et al.** *J. Biochem.,* 1998, vol. 123, 386
- **CUMMINS et al.** *Plant Mol. Biol.,* 1992, vol. 19, 873-876
- **LANAHAN et al.** *Plant Cell,* 1992, vol. 4, 203-211

- **SKRIVER et al.** *Proc Natl Acad Sci USA,* 1991, vol. 88, 7266-7270
- **CEJUDO et al.** *Plant Mol Biol,* 1992, vol. 20, 849-856
- **KALLA et al.** *Plant J.,* 1994, vol. 6, 849-60
- **LEAH et al.** *Plant J.,* 1994, vol. 4, 579-89
- **SELINGER et al.** *Genetics,* 1998, vol. 149, 1125-38
- **TAKAIWA et al.** *Mol Gen Genet,* 1986, vol. 208, 15-22
- **COLOT et al.** *Mol Gen Genet,* 1989, vol. 216, 81-90
- **ANDERSON et al.** *NAR,* 1989, vol. 17, 461-2
- **RAFALSKI et al.** *EMBO,* 1984, vol. 3, 1409-15
- **CHO et al.** *Theor Appl Genet,* 1999, vol. 98, 1253-62
- **MULLER et al.** *Plant J,* 1993, vol. 4, 343-55
- **SORENSON et al.** *Mol Gen Genet,* 1996, vol. 250, 750-60
- **MENA et al.** *Plant J,* 1998, vol. 116 (1), 53-62
- **ONATE et al.** *J Biol Chem,* 1999, vol. 274 (14), 9175-82
- **VICENTE-CARBAJOSA et al.** *Plant J,* 1998, vol. 13, 629-640
- **WU et al.** *Plant Cell Physiol,* 1998, vol. 39 (8), 885-889
- **NAKASE et al.** *Plant Molec Biol,* 1997, vol. 33, 513-522
- **RUSSELL et al.** *Trans Res,* 1997, vol. 6, 157-68
- **OPSAHL-FERSTAD et al.** *Plant J,* 1997, vol. 12, 235-46
- **DEROSE et al.** *Plant Mol Biol,* 1996, vol. 32, 1029-35
- **SKIVER et al.** *Proc Natl Acad Sci USA,* 1991, vol. 88, 7266-7270
- **FUKAVAMA.** *Plant Physiol.,* November 2001, vol. 127 (3), 1136-46
- **KAUSCH et al.** *Plant Mol Biol.,* January 2001, vol. 45 (1), 1-15
- **LIN et al.** *DNA Seq.,* August 2004, vol. 15 (4), 269-76
- **NOMURA et al.** *Plant Mol Biol.,* September 2000, vol. 44 (1), 99-106
- **PANGULURI et al.** *Indian J Exp Biol.,* April 2005, vol. 43 (4), 369-72
- **WAGNER ; KOHORN.** *Plant Cell,* 2001, vol. 13 (2), 303-318
- **TRIBBLE et al.** *J. Biol. Chem.,* 2000, vol. 275, 22255-22267
- **VELMURUGAN et al.** *J. Cell Biol.,* 2000, vol. 149, 553-566
- **BUCHMAN ; BERG.** *Mol. Cell biol.,* 1988, vol. 8, 4395-4405
- **CALLIS et al.** *Genes Dev,* 1987, vol. 1, 1183-1200
- The Maize Handbook. Springer, 1994
- **GAULTIER et al.** *Nucl Ac Res,* 1987, vol. 15, 6625-6641
- **INOUE et al.** *Nucl Ac Res,* 1987, vol. 15, 6131-6148
- **INOUE et al.** *FEBS Lett.,* 1987, vol. 215, 327-330
- **HASELHOFF ; GERLACH.** *Nature,* 1988, vol. 334, 585-591
- **BARTEL ; SZOSTAK.** *Science,* 1993, vol. 261, 1411-1418
- **ANGELL ; BAULCOMBE.** *Plant J,* 1999, vol. 20 (3), 357-62
- **HELENE, C.** *Anticancer Drug Res.,* 1991, vol. 6, 569-84
- **HELENE et al.** *Ann. N.Y. Acad. Sci.,* 1992, vol. 660, 27-36
- **MAHER, L.J.** *Bioassays,* 1992, vol. 14, 807-15
- **SCHWAB et al.** *Dev. Cell,* 2005, vol. 8, 517-527
- **SCHWAB et al.** *Plant Cell,* 2006, vol. 18, 1121-1133
- **KRENS, F.A. et al.** *Nature,* 1982, vol. 296, 72-74
- **NEGRUTIU I et al.** *Plant Mol Biol,* 1987, vol. 8, 363-373
- **SHILLITO R.D. et al.** *Bio/Technol,* 1985, vol. 3, 1099-1102
- **CROSSWAY A et al.** *Mol. Gen Genet,* 1986, vol. 202, 179-185
- **KLEIN TM et al.** *Nature,* 1987, vol. 327, 70
- **CLOUGH ; BENT.** *Plant J.,* 1998, vol. 16, 735-743
- **ALDEMITA ; HODGES.** *Planta,* 1996, vol. 199, 612-617
- **CHAN et al.** *Plant Mol Biol,* 1993, vol. 22 (3), 491-506
- **HIEI et al.** *Plant J,* 1994, vol. 6 (2), 271-282
- **ISHIDA et al.** *Nat. Biotechnol,* 1996, vol. 14 (6), 745-50
- **FRAME et al.** *Plant Physiol,* 2002, vol. 129 (1), 13-22
- Techniques for Gene Transfer. **B. JENES et al.** Transgenic Plants, Vol. 1, Engineering and Utilization. Academic Press, 1993, vol. 1, 128-143
- *Potrykus Annu. Rev. Plant Physiol. Plant Molec. Biol.,* 1991, vol. 42, 205-225
- **BEVAN et al.** *Nucl. Acids Res.,* 1984, vol. 12, 8711
- **HÖFGEN ; WILLMITZER.** *Nucl. Acid Res.,* 1988, vol. 16, 9877
- Vectors for Gene Transfer in Higher Plants. **F.F. WHITE.** Transgenic Plants, Vol. 1, Engineering and Utilization. Academic Press, 1993, 15-38
- **FELDMAN, KA ; MARKS MD.** *Mol Gen Genet,* 1987, vol. 208, 1-9
- **FELDMANN K.** Methods in Arabidopsis Research. Word Scientific, 1992, 274-289

- **CHANG.** *Plant J.,* 1994, vol. 5, 551-558
- **KATAVIC.** *Mol Gen Genet,* 1994, vol. 245, 363-370
- **BECHTHOLD, N.** *C R Acad Sci Paris Life Sci,* 1993, vol. 316, 1194-1199
- **CLOUGH, SJ ; BENT AF.** *The Plant J.,* 1998, vol. 16, 735-743
- **KLAUS et al.** *Nature Biotechnology,* 2004, vol. 22 (2), 225-229
- **BOCK.** Transgenic plastids in basic research and plant biotechnology. *J Mol Biol.,* 21 September 2001, vol. 312 (3), 425-38
- **MALIGA, P.** Progress towards commercialization of plastid transformation technology. *Trends Biotechnol.,* 2003, vol. 21, 20-28
- **HAYASHI et al.** *Science,* 1992, 1350-1353
- **REDEI GP ; KONCZ C.** Methods in Arabidopsis Research. World Scientific Publishing Co, 1992, 16-82
- **FELDMANN et al.** Arabidopsis. Cold Spring Harbor Laboratory Press, 1994, 137-172
- **LIGHTNER J ; CASPAR T.** Methods on Molecular Biology. Humana Press, 1998, vol. 82, 91-104
- **MCCALLUM et al.** *Nat Biotechnol,* 2000, vol. 18, 455-457
- **STEMPLE.** *Nat Rev Genet,* 2004, vol. 5 (2), 145-50
- **OFFRINGA et al.** *EMBO J,* 1990, vol. 9 (10), 3077-84
- **TERADA et al.** *Nat Biotech,* 2002, vol. 20 (10), 1030-4
- **IIDA ; TERADA.** *Curr Opin Biotech,* 2004, vol. 15 (2), 132-8
- **MILLER et al.** *Nature Biotechnol.,* 2007, vol. 25, 778-785
- **RABBANI et al.** *Plant Physiol,* 2003, vol. 133, 1755-1767
- **SAMBROOK J ; FRITSCH EF ; MANIATIS T.** Molecular Cloning, A Laboratory Manual. 1989
- **LANDER et al.** *Genomics,* 1987, vol. 1, 174-181
- **BOTSTEIN et al.** *Am. J. Hum. Genet.,* 1980, vol. 32, 314-331
- **BERNATZKY ; TANKSLEY.** *Plant Mol. Biol. Reporter,* 1986, vol. 4, 37-41
- **HOHEISEL et al.** Non-mammalian Genomic Analysis: A Practical Guide. Academic press, 1996, 319-346
- **TRASK.** *Trends Genet.,* 1991, vol. 7, 149-154
- **LAAN et al.** *Genome Res.,* 1995, vol. 5, 13-20
- **KAZAZIAN.** *J. Lab. Clin. Med,* 1989, vol. 11, 95-96
- **SHEFFIELD et al.** *Genomics,* 1993, vol. 16, 325-332
- **LANDEGREN et al.** *Science,* 1988, vol. 241, 1077-1080
- **SOKOLOV.** *Nucleic Acid Res.,* 1990, vol. 18, 3671
- **WALTER et al.** *Nat. Genet.,* 1997, vol. 7, 22-28
- **DEAR ; COOK.** *Nucleic Acid Res.,* 1989, vol. 17, 6795-6807
- **SAMBROOK.** Molecular Cloning: a laboratory manual. Cold Spring Harbor Laboratory Press, 2001
- Current Protocols in Molecular Biology, Current Protocols. 1994, vol. 1, 2
- **R.D.D. CROY.** Plant Molecular Biology Labfax. BIOS Scientific Publications Ltd, 1993
- **ALTSCHUL et al.** *J. Mol. Biol.,* 1990, vol. 215, 403-410
- **ALTSCHUL et al.** *Nucleic Acids Res.,* 1997, vol. 25, 3389-3402
- **THOMPSON et al.** *Nucleic Acids Research,* 1994, vol. 22, 4673
- **ZDOBNOV E.M. ; APWEILER R.** InterProScan - an integration platform for the signature-recognition methods in InterPro. *Bioinformatics,* 2001, vol. 17 (9), 847-8
- Fitting a mixture model by expectation maximization to discover motifs in biopolymers. **TIMOTHY L. BAILEY ; CHARLES ELKAN.** Pro-ceedings of the Second International Conference on Intelligent Systems for Mo-lecular Biology. AAAI Press, 1994, 28-36
- **I.JONASSEN ; J.F.COLLINS ; D.G.HIGGINS.** Finding flexible patterns in unaligned protein sequences. *Protein Science,* 1995, vol. 4, 1587-1595
- **I.JONASSEN.** Effi-cient discovery of conserved patterns using a pattern graph. *CABIOS,* February 1997
- *Trends in Genetics,* 2000, vol. 16 (6), 276
- **OLOF EMANUELSSON ; SØREN BRUNAK ; GUNNAR VON HEIJNE ; HENRIK NIELSEN.** Locating proteins in the cell using TargetP, SignalP, and related tools. *Nature Protocols,* 2007, vol. 2, 953-971, http://www.cbs.dtu.dk/services/TargetP
- **PARK ; KANEHISA.** *Bioinformatics,* 2003, vol. 19, 1656-1663
- **ISHIDA et al.** *Nature Biotech,* 1996, vol. 14 (6), 745-50
- **BABIC et al.** *Plant Cell Rep,* 1998, vol. 17, 183-188
- **MCKERSIE et al.** *Plant Physiol,* 1999, vol. 119, 839-847
- **BROWN DCW ; A ATANASSOV.** *Plant Cell Tissue Organ Culture,* 1985, vol. 4, 111-112
- **WALKER et al.** *Am J Bot,* 1978, vol. 65, 654-659
- **GAMBORG et al.** *Exp. Cell Res.,* 1968, vol. 50, 151-158
- **MURASHIGE, T. ; SKOOG, .** *Physiol. Plant,* 1962, vol. 15, 473-497
- **GAMBORG et al.** *Exp. Cell Res.,* vol. 50, 151-8

- **HUSSEY, G. ; HEPHER, A.** Annals of Botany. 1978, vol. 42, 477-9
- **LINSEY, K. ; GALLOIS, P.** *Journal of Experimental Botany,* 1990, vol. 41 (226), 529-36
- **ARENCIBIA et al.** *Transgenic Research,* 1998, vol. 7, 213-22
- **ENRIQUEZ-OBREGON et al.** *Planta,* 1998, vol. 206, 20-27
- **GAMBORG, O. et al.** *Exp. Cell Res.,* 1968, vol. 50, 151-8
- **SNYMAN et al.** *S. Afr. J. Bot,* 1996, vol. 62, 151-154
- **BECK et al.** *Gene,* 1982, vol. 19, 327-336
- **LAST et al.** *Theor. Appl. Genet.,* 1991, vol. 81, 581-588
- **SNYMAN et al.** *Acta Horticulturae,* 2001, vol. 560, 105-108